# EUROPEAN PATENT APPLICATION

(11) **EP 2 383 343 A2**
(43) Date of publication of application: **02.11.2011**
(21) Application number: 11153574.6
(22) Date of filing: 03.03.2005
(51) Int. Cl.: C12N 15/38, A61K 39/25, A61P 31/22, C12N 5/10, C12N 7/00, C07K 14/04, C12N 7/04

(54) **Recombinant varicella-zoster virus**

(30) Priority: 05.03.2004 JP 2004063277
(62) Divisional of application: 05719956.4
(71) Applicant: The Research Foundation for Microbial Diseases of Osaka University, Osaka 565-0871 (JP)
(72) Inventor: Nagaike, Kazuhiro, Kagawa 7680100 (JP); Mori, Yasuko, Osaka 5670046 (JP); Gomi, Yasuyuki, Kagawa 7680001 (JP); Takahashi, Michiaki, Osaka 5650875 (JP); Yamanishi, Kouichi, Osaka 5630104 (JP)
(74) Representative: Train, Matthew

(57) **Abstract**

A recombinant varicella-zoster virus; a process for producing the same; a pharmacological composition containing recombinant varicella-zoster virus; a vector containing a genomic gene of varicella-zoster virus and BAC vector sequence; cells containing the above vector; a fragment capable of homologous recombination with a genome of varicella-zoster virus; and a nucleic acid cassette containing the BAC vector sequence. For these, there is provided a process for producing recombinant varicella-zoster virus, comprising use of the BAC vector sequence.

## Description

### BACKGROUND OF THE INVENTION

### 1. FIELD OF THE INVENTION:

The present invention relates to recombinant varicella-zoster virus,particularly recombinant varicella-zoster virusprepared using BAC (*E. coli* artificial chromosome), and a pharmaceutical composition comprising such a virus. Further, the present invention relates to a vector comprising a varicella-zoster virus genomic gene and a BAC vector sequence, and a cell containing such a vector. Further, the present invention relates to a method for producing recombinant varicella-zoster virus. Further, the present invention relates to a nucleic acid cassette comprising a fragment capable of homologous recombination with a varicella-zoster virus genome, and a BAC vector sequence.

### 2. DESCRIPTION OF THE RELATED ART:

Varicella-zoster virus (VZV) is a virus which belongs to viruses of the family *Herpesviridae*, and is responsible for diseases (varicella and zoster) which exhibit two different presentations. Early infection to this virus causes varicella (chicken pox). Then, the virus latently infects the ganglion. After a long period of time, this virus is reactivated by some cause, and then presents as zoster, which is a symptom that presents when virus particles are formed, the virus particles arrive at the epidermic cell through a nerve cell and form varicella in the region where nerve cells are present.

The VZV genome is double-stranded DNA of about 125000 bases. The whole base sequence has been determined by Davison *et al.* It is known that at least 72 genes are present on the genome.

The development of VZV vaccine is difficult. Oka strain of VZV vaccine is the one and only vaccine for a varicella virus in the world, developed by Takahashi, *et al.* (Japanese Laid-Open Publication No. 53-41202). The existing attenuated live varicella vaccine has been produced by employing virus derived from the attenuated live varicella virus Oka strain used as a seed, and has been practiced all over the world (Requirements for Varicella Vaccine (Live) Adopted 1984; Revised 1993: WHO Technical Report Series, No. 848, pp.22-38, 1994). This Oka strain is obtained from a virus (Oka original strain) isolated from an affected infant that presents typical varicella, by passage through several generations employing human diploid cell after passage through 12 generations, employing human embryonic lung cell at 34 centigrade, and through 11 generations by employing guinea-pig embryonal cell. The Oka original strain is of high pathogenicity. On the contrary, it is recognized that the Oka vaccine strain (Oka strain) has very little side-effects to a normal child. As such, the Oka strain is useful as a vaccine strain having very little pathogenicity.

A virus vaccine has a possibility of changing its genotype of the virus through passage. There is also a possibility that the Oka strain has a genetic variety because a lot of passages are done in the process of preparing the Oka strain. Practically, to ensure its safety and effectiveness, considering genetic changes of a virus through passages in the process of producing a vaccine, a seed lot system has been established that limits the number of passages of a varicella seed virus which is approved to be produced, that is, employing a virus as a vaccine within 10 generations from the total number of passages, on the basis that the number of passages is 0 at the time of approval of seed.

On the other hand, from the follow-up of the effect of a varicella vaccine and post-marketing Surveillance (PMS), or in terms of epidemiology, the analysis of virological difference between a fresh field strain of varicella virus which is separated from a varicella patient by natural infection and a vaccine strain derived from the above-mentioned Oka strain has been necessary, resulting in that the various analyses employing the techniques from immunology, genetic engineering, and the like have been already done. For example, the judgment based on trials, such as the differences of gene structures, DNA base sequences, and the like between varicella virus strains (Journal of General Virology, 59, 660-668, 1986; 67, 1759-1816, 1986), presence of restriction enzyme Pst I site (Japanese Journal of Experimental Medicine, 59, 233-237, 1989), RFLP (Restriction Fragment Length Polymorphism) employing PCR (Polymerase Chain Reaction) (Journal of Virology, 66, 1016-1020, 1992), and the combination of the above-mentioned presence of Pst I site and RFLP (Journal of Clinical Microbiology, 33, 658-660, 1995), have been reported. Although these trials propose conditions for identifying a fresh field strain from the vaccine strain derived from the Oka strain, it lacks the reliability and is not conclusive because of the genetic variety of the Oka Strain itself, and therefore, there still exist problems in terms of quality control. Further, the method of identifying Oka strain of a varicella virus by employing the gene 14 region of a varicella virus (US Patent No. 6,093,535), the method of identifying a virus strain for an attenuated live varicella vaccine by employing the gene 62 region (International Publication No. WO 00/50603), and the like are also known. These techniques make it possible to identify the differences among the following three strains; the Oka strain of varicella virus (high virulent parent strain), a vaccine strain derived therefrom (an attenuated Oka strain), and a varicella virus strain other than the Oka strain. However, the criterion of preparation of an attenuated live varicella vaccine for quality control and quality assurance would not be sufficient.

As it is now, quality control by evaluating and identifying the quality of vaccine, such as by direct or quantitative gene analysis for genome DNA of seed virus and vaccine virus has not been practiced. Therefore, the accuracy of quality control and quality assurance of an attenuated strain for live vaccine cannot be calculated, and therefore, is ambiguous. Accordingly, to increase the accuracy of quality control and quality assurance is extremely important to secure and ensure the effectiveness, safety, and homogeneity of an attenuated live varicella vaccine. However, as mentioned above, the method for the foregoing has not been established, and the problems have still remained to be solved as tasks of pressing urgency.

Also, to develop an altered varicella-zoster virus vaccine which is superior to the Oka strain, recombinant varicella-zoster virus by mutagenesis, and a production method thereof has been desired.

### DISCLOSURE OF THE INVENTION

### THE PROBLEM TO BE SOLVED BY THE INVENTION

An object of the present invention is to increase the accuracy of quality control and quality assurance, and securing and ensuring the effectiveness, safety, and homogeneity of an attenuated live varicella vaccine. Further, a problem to be solved by the present invention is, in order to develop variant varicella-zoster virus vaccine superior to Oka strain, establishing a method to produce recombinant varicella-zoster virus by mutagenesis, and producing such a virus.

To achieve this, the present invention provides recombinant varicella-zoster virus, and a production method thereof, e.g., a method for producing recombinant varicella-zoster virus from a single virus strain using a BAC (Bacterial artificial chromosome).

### SUMMARY OF THE INVENTION

The present inventors developed a method for producing recombinant varicella-zoster virus using a BAC vector sequence to complete the present invention.

Therefore, the present invention provides the following.
1. A recombinant varicella-zoster virus.
2. A recombinant varicella-zoster virus of item 1, comprising a BAC vector sequence.
3. The recombinant varicella-zoster virus of item 2, wherein at least part of the BAC vector sequence is inserted into a non-essential region of a varicella-zoster virus genome.
4. The recombinant varicella-zoster virus of item 3, wherein the non-essential region is selected from the group consisting of the following regions:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12,
   the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38,
   the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56,
   the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
5. The recombinant varicella-zoster virus of item 4, wherein the non-essential region is the region flanking the ORF of gene 11, or the region flanking the ORF of gene 12.
6. The recombinant varicella-zoster virus of item 2, wherein at least part of the BAC vector sequence is inserted into the region in the ORF of gene 62 of a varicella-zoster virus genome.
7. The recombinant varicella-zoster virus of item 2, wherein the BAC vector sequence comprises a recombinant protein dependent recombinant sequence.
8. The recombinant varicella-zoster virus of item 2, wherein the BAC vector sequence comprises a selectable marker.
9. The recombinant varicella-zoster virus of item 8, wherein the selectable marker is a drug selectable marker.
10. The recombinant varicella-zoster virus of item 2, wherein the selectable marker is a gene encoding green fluorescent protein.
11. The recombinant varicella-zoster virus of item 2, wherein the varicella-zoster virus genome is derived from a wild type strain.
12. The recombinant varicella-zoster virus of item 2, wherein the varicella-zoster virus genome is derived from a mutant type strain.
13. The recombinant varicella-zoster virus of item 2, wherein the varicella-zoster virus genome is derived from Oka vaccine strain.
14. The recombinant varicella-zoster virus of item 2, wherein the varicella-zoster virus genome has mutations in gene 62 and gene 6.
15. The recombinant varicella-zoster virus of item 14, wherein the gene 62 comprises at least the base substitutions of the following (a) - (d) in SEQ ID NO. 5:
   (a) base substitution at position 2110 for G;
   (b) base substitution at position 3100 for G;
   (c) base substitution at position 3818 for C; and
   (d) base substitution at position 4006 for G,
   and, the gene 6 comprises at least a base substitution at porision 5745 for G, in SEQ ID NO. 8.
16. The recombinant varicella-zoster virus of item 2, wherein the BAC vector sequence comprises the sequence set forth in SEQ ID NO. : 7.
17. A pharmaceutical composition comprising the virus of item 1.
18. The pharmaceutical composition of item 17, wherein the composition is in the form of a vaccine.
19. A vector comprising a varicella-zoster virus essential gene and a BAC vector sequence other than the gene 62.
20. The vector of item 19, further comprising the gene 62.
21. The vector of item 19, wherein a mammalian cell produces a varicella-zoster virus when the vector is introduced into the mammalian cell.
22. The vector of item 19, wherein a portion where a sequence derived from the varicella-zoster virus genome is linked to the BAC vector sequence is within a non-essential region of the varicella-zoster virus genome.
23. The vector of item 22, wherein the non-essential region is selected from the group consisting of the following regions of:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
24. The vector of item 23, wherein the portion for linking is the region flanking the ORF of gene 11 and the region flanking the ORF of gene 12.
25. The vector of item 19, wherein a portion where a sequence derived from the varicella-zoster virus genome is linked to the BAC vector sequence is in the ORF of gene 62 of the varicella-zoster virus genome.
26. The vector of item 19, wherein the BAC vector sequence comprises recombinant protein dependent recombinant sequence.
27. The vector of item 19, wherein the BAC vector sequence comprises a selectable marker.
28. The vector of item 27, wherein the selectable marker is drug selectable marker.
29. The vector of item 27, wherein the selectable marker is a gene encoding green fluorescent protein.
30. The vector of item 19, wherein the varicella-zoster virus genome is derived from a wild type strain.
31. The vector of item 19, wherein the varicella-zoster virus genome is derived from a mutant type strain.
32. The vector of item 19, wherein the varicella-zoster virus genome is derived from Oka vaccine strain.
33. The vector of item 19, wherein the varicella-zoster virus genome has mutations in gene 62 and gene 6.
34. The vector of item 33, wherein the gene 62 comprises at least the base substitutions of the following (a)-(d) in SEQ ID NO. 5:
   (a) base substitution at position 2110 for G;
   (b) base substitution at position 3100 for G;
   (c) base substitution at position 3818 for C; and
   (d) base substitution at position 4006 for G,
   and, the gene 6 comprises at least a base substitution at position 5745 for G, in SEQ ID NO. 8.
35. The vector of item 19, wherein the BAC vector sequence comprises the sequence set forth in SEQ ID NO.: 7.
36. A cell comprising the vector of item 19.
37. The cell of item 36, wherein the cell is a bacterial cell.
38. The bacterial cell of item 37, wherein the bacterial cell is *E*. *coli*.
39. The cell of item 36, wherein the cell is a mammalian cell.
40. The mammalian cell of item 39, wherein the mammalian cell is derived from human.
41. A virus produced by the mammalian cell of item 39.
42. A pharmaceutical composition comprising the virus of item 41.
43. The pharmaceutical composition of item 42, wherein the composition is in the form of a vaccine.
44. A method to produce recombinant varicella-zoster virus, comprising:
   introducing a vector comprising a varicella-zoster virus genome essential gene other than gene 62 and a BAC vector sequence into a mammalian host cell; and
   culturing the mammalian host cell to produce recombinant varicella-zoster virus.
45. The method of item 44, wherein the vector further comprises the gene 62.
46. The method of item 44, wherein the mammalian host cell is derived from human.
47. The method of item 44, wherein the BAC vector sequence comprises at least two recombinant protein dependent recombinant sequences.
48. The method of item 47, further comprising a step of recombination between the two recombinant protein dependent recombinant sequences.
49. The method of item 44, wherein a portion where a sequence derived from the varicella-zoster virus genome is linked to the BAC vector sequence is within a non-essential region of the varicella-zoster virus genome.
50. The method of item 49, wherein the non-essential region is selected from the group consisting of the following regions of:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12,
   the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
51. The vector of item 50, wherein the portion which is linked is the region flanking the ORF of gene 11 or the region flanking the ORF of gene 12.
52. The vector of item 44, wherein a portion where a sequence derived from the varicella-zoster virus genome is linked to the BAC vector sequence is in the ORF of gene 62 of the varicella-zoster virus genome.
53. The method of item 44, wherein the BAC vector sequence comprises a recombinant protein dependent recombinant sequence.
54. The method of item 44, wherein the BAC vector sequence comprises a selectable marker.
55. The method of item 54, wherein the selectable marker is a drug selectable marker.
56. The method of item 54, wherein the selectable marker is a gene encoding green fluorescent protein.
57. The method of item 44, wherein the varicella-zoster virus genome is derived from a wild type strain.
58. The method of item 44, wherein the varicella-zoster virus genome is derived from a mutant type strain.
59. The method of item 44, wherein the varicella-zoster virus genome is derived from Oka vaccine strain.
60. The method of item 44, wherein the varicella-zoster virus genome has mutations in gene 62 and gene 6.
61. The method of item 60, wherein the gene 62 comprises at least the base substitutions of the following (a)-(d) in SEQ ID NO. 5:
   (a) base substitution at position 2110 for G;
   (b) base substitution at position 3100 for G;
   (c) base substitution at position 3818 for C; and
   (d) base substitution at position 4006 for G,
   and, the gene 6 comprises at least a base substitution at position 5745 for G, in SEQ ID NO. 8.
62. The method of item 44, wherein the BAC vector sequence comprises the sequence set forth in SEQ ID NO.: 7.
63. A virus produced by the method of item 44.
64. A pharmaceutical composition comprising the virus of item 63.
65. The pharmaceutical composition of item 64, wherein the composition is in the form of a vaccine.
66. A method to introduce a mutation into the vector of item 19, comprising:
   introducing the vector into a bacterial host cell;
   introducing a plasmid vector comprising a fragment consisting of a portion of varicella-zoster virus genome into the bacterial host cell, wherein the fragment has at least one mutation;
   culturing the bacterial host cell;
   isolating a vector having BAC sequence from the cultured bacterial host cell.
67. A method to introduce a mutation into the vector of item 19, comprising:
   introducing the vector into a bacterial host cell;
   introducing a first plasmid vector comprising a first fragment consisting of a portion of varicella-zoster virus genome into the bacterial host cell, wherein the first fragment has at least one mutation;
   introducing a second plasmid vector comprising a second fragment consisting of a portion of varicella-zoster virus genome into the bacterial host cell, wherein the second fragment has at least one mutation, and the first fragment is different from the second fragment;
   culturing the bacterial host cell;
   isolating a vector having BAC sequence from the cultured bacterial host cell.
68. A nucleic acid cassette comprising a first fragment which can recombine with varicella-zoster virus genome in a bacterial cell, BAC vector sequence, and a second fragment which can recombine with varicella-zoster virus genome in a bacterial cell,
   wherein the both ends of the BAC sequence are linked to the first and the second fragment, respectively.
69. The nucleic acid cassette of item 68, wherein the first fragment and the second fragment are at least 1 kb.
70. The nucleic acid cassette of item 68, wherein the first fragment and the second fragment are at least 1.5 kb.
71. The nucleic acid cassette of item 68, wherein the first fragment and the second fragment are at least 2 kb.
72. The nucleic acid cassette of item 68, wherein the first fragment and the second fragment are at least 80% identical with a varicella-zoster virus genome sequence.
73. The nucleic acid cassette of item 68, wherein the first fragment and the second fragment are at least 85% identical with a varicella-zoster virus genome sequence.
74. The nucleic acid cassette of item 68, wherein the first fragment and the second fragment are at least 90% identical with a varicella-zoster virus genome sequence.
75. The nucleic acid cassette of item 68, wherein the first fragment and the second fragment are at least 95% identical with a varicella-zoster virus genome sequence.
76. The nucleic acid cassette of item 68, wherein each of the first fragment and the second fragment are independently selected from the group consisting of the following regions of varicella-zoster virus genome:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
77. The nucleic acid cassette of item 68, wherein each of the first fragment andthe second fragment is independently at least 80% identical with the region selected from the group consisting of the following regions of varicella-zoster virus genome:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12,
   the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38,
   the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56,
   the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64,
   the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
78. The nucleic acid cassette of item 68, wherein each of the first fragment andthe second fragment is independently at least 85% identical with the region selected from the group consisting of the following regions of varicella-zoster virus genome:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12,
   the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38,
   the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56,
   the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64,
   the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
79. The nucleic acid cassette of item 68, wherein each of the first fragment andthe second fragment is independently at least 90% identical with the region selected from the group consisting of the following regions of varicella-zoster virus genome:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12,
   the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38,
   the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56,
   the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64,
   the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
80. The nucleic acid cassette of item 68, wherein each of the first fragment andthe second fragment is independently at least 95% identical with the region selected from the group consisting of the following regions of varicella-zoster virus genome:
   the region in the ORF of gene 7, the region in the ORF of gene 8,
   the region in the ORF of gene 9, the region in the ORF of gene 10,
   the region in the ORF of gene 11, the region in the ORF of gene 12,
   the region in the ORF of gene 13, the region in the ORF of gene 14,
   the region in the ORF of gene 15, the region in the ORF of gene 17,
   the region in the ORF of gene 18, the region in the ORF of gene 19,
   the region in the ORF of gene 38, the region in the ORF of gene 39,
   the region in the ORF of gene 46, the region in the ORF of gene 47,
   the region in the ORF of gene 48, the region in the ORF of gene 49,
   the region in the ORF of gene 50, the region in the ORF of gene 56,
   the region in the ORF of gene 57, the region in the ORF of gene 58,
   the region in the ORF of gene 59, the region in the ORF of gene 61,
   the region in the ORF of gene 62, the region in the ORF of gene 63,
   the region in the ORF of gene 64, the region in the ORF of gene 65,
   the region in the ORF of gene 66, the region in the ORF of gene 67,
   the region in the ORF of gene 68, the region in the ORF of gene 69,
   the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12,
   the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38,
   the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56,
   the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64,
   the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.
81. The nucleic acid cassette of item 68, wherein the first fragment and the second fragment are derived from different regions.
82. The nucleic acid cassette of item 72, wherein each of the first fragment and the second fragment are independently from the region flanking the ORF of gene 11 and the region flanking the ORF of gene 12.
83. The nucleic acid cassette of item 68, wherein the BAC vector sequence comprises a recombinant protein dependent recombinant sequence.
84. The nucleic acid cassette of item 68, wherein the BAC vector sequence comprises a selectable marker.
85. The nucleic acid cassette of item 84, wherein the selectable marker is drug selectable marker.
86. The nucleic acid cassette of item 68, wherein the selectable marker is a gene encoding green fluorescent protein.
87. The nucleic acid cassette of item 68, wherein the varicella-zoster virus genome is derived from a wild type strain.
88. The nucleic acid cassette of item 68, wherein the varicella-zoster virus genome is derived from a mutant type strain.
89. The nucleic acid cassette of item 68, wherein the varicella-zoster virus genome is derived from Oka vaccine strain.
90. The nucleic acid cassette of item 68, wherein the BAC vector sequence comprises the sequence set forth in SEQ ID NO.: 7.
91. The nucleic acid cassette of item 68, having a nucleic acid sequence set forth in SEQ ID NO.: 2.

The present invention provides recombinant varicella-zoster virus, and a production method thereof. For example, the present invention provides a method for producing recombinant varicella-zoster virus from a single viral strain using a BAC (*E. coli* artificial chromosome), and recombinant varicella-zoster virus produced by the method. Further, the present invention provides a pharmaceutical composition comprising recombinant varicella-zoster virus.

Further, the present invention provides a vector comprising a varicella-zoster viral genomic gene and a BAC vector sequence, and a cell containing such a vector, and a nucleic acid cassette comprising a fragment capable of homologous recombination with a varicella-zoster virus genome, and a BAC vector sequence.

### BRIEF DESCRIPTION OF THE DRAWINGS

Figure 1 is a schematic diagram showing the production of the Oka strain genome of varicella-zoster virus and recombinant varicella-zoster virus.
Figure 2 shows the comparison of proliferation in vitro between the Oka strain genome of varicella-zoster virus (parent strain) and the recombinant varicella-zoster virus (rV02).

### DESCRIPTION OF THE PREFERRED EMBODIMENTS

Hereinafter, the present invention will be described. It should be understood throughout the present specification that expression of a singular form includes the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertains. If there is contradiction, the present specification (including the definition) precedes.

Hereinafter, the present invention will be described. It should be understood throughout the present specification that expression of a singular form includes the concept of their plurality unless otherwise mentioned. Accordingly, for example, it should be understood that a singular article (for example, "a", "an", "the" in English) comprises the concepts of plural form unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertains. If there is contradiction, the present specification (including the definition) precedes.

### (Definition of Terms)

The definitions of terms used herein are described below.

As used herein, the term "essential gene" in relation to varicella-zoster virus refers to a gene which is essential for the growth of the varicella-zoster virus. Also, the term "non-essential gene" in relation to varicella-zoster virus refers to a gene which is not essential for the growth of the varicella-zoster virus, and in the absence of which the varicella-zoster virus can grow. Examples of non-essential genes of human varicella-zoster virus include, but are not limited to: gene 7, gene 8, gene 9, gene 10, gene 11, gene 12, gene 13, gene 14, gene 15, gene 17, gene 18, gene 19, gene 38, gene 39, gene 46, gene 47, gene 48, gene 49, gene 50, gene 56, gene 57, gene 58, gene 59, gene 61, gene 63, gene 64, gene 65, gene 66, gene 67, gene 68, gene 69, gene 70.

When a gene in a viral genome is an essential gene, the virus cannot grow in the absence of the gene. Therefore, by deleting an arbitrary gene in a viral genome and detecting the growth of the virus, it is possible to determine whether the gene is an essential gene or a non-essential gene.

As used herein, the term "wild strain" in relation to varicella-zoster virus refers to a varicella-zoster virus strain which is not artificially modified and is isolated from nature. An example of a wild strain includes, but is not limited to, Dumas strain identified by Davison, A.J. and Scott, J. E. (J. Gen. Virol. 67 (Pt 9), 1759-1816 (1986). The nucleic acid sequence of Dumas strain is set forth in SEQ ID NO.: 5. The number of each ORF and the site thereof in the Dumas strain are described below.

| ORF Name | Reading frame direction | Site on genome | Number acid of amino residues |
|---|---|---|---|
| ORF1 | 3' →5' direction | 589 to 915 | amino acid 1-108 |
| ORF2 | 5' →3' direction | 1134 to 1850 | amino acid 1-238 |
| ORF3 | 3' →5' direction | 1908 to 2447 | amino acid 1-179 |
| ORF4 | 3' →5' direction | 2783 to 4141 | amino acid 1-452 |
| ORF5 | 3' →5' direction | 4252 to 5274 | amino acid 1-340 |
| ORF6 | 3' →5' direction | 5326 to 8577 | amino acid 1-1083 |
| ORF7 | 5' →3' direction | 8607 to 9386 | amino acid 1-259 |
| ORF8 | 3' →5' direction | 9477 to 10667 | amino acid 1-396 |
| ORF9 | 5' →3' direction | 11009 to 11917 | amino acid 1-302 |
| ORF9A | 5' →3' direction | 10642 to 10902 | amino acid 1-87 |
| ORF10 | 5' →3' direction | 12160 to 13392 | amino acid 1-410 |
| ORF11 | 5' →3' direction | 13590 to 16049 | amino acid 1-819 |
| ORF12 | 5' →3' direction | 16214 to 18199 | amino acid 1-661 |
| ORF13 | 5' →3' direction | 18441 to 19346 | amino acid 1-301 |
| ORF14 | 3' →5' direction | 19431 to 21113 | amino acid 1-560 |
| ORF15 | 3' →5' direction | 21258 to 22478 | amino acid 1-406 |
| ORF16 | 3' →5' direction | 22568 to 23794 | amino acid 1-408 |
| ORF17 | 5' →3' direction | 24149 to 25516 | amino acid 1-455 |
| ORF18 | 3' →5' direction | 25573 to 26493 | amino acid 1-306 |
| ORF19 | 3' →5' direction | 26518 to 28845 | amino acid 1-775 |
| ORF20 | 3' →5' direction | 29024 to 30475 | amino acid 1-483 |
| ORF21 | 5' →3' direction | 30759 to 33875 | amino acid 1-1038 |
| ORF22 | 5' →3' direction | 34083 to 42374 | amino acid 1-2763 |
| ORF23 | 3' →5' direction | 42431 to 43138 | amino acid 1-235 |
| ORF24 | 3' →5' direction | 43212 to 44021 | amino acid 1-269 |
| ORF25 | 3' →5' direction | 44148 to 44618 | amino acid 1-156 |
| ORF26 | 5' →3' direction | 44506 to 46263 | amino acid 1-585 |
| ORF27 | 5' →3' direction | 46127 to 47128 | amino acid 1-333 |
| ORF28 | 3' →5' direction | 47052 to 50636 | amino acid 1-1194 |
| ORF29 | 5' →3' direction | 50857 to 54471 | amino acid 1-1204 |
| ORF30 | 5' →3' direction | 54651 to 56963 | amino acid 1-770 |
| ORF31 | 5' →3' direction | 57008 to 59614 | amino acid 1-868 |
| ORF32 | 5' →3' direction | 59766 to 60197 | amino acid 1-143 |
| ORF33 | 3' →5' direction | 60321 to 62138 | amino acid 1-605 |
| ORF33.5 | 3' →5' direction | 60321 to 61229 | amino acid 1-301 |
| ORF34 | 3' →5' direction | 62171 to 63910 | amino acid 1-579 |
| ORF35 | 3' →5' direction | 63977 to 64753 | amino acid 1-258 |
| ORF36 | 5' →3' direction | 64807 to 65832 | amino acid 1-341 |
| ORF37 | 5' →3' direction | 66074 to 68599 | amino acid 1-841 |
| ORF38 | 3' →5' direction | 68668 to 70293 | amino acid 1-541 |
| ORF39 | 5' →3' direction | 70633 to 71355 | amino acid 1-240 |
| ORF40 | 5' →3' direction | 71540 to 75730 | amino acid 1-1396 |
| ORF41 | 5' →3' direction | 75847 to 76797 | amino acid 1-316 |
| ORF42+45 | 3' →5' direction | 76851 to 78038 and 81538 to 82593 | amino acid 1-747 |
| ORF43 | 5' →3' direction | 78170 to 80200 | amino acid 1-676 |
| ORF44 | 5' →3' direction | 80360 to 81451 | amino acid 1-363 |
| ORF46 | 5' →3' direction | 82719 to 83318 | amino acid 1-199 |
| ORF47 | 5' →3' direction | 83168 to 84700 | amino acid 1-510 |
| ORF48 | 5' →3' direction | 84667 to 86322 | amino acid 1-551 |
| ORF49 | 5' →3' direction | 86226 to 86471 | amino acid 1-81 |
| ORF50 | 3' →5' direction | 86575 to 87882 | amino acid 1-435 |
| ORF51 | 5' →3' direction | 87881 to 90388 | amino acid 1-835 |
| ORF52 | 5' →3' direction | 90493 to 92808 | amino acid 1-771 |
| ORF53 | 3' →5' direction | 92855 to 93850 | amino acid 1-331 |
| ORF54 | 3' →5' direction | 93675 to 95984 | amino acid 1-769 |
| ORF55 | 5' →3' direction | 95996 to 98641 | amino acid 1-881 |
| ORF56 | 5' →3' direction | 98568 to 99302 | amino acid 1-244 |
| ORF57 | 3' →5' direction | 99411 to 99626 | amino acid 1-71 |
| ORF58 | 3' →5' direction | 99607 to 100272 | amino acid 1-221 |
| ORF59 | 3' →5' direction | 100302 to 101219 | amino acid 1-305 |
| ORF60 | 3' →5' direction | 101170 to 101649 | amino acid 1-159 |
| ORF61 | 3' →5' direction | 103082 to 104485 | amino acid 1-467 |
| ORF62 | 3' →5' direction | 105201 to 109133 | amino acid 1-1310 |
| ORF63 | 5' →3' direction | 110581 to 111417 | amino acid 1-278 |
| ORF64 | 5' →3' direction | 111565 to 112107 | amino acid 1-180 |
| ORF65 | 3' →5' direction | 112332 to 112640 | amino acid 1-102 |
| ORF66 | 5' →3' direction | 113037 to 114218 | amino acid 1-393 |
| ORF67 | 5' →3' direction | 114496 to 115560 | amino acid 1-354 |
| ORF68 | 5' →3' direction | 115808 to 117679 | amino acid 1-623 |
| ORF69 | 3' →5' direction | 117790 to 118332 | amino acid 1-180 |
| ORF70 | 3'→5' direction | 118480 to 119316 | amino acid 1-278 |
| ORF71 | 5→3' direction | 120764 to 124696 | amino acid 1-1310 |

In the above-described table, "5'→3' direction" indicates that the ORF has the same direction as that of the nucleic acid sequence of SEQ ID NO.: 5. "3'→5' direction" indicates that the ORF has a reverse direction with respect to that of the nucleic acid sequence of SEQ ID NO. : 5. By identifying a sequence homologous to the nucleic acid sequence and/or the amino acid sequence of the ORF, those skilled in the art can easily identify the ORF in the genome of a strain other than Dumas strain.

As used herein, the term "mutant strain" refers to a varicella-zoster virus strain which has a mutation due to mutagenesis, multiple subculturings or the like. Mutagenesis of a varicella-zoster virus strain may be either random mutagenesis or site-specific mutagenesis.

The terms "attenuated virus" as used herein is a type of a virus mutant strain and refer to the one that has lower virulence than wild strain. Two methods for deciding whether the virulence of a virus mutant strain is lower than that of wild strain or not (that is, the method for examining the pathogenicity of varicella-zoster virus) have been established.

As a method using an animal model, the method for evaluating the pathogenicity by producing a severe combined immunodeficient (SCID) mouse to which human skin is transplanted, and then, to infect the mouse with varicella-zoster virus is well-known (J. Virol. 1998 Feb; 72(2): 965-74,).

On the other hand, as a method for evaluating the pathogenicity *in vitro,* the method for observing CPE (cytopathic effect) of melanoma after culturing for 7-8 days where a monolayer culture human melanoma is inserted to the lower layer and a cord-blood mononuclear cell (CBMC) which is infected with varicella-zoster cell virus is inserted to the upper layer of a two-layer well separated by a trans-well of pore size 3 µm, is also well-known (J. Virol. 2000 Feb; 74(4): 1864-70).

Although it is not the method for the pathogenicity directly from the study of the present inventors (J Virol. 2002 Nov; 76(22): 11447-59), which is understood to indicate that there are close relationships between the pathogenicity and the proliferation of a virus, it is also possible to evaluate the pathogenicity indirectly by examining the proliferation of cell-to-cell employing the infectious center assay.

The method for attenuating a virus artificially is well known. For example, a varicella-zoster virus comprises at least the base substitutions of the following (a)-(d) in the gene 62 in SEQ ID NO. 5 :
(a) base substitution at position 2110 for G;
(b) base substitution at position 3100 for G;
(c) base substitution at position 3818 for C; and
(d) base substitution at position 4006 for G,
and comprises at least a base substitution at position 5745 for G, in the gene 6 in SEQ ID NO. 8
is available as an attenuated virus.

In addition to the base substitutions of (a) - (d), instead of employing the above-mentioned varicella-zoster virus, an attenuated varicella virus which comprises at least one or more base substitutions of following (e)-(g):
(e) base substitution at position 1251 for G;
(f) base substitution at position 2226 for G; and
(g) base substitution at position 3657 for G,
is available.

In addition to one or more base substitutions of (a)-(g), instead of employing the above-mentioned varicella-zoster virus, an attenuated varicella virus which comprises at least one or more base substitutions of the following (h)-(o):
(h) base substitution at position 162 for C;
(i) base substitution at position 225 for C;
(j) base substitution at position 523 for C;
(k) base substitution at position 1565 for C;
(1) base substitution at position 1763 for C;
(m) base substitution at position 2652 for C;
(n) base substitution at position 4052 for C; and
(o) base substitution at position 4193 for C,
is available.

Alternatively, as an "attenuated virus", a virus which comprises at least one or more base substitutions selected from the following group in the gene 62:
(a) base substitution at position 2110 for G;
(b) base substitution at position 3100 for G;
(c) base substitution at position 3818 for C;
(d) base substitution at position 4006 for G;
(e) base substitution at position 1251 for G;
(f) base substitution at position 2226 for G;
(g) base substitution at position 3657 for G;
(h) base substitution at position 162 for C;
(i) base substitution at position 225 for C;
(j) base substitution at position 523 for C;
(k) base substitution at position 1565 for C;
(l) base substitution at position 1763 for C;
(m) base substitution at position 2652 for C;
(n) base substitution at position 4052 for C; and
(o) base substitution at position 4193 for C,
can be employed.

The terms "protein", "polypeptide", "oligopeptide" and "peptide" as used herein have the same meaning and refer to an amino acid polymer having any length.

The terms "polynucleotide", "oligonucleotide", and "nucleic acid" as used herein have the same meaning and refer to a nucleotide polymer having any length. Unless otherwise indicated, a particular nucleic acid sequence also implicitly encompasses conservatively-modified variants thereof (e.g. degenerate codon substitutions) and complementary sequences as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al., Mol. Cell. Probes 8:91-98(1994)).

As used herein, the term "gene" refers to an element defining a genetic trait. A gene is typically arranged in a given sequence on a chromosome. Agene which defines the primary structure of a protein is called a structural gene. A gene which regulates the expression of a structural gene is called a regulatory gene. As used herein, "gene" may refer to "polynucleotide", "oligonucleotide", "nucleic acid", and "nucleic acid molecule" and/or "protein", "polypeptide", "oligopeptide" and "peptide". As used herein, the term "open reading frame" or "ORF" in relation to a gene, refers to a reading frame which is one of three frames obtained by sectioning the base sequence of a gene at intervals of three bases, and has a start codon and a certain length without a stop codon appearing partway, and has the possibility of actually coding a protein. The entire base sequence of the genome of varicella-zoster virus has been determined, identifying at least 71 genes. Each of the genes is known to have an open reading frame (ORF).

As used herein, the term "region within an ORF" in relation to a gene in a varicella-zoster virus genome, refers to a region in which there are bases constituting the ORF in the gene within the varicella-zoster virus genome.

As used herein, the term "region flanking an ORF" in relation to a gene in a varicella-zoster virus genome, refers to a region in which there are bases existing in the vicinity of the ORF in the gene within the varicella-zoster virus genome, and which does not correspond to a region within the ORF of the gene or other genes.

As used herein, the term "homology" of a gene refers to the proportion of identity between two or more gene sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other.

Similarity comparison and homology calculation of base sequences are herein performed using BLAST (sequence analyzing tool) with the default parameters.

As used herein, the term "expression" of a gene, a polynucleotide, a polypeptide, or the like, indicates that the gene or the like is affected by a predetermined action *in vivo* to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, the term "fragment" refers to a polypeptide or polynucleotide having a sequence length ranging from 1 to n-1 with respect to the full length of the reference polypeptide or polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more nucleotides. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100, 200, 300, 400, 500, 600, 600, 700, 800, 900, 1000 or more nucleotides. Lengths represented by integers which are not herein specified (e.g., 11 and the like) may be appropriate as a lower limit.

A polypeptide encoded by a gene in a BAC vector may have at least one (e.g., one or several) amino acid substitution, addition, and/or deletion or at least one sugar chain substitution, addition, and/or deletion as long as they have substantially the same function as that of a corresponding naturally-occurring polypeptide.

As used herein, the term "sugar chain" refers to a compound which is made up of a series of at least one sugar unit (a monosaccharide and/or its derivative). When two or more sugars unit is linked, the sugars unit are joined by dehydrocondensation due to glycosidic bonds. Examples of such a sugar chain include, but are not limited to, polysaccharides contained in organisms (glucose, galactose, mannose, fucose, xylose, N-acetylglucosamine, N-acetylgalactosamine, sialic acid, and complexes and derivates thereof), and degraded polysaccharides, sugar chains degraded or induced from complex biological molecules (e.g., glycoproteins, proteoglycan, glycosaminoglycan, glycolipids, etc.), and the like. Therefore, the term "sugar chain" may be herein used interchangeably with "polysaccharide", "carbohydrate", and "hydrocarbon". Unless otherwise specified, the term "sugar chain" as used herein includes both a sugar chain and a sugar chain-containing substance.

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within ±2, more preferably within ±1, and even more preferably within ±0.5. It is understood in the art that such an amino acid substitution based on hydrophobicity is efficient. A hydrophilicity index is also useful for modification of an amino acid sequence of the present invention. As described in US Patent No. 4, 554, 101, amino acid residues are given the following hydrophilicity indices: arginine (+3.0); lysine (+3.0);asparticacid (+3.0±1); glutamic acid (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within ±2, more preferably ±1, and even more preferably ±0.5.

The term "conservative substitution" as used herein refers to amino acid substitution in which a substituted amino acid and a substituting amino acid have similar hydrophilicity indices or/and hydrophobicity indices. For example, conservative substitution is carried out between amino acids having a hydrophilicity or hydrophobicity index of within ±2, preferably within ±1, and more preferably within ±0.5. Examples of conservative substitution include, but are not limited to, substitutions within each of the following residue pairs: arginine and lysine; glutamic acid and aspartic acid; serine and threonine; glutamine and asparagine; and valine, leucine, and isoleucine, which are well known to those skilled in the art.

As used herein, the term "variant" refers to a substance, such as a polypeptide, polynucleotide, or the like, which differs partially from the original substance. Examples of such a variant include a substitution variant, an addition variant, a deletion variant, a truncated variant, an allelic variant, and the like. Examples of such a variant include, but are not limited to, a nucleotide or polypeptide having one or several substitutions, additions and/or deletions or a nucleotide or polypeptide having at least one substitution, addition and/or deletion. The term "allele" as used herein refers to a genetic variant located at a locus identical to a corresponding gene, where the two genes are distinguished from each other. Therefore, the term "allelic variant" as used herein refers to a variant which has an allelic relationship with a given gene. Such an allelic variant ordinarily has a sequence the same as or highly similar to that of the corresponding allele, and ordinarily has almost the same biological activity, though it rarely has different biological activity. The term "species homolog" or "homolog" as used herein refers to one that has an amino acid or nucleotide homology with a given gene in a given species (preferably at least 60% homology, more preferably at least 80%, at least 85%, at least 90%, and at least 95% homology). A method for obtaining such a species homolog is clearly understood from the description of the present specification. The term "ortholog" (also called orthologous genes) refers to genes in different species derived from a common ancestry (due to speciation). For example, in the case of the hemoglobin gene family having multigene structure, human and mouse α-hemoglobin genes are orthologs, while the human α-hemoglobin gene and the human β-hemoglobin gene are paralogs (genes arising from gene duplication). Orthologs are useful for estimation of molecular phylogenetic trees. Usually, orthologs in different species may have a function similar to that of the original species. Therefore, orthologs of the present invention may be useful in the present invention.

As used herein, the term "conservative (or conservatively modified) variant" applies to both amino acid and nucleic acid sequences. With respect to particular nucleic acid sequences, conservatively modified variants refer to those nucleic acids which encode identical or essentially identical amino acid sequences. Because of the degeneracy of the genetic code, a large number of functionally identical nucleic acids encode any given protein. For example, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" which represent one species of conservatively modified variation. Every nucleic acid sequence herein which encodes a polypeptide also describes every possible silent variation of the nucleic acid. Those skilled in the art will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence. Preferably, such modification may be performed while avoiding substitution of cysteine which is an amino acid capable of largely affecting the higher-order structure of a polypeptide.

In order to prepare a BAC vector containing a gene encoding a functionally equivalent polypeptide, amino acid additions, deletions, or modifications can be performed in addition to amino acid substitutions. Amino acid substitution(s) refers to the replacement of at least one amino acid of an original peptide chain with different amino acids, such as the replacement of 1 to 10 amino acids, preferably 1 to 5 amino acids, and more preferably 1 to 3 amino acids with different amino acids. Amino acid addition(s) refers to the addition of at least one amino acid to an original peptide chain, such as the addition of 1 to 10 amino acids, preferably 1 to 5 amino acids, and more preferably 1 to 3 amino acids to an original peptide chain. Amino acid deletion (s) refers to the deletion of at least one amino acid, such as the deletion of 1 to 10 amino acids, preferably 1 to 5 amino acids, and more preferably 1 to 3 amino acids. Amino acid modification includes, but are not limited to, amidation, carboxylation, sulfation, halogenation, truncation, lipidation, alkylation, glycosylation, phosphorylation, hydroxylation, acylation (e.g., acetylation), and the like. Amino acids to be substituted or added may be naturally-occurring or nonnaturally-occurring amino acids, or amino acid analogs. Naturally-occurring amino acids are preferable.

As used herein, a nucleic acid form of a polypeptide refers to a nucleic acid molecule capable of expressing a protein form of the polypeptide. This nucleic acid molecule may have a nucleic acid sequence, a part of which is deleted or substituted with another base, or alternatively, into which another nucleic acid sequence is inserted, as long as an expressed polypeptide has substantially the same activity as that of a naturally occurring polypeptide. Alternatively, another nucleic acid may be linked to the 5' end and/or the 3' end of the nucleic acid molecule. The nucleic acid molecule may be a nucleic acid molecule which is hybridizable to a gene encoding a polypeptide under stringent conditions and encodes a polypeptide having substantially the same function as that polypeptide. Such a gene is known in the art and is available in the present invention.

Such a nucleic acid can be obtained by a well known PCR technique, or alternatively, can be chemically synthesized. These methods may be combined with, for example, site-specific mutagenesis, hybridization, or the like.

As used herein, the term "substitution, addition or deletion" for a polypeptide or a polynucleotide refers to the substitution, addition or deletion of an amino acid or its substitute, or a nucleotide or its substitute, with respect to the original polypeptide or polynucleotide, respectively. This is achieved by techniques well-known in the art, including a site-specific mutagenesis technique, and the like. A polypeptide or a polynucleotide may have any number (>0) of substitutions, additions, or deletions. The number can be as large as a variant having such a number of substitutions, additions or deletions which maintains an intended function. For example, such a number may be one or several, and preferably within 20% or 10% of the full length, or no more than 100, no more than 50, no more than 25, or the like.

The structure of polymers (e.g., polypeptide structure) may be described at various levels. This structure is generally described in, for example, Alberts et al., Molecular Biology of the Cell (3rd Ed., 1994), and Cantor and Schimmel, Biophysical Chemistry Part I: The Conformation of Biological Macromolecules (1980). General molecular biological techniques available in the present invention can be easily carried out by the those skilled in the art by referencing Ausubel F. A. et al. eds. (1988), Current Protocols in Molecular Biology, Wiley, New York, NY; Sambrook J. et al., (1987) Molecular Cloning: A Laboratory Manual, 2nd Ed., Cold Spring Harbor Laboratory Press, Cold Spring Harbor, NY, or the like.

When mentioning genes in the present specification, "vector" refers to an agent which can transfer a polynucleotide sequence of interest to a target cell. Examples of such a vector include vectors which are capable of self replication or capable of being incorporated into a chromosome within host cells (e.g., prokaryotic cells, yeast, animal cells, plant cells, insect cells, whole animals, and whole plants), and contain a promoter at a site suitable for transcription of a polynucleotide of the present invention.

The term "BAC vector" refers to a plasmid which is produced using F plasmid of *E. coli* and a vector which can stably maintain and grow a large size DNA fragment of about 300 kb or more in bacteria, such as *E. coli* and the like. The BAC vector contains at least a region essential for the replication of the BAC vector. Examples of such a region essential for replication include, but are not limited to, the replication origin of F plasmid (oriS) and variants thereof.

As used herein, the term "BAC vector sequence" refers to a sequence comprising a sequence essential for the function of a BAC vector. Optionally, the BAC vector sequence may further comprise a "recombinant protein-dependent recombinant sequence" and/or a "selectable marker".

As used herein, the term "recombinant" in relation to nucleic acid is used interchangeably with the term "homologous recombination", and indicates that two different homologous nucleic acid molecules encounter each other, crossover occurs, and a new combination of nucleic acid is generated. As used herein, homologous recombination includes both "recombinant protein-dependent recombination" and "recombinant protein-independent recombination". The term "recombinant protein-dependent recombination" refers to homologous recombination which occurs in the presence of a recombinant protein, but not in the absence of a recombinant protein. The term "recombinant protein-independent recombination" refers to homologous recombination which occurs irrespective of the presence or absence of a recombinant protein. As used herein, the term "recombinant protein-dependent recombinant sequence" refers to a sequence which causes recombinant protein-dependent recombination. The term "recombinant protein-independent recombinant sequence" refers to a sequence which causes recombinant protein-independent recombination. The recombinant protein-dependent recombinant sequence causes recombination in the presence of a recombinant protein, but not in the absence of a recombinant protein. A recombinant protein preferably acts specifically on a recombinant protein-dependent recombinant sequence, and does not act on sequences other than the recombinant protein-dependent recombinant sequence.

Examples of representative pairs of a recombinant protein-dependent recombinant sequence and a recombinant protein include, but are not limited to: a combination of a bacteriophage P1-derived loxP (locus of crossover of P1) sequence and a Cre (cyclization recombination) protein, a combination of Flp protein and FRT site, a combination of ϕC31 and attB or attP (Thorpe, Helena M.; Wilson, Stuart E.; Smith, Margaret C.M., Control of directionality in the site-specific recombination system of the Streptomyces phage ϕC31., Molecular Microbiology (2000), 38 (2), 232-241.), a combination of resolvase and res site(Sadowski P., Site-specific recombinases: changing partners and doing the twist, J. Bacteriol., February 1986; 165(2) 341-7) (generally, Sauer B., Site-specific recombination: developments and applications., Curr. Opin. Biotechnol., 1994 Oct; 5 (5) : 521-7).

As used herein, the term "selectable marker" refers to a gene which functions as an index for selection of a host cell containing a BAC vector. Examples of a selectable marker include, but are not limited to, fluorescent markers, luminiscent markers, and drug selectable markers. An example of a "fluorescent marker" is, but is not limited to, a gene encoding a fluorescent protein, such as a green fluorescent protein (GFP). An example of a "luminiscent marker" is, but is not limited to, a gene encoding a luminescent protein, such as luciferase. An example of a "drug selectable marker" is, but is not limited to, a gene encoding a protein selected from the group consisting of: dihydrofolate reductase gene, glutamine synthase gene, aspartic acid transaminase, metallothionein (MT), adenosine deaminase (ADA), adenosine deaminase (AMPD1, 2), xanthine-guanine-phosphoribosyl transferase, UMP synthase, P-glycoprotein, asparagine synthase, and ornithine decarboxylase. Examples of a combination of a drug selectable marker and a drug include : a combination of dihydrofolate reductase gene (DHFR) and methotrexate (MTX), a combination of glutamine synthase (GS) gene and methionine sulfoximine (Msx), a combination of aspartic acid transaminase (CAD) gene and N-phosphonacetyl-L-aspartate) (PALA), a combination of MT gene and cadmium (Cd2⁺), a combination of adenosine deaminase (ADA) gene and adenosine, alanosine, or 2'-deoxycoformycin, a combination of adenosine deaminase (AMPD1, 2) gene and adenine, azaserine, or coformycin, a combination of xanthine-guanine-phosphoribosyltransferase gene and mycophenolic acid, a combination of UMP synthase gene and 6-azaulysine or pyrazofuran, a combination of P-glycoprotein (P-gp, MDR) gene and multiple drugs, a combination of asparagine synthase (AS) gene and β-aspartyl hydroxamic acid or albizziinn, and a combination of ornithine decarboxylase (ODC) gene and α-difluoromethyl-ornithine (DFMO).

As used herein, the term "expression vector" refers to a nucleic acid sequence comprising a structural gene and a promoter for regulating expression thereof, and in addition, various regulatory elements in a state that allows them to operate within host cells. The regulatory element may include, preferably, terminators, selectable markers such as drug-resistance genes (e.g., a kanamycin resistance gene, a hygromycin resistance gene, etc.), and enhancers. It is well known to those skilled in the art that the type of an organism (e.g., a plant) expression vector and the type of a regulatory element may vary depending on the host cell. In the case of plants, a plant expression vector for use in the present invention may further have a T-DNA region. A T-DNA region enhances the efficiency of gene transfer, especially when a plant is transformed using Agrobacterium.

As used herein, the term "recombinant vector" refers to a vector which can transfer a polynucleotide sequence of interest to a target cell. Examples of such a vector include vectors which are capable of self replication or capable of being incorporated into a chromosome within host cells (e.g., prokaryotic cells, yeast, animal cells, plant cells, insect cells, whole animals, and whole plants), and contain a promoter at a site suitable for transcription of a polynucleotide of the present invention.

As used herein, the term "terminator" refers to a sequence which is located downstream of a protein-encoding region of a gene and which is involved in the termination of transcription when DNA is transcribed into mRNA, and the addition of a poly A sequence. It is known that a terminator contributes to the stability of mRNA, and has an influence on the amount of gene expression. Examples of a terminator include, but are not limited to, terminators derived from mammals, the CaMV35S terminator, the terminator of the nopaline synthase gene (Tnos), the terminator of the tobacco PR1a gene, and the like.

As used herein, the term "promoter" refers to a base sequence which determines the initiation site of transcription of a gene and is the region in the ORF of DNA which directly regulates the frequency of transcription. Transcription is started by RNA polymerase binding to a promoter. A promoter region is usually located within about 2 kbp upstream of the first exon of a putative protein coding region. Therefore, it is possible to estimate a promoter region by predicting a protein coding region in a genomic base sequence using DNA analysis software. A putative promoter region is usually located upstream of a structural gene, but depending on the structural gene, i.e., a putative promoter region may be located downstream of a structural gene. Preferably, a putative promoter region is located within about 2 kbp upstream of the translation initiation site of the first exon.

As used herein, the term "constitutive" for expression of a promoter of the present invention refers to a character of the promoter that the promoter is expressed in a substantially constant amount in all tissues of an organism no matter whether the growth stage of the organism is a juvenile phase or a mature phase. Specifically, when Northern blotting analysis is performed under the same conditions as those described in examples of the present specification, expression is considered to be constitutive according to the definition of the present invention if substantially the same amount of expression is observed at the same or corresponding site at any time (e.g., two or more time points (e.g., day 5 and day 15)), for example. Constitutive promoters are considered to play a role in maintaining the homeostasis of organisms in a normal growth environment. These characters can be determined by extracting RNA from any portion of an organism and analyzing the expression amount of the RNA by Northern blotting or quantitating expressed proteins by Western blotting.

An "enhancer" may be used so as to enhance the expression efficiency of a gene of interest. When used in animals, an enhancer region containing an upstream sequence within the SV40 promoter is preferable. One or more enhancers may be used, or no enhancer may be used.

As used herein, the term "operatively linked" indicates that a desired sequence is located such that expression (operation) thereof is under control of a transcription and translation regulatory sequence (e.g., a promoter, an enhancer, and the like) oratranslationregulatory sequence. In order for a promoter to be operatively linked to a gene, typically, the promoter is located immediately upstream of the gene. A promoter is not necessarily adjacent to a structural gene.

As used herein, the terms "transformation", "transduction" and "transfection" are used interchangeably unless otherwise mentioned, and refers to introduction of a nucleic acid into host cells. As a transformation method, any technique for introducing DNA into host cells can be used, including various well-known techniques, such as, for example, the electroporation method, the particle gun method (gene gun), the calcium phosphate method, and the like.

As used herein, the term "transformant" refers to the whole or a part of an organism, such as a cell, which is produced by transformation. Examples of a transformant include prokaryotic cells, yeast, animal cells, plant cells, insect cells and the like. Transformants may be referred to as transformed cells, transformed tissue, transformed hosts, or the like, depending on the subject. As used herein, all of the forms are encompassed, however, a particular form may be specified in a particular context.

Examples of prokaryotic cells include prokaryotic cells of the genera *Escherichia, Serratia, Bacillus, Brevibacterium, Corynebacterium, Microbacterium, Pseudomonas,* and the like, e.g., *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Escherichia coli* BL21(DE3), *Escherichia coli* BL21(DE3)pLysS, *Escherichia coli* HMS174(DE3), *Escherichia coli* HMS174 (DE3) pLysS, *Serratia ficaria, Serratia fonticola, Serratia liquefaciens, Serratia* marcescens, *Bacillus subtilis, Bacillus amyloliquefaciens, Brevibacterium* ammmoniagenes, *Brevibacterium* immariophilum ATCC14068, *Brevibacterium saccharolyticum* ATCC14066, *Corynebacterium glutamicum* ATCC13032, *Corynebacterium glutamicum* ATCC14067, *Corynebacterium glutamicum* ATCC13869, *Corynebacterium* acetoacidophilum ATCC13870, *Microbacterium ammoniaphilum* ATCC15354, *Pseudomonas* sp.D-0110, and the like.

Examples of animal cells include human MRC-5 cells, human HEL cells, human WI-38 cells, mouse myeloma cells, rat myeloma cells, human myeloma cells, mouse hybridoma cells, CHO cells derived from chinese hamster, BHK cells, African green monkey kidney cells, human leukemia cells, HBT5637 (Japanese Laid-Open Publication No. 63-299), human colon cancer cell strains. Mouse myeloma cells include ps20, NSO, and the like. Rat myeloma cells include YB2/0, and the like. Human fetus kidney cells includes HEK293 (ATCC: CRL-1573), and the like. Human leukemia cells include BALL-1, and the like. African green monkey kidney cells include COS-1, COS-7, vero cell and the like. Human colon cancer cell strains include HCT-15, and the like.

The term "animal" is used herein in its broadest sense and refers to vertebrates and invertebrates (e.g., arthropods). Examples of animals include, but are not limited to, any of the class *Mammalia,* the class Aves, the class Reptilia, the class *Amphibia,* the class *Pisces,* the class *Insecta,* the class Vermes, and the like.

As used herein, the term "tissue" in relation to organisms refers to an aggregate of cells having substantially the same function. Therefore, a tissue may be a part of an organ. Organs usually have cells having the same function, and may have coexisting cells having slightly different functions. Therefore, as used herein, tissues may have various kinds of cells as long as a certain property is shared by the cells.

As used herein, the term "organ" refers to a structure which has a single independent form and in which one or more tissues are associated together to perform a specific function. In plants, examples of organs include, but are not limited to, callus, root, stem, trunk, leaf, flower, seed, embryo bud, embryo, fruit, and the like. In animals, examples of organs include, but are not limited to, stomach, liver, intestine, pancreas, lung, airway, nose, heart, artery, vein, lymph node (lymphatic system), thymus, ovary, eye, ear, tongue, skin, and the like.

As used herein, the term "transgenic" refers to incorporation of a specific gene into an organism (e.g., plants or animals (mice, etc.)) or such an organism having an incorporated gene.

When organisms of the present invention are animals, the transgenic organisms can be produced by a microinjection method (a trace amount injection method), a viral vector method, an embryonic stem (ES) cell method, a sperm vector method, a chromosome fragment introducing method (transsomic method), an episome method, or the like. These transgenic animal producing techniques are well known in the art.

As used herein, the term "screening" refers to selection of a substance, a host cell, a virus, or the like having a given specific property of interest from a number of candidates using a specific operation/evaluation method. It will be understood that the present invention encompasses viruses having desired activity obtained by screening.

As used herein, the terms "chip" or "microchip" are used interchangeably to refer to a micro integrated circuit which has versatile functions and constitutes a portion of a system. Examples of a chip include, but are not limited to, DNA chips, protein chips, and the like.

As used herein, the term "array" refers to a substrate (e.g., a chip, etc.) which has a pattern of a composition containing at least one (e.g., 1000 or more, etc.) target substances (e.g., DNA, proteins, cells, etc.), which are arrayed. Among arrays, patterned substrates having a small size (e.g., 10×10 mm, etc.) are particularly referred to as microarrays. The terms "microarray" and "array" are used interchangeably. Therefore, a patterned substrate having a larger size than that which is described above may be referred to as a microarray. For example, an array comprises a set of desired transfection mixtures fixed to a solid phase surface or a film thereof. An array preferably comprises at least 10² antibodies of the same or different types, more preferably at least 10³, even more preferably at least 10⁴, and still even more preferably at least 10⁵. These antibodies are placed on a surface of up to 125×80 mm, more preferably 10×10 mm. An array includes, but is not limited to, a 96-well microtiter plate, a 384-well microtiter plate, a microtiter plate the size of a glass slide, and the like. A composition to be fixed may contain one or a plurality of types of target substances. Such a number of target substance types may be in the range of from one to the number of spots, including, without limitation, about 10, about 100, about 500, and about 1,000.

As described above, any number of target substances (e.g., biomolecular, such as cells) may be provided on a solid phase surface or film, typically including no more than 10⁸ biological molecules per substrate, in another embodiment no more than 10⁷ biological molecules, no more than 10⁶ biological molecules, no more than 10⁵ biological molecules, no more than 10⁴ biological molecules, no more than 10³ biological molecules, or no more than 10² biological molecules. A composition containing more than 10⁸ biological molecule target substances may be provided on a substrate. In these cases, the size of a substrate is preferably small. Particularly, the size of a spot of a composition containing target substances (e.g., such as cells) may be as small as the size of a single biological molecule (e.g., 1 to 2 nm order). In some cases, the minimum area of a substrate may be determined based on the number of biological molecules on a substrate.

"Spots" of biological molecules may be provided on an array. As used herein, the term "spot" refers to a certain set of compositions containing target substances. As used herein, the term "spotting" refers to an act of preparing a spot of a composition containing a certain target substance on a substrate or plate. Spotting may be performed by any method, for example, pipetting or the like, or alternatively, using an automatic device. These methods are well known in the art.

As used herein, the term "address" refers to a unique position on a substrate, which may be distinguished from other unique positions. Addresses are appropriately associated with spots. Addresses can have any distinguishable shape such that substances at each address may be distinguished from substances at other addresses (e.g., optically) . A shape defining an address may be, for example, without limitation, a circle, an ellipse, a square, a rectangle, or an irregular shape. Therefore, the term "address" is used to indicate an abstract concept, while the term "spot" is used to indicate a specific concept. Unless it is necessary to distinguish them from each other, the terms "address" and "spot" may be herein used interchangeably.

The size of each address particularly depends on the size of the substrate, the number of addresses on the substrate, the amount of a composition containing target substances and/or available reagents, the size of microparticles, and the level of resolution required for any method used for the array. The size of each address may be, for example, in the range of from 1-2 nm to several centimeters, though the address may have any size suited to an array.

The spatial arrangement and shape which define an address are designed so that the microarray is suited to a particular application. Addresses may be densely arranged or sparsely distributed, or subgrouped into a desired pattern appropriate for a particular type of material to be analyzed.

As used herein, the term "support" refers to a material which can carry cells, bacteria, viruses, polynucleotides, or polypeptides. Such a support may be made from any solid material which has a capability of binding to a biological molecule as used herein via covalent or noncovalent bond, or which may be induced to have such a capability.

Examples of materials used for supports include any material capable of forming a solid surface, such as, without limitation, glass, silica, silicon, ceramics, silicon dioxide, plastics, metals (including alloys), naturally-occurring and synthetic polymers (e.g., polystyrene, cellulose, chitosan, dextran, and nylon), and the like. Preferably, a support comprises a portion for producing hydrophobic bonds. A support may be formed of layers made of a plurality of materials. For example, a support may be made of an inorganic insulating material, such as glass, quartz glass, alumina, sapphire, forsterite, silicon carbide, silicon oxide, silicon nitride, or the like. A support may be made of an organic material, such as polyethylene, ethylene, polypropylene, polyisobutylene, polyethylene terephthalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene-acrylonitrile copolymer, acrylonitrile-butadiene-styrene copolymer, silicone resin, polyphenylene oxide, polysulfone, and the like. Alternatively, nitrocellulose film, nylon film, PVDF film, or the like, which are used in blotting, may be used as a material for a support.

The varicella-zoster virus of the present invention can be used as an ingredient of a pharmaceutical composition for the treatment, prevention, and/or therapy of infectious diseases.

As used herein, the term "effective amount" in relation to a drug refers to an amount which causes the drug to exhibit intended efficacy. As used herein, an effective amount corresponding to a smallest concentrationmaybe referred to as a minimumeffective amount. Such a minimum effective amount is well known in the art. Typically, the minimum effective amount of a drug has been determined or can be determined as appropriate by those skilled in the art. The determination of such an effective amount can be achieved by actual administration, use of an animal model, or the like. The present invention is also useful for the determination of such an effective amount.

As used herein, the term "pharmaceutically acceptable carrier" refers to a material which is used for production of a pharmaceutical agent or an agricultural chemical (e.g., an animal drug), and has no adverse effect on effective ingredients. Examples of such a pharmaceutically acceptable carrier include, but are not limited to: antioxidants, preservatives, colorants, flavoring agents, diluents, emulsifiers, suspending agents, solvents, fillers, bulking agents, buffers, delivery vehicles, excipients, and/or agricultural or pharmaceutical adjuvants.

The type and amount of a pharmaceutical agent used in the treatment method of the present invention can be easily determined by those skilled in the art based on information obtained by the method of the present invention (e.g., information relating to a disease) in view of the purpose of use, the target disease (type, severity, etc.), the subject' s age, size, sex, and case history, the morphology and type of a site of a subject of administration, or the like. The frequency of subjecting a subject (patient) to the monitoring method of the present invention is also easily determined by those skilled in the art with respect to the purpose of use, the target disease (type, severity, etc.), the subject' s age, size, sex, and case history, the progression of the therapy, and the like. Examples of the frequency of monitoring the state of a disease include once per day to once per several months (e.g., once per week to once per month). Preferably, monitoring is performed once per week to once per month with reference to the progression.

As usedherein, the term "instructions" refers to a description of the method of the present invention for a person who performs administration, such as a medical doctor, a patient, or the like. Instructions state when to administer a medicament of the present invention, such as immediately after or before radiation therapy (e. g. , within 24 hours, etc.). The instructions are prepared in accordance with a format defined by an authority of a country in which the present invention is practiced (e.g., Health, Labor and Welfare Ministry in Japan, Food and Drug Administration (FDA) in the U.S., and the like), explicitly describing that the instructions are approved by the authority. The instructions are so-called package insert and are typically provided in paper media. The instructions are not so limited and may be provided in the form of electronic media (e.g., web sites, electronic mails, and the like provided on the Internet).

In a therapy of the present invention, two or more pharmaceutical agents may be used as required. When two or more pharmaceutical agents are used, these agents may have similar properties or may be derived from similar origins, or alternatively, may have different properties or may be derived from different origins. A method of the present invention can be used to obtain information about the drug resistance level of a method of administering two or more pharmaceutical agents.

In the present invention, it will be appreciated by those skilled in the art that once the analysis result of a certain sugar chain structure has been correlated with a level of a disease concerning a similar type of organism, culture cell, tissue, animal (e.g., a mouse for a human) or the like, a corresponding sugar chain structure can be correlated with the disease level. Such matters are described and supported in, for example, "Doubutsu Baiyosaibo Manual (Animal Culture Cell Manual), Seno et al. eds., Kyoritsu shuppan, 1993, the entirety of which is hereby incorporated by reference.

### (General techniques used herein)

Techniques used herein are within the technical scope of the present invention unless otherwise specified. These techniques are commonly used in the fields of sugar chain science, fluidics, micromachining, organic chemistry, biochemistry, genetic engineering, molecular biology, microbiology, genetics, and their relevant fields. The techniques are well described in documents described below and the documents mentioned herein elsewhere.

Micromachining is described in, for example, Campbell, S.A. (1996), The Science and Engineering of Microelectronic Fabrication, Oxford University Press; Zaut, P.V. (1996), Micromicroarray Fabrication: a Practical Guide to Semiconductor Processing, Semiconductor Services; Madou, M.J. (1997), Fundamentals of Microfabrication, CRC1 5 Press; Rai-Choudhury, P. (1997), Handbook of Microlithography, Micromachining & Microfabrication: Microlithography; and the like, the relevant portions of which are hereby incorporated by reference.

Molecular biology techniques, biochemistry techniques, and microbiology techniques used herein are well known and commonly used in the art, and are described in, for example, Maniatis, T. et al. (1989), Molecular Cloning: A Laboratory Manual, Cold Spring Harbor and its 3rd Ed. (2001); Ausubel, F.M. et al. eds, Current Protocols in Molecular Biology, John Wiley & Sons Inc., NY, 10158 (2000) ; Innis, M.A. (1990), PCR Protocols: A Guide to Methods and Applications, Academic Press; Innis, M.A. et al. (1995), PCR Strategies, Academic Press; Sninsky, J.J. et al. (1999), PCR Applications: Protocols for Functional Genomics, Academic Press; Gait, M.J. (1985), Oligonucleotide Synthesis: A Practical Approach, IRL Press; Gait, M.J. (1990), Oligonucleotide Synthesis: A Practical Approach, IRL Press; Eckstein, F. (1991), Oligonucleotides and Analogues: A Practical Approach, IRL Press; Adams, R.L. et al. (1992), The Biochemistry of the Nucleic Acids, Chapman & Hall; Shabarova, Z. et al. (1994), Advanced Organic Chemistry of Nucleic Acids, Weinheim; Blackburn, G.M. et al. (1996), Nucleic Acids in Chemistry and Biology, Oxford University Press; Hermanson, G.T. (1996), Bioconjugate Techniques, Academic Press; Method in Enzymology 230, 242, 247, Academic Press, 1994; Special issue, Jikken Igaku (Experimental Medicine) "Idenshi Donyu & Hatsugenkaiseki Jikkenho (Experimental Method for Gene introduction & Expression Analysis)", Yodo-sha, 1997; and the like. Relevant portions (or possibly the entirety) of each of these publications are herein incorporated by reference.

### (Description of preferred embodiments)

Hereinafter, the present invention will be described by way of embodiments. Embodiments described below are provided only for illustrative purposes. Accordingly, the scope of the present invention is not limited by the embodiments except as by the appended claims. It will be clearly appreciated by those skilled in the art that variations and modifications can be made without departing from the scope of the present invention with reference to the specification.

According to an aspect of the present invention, recombinant varicella-zoster virusisprovided. Preferably,the varicella-zoster virus contains a BAC vector sequence in its genome sequence. By constructing a varicella-zoster virus genome containing a BAC vector sequence, it becomes possible to handle the varicella-zoster virus genome as the BAC molecule in bacteria. A BAC vector sequence used herein preferably contains an origin of replication derived from F plasmid, or alternatively may contain any origin of replication other than an origin of replication derived from F plasmid, as long as it has a sequence of 300 kb or more and can be held and grown as a bacterial artificial sequence in bacterial cells. The BAC vector of the present invention can be maintained and/or grow in bacterial host cells, preferably *E. coli* cells. Preferably, a portion of the BAC vector is inserted into a non-essential region of a varicella-zoster virus genome, so that it is possible to manipulate it as a BAC containing the varicella-zoster virus genome. When the BAC containing the varicella-zoster virus genome is introduced into a mammalian cell, the recombinant varicella-zoster virus can be produced and grown. As a host cell for the recombinant varicella-zoster virus, any mammalian cell which can grow a wild-type varicella-zoster virus strain can be used. Preferably, such a host cell is derived from a human, including, for example, but being not limited to, human MRC-5 cell, human HEL cell, and human WI-38.

### (Method for producing a BAC vector containing a human varicella-zoster virus genome)

Various techniques can be used to produce a BAC vector containing a human varicella-zoster virus by using a human varicella-zoster virus genome and a BAC vector.

An example of the technique using homologous recombination is a technique using a nucleic acid having a linear BAC vector sequence linked with a sequence homologous to a human varicella-zoster virus genome.

A method for producing a BAC vector comprising a human varicella-zoster virus genome by using a nucleic acid having a linear BAC vector sequence linked with a sequence homologous to a human varicella-zoster virus genome representatively comprises the steps of: (1) introducing the nucleic acid along with the human varicella-zoster virus genome into appropriate hosts (for example, into human established cell); (2) culturing the host cells to elicit homologous recombination between the homologous sequence linked with the linear BAC vector sequence and the human varicella-zoster virus genome sequence; (3) screening the host cells for one which contains the human varicella-zoster virus genome sequence having the BAC vector sequence incorporated due to the homologous recombination; (4) culturing the host cell and extracting a circular virus DNA.

Alternatively, in order to produce a BAC containing a human varicella-zoster virus genome using a human varicella-zoster virus genome and a BAC sequence, various methods, such as use of nucleic acid fragments obtained using restriction enzymes or the like, can be employed instead of homologous recombination.

A non-essential region of the varicella-zoster virus genome for introducing a BAC vector sequence thereinto selected from the group consisting of: the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

Preferably non-essential regions are regions flanking gene 11 or regions flanking gene 12. This is because gene 11 and gene 12 are contiguous non-essential genes on the varicella-zoster virus genome, so that it is easy to design a nucleic acid for homologous recombination. Alternatively, a part of a BAC vector sequence may be inserted to the region in the ORF of gene 62 of varicella-zoster virus genome.

A BAC vector sequence used in the present invention preferably includes a recombinant protein-dependent recombinant sequence and/or a selectable marker. Preferably, the selectable marker sequence is a drug selectable marker and/or a gene encoding a green fluorescent protein. This is because the presence of a desired gene can be easily confirmed.

Varicella-zoster virus employed as a starting material in the present invention may be from wild strain or mutated strain. Preferably, an attenuated virus, for example, varicella-zoster virus having mutation in Oka vaccine strain or the gene 62 is used as varicella-zoster virus as a starting material. As an "attenuated varicella-zoster virus", a virus which comprises at least one of mutation of the gene 62, or more than one combination of mutation selected from the following group:
(a) base substitution at position 2110 for G;
(b) base substitution at position 3100 for G;
(c) base substitution at position 3818 for C;
(d) base substitution at position 4006 for G;
(e) base substitution at position 1251 for G;
(f) base substitution at position 2226 for G;
(g) base substitution at position 3657 for G;
(h) base substitution at position 162 for C;
(i) base substitution at position 225 for C;
(j) base substitution at position 523 for C;
(k) base substitution at position 1565 for C;
(l) base substitution at position 1763 for C;
(m) base substitution at position 2652 for C;
(n) base substitution at position 4052 for C; and
(o) base substitution at position 4193 for C,
is included.

According to another aspect of the present invention, a vector used for production of the above-described virus and a method for producing the above-described virus are provided. According to still another aspect of the present invention, a pharmaceutical composition comprising the above-described virus and a pharmaceutical composition in the form of a vaccine are provided.

The recombinant human varicella-zoster virus of the present invention can be used as a vaccine, since it has many proteins which have the same structure as that of wild virus.

According to still another aspect of the present invention, a method for introducing mutation into a vector for producing a vaccine of the present invention is provided. The method comprises the steps of: introducing a vector into a bacterial host cell; introducing a plasmid vector containing a fragment consisting of a portion of a human varicella-zoster virus genome into the bacterial host cell, wherein the fragment has at least one mutation; culturing the bacterial host cell; and isolating a vector having a BAC vector sequence from the cultured bacterial host cell. In the above-described method, homologous recombination occurs between the vector for producing a vaccine of the present invention and the plasmid vector containing the fragment consisting of the portion of the human varicella-zoster virus genome, in bacterial host cells. As a result, the vector for producing the vaccine of the present invention has a mutation in the fragment consisting of the portion of the human varicella-zoster virus genome.

In the above-described method, the step of introducing the vector into bacterial host cells can be achieved by using various well-known methods, such as electroporation and the like. Similarly, the plasmid vector containing the fragment consisting of the portion of the human varicella-zoster virus genome can be introduced into bacterial host cells. As a technique for introducing a mutation into the fragment, a technique for introducing a mutation by using PCR is well known. For example, by using heat-resistant polymerase having no proofreading function, where one of the four nucleotides is in lower quantity, it is possible to introduce a mutation randomly. Alternatively, by PCR using a primer having a mutated base sequence, it is possible to introduce a desired mutation into a desired site. When the bacterial cell is cultured, homologous recombination occurs between the vector for producing the vaccine of the present invention and the plasmid vector containing the fragment consisting of the portion of the human varicella-zoster virus genome. As a result, the vector for producing the vaccine of the present invention has a mutation in the fragment consisting of the portion of the human varicella-zoster virus genome. In order to prepare a BAC vector sequence from a bacterial host cell, various well-known techniques (e.g., the alkaline method, etc.) and commercially available kits can be used.

According to another aspect of the present invention, another method for introducing a mutation into a vector for producing the vaccine of the present invention is provided. The method comprises the steps of: introducing the vector into a bacterial host cell; introducing a first plasmid vector containing a first fragment consisting of a portion of a human varicella-zoster virus genome into the bacterial host cell, wherein the first fragment has at least one mutation; introducing a second plasmid vector containing a second fragment consisting of a portion of the human varicella-zoster virus genome into the bacterial host cell, wherein the second fragment has at least one mutation and the second fragment is different from the first fragment; culturing the bacterial host cell; and isolating a vector having a BAC vector sequence from the cultured bacterial host cell.

According to an aspect of the present invention, a nucleic acid cassette which may be used for producing the vaccine of the present invention, is provided. The nucleic acid cassette preferably comprises a first fragment capable of homologous recombination with a human varicella-zoster virus genome in a host cell, a BAC vector sequence, and a second fragment capable of homologous recombination with a human varicella-zoster virus genome in the host cell, wherein the opposite ends of the BAC sequence are linked with the first fragment and second fragments, respectively. In this case, the first fragment and the second fragment are preferably at least 1 kb, at least 1. 5 kb, or at least 2 kb in length. The first fragment and the second fragment preferably are at least 80% identity, at least 85% identity, at least 90% identity, or at least 95% identity to the human varicella-zoster virus genome sequence.

Preferably, the first and second fragments are independently derived from regions selected from the group consisting of the following regions of the varicella-zoster virus genome, or independently have at least 80%, 85%, 90%, or 95% identity to regions selected from the group consisting of the following regions of the varicella-zoster virus genome: the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, and the region flanking the ORF of gene 70.

Preferably, the first and second fragments are derived from different regions of the human varicella-zoster virus genome. The first and second fragments may be independently derived from a region flanking the ORF of gene 11 or 12. Preferably, the BAC vector sequence comprises a recombinant protein-dependent recombinant sequence and/or a selectable marker in order to control homologous recombination and easily detect a desired gene. The selectable marker may be either a drug selectable marker or a gene encoding a fluorescent protein (e.g., a green fluorescent protein, etc.). Representatively, the BAC vector sequence has a nucleic acid sequence set forth in SEQ ID NO. : 2, and the nucleic acid cassette has a nucleic acid sequence set forth in SEQ ID No.: 2.

### (Preparation of recombinant varicella-zoster virus containing a mutated gene)

A method of the present invention can be used to easily prepare a varicella-zoster virus having a varicella-zoster virus genome into which a mutated gene is introduced.

Such mutation introduction can be performed by using a method described below.

Into *E. coli*, (a) VZV-BAC-DNAplasmidand (b) a shuttle vector or a PCR product having a partial sequence of a varicella-zoster virus genome with any mutation as a mutated nucleic acid, are introduced. Homologous recombination is allowed to occur between VZV-BAC-DNA plasmid and the shuttle vector or PCR product, so that a foreign gene mutation can be introduced into VZV-BAC-DNA plasmid. Alternatively, a transposon can be used to randomly introduce a mutation. The VZV-BAC-DNA plasmid into which the mutation has been introduced, can be easily selected and grown in *E. coli.* By causing VZV-BAC-DNA having the mutation to produce a virus, the recombinant varicella-zoster virus can be obtained (Markus Wagner, TRENDS in Microbilogy, Vol. 10, No. 7, July 2002). Specific examples will be described below.
(1) Use of a temperature sensitive shuttle vector containing a mutated varicella-zoster virus as a mutated nucleic acid:
   Firstly, the shuttle vector and VZV-BAC-DNAplasmid are allowed to recombine via a first homologous region to generate a cointegrate in which the shuttle vector is linked with VZV-BAC-DNA plasmid. Next, since the replication origin of the shuttle vector is temperature-sensitive, the shuttle plasmid is removed. In a second recombination event, the cointegrated portion is removed. When the second recombination event occurs via the first homologous region, a plasmid having the same sequence as that of VZV-BAC-DNA used for the recombination is generated. In contrast, when the second recombination event occurs via a second homologous region different from the first homologous region, a modified VZV-BAC-DNAplasmid having the foreign gene contained in the shuttle vector is obtained. When the first homologous region and the second homologous region have substantially the same length, the probability that the second recombination event occurs in the second homologous region is substantially the same as the probability that the second recombination event occurs in the first homologous region. Therefore, about half of the resultant VZV-BAC-DNA plasmids are plasmids having the same sequence as that which has been used in the recombination, while about half thereof are plasmids having the foreign gene which has been introduced into the shuttle vector.
(2) Use of a linear DNA fragment:
   In this method, for example, by utilizing the recombination function of recET derived from prophage Rac or the recombination function of redαβ derived from bacteriophage λ, a linear DNA fragment is used to introduce a mutation into a circular VZV-BAC-DNA molecule. Specifically, a selectable marker flanking a target sequence and a linear DNA fragment containing a homologous sequence are introduced along with VZV-BAC-DNA into *E. coli* capable of homologous recombination. In order to avoid the degradation of the linear DNA within *E. coli,* it is preferable to use *E. coli* lacking exonuclease or cause expression of redγ (gam) which is an exonuclease inhibitor derived from a bacteriophage. The linear DNA has a region homologous to VZV-BAC-DNA plasmid on the opposite ends thereof. Homologous recombination occurs via the homologous region, thereby making it possible to introduce a desired sequence of the linear DNA fragment into VZV-BAC-DNA. RecET and redαβ exhibit homologous recombination via a homologous sequence having a length of about 25 to 50 nucleotides. Therefore, the recombination functions of recET and redαβ can be used more easily than recA-mediated homologous recombination.
(3) Use of a transposon:
   The function of a transposon element to insert into a nucleic acid in *E. coli* is used. For example, a transposon element containing a desired foreign gene and VZV-BAC-DNA are introduced into *E. coli* so that the transposon element is randomly inserted into VZV-BAC-DNA. Thereby, VZV-BAC-DNA having the inserted foreign gene is obtained.

Further, for example, it is also possible to introduce a random mutation in recombinant varicella-zoster virus genome by treating a host cell having recombinant varicella-zoster virus such as VZV-BAC-DNA employing a mutagenic agent (for example, nitrosoguanidine).

### (Formulation)

The present invention also provides methods of treatment and/or prevention of diseases or disorders (e.g., infectious diseases) by administration to a subject of an effective amount of a therapeutic/prophylactic agent. By the therapeutic/prophylactic agent is meant a composition of the present invention in combination with a pharmaceutically acceptable carrier type (e. g., a sterile carrier).

The therapeutic/prophylactic agent will be formulated and dosed in a fashion consistent with good medical practice, taking into account the clinical condition of the individual patient (especially the side effects of treatment with the therapeutic/prophylactic agent alone), the site of delivery, the method of administration, the scheduling of administration, and other factors known to those skilled in the art. The "effective amount" for purposes herein is thus determined by such considerations.

Asa generalproposition,the totalpharmaceutically effective amount of the therapeutic/prophylactic agent administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the cellular physiologically active material of the present invention. If given continuously, the therapeutic/prophylactic agent is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

The therapeutic/prophylactic agents can be administered orally, rectally, parenterally, intracistemally, intravaginally, intraperitoneally, topically (as by powders, ointments, gels, drops or transdermal patch), or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The therapeutic/prophylactic agents of the invention are also suitably administered by sustained-release systems. Suitable examples of sustained-release therapeutic/prophylactic agents are administered orally, rectally, parenterally, intracistemally, intravaginally, intraperitoneally, topically (as by powders, ointments, gels, drops or transdermal patch), or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

For parenteral administration, in one embodiment, the therapeutic/prophylactic agent is formulated generally by mixing it at the desired degree of purity, in a unit dosage injectable form (solution, suspension, or emulsion), with a pharmaceutically acceptable carrier, i. e., one that is non-toxic to recipients at the dosages and concentrations employed and is compatible with other ingredients of the formulation. For example, the formulation preferably does not include oxidizing agents and other compounds that are known to be deleterious to the therapeutic/prophylactic agent.

Generally, the formulations are prepared by contacting the therapeutic/prophylactic agent uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

Any pharmaceutical used for therapeutic administration can be free from organisms and viruses other than a virus as an effective ingredient, i.e., sterile. Sterility is readily accomplished by filtration through sterile filtration membranes (e.g., 0.2 micron membranes). Therapeutic/prophylactic agents generally are placed into a container having a sterile access port, for example, an intravenous solution bag or vial having a stopper pierceable by a hypodermic injection needle.

Therapeutic/prophylactic agents ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous therapeutic/prophylactic agent solution, and the resulting mixture is lyophilized. The infusion solution is prepared by reconstituting the lyophilized therapeutic/prophylactic agent using bacteriostatic Water-for-injection.

The invention also provides a pharmaceutical pack or kit comprising one or more containers filled with one or more of the ingredients of the therapeutic/prophylactic agents of the invention. Associated with such container (s) can be a notice in the formprescribed by a governmental agency regulating the manufacture, use or sale of pharmaceuticals or biological products, which notice reflects approval by the agency of manufacture, use or sale for human administration. In addition, the therapeutic/prophylactic agents may be employed in conjunction with other therapeutic compounds.

The therapeutic/prophylactic agents of the invention may be administered alone or in combination with other therapeutic agents. Therapeutic/prophylactic agents that may be administered in combination with the therapeutic/prophylactic agents of the invention, include but not limited to, chemotherapeutic agents, antibiotics, steroidal and nonsteroidal anti-inflammatories, conventional immunotherapeutic agents, cytokines and/or growth factors. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.

In certain embodiments, the therapeutic/prophylactic agents of the invention are administered in combination with antiretroviral agents, nucleoside reverse transcriptase inhibitors, nonnucleoside reverse transcriptase inhibitors, and/or protease inhibitors.

In a further embodiment, the therapeutic/prophylactic agents of the invention are administered in combination with an antibiotic agent. Antibiotic agents that may be used include, but are not limited to, aminoglycoside antibiotics, polyene antibiotics, penicillin antibiotics, cephem antiboitics, peptide antibiotics, microride antibiotics, and tetracycline antibiotics.

In an additional embodiment, the therapeutic/prophylactic agents of the invention are administered alone or in combination with an anti-inflammatory agent. Anti-inflammatory agents that may be administered with the therapeutic/prophylactic agents of the invention include, but are not limited to, glucocorticoids and the nonsteroidal anti-inflammatories, aminoarylcarboxylic acid derivatives, arylacetic acid derivatives, arylbutyric acid derivatives, arylcarboxylic acids, arylpropionic acid derivatives, pyrazoles, pyrazolones, salicylic acid derivatives, thiazinecarboxamides, e-acetamidocaproic acid, S-adenosylmethionine, 3-amino-4-hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazole, and tenidap.

In a further embodiment, the therapeutic/prophylactic agent of the present invention is administered in combination with other therapeutic/prophylactic regimens (e.g., radiation therapy).

Hereinafter, the present invention will be described by way of examples. However, the present invention is not limited to these examples.

### (Example 1)

### (Preparation of recombinant varicella-zoster virus)

### (1: Preparation of BAC plasmid)

Plasmid PHA-2 used was kindly provided by Markus Wagner and Ulrich H. Koszinowski (Adler et al., (2000), J. Virol. 74: 6964-74). To prepare a recombinant virus, the region between gene 11 and gene 12 of varicella-zoster virus virus genome is selected as the insertion point of a BAC vector. This is because the insertion of a foreign nucleic acid into such a non-essential region was expected to have no adverse effect on the replication of varicella-zoster virus.

Fragments of the gene 11 ORF and the gene 12 ORF of varicella-zoster virus Oka strain was amplified with the genomic DNA of varicella-zoster virus Oka original strain as a template using primers VZ11F (SEQ ID NO.: 1) and VZ11R (SEQ ID NO.: 2) and primers VZ12F (SEQ ID NO.: 3) and VZ12R (SEQ ID NO.: 4), respectively.

### (2: Preparation of primers used for producing recombinant plasmid)

**[Table 1]**

| Primers used to produce a recombinant plasmid | | |
|---|---|---|
| Primer | Sequence | Product (base pair) and plasmid |
| VZ11F | 5-LLL ACTAGT GCGGCCGC TTACGAAAACGTGCATG-3 SpeI NotI | VZ ORF11(2052) SK/VZ11-12 |
| VZ11R | 5'-C[?] [?]G ACCTGCT TTTATTTTACAAACTCCTTTGTGG 3' BfuAI | |
| VZ12 | 5'GC GC AC[?] AGG[?] CTCTGG TTAGACCTT AAAATTGG-3' Sac[?]I | WORF12(2161) SK/VZ11-12 |
| VZ12R | 5'-TATA GCGGCCGC TTTTAATCTGGTTGTGGAAATG-3' NotI | |

In the table, a restriction enzyme site in the sequence of an oligonucleotide is underlined, while an italicized sequence indicates a base which does not exist in the VZV sequence.

Fragments of the gene 11 ORF and the gene 12 ORF, which were produced by PCR were digested by SpeI/SexAI and NotI/SexAI, respectively. Two PCR fragments were cloned to pBluescript SK- (Stratagene) digested by SpeI and NotI. The resultant plasmid was designated as SK/VZ11-12.

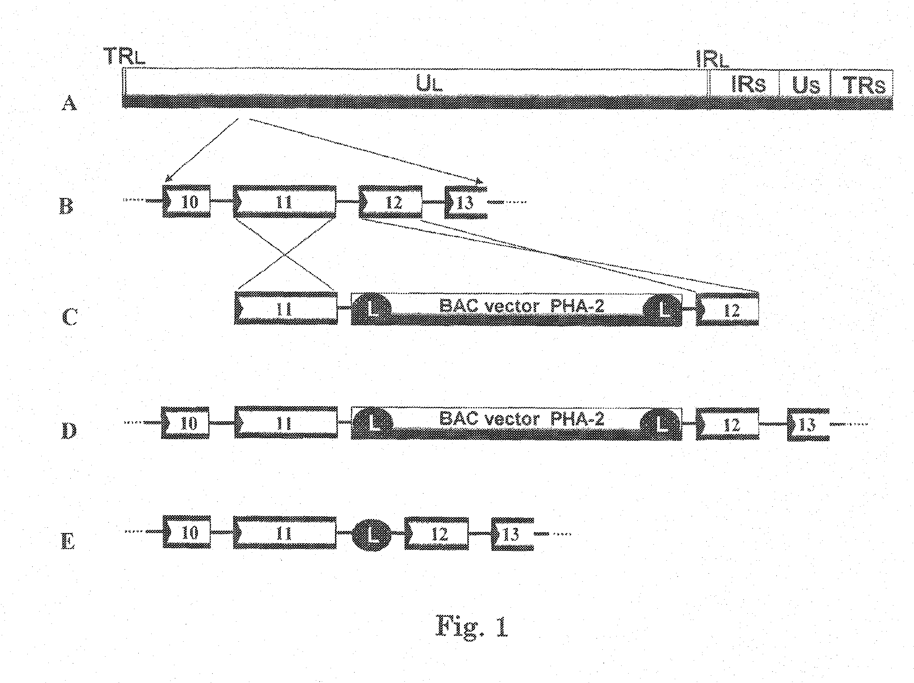

Plasmid pHA-2 was digested by Pacl, and then, this site was blunt-ended by treatment with T4 DNA polymerase. This plasmid was cloned into the SexAI site which was blunt-ended in SK/VZ11-12. The resulting plasmid was designated as pHA-2/VZ11-12 (Figure 1C).

As shown in Figure 1, VZV genome (A) is about 125 kbp in length, and comprises terminal repeat (TR) DNA domain, uniquely long (UR) DNA domain, internal repeat (IR) DNA domain, and uniquely short (US) DNA domain. To construct a recombinant plasmid PHA-2/VZV11-12(C), as mentioned above, an ORF 11 fragment and an ORF 12 fragment of the VZV genome were produced by PCR amplification using appropriate primers. This recombinant plasmid pHA-2/VZ11-12 included flanking homologous sequence of about 2.0 kbp, which is adjacent to loxP site (L), and the BAC vector.

### (3: Preparation of recombinant virus by homologous recombination)

The prepared plasmid pHA-2/VZ11-12 contains a guanine phosphoribosyl transferase (gpt) gene as a selectable marker. The BAC vector sequence is sandwiched between two loxP sequences. Therefore, the BAC vector sequence sandwiched between the loxP sequences can be efficiently removed by the action of Cre recombinase. In addition, cells into which the plasmid containing the BAC vector sequence has been introduced can be easily confirmed by the fluorescence of a green fluorescent protein (GFP).

The plasmid was digested with NotI for linearization. Nucleofector unit (Amaxa) was used to transfect HEL cells cultured to confluent in a 75-cm² plastic flask with 0.2 µg of the linearized pHA-2/VZ11-12 by electroporation. One day after transfection, the transfected cells were infected with varicella-zoster virus Oka strain.

Mycophenolic acid (50 µM) and 200 µM xanthine were used to screen recombinant viruses based on the gpt gene. In the HEL cells, the cytopathic effect (CPE) typical for varicella-zoster virus was observed. Some of the cells could be confirmed under a microscope to express the GFP. This result indicates that the BAC vector was inserted into the varicella-zoster virus genome and the GFP gene was expressed.

### (4: Enrichment of recombinant virus and introduction of it into E. coli)

A recombinant virus was enriched by selection using the gpt gene in combination with mycophenolic acid and xanthine, and use of limiting dilution assay of 96 well plate. From the infected cells, circular virus DNA was extracted by Hirt's method (Hirt, (1967), J. M. Biol, 26: 365-9). The extracted DNA was introduced into *E. coli* DH10B by the electroporation method (0.2-cm cuvette, 2.5 kV) using the gene pulser (Bio-Rad) for transformation. *E. coli* containing VZV-BAC-DNA was obtained by screening on agar containing 17 µg/ml chloramphenicol.

### (5: Stability of VZV-BAC-DNA plasmid in E. coli)

*E. coli* including the BAC vector (VZV-BAC-DNA) including varicella-zoster virus genome was cultured for 22-24 hours in LB Broth, passaged 3 times by employing the same method, and finally, selected on an agar-plate including chloramphenicol. Five clones were picked from the passaged *E. coli,* and each of the five clones were cultured in large scale in LB Broth by employing the same method, and DNA was extracted. VZV-BAC-DNA was extracted from E. coliusing the Nucleobond PC 100 kit (Macherey-Nagel) in accordance with the protocol accompanying the kit. The resultant five clones and the original VZV-BAC-DNA were digested with restriction enzyme BamHI. The restriction enzyme patterns were verified on an agarose gel by electrophoresis (the result is not shown). All five clones were compared with the original VZV BAC plasmid. As a result, the identical restriction enzyme pattern was shown on the agarose gel. This indicated high stability of VZV plasmid in *E. coli.*

In these Figures, the original VZV-BAC-DNA and the VZV-BAC-DNA, which was passaged 3 times showed the identical electrophoratic pattern. This indicates that VZV-BAC-DNA plasmid is stable in *E. coli.*

### (6: Production of virus from VZV-BAC-DNA)

BAC cloned VZV rV01 (Figure 1D) was produced in HEL cells by homologous recombination of a recombinant plasmid PHA-2/VZV11-12 and VZV virus. Specifically, HEL cells cultured to confluent in a 75-cm² plastic flask were transfected with 1 µg VZV-BAC-DNA using Nucleofector unit (Amaxa), and then the HEL cells were cultured to confluency in a 75-cm² plastic flask and were passaged two days after transfection. Two or three days after passage, typical CPE of varicella-zoster was observed. Also, cells, in which CPE were observed, were confirmed under a fluorescent microscope to express the GFP gene. Consequently, it was confirmed that the recombinant varicella-zoster virus could be produced using VZV-BAC-DNA. The produced recombinant varicella-zoster virus was designated as rV01 (Figure 1D). VZV BAC plasmid was produced by introducing circular BAC cloned genome in *E. coli.*

### (7: Cutting out of BAC vector sequence)

Recombinant adenovirus (AxCANCre) capable of expressing Cre recombinase (Kanegae et al., (1995) Nucleic Acids Res 23:3816-21) was kindly provided by Yasushi Kawaguchi of the Nagoya University. VZV rV02 (Figure 1E; L represents loxP site) was produced by superinfection with BAC cloned VZV rV01 and the recombinant adenovirus (AxCANCre) using this recombinant adenovirus. Specifically, HEL cells were infected with the recombinant adenovirus at a MOI (multiplicity of infection) of 100. After 2 hours of virus adsorption, the cells were washed with PBS (-), followed by culturing in DMEM medium containing 5% FCS. The HEL cells were superinfected with recombinant varicella-zoster virus rV01 24 hours after infection with recombinant adenovirus. A control experiment was conducted to confirm that the recombinant adenovirus expressed Cre recombinase and that a BAC vector sequence was efficiently cut out from rV01 genome. The resultant varicella virus was designated rV02 (Figure 1, E). The result of DNA sequencing confirmed that rV02 was a nucleic acid produced by cutting out a BAC vector sequence from rV01.

DNA which is extracted from cells infected with varicella-zoster virus Oka strain and VZV-BAC-DNA derived from *E. coli* were digested by restrection enzyme BamHI. Fragments derived from recombinant varicella-zoster virus rV02 DNA are larger than those of varicella-zoster virus Oka strain DNA due to the remaining one loxP sequence.

Regarding the electrophoratic pattern of VZV-BAC-DNA compared with parent strain, due to insertion of BAC vector, BamHI fragments of about 8.1 kbp disappeared, while BamHI fragments of about 7.8 kbp and 9.2 kbp were added. Also,a BamHI fragment of about 8.2 kbp of DNA, which was extracted from recombinant varicella virus rV02 infected cells, increased in size in comparison with the about 8.1 kbp BamHI fragment of the parent varicella-zoster virus, because of one loxP sequence which remains when BAC vector sequence was cut out.

### (Example 2)

### (Characterization of recombinant varicella-zoster virus)

### (1: Comparison of growth ability of recombinant viruses)

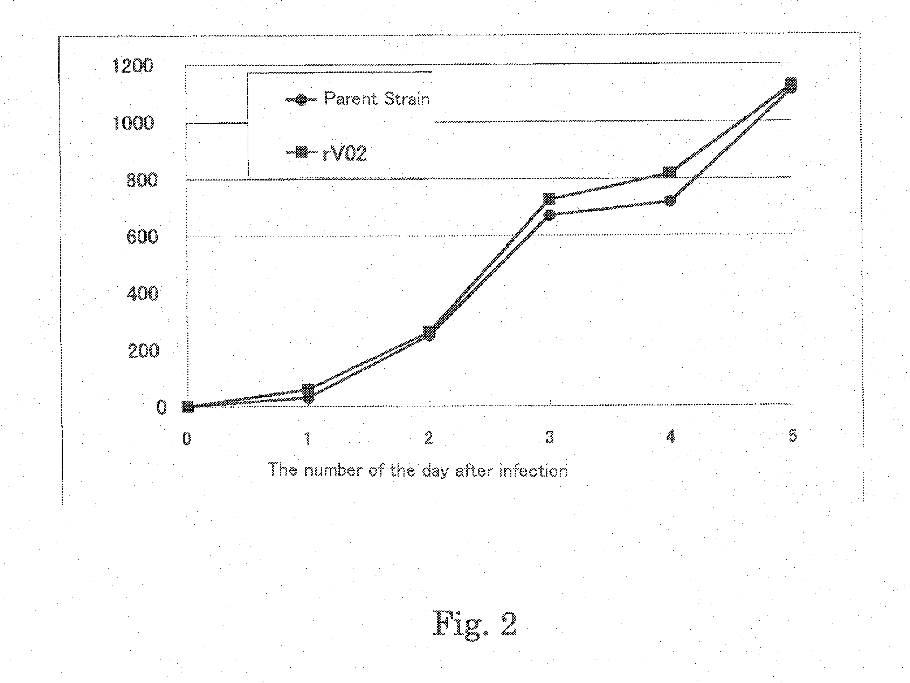

Varicella-zoster virus Oka strain and the obtained recombinant varicella-zoster virus rV02 are compared in terms of the growth ability in HEL cells using the infectious center assay method (Gomi et al., (2002) J. Virol 76 :11447-59). HEL cells in a 35mm dish were infected at a MOI of 0.01 PFU/cell, and the infected cells were washed. After varicella-zoster virus Oka strain and rV02 strain with which HEL cells were infected were cultured from day 0 to day 5, and harvested with trypsin, these strains were infected to new HEL cells to compare the replication ability thereof. The numbers of the infected cells are normalized to the initial virus titer/dish. Multiple growth indicates the number of infected cells transmitted from one infected cell at 0 day. The result is shown at Figure 2. As shown in Figure 2, it indicates that the obtained recombinant varicella virus rV02 exhibits the same replication ability as that of varicella virus Oka strain (parent strain) *in vitro*.

### (Example 3)

### (Production of mutant recombinant varicella-zoster virus with low pathogenicity)

According to the present invention, it is possible to prepare a mutant recombinant varicella-zoster virus and to obtain a mutant varicella-zoster virus strain with low pathogenicity in a mutated virus using the following method.

### (1: Preparation of mutant recombinantvaricella-zoster virus)

As a method for preparing mutant recombinant varicella-zoster virus including, for example, homologous recombination between a nucleic acid containing a mutated gene and VZV-BAC-DNA plasmid to produce mutant recombinant varicella-zoster virus. A mutated gene, which is used to cause homologous recombination with VZV-BAC-DNA plasmid may include random mutation and may include site-directed mutation. By employing each of the above methods, it is possible to obtain a population of mutant recombinant varicella-zoster virus with random mutation and a population of mutant recombinant varicella-zoster virus with site-directed mutation. The detailed description of the foregoing is as follows.

### (1.1: Preparation of mutant recombinant varicella-zoster virus with random mutation)

It is known that some of viruses which contain mutation in gene 62 of varicella-zoster virus genome are attenuated viruses. Therefore, in the present Example, gene 62 to which a mutation is randomly introduced using PCR is produced. The method of mutagenesis using PCR is well known. For example, it is possible to introduce a mutation randomly by using thermostable polymerase without proofreading function under the condition that the amount of one of the four nucleotides is small. Optionally, a marker gene such as a drug-resistance gene may be linked to the mutated 62 gene.

Thus, the prepared mutated gene 62 is introduced into *E. coli* with VZV-BAC-DNA plasmid according to Example 1 (4: Enrichment of recombinant virus and introduction of it into *E. coli*), and then, homologous recombination was allowed to occur between mutated gene 62 and VZV-BAC-DNA. After that, according to the method described in Example 1, DNA of varicella-zoster virus which causes homologous recombination is isolated and introduced to *E. coli,* and *E. coli* including VZV-BAC-DNA which causes homologous recombination is obtained.

The obtained plurality of *E. coli* contains VZV-BAC-DNA including gene 62 having mutations which are different from each other. Then, the degree of pathogenicity of varicella-zoster virus which is produced by mutant VZV-BAC-DNA included in each *E. coli* is screened using a method below (2: method of examining the pathogenicity of varicella-zoster virus).

### (1.2: Preparation of mutant recombinant varicella-zoster virus containing a site-directed mutation)

The methods for introducing the desired site-directed mutation is well-known in the art. For example, the full-length gene containing the desired mutation is prepared by conducting PCR using primers containing the desired mutation so as to prepare the fragment of the gene containing the desired mutation, and then, by further conducting PCR using the fragments of the mutated gene or by treating with an enzyme, such as a restriction enzyme.

Thus, mutant recombinant varicella-zoster virus containing a site-directed mutation is prepared using the procedure of above-mentioned (1.1.), regarding the prepared mutated gene.

### (2: method of examining the pathogenicity of varicella-zoster virus)

The two methods for examining the pathogenicity of varicella-zoster virus have been established.

As a method using an animal model, the method for evaluating the pathogenicity by producing a severe combined immunodeficient (SCID) mouse to which human skin is transplanted, and then, to infect the mouse with varicella-zoster virus is well-known (J. Viro 1. 1998 Feb; 72(2): 965-74,).

On the other hand, as a method for evaluating the pathogenicity *in vitro* is well-known, which comprises: placing monolayer cultured human melanoma in a lower-well of a two-layered well, which are separated by a trans-well of pore size 3 µm; placing cord-blood mononuclear cells (CBMC) infected with varicella-zoster virus in the upper-well; and culturing the cells for 7-8 days; then observing CPE (cytopathic effect) of the melanoma (J. Virol. 2000 Feb; 74(4): 1864-70).

Although it is not the method for confirming the pathogenicity directly, according to the previous study of the present inventors (J. Virol. 2002 Nov; 76(22): 11447-59), close relationships between the pathogenicity and the proliferation of a virus is understood, thus, it is also possible to evaluate the pathogenicity indirectly by examining the proliferation of cell-to-cell employing infectious center assay.

### (Example 4)

### (Production of vaccine)

The recombinant varicella-zoster virus obtained in Example 1 is inoculated in MRC-5 cell culture in 20 Roux bottles having a culture area of 210 cm², followed by culturing. After completion of culturing, culture medium is discarded, and the infected cells in each Roux bottle are washed with 200 ml of PBS(-) twice. Next, 20 ml of 0.03% (w/v) EDTA-3Na is overlaid on the infected cells in each Roux bottle, so that the cells are detached from the wall of the Rouxbottle and suspended. The infected cell suspension in each Roux bottle is pooled, followed by centrifugation at 2, 000 rpm for 10 minutes at 4°C to collect a pellet of the infected cells. The cells are resuspended in 100 ml of PBS (-), followed by freezing and thawing once. Next, the cells are subjected to ultrasonication in ice bath (20 KHz, 150 mA, 0.3 sec/ml), followed by centrifugation at 3, 000 rpm for 20 minutes at 4°C. The supernatant containing viruses released from the cells is collected, which is used as a live vaccine stock solution. 30 ml of the stock solution is sampled for examination, while saccharose and gelatin hydolysate dissolved in PBS(-), which serves as a vaccine stabilizer, is added and mixed into the remaining stock solution (70 ml) to a final concentration of 5% (w/v) and 2.5% (w/v). As a result, 140 ml of a live vaccine final bulk was prepared. 30 ml of the final bulk is sampled for examination. Thereafter, the remainder of the bulk is dispensed into 3 ml volume vials (0.5 ml for each). After lyophilization, the vial is filled with nitrogen gas and is closed with a rubber cap to hermetically seal the inside of the vial. The live vaccine aliquots are preserved at 4°C. Immediately before use, 0.5 ml of distilled water for injection is added to the lyophilized contents which are completely dissolved. On the other hand, the above-described sampled vaccine stock solution and final bulk, and 20 aliquots are subjected to assay tests. The tests are conducted to confirm safety, effectiveness, and uniformity for qualification of a live vaccine, taking into consideration the Guidelines for Biological Formulations defined under Notice No. 195 of Ministry of Health and Welfare (1989) and the guideline "recombinant precipitation hepatitis B vaccine (derived from yeast)" as defined therein. The results of the tests show that the above-described aliquot has a virus content of 2x10⁴ PFU (plaque-forming unit)/0.5 ml. When passing each test described in the guidelines, the vaccine is subsequently used as a qualified live vaccine.

### (Example 5)

### (Determination of immunogenicity of recombinant varicella-zoster virus vaccine)

Immunogenicity of recombinant varicella-zoster virus vaccine strain produced in Example 4 is measured using guinea-pigs. Oka strain live vaccine is used as a control. These vaccines are subcutaneously vaccined to each of three guinea pigs of 3 weeks old (average weight is 250g). Vaccination is adjusted by diluting each vaccine using PBS (-) so that the amount of recombinant strain and Oka strain live vaccine is 3, 000 PFU/guinea pig or 2, 000 PFU/guinea pig. Four, six, and eight weeks after vaccination, blood is collected from the vein in the femoral region of each vaccined guinea pig to measure the antibody value in the blood. The Neutralizing test method (Journal of General Virology, 61, 255-269, 1982) is employed for measurement of antibody value. It is confirmed that recombinant varicella-zoster virus vaccine elicit anti-VZV antibody to the same degree with Oka strain. From these results, recombinant varicella-zoster virus vaccine with good immunogenicity is selected.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

The present invention provides a method for producing recombinant varicella-zoster virus from a single virus strain using, for example, BAC (*E. coli* artificial chromosome), and recombinant varicella-zoster virus produced by the method. The present invention also provides a pharmaceutical composition comprising recombinant varicella-zoster virus.

Further, the present invention provides a vector comprising a varicella-zoster virus genomic gene and a BAC vector sequence, a cell containing such a vector, and a nucleic acid cassette comprising a fragment capable of homologous recombination with a varicella-zoster virus genome, and a BAC vector sequence.

### (Description of sequence table)

SEQ ID NO.: 1, VZ11F primer
SEQ ID NO.: 2, VZ11R primer
SEQ ID NO.: 3, VZ12F primer
SEQ ID NO.: 4, VZ12R primer
SEQ ID NO.: 5, sequence of the gene 62
SEQ ID NO.: 6, sequence of the gene 62
SEQ ID NO.: 7, sequence of plasmid PHA-2
SEQ ID NO.: 8, varicella-zoster virus Dumas strain
SEQ ID NO.: 9, 5'→3' direction 1134 to 1850 of SEQ ID NO.: 8, nucleic acid sequence encoded (gene 2)
SEQ ID NO.: 10, 5'→3' direction 8607 to 9386 of SEQ ID NO.: 8, nucleic acid sequence encoded (gene 7)
SEQ ID NO.: 11, 5'→3' direction 10642 to 10902 of SEQ ID NO.: 8, nucleic acid sequence encoded (gene 9A)
SEQ ID NO.: 12, 5'→3' direction 11009 to 11917 of SEQ ID NO.: 8, nucleic acid sequence encoded (gene 9)
SEQ ID NO.: 13, 5'→3' direction 12160 to 13392 of SEQ ID NO.: 8, nucleic acid sequence encoded (gene 10)
SEQ ID NO.: 14, 5'→3' direction 13590 to 16049 of SEQ ID NO.: 8, nucleic acid sequence encoded (gene 11)
SEQ ID NO.: 15, 5'→3' direction 16214 to 18199 of SEQ ID NO.: 8, nucleic acid sequence encoded (gene 12)
SEQ ID NO.: 16, 5'→3' direction 18441 to 19346 of SEQ ID NO.: 8, nucleic acid sequence encoded (gene 13)
SEQ ID NO.: 17, 5'→3' direction 24149 to 25516 of SEQ ID NO.: 8, nucleic acid sequence encoded (gene 17)
SEQ ID NO.: 18, 5'→3' direction 30759 to 33875 of SEQ ID NO.: 8, nucleic acid sequence encoded (gene 21)
SEQ ID NO.: 19, 5'→3' direction 34083 to 42374 of SEQ ID NO.: 8, nucleic acid sequence encoded (gene 22)
SEQ ID NO.: 20, 5'→3' direction 44506 to 46263 of SEQ ID NO.: 8, nucleic acid sequence encoded (gene 26)
SEQ ID NO.: 21, 5'→3' direction 50857 to 54471 of SEQ ID NO.: 8, nucleic acid sequence encoded (gene 29)
SEQ ID NO.: 22, 5'→3' direction 54651 to 56963 of SEQ ID NO.: 8, nucleic acid sequence encoded (gene 30)
SEQ ID NO.: 23, 5'→3' direction 57008 to 59614 of SEQ ID NO.: 8, nucleic acid sequence encoded (gene 31)
SEQ ID NO.: 24, 5'→3' direction 59766 to 60197 of SEQ ID NO.: 8, nucleic acid sequence encoded (gene 32)
SEQ ID NO.: 25, 5'→3' direction 64807 to 65832 of SEQ ID NO.: 8, nucleic acid sequence encoded (gene 36)
SEQ ID NO.: 26, 5'→3' direction 66074 to 68599 of SEQ ID NO.: 8, nucleic acid sequence encoded (gene 37)
SEQ ID NO.: 27, 5'→3' direction 70633 to 71355 of SEQ ID NO.: 8, nucleic acid sequence encoded (gene 39)
SEQ ID NO.: 28, 5'→3' direction 71540 to 75730 of SEQ ID NO.: 8, nucleic acid sequence encoded (gene 40)
SEQ ID NO.: 29, 5'→3' direction 75847 to 76797 of SEQ ID NO.: 8, nucleic acid sequence encoded (gene 41)
SEQ ID NO.: 30, 5'→3' direction 78170 to 80200 of SEQ ID NO.: 8, nucleic acid sequence encoded (gene 43)
SEQ ID NO.: 31, 5'→3' direction 80360 to 81451 of SEQ ID NO.: 8, nucleic acid sequence encoded (gene 44)
SEQ ID NO.: 32, 5'→3' direction 82719 to 83318 of SEQ ID NO.: 8, nucleic acid sequence encoded (gene 46)
SEQ ID NO.: 33, 5'→3' direction 84667 to 86322 of SEQ ID NO.: 8, nucleic acid sequence encoded (gene 48)
SEQ ID NO.: 34, 5'→3' direction 87881 to 90388 of SEQ ID NO.: 8, nucleic acid sequence encoded (gene 51)
SEQ ID NO.: 35, 5'→3' direction 90493 to 92808 of SEQ ID NO.: 8, ucleic acid sequence encoded (gene 52)
SEQ ID NO.: 36, 5'→3' direction 95996 to 98641 of SEQ ID NO.: 8, nucleic acid sequence encoded (gene 55)
SEQ ID NO.: 37, 5'→3' direction 110581 to 111417 of SEQ ID NO.: 8, nucleic acid sequence encoded (gene 63)
SEQ ID NO.: 38, 5'→3' direction 111565 to 112107 of SEQ ID NO.: 8, nucleic acid sequence encoded (gene 64)
SEQ ID NO.: 39, 5'→3' direction 113037 to 114218 of SEQ ID NO.: 8, nucleic acid sequence encoded (gene 66)
SEQ ID NO.:40, 5'→3' direction 114496 to 115560 of SEQ ID NO.: 8, nucleic acid sequence encoded (gene 67)
SEQ ID NO.: 41, 5'→3' direction 115808 to 117679 of SEQ ID NO.: 8, nucleic acid sequence encoded (gene 68)
SEQ ID NO.: 42, 5'→3' direction 120764 to 124696 of SEQ ID NO.: 8, nucleic acid sequence encoded (gene 71)
SEQ ID NO.: 43 partial sequence of SEQ ID No.:8 (gene 27)
SEQ ID NO.: 44, 5'→3' direction 1 to 999 of SEQ ID NO.: 43, nucleic acid sequence encoded (gene 27)
SEQ ID NO.: 45, partial sequence of SEQ ID No.:8 (gene 47)
SEQ ID NO.: 46, 5'→3' direction 1 to 1530 of SEQ ID NO. : 45, nucleic acid sequence encoded (gene 47)
SEQ ID NO.: 47, partial sequence of SEQ ID No.:8
SEQ ID NO. : 48, 5'→3' direction 1 to 243 of SEQ ID NO. : 47, nucleic acid sequence encoded (gene 49)
SEQ ID NO.: 49, partial sequence of SEQ ID No.:8
SEQ ID NO.: 50, 5'→3' direction 1 to 732 of SEQ ID NO.: 49, nucleic acid sequence encoded (gene 56)
SEQ ID NO.: 51, partial sequence of SEQ ID No.:8
SEQ ID NO.: 52, 3'→5' direction 118480 to 119316 of SEQ ID NO.: 8, nucleic acid sequence encoded (corresponding to 5569 to 6405 of SEQ ID No.: 51)(gene 70)
SEQ ID NO.: 53, 3'→5' direction 117790 to 118332 of SEQ ID NO.: 8, nucleic acid sequence encoded (corresponding to 6553 to 7095 of SEQ ID No.: 51)(gene 69)
SEQ ID NO.: 54, 3'→5' direction 112332 to 112640 of SEQ ID NO.: 8, nucleic acid sequence encoded (corresponding to 12245 to 12553 of SEQ ID No.: 51)(gene 65)
SEQ ID NO.: 55, 3'→5' direction 105201 to 109133 of SEQ ID NO.: 8, nucleic acid sequence encoded (corresponding to 15752 to 19684 of SEQ ID No.: 51)(gene 62)
SEQ ID NO.: 56, 3'→5' direction 103082 to 104485 of SEQ ID NO.: 8, nucleic acid sequence encoded (corresponding to 20400 to 21803 of
SEQ ID No.: 51)(gene 61)
SEQ ID NO.: 57, 3'→5' direction 100302 to 101219 of SEQ ID NO.: 8, nucleic acid sequence encoded (corresponding to 23666 to 24583 of SEQ ID No.: 51)(gene 59)
SEQ ID NO.: 58, 3'→5' direction 99411 to 99626 of SEQ ID NO.: 8, nucleic acid sequence encoded (corresponding to 25259 to 25474 of SEQ ID No.: 51)(gene 57)
SEQ ID NO.: 59, 3'→5' direction 92855 to 93850 of SEQ ID NO.: 8, nucleic acid sequence encoded (corresponding to 31035 to 32030 of SEQ ID No.: 51)(gene 53)
SEQ ID NO.: 60, 3'→5' direction 68668 to 70293 of SEQ ID NO.: 8, nucleic acid sequence encoded (corresponding to 54592 to 56217 of SEQ ID No.: 51)(gene 38)
SEQ ID NO.: 61, 3'→5' direction 63977 to 64753 of SEQ ID NO.: 8, nucleic acid sequence encoded (corresponding to 60132 to 60908 of SEQ ID No.: 51)(gene 35)
SEQ ID NO.: 62, 3'→5' direction 62171 to 63910 of SEQ ID NO.: 8, nucleic acid sequence encoded (corresponding to 60975 to 62714 of SEQ ID No.: 51)(gene 34)
SEQ ID NO.: 63, 3'→5' direction 60321 to 62138 of SEQ ID NO.: 8, nucleic acid sequence encoded (corresponding to 62747 to 64564 of SEQ ID No.: 51)(gene 33)
SEQ ID NO.: 64, 3'→5' direction 47052 to 50636 of SEQ ID NO.: 8, nucleic acid sequence encoded (corresponding to 74249 to 77833 of SEQ ID No.: 51)(gene 28)
SEQ ID NO.: 65, 3'→5' direction 44148 to 44618 of SEQ ID NO.: 8, nucleic acid sequence encoded (corresponding to 80267 to 80737 of SEQ ID No.: 51)(gene 25)
SEQ ID NO.: 66, 3'→5' direction 43212 to 44021 of SEQ ID NO.: 8, nucleic acid sequence encoded (corresponding to 80864 to 81673 of SEQ ID No.: 51)(gene 24)
SEQ ID NO.: 67, 3'→5' direction 42431 to 43138 of SEQ ID NO.: 8, nucleic acid sequence encoded (corresponding to 81747 to 82454 of SEQ ID No.: 51)(gene 23)
SEQ ID NO.: 68, 3'→5' direction 29024 to 30475 of SEQ ID NO.: 8, nucleic acid sequence encoded (corresponding to 94410 to 95861 of SEQ ID No.: 51)(gene 20)
SEQ ID NO.: 69, 3'→5' direction 26518 to 28845 of SEQ ID NO.: 8, nucleic acid sequence encoded (corresponding to 96040 to 98392 of SEQ ID No.: 51)(gene 19)
SEQ ID NO.: 70, 3'→5' direction 25573 to 26493 of SEQ ID NO.: 8, nucleic acid sequence encoded (corresponding to 98392 to 99312 of SEQ ID No.: 51)(gene 18)
SEQ ID NO.: 71, 3'→5' direction 22568 to 23794 of SEQ ID NO.: 8, nucleic acid sequence encoded (corresponding to 101091 to 102317 of SEQ ID No.: 51)(gene 16)
SEQ ID NO.: 72, 3'→5' direction 21258 to 22478 of SEQ ID NO.: 8, nucleic acid sequence encoded (corresponding to 102407 to 103627 of SEQ ID No.: 51)(gene 15)
SEQ ID NO.: 73, 3'→5' direction 19431 to 21113 of SEQ ID NO.: 8, nucleic acid sequence encoded (corresponding to 103772 to 105454 of SEQ ID No.: 51)(gene 14)
SEQ ID NO.: 74, 3'→5' direction 9477 to 10667 of SEQ ID NO.: 8, nucleic acid sequence encoded (corresponding to 114218 to 115408 of SEQ ID No.: 51)(gene 8)
SEQ ID NO.: 75, 3'→5' direction 5326 to 8577 of SEQ ID NO.: 8, nucleic acid sequence encoded (corresponding to 116308 to 119559 of SEQ ID No.: 51)(gene 6)
SEQ ID NO.: 76, 3'→5' direction 4252 to 5274 of SEQ ID NO.: 8, nucleic acid sequence encoded (corresponding to 119611 to 120633 of SEQ ID No.: 51)(gene 5)
SEQ ID NO.: 77, 3'→5' direction 2783 to 4141 of SEQ ID NO.: 8, nucleic acid sequence encoded (corresponding to 120744 to 122102 of SEQ ID No.: 51)(gene 4)
SEQ ID NO.: 78, 3'→5' direction 1908 to 2447 of SEQ ID NO.: 8, nucleic acid sequence encoded (corresponding to 122438 to 122977 of SEQ ID No.: 51)(gene 3)
SEQ ID NO.: 79, 3'→5' direction 589 to 915 of SEQ ID NO. : 8, nucleic acid sequence encoded (corresponding to 123970 to 124296 of SEQ ID No.: 51)(gene 1)
SEQ ID NO.: 80, partial sequence of SEQ ID No.:51
SEQ ID NO.: 81, 3'→5' direction 1 to 1056 and 4556 to 5740 of SEQ ID NO.: 80, nucleic acid sequence encoded (corresponding to 46847 to 48034 and 42292 to 43347 of SEQ ID No.: 51) (gene 42 and gene 45)
SEQ ID NO.: 82, partial sequence of SEQ ID No.:51
SEQ ID NO.: 83, 3'→5' direction 1 to 1305 of SEQ ID NO. : 82, nucleic acid sequence encoded (corresponding to 123580 to 124884 of SEQ ID No.: 51)(gene 50)
SEQ ID NO.: 84, partial sequence of SEQ ID No.:51
SEQ ID NO.: 85, 3'→5' direction 1 to 2307 of SEQ ID NO. : 84, nucleic acid sequence encoded (corresponding to 122578 to 124884 of SEQ ID No.: 51)(gene 54)
SEQ ID NO.: 86, partial sequence of SEQ ID No.:51
SEQ ID NO. : 87, 3'→5' direction 1 to 663 of SEQ ID NO.: 86, nucleic acid sequence encoded (corresponding to 124222 to 124884 of SEQ ID No.: 51)(gene 58)
SEQ ID NO.: 88, partial sequence of SEQ ID No.:51
SEQ ID NO.: 89, 3'→5' direction 1 to 427 of SEQ ID NO.: 88, nucleic acid sequence encoded (corresponding to 124458 to 124884 of SEQ ID No.: 51) (gene 60)
SEQ ID NO.: 90, partial sequence of SEQ ID No.:51
SEQ ID NO. : 91, 3'→5' direction 1 to 903 of SEQ ID NO.: 90, nucleic acid sequence encoded (corresponding to 60321 to 61229 of SEQ ID No.: 51)(gene 33.5)

## Claims

1. A recombinant varicella-zoster virus.

2. A vector comprising a varicella-zoster virus essential gene and a BAC vector sequence other than the gene 62.

3. A cell comprising the vector of claim 2.

4. A method to produce recombinant varicella-zoster virus, comprising:
introducing a vector comprising a varicella-zoster virus genome essential gene other than gene 62 and BAC vector sequence into a mammalian host cell; and
culturing the mammalian host cell to produce recombinant varicella-zoster virus.

5. A virus produced by the method of claim 4.

6. A method to introduce a mutation into the vector of claim 2, comprising:
introducing the vector into a bacterial host cell;
introducing a plasmid vector comprising a fragment consisting of a portion of varicella-zoster virus genome into the bacterial host cell, wherein the fragment has at least one mutation;
culturing the bacterial host cell;
isolating a vector having BAC sequence from the cultured bacterial host cell.

7. A method to introduce a mutation into the vector of claim 2, comprising:
introducing the vector into a bacterial host cell;
introducing a first plasmid vector comprising a first fragment consisting of a portion of varicella-zoster virus genome into the bacterial host cell, wherein the first fragment has at least one mutation;
introducing a second plasmid vector comprising a second fragment consisting of a portion of varicella-zoster virus genome into the bacterial host cell, wherein the second fragment has at least one mutation, and the first fragment is different from the second fragment;
culturing the bacterial host cell;
isolating a vector having BAC sequence from the cultured bacterial host cell.

8. A nucleic acid cassette comprising a first fragment which can recombine with varicella-zoster virus genome in a bacterial cell, BAC vector sequence, and a second fragment which can recombine with varicella-zoster virus genome in a bacterial cell,
wherein the both ends of the BAC sequence are linked to the first and the second fragment, respectively.

9. The vector of claim 2 or the method of claim 4, wherein a portion where a sequence derived from the varicella-zoster virus genome is linked to the BAC vector sequence is within a non-essential region of the varicella-zoster virus genome.

10. A recombinant varicella-zoster virus of claim 1, comprising BAC vector sequence.

11. The recombinant varicella-zoster virus of claim 10, wherein at least part of the BAC vector sequence is inserted into a non-essential region of a varicella-zoster virus genome.

12. The vector or method of claim 9 or the recombinant varicella-zoster virus of claim 11, wherein the non-essential region is selected from the group consisting of the following regions:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, and the region flanking the ORF of gene 70.

13. The recombinant varicella-zoster virus, vector or method of claim 12, wherein the non-essential region is the region flanking the ORF of gene 11, or the region flanking the ORF of gene 12.

14. The recombinant varicella-zoster virus of claim 10, wherein at least part of the BAC vector sequence is inserted into the region in the ORF of gene 62 of a varicella-zoster virus genome; or the vector of claim 2 or method of claim 4, wherein a portion where a sequence derived from the varicella-zoster virus genome is linked to the BAC vector sequence is in the ORF of gene 62 of a varicella-zoster virus genome.

15. The recombinant varicella-zoster virus of claim 10, the vector of claim 2, the method of claim 4 or the nucleic acid cassette of claim 8, wherein the BAC vector sequence comprises recombinant protein dependent recombinant sequence.

16. The recombinant varicella-zoster virus of claim 10, the vector of claim 2, the method of claim 4 or the nucleic acid cassette of claim 8, wherein the BAC vector sequence comprises a selectable marker.

17. The recombinant varicella-zoster virus, vector, method or nucleic acid cassette of claim 16, wherein the selectable marker is a drug selectable marker or a gene encoding green fluorescent protein.

18. The recombinant varicella-zoster virus of claim 10, the vector of claim 2, the method of claim 4 or the nucleic acid cassette of claim 8, wherein the varicella-zoster virus genome is derived from a wild type strain, a mutant type strain, or Oka vaccine strain.

19. The recombinant varicella-zoster virus of claim 10, the vector of claim 2 or the method of claim 4, wherein the varicella-zoster virus genome have mutations in gene 62 and gene 6.

20. The recombinant varicella-zoster virus, vector, or method of claim 19, wherein the gene 62 comprises at least the base substitutions of the following (a)-(d) in SEQ ID NO.5:
(a) base substitution at position 2110 for G;
(b) base substitution at position 3100 for G;
(c) base substitution at position 3818 for C; and
(d) base substitution at position 4006 for G,
and, the gene 6 comprises at least a base substitution at position 5745 for G, in SEQ ID NO.8.

21. The recombinant varicella-zoster virus of claim 10, the vector of claim 2, the method of claim 4 or the nucleic acid cassette of claim 8, wherein the BAC vector sequence comprises the sequence set forth in SEQ ID NO.: 7.

22. A pharmaceutical composition comprising the virus of claim 1 or claim 5.

23. The pharmaceutical composition of claim 22, wherein the composition is in the form of a vaccine.

24. The vector of claim 2 or the method of claim 4, wherein the vector further comprises the gene 62.

25. The vector of claim 2, wherein a mammalian cell produces a varicella-zoster virus when the vector is introduced into the mammalian cell.

26. The cell of claim 3, wherein the cell is a bacterial cell, preferably *E. coli,* or a mammalian cell, preferably derived from human.

27. A virus produced by a cell of claim 26, wherein the cell is a mammalian cell.

28. A pharmaceutical composition comprising the virus of claim 27, preferably wherein the composition is in the form of a vaccine.

29. The method of claim 4, wherein the mammalian host cell is derived from human.

30. The method of claim 4, wherein the BAC vector sequence comprises at least two recombinant protein dependent recombinant sequences.

31. The method of claim 30, further comprising a step of recombination between the two recombinant protein dependent recombinant sequences.

32. The nucleic acid cassette of claim 8, wherein the first fragment and the second fragment are at least 1kb, preferably at least 1.5kb, more preferably at least 2kb.

33. The nucleic acid cassette of claim 8, wherein the first fragment and the second fragment are at least 80%, preferably at least 85%, 90% or 95%, identical with a varicella-zoster virus genome sequence.

34. The nucleic acid cassette of claim 8, wherein each of the first fragment and the second fragment are independently selected from the group consisting of the following regions of varicella-zoster virus genome:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

35. The nucleic acid cassette of claim 8, wherein each of the first fragment and the second fragment is independently at least 80%, preferably at least 85%, 90% or 95%, identical with the region selected from the group consisting of the following regions of varicella-zoster virus genome:
the region in the ORF of gene 7, the region in the ORF of gene 8, the region in the ORF of gene 9, the region in the ORF of gene 10, the region in the ORF of gene 11, the region in the ORF of gene 12, the region in the ORF of gene 13, the region in the ORF of gene 14, the region in the ORF of gene 15, the region in the ORF of gene 17, the region in the ORF of gene 18, the region in the ORF of gene 19, the region in the ORF of gene 38, the region in the ORF of gene 39, the region in the ORF of gene 46, the region in the ORF of gene 47, the region in the ORF of gene 48, the region in the ORF of gene 49, the region in the ORF of gene 50, the region in the ORF of gene 56, the region in the ORF of gene 57, the region in the ORF of gene 58, the region in the ORF of gene 59, the region in the ORF of gene 61, the region in the ORF of gene 62, the region in the ORF of gene 63, the region in the ORF of gene 64, the region in the ORF of gene 65, the region in the ORF of gene 66, the region in the ORF of gene 67, the region in the ORF of gene 68, the region in the ORF of gene 69, the region in the ORF of gene 70, the region flanking the ORF of gene 7, the region flanking the ORF of gene 8, the region flanking the ORF of gene 9, the region flanking the ORF of gene 10, the region flanking the ORF of gene 11, the region flanking the ORF of gene 12, the region flanking the ORF of gene 13, the region flanking the ORF of gene 14, the region flanking the ORF of gene 15, the region flanking the ORF of gene 17, the region flanking the ORF of gene 18, the region flanking the ORF of gene 19, the region flanking the ORF of gene 38, the region flanking the ORF of gene 39, the region flanking the ORF of gene 46, the region flanking the ORF of gene 47, the region flanking the ORF of gene 48, the region flanking the ORF of gene 49, the region flanking the ORF of gene 50, the region flanking the ORF of gene 56, the region flanking the ORF of gene 57, the region flanking the ORF of gene 58, the region flanking the ORF of gene 59, the region flanking the ORF of gene 61, the region flanking the ORF of gene 62, the region flanking the ORF of gene 63, the region flanking the ORF of gene 64, the region flanking the ORF of gene 65, the region flanking the ORF of gene 66, the region flanking the ORF of gene 67, the region flanking the ORF of gene 68, the region flanking the ORF of gene 69, the region flanking the ORF of gene 70.

36. The nucleic acid cassette of claim 8, wherein the first fragment and the second fragment are derived from different regions.

37. The nucleic acid cassette of claim 33, wherein each of the first fragment and the second fragment are independently from the region flanking the ORF of gene 11 or the region flanking the ORF of gene 12.

38. The nucleic acid cassette of claim 8, having a nucleic acid sequence set forth in SEQ ID NO.: 2.


SEQUENCE LISTING

<110>  The Research Foundation for Microbial Diseases of Osaka
       University

<120>  Recombinant varicella-zoster virus

<130>  P/51854.EP02

<150>  JP 2004-063277
<151>  2004-03-05

<160>  91

<170>  PatentIn version 3.2

<210>  1
<211>  35
<212>  DNA
<213>  Artificial

<220>
<223>  primer

<400>  1
tataactagt gcggccgctt acgaaaacgt gcatg                                35

<210>  2
<211>  34
<212>  DNA
<213>  Artificial

<220>
<223>  primer

<400>  2
cgcgacctgg tttattttac aaactccttt gtgg                                 34

<210>  3
<211>  34
<212>  DNA
<213>  Artificial

<220>
<223>  primer

<400>  3
gcgcaccagg tctctgttta gaccttaaaa tttg                                 34

<210>  4
<211>  34
<212>  DNA
<213>  Artificial

<220>
<223>  primer

<400>  4
tatagcggcc gcttttaatc tggttgtgga aatg                                 34

<210>  5
<211>  4226
<212>  DNA
<213>  Varicella zoster

<220>
<221>  CDS
<222>  (229)..(4158)

<400>  5
   atactatggt ccatgaactt cccgcctcga gtctcgtcca atcactacat cgtcttatca      60
   ttaagaatat ttacacggtg acgacacggg gaggaaatat gcggtcgagg ggggggcaca      120
   acacgtttta agtactgttg gaactccctc accaaccgca aycgcaatcc tttgaaggct     180
   gcgagagcgt ttggaaaact cgggtacgtc taaattcacc ccagygcg atg gat acg      237
                                                        Met Asp Thr
                                                        1
   ccg ccg atg cag cgc tct aca ccc caa cgc gcg ggg tcg cct gat act       285
   Pro Pro Met Gln Arg Ser Thr Pro Gln Arg Ala Gly Ser Pro Asp Thr
       5                   10                  15
   ttg gag tta atg gac ctg ttg gac gcg gcc gcc gcg gcc gcc gaa cac      333
   Leu Glu Leu Met Asp Leu Leu Asp Ala Ala Ala Ala Ala Ala Glu His
   20                  25                  30                  35
   agg gcc cgg gtg gtc acc tcg agt cag cct gac gat cta cta ttt gga       381
   Arg Ala Arg Val Val Thr Ser Ser Gln Pro Asp Asp Leu Leu Phe Gly
                   40                  45                  50
   gag aac ggg gtc atg gtg gga cgg gaa cat gag atc gtt tca att ccc       429
   Glu Asn Gly Val Met Val Gly Arg Glu His Glu Ile Val Ser Ile Pro
               55                  60                  65
   tcc gta tcg gga ctt caa cca gaa ccc aga acg gaa gat gtt ggc gaa       477
   Ser Val Ser Gly Leu Gln Pro Glu Pro Arg Thr Glu Asp Val Gly Glu
           70                  75                  80
   gag cta aca caa gac gac tac gta tgc gag gac ggt cag gat cta ayg       525
   Glu Leu Thr Gln Asp Asp Tyr Val Cys Glu Asp Gly Gln Asp Leu Xaa
       85                  90                  95
   ggc tcg cct gta atc ccg ctg gcc gag gtc ttc cac acc cga ttc tcg       573
   Gly Ser Pro Val Ile Pro Leu Ala Glu Val Phe His Thr Arg Phe Ser
   100                 105                 110                 115
   gag gcc ggc gcg cga gaa cca aca gga gcc gat cgc tcc ctc gag aca       621
   Glu Ala Gly Ala Arg Glu Pro Thr Gly Ala Asp Arg Ser Leu Glu Thr
                   120                 125                 130
   gtc tct ctc gga acg aag ctt gct agg tct cca aaa cca ccg atg aac       669
   Val Ser Leu Gly Thr Lys Leu Ala Arg Ser Pro Lys Pro Pro Met Asn
               135                 140                 145
   gat ggg gaa acg ggc aga ggt acg acc cct ccg ttc ccg cag gcc ttc       717
   Asp Gly Glu Thr Gly Arg Gly Thr Thr Pro Pro Phe Pro Gln Ala Phe
           150                 155                 160
   tcc cct gta tcc ccc gcg tct cct gtt gga gac gcc gcc ggg aac gat       765
   Ser Pro Val Ser Pro Ala Ser Pro Val Gly Asp Ala Ala Gly Asn Asp
       165                 170                 175
   caa cgg gaa gac cag cgg tct ata ccc cga caa acg acg aga gga aat       813
   Gln Arg Glu Asp Gln Arg Ser Ile Pro Arg Gln Thr Thr Arg Gly Asn
   180                 185                 190                 195
   tca cca ggt ttg ccg tcg gtg gtc cat cga gac aga caa act cag tcc       861
   Ser Pro Gly Leu Pro Ser Val Val His Arg Asp Arg Gln Thr Gln Ser
                   200                 205                 210
   atc tcg ggt aaa aag ccg ggc gat gag caa gcg ggt cat gcg cat gca 909
   Ile Ser Gly Lys Lys Pro Gly Asp Glu Gln Ala Gly His Ala His Ala
   215 220 225
   tcg ggg gac gga gta gtt ctc cag aaa act caa cgg ccc gct cag gga 957
   Ser Gly Asp Gly Val Val Leu Gln Lys Thr Gln Arg Pro Ala Gln Gly
   230 235 240
   aag agc ccg aag aaa aag act ttg aag gtt aag gtc cca ctc ccg gcg 1005
   Lys Ser Pro Lys Lys Lys Thr Leu Lys Val Lys Val Pro Leu Pro Ala
   245 250 255
   cgg aaa ccc ggt gga cct gta ccc ggc ccg gtt gag caa ttg tac cac 1053
   Arg Lys Pro Gly Gly Pro Val Pro Gly Pro Val Glu Gln Leu Tyr His
   260 265 270 275
   gtc ctt tcg gac agc gtt ccc gct aag ggg gca aag gcg gac ctg ccg 1101
   Val Leu Ser Asp Ser Val Pro Ala Lys Gly Ala Lys Ala Asp Leu Pro
   280 285 290
   ttt gag acc gat gat acc cgc cca agg aaa cat gat gcc cgg ggt ata 1149
   Phe Glu Thr Asp Asp Thr Arg Pro Arg Lys His Asp Ala Arg Gly Ile
   295 300 305
   aca cct cgc gtc cct gga cgt tcg tcg ggg ggc aaa cct aga gcg ttt 1197
   Thr Pro Arg Val Pro Gly Arg Ser Ser Gly Gly Lys Pro Arg Ala Phe
   310 315 320
   ttg gcc ctg ccg gga aga tcc cac gca cca gac ccg att gag gat gac 1245
   Leu Ala Leu Pro Gly Arg Ser His Ala Pro Asp Pro Ile Glu Asp Asp
   325 330 335
   agc ccg gtg gag aaa aag cca aag agt cgt gag ttt gtt tcg tct tca 1293
   Ser Pro Val Glu Lys Lys Pro Lys Ser Arg Glu Phe Val Ser Ser Ser
   340 345 350 355
   tcc tct tcc tcg tcg tgg gga tcg tca tcg gag gat gaa gac gat gaa 1341
   Ser Ser Ser Ser Ser Trp Gly Ser Ser Ser Glu Asp Glu Asp Asp Glu
   360 365 370
   ccc cgg cgc gtt tcg gtg gga agt gaa act aca ggc agc agg tcc gga 1389
   Pro Arg Arg Val Ser Val Gly Ser Glu Thr Thr Gly Ser Arg Ser Gly
   375 380 385
   cgc gaa cac gcc cct tcc ccg tca aat tcg gat gat tcg gac tca aat 1437
   Arg Glu His Ala Pro Ser Pro Ser Asn Ser Asp Asp Ser Asp Ser Asn
   390 395 400
   gat ggt ggg tcg acg aaa caa aat atc caa ccg gga tat cga tcc atc 1485
   Asp Gly Gly Ser Thr Lys Gln Asn Ile Gln Pro Gly Tyr Arg Ser Ile
   405 410 415
   agc ggt ccc gat ccg agg att cgt aag acc aaa cgt ctt gcg ggg gaa 1533
   Ser Gly Pro Asp Pro Arg Ile Arg Lys Thr Lys Arg Leu Ala Gly Glu
   420 425 430 435
   ccg ggg cgc cag aga cag aaa tca ttt tcc ctg ccg cga tcc aga acc 1581
   Pro Gly Arg Gln Arg Gln Lys Ser Phe Ser Leu Pro Arg Ser Arg Thr
   440 445 450
   ccg ata att ccc ccg gtg tcg ggg ccg ctc atg atg ccc gac gga agc 1629
   Pro Ile Ile Pro Pro Val Ser Gly Pro Leu Met Met Pro Asp Gly Ser
   455 460 465
   cct tgg ccc gga tcg gcg ccc ctc cca tcc aac agg gtg cgg ttt gga 1677
   Pro Trp Pro Gly Ser Ala Pro Leu Pro Ser Asn Arg Val Arg Phe Gly
   470 475 480
   ccg tcc ggg gag acc aga gag ggt cac tgg gag gat gag gct gtg aga 1725
   Pro Ser Gly Glu Thr Arg Glu Gly His Trp Glu Asp Glu Ala Val Arg
   485 490 495
   gcg gcg cgg gct cgt tac gag gcc tca act gaa ccc gyg ccg ctt tac 1773
   Ala Ala Arg Ala Arg Tyr Glu Ala Ser Thr Glu Pro Xaa Pro Leu Tyr
   500 505 510 515
   gtg ccg gag ttg gga gat ccg gct aga cag tac cgc gcg ctg att aac 1821
   Val Pro Glu Leu Gly Asp Pro Ala Arg Gln Tyr Arg Ala Leu Ile Asn
   520 525 530
   ctg atc tac tgt cca gac aga gac cct ata gca tgg ctc cag aac ccc 1869
   Leu Ile Tyr Cys Pro Asp Arg Asp Pro Ile Ala Trp Leu Gln Asn Pro
   535 540 545
   aag ctg acc ggt gtc aac tcg gcc ctg aac cag ttc tac caa aag ctg 1917
   Lys Leu Thr Gly Val Asn Ser Ala Leu Asn Gln Phe Tyr Gln Lys Leu
   550 555 560
   ttg cca ccg gga cgg gcg ggt acc gcc gtt acg ggg agc gta gcg tct 1965
   Leu Pro Pro Gly Arg Ala Gly Thr Ala Val Thr Gly Ser Val Ala Ser
   565 570 575
   ccc gtt ccg cat gta ggc gaa gcc atg gcc acg ggg gag gcc ctc tgg 2013
   Pro Val Pro His Val Gly Glu Ala Met Ala Thr Gly Glu Ala Leu Trp
   580 585 590 595
   gct ctc ccc cac gcg gcc gcg gcc gtg gct atg agc cgt cgg tac gac 2061
   Ala Leu Pro His Ala Ala Ala Ala Val Ala Met Ser Arg Arg Tyr Asp
   600 605 610
   cgg gcc caa aaa cac ttt atc cta cag agt ctc cgc aga gcc ttt gcc 2109
   Arg Ala Gln Lys His Phe Ile Leu Gln Ser Leu Arg Arg Ala Phe Ala
   615 620 625
   ggc atg gca tac ccc gag gca acg ggc tcc agt ccg gcg gcg cgg atc 2157
   Gly Met Ala Tyr Pro Glu Ala Thr Gly Ser Ser Pro Ala Ala Arg Ile
   630 635 640
   tcc cgc ggt cac cct tct cca aca acc ccg gcc aca cag act ccc gac 2205
   Ser Arg Gly His Pro Ser Pro Thr Thr Pro Ala Thr Gln Thr Pro Asp
   645 650 655
   cct cag ccg tcg gcc gcc gcg cgc tct ctt tct gtg tgt cca ccg gat 2253
   Pro Gln Pro Ser Ala Ala Ala Arg Ser Leu Ser Val Cys Pro Pro Asp
   660 665 670 675
   gat cgt tta cga act ccg cgc aag cgc aag tcc cag cca gtc gag agc 2301
   Asp Arg Leu Arg Thr Pro Arg Lys Arg Lys Ser Gln Pro Val Glu Ser
   680 685 690
   aga agc ctc ctc gac aag att agg gag aca ccc gtc gcg gac gcc cgg 2349
   Arg Ser Leu Leu Asp Lys Ile Arg Glu Thr Pro Val Ala Asp Ala Arg
   695 700 705
   gtt gca gac gat cat gtg gtt tcc aag gcc aag agg cgg gta tcc gag 2397
   Val Ala Asp Asp His Val Val Ser Lys Ala Lys Arg Arg Val Ser Glu
   710 715 720
   ccc gtg acc atc acc tcg ggc cct gtg gtg gat ccc ccc gcc gta ata 2445
   Pro Val Thr Ile Thr Ser Gly Pro Val Val Asp Pro Pro Ala Val Ile
   725 730 735
   acg atg cca ctt gac gga ccg gcc cca aac ggg gga ttt cgg cgt att 2493
   Thr Met Pro Leu Asp Gly Pro Ala Pro Asn Gly Gly Phe Arg Arg Ile
   740 745 750 755
   ccc cgg ggg gcc ctg cat acc ccg gtc ccg tcg gac cag gct cgc aag 2541
   Pro Arg Gly Ala Leu His Thr Pro Val Pro Ser Asp Gln Ala Arg Lys
   760 765 770
   gcg tac tgt acc ccc gaa acc atc gcc cgt ctg gtc gac gac cca ttg 2589
   Ala Tyr Cys Thr Pro Glu Thr Ile Ala Arg Leu Val Asp Asp Pro Leu
   775 780 785
   ttt ccc acg gcc tgg cgc cct gcg cta agc ttt gat ccc ggc gcc ttg 2637
   Phe Pro Thr Ala Trp Arg Pro Ala Leu Ser Phe Asp Pro Gly Ala Leu
   790 795 800
   gcg gaa atc gcc gct cgg cgt ccg ggc gga gga gac cga cgg ttt ggt 2685
   Ala Glu Ile Ala Ala Arg Arg Pro Gly Gly Gly Asp Arg Arg Phe Gly
   805 810 815
   cca ccc agc gga gtg gag gcg ctg cga cgg agg tgc gcc tgg atg cgg 2733
   Pro Pro Ser Gly Val Glu Ala Leu Arg Arg Arg Cys Ala Trp Met Arg
   820 825 830 835
   cag atc cca gac ccg gag gat gtg agg ctt ctg atc atc tac gat ccg 2781
   Gln Ile Pro Asp Pro Glu Asp Val Arg Leu Leu Ile Ile Tyr Asp Pro
   840 845 850
   ttg ccc gga gag gac atc aac ggc ccc ctc gag agc acc ctc gcg aca 2829
   Leu Pro Gly Glu Asp Ile Asn Gly Pro Leu Glu Ser Thr Leu Ala Thr
   855 860 865
   gat ccg gga ccg tca tgg agt cca tcc cga ggg gga ctg tct gtg gtc 2877
   Asp Pro Gly Pro Ser Trp Ser Pro Ser Arg Gly Gly Leu Ser Val Val
   870 875 880
   ctg gca gcc ctg agt aac cgg ttg tgc ctg ccg agc act cat gcc tgg 2925
   Leu Ala Ala Leu Ser Asn Arg Leu Cys Leu Pro Ser Thr His Ala Trp
   885 890 895
   gcc ggg aac tgg acc ggc ccg ccg gac gtg tcc gct ttg aac gcc cgg 2973
   Ala Gly Asn Trp Thr Gly Pro Pro Asp Val Ser Ala Leu Asn Ala Arg
   900 905 910 915
   ggc gtt tta tta ctg tcg acc cga gac ctg gcc ttt gcc ggg gcc gtc 3021
   Gly Val Leu Leu Leu Ser Thr Arg Asp Leu Ala Phe Ala Gly Ala Val
   920 925 930
   gag tat cta ggc tcg cgg ttg gcc tct gcc cgg cgc cgg ttg ctg gtg 3069
   Glu Tyr Leu Gly Ser Arg Leu Ala Ser Ala Arg Arg Arg Leu Leu Val
   935 940 945
   ttg gac gcg gtg gcc ctc gag agg tgg ccc ggg gat gga ccc gct ttg 3117
   Leu Asp Ala Val Ala Leu Glu Arg Trp Pro Gly Asp Gly Pro Ala Leu
   950 955 960
   tct cag tat cac gtg tac gtc cgg gcc ccg gcg cga ccg gac gcc cag 3165
   Ser Gln Tyr His Val Tyr Val Arg Ala Pro Ala Arg Pro Asp Ala Gln
   965 970 975
   gcc gtc gtc cga tgg cca gac tcg gcg gtc aca gaa gga ctc gcc cgg 3213
   Ala Val Val Arg Trp Pro Asp Ser Ala Val Thr Glu Gly Leu Ala Arg
   980 985 990 995
   gcc gtg ttt gca tcg tcg cgc acc ttt ggg cca gcg agt ttt gct 3258
   Ala Val Phe Ala Ser Ser Arg Thr Phe Gly Pro Ala Ser Phe Ala
   1000 1005 1010
   cgt atc gag act gcg ttt gcc aac ctg tac ccg ggc gaa caa ccc 3303
   Arg Ile Glu Thr Ala Phe Ala Asn Leu Tyr Pro Gly Glu Gln Pro
   1015 1020 1025
   ctg tgt ttg tgc cgc ggt ggg aac gtc gca tac acc gtg tgt acc 3348
   Leu Cys Leu Cys Arg Gly Gly Asn Val Ala Tyr Thr Val Cys Thr
   1030 1035 1040
   cgc gcg ggc ccc aag acc cgc gtc ccc ctg tcg ccc cgt gaa tac 3393
   Arg Ala Gly Pro Lys Thr Arg Val Pro Leu Ser Pro Arg Glu Tyr
   1045 1050 1055
   cgg cag tac gtg ctg ccg ggt ttt gac ggt tgc aag gac ctc gcg 3438
   Arg Gln Tyr Val Leu Pro Gly Phe Asp Gly Cys Lys Asp Leu Ala
   1060 1065 1070
   cga cag tct cgg ggt ctg ggg ctc ggg gca gcc gac ttt gtg gac 3483
   Arg Gln Ser Arg Gly Leu Gly Leu Gly Ala Ala Asp Phe Val Asp
   1075 1080 1085
   gag gcg gca cat agc cac cgc gca gca aac cga tgg ggc ctg ggt 3528
   Glu Ala Ala His Ser His Arg Ala Ala Asn Arg Trp Gly Leu Gly
   1090 1095 1100
   gcc gcg ctt cga ccc gtc ttc ctt ccc gag gga cgg aga ccg ggg 3573
   Ala Ala Leu Arg Pro Val Phe Leu Pro Glu Gly Arg Arg Pro Gly
   1105 1110 1115
   gcc gcc ggg ccg gag gcc ggc gac gta ccc acc tgg gcg agg gtg 3618
   Ala Ala Gly Pro Glu Ala Gly Asp Val Pro Thr Trp Ala Arg Val
   1120 1125 1130
   ttt tgc cgc cac gcc ctg ctg gaa ccc gac cct gcc gcg gaa cca 3663
   Phe Cys Arg His Ala Leu Leu Glu Pro Asp Pro Ala Ala Glu Pro
   1135 1140 1145
   ctc gtg ctt cca ccc gtg gcc ggt cgg tcg gtg gcg ctg tat gcg 3708
   Leu Val Leu Pro Pro Val Ala Gly Arg Ser Val Ala Leu Tyr Ala
   1150 1155 1160
   tcg gcg gac gag gct cgg aat gcc ctc ccc ccg att ccc aga gta 3753
   Ser Ala Asp Glu Ala Arg Asn Ala Leu Pro Pro Ile Pro Arg Val
   1165 1170 1175
   atg tgg ccg ccc ggt ttt ggg gcc gcg gag acg gtg ttg gag ggg 3798
   Met Trp Pro Pro Gly Phe Gly Ala Ala Glu Thr Val Leu Glu Gly
   1180 1185 1190
   agc gac gga aca cgg ttc gcg ttc gga cac cac ggg ggc tcg gaa 3843
   Ser Asp Gly Thr Arg Phe Ala Phe Gly His His Gly Gly Ser Glu
   1195 1200 1205
   cgg ccg gca gaa acc cag gcg ggg cga cag cgg cgc acc gca gac 3888
   Arg Pro Ala Glu Thr Gln Ala Gly Arg Gln Arg Arg Thr Ala Asp
   1210 1215 1220
   gac aga gaa cac gct ttg gag ccg gac gat tgg gag gtg ggg tgt 3933
   Asp Arg Glu His Ala Leu Glu Pro Asp Asp Trp Glu Val Gly Cys
   1225 1230 1235
   gaa gac gcg tgg gac agc gag gag ggg ggc ggg gac gac ggg gac 3978
   Glu Asp Ala Trp Asp Ser Glu Glu Gly Gly Gly Asp Asp Gly Asp
   1240 1245 1250
   gca ccg ggg tca tcc ttt ggg gtg agc gtc gtg tcg gtg gcc ccg 4023
   Ala Pro Gly Ser Ser Phe Gly Val Ser Val Val Ser Val Ala Pro
   1255 1260 1265
   ggt gtg ctg cga gac cgc cgg gtg ggc tyg cgc ccg gcg gtc aag 4068
   Gly Val Leu Arg Asp Arg Arg Val Gly Xaa Arg Pro Ala Val Lys
   1270 1275 1280
   gtg gag ctg ttg tcc  tcg tcc tcg tcc tcc  gag gac gag gac gat       4113
   Val Glu Leu Leu Ser  Ser Ser Ser Ser Ser  Glu Asp Glu Asp Asp
                   1285                 1290                 1295
   gtg tgg gga ggg cgc  ggg ggg agg agc ccc  ccg cag agt cgg ggg       4158
   Val Trp Gly Gly Arg  Gly Gly Arg Ser Pro  Pro Gln Ser Arg Gly
                   1300                 1305                 1310
   tgacggagtc ccctcctttt ctcgtgagcg ccacyggcgc gcggactgtt tgttgtttgt   4218
   taataaaa                                                            4226


<210>  6
<211>  1310
<212>  PRT
<213>  Varicella zoster

<220>
<221>  misc_feature
<222>  (99)..(99)
<223>  The 'Xaa' at location 99 stands for Thr, or Met.

<220>
<221>  misc_feature
<222>  (512)..(512)
<223>  The 'Xaa' at location 512 stands for Ala, or Val.

<220>
<221>  misc_feature
<222>  (1275)..(1275)
<223>  The 'Xaa' at location 1275 stands for Ser, Leu, or Xaa.

<400>  6

Met Asp Thr Pro Pro Met Gln Arg Ser Thr Pro Gln Arg Ala Gly Ser
1               5                   10                  15

Pro Asp Thr Leu Glu Leu Met Asp Leu Leu Asp Ala Ala Ala Ala Ala
            20                  25                  30

Ala Glu His Arg Ala Arg Val Val Thr Ser Ser Gln Pro Asp Asp Leu
        35                  40                  45

Leu Phe Gly Glu Asn Gly Val Met Val Gly Arg Glu His Glu Ile Val
    50                  55                  60

Ser Ile Pro Ser Val Ser Gly Leu Gln Pro Glu Pro Arg Thr Glu Asp
65                  70                  75                  80

Val Gly Glu Glu Leu Thr Gln Asp Asp Tyr Val Cys Glu Asp Gly Gln
                85                  90                  95

Asp Leu Xaa Gly Ser Pro Val Ile Pro Leu Ala Glu Val Phe His Thr
            100                 105                 110

Arg Phe Ser Glu Ala Gly Ala Arg Glu Pro Thr Gly Ala Asp Arg Ser
        115                 120                 125

Leu Glu Thr Val Ser Leu Gly Thr Lys Leu Ala Arg Ser Pro Lys Pro
    130                 135                 140

Pro Met Asn Asp Gly Glu Thr Gly Arg Gly Thr Thr Pro Pro Phe Pro
145                 150                 155                 160

Gln Ala Phe Ser Pro Val Ser Pro Ala Ser Pro Val Gly Asp Ala Ala
                165                 170                 175

Gly Asn Asp Gln Arg Glu Asp Gln Arg Ser Ile Pro Arg Gln Thr Thr
            180                 185                 190

Arg Gly Asn Ser Pro Gly Leu Pro Ser Val Val His Arg Asp Arg Gln
        195                 200                 205

Thr Gln Ser Ile Ser Gly Lys Lys Pro Gly Asp Glu Gln Ala Gly His
    210                 215                 220

Ala His Ala Ser Gly Asp Gly Val Val Leu Gln Lys Thr Gln Arg Pro
225                 230                 235                 240

Ala Gln Gly Lys Ser Pro Lys Lys Lys Thr Leu Lys Val Lys Val Pro
                245                 250                 255

Leu Pro Ala Arg Lys Pro Gly Gly Pro Val Pro Gly Pro Val Glu Gln
            260                 265                 270

Leu Tyr His Val Leu Ser Asp Ser Val Pro Ala Lys Gly Ala Lys Ala
        275                 280                 285

Asp Leu Pro Phe Glu Thr Asp Asp Thr Arg Pro Arg Lys His Asp Ala
    290                 295                 300

Arg Gly Ile Thr Pro Arg Val Pro Gly Arg Ser Ser Gly Gly Lys Pro
305                 310                 315                 320

Arg Ala Phe Leu Ala Leu Pro Gly Arg Ser His Ala Pro Asp Pro Ile
                325                 330                 335

Glu Asp Asp Ser Pro Val Glu Lys Lys Pro Lys Ser Arg Glu Phe Val
            340                 345                 350

Ser Ser Ser Ser Ser Ser Ser Ser Trp Gly Ser Ser Ser Glu Asp Glu
        355                 360                 365

Asp Asp Glu Pro Arg Arg Val Ser Val Gly Ser Glu Thr Thr Gly Ser
    370                 375                 380

Arg Ser Gly Arg Glu His Ala Pro Ser Pro Ser Asn Ser Asp Asp Ser
385                 390                 395                 400

Asp Ser Asn Asp Gly Gly Ser Thr Lys Gln Asn Ile Gln Pro Gly Tyr
                405                 410                 415

Arg Ser Ile Ser Gly Pro Asp Pro Arg Ile Arg Lys Thr Lys Arg Leu
            420                 425                 430

Ala Gly Glu Pro Gly Arg Gln Arg Gln Lys Ser Phe Ser Leu Pro Arg
        435                 440                 445

Ser Arg Thr Pro Ile Ile Pro Pro Val Ser Gly Pro Leu Met Met Pro
    450                 455                 460

Asp Gly Ser Pro Trp Pro Gly Ser Ala Pro Leu Pro Ser Asn Arg Val
465                 470                 475                 480

Arg Phe Gly Pro Ser Gly Glu Thr Arg Glu Gly His Trp Glu Asp Glu
                485                 490                 495

Ala Val Arg Ala Ala Arg Ala Arg Tyr Glu Ala Ser Thr Glu Pro Xaa
            500                 505                 510

Pro Leu Tyr Val Pro Glu Leu Gly Asp Pro Ala Arg Gln Tyr Arg Ala
        515                 520                 525

Leu Ile Asn Leu Ile Tyr Cys Pro Asp Arg Asp Pro Ile Ala Trp Leu
    530                 535                 540

Gln Asn Pro Lys Leu Thr Gly Val Asn Ser Ala Leu Asn Gln Phe Tyr
545                 550                 555                 560

Gln Lys Leu Leu Pro Pro Gly Arg Ala Gly Thr Ala Val Thr Gly Ser
                565                 570                 575

Val Ala Ser Pro Val Pro His Val Gly Glu Ala Met Ala Thr Gly Glu
            580                 585                 590

Ala Leu Trp Ala Leu Pro His Ala Ala Ala Ala Val Ala Met Ser Arg
        595                 600                 605

Arg Tyr Asp Arg Ala Gln Lys His Phe Ile Leu Gln Ser Leu Arg Arg
    610                 615                 620

Ala Phe Ala Gly Met Ala Tyr Pro Glu Ala Thr Gly Ser Ser Pro Ala
625                 630                 635                 640

Ala Arg Ile Ser Arg Gly His Pro Ser Pro Thr Thr Pro Ala Thr Gln
                645                 650                 655

Thr Pro Asp Pro Gln Pro Ser Ala Ala Ala Arg Ser Leu Ser Val Cys
            660                 665                 670

Pro Pro Asp Asp Arg Leu Arg Thr Pro Arg Lys Arg Lys Ser Gln Pro
        675                 680                 685

Val Glu Ser Arg Ser Leu Leu Asp Lys Ile Arg Glu Thr Pro Val Ala
    690                 695                 700

Asp Ala Arg Val Ala Asp Asp His Val Val Ser Lys Ala Lys Arg Arg
705                 710                 715                 720

Val Ser Glu Pro Val Thr Ile Thr Ser Gly Pro Val Val Asp Pro Pro
                725                 730                 735

Ala Val Ile Thr Met Pro Leu Asp Gly Pro Ala Pro Asn Gly Gly Phe
            740                 745                 750

Arg Arg Ile Pro Arg Gly Ala Leu His Thr Pro Val Pro Ser Asp Gln
        755                 760                 765

Ala Arg Lys Ala Tyr Cys Thr Pro Glu Thr Ile Ala Arg Leu Val Asp
    770                 775                 780

Asp Pro Leu Phe Pro Thr Ala Trp Arg Pro Ala Leu Ser Phe Asp Pro
785                 790                 795                 800

Gly Ala Leu Ala Glu Ile Ala Ala Arg Arg Pro Gly Gly Gly Asp Arg
                805                 810                 815

Arg Phe Gly Pro Pro Ser Gly Val Glu Ala Leu Arg Arg Arg Cys Ala
            820                 825                 830

Trp Met Arg Gln Ile Pro Asp Pro Glu Asp Val Arg Leu Leu Ile Ile
        835                 840                 845

Tyr Asp Pro Leu Pro Gly Glu Asp Ile Asn Gly Pro Leu Glu Ser Thr
    850                 855                 860

Leu Ala Thr Asp Pro Gly Pro Ser Trp Ser Pro Ser Arg Gly Gly Leu
865                 870                 875                 880

Ser Val Val Leu Ala Ala Leu Ser Asn Arg Leu Cys Leu Pro Ser Thr
                885                 890                 895

His Ala Trp Ala Gly Asn Trp Thr Gly Pro Pro Asp Val Ser Ala Leu
            900                 905                 910

Asn Ala Arg Gly Val Leu Leu Leu Ser Thr Arg Asp Leu Ala Phe Ala
        915                 920                 925

Gly Ala Val Glu Tyr Leu Gly Ser Arg Leu Ala Ser Ala Arg Arg Arg
    930                 935                 940

Leu Leu Val Leu Asp Ala Val Ala Leu Glu Arg Trp Pro Gly Asp Gly
945                 950                 955                 960

Pro Ala Leu Ser Gln Tyr His Val Tyr Val Arg Ala Pro Ala Arg Pro
                965                 970                 975

Asp Ala Gln Ala Val Val Arg Trp Pro Asp Ser Ala Val Thr Glu Gly
            980                 985                 990

Leu Ala Arg Ala Val Phe Ala Ser  Ser Arg Thr Phe Gly  Pro Ala Ser
        995                 1000                 1005

Phe Ala  Arg Ile Glu Thr Ala  Phe Ala Asn Leu Tyr  Pro Gly Glu
    1010                 1015                 1020

Gln Pro  Leu Cys Leu Cys Arg  Gly Gly Asn Val Ala  Tyr Thr Val
    1025                 1030                 1035

Cys Thr  Arg Ala Gly Pro Lys  Thr Arg Val Pro Leu  Ser Pro Arg
    1040                 1045                 1050

Glu Tyr  Arg Gln Tyr Val Leu  Pro Gly Phe Asp Gly  Cys Lys Asp
    1055                 1060                 1065

Leu Ala  Arg Gln Ser Arg Gly  Leu Gly Leu Gly Ala  Ala Asp Phe
    1070                 1075                 1080

Val Asp  Glu Ala Ala His Ser  His Arg Ala Ala Asn  Arg Trp Gly
    1085                 1090                 1095

Leu Gly  Ala Ala Leu Arg Pro  Val Phe Leu Pro Glu  Gly Arg Arg
    1100                 1105                 1110

Pro Gly  Ala Ala Gly Pro Glu  Ala Gly Asp Val Pro  Thr Trp Ala
    1115                 1120                 1125

Arg Val  Phe Cys Arg His Ala  Leu Leu Glu Pro Asp  Pro Ala Ala
    1130                 1135                 1140

Glu Pro  Leu Val Leu Pro Pro  Val Ala Gly Arg Ser  Val Ala Leu
    1145                 1150                 1155

Tyr Ala  Ser Ala Asp Glu Ala  Arg Asn Ala Leu Pro  Pro Ile Pro
    1160                 1165                 1170

Arg Val  Met Trp Pro Pro Gly  Phe Gly Ala Ala Glu  Thr Val Leu
    1175                 1180                 1185

Glu Gly  Ser Asp Gly Thr Arg  Phe Ala Phe Gly His  His Gly Gly
    1190                 1195                 1200

Ser Glu Arg Pro Ala Glu Thr Gln Ala Gly Arg Gln Arg Arg Thr
    1205                1210                1215

Ala Asp Asp Arg Glu His Ala Leu Glu Pro Asp Asp Trp Glu Val
    1220                1225                1230

Gly Cys Glu Asp Ala Trp Asp Ser Glu Glu Gly Gly Gly Asp Asp
    1235                1240                1245

Gly Asp Ala Pro Gly Ser Ser Phe Gly Val Ser Val Val Ser Val
    1250                1255                1260

Ala Pro Gly Val Leu Arg Asp Arg Arg Val Gly Xaa Arg Pro Ala
    1265                1270                1275

Val Lys Val Glu Leu Leu Ser Ser Ser Ser Ser Ser Glu Asp Glu
    1280                1285                1290

Asp Asp Val Trp Gly Gly Arg Gly Gly Arg Ser Pro Pro Gln Ser
    1295                1300                1305

Arg Gly
    1310

<210>  7
<211>  8878
<212>  DNA
<213>  Artificial

<220>
<223>  plasmid

<400>  7
ttaattaagg ccgcagcttc ctagataact tcgtatagca tacattatac gaagttatgg      60

atctcccgcc cagcgtcttg tcattggcga actcgaacac gcagatgcag tcggggcggc     120

gcggtcccag gtccacttcg catattaagg tgacacgcgc ggcctcgaac acagctgcag     180

gccatgagcg aaaaatacat cgtcacctgg gacatgttgc agatccatgc acgtaaactc     240

gcaagccgac tgatgccttc tgaacaatgg aaaggcatta ttgccgtaag ccgtggcggt     300

ctggtaccgg gtgcgttact ggcgcgtgaa ctgggtattc gtcatgtcga taccgtttgt     360

atttccagct acgatcacga caaccagcgc gagcttaaag tgctgaaacg cgcagaaggc     420

gatggcgaag gcttcatcgt tattgatgac ctggtggata ccggtggtac tgcggttgcg     480

attcgtgaaa tgtatccaaa agcgcacttt gtcaccatct tcgcaaaacc ggctggtcgt     540

ccgctggttg atgactatgt tgttgatatc ccgcaagata cctggattga acagccgtgg     600

gatatgggcg tcgtattcgt cccgccaatc tccggtcgct aaccggtagc ggatcatcta     660

gacccgggta ccgttaactt gtttattgca gcttataatg gttacaaata aagcaatagc     720

atcacaaatt tcacaaataa agcatttttt tcactgcatt ctagttgtgg tttgtccaaa     780

ctcatcaatg tatcttatca tgtctggatc cccattctca tgtttgacag cttatcatcg     840

aatttctgcc attcatccgc ttattatcac ttattcaggc gtagcaacca ggcgtttaag 900
ggcaccaata actgccttaa aaaaattacg ccccgccctg ccactcatcg cagtactgtt 960
gtaattcatt aagcattctg ccgacatgga agccatcaca gacggcatga tgaacctgaa 1020
tcgccagcgg catcagcacc ttgtcgcctt gcgtataata tttgcccatg gtgaaaacgg 1080
gggcgaagaa gttgtccata ttggccacgt ttaaatcaaa actggtgaaa ctcacccagg 1140
gattggctga gacgaaaaac atattctcaa taaacccttt agggaaatag gccaggtttt 1200
caccgtaaca cgccacatct tgcgaatata tgtgtagaaa ctgccggaaa tcgtcgtggt 1260
attcactcca gagcgatgaa aacgtttcag tttgctcatg gaaaacggtg taacaagggt 1320
gaacactatc ccatatcacc agctcaccgt ctttcattgc catacggaat tccggatgag 1380
cattcatcag gcgggcaaga atgtgaataa aggccggata aaacttgtgc ttatttttct 1440
ttacggtctt taaaaaggcc gtaatatcca gctgaacggt ctggttatag gtacattgag 1500
caactgactg aaatgcctca aaatgttctt tacgatgcca ttgggatata tcaacggtgg 1560
tatatccagt gatttttttc tccattttag cttccttagc tcctgaaaat ctcgataact 1620
caaaaaatac gcccggtagt gatcttattt cattatggtg aaagttggaa cctcttacgt 1680
gccgatcaac gtctcatttt cgccaaaagt tggcccaggg cttcccggta tcaacaggga 1740
caccaggatt tatttattct gcgaagtgat cttccgtcac aggtatttat tcgcgataag 1800
ctcatggagc ggcgtaaccg tcgcacagga aggacagaga aagcgcggat ctgggaagtg 1860
acggacagaa cggtcaggac ctggattggg gaggcggttg ccgccgctgc tgctgacggt 1920
gtgacgttct ctgttccggt cacaccacat acgttccgcc attcctatgc gatgcacatg 1980
ctgtatgccg gtataccgct gaaagttctg caaagcctga tgggacataa gtccatcagt 2040
tcaacggaag tctacacgaa ggtttttgcg ctggatgtgg ctgcccggca ccgggtgcag 2100
tttgcgatgc cggagtctga tgcggttgcg atgctgaaac aattatcctg agaataaatg 2160
ccttggcctt tatatggaaa tgtggaactg agtggatatg ctgtttttgt ctgttaaaca 2220
gagaagctgg ctgttatcca ctgagaagcg aacgaaacag tcgggaaaat ctcccattat 2280
cgtagagatc cgcattatta atctcaggag cctgtgtagc gtttatagga agtagtgttc 2340
tgtcatgatg cctgcaagcg gtaacgaaaa cgatttgaat atgccttcag gaacaataga 2400
aatcttcgtg cggtgttacg ttgaagtgga gcggattatg tcagcaatgg acagaacaac 2460
ctaatgaaca cagaaccatg atgtggtctg tccttttaca gccagtaggc tcgccgcagt 2520
cgagcgacgg cgaagccctc gagtgagcga ggaagcacca gggaacagca cttatatatt 2580
ctgcttacac acgatgcctg aaaaaacttc ccttggggtt atccacttat ccacggggat 2640
atttttataa ttattttttt tatagttttt agatcttctt ttttagagcg ccttgtaggc 2700
ctttatccat gctggttcta gagaaggtgt tgtgacaaat tgccctttca gtgtgacaaa 2760
tcaccctcaa atgacagtcc tgtctgtgac aaattgccct taaccctgtg acaaattgcc 2820
ctcagaagaa gctgtttttt cacaaagtta tccctgctta ttgactcttt tttatttagt 2880

gtgacaatct aaaaacttgt cacacttcac atggatctgt catggcggaa acagcggtta 2940
tcaatcacaa gaaacgtaaa aatagcccgc gaatcgtcca gtcaaacgac ctcactgagg 3000
cggcatatag tctctcccgg gatcaaaaac gtatgctgta tctgttcgtt gaccagatca 3060
gaaaatctga tggcacccta caggaacatg acggtatctg cgagatccat gttgctaaat 3120
atgctgaaat attcggattg acctctgcgg aagccagtaa ggatatacgg caggcattga 3180
agagtttcgc ggggaaggaa gtggtttttt atcgccctga agaggatgcc ggcgatgaaa 3240
aaggctatga atcttttcct tggtttatca aacgtgcgca cagtccatcc agagggcttt 3300
acagtgtaca tatcaaccca tatctcattc ccttctttat cgggttacag aaccggttta 3360
cgcagtttcg gcttagtgaa acaaaagaaa tcaccaatcc gtatgccatg cgtttatacg 3420
aatccctgtg tcagtatcgt aagccggatg gctcaggcat cgtctctctg aaaatcgact 3480
ggatcataga gcgttaccag ctgcctcaaa gttaccagcg tatgcctgac ttccgccgcc 3540
gcttcctgca ggtctgtgtt aatgagatca acagcagaac tccaatgcgc ctctcataca 3600
ttgagaaaaa gaaaggccgc cagacgactc atatcgtatt ttccttccgc gatatcactt 3660
ccatgacgac aggatagtct gagggttatc tgtcacagat ttgagggtgg ttcgtcacat 3720
ttgttctgac ctactgaggg taatttgtca cagttttgct gtttccttca gcctgcatgg 3780
attttctcat actttttgaa ctgtaatttt taaggaagcc aaatttgagg gcagtttgtc 3840
acagttgatt tccttctctt tcccttcgtc atgtgacctg atatcggggg ttagttcgtc 3900
atcattgatg agggttgatt atcacagttt attactctga attggctatc cgcgtgtgta 3960
cctctacctg gagtttttcc cacggtggat atttcttctt gcgctgagcg taagagctat 4020
ctgacagaac agttcttctt tgcttcctcg ccagttcgct cgctatgctc ggttacacgg 4080
ctgcggcgag cgctagtgat aataagtgac tgaggtatgt gctcttctta tctccttttg 4140
tagtgttgct cttattttaa acaactttgc ggttttttga tgactttgcg attttgttgt 4200
tgctttgcag taaattgcaa gatttaataa aaaaacgcaa agcaatgatt aaaggatgtt 4260
cagaatgaaa ctcatggaaa cacttaacca gtgcataaac gctggtcatg aaatgacgaa 4320
ggctatcgcc attgcacagt ttaatgatga cagcccggaa gcgaggaaaa taacccggcg 4380
ctggagaata ggtgaagcag cggatttagt tggggtttct tctcaggcta tcagagatgc 4440
cgagaaagca gggcgactac cgcacccgga tatggaaatt cgaggacggg ttgagcaacg 4500
tgttggttat acaattgaac aaattaatca tatgcgtgat gtgtttggta cgcgattgcg 4560
acgtgctgaa gacgtatttc caccggtgat cggggttgct gcccataaag gtggcgttta 4620
caaaacctca gtttctgttc atcttgctca ggatctggct ctgaaggggc tacgtgtttt 4680
gctcgtggaa ggtaacgacc cccagggaac agcctcaatg tatcacggat gggtaccaga 4740
tcttcatatt catgcagaag acactctcct gcctttctat cttggggaaa aggacgatgt 4800
cacttatgca ataaagccca cttgctggcc ggggcttgac attattcctt cctgtctggc 4860
tctgcaccgt attgaaactg agttaatggg caaatttgat gaaggtaaac tgcccaccga 4920

tccacacctg atgctccgac tggccattga aactgttgct catgactatg atgtcatagt 4980
tattgacagc gcgcctaacc tgggtatcgg cacgattaat gtcgtatgtg ctgctgatgt 5040
gctgattgtt cccacgcctg ctgagttgtt tgactacacc tccgcactgc agtttttcga 5100
tatgcttcgt gatctgctca agaacgttga tcttaaaggg ttcgagcctg atgtacgtat 5160
tttgcttacc aaatacagca atagtaatgg ctctcagtcc ccgtggatgg aggagcaaat 5220
tcgggatgcc tggggaagca tggttctaaa aaatgttgta cgtgaaacgg atgaagttgg 5280
taaaggtcag atccggatga gaactgtttt tgaacaggcc attgatcaac gctcttcaac 5340
tggtgcctgg agaaatgctc tttctatttg ggaacctgtc tgcaatgaaa ttttcgatcg 5400
tctgattaaa ccacgctggg agattagata atgaagcgtg cgcctgttat tccaaaacat 5460
acgctcaata ctcaaccggt tgaagatact tcgttatcga caccagctgc cccgatggtg 5520
gattcgttaa ttgcgcgcgt aggagtaatg gctcgcggta atgccattac tttgcctgta 5580
tgtggtcggg atgtgaagtt tactcttgaa gtgctccggg gtgatagtgt tgagaagacc 5640
tctcgggtat ggtcaggtaa tgaacgtgac caggagctgc ttactgagga cgcactggat 5700
gatctcatcc cttcttttct actgactggt caacagacac cggcgttcgg tcgaagagta 5760
tctggtgtca tagaaattgc cgatgggagt cgccgtcgta aagctgctgc acttaccgaa 5820
agtgattatc gtgttctggt tggcgagctg gatgatgagc agatggctgc attatccaga 5880
ttgggtaacg attatcgccc aacaagtgct tatgaacgtg gtcagcgtta tgcaagccga 5940
ttgcagaatg aatttgctgg aaatatttct gcgctggctg atgcggaaaa tatttcacgt 6000
aagattatta cccgctgtat caacaccgcc aaattgccta aatcagttgt tgctcttttt 6060
tctcaccccg gtgaactatc tgcccggtca ggtgatgcac ttcaaaaagc ctttacagat 6120
aaagaggaat tacttaagca gcaggcatct aaccttcatg agcagaaaaa agctggggtg 6180
atatttgaag ctgaagaagt tatcactctt ttaacttctg tgcttaaaac gtcatctgca 6240
tcaagaacta gtttaagctc acgacatcag tttgctcctg gagcgacagt attgtataag 6300
ggcgataaaa tggtgcttaa cctggacagg tctcgtgttc caactgagtg tatagagaaa 6360
attgaggcca ttcttaagga acttgaaaag ccagcaccct gatgcgacca cgttttagtc 6420
tacgtttatc tgtctttact taatgtcctt tgttacaggc cagaaagcat aactggcctg 6480
aatattctct ctgggcccac tgttccactt gtatcgtcgg tctgataatc agactgggac 6540
cacggtccca ctcgtatcgt cggtctgatt attagtctgg gaccacggtc ccactcgtat 6600
cgtcggtctg attattagtc tgggaccacg gtcccactcg tatcgtcggt ctgataatca 6660
gactgggacc acggtcccac tcgtatcgtc ggtctgatta ttagtctggg accatggtcc 6720
cactcgtatc gtcggtctga ttattagtct gggaccacgg tcccactcgt atcgtcggtc 6780
tgattattag tctggaacca cggtcccact cgtatcgtcg gtctgattat tagtctggga 6840
ccacggtccc actcgtatcg tcggtctgat tattagtctg ggaccacgat cccactcgtg 6900
ttgtcggtct gattatcggt ctgggaccac ggtcccactt gtattgtcga tcagactatc 6960

agcgtgagac tacgattcca tcaatgcctg tcaagggcaa gtattgacat gtcgtcgtaa 7020
cctgtagaac ggagtaacct cggtgtgcgg ttgtatgcct gctgtggatt gctgctgtgt 7080
cctgcttatc cacaacattt tgcgcacggt tatgtggaca aaatacctgg ttacccaggc 7140
cgtgccggca cgttaaccgg gctgcatccg atgcaagtgt gtcgctgtcg agtttaaaca 7200
tgcatagtta ttaatagtaa tcaattacgg ggtcattagt tcatagccca tatatggagt 7260
tccgcgttac ataacttacg gtaaatggcc cgcctggctg accgcccaac gacccccgcc 7320
cattgacgtc aataatgacg tatgttccca tagtaacgcc aatagggact ttccattgac 7380
gtcaatgggt ggagtattta cggtaaactg cccacttggc agtacatcaa gtgtatcata 7440
tgccaagtac gccccctatt gacgtcaatg acggtaaatg gcccgcctgg cattatgccc 7500
agtacatgac cttatgggac tttcctactt ggcagtacat ctacgtatta gtcatcgcta 7560
ttaccatggt gatgcggttt tggcagtaca tcaatgggcg tggatagcgg tttgactcac 7620
ggggatttcc aagtctccac cccattgacg tcaatgggag tttgttttgg caccaaaatc 7680
aacgggactt tccaaaatgt cgtaacaact ccgccccatt gacgcaaatg ggcggtaggc 7740
gtgtacggtg ggaggtctat ataagcagag ctggtttagt gaaccgtcag atccgctagc 7800
gctaccggtc gccaccatgg tgagcaaggg cgaggagctg ttcaccgggg tggtgcccat 7860
cctggtcgag ctggacggcg acgtaaacgg ccacaagttc agcgtgtccg gcgagggcga 7920
gggcgatgcc acctacggca agctgaccct gaagttcatc tgcaccaccg gcaagctgcc 7980
cgtgccctgg cccaccctcg tgaccaccct gacctacggc gtgcagtgct tcagccgcta 8040
ccccgaccac atgaagcagc acgacttctt caagtccgcc atgcccgaag gctacgtcca 8100
ggagcgcacc atcttcttca aggacgacgg caactacaag acccgcgccg aggtgaagtt 8160
cgagggcgac accctggtga accgcatcga gctgaagggc atcgacttca aggaggacgg 8220
caacatcctg gggcacaagc tggagtacaa ctacaacagc cacaacgtct atatcatggc 8280
cgacaagcag aagaacggca tcaaggtgaa cttcaagatc cgccacaaca tcgaggacgg 8340
cagcgtgcag ctcgccgacc actaccagca gaacaccccc atcggcgacg gccccgtgct 8400
gctgcccgac aaccactacc tgagcaccca gtccgccctg agcaaagacc ccaacgagaa 8460
gcgcgatcac atggtcctgc tggagttcgt gaccgccgcc gggatcactc tcggcatgga 8520
cgagctgtac aagtccggac tcagatccac cggatctaga taactgatca taatcagcca 8580
taccacattt gtagaggttt tacttgcttt aaaaaacctc ccacacctcc ccctgaacct 8640
gaaacataaa atgaatgcaa ttgttgttgt taacttgttt attgcagctt ataatggtta 8700
caaataaagc aatagcatca caaatttcac aaataaagca tttttttcac tgcattctag 8760
ttgtggtttg tccaaactca tcaatgtatc ttaaatggcc gcataacttc gtatagcata 8820
cattatacga agttatctag cagatctgaa ttcgatatca agctgcggcc ttaattaa 8878

<210> 8
<211> 124884

<212>  DNA
<213>  Varicella zoster


<220>
<221>  CDS
<222>  (1134)..(1850)

<220>
<221>  CDS
<222>  (8607)..(9386)

<220>
<221>  CDS
<222>  (10642)..(10902)

<220>
<221>  CDS
<222>  (11009)..(11917)

<220>
<221>  CDS
<222>  (12160)..(13392)

<220>
<221>  CDS
<222>  (13590)..(16049)

<220>
<221>  CDS
<222>  (16214)..(18199)

<220>
<221>  CDS
<222>  (18441)..(19346)

<220>
<221>  CDS
<222>  (24149)..(25516)

<220>
<221>  CDS
<222>  (30759)..(33875)

<220>
<221>  CDS
<222>  (34083)..(42374)

<220>
<221>  CDS
<222>  (44506)..(46263)

<220>
<221>  CDS
<222>  (50857)..(54471)

<220>
<221>  CDS
<222>  (54651)..(56963)

<220>
<221>  CDS
<222>  (57008)..(59614)

<220>
<221>  CDS
<222>  (59766)..(60197)

<220>
<221>  CDS
<222>  (64807)..(65832)

<220>
<221>  CDS
<222>  (66074)..(68599)

<220>
<221>  CDS
<222>  (70633)..(71355)

<220>
<221>  CDS
<222>  (71540)..(75730)

<220>
<221>  CDS
<222>  (75847)..(76797)

<220>
<221>  CDS
<222>  (78170)..(80200)

<220>
<221>  CDS
<222>  (80360)..(81451)

<220>
<221>  CDS
<222>  (82719)..(83318)

<220>
<221>  CDS
<222>  (84667)..(86322)

<220>
<221>  CDS
<222>  (87881)..(90388)

<220>
<221>  CDS
<222>  (90493)..(92808)

<220>
<221>  CDS
<222>  (95996)..(98641)

<220>
<221>  CDS
<222>  (110581)..(111417)

<220>
<221>  CDS
<222>  (111565)..(112107)

<220>
<221>  CDS
<222>  (113037)..(114218)

<220>
<221>  CDS
<222>  (114496)..(115560)

<220>
<221>  CDS
<222>  (115808)..(117679)

<220>
<221>  CDS
<222>  (120764)..(124696)

<400>  8
aggccagccc tctcgcggcc ccctcgagag agaaaaaaaa aagcgacccc acctccccgc      60

gcgtttgcgg ggcgaccatc gggggggatg ggattttttg ccgggaaacc cccccccgcc     120

agcctttaac aaaacccgcg ccttttgcgt ccacccctcg tttactgctc ggatggcgac     180

cgtgcactac tcccgccgac ctgggacccc gccggtcacc ctcacgtcgt cccccagcat     240

ggatgacgtt gcgaccccca tcccctacct acccacatac gccgaggccg tggcagacgc     300

gcccccccct tacagaagcc gcgagagtct ggtgttctcc ccgcctcttt ttcctcacgt     360

ggagaatggc accacccaac agtcttacga ttgcctagac tgcgcttatg atggaatcca     420

cagacttcag ctggcttttc taagaattcg caaatgctgt gtaccggctt ttttaattct     480

ttttggtatt ctcaccctta ctgctgtcgt ggtcgccatt gttgccgttt ttcccgagga     540

acctcccaac tcaactacat gaaactactg tccggaaggg gaaggtattt attctcgctt     600

gcagcttgtc gcgcgtgtat gcacaacaaa agctatatat gtcaccaaag ccaacgtcgc     660

catctggagt actacaccca gtacgttgca taacctgtcc atttgcattt tcagttgcgc     720

ggacgccttt ctccgggatc gtggccttgg gacatcaacc agtggaataa gaaccgccgg     780

tggtcttgtt tgaacgacga gtggcgacgc gttgttctgc ataagctctg tatgctgata     840

cataaacaca gagtctgtat cgctatcaga ttcccgaaca ccttccggta ccccatactc     900

cgataccctg gacattgcgg atcccaaaaa tataatatta acaggatttg cttatacttt     960

gctacagctt atataaattt atgtgcgata catcttaagt gcatccgtac gttatttata    1020

cattgcctgt cacgtgaaaa gactgtgtta cccaataaag gttctacaaa aaatgcttta    1080

ttgggtgttt gtttaatagc tattatcgta acccaccccc gtaaaatcat aaa atg       1136
                                                           Met
                                                           1

cat gta att tct gag aca ctt gca tat ggg cat gtt ccc gca ttt att      1184
His Val Ile Ser Glu Thr Leu Ala Tyr Gly His Val Pro Ala Phe Ile
            5                   10                  15

atg ggc tcc act ctg gtg cgt ccc agt tta aac gcc acc gcc gag gaa      1232
Met Gly Ser Thr Leu Val Arg Pro Ser Leu Asn Ala Thr Ala Glu Glu
        20                  25                  30

aat ccc gcg tca gaa acg cga tgt tta tta cga gtg ctt gcg ggg aga      1280
Asn Pro Ala Ser Glu Thr Arg Cys Leu Leu Arg Val Leu Ala Gly Arg
    35                  40                  45

act gta gac ctg cca ggc gga gga acg tta cac att acc tgt acc aaa      1328
Thr Val Asp Leu Pro Gly Gly Gly Thr Leu His Ile Thr Cys Thr Lys
50                  55                  60                  65

acc tat gta att att ggc aaa tat agc aaa ccc ggc gaa cgt ctt agc      1376
Thr Tyr Val Ile Ile Gly Lys Tyr Ser Lys Pro Gly Glu Arg Leu Ser
                70                  75                  80

ctt gcc cgt cta ata ggg cgt gca atg acg cct gga ggt gca agg aca      1424
Leu Ala Arg Leu Ile Gly Arg Ala Met Thr Pro Gly Gly Ala Arg Thr
            85                  90                  95

ttt att att ttg gcg atg aag gaa aag cga tcc aca acg ctt ggg tat 1472
Phe Ile Ile Leu Ala Met Lys Glu Lys Arg Ser Thr Thr Leu Gly Tyr
        100                 105                 110

gaa tgt ggt acg ggc ttg cat tta ctg gct cca tct atg ggt aca ttt 1520
Glu Cys Gly Thr Gly Leu His Leu Leu Ala Pro Ser Met Gly Thr Phe
    115                 120                 125

ctc cgc aca cac ggt tta agt aac aga gat ctc tgt tta tgg cgg ggt 1568
Leu Arg Thr His Gly Leu Ser Asn Arg Asp Leu Cys Leu Trp Arg Gly
130                 135                 140                 145

aat att tat gat atg cat atg caa cgt ctt atg ttt tgg gag aat atc 1616
Asn Ile Tyr Asp Met His Met Gln Arg Leu Met Phe Trp Glu Asn Ile
                150                 155                 160

gcg caa aat acc act gaa aca cct tgt ata acg tcg acg tta aca tgc 1664
Ala Gln Asn Thr Thr Glu Thr Pro Cys Ile Thr Ser Thr Leu Thr Cys
            165                 170                 175

aac ttg aca gaa gac tct ggt gaa gcc gca ctt acc acg tca gac cga 1712
Asn Leu Thr Glu Asp Ser Gly Glu Ala Ala Leu Thr Thr Ser Asp Arg
        180                 185                 190

ccc act ctc cca acc cta aca gcc caa gga aga cca aca gtt tcc aac 1760
Pro Thr Leu Pro Thr Leu Thr Ala Gln Gly Arg Pro Thr Val Ser Asn
    195                 200                 205

att cgt gga ata ttg aaa gga tcc ccc cgt caa cag ccg gtc tgt cac 1808
Ile Arg Gly Ile Leu Lys Gly Ser Pro Arg Gln Gln Pro Val Cys His
210                 215                 220                 225

cgg gtt aga ttt gcc gaa cct acg gag ggc gta ttg atg taa 1850
Arg Val Arg Phe Ala Glu Pro Thr Glu Gly Val Leu Met
                230                 235

tcactaaata aaatacacct tttttcgatt gtacgtattt ttatttaaat gtgtagttca 1910

tagtccgccg acagccgctc gggcttttcc cccacataca acatgatcgt atgcctcgga 1970

tgcaccggtc caacactccg ccgagaaggg ggatttacaa tgacagtgat acccaatagc 2030

cgccagatgt acacccagct gtccggactc cagcatcatc tgctgagttg cggcgctgaa 2090

gggtgcatcg catagggtgt tataattagc catttccggt aacagtcgtt gggaatttag 2150

gaggctgcaa aacggctgta ggtcaacata cattggggat tcagatggtt tatctcgacg 2210

tccaagtcca atcaaaaaag cgtgtaaatc atcagcccgg ccgcatgttg ctcgaagagc 2270

acataacctc ttaacaccgt acagagggga tggcgtcggt gcatgtgagt tggcagggca 2330

tgtccacgtt gtttccaacg ccagtggcgg tataacttgt gtaaacgacg ccaacgggtc 2390

aggtttaaga ttcactcgga tgggttgact gctttcggaa gctcccgttg tatccattaa 2450

ttaaacgttc ggtacacgtc tggtgtgtgt tttacccgaa tcagagacgg aattgcaaag 2510

atattggttt gaaagcaatg taatcccgcc catatatccc caacgtcgcc ttaaaaactc 2570

ccacaatatt acatttttat tagtctttta ttaatataga atcacataaa caattgataa 2630

aatcaagggg tggtgtataa tgattaaaaa tataaattga tatgttttac aagcatgaaa 2690

taggtattta ctattctaac aggtaaatat gcttaatgat taaaaataca aattagtatg 2750

ttttgacaag catgaaaaag gtatttttta ttttagcagt taaaggtact acacttaaaa 2810

tatttaccgt atggacgggc gtcagaaaga tgcccggccc aagttgagag ggtacattca 2870
acacgaccac actcgcgttg gtgggtgatt agggcctcta aaacaccggc cagacatgac 2930
ccgggtgtat attcttgtaa cacttgaacg ttacaactga tatcatcata ttccacaaat 2990
ttagagccac ggacaactat attagcaatg cgggcaatca taacaaacat ataagtagta 3050
atacacgtga tatcactaaa acgttgctgg cgcaacagtt cggggagagt acgagacccc 3110
aaatcgttgt ccctgtttag aagaagacat cttacaaaag gccccagctt taactttaaa 3170
ttctccaaaa gtgacttcga ggttgcaaca atgggattat ttgtgtagat gggcaagttt 3230
tttgccgcta acattttaat ccacgttaac agttcatccg cagactccaa cgcttcaatc 3290
aaagattctc cacgtatgac tctctcacgc aacgcgcggg caatacgtga gtccatttta 3350
tatgactcaa aggtacgata aagttcatgt ccgtacaaca tcaactccgg ccaagatgtg 3410
ttttgtttta tccccggaaa acatccaccg gaagcccatg aatcaccctc ttgtattgtg 3470
gcatatcgga ctaccagttt ttcaattgtt tcatctaaat ggcgtaccga gtcaatggtc 3530
acgctggctc ccgcggtgga gacgacttca atagcacggc ccgtaattcg atcgaccggg 3590
atatcatact cttttcgaat acgctctcgg cgggcgtctc tcttggaaaa tcgcaacctg 3650
tacgattcgt catgtgtctg atcatttctt tctcccgtgg tcattgcagg aggcgttgta 3710
ggacgccgtc ttcgatttga cagggatcga tcacggtgtt ttcttgaact ttgagtgtta 3770
taagatctgg atgatcgtcg atgtccccgt tcgatgcgtg catatccagt ctccacgtct 3830
cttcctccat gatggtttga atcgggtaat acaacaacca aagttttcgg gcgattgtgg 3890
tggtagcttt cacgccttcc gtgccttcgt ttggaatacc gtggattata tgctgtatct 3950
gcagtacgct ccacatacac agttctagac gttgtggagt cctcgcctgg agtggagcca 4010
atagcttcat catttgccca atcggtgact tccaatgcaa agtcatccga aggttcgtct 4070
ggtagcaaat tcataaagtc ttcacaaata gtagacacgt ctgggtcggt tggaattgaa 4130
gcagaggcca tggctgcaaa atatctgaca attgcgtgtt tgcagttgcc tgtatcttcc 4190
gccaatgttg tagaatttat aggctcaccc aaccccgcaa tgggcgtgtt tagtcacatg 4250
attaatgctt ctgggagttt tcactttccc caaacaagct tacctgcacc ctttgttcgt 4310
aatgcataaa aataaccact gctatagcaa atatgacgat ataaaaacat tttatagcaa 4370
ggccggacat tactgtagcg caacatgttg tgcatatacc acgtattccc cccgtattga 4430
tatgatttaa atgattatcc ttggttggtt ttggtctaac ataagatata agctctacta 4490
tagcgagcgt gcatacaaca acccaggcca gaatccgaat gtatgtgggg tataataacg 4550
cgcatggtgt atatgcaacg ccaagcgtta aaagcacaat acatccagat gatatatgag 4610
cgataacctc caaaagcatc aataacgtaa cacctttatg catatataaa aaacttatag 4670
ggtcagcatt aaatacttta ctcataccat cccgtcgcat ggaaacatca cataacaacc 4730
ttgccaactt tgtatatggg taaccaagaa gaatgttcga aataacccgt gttacgtaat 4790
tcagtgaata tgatgtgggg gatattaact cacaggatga tcggaatggc ccaaacatac 4850

gacgtattcg tcgaaattgt aaatacatac catatacaaa ccatgcaaaa aaaatcattt 4910
ttagctgcac gcaccaaaaa taagcgtgac aattacgtgt tcccagaaca attcgaattt 4970
tgtcatgcaa aggtgtagaa atagcggttt ttaccatagt atctcctgat aatagatttt 5030
cccggcagct gtaatcgtat ccagataggc catccaaaaa cgttgagtgg tttacaaacg 5090
ttacatatat aagagagttg ttataagacc cccatacaac cggtccacca ttaatcaccg 5150
tggttgcata cacacactca tgttcaaact ttacacgagc ggtataccat agggtaaaaa 5210
cagcatgtcc gctaagtaga cacataatta taaaatgttc tgtcttgatt cctaaagcct 5270
gcatgacccg tggaagatgg caattcaagc acgatgtagt atcacacggt tggtgttaac 5330
tcgaagttaa atttggataa ttaggtactt ctagagtaaa gattgtatgc atgcgattgc 5390
tatcgcactt tgtagcaaaa cattgttgtg caagcgaaat acacaaacgg ttgtgatgat 5450
ccactcgcag agacacaaat gtccggggag ccgttcttcc tccgcgatgg ggatatcgaa 5510
gacaagtgaa cccttttgtt ccgcatatga gctgaaataa cacccagtcc cttttgatgg 5570
cgatacactt tgatgatgtt aaggtatatt cgcgatcacg cccggggaaa tgaacagcaa 5630
tatgctccac aatagattct aatattgtgc tgtcgacaaa ggcctccagt gtaaatgcgt 5690
ccagacaagt taccccgcgc tcttttagag cctttgttaa agatatttgc ggggagctaa 5750
atatttgttt attacgcgca accttacgtt caaaaaactc tgcgtattcc cccccaaggt 5810
tatgtaaaat aaattgcact ggaacattcg actgcggtct tgaatgaaaa tgaaagtttg 5870
ccgggtttct atgtgatgtc acaaacgcta atatatcaat acactgctca ggtacaacat 5930
aaaatgggag tagttgtcca accgccgtcc ctgtggttgt tactttggag aaaaaaggca 5990
gtcttaaact atgtccgtgg ctataaacac cagtatctat aaacgaaaag tcccgtaaat 6050
acggaccaat atattcaaca aattcccgtt ccagcaacac cgcttgctgt aatatttgtg 6110
caaacccctt taaagtggaa gaccccacta acgcataggg atttgggatt ggtacgcata 6170
ccctgaaacc tattttctct ttacagttac agggtagagt ttcatgcaag ttttcattgt 6230
ttgatacatc ggcgtgtgta tggacttcag acgttgtctg tgtatcaaaa aaccatacat 6290
cctctgtata attctcttct acacacgtgt ataattcgcc attttctatg taaaaatcga 6350
tgtcagaatg gctggttata tccaataaat tatcatcatc caacacctca acggtaggtt 6410
caggacatgc agttttataa aaataacatg ggtctttgtt agggtttacc acggcctttg 6470
gaaaaagtaa ttgcatggcc gttaaaatac catgacgaaa tgctcgcatg ccggcatgta 6530
aaatacccaa tgggatgggt tttcttatat gaaagtctac atcaagtatg aggtttgtga 6590
ttataagatt tgtattaaat agctcattcc tgtttatata aagctgatct ttgggtatgt 6650
ttgatgaaat tttagaaacg tttttaacag acgtagataa tagtaaagtc aactgcatat 6710
ctcgtagtga agcggcaaca aaattacatg gattaatttg tttaaggtcc tccgcaatta 6770
atcgagcctc gtgcggtaaa gtgtaacggt ttgttattga tgaccacgta tcattagcaa 6830
taacagcaaa tgcttgggcg ccgtgaggca aggctacccg atatacaggc attggtccag 6890

ttacctcaga atggccgatg agggcttcta atggagtttt ataactcagg atggatacat 6950

catgtgtggc tatcccagtg gcagcagaga aaaacagtaa tagttttgta atccccgggc 7010

tcgtatcaaa accagtacga ccactttggt taggtgtatc gtttgcaaag ttggctgctc 7070

gtaacgcctc cgcggaaaca cccgaatcct caaaattaga caattcgtca aaaccgggtg 7130

gatttgaggg aatagtggag gaccatccat atggactaaa ttgtttttca atgttttcca 7190

cacgacgagt tagcgttgta gctaggtcac atacgcctat aaacttgcta ggttttgcgg 7250

catacgtaag acttaaagta tatgttttag taattgtata tttatgtcca atctcaggtc 7310

caagttcagt gacatcacaa attacgttct tttttatata gtcacgcatg ttgagacgag 7370

aacgtacatg attaaaaaaa ttagcagtag ctctttttcc caggttggat gattttaaga 7430

ggaccggttt attcacaaaa tctgagtatg taaccgcttg taggtggtct gcgatctgtt 7490

tccgattgaa acattcaaaa tgtgccagat aaatataatc aacaaattca cggtctggaa 7550

ctttaaggcc ttttctatcg ttggtaatat actccgatac tgcgtgtatt tccgttgtgt 7610

ctgtatgtat tcgctgtaaa atgtacgata gagcattttt ggctgtcaaa cctcgtgtat 7670

atgttgagga acaacaaaac atggaaagtt tatcaaaaga caacaagtcc gaaatattgt 7730

acccactaca attaggtaat gccgggactt ggtaagttaa aaacaaatct ttaattgcct 7790

gtaagtcata taagggggtt tccaacgtat tgtaacttgt gtccgtttgt aacaagtaat 7850

agcgtgtagc caacactagc gttttttcag agggtccaaa tcgaacaata taccaaaacg 7910

gcgagcatcc atacccccag tagagtcgtc gatatgcagc caatacttga cgttcgtaat 7970

gggcatataa tgatgttagc tcctgacgac caacggattt tttaactaac ttgcagagtg 8030

ttgcctctgt gatgcatagg ccgttgtccg ataatccctt tcggtttaaa tggtgtgttg 8090

ttaccatcag agtttgtata acttccgagt gaatgtcaaa cgtctccgat atacataggg 8150

tatcagatat tatatgcgga tttaggggtg ctccatacca taacgcctta tataaagctt 8210

taaaatcagt ttgggtttta aaacaacaaa aaaatatagg ccagacccgg gatcgtacat 8270

ctccagttga aaatccacca attaaataaa aaataacgtt gacgtcccta ctacaaaata 8330

aatgcattat ttggttttct tcatcgtttt cagttacttc acgtgggcgt ttagttggga 8390

ttacttgcgt gatctcttcc ctcccatttt tgacaaagac gtcatctaag tcgggagtcc 8450

aagtataact caccacatac agaggttctg tgcttatctg cccggtaagc aacaacagcg 8510

agtgggagat tgcacatccc tttgtggcaa ataataaccg aatcgtcggt ttggaggatt 8570

tatccatagt tcaatacgtt ggaaagccag tcaatc atg cag acg gtg tgt gcc 8624
Met Gln Thr Val Cys Ala
    240

agc tta tgt gga tat gct cga ata cca act gaa gag cca tct tat gaa 8672
Ser Leu Cys Gly Tyr Ala Arg Ile Pro Thr Glu Glu Pro Ser Tyr Glu
245                 250                 255                 260

gag gtg cgt gta aac acg cac ccc caa gga gcc gcc ctg ctc cgc ctc 8720
Glu Val Arg Val Asn Thr His Pro Gln Gly Ala Ala Leu Leu Arg Leu
                265                 270                 275

caa gag gct tta acc gct gtg aat gga tta ttg cct gca cct cta acg 8768
Gln Glu Ala Leu Thr Ala Val Asn Gly Leu Leu Pro Ala Pro Leu Thr
            280                 285                 290

tta gaa gac gta gtc gct tct gca gat aat acc cgt cgt ttg gtc cgc 8816
Leu Glu Asp Val Val Ala Ser Ala Asp Asn Thr Arg Arg Leu Val Arg
        295                 300                 305

gcc cag gct ttg gcg cga act tac gct gca tgt tct cgt aac att gaa 8864
Ala Gln Ala Leu Ala Arg Thr Tyr Ala Ala Cys Ser Arg Asn Ile Glu
    310                 315                 320

tgt tta aaa cag cac cat ttt act gaa gat aac ccc ggt ctt aac gcc 8912
Cys Leu Lys Gln His His Phe Thr Glu Asp Asn Pro Gly Leu Asn Ala
325                 330                 335                 340

gtg gtc cgt tca cac atg gaa aac tca aaa cgg ctt gct gat atg tgt 8960
Val Val Arg Ser His Met Glu Asn Ser Lys Arg Leu Ala Asp Met Cys
                345                 350                 355

tta gct gca att acc cat ttg tat tta tcg gtt ggc gcg gtg gat gtt 9008
Leu Ala Ala Ile Thr His Leu Tyr Leu Ser Val Gly Ala Val Asp Val
            360                 365                 370

act acg gat gat att gtc gat caa acc ctg aga atg acc gct gaa agt 9056
Thr Thr Asp Asp Ile Val Asp Gln Thr Leu Arg Met Thr Ala Glu Ser
        375                 380                 385

gaa gtg gtc atg tct gat gtt gtt ctt ttg gag aaa act ctt ggg gtc 9104
Glu Val Val Met Ser Asp Val Val Leu Leu Glu Lys Thr Leu Gly Val
    390                 395                 400

gtt gct aaa cct cag gca tcg ttt gat gtt tcc cac aac cat gaa tta 9152
Val Ala Lys Pro Gln Ala Ser Phe Asp Val Ser His Asn His Glu Leu
405                 410                 415                 420

tct ata gct aaa ggg gaa aat gtg ggt tta aaa aca tca cct att aaa 9200
Ser Ile Ala Lys Gly Glu Asn Val Gly Leu Lys Thr Ser Pro Ile Lys
                425                 430                 435

tcg gag gcg aca caa tta tct gaa att aaa ccc cca ctt ata gaa gta 9248
Ser Glu Ala Thr Gln Leu Ser Glu Ile Lys Pro Pro Leu Ile Glu Val
            440                 445                 450

tcg gat aat aac aca tct aac cta aca aaa aaa acg tat ccg aca gaa 9296
Ser Asp Asn Asn Thr Ser Asn Leu Thr Lys Lys Thr Tyr Pro Thr Glu
        455                 460                 465

act ctt cag ccc gtg ttg acc cca aaa cag acg caa gat gta caa cgc 9344
Thr Leu Gln Pro Val Leu Thr Pro Lys Gln Thr Gln Asp Val Gln Arg
    470                 475                 480

aca acc ccc gcg atc aag aaa tcc cat gtt atg ctt gta taa 9386
Thr Thr Pro Ala Ile Lys Lys Ser His Val Met Leu Val
485                 490                 495

atattgaaat aaaaactaaa aacgtttctg gtgtatgttt ttattttgta tataaaatta 9446

aaacattgct ggctggcgtg gttattacat ttaatgtttt agtagaaaat cgacatcgtt 9506

tgtttcttta tcagttgaac caaatccacg cgttccccgt tcgctgggtg tggctattag 9566

atctaacgtt ttagtaaaat accattgtac acccggtatg ccacatttac cgcggatagc 9626

ataaggaaat gcaatattac ttaaaacgtt gtgttttaag tgtatttggg tgttgtgatc 9686

tattaacagg acctgtgcaa gacgatctcc cgtttttata cgtatgtcat cacccgtgag 9746

attatatacg tagaatttac agtgttctcc tgcaggccat gccgttggac acacgataat 9806

gcctgatcgg cttttcgatg atcttccaaa aatataagcg tttatactcg gatgttgtaa 9866

gtcccagtct cttataatcg gtaagacaat ttttataaat tcattccttt ttaaatatag 9926

gttatatggt acacaaatat catatcccgc gtcttcttgg cgttttggat tgatgatatg 9986

tttgtaggtt aagggaacat cgatatggta ttctgcagaa tccctatgta aaggttgccc 10046

ctgctgtacc gtggaaatat cagcaaattc aggtataacg ggtttttcat aatttgacgg 10106

cgagtttgat aagggttgaa cttgtatcga tttaaaaatt ggatccagat gtttaagaac 10166

gttttttggg agaaggcgac tttgtcttaa ttttaccggg aacaagtaga ttgttaaatg 10226

tccgggtaaa ataacggtta ctcctggccg gtaatacaaa agggctgaaa ttactcctct 10286

gtaacccgca tcaataactc cgttggcgac aaaaaaattg tcttcatcag caagggcagt 10346

atctttgcat tgaattaaca acagtgcgta ttcattggga ggcgccgact taaccaacag 10406

ctccaactgc tgcatataaa aaccgccccg tgttacagat ttttcagatg gcagttcgag 10466

tttcttgtgg ttccggagta acaacggttg atgtcgactt actttatcgt ctaacacgca 10526

ttgcagcgta tctgcacatt caggttgaac ttctattaaa attgtatctt ttaaacaccg 10586

attcggaata gtttggctac aaaacatatc acctgtattt actgccgttt ccaag atg 10644
Met

gga tca att acc gct tcg ttc ata tta ata acg atg caa att tta ttt 10692
Gly Ser Ile Thr Ala Ser Phe Ile Leu Ile Thr Met Gln Ile Leu Phe
    500                 505                 510

ttt tgt gaa gac agc agt ggg gag cca aac ttt gca gaa cgg aat ttt 10740
Phe Cys Glu Asp Ser Ser Gly Glu Pro Asn Phe Ala Glu Arg Asn Phe
515                 520                 525                 530

tgg cat gcc agc tgt tcg gct cgt gga gtt tat atc gac gga tca atg 10788
Trp His Ala Ser Cys Ser Ala Arg Gly Val Tyr Ile Asp Gly Ser Met
                535                 540                 545

atc acc acc ctt ttc ttc tac gca tcc ctt ttg ggg gtg tgt gta gcc 10836
Ile Thr Thr Leu Phe Phe Tyr Ala Ser Leu Leu Gly Val Cys Val Ala
            550                 555                 560

ctt att tcg tta gct tat cat gcg tgt ttc cgg tta ttt act cgt tct 10884
Leu Ile Ser Leu Ala Tyr His Ala Cys Phe Arg Leu Phe Thr Arg Ser
        565                 570                 575

gta tta cgc agc acg tgg taaacccgtt tgcctataaa aggggcaggc 10932
Val Leu Arg Ser Thr Trp
    580

gtgtataaga gggcccctgt ttaatacgcg gtctgccgtg tttggatatt tcacgaccct 10992

atcgtttatt tacgta atg gca tct tcc gac ggt gac aga ctt tgt cgc tct 11044
Met Ala Ser Ser Asp Gly Asp Arg Leu Cys Arg Ser
585                 590                 595

aat gca gtg cgt cgt aaa aca acg cct agt tat tcc gga caa tat cga 11092
Asn Ala Val Arg Arg Lys Thr Thr Pro Ser Tyr Ser Gly Gln Tyr Arg
            600                 605                 610

acc gcg cgg cga agt gtg gtc gta gga ccc ccc gat gat tca gac gac 11140

Thr Ala Arg Arg Ser Val Val Val Gly Pro Pro Asp Asp Ser Asp Asp
        615                 620                 625

tcg ttg ggt tac att acc aca gtt ggg gcc gat tct cct tct cca gtg     11188
Ser Leu Gly Tyr Ile Thr Thr Val Gly Ala Asp Ser Pro Ser Pro Val
    630                 635                 640

tac gcg gat ctt tat ttt gaa cat aaa aat acg acc cct cgc gta cat     11236
Tyr Ala Asp Leu Tyr Phe Glu His Lys Asn Thr Thr Pro Arg Val His
645                 650                 655                 660

caa cca aac gac tcc agc gga tcg gaa gat gac ttt gaa gac atc gat     11284
Gln Pro Asn Asp Ser Ser Gly Ser Glu Asp Asp Phe Glu Asp Ile Asp
                665                 670                 675

gaa gta gtg gcc gcc ttt cgg gag gcc cgt ttg aga cat gaa ctg gtt     11332
Glu Val Val Ala Ala Phe Arg Glu Ala Arg Leu Arg His Glu Leu Val
            680                 685                 690

gaa gat gct gta tat gaa aac ccg cta agt gta gaa aaa cca tct aga     11380
Glu Asp Ala Val Tyr Glu Asn Pro Leu Ser Val Glu Lys Pro Ser Arg
        695                 700                 705

tct ttt act aaa aat gcg gcg gtt aaa cct aaa tta gag gat tca ccg     11428
Ser Phe Thr Lys Asn Ala Ala Val Lys Pro Lys Leu Glu Asp Ser Pro
    710                 715                 720

aag cga gct ccc ccg gga gca ggc gca att gcc agc ggg aga cca att     11476
Lys Arg Ala Pro Pro Gly Ala Gly Ala Ile Ala Ser Gly Arg Pro Ile
725                 730                 735                 740

tcc ttc agc act gca cca aaa acc gca aca agc tcg tgg tgc ggt cct     11524
Ser Phe Ser Thr Ala Pro Lys Thr Ala Thr Ser Ser Trp Cys Gly Pro
                745                 750                 755

acg cca tca tat aac aaa cgc gtc ttt tgt gaa gcg gtc cgg cgc gta     11572
Thr Pro Ser Tyr Asn Lys Arg Val Phe Cys Glu Ala Val Arg Arg Val
            760                 765                 770

gcc gcc atg cag gca caa aag gct gcc gaa gcg gct tgg aat agt aat     11620
Ala Ala Met Gln Ala Gln Lys Ala Ala Glu Ala Ala Trp Asn Ser Asn
        775                 780                 785

ccc cca agg aat aac gcc gaa tta gac cgt ttg tta acc gga gcc gtt     11668
Pro Pro Arg Asn Asn Ala Glu Leu Asp Arg Leu Leu Thr Gly Ala Val
    790                 795                 800

att cgt att acg gtg cat gag ggt tta aat tta ata caa gcc gct aat     11716
Ile Arg Ile Thr Val His Glu Gly Leu Asn Leu Ile Gln Ala Ala Asn
805                 810                 815                 820

gaa gca gac cta ggt gaa gga gca tcg gta tcc aaa cgt gga cat aat     11764
Glu Ala Asp Leu Gly Glu Gly Ala Ser Val Ser Lys Arg Gly His Asn
                825                 830                 835

cga aaa act gga gat tta cag ggg ggc atg ggt aat gaa cct atg tac     11812
Arg Lys Thr Gly Asp Leu Gln Gly Gly Met Gly Asn Glu Pro Met Tyr
            840                 845                 850

gca caa gtt cgt aag cca aaa agt cga acg gat aca caa acg act ggg     11860
Ala Gln Val Arg Lys Pro Lys Ser Arg Thr Asp Thr Gln Thr Thr Gly
        855                 860                 865

cgt ata act aat cga agt agg gcc cgt tct gca tca aga act gat acg     11908
Arg Ile Thr Asn Arg Ser Arg Ala Arg Ser Ala Ser Arg Thr Asp Thr
    870                 875                 880

cga aaa tag ggatataatt acgcagtaac ggtttacccg gtattatgta             11957

Arg Lys
885

taataaataa acgtataaaa gacagtcgtg gtttgtgttt attataaatg tgtattatat 12017

gtcacatatt ataaactgtt taaatagtac cacgtggtat tatgaacagt ttataatcag 12077

ttgctaccaa acaaacccca ttagacggcg ggttttgata aagggaatcg cttatttaaa 12137

ctaaagattt tactctataa gt atg gag tgt aat tta gga acc gaa cat cct 12189
Met Glu Cys Asn Leu Gly Thr Glu His Pro
            890                 895

agt aca gat acg tgg aat cgt agt aaa acg gaa caa gcg gtt gtg gac 12237
Ser Thr Asp Thr Trp Asn Arg Ser Lys Thr Glu Gln Ala Val Val Asp
            900                 905                 910

gca ttt gat gaa tcg ttg ttt ggt gat gta gca tcg gat att gga ttt 12285
Ala Phe Asp Glu Ser Leu Phe Gly Asp Val Ala Ser Asp Ile Gly Phe
        915                 920                 925

gaa acg tcg tta tat tca cat gca gtt aaa act gct ccg tct ccg cct 12333
Glu Thr Ser Leu Tyr Ser His Ala Val Lys Thr Ala Pro Ser Pro Pro
    930                 935                 940

tgg gta gct agc cct aaa att tta tat caa cag tta ata cgg gat ctt 12381
Trp Val Ala Ser Pro Lys Ile Leu Tyr Gln Gln Leu Ile Arg Asp Leu
945                 950                 955                 960

gat ttt tca gaa ggg ccg cgt tta cta tca tgt ctt gaa acc tgg aac 12429
Asp Phe Ser Glu Gly Pro Arg Leu Leu Ser Cys Leu Glu Thr Trp Asn
                965                 970                 975

gag gat tta ttc tca tgt ttt cct att aat gag gac cta tat tcc gat 12477
Glu Asp Leu Phe Ser Cys Phe Pro Ile Asn Glu Asp Leu Tyr Ser Asp
            980                 985                 990

atg atg gtt tta tcc ccg gat cca gat gac gtt atc tca acc gtt tca 12525
Met Met Val Leu Ser Pro Asp Pro Asp Asp Val Ile Ser Thr Val Ser
        995                 1000                1005

acc aaa gac cat gtt gaa atg ttt aat tta aca acc cgg ggt tcc 12570
Thr Lys Asp His Val Glu Met Phe Asn Leu Thr Thr Arg Gly Ser
    1010                1015                1020

gtt cga ttg cct agt cca cca aag caa ccg acg ggg ctt cca gct 12615
Val Arg Leu Pro Ser Pro Pro Lys Gln Pro Thr Gly Leu Pro Ala
    1025                1030                1035

tac gtt cag gag gtc cag gat tcg ttt acc gta gaa cta cgc gcc 12660
Tyr Val Gln Glu Val Gln Asp Ser Phe Thr Val Glu Leu Arg Ala
    1040                1045                1050

cgg gaa gaa gca tac aca aaa cta cta gtt act tat tgt aaa tcg 12705
Arg Glu Glu Ala Tyr Thr Lys Leu Leu Val Thr Tyr Cys Lys Ser
    1055                1060                1065

att ata cgt tat ctc caa gga acg gcg aaa agg acg aca ata ggt 12750
Ile Ile Arg Tyr Leu Gln Gly Thr Ala Lys Arg Thr Thr Ile Gly
    1070                1075                1080

ctt aat ata caa aac cct gac cag aaa gct tac acg caa ctc agg 12795
Leu Asn Ile Gln Asn Pro Asp Gln Lys Ala Tyr Thr Gln Leu Arg
    1085                1090                1095

caa agt att cta ctt aga tat tat cgt gag gtg gca agt ttg gcg 12840
Gln Ser Ile Leu Leu Arg Tyr Tyr Arg Glu Val Ala Ser Leu Ala
    1100                1105                1110

cgt ctt ctg tac cta cat tta tat tta acc gta acg cgt gaa ttt 12885
Arg Leu Leu Tyr Leu His Leu Tyr Leu Thr Val Thr Arg Glu Phe
    1115                1120                1125

tcc tgg cgt ttg tac gcc agt caa tct gca cac ccg gac gtg ttt 12930
Ser Trp Arg Leu Tyr Ala Ser Gln Ser Ala His Pro Asp Val Phe
    1130                1135                1140

gcg gct tta aaa ttc acc tgg acc gaa cgt cga cag ttc acg tgt 12975
Ala Ala Leu Lys Phe Thr Trp Thr Glu Arg Arg Gln Phe Thr Cys
    1145                1150                1155

gcg ttt cat cct gta tta tgc aac cac ggc att gtg tta tta gaa 13020
Ala Phe His Pro Val Leu Cys Asn His Gly Ile Val Leu Leu Glu
    1160                1165                1170

ggg aaa cca cta aca gcg tct gcc ttg agg gaa ata aat tac cgc 13065
Gly Lys Pro Leu Thr Ala Ser Ala Leu Arg Glu Ile Asn Tyr Arg
    1175                1180                1185

cgc cga gaa ctg gga ctg cct cta gtt aga tgt ggt ctt gtt gaa 13110
Arg Arg Glu Leu Gly Leu Pro Leu Val Arg Cys Gly Leu Val Glu
    1190                1195                1200

gaa aac aaa tct ccg ttg gtt caa caa ccc tca ttt tcg gtt cat 13155
Glu Asn Lys Ser Pro Leu Val Gln Gln Pro Ser Phe Ser Val His
    1205                1210                1215

tta cca cgg tcg gtg ggt ttt ctt acc cac cac att aag cgt aag 13200
Leu Pro Arg Ser Val Gly Phe Leu Thr His His Ile Lys Arg Lys
    1220                1225                1230

tta gac gca tat gcg gtc aaa cat cct caa gaa ccg aga cat gta 13245
Leu Asp Ala Tyr Ala Val Lys His Pro Gln Glu Pro Arg His Val
    1235                1240                1245

cga gcg gat cat cct tac gca aaa gtt gtt gaa aat aga aac tac 13290
Arg Ala Asp His Pro Tyr Ala Lys Val Val Glu Asn Arg Asn Tyr
    1250                1255                1260

ggt agt agc atc gaa gct atg att tta gca cct ccg tcc cca tcc 13335
Gly Ser Ser Ile Glu Ala Met Ile Leu Ala Pro Pro Ser Pro Ser
    1265                1270                1275

gag atc ctg ccg ggg gac cca cca cgc cca ccc acg tgt ggg ttt 13380
Glu Ile Leu Pro Gly Asp Pro Pro Arg Pro Pro Thr Cys Gly Phe
    1280                1285                1290

tta acg cgt taa acgtcattgg ggtagagggt gtaaataaat tacgaaaacg 13432
Leu Thr Arg
    1295

tgcatgcgtt ttttattttt acaatgcgcc gtatatggta tgtctgtcat gtgctctaaa 13492

gtcccatata taaaagaagc cccaacgagt gtatgcgtat tgcgtaccgc gaccctggga 13552

tgttttacag gcgcgtttgt ttgtctcggt tataagt atg cag tcg ggt cat tat 13607
Met Gln Ser Gly His Tyr
            1300

aac cgg agg caa tcc cgc cga cag cgg ata tcg tct aat acc aca 13652
Asn Arg Arg Gln Ser Arg Arg Gln Arg Ile Ser Ser Asn Thr Thr
        1305                1310                1315

gac tcc ccc cgt cac aca cac gga aca cgt tat cgg tca acc aat 13697
Asp Ser Pro Arg His Thr His Gly Thr Arg Tyr Arg Ser Thr Asn
        1320                1325                1330

tgg tat aca cac cca ccc cag ata ttg tcc aat tca gaa aca tta 13742
Trp Tyr Thr His Pro Pro Gln Ile Leu Ser Asn Ser Glu Thr Leu
        1335                1340                1345

gtt gcg gtt caa gaa cta ctg aac tcc gag atg gat cag gac agc 13787
Val Ala Val Gln Glu Leu Leu Asn Ser Glu Met Asp Gln Asp Ser
        1350                1355                1360

agt tct gac gca tcg gat gat ttt ccg gga tac gcc tta cat cat 13832
Ser Ser Asp Ala Ser Asp Asp Phe Pro Gly Tyr Ala Leu His His
        1365                1370                1375

tct aca tat aat gga tcc gaa caa aat aca tca act tcc aga cat 13877
Ser Thr Tyr Asn Gly Ser Glu Gln Asn Thr Ser Thr Ser Arg His
        1380                1385                1390

gaa aat cgc ata ttt aaa tta acg gag agg gaa gct aat gag gaa 13922
Glu Asn Arg Ile Phe Lys Leu Thr Glu Arg Glu Ala Asn Glu Glu
        1395                1400                1405

atc aac atc aat acg gac gcg atc gac gac gag gga gag gcg gag 13967
Ile Asn Ile Asn Thr Asp Ala Ile Asp Asp Glu Gly Glu Ala Glu
        1410                1415                1420

gag gga gag gcg gag gag gac gcg atc gac gac gag gga gag gcg 14012
Glu Gly Glu Ala Glu Glu Asp Ala Ile Asp Asp Glu Gly Glu Ala
        1425                1430                1435

gag gag gga gag gcg gag gag gac gcg att gac gac gag gga gag 14057
Glu Glu Gly Glu Ala Glu Glu Asp Ala Ile Asp Asp Glu Gly Glu
        1440                1445                1450

gcg gag gag gga gag gcg gag gag gac gcg att gac gac gag gga 14102
Ala Glu Glu Gly Glu Ala Glu Glu Asp Ala Ile Asp Asp Glu Gly
        1455                1460                1465

gag gcg gag gag gga gag gcg gag gag gga gag gcg gag gag gga 14147
Glu Ala Glu Glu Gly Glu Ala Glu Glu Gly Glu Ala Glu Glu Gly
        1470                1475                1480

gag gcg gag gag gac gcg atc gac gac gag gga gag gcg gag gag 14192
Glu Ala Glu Glu Asp Ala Ile Asp Asp Glu Gly Glu Ala Glu Glu
        1485                1490                1495

gac gcg gcg gag gag gac gcg atc gac gac gag gga gag gcg gag 14237
Asp Ala Ala Glu Glu Asp Ala Ile Asp Asp Glu Gly Glu Ala Glu
        1500                1505                1510

gag gat tat ttt tct gta agt caa gtt tgc agt cga gac gcg gat 14282
Glu Asp Tyr Phe Ser Val Ser Gln Val Cys Ser Arg Asp Ala Asp
        1515                1520                1525

gag gtt tat ttt acg tta gac ccg gaa ata agt tac agt acc gat 14327
Glu Val Tyr Phe Thr Leu Asp Pro Glu Ile Ser Tyr Ser Thr Asp
        1530                1535                1540

ctt cgc att gca aag gtt atg gag cct gcg gta tca aag gaa ctt 14372
Leu Arg Ile Ala Lys Val Met Glu Pro Ala Val Ser Lys Glu Leu
        1545                1550                1555

aat gta tca aaa cgt tgt gtt gaa cct gtt acc cta aca ggc tct 14417
Asn Val Ser Lys Arg Cys Val Glu Pro Val Thr Leu Thr Gly Ser
        1560                1565                1570

atg tta gcg cat aat ggg ttt gat gag tcc tgg ttt gct atg cgc 14462
Met Leu Ala His Asn Gly Phe Asp Glu Ser Trp Phe Ala Met Arg
        1575                1580                1585

gaa tgt acc cgt cgc gaa tat att acg gtc caa gga tta tac gac 14507
Glu Cys Thr Arg Arg Glu Tyr Ile Thr Val Gln Gly Leu Tyr Asp
        1590                1595                1600

cca att cat tta cgg tat cag ttt gat act tcc cgg atg aca ccc 14552
Pro Ile His Leu Arg Tyr Gln Phe Asp Thr Ser Arg Met Thr Pro
        1605                1610                1615

cca cag att ttg aga act ata cca gcc ctt cct aac atg aca ctt 14597
Pro Gln Ile Leu Arg Thr Ile Pro Ala Leu Pro Asn Met Thr Leu
        1620                1625                1630

ggt gaa ctt tta ttg att ttt cct att gaa ttt atg gcc cag cca 14642
Gly Glu Leu Leu Leu Ile Phe Pro Ile Glu Phe Met Ala Gln Pro
        1635                1640                1645

att tct ata gaa cgt att tta gtt gaa gat gta ttt tta gat agg 14687
Ile Ser Ile Glu Arg Ile Leu Val Glu Asp Val Phe Leu Asp Arg
        1650                1655                1660

cgg gct tcc agt aaa aca cat aaa tac ggc ccg cgt tgg aat tcc 14732
Arg Ala Ser Ser Lys Thr His Lys Tyr Gly Pro Arg Trp Asn Ser
        1665                1670                1675

gtc tac gca ctt cca tat aat gcg ggt aaa atg tat gta caa cac 14777
Val Tyr Ala Leu Pro Tyr Asn Ala Gly Lys Met Tyr Val Gln His
        1680                1685                1690

att cct ggg ttt tat gac gtg tcc tta cgt gct gtg ggc caa gga 14822
Ile Pro Gly Phe Tyr Asp Val Ser Leu Arg Ala Val Gly Gln Gly
        1695                1700                1705

acg gcc att tgg cat cac atg ata tta tcc aca gca gca tgc gct 14867
Thr Ala Ile Trp His His Met Ile Leu Ser Thr Ala Ala Cys Ala
        1710                1715                1720

att tct aat cgc att tca cat gga gat gga tta gga ttt ttg tta 14912
Ile Ser Asn Arg Ile Ser His Gly Asp Gly Leu Gly Phe Leu Leu
        1725                1730                1735

gac gcg gca att cgt att agc gca aac tgt att ttt ttg gga cgt 14957
Asp Ala Ala Ile Arg Ile Ser Ala Asn Cys Ile Phe Leu Gly Arg
        1740                1745                1750

aac gat aat ttt ggc gtg ggg gat cca tgt tgg tta gaa gac cat 15002
Asn Asp Asn Phe Gly Val Gly Asp Pro Cys Trp Leu Glu Asp His
        1755                1760                1765

ctt gcc gga tta cca cga gaa gcc gta ccc gac gta ctc caa gtg 15047
Leu Ala Gly Leu Pro Arg Glu Ala Val Pro Asp Val Leu Gln Val
        1770                1775                1780

aca cag ttg gtt ttg cca aat cgg ggt cca acg gtt gcc att atg 15092
Thr Gln Leu Val Leu Pro Asn Arg Gly Pro Thr Val Ala Ile Met
        1785                1790                1795

cgt ggt ttt ttt ggg gcg ttg gca tat tgg ccc gaa cta aga att 15137
Arg Gly Phe Phe Gly Ala Leu Ala Tyr Trp Pro Glu Leu Arg Ile
        1800                1805                1810

gct ata agt gaa cca tct aca tct ttg gtg cga tat gct acc ggt 15182
Ala Ile Ser Glu Pro Ser Thr Ser Leu Val Arg Tyr Ala Thr Gly
        1815                1820                1825

cac atg gaa ctt gcc gaa tgg ttt tta ttt tca cgt aca cat agt 15227
His Met Glu Leu Ala Glu Trp Phe Leu Phe Ser Arg Thr His Ser
        1830                1835                1840

tta aag cca caa ttt acc cca acg gaa cgg gaa atg tta gcg tca 15272
Leu Lys Pro Gln Phe Thr Pro Thr Glu Arg Glu Met Leu Ala Ser
        1845                1850                1855

ttt ttt acg ttg tat gtt act ctt ggt gga gga atg ttg aac tgg 15317
Phe Phe Thr Leu Tyr Val Thr Leu Gly Gly Gly Met Leu Asn Trp
        1860                1865                1870

atc tgt aga gca act gca atg tat tta gct gct cct tac cat tcc 15362
Ile Cys Arg Ala Thr Ala Met Tyr Leu Ala Ala Pro Tyr His Ser
        1875                1880                1885

cgt tcg gct tac atc gcg gtc tgt gaa tct ctg ccc tat tac tat 15407
Arg Ser Ala Tyr Ile Ala Val Cys Glu Ser Leu Pro Tyr Tyr Tyr
        1890                1895                1900

atc ccg gtt aat agt gac ctg tta tgt gat tta gag gta tta ctg 15452
Ile Pro Val Asn Ser Asp Leu Leu Cys Asp Leu Glu Val Leu Leu
        1905                1910                1915

tta ggc gag gtc gac ctc cca act gtt tgt gaa tcc tac gca act 15497
Leu Gly Glu Val Asp Leu Pro Thr Val Cys Glu Ser Tyr Ala Thr
        1920                1925                1930

att gca cac gaa tta acc gga tat gag gct gtt cgc aca gca gcc 15542
Ile Ala His Glu Leu Thr Gly Tyr Glu Ala Val Arg Thr Ala Ala
        1935                1940                1945

aca aat ttt atg ata gag ttt gcc gat tgt tat aag gaa agt gag 15587
Thr Asn Phe Met Ile Glu Phe Ala Asp Cys Tyr Lys Glu Ser Glu
        1950                1955                1960

acc gat tta atg gta agc gcg tac ctg ggg gcc gtt tta ttg tta 15632
Thr Asp Leu Met Val Ser Ala Tyr Leu Gly Ala Val Leu Leu Leu
        1965                1970                1975

caa cgg gtg ttg ggt cat gca aat ctt ctt ttg ttg ctt ctc tcc 15677
Gln Arg Val Leu Gly His Ala Asn Leu Leu Leu Leu Leu Leu Ser
        1980                1985                1990

ggt gct gcg ttg tac gga gga tgt tca att tac atc ccc cga ggt 15722
Gly Ala Ala Leu Tyr Gly Gly Cys Ser Ile Tyr Ile Pro Arg Gly
        1995                2000                2005

att tta gat gca tat aat act tta atg ttg gca gca agt cct ctt 15767
Ile Leu Asp Ala Tyr Asn Thr Leu Met Leu Ala Ala Ser Pro Leu
        2010                2015                2020

tac gct cac caa act tta aca tcc ttt tgg aaa gac cgc gat gat 15812
Tyr Ala His Gln Thr Leu Thr Ser Phe Trp Lys Asp Arg Asp Asp
        2025                2030                2035

gca atg caa act ttg ggg att cga ccg aca acg gac gtt tta ccc 15857
Ala Met Gln Thr Leu Gly Ile Arg Pro Thr Thr Asp Val Leu Pro
        2040                2045                2050

aaa gag caa gac agg ata gtt cag gca tca cct ata gag atg aac 15902
Lys Glu Gln Asp Arg Ile Val Gln Ala Ser Pro Ile Glu Met Asn
        2055                2060                2065

ttc cgt ttt gtg gga ttg gag acc atc tat ccc cga gaa cag ccc 15947
Phe Arg Phe Val Gly Leu Glu Thr Ile Tyr Pro Arg Glu Gln Pro
        2070                2075                2080

att ccc tcc gtg gac cta gcc gaa aat ctt atg caa tac agg aat 15992
Ile Pro Ser Val Asp Leu Ala Glu Asn Leu Met Gln Tyr Arg Asn
        2085                2090                2095

gaa att ctg ggt ttg gat tgg aaa agc gta gcc atg cat tta cta 16037
Glu Ile Leu Gly Leu Asp Trp Lys Ser Val Ala Met His Leu Leu
        2100                2105                2110

cga aaa tat taa gggttgtgat tttttcatt aggatgaaaa gaacgtttcc 16089
Arg Lys Tyr
        2115

tagccacacc cacaaaggag tttgtaaaat aaaatctctg tttagacctt aaaatttgtt 16149

gtgtgtgttg tgtggggggt ccgtgaggat cgacctttac aagatataat ttgtccatat 16209

cgca atg ttt tct cgg ttt gcg cgt tcc ttt tcc agc gat gat aga 16255
Met Phe Ser Arg Phe Ala Arg Ser Phe Ser Ser Asp Asp Arg
                2120                2125

acg cgt aaa tct tat gat ggt agt tac caa agt ttt aat gcc ggc 16300
Thr Arg Lys Ser Tyr Asp Gly Ser Tyr Gln Ser Phe Asn Ala Gly
2130                2135                2140

gaa cgt gat ttg ccc aca cct acc cgg gac tgg tgt tct att tcc 16345
Glu Arg Asp Leu Pro Thr Pro Thr Arg Asp Trp Cys Ser Ile Ser
2145                2150                2155

caa cgc ata acc agc gag cgc gtg agg gat gga tgt ctt att cca 16390
Gln Arg Ile Thr Ser Glu Arg Val Arg Asp Gly Cys Leu Ile Pro
2160                2165                2170

acg ccc ggc gag gct ttg gag acg gcg gta aag gct tta tct gaa 16435
Thr Pro Gly Glu Ala Leu Glu Thr Ala Val Lys Ala Leu Ser Glu
2175                2180                2185

aag acc gac agc cta aca tcg ccg gtt tta caa agt acc gaa aga 16480
Lys Thr Asp Ser Leu Thr Ser Pro Val Leu Gln Ser Thr Glu Arg
2190                2195                2200

cac agt gtt ctg ctt gga tta cac cat aat aat gtt cct gaa tcg 16525
His Ser Val Leu Leu Gly Leu His His Asn Asn Val Pro Glu Ser
2205                2210                2215

ttg gtg gtc tcg tgt atg tct aac gat gtt cat gac ggg ttt atg 16570
Leu Val Val Ser Cys Met Ser Asn Asp Val His Asp Gly Phe Met
2220                2225                2230

cag cgt tat atg gaa aca att caa aga tgt ttg gat gac ctg aaa 16615
Gln Arg Tyr Met Glu Thr Ile Gln Arg Cys Leu Asp Asp Leu Lys
2235                2240                2245

ctt tct ggg gat gga ctt tgg tgg gtt tat gaa aat aca tat tgg 16660
Leu Ser Gly Asp Gly Leu Trp Trp Val Tyr Glu Asn Thr Tyr Trp
2250                2255                2260

cag tat ctc aaa tac acc aca gga gcc gag gta ccg gtg act tca 16705
Gln Tyr Leu Lys Tyr Thr Thr Gly Ala Glu Val Pro Val Thr Ser
2265                2270                2275

gag aag gta aat aaa aag tct aaa tcc acg gtt ttg ttg ttt tca 16750
Glu Lys Val Asn Lys Lys Ser Lys Ser Thr Val Leu Leu Phe Ser
2280                2285                2290

tcc gta gtt gcc aat aaa cca ata tcc aga cat cct ttt aaa tct 16795
Ser Val Val Ala Asn Lys Pro Ile Ser Arg His Pro Phe Lys Ser
2295                2300                2305

aaa gtt ata aat tcg gat tac cgg gga ata tgt cag gag cta cgt 16840
Lys Val Ile Asn Ser Asp Tyr Arg Gly Ile Cys Gln Glu Leu Arg
2310                2315                2320

gag gcg tta gga gct gtg caa aag tat atg tat ttt atg cgt cca 16885
Glu Ala Leu Gly Ala Val Gln Lys Tyr Met Tyr Phe Met Arg Pro
2325                2330                2335

gat gat cct aca aac ccc agc ccg gat aca aga ata cgt gta caa 16930
Asp Asp Pro Thr Asn Pro Ser Pro Asp Thr Arg Ile Arg Val Gln
2340                2345                2350

gaa att gcg gct tac acg gct act ggc tac ggg tgg atg tta tgg 16975
Glu Ile Ala Ala Tyr Thr Ala Thr Gly Tyr Gly Trp Met Leu Trp
2355                2360                2365

ttc ttg gac gtt gtg gac gcc agg gta tgt cgc cat ctc aaa ctt 17020
Phe Leu Asp Val Val Asp Ala Arg Val Cys Arg His Leu Lys Leu
2370                2375                2380

caa ttt cga cgg att cga ggg ccg cgc gcg tct gtt att cca gat 17065
Gln Phe Arg Arg Ile Arg Gly Pro Arg Ala Ser Val Ile Pro Asp
2385                2390                2395

gat ttg ctt aga cga cat tta aaa acg ggt cct gcg gtc tca gcg 17110
Asp Leu Leu Arg Arg His Leu Lys Thr Gly Pro Ala Val Ser Ala
2400                2405                2410

ggc aca gga gtt gcg ttt att tta gca gca aca act gcc agc gct 17155
Gly Thr Gly Val Ala Phe Ile Leu Ala Ala Thr Thr Ala Ser Ala
2415                2420                2425

ctt act gcg ctt ttg cgt att agt gta tta tgg cga aag gaa gag 17200
Leu Thr Ala Leu Leu Arg Ile Ser Val Leu Trp Arg Lys Glu Glu
2430                2435                2440

tgg cgg gat ggt tta aat gga acc gca gct gca att gtt gcg gcg 17245
Trp Arg Asp Gly Leu Asn Gly Thr Ala Ala Ala Ile Val Ala Ala
2445                2450                2455

gtt gaa ctt att acg ctt ttg cac cac cat ttt caa tac tta att 17290
Val Glu Leu Ile Thr Leu Leu His His His Phe Gln Tyr Leu Ile
2460                2465                2470

aat atg atg ctt att gga tat gca tgt tgg ggg gat ggg gga tta 17335
Asn Met Met Leu Ile Gly Tyr Ala Cys Trp Gly Asp Gly Gly Leu
2475                2480                2485

aac gat cct tat ata tta aag gcg cta cgt gcc cag gga cgg ttt 17380
Asn Asp Pro Tyr Ile Leu Lys Ala Leu Arg Ala Gln Gly Arg Phe
2490                2495                2500

tta tat ttt gcg ggt cag ttg gtc aga aca atg tca aca cac agt 17425
Leu Tyr Phe Ala Gly Gln Leu Val Arg Thr Met Ser Thr His Ser
2505                2510                2515

tgg gtt gtg tta gag acc agc acc cat atg tgg ttt tcc cgg gcc 17470
Trp Val Val Leu Glu Thr Ser Thr His Met Trp Phe Ser Arg Ala
2520                2525                2530

gtg gcg cag agt att tta gca cat ggg ggt aaa ccc aca aag tat 17515
Val Ala Gln Ser Ile Leu Ala His Gly Gly Lys Pro Thr Lys Tyr
2535                2540                2545

tat gct cag gtt ctt gcc gcc agt aaa cgg tat act ccg tta cat 17560
Tyr Ala Gln Val Leu Ala Ala Ser Lys Arg Tyr Thr Pro Leu His
2550                2555                2560

tta aga cgt ata tcc gaa cca tcg agt gtg tct gat cag ccg tat 17605
Leu Arg Arg Ile Ser Glu Pro Ser Ser Val Ser Asp Gln Pro Tyr
2565                2570                2575

att cgt ttt aat cga ctg gga tct cca ata ggg aca ggt ata ggg 17650
Ile Arg Phe Asn Arg Leu Gly Ser Pro Ile Gly Thr Gly Ile Gly
2580                2585                2590

aat ttg gaa tgt gtc tgt tta acg gga aat tat tta tct gac gac 17695
Asn Leu Glu Cys Val Cys Leu Thr Gly Asn Tyr Leu Ser Asp Asp
2595                2600                2605

gta aat gca agt tcg cat gta att aat aca gaa gca ccg tta aac 17740
Val Asn Ala Ser Ser His Val Ile Asn Thr Glu Ala Pro Leu Asn
2610                2615                2620

agt ata gca ccc gat aca aat aga cag cgg act tct cgc gtt tta 17785
Ser Ile Ala Pro Asp Thr Asn Arg Gln Arg Thr Ser Arg Val Leu
2625                2630                2635

gtt cgt cca gac acg ggt ttg gat gta act gtc cga aaa aac cac 17830
Val Arg Pro Asp Thr Gly Leu Asp Val Thr Val Arg Lys Asn His
2640                2645                2650

tgt ctg gac ata ggc cat acg gac ggt agt cca gtt gac cca acg 17875
Cys Leu Asp Ile Gly His Thr Asp Gly Ser Pro Val Asp Pro Thr
2655                2660                2665

tat cct gat cat tac acc cgg ata aag gcg gaa tat gaa ggt ccg 17920
Tyr Pro Asp His Tyr Thr Arg Ile Lys Ala Glu Tyr Glu Gly Pro
2670                2675                2680

gtt cgg gat gaa tca aac aca atg ttt gac caa aga tcg gat tta 17965
Val Arg Asp Glu Ser Asn Thr Met Phe Asp Gln Arg Ser Asp Leu
2685                2690                2695

cgt cac ata gaa acc caa gca tct tta aat gat cac gta tat gaa 18010
Arg His Ile Glu Thr Gln Ala Ser Leu Asn Asp His Val Tyr Glu
2700                2705                2710

aat ata cca ccc aag gaa gtg ggt ttt aac tca tct tca gac ctg 18055
Asn Ile Pro Pro Lys Glu Val Gly Phe Asn Ser Ser Ser Asp Leu
2715                2720                2725

gat gtg gat agc ctt aac ggg tac acc tcc gga gac atg cat aca 18100
Asp Val Asp Ser Leu Asn Gly Tyr Thr Ser Gly Asp Met His Thr
2730                2735                2740

gac gat gac tta tca cca gat ttt ata ccc aac gac gtt ccc gtt 18145
Asp Asp Asp Leu Ser Pro Asp Phe Ile Pro Asn Asp Val Pro Val
2745                2750                2755

aga tgt aaa acc acg gtt acg ttt agg aaa aat acg cct aag agt 18190
Arg Cys Lys Thr Thr Val Thr Phe Arg Lys Asn Thr Pro Lys Ser
2760                2765                2770

cat cat taa gtacagcggt taatagatag ttatggacta ggcactttgg 18239
His His
2775

cggtcatttc cacaaccagg ttaaaattgg gggatttggg agaaaatagt ctattgcgta 18299

ttttctgttc aataattgga ctgcgttatt taaaggtctg attggttgat tgggttataa 18359

aaggaattac tcctttaaat tttacttaat gtacccacaa tatcaagtgg tcgtttgtat 18419

ttaacgatta ttaccggtac c atg gga gac ttg tca tgt tgg aca aag gtg 18470
Met Gly Asp Leu Ser Cys Trp Thr Lys Val
            2780                2785

ccg ggt ttt acg tta acc ggc gaa ctt cag tac tta aaa caa gtg 18515

Pro Gly Phe Thr Leu Thr Gly Glu Leu Gln Tyr Leu Lys Gln Val
            2790                2795                2800

gat gat att tta agg tat gga gtt cgg aaa cgc gat cga aca gga      18560
Asp Asp Ile Leu Arg Tyr Gly Val Arg Lys Arg Asp Arg Thr Gly
            2805                2810                2815

atc gga acg tta tct tta ttt gga atg caa gct cga tac aat ttg      18605
Ile Gly Thr Leu Ser Leu Phe Gly Met Gln Ala Arg Tyr Asn Leu
            2820                2825                2830

cga aat gaa ttt cct ctt tta act aca aag cgt gtt ttt tgg agg      18650
Arg Asn Glu Phe Pro Leu Leu Thr Thr Lys Arg Val Phe Trp Arg
            2835                2840                2845

gcc gtc gtg gaa gag ttg tta tgg ttt atc cgc ggg tca acc gat      18695
Ala Val Val Glu Glu Leu Leu Trp Phe Ile Arg Gly Ser Thr Asp
            2850                2855                2860

tcc aaa gaa ctc gcc gct aaa gat ata cac ata tgg gat ata tac      18740
Ser Lys Glu Leu Ala Ala Lys Asp Ile His Ile Trp Asp Ile Tyr
            2865                2870                2875

gga tcg agc aaa ttt cta aat agg aat ggc ttc cat aaa aga cac      18785
Gly Ser Ser Lys Phe Leu Asn Arg Asn Gly Phe His Lys Arg His
            2880                2885                2890

acg ggg gac ctt ggc ccc att tac ggc ttc cag tgg aga cat ttt      18830
Thr Gly Asp Leu Gly Pro Ile Tyr Gly Phe Gln Trp Arg His Phe
            2895                2900                2905

gga gcg gaa tat aaa gac tgt caa tca aac tat tta cag caa gga      18875
Gly Ala Glu Tyr Lys Asp Cys Gln Ser Asn Tyr Leu Gln Gln Gly
            2910                2915                2920

atc gat cag ctg caa act gtt ata gat aca att aaa aca aac cca      18920
Ile Asp Gln Leu Gln Thr Val Ile Asp Thr Ile Lys Thr Asn Pro
            2925                2930                2935

gaa agc cga cga atg att ata tcg tct tgg aat cca aag gat atc      18965
Glu Ser Arg Arg Met Ile Ile Ser Ser Trp Asn Pro Lys Asp Ile
            2940                2945                2950

ccc tta atg gta cta cct cca tgt cac acg tta tgt cag ttt tac      19010
Pro Leu Met Val Leu Pro Pro Cys His Thr Leu Cys Gln Phe Tyr
            2955                2960                2965

gtt gca aac ggt gaa tta tcc tgc caa gta tac cag aga tcg ggg      19055
Val Ala Asn Gly Glu Leu Ser Cys Gln Val Tyr Gln Arg Ser Gly
            2970                2975                2980

gat atg ggc ctt ggg gta ccg ttc aac att gct gga tat gca ctt      19100
Asp Met Gly Leu Gly Val Pro Phe Asn Ile Ala Gly Tyr Ala Leu
            2985                2990                2995

ctt acc tac ata gta gcg cat gtt aca gga ctt aaa acc gga gat      19145
Leu Thr Tyr Ile Val Ala His Val Thr Gly Leu Lys Thr Gly Asp
            3000                3005                3010

tta att cat aca atg ggg gat gca cat att tac ttg aat cat ata      19190
Leu Ile His Thr Met Gly Asp Ala His Ile Tyr Leu Asn His Ile
            3015                3020                3025

gat gct tta aaa gtg cag cta gct cga tcc cca aaa cct ttt cct      19235
Asp Ala Leu Lys Val Gln Leu Ala Arg Ser Pro Lys Pro Phe Pro
            3030                3035                3040

tgc ctt aaa att att cga aat gta aca gat ata aac gac ttt aaa      19280

Cys Leu Lys Ile Ile Arg Asn Val Thr Asp Ile Asn Asp Phe Lys
            3045                3050                3055

tgg gac gat ttt cag ctt gat gga tat aat cca cac ccc ccc cta 19325
Trp Asp Asp Phe Gln Leu Asp Gly Tyr Asn Pro His Pro Pro Leu
            3060                3065                3070

aaa atg gaa atg gct ctt taa tggattttta aatgttgtca agacagtaga 19376
Lys Met Glu Met Ala Leu
            3075

tgtgttgcga atgtaataaa atgatataca cagacgcgtt tggttggttt ctgtttatga 19436

acagcaacgg atgcataggg ttgcgataac tgcgataaga cccaatgtcc caaggataga 19496

tatcacacca attataactg ctacaacgga aaatgtagtg gcgtaggtag atgcatcgta 19556

ggtataaacg gccgaaaacg gagggaattt tttagggtaa ccatctagat gacacgaata 19616

ggtgataggt ccgtcgagtt ccgatgttgg acaagaactt tgcatgttta caaaccgttt 19676

gttttgatca cacaccccag taatctcact gttttcgtgg ttaatgggag aatcgttaac 19736

ccaccatacg aaatgtacaa cgccacgtgg cacacatttt gccgtacata ctatgtgtcc 19796

atcaataata cctatagaca cgttgggaaa tggatagacg tcaggggtaa cgacagcaga 19856

atatttcata ttagagacgc catcccgaat ccataaaaca ttacattgga tggctggggg 19916

tgggtaatcc atttgttttt gctgtggaat tcgtaccgcc gaaacataac taaataatcc 19976

attggcatat tcttgtattg catcggttat aaaatttttt ccgatgttac caaaccttga 20036

agtccaccga acacgtaccg agtgcggtgg ataatacttt gatacgttac agtaggctgc 20096

gtatgtctgt ccggttaaga ctggatcgcc gacaacggta atatttggac gataatacgt 20156

tgtaactgta atactgtgtt ccgatatgac gttcttagtt tttgtattaa cgactcgcca 20216

aatatacgtt ccctccgtgg tagcatccat agataaaatt gttacagaaa aatcagacgt 20276

tgttttaaca tctggtatta cataattttc cttagcgtgt gtaaatatct cagggttgtt 20336

tattaagttt aaatcggcac tgttgctata taacataacc ggtaaatctg gcatgcgtat 20396

taacgcattg cccagttgac ggtgcggatc tataaggtga cgcgtaaacc aaacttcaat 20456

atgaagatcg gggcgtataa gcgacttcca ccttgttata tttgaacctt ccggatctaa 20516

agaatattgt tcatatgttt tttgttgctg cttaaaggcc gcctgttgtc cggtcgttag 20576

acgcatgtaa caaggcatga taaatgtgtg aaaatagggt atggattgta ttccgccgtg 20636

aacgcattgt atattttcat atagaaaagg tggttgtgaa tgttgggtgt tggctgcggg 20696

atcgggcttt cgggaagcgg ccgaggtggg cgcgacggcg ggatcgggct ttcgggtagc 20756

ggccgaggtg ggcgcgacgg cgggatcggg ctttcgggaa gcggccgagg tgggcgcgac 20816

ggcgggatcg ggctttcggg tagcggccga ggtgggcgcg acggcgggat cgggctttcg 20876

ggaagcggcc gaggtgggcg cgacggcggg atcgggcttt cgggaagcgg ccgaggtggg 20936

cgcgacggcg ggatcgggct ttcgggaagc ggccgaggtg ggcgcgacgg cgggatcggg 20996

ctttcgggta gcggccgagg tatataattc agttatactt acgggtgtgg gttgagattc 21056

agtcgataat tgtatacacg cgatcgttaa aattaaattt atttgtatcc gcttcatcct 21116

ggtttttatt gacacatcca cgctcccctt aaataaaaga ttaaaacacc caccgcggaa 21176

tttaaatgat ggaaacgttt ttttcgacat tgggaataat aaaaacggct tttgcaactt 21236

taaaaacttt atttatctcg attacgatac atatgtacca catagatagc atagatttat 21296

tataatataa acacacacgt gatatacttt agtgatatga gatgccataa aacagtcaat 21356

aggtttaacg cttagtctca tcatctgaat acacgtcaaa cccgccgcaa ctgttgatgt 21416

tagaattata atagctcccc atgaaatgcc ggcaaatgtt acagctatac ccgtcaccga 21476

ggtcgttgta tataatacaa ttacccatag gttttttttt tcttgatata aaacggcaaa 21536

accctgtaac ccaaatgcta taatatgacc tcctattgaa actgctaacg ttacttgtgt 21596

aagtttgata aaatgattta atttaattat atgtgagatt gcccacatta atggggtaac 21656

tatatataac accgggggta taacagacat tatacgaatt cctttaaaca cgcgtttaag 21716

ggtccgggaa ctttctcgat ggtcacatac tctcccgcgg tcattttgtg tatatacaac 21776

ggcaaaacct aaatctgtat aagtgtttaa ttgcttatgg cgatttttac gatatataca 21836

cgtatcttgc aaatcggtgg cggcatcgac aattgaaact agtgtgacaa tagatataca 21896

caatccaata agaacctcat atttactgac atacatatat aaaataacgg ttagtaaacc 21956

tcccaaccca gttcccaaca tcataacata aaaataaata tgcggtccat tgaatgtcgt 22016

aacaaagttg tagtaatgga tatgcacagc agccactgtt ccggtaatcg cggatatgga 22076

aattcccagt aattctacaa atggaagatc ccgggatatt gggcaaccaa ccgcccataa 22136

cacagcaaaa cccaacacga ccaccgtctg caaacatcgt cccaattttg ctaatgtgcg 22196

tagaaatttc acggatgttg gccataaccc cgaaacgacg atcaacccca taatagttgc 22256

attgacggca gcttcgcaga cgtgatattg taaaattaac ccggacgtga taacgcttgc 22316

ttgtagtccc acgagaaaca accgcgatgc tgaggttatt gcacacgaat tacattcttg 22376

agggtttccg acacatcctt ggattgattg agcgcggatt aattctctgt ctaacacacc 22436

caggttttca tcatggacag ctctttcacc attcacggcc atgtcttaag tttaataatt 22496

caaaacaaat aaaaatgtgt tcatctatgg tacacacaag tttgtatgta aaatataagc 22556

aaaagttgca cttatttaac tgtacatatt acgtcagatt cacgtgataa ttcagaataa 22616

tccagggttc ctgcagggtc cactggagga gccacacaat attcgcgaat tccgattccc 22676

tcctgccatg tggtttcggg gagtttcccc cccattttat ttccggtatt tttttcgttt 22736

ctttttgtta ataaattgcg tctttttttt aatggtggtt catccttcac agattccatg 22796

ttcgcaaata attgcatcga ggttaatttt tctttaaggt ctttgggact taagaacgtt 22856

gcataaaaaa aagaatgcac gggtgcggaa cgttggatat acaatccaac catgggggag 22916

ttagttaagg cgagataaaa attaatataa cacgtctcat cccgtgttaa cttaagattt 22976

tgtacggcag aacggaatcc actgtgtgtt tccaataata ctccaaattc acgcatactc 23036

ccgctgccat aaacaacatt attaaggatc ctttttgaat ttgtgattga gcgtattaaa 23096

ttatatggtg taggcttgct tccgtttata tccaaggaaa cattaaatga gataaaacca 23156

cccccggcgg tctggatgta catatccgtg gctgttagaa tgaagcatgt tgtaaaccca 23216

aaagttttaa gtagtcgctg taaacgggtg aattgatcgc gttttaagca aatgcttata 23276

tctggagtta gatttggaaa catcattgta taacaagcga gttcacgttt tacaacttgt 23336

ttgtaacatt gtacttgatc atctggacca caatcacccg ggcgttgcca taccatcgtt 23396

tggataatac tccgctcggg gggttgtccg gtaaatttaa aatataaccg tgttggggtc 23456

gacggatctt ttgtatggcg aaacgcgtca ataagcgagg accgtccctc cgttgccgcg 23516

agtacaacca ttctcggccc agtccaatta tactggtcaa acatatttgc cggtatagga 23576

atatacagtt gttctgtttc caaactacag tgaataatta atccttcgtc gctgaatatt 23636

aaaatagaat cccttagtct attaaccaga ggtgatatag acgaaattaa accagtaagc 23696

gttttttccg ttaaaacagc tctggcgatt tctggggcgt caaaacccgc atgcaattcc 23756

atgtccaaag catcgtctgt acgcgacctc aaatccataa tttactactt aaaatgttta 23816

ctatagaaaa agtaatcata tgtaaacaca cgagtttcgt taatatgttt gtttaacccg 23876

atccggtgac ttaagtacat aaacaggcat gatatttgaa tagtacggcc catgggaggg 23936

aacatttcca cgtgttccaa tacagggggt gttccttaat agggactgtg caataaaata 23996

cgtaagaagt taccagattt gatgtaatgt ttgtcataaa aaatatgtac atcattatat 24056

acgtctgtaa ttaacacaag atcacatcga agaattactg aagccgctgt gaaacctttc 24116

acaagacgat ataaacttgg ttaagtgtat tg atg ggg ctc ttt gga ctg aca 24169
Met Gly Leu Phe Gly Leu Thr
        3080

cgc ttt atc cat gaa cat aaa ctg gtt aaa ccc agc atc att tca 24214
Arg Phe Ile His Glu His Lys Leu Val Lys Pro Ser Ile Ile Ser
3085                3090                3095

acg cca ccc gga gtt tta acc ccc gtg gcg gta gac gta tgg aac 24259
Thr Pro Pro Gly Val Leu Thr Pro Val Ala Val Asp Val Trp Asn
3100                3105                3110

gtc atg tac aca ttg ttg gaa cgt tta tac cct gtg ggt aaa cgc 24304
Val Met Tyr Thr Leu Leu Glu Arg Leu Tyr Pro Val Gly Lys Arg
3115                3120                3125

gag aat tta cac gga cca tct gta acg ata cat tgt ctt gga gtc 24349
Glu Asn Leu His Gly Pro Ser Val Thr Ile His Cys Leu Gly Val
3130                3135                3140

tta ttg cgg cta tta aca caa cgg tca tac tat ccg ata ttt gta 24394
Leu Leu Arg Leu Leu Thr Gln Arg Ser Tyr Tyr Pro Ile Phe Val
3145                3150                3155

ttg gaa cgt tgt aca gac ggc cca tta tca cgt gga gcc aag gca 24439
Leu Glu Arg Cys Thr Asp Gly Pro Leu Ser Arg Gly Ala Lys Ala
3160                3165                3170

att atg tca cgg gcc atg aac cac gat gaa agg gga acc tcg gac 24484
Ile Met Ser Arg Ala Met Asn His Asp Glu Arg Gly Thr Ser Asp
3175                3180                3185

tta acc cgt gtt cta cta tca tcc aac aca tca tgt tct atc aag 24529
Leu Thr Arg Val Leu Leu Ser Ser Asn Thr Ser Cys Ser Ile Lys
3190                3195                3200

tat aac aaa aca tcg gaa aca tat gac agt gtg ttt cga aac tct 24574
Tyr Asn Lys Thr Ser Glu Thr Tyr Asp Ser Val Phe Arg Asn Ser
3205                3210                3215

tcc acg agt tgt att cct agc gaa gaa aac aaa tcc cag gat atg 24619
Ser Thr Ser Cys Ile Pro Ser Glu Glu Asn Lys Ser Gln Asp Met
3220                3225                3230

ttt ttg gac ggt tgt cca cga caa act gac aag acg atc tgc ctg 24664
Phe Leu Asp Gly Cys Pro Arg Gln Thr Asp Lys Thr Ile Cys Leu
3235                3240                3245

cgc gac caa aac gta tgc agt ctt acc tct aca atg cca tcc cga 24709
Arg Asp Gln Asn Val Cys Ser Leu Thr Ser Thr Met Pro Ser Arg
3250                3255                3260

gga cat cct aac cat cga tta tat cac aaa ttg tgt gca agt ctt 24754
Gly His Pro Asn His Arg Leu Tyr His Lys Leu Cys Ala Ser Leu
3265                3270                3275

att aga tgg atg ggg tat gca tac gtc gag gcg gtt gac att gag 24799
Ile Arg Trp Met Gly Tyr Ala Tyr Val Glu Ala Val Asp Ile Glu
3280                3285                3290

gcg gac gag gca tgt gca aac tta ttt cat acg cgt aca gtg gct 24844
Ala Asp Glu Ala Cys Ala Asn Leu Phe His Thr Arg Thr Val Ala
3295                3300                3305

ttg gtt tat acg aca gat act gat tta ctc ttc atg ggc tgt gat 24889
Leu Val Tyr Thr Thr Asp Thr Asp Leu Leu Phe Met Gly Cys Asp
3310                3315                3320

att ttg tta gat gca att cct atg ttt gct cca gta gta cga tgt 24934
Ile Leu Leu Asp Ala Ile Pro Met Phe Ala Pro Val Val Arg Cys
3325                3330                3335

cgc gat ttg ctt caa tat tta gga att aca tac cct gaa ttt ttg 24979
Arg Asp Leu Leu Gln Tyr Leu Gly Ile Thr Tyr Pro Glu Phe Leu
3340                3345                3350

gtt gcc ttt gtt cgc tgt cag acc gat ttg cat aca agt gac aac 25024
Val Ala Phe Val Arg Cys Gln Thr Asp Leu His Thr Ser Asp Asn
3355                3360                3365

cta aaa tct gtt cag caa gtt att cag gat acc ggc ctg aaa gtt 25069
Leu Lys Ser Val Gln Gln Val Ile Gln Asp Thr Gly Leu Lys Val
3370                3375                3380

cca cat caa atg gac act tca acg cgc tcc ccc act tac gac tcg 25114
Pro His Gln Met Asp Thr Ser Thr Arg Ser Pro Thr Tyr Asp Ser
3385                3390                3395

tgg aga cat ggc gag gtt ttc aaa agt ctt acc gta gcc acg tcg 25159
Trp Arg His Gly Glu Val Phe Lys Ser Leu Thr Val Ala Thr Ser
3400                3405                3410

ggt aaa aca gaa aac gga gtg tcc gtt tcc aaa tat gca tct aac 25204
Gly Lys Thr Glu Asn Gly Val Ser Val Ser Lys Tyr Ala Ser Asn
3415                3420                3425

cga tcg gag gtg aca gta gac gcc agt tgg gct tta aac ctt ctg 25249
Arg Ser Glu Val Thr Val Asp Ala Ser Trp Ala Leu Asn Leu Leu
3430                3435                3440

cca ccc tca tcc tcc cca ttg gat aat ttg gaa cgc gca ttt gtt 25294
Pro Pro Ser Ser Ser Pro Leu Asp Asn Leu Glu Arg Ala Phe Val
3445                3450                3455

gaa cat ata atc gcc gtg gta act cca ttg acc cgc ggt cgc cta 25339
Glu His Ile Ile Ala Val Val Thr Pro Leu Thr Arg Gly Arg Leu
3460                3465                3470

aag tta atg aaa cgt gta aat att atg caa aat acg gca gac cca 25384
Lys Leu Met Lys Arg Val Asn Ile Met Gln Asn Thr Ala Asp Pro
3475                3480                3485

tat atg gtt att aac acc tta tat cat aac tta aag ggg gaa aaa 25429
Tyr Met Val Ile Asn Thr Leu Tyr His Asn Leu Lys Gly Glu Lys
3490                3495                3500

atg gct cgc caa tac gca cgt att ttt aaa cag ttt att cct act 25474
Met Ala Arg Gln Tyr Ala Arg Ile Phe Lys Gln Phe Ile Pro Thr
3505                3510                3515

cca ctc cca cta aac act gta tta aca aaa tat tgg aat taa 25516
Pro Leu Pro Leu Asn Thr Val Leu Thr Lys Tyr Trp Asn
3520                3525                3530

aacacacata agagcgactt aatggttcat tgttttattt tgctcgtata tacatgttat 25576

aaatcgttta tcactgtgcc cgcataagat gtactgtgtc tctcaaaaaa atttgtgttt 25636

ttatctgcaa tcataaatgc aagtggaaag tccgaatcgg gaggtggggt gttaaatagt 25696

tttggtacat taatcgctga taaaagcctg tccgcgctga atttcacgta ttgtgtaatt 25756

gcatcgacgt tcaccaaacg ggttttgggt gcatgggatt ttaaaaacgc acactcgatt 25816

tcaacggctt ccgaaaacag ttgatgtatt ctggtgatag cgggtttttc gggtacatag 25876

ttattgtata tacaacacga tgcgctggta tgtatggctt catctcggct tataaggtcg 25936

ttaaattgac aagttacaac aaatagtccg ttattgcgta aatatgcaat agccgcgaac 25996

gatgatacaa aaaaaatgcc ctctataaga atcattagta tatatttttc tgcaacggat 26056

gggttgtccc gtaccttttc ttccaaccat tgtacttttt gttggatcga cggattatta 26116

atagtgacat ttacgtattg tacccgcaac gattcatccc ctctgaacaa cattagttga 26176

atttgactat agacacgcgc gtggacaacc tcgatgcact cttgttcaat gtagtaatgg 26236

tgaatatcct tttgggaaaa gagttgggtt agagagccca aattaacatt taccagatca 26296

tctgccgccg ataaaaatgt aaaaataaat ctgtagaata ttagttcatc ttccgttaaa 26356

cagtccaagt attgataatc atcttcaatg ataaaatcgc tttctaacca acgattcgaa 26416

atgctcaggg cacgtaaatt gtttatatct ggacactccg gcctgtaaaa aaaatgactg 26476

caatctttct gatccatttt ggaatagttt cccgtgtaaa tttataaagc acaactggta 26536

caggttaatt cgcctcccgc aaacagtccg ctgttcgtag ctttacgaat tttacagtag 26596

tacatacccg ttttaaggcc ggctttatag gcacgtataa gcaaattcat tattttggag 26656

gcgggaattg tcccgtctgg gcgttcctca ataaataaag tcattgattg actttggtca 26716

ataaatggcg ccctttctgc acacatatca acgagatcct cttgctcata ttcaaacgct 26776

gttttatatt ttaagagtgg gtgactatta gataaacagc caaacgaacg tattactgac 26836

cattggtttt tctcaagtat gtttataact tccagtcgtt tttcttcaca tgaatacata 26896

tctcttagtt cgtccataag gtctaagttg ggtctaagta actcacccga ggtggtgacc 26956

ttactaaaca tattattata aattggagag aaaccctcac tgcactccgt tacctgtgca 27016
gatgaaactg tgggcattaa cgctaagaac tgcgagttgt ataacccata agcgcaaata 27076
tcatctcgca gggtacacca tggtaaatct aaataactta tcgtagaaaa cccatcttgg 27136
tgtaaccatc ccttagcata tttactttcg gtaaaaccct taaacggggc taagccgcca 27196
atcttacaca tttccatgct tgttttcatt gtctcataca acattaactc cgctatttgt 27256
acatttaacc gtctagctgg ttgggaagtt aaatcaaatc ctaagcggag acaagttgta 27316
tgtaaccctt gtatgccaat gccaagtgat cggttgtttt ttacaccttt acatgatttt 27376
ttacatggaa agttcccagc cgccaggacc ccgtttaaaa aaataacagt cgttcttgct 27436
gtcaattgaa ggtcgtttaa attaaatgac actgggcctt tggataagca cgttgtaaga 27496
tttatgctgg caagattaca tacgccatgt tgatgagcgt ctgccttttg aacaatttcc 27556
gtacacaaat ttgaccccgt gatagcattt ccttgggtat tcatatgata attacgatta 27616
caggcatctt tgaacattaa aaaggggctt cctgttacag cagcactgcg tatgattgtg 27676
aatgcgatat cttgaatggg aacagaagaa acgcctaatc cttctctctc taaacgtaaa 27736
taggttgaag tgaatgcctc cccgtgtaat gttcgaagga tatcggctct gttatcaaaa 27796
agagtccact gaacattact agcccctttt agatagctta ggtatctttc aaaaaataaa 27856
tctggggtcc ataaacaaca aaatatgtta tcacatcgaa atatttcatc acgaaccaac 27916
attccacgtg tggccaaaac agtttgtaga tcgacgtgcc atggttctat gtaaacacaa 27976
actccagttg gtcgttcaca atcactgtta attgccataa ccatgcaatc taaaagtttt 28036
aaaactgcaa gaagaccttt cgtttgattt tccgtaggta ttaaattcag actctgtaga 28096
gaaattccca ctccacctcg actttgtaat accgttccca catcgcctgt gatagctcga 28156
acagctctcc caacagtgat ggattccggg tccattaaat aacaactggc cgttgccccg 28216
gtctctcgac ctaaaaacat cataaccggt gtagccggga caattttctg acatgccaac 28276
gctgtgaaaa atacccgaca gacatcagtc catgtataac catcatttat tccgggaata 28336
agagttgcga ttttaggcag gtttacgatt tctgttgtca cggtggccgc cagtcttaaa 28396
aagaattggc aaagcgactc taatttacct tcctctaact tagttaaata aaagtcttcg 28456
tactttaaag cagactgtag tccaagggta gctaaagcgg ggtattgatc tttcaaaaac 28516
ggttctaata tagcccgacg aatttcgtcc ctccgccctt caattgcttg gcggactcgg 28576
ggagttaaac agagaattgg ggaagtcaac cacgtttcca tggaaacgga tcgtaggtta 28636
atacggcaat ggataagttc tccacaacat cggtacactc gctcatcttg tcgcgtcacc 28696
gccttaagtt ttgagacgat agtgctaata tactccatta attccaccgg tgtggttgat 28756
tcgggcggaa tgatgtattc cttgtagcca tgttgacata atcggtttat aatgtcatga 28816
accgtattaa aaattctttt gaactccata acggataacg tatttaggct ccggaataaa 28876
cctttaaacc ctaaactcac agctgagtta gttctacaat attgtagact cccttatata 28936
tggttacgta cagcctgccc ctccccagta tataatatca cgcaaaaccc acgctatgtt 28996

aaattcagtt tattttacat acatgcttta ataataacat tcgttccatg tatttgtacc 29056

cccccacaca accccctcta accaaatagt tggcacgtta taacctccga accgttccat 29116

gcgtcttgta taacgcacag actctgatgg aattgttcca attaacgtat atgccgcata 29176

catgcaggat aattgtgtgg gaagtccccg aaaatcgccg gtccattgat acaatcgctg 29236

tctagccaag ttccaattta ctcctgtaat ttcgccaata ctacatcgag ggcttgtcgg 29296

gtcattggat aactgcacaa gcggcaacgc ccttgtgtta tatggctggt gggtatttgc 29356

aaccccttca gtcccccagg cggcattttc agctcgtatg cgtcctaaca ggaagccaat 29416

accacgacca aaacattgtt cgtttagttg gcttaatgca agatgcagtc ttacaccttc 29476

tcgttggcgt cgctgtgtat atacaaaaac caagaacaca tgcttcagtc cgtccgcgga 29536

aagatgtaaa tctttgtcaa cgtcccaaaa tacgcaggcc gggatgttgg ctgtgaccct 29596

gcgagttgaa gttttgtctg tacgtgcagc ttcttgggga cctttggcca cggcggttat 29656

attgcataaa ttatcctgaa tggtatattc cagcagggac ccaaaaaaac ttataaatcg 29716

atgtggaaat acatgacatt gtaccatcgc acgtaaacac tccgaaaacc ttatgagccg 29776

cgtttccata cgactgcatc cataggcaga aacaattgct gttctgttgg catccgctgc 29836

ctgtttatcc gtatattctt ctgcccggca tgcggcgatg aaacttaatg acgttacata 29896

tgctctaagc cccccacctt ctccaacggt ccaaggagcc gtgcaggcat tgaataggtt 29956

tcgtaaaccc tctagtagta catcggggtc acgtccagcc tgtgtaagtg tattagcttc 30016

tccaatcatg tcagatggat gacgaaggat taagacgatt gacccagcat gctcaatgtc 30076

cggacgaaaa aaatcggtta atgacacttg ttggattagc tgtgtcgttg atttaaaatt 30136

atttaacggg agtctaatgg taacttgcgg gttaccaatt gaagttggat ttatttgaat 30196

gttgttcata cgattaataa caattgaacg gggggttact tgaatagacg cggtttctgt 30256

acgttttggt ggtacatgta tcggttgttt gttcagacct ccaaagcgag ggccaattgt 30316

taaatcgcga ctccaatttc cgaagaagcc cggagcataa gtcatatgaa gcccgttccc 30376

tatttgaata aaacggttat ttcctaaaag actgatatta gttccacata gcgtttgttc 30436

gtttaaagta aaatgcgagt tggttggttg actccccata gctgaggggt taaattcaca 30496

caatgcaatc gtgacgtggt actatctgaa atgttgcctg gggtatgtgt acacattata 30556

cagtcgtagt accgtttata taatgttagg taggaggagc ctataaaaat attttgattg 30616

gcgttaaaag gttcttcaac ttaccgtgac gtccttttta ttaacatgcg tttttattga 30676

tgttacattt atgtcttttc attccggacg gatgtagctt tttcatatca cgttataaag 30736

ttaagtcagc gtagaatata cc atg gaa gaa cca att tgt tat gat aca 30785
Met Glu Glu Pro Ile Cys Tyr Asp Thr
        3535                3540

caa aaa ctt ttg gat gat tta agt aac ttg aaa gta caa gaa gcg 30830
Gln Lys Leu Leu Asp Asp Leu Ser Asn Leu Lys Val Gln Glu Ala
            3545                3550                3555

gac aac gaa aga cca tgg tca cca gag aaa aca gaa atc gcc aga 30875

Asp Asn Glu Arg Pro Trp Ser Pro Glu Lys Thr Glu Ile Ala Arg
            3560                3565                3570

gtt aag gta gtt aag ttt tta cga tct acc cag aaa att cca gct 30920
Val Lys Val Val Lys Phe Leu Arg Ser Thr Gln Lys Ile Pro Ala
            3575                3580                3585

aaa cat ttt att cag ata tgg gaa ccc ctg cat tct aat atc tgt 30965
Lys His Phe Ile Gln Ile Trp Glu Pro Leu His Ser Asn Ile Cys
            3590                3595                3600

ttt gta tat tcc aat aca ttt ttg gcg gag gct gct ttc acg gcc 31010
Phe Val Tyr Ser Asn Thr Phe Leu Ala Glu Ala Ala Phe Thr Ala
            3605                3610                3615

gaa aat tta ccc gga ctg ttg ttt tgg aga cta gat cta gac tgg 31055
Glu Asn Leu Pro Gly Leu Leu Phe Trp Arg Leu Asp Leu Asp Trp
            3620                3625                3630

acg ata gag gag cca ggt aat agc tta aaa att tta acc cag cta 31100
Thr Ile Glu Glu Pro Gly Asn Ser Leu Lys Ile Leu Thr Gln Leu
            3635                3640                3645

tca agt gta gta caa gat tcc gag acg tta cat cgt tta tcg gcc 31145
Ser Ser Val Val Gln Asp Ser Glu Thr Leu His Arg Leu Ser Ala
            3650                3655                3660

aat aaa tta cga acc tcg tct aaa ttt gga ccc gtt tcg ata cac 31190
Asn Lys Leu Arg Thr Ser Ser Lys Phe Gly Pro Val Ser Ile His
            3665                3670                3675

ttc att ata acg gac tgg ata aat atg tac gag gtc gcc tta aag 31235
Phe Ile Ile Thr Asp Trp Ile Asn Met Tyr Glu Val Ala Leu Lys
            3680                3685                3690

gat gca aca aca gcc att gaa tca cca ttc act cac gct cgt att 31280
Asp Ala Thr Thr Ala Ile Glu Ser Pro Phe Thr His Ala Arg Ile
            3695                3700                3705

gga atg ttg gaa agc gcc att gca gct tta aca caa cat aaa ttt 31325
Gly Met Leu Glu Ser Ala Ile Ala Ala Leu Thr Gln His Lys Phe
            3710                3715                3720

gcg atc att tac gat atg cca ttt gtt caa gag ggg att cgt gtt 31370
Ala Ile Ile Tyr Asp Met Pro Phe Val Gln Glu Gly Ile Arg Val
            3725                3730                3735

tta aca caa tat gca gga tgg ctt ctt ccg ttt aat gtt atg tgg 31415
Leu Thr Gln Tyr Ala Gly Trp Leu Leu Pro Phe Asn Val Met Trp
            3740                3745                3750

aat cag att caa aat agc tca ctc act cct cta aca cga gcc ctt 31460
Asn Gln Ile Gln Asn Ser Ser Leu Thr Pro Leu Thr Arg Ala Leu
            3755                3760                3765

ttt ata atc tgt atg att gat gaa tat ctc acg gaa acg cca gta 31505
Phe Ile Ile Cys Met Ile Asp Glu Tyr Leu Thr Glu Thr Pro Val
            3770                3775                3780

cat agc ata tca gaa tta ttt gca gat act gta aat tta att aaa 31550
His Ser Ile Ser Glu Leu Phe Ala Asp Thr Val Asn Leu Ile Lys
            3785                3790                3795

gat gag gcg ttc gta tcc atc gaa gaa gcg gta acg aat cca cga 31595
Asp Glu Ala Phe Val Ser Ile Glu Glu Ala Val Thr Asn Pro Arg
            3800                3805                3810

acg gtg cac gag tca cga att tcc tca gct ctg gct tat cga gac 31640

Thr Val His Glu Ser Arg Ile Ser Ser Ala Leu Ala Tyr Arg Asp
            3815                3820                3825

cct tat gtt ttt gag aca tcc ccg gga atg ctt gct agg aga ctt 31685
Pro Tyr Val Phe Glu Thr Ser Pro Gly Met Leu Ala Arg Arg Leu
            3830                3835                3840

aga tta gac aat ggt ata tgg gaa agc aac ctc tta tcg ttg tcc 31730
Arg Leu Asp Asn Gly Ile Trp Glu Ser Asn Leu Leu Ser Leu Ser
            3845                3850                3855

acc ccc gga att cat att gag gcg ctg tta cat tta cta aac tcc 31775
Thr Pro Gly Ile His Ile Glu Ala Leu Leu His Leu Leu Asn Ser
            3860                3865                3870

gac ccg gaa gcg gaa acc aca tct gga agt aat gta gca gaa cac 31820
Asp Pro Glu Ala Glu Thr Thr Ser Gly Ser Asn Val Ala Glu His
            3875                3880                3885

acc cgt ggc att tgg gaa aag gtt cag gct agt aca tcg cct agt 31865
Thr Arg Gly Ile Trp Glu Lys Val Gln Ala Ser Thr Ser Pro Ser
            3890                3895                3900

atg tta ata agc acc ctt gcc gaa tcc ggg ttt aca aga ttt tca 31910
Met Leu Ile Ser Thr Leu Ala Glu Ser Gly Phe Thr Arg Phe Ser
            3905                3910                3915

tgc aaa ttg cta cgt cgg ttt att gct cac cac aca ctc gcc ggt 31955
Cys Lys Leu Leu Arg Arg Phe Ile Ala His His Thr Leu Ala Gly
            3920                3925                3930

ttt att cac gga agc gtt gta gca gac gag cat att aca gat ttc 32000
Phe Ile His Gly Ser Val Val Ala Asp Glu His Ile Thr Asp Phe
            3935                3940                3945

caa caa aca cta gga tgt ctc gct tta gtg ggt gga ctg gca tac 32045
Gln Gln Thr Leu Gly Cys Leu Ala Leu Val Gly Gly Leu Ala Tyr
            3950                3955                3960

caa tta gtg gaa acg tac gct cct act acc gag tat gtg tta aca 32090
Gln Leu Val Glu Thr Tyr Ala Pro Thr Thr Glu Tyr Val Leu Thr
            3965                3970                3975

tat aca cgg aca gta aac gag acc gaa aaa cgg tat gaa acg cta 32135
Tyr Thr Arg Thr Val Asn Glu Thr Glu Lys Arg Tyr Glu Thr Leu
            3980                3985                3990

tta ccc gcc tta gga tta cca ccg gga ggc ctg gga caa att atg 32180
Leu Pro Ala Leu Gly Leu Pro Pro Gly Gly Leu Gly Gln Ile Met
            3995                4000                4005

cgg cgc tgt ttt gct cca cga ccc ctt att gaa agt ata caa gcg 32225
Arg Arg Cys Phe Ala Pro Arg Pro Leu Ile Glu Ser Ile Gln Ala
            4010                4015                4020

aca cgc gta ata cta ctt aat gaa att tca cat gca gaa gct aga 32270
Thr Arg Val Ile Leu Leu Asn Glu Ile Ser His Ala Glu Ala Arg
            4025                4030                4035

gag aca aca tat ttt aag caa aca cat aat caa tcc tca ggt gcg 32315
Glu Thr Thr Tyr Phe Lys Gln Thr His Asn Gln Ser Ser Gly Ala
            4040                4045                4050

tta tta cca caa gca gga caa agt gcc gta cgc gaa gcc gta cta 32360
Leu Leu Pro Gln Ala Gly Gln Ser Ala Val Arg Glu Ala Val Leu
            4055                4060                4065

acc tgg ttt gac cta cgt atg gat tca aga tgg ggt att act ccc 32405

Thr Trp Phe Asp Leu Arg Met Asp Ser Arg Trp Gly Ile Thr Pro
            4070                4075                4080

ccg gtg gat gtg ggt atg aca cct cct att tgt gtt gat cca ccg 32450
Pro Val Asp Val Gly Met Thr Pro Pro Ile Cys Val Asp Pro Pro
            4085                4090                4095

gct aca ggg ttg gaa gct gtc atg ata aca gaa gca cta aag att 32495
Ala Thr Gly Leu Glu Ala Val Met Ile Thr Glu Ala Leu Lys Ile
            4100                4105                4110

gca tat cct acc gaa tat aat cgc tct agc gtg ttt gtg gaa ccg 32540
Ala Tyr Pro Thr Glu Tyr Asn Arg Ser Ser Val Phe Val Glu Pro
            4115                4120                4125

tcg ttt gtg cct tat att att gca aca agc acg ctt gat gcc ctt 32585
Ser Phe Val Pro Tyr Ile Ile Ala Thr Ser Thr Leu Asp Ala Leu
            4130                4135                4140

tcg gca aca ata gct ttg tct ttt gat aca cgg gga ata cag caa 32630
Ser Ala Thr Ile Ala Leu Ser Phe Asp Thr Arg Gly Ile Gln Gln
            4145                4150                4155

gcc ttg tct att ctt cag tgg gct cgc gat tat gga tcc gga acc 32675
Ala Leu Ser Ile Leu Gln Trp Ala Arg Asp Tyr Gly Ser Gly Thr
            4160                4165                4170

gtg ccc aat gca gat gga tat cgc aca aaa cta tct gct ctt ata 32720
Val Pro Asn Ala Asp Gly Tyr Arg Thr Lys Leu Ser Ala Leu Ile
            4175                4180                4185

aca ata tta gaa cct ttt acc cgt aca cac ccc cca gta ctt tta 32765
Thr Ile Leu Glu Pro Phe Thr Arg Thr His Pro Pro Val Leu Leu
            4190                4195                4200

cca tct cac gtt tct act ata gat tcc ctt ata tgc gaa ctt cat 32810
Pro Ser His Val Ser Thr Ile Asp Ser Leu Ile Cys Glu Leu His
            4205                4210                4215

cgg act gtt ggc att gcc gtt gac ctg ctt ccc cag cac gtc cgt 32855
Arg Thr Val Gly Ile Ala Val Asp Leu Leu Pro Gln His Val Arg
            4220                4225                4230

cct ttg gtt cct gac cgt cct tct att aca aat agc gtt ttt tta 32900
Pro Leu Val Pro Asp Arg Pro Ser Ile Thr Asn Ser Val Phe Leu
            4235                4240                4245

gca act ctc tat tat gat gaa ctt tac ggt cgt tgg acc cga ctg 32945
Ala Thr Leu Tyr Tyr Asp Glu Leu Tyr Gly Arg Trp Thr Arg Leu
            4250                4255                4260

gat aaa aca tcg cag gcg ttg gtt gaa aat ttt aca tcc aac gcg 32990
Asp Lys Thr Ser Gln Ala Leu Val Glu Asn Phe Thr Ser Asn Ala
            4265                4270                4275

tta gtg gtt tct cgg tac atg tta atg tta caa aaa ttt ttt gcg 33035
Leu Val Val Ser Arg Tyr Met Leu Met Leu Gln Lys Phe Phe Ala
            4280                4285                4290

tgt cgt ttt tat cca acg cca gat ctt cag gct gtt ggt atc tgt 33080
Cys Arg Phe Tyr Pro Thr Pro Asp Leu Gln Ala Val Gly Ile Cys
            4295                4300                4305

aac cca aag gtt gaa cgc gat gaa caa ttt ggg gta tgg cgt tta 33125
Asn Pro Lys Val Glu Arg Asp Glu Gln Phe Gly Val Trp Arg Leu
            4310                4315                4320

aac gat ctt gct gat gcg gtt ggt cat att gtt ggg aca ata caa 33170

Asn Asp Leu Ala Asp Ala Val Gly His Ile Val Gly Thr Ile Gln
            4325                4330                4335

gga atc cga acg caa atg aga gtg gga ata tcc agc ctg cgc aca 33215
Gly Ile Arg Thr Gln Met Arg Val Gly Ile Ser Ser Leu Arg Thr
            4340                4345                4350

att atg gcc gat gct tcc tca gcc ctt agg gaa tgt gaa aat tta 33260
Ile Met Ala Asp Ala Ser Ser Ala Leu Arg Glu Cys Glu Asn Leu
            4355                4360                4365

atg act aaa acc tcc act tct gct att ggg cct ctt ttt tca acg 33305
Met Thr Lys Thr Ser Thr Ser Ala Ile Gly Pro Leu Phe Ser Thr
            4370                4375                4380

atg gct tcc cgg tat gca cgg ttt aca cag gat caa atg gac att 33350
Met Ala Ser Arg Tyr Ala Arg Phe Thr Gln Asp Gln Met Asp Ile
            4385                4390                4395

tta atg cgt gtt gac aaa cta aca aca gga gaa aat ata ccc ggt 33395
Leu Met Arg Val Asp Lys Leu Thr Thr Gly Glu Asn Ile Pro Gly
            4400                4405                4410

ctt gca aat gta gag att ttt tta aat agg tgg gaa cga ata gca 33440
Leu Ala Asn Val Glu Ile Phe Leu Asn Arg Trp Glu Arg Ile Ala
            4415                4420                4425

aca gct tgt agg cat gcc acg gca gtc ccg tcg gcc gaa tct att 33485
Thr Ala Cys Arg His Ala Thr Ala Val Pro Ser Ala Glu Ser Ile
            4430                4435                4440

gca acc gtg tgt aat gaa ttg agg cgc ggt tta aaa aat ata caa 33530
Ala Thr Val Cys Asn Glu Leu Arg Arg Gly Leu Lys Asn Ile Gln
            4445                4450                4455

gag gat cgt gta aat gcc cca acc tca tat atg agt cac gcc cga 33575
Glu Asp Arg Val Asn Ala Pro Thr Ser Tyr Met Ser His Ala Arg
            4460                4465                4470

aat ctg gaa gat cac aag gca gca gtt tca ttc gtt atg gac tcc 33620
Asn Leu Glu Asp His Lys Ala Ala Val Ser Phe Val Met Asp Ser
            4475                4480                4485

agg caa cag ttt att gtg gat tct gga cct cag atg ggc gcg gtt 33665
Arg Gln Gln Phe Ile Val Asp Ser Gly Pro Gln Met Gly Ala Val
            4490                4495                4500

tta act tca caa tgt aat ata gga aca tgg gag aat gta aat gca 33710
Leu Thr Ser Gln Cys Asn Ile Gly Thr Trp Glu Asn Val Asn Ala
            4505                4510                4515

acg ttt tta cat gat aat gtt aaa ata acg aca acg gtc aga gac 33755
Thr Phe Leu His Asp Asn Val Lys Ile Thr Thr Thr Val Arg Asp
            4520                4525                4530

gta att tca gag gct ccg acg ctg ata ata gga caa aga tgg ctt 33800
Val Ile Ser Glu Ala Pro Thr Leu Ile Ile Gly Gln Arg Trp Leu
            4535                4540                4545

cgt cca gat gag att tta tct aat gta gat ttg cgt ctt ggc gta 33845
Arg Pro Asp Glu Ile Leu Ser Asn Val Asp Leu Arg Leu Gly Val
            4550                4555                4560

ccc ggg aat aca agt ggg agt gac cct taa tataaaacag gcgtgtttat 33895
Pro Gly Asn Thr Ser Gly Ser Asp Pro
            4565                4570

gtacattaaa gtatttgtgg tttttattga ctgggcgttt cgtttgtata acgctgttgt 33955

tgctagtatt ttcataacct cctaggtttt tggagctaca cgtgcttatt caacgctctt 34015

tgggatttga atcatcgtaa acgtagcgtc cctaccagtt gagcgcgtaa ttttcgtaag 34075

caataaa atg gat ata att ccg cct ata gct gtc act gtt gcg gga gtg 34124
Met Asp Ile Ile Pro Pro Ile Ala Val Thr Val Ala Gly Val
                4575                4580

gga agc cgt aat caa ttt gac ggt gcc ctg gga ccg gcg tca ggt 34169
Gly Ser Arg Asn Gln Phe Asp Gly Ala Leu Gly Pro Ala Ser Gly
4585                4590                4595

ctg tca tgt tta aga aca tct tta tcg ttt ttg cat atg aca tat 34214
Leu Ser Cys Leu Arg Thr Ser Leu Ser Phe Leu His Met Thr Tyr
4600                4605                4610

gcg cat gga att aat gca acc ctg tca tca gac atg att gat gga 34259
Ala His Gly Ile Asn Ala Thr Leu Ser Ser Asp Met Ile Asp Gly
4615                4620                4625

tgt tta caa gag ggt gca gca tgg act acg gat ctg tct aat atg 34304
Cys Leu Gln Glu Gly Ala Ala Trp Thr Thr Asp Leu Ser Asn Met
4630                4635                4640

ggg agg ggt gtc cca gat atg tgt gct ctt gtt gat ctc ccc aat 34349
Gly Arg Gly Val Pro Asp Met Cys Ala Leu Val Asp Leu Pro Asn
4645                4650                4655

cga att tca tat att aaa ctg ggg gac act acc agt acg tgc tgc 34394
Arg Ile Ser Tyr Ile Lys Leu Gly Asp Thr Thr Ser Thr Cys Cys
4660                4665                4670

gtt ttg tct aga ata tac ggc gat agc cat ttt ttt acc gtt cca 34439
Val Leu Ser Arg Ile Tyr Gly Asp Ser His Phe Phe Thr Val Pro
4675                4680                4685

gac gag ggt ttt atg tgc aca caa att ccc gct aga gcg ttt ttc 34484
Asp Glu Gly Phe Met Cys Thr Gln Ile Pro Ala Arg Ala Phe Phe
4690                4695                4700

gat gat gtg tgg atg gga cgt gaa gag tcg tat aca att ata act 34529
Asp Asp Val Trp Met Gly Arg Glu Glu Ser Tyr Thr Ile Ile Thr
4705                4710                4715

gta gac tca acg gga atg gcc atc tat cgt cag gga aac ata tct 34574
Val Asp Ser Thr Gly Met Ala Ile Tyr Arg Gln Gly Asn Ile Ser
4720                4725                4730

ttt att ttt gat cca cat ggc cat ggg act ata gga cag gct gta 34619
Phe Ile Phe Asp Pro His Gly His Gly Thr Ile Gly Gln Ala Val
4735                4740                4745

gtt gtt cgg gtg aat acc acg gat gtg tac tct tat atc gca tcg 34664
Val Val Arg Val Asn Thr Thr Asp Val Tyr Ser Tyr Ile Ala Ser
4750                4755                4760

gag tat acc cac cgc ccc gat aac gta gaa tcc caa tgg gcc gct 34709
Glu Tyr Thr His Arg Pro Asp Asn Val Glu Ser Gln Trp Ala Ala
4765                4770                4775

gca tta gtt ttt ttt gtc acc gca aac gac ggt ccc gta agc gaa 34754
Ala Leu Val Phe Phe Val Thr Ala Asn Asp Gly Pro Val Ser Glu
4780                4785                4790

gaa gcg cta tct tcg gca gta acg ctt ata tac gga agc tgt gat 34799
Glu Ala Leu Ser Ser Ala Val Thr Leu Ile Tyr Gly Ser Cys Asp
4795                4800                4805

aca tat ttt aca gat gaa caa tat tgc gaa aaa ctg gtt aca gct 34844
Thr Tyr Phe Thr Asp Glu Gln Tyr Cys Glu Lys Leu Val Thr Ala
4810                4815                4820

caa cat ccg ttg ctt ctt tca cct cct aat tcc acg aca att gtg 34889
Gln His Pro Leu Leu Leu Ser Pro Pro Asn Ser Thr Thr Ile Val
4825                4830                4835

ctt aat aaa tcg tct ata gta cct ctt cac caa aac gtt ggt gaa 34934
Leu Asn Lys Ser Ser Ile Val Pro Leu His Gln Asn Val Gly Glu
4840                4845                4850

agt gta tcc ttg gaa gca acc cta cat tca acg tta acc aac acg 34979
Ser Val Ser Leu Glu Ala Thr Leu His Ser Thr Leu Thr Asn Thr
4855                4860                4865

gtt gca ctg gac cct aga tgt agt tac agc gag gtt gat cct tgg 35024
Val Ala Leu Asp Pro Arg Cys Ser Tyr Ser Glu Val Asp Pro Trp
4870                4875                4880

cat gcg gtt cta gaa aca acc tcg act ggg tct ggc gtt ttg gat 35069
His Ala Val Leu Glu Thr Thr Ser Thr Gly Ser Gly Val Leu Asp
4885                4890                4895

tgt cgt cgt aga cgc cgt cct tca tgg act cct cct tca agc gag 35114
Cys Arg Arg Arg Arg Arg Pro Ser Trp Thr Pro Pro Ser Ser Glu
4900                4905                4910

gaa aat tta gct tgt atc gac gat ggc ttg gta aat aat aca cat 35159
Glu Asn Leu Ala Cys Ile Asp Asp Gly Leu Val Asn Asn Thr His
4915                4920                4925

tcc acg gat aat tta cat aaa ccc gct aaa aag gtt ctc aaa ttt 35204
Ser Thr Asp Asn Leu His Lys Pro Ala Lys Lys Val Leu Lys Phe
4930                4935                4940

aaa cca act gta gac gtg ccg gat aaa aca caa gtg gca cat gta 35249
Lys Pro Thr Val Asp Val Pro Asp Lys Thr Gln Val Ala His Val
4945                4950                4955

tta ccc cgc cta cga gaa gtt gct aac acc cca gac gtt gtg tta 35294
Leu Pro Arg Leu Arg Glu Val Ala Asn Thr Pro Asp Val Val Leu
4960                4965                4970

aat gta tcc aat gta gat acg cct gaa tcc agt ccc act ttt tca 35339
Asn Val Ser Asn Val Asp Thr Pro Glu Ser Ser Pro Thr Phe Ser
4975                4980                4985

cgg aac atg aat gta gga agc agt ttg aaa gat cgg aag cca ttt 35384
Arg Asn Met Asn Val Gly Ser Ser Leu Lys Asp Arg Lys Pro Phe
4990                4995                5000

cta ttt gaa cag agt ggt gat gtc aac atg gtt gtc gaa aaa cta 35429
Leu Phe Glu Gln Ser Gly Asp Val Asn Met Val Val Glu Lys Leu
5005                5010                5015

cta caa cat ggg cat gaa att agc aat gga tac gta caa aat gcg 35474
Leu Gln His Gly His Glu Ile Ser Asn Gly Tyr Val Gln Asn Ala
5020                5025                5030

gtg ggt acg ttg gat act gtt att acc ggt cat aca aat gtt ccc 35519
Val Gly Thr Leu Asp Thr Val Ile Thr Gly His Thr Asn Val Pro
5035                5040                5045

att tgg gta aca agg ccc ttg gtt atg cca gac gaa aag gat cca 35564
Ile Trp Val Thr Arg Pro Leu Val Met Pro Asp Glu Lys Asp Pro
5050                5055                5060

ttg gag ctt ttt att aac ctc acc att ttg cgt tta acg gga ttt 35609
Leu Glu Leu Phe Ile Asn Leu Thr Ile Leu Arg Leu Thr Gly Phe
5065                5070                5075

gtg gtg gaa aat gga aca cgt aca cat cat ggt gct aca agc gtt 35654
Val Val Glu Asn Gly Thr Arg Thr His His Gly Ala Thr Ser Val
5080                5085                5090

gta tca gac ttt ata ggt ccc ctt ggg gaa att tta aca gga ttt 35699
Val Ser Asp Phe Ile Gly Pro Leu Gly Glu Ile Leu Thr Gly Phe
5095                5100                5105

ccc tcc gcc gcg gaa ctt ata cgc gtt aca agt ttg ata tta aca 35744
Pro Ser Ala Ala Glu Leu Ile Arg Val Thr Ser Leu Ile Leu Thr
5110                5115                5120

aac atg ccg ggg gcg gaa tat gct att aaa act gtt ctc cgg aaa 35789
Asn Met Pro Gly Ala Glu Tyr Ala Ile Lys Thr Val Leu Arg Lys
5125                5130                5135

aaa tgt aca att ggc atg ctc att atc gct aag ttt ggt cta gtt 35834
Lys Cys Thr Ile Gly Met Leu Ile Ile Ala Lys Phe Gly Leu Val
5140                5145                5150

gcc atg cgg gtt cag gat aca acc ggc gct tta cat gcc gaa cta 35879
Ala Met Arg Val Gln Asp Thr Thr Gly Ala Leu His Ala Glu Leu
5155                5160                5165

gat gtg tta gaa gcg gat cta gga ggt tcg tcg ccc ata gac ctc 35924
Asp Val Leu Glu Ala Asp Leu Gly Gly Ser Ser Pro Ile Asp Leu
5170                5175                5180

tat tct aga ctg tcg aca ggt ctt ata agt ata cta aat tcg cct 35969
Tyr Ser Arg Leu Ser Thr Gly Leu Ile Ser Ile Leu Asn Ser Pro
5185                5190                5195

att att tct cat ccc gga ctt ttt gcc gag ctt att cca acc cgt 36014
Ile Ile Ser His Pro Gly Leu Phe Ala Glu Leu Ile Pro Thr Arg
5200                5205                5210

aca ggg tcc ctg tct gaa cga ata cgt ctt ctt tgt gaa tta gtc 36059
Thr Gly Ser Leu Ser Glu Arg Ile Arg Leu Leu Cys Glu Leu Val
5215                5220                5225

tcg gcc cgg gag aca cgc tat atg cgt gaa cac acc gcg ctt gtt 36104
Ser Ala Arg Glu Thr Arg Tyr Met Arg Glu His Thr Ala Leu Val
5230                5235                5240

tct agt gta aag gct tta gag aat gca tta cgg tct acc cgc aat 36149
Ser Ser Val Lys Ala Leu Glu Asn Ala Leu Arg Ser Thr Arg Asn
5245                5250                5255

aaa att gat gcc att caa ata cca gaa gtt ccc cag gaa ccc ccg 36194
Lys Ile Asp Ala Ile Gln Ile Pro Glu Val Pro Gln Glu Pro Pro
5260                5265                5270

gaa gaa acc gac att cca ccc gaa gag tta att cgg cgt gta tat 36239
Glu Glu Thr Asp Ile Pro Pro Glu Glu Leu Ile Arg Arg Val Tyr
5275                5280                5285

gag ata cga tcc gaa gtt aca atg cta ttg acc tcg gct gtt aca 36284
Glu Ile Arg Ser Glu Val Thr Met Leu Leu Thr Ser Ala Val Thr
5290                5295                5300

gaa tac ttc acc cgc gga gtg tta tat agc aca cgg gcc ttg atc 36329
Glu Tyr Phe Thr Arg Gly Val Leu Tyr Ser Thr Arg Ala Leu Ile
5305                5310                5315

gct gaa caa tcc cct agg cgt ttt cgg gtc gcg acc gca agt acg 36374
Ala Glu Gln Ser Pro Arg Arg Phe Arg Val Ala Thr Ala Ser Thr
5320                5325                5330

gca ccc att caa cgg ctt tta gat tct ctt ccg gaa ttc gac gct 36419
Ala Pro Ile Gln Arg Leu Leu Asp Ser Leu Pro Glu Phe Asp Ala
5335                5340                5345

aaa tta acg gca atc ata tcg tcc ctg tct ata cac cct cct cct 36464
Lys Leu Thr Ala Ile Ile Ser Ser Leu Ser Ile His Pro Pro Pro
5350                5355                5360

gag act ata caa aat ctc ccc gtc gta tct ctg tta aaa gag ctt 36509
Glu Thr Ile Gln Asn Leu Pro Val Val Ser Leu Leu Lys Glu Leu
5365                5370                5375

att aaa gaa ggg gaa gat tta aac aca gac acg gct ctc gta tcg 36554
Ile Lys Glu Gly Glu Asp Leu Asn Thr Asp Thr Ala Leu Val Ser
5380                5385                5390

tgg tta tct gta gtc ggg gaa gct caa acc gca ggt tac tta tcc 36599
Trp Leu Ser Val Val Gly Glu Ala Gln Thr Ala Gly Tyr Leu Ser
5395                5400                5405

aga cga gag ttc gat gaa tta tca cgt aca att aaa acc att aat 36644
Arg Arg Glu Phe Asp Glu Leu Ser Arg Thr Ile Lys Thr Ile Asn
5410                5415                5420

aca cgc gca acg caa cgg gct tcc gcg gaa gca gag ttg tct tgc 36689
Thr Arg Ala Thr Gln Arg Ala Ser Ala Glu Ala Glu Leu Ser Cys
5425                5430                5435

ttt aat acg cta agc gcg gcc gta gac caa gcc gta aag gac tat 36734
Phe Asn Thr Leu Ser Ala Ala Val Asp Gln Ala Val Lys Asp Tyr
5440                5445                5450

gaa aca tat aac aat ggt gag gtc aag tat cct gaa ata aca cgg 36779
Glu Thr Tyr Asn Asn Gly Glu Val Lys Tyr Pro Glu Ile Thr Arg
5455                5460                5465

gat gat tta tta gca aca att gta cgt gct aca gac gat ttg gtg 36824
Asp Asp Leu Leu Ala Thr Ile Val Arg Ala Thr Asp Asp Leu Val
5470                5475                5480

cga cag ata aaa att tta agt gat cca atg atc caa tcc ggt tta 36869
Arg Gln Ile Lys Ile Leu Ser Asp Pro Met Ile Gln Ser Gly Leu
5485                5490                5495

caa cct tcg att aaa aga cga ttg gaa aca agg ctt aaa gag gtt 36914
Gln Pro Ser Ile Lys Arg Arg Leu Glu Thr Arg Leu Lys Glu Val
5500                5505                5510

cag acg tat gca aac gag gcc cga acc aca cag gac aca ata aag 36959
Gln Thr Tyr Ala Asn Glu Ala Arg Thr Thr Gln Asp Thr Ile Lys
5515                5520                5525

agt cga aaa cag gcg gca tat aat aaa ctc ggg ggg tta ctt cgc 37004
Ser Arg Lys Gln Ala Ala Tyr Asn Lys Leu Gly Gly Leu Leu Arg
5530                5535                5540

ccg gta acc ggt ttt gtg gga ctt agg gct gca gta gat tta tta 37049
Pro Val Thr Gly Phe Val Gly Leu Arg Ala Ala Val Asp Leu Leu
5545                5550                5555

ccg gaa ctt gct tct gag tta gat gtc caa gga gcc ctg gta aat 37094
Pro Glu Leu Ala Ser Glu Leu Asp Val Gln Gly Ala Leu Val Asn
5560                5565                5570

ctc agg acc aaa gtc tta gag gcg ccg gta gag atc cgt tct caa 37139
Leu Arg Thr Lys Val Leu Glu Ala Pro Val Glu Ile Arg Ser Gln
5575                5580                5585

ctt acg ggt gat ttc tgg gcg tta ttt aac caa tat cga gac att 37184
Leu Thr Gly Asp Phe Trp Ala Leu Phe Asn Gln Tyr Arg Asp Ile
5590                5595                5600

tta gaa cat ccc gga aac gca cgc aca tct gtc tta gga gga ctg 37229
Leu Glu His Pro Gly Asn Ala Arg Thr Ser Val Leu Gly Gly Leu
5605                5610                5615

gga gct tgt ttt aca gct att atc gaa att gtg ccg ata cct acg 37274
Gly Ala Cys Phe Thr Ala Ile Ile Glu Ile Val Pro Ile Pro Thr
5620                5625                5630

gag tat aga cca tca ttg ctt gcg ttt ttt ggt gac gtg gca gat 37319
Glu Tyr Arg Pro Ser Leu Leu Ala Phe Phe Gly Asp Val Ala Asp
5635                5640                5645

gtg ctt gca tcc gac atc gcg acc gta tct act aac ccg gaa agt 37364
Val Leu Ala Ser Asp Ile Ala Thr Val Ser Thr Asn Pro Glu Ser
5650                5655                5660

gag tcc gcc ata aac gct gtt gtt gca act ctt agt aaa gcg acg 37409
Glu Ser Ala Ile Asn Ala Val Val Ala Thr Leu Ser Lys Ala Thr
5665                5670                5675

tta gtt tca tct aca gtg cca gcc tta tcc ttt gtg ttg tcg tta 37454
Leu Val Ser Ser Thr Val Pro Ala Leu Ser Phe Val Leu Ser Leu
5680                5685                5690

tat aaa aaa tat cag gct tta caa caa gaa att acg aat acc cat 37499
Tyr Lys Lys Tyr Gln Ala Leu Gln Gln Glu Ile Thr Asn Thr His
5695                5700                5705

aag ttg act gaa tta caa aaa caa ctt gga gat gac ttc tcc acc 37544
Lys Leu Thr Glu Leu Gln Lys Gln Leu Gly Asp Asp Phe Ser Thr
5710                5715                5720

cta gct gtc tca tct gga cac ttg aag ttt ata tca tct tca aat 37589
Leu Ala Val Ser Ser Gly His Leu Lys Phe Ile Ser Ser Ser Asn
5725                5730                5735

gta gat gat tat gaa ata aac gat gcg ata tta tca ata caa aca 37634
Val Asp Asp Tyr Glu Ile Asn Asp Ala Ile Leu Ser Ile Gln Thr
5740                5745                5750

aat gtg cac gcc cta atg gat acg gtt aaa ctt gtt gaa gtt gaa 37679
Asn Val His Ala Leu Met Asp Thr Val Lys Leu Val Glu Val Glu
5755                5760                5765

ctg caa aag cta ccc ccc cat tgt att gct ggg aca tct acc tta 37724
Leu Gln Lys Leu Pro Pro His Cys Ile Ala Gly Thr Ser Thr Leu
5770                5775                5780

tct cga gta gta aag gat ctt cat aaa ctc gtc aca atg gca cat 37769
Ser Arg Val Val Lys Asp Leu His Lys Leu Val Thr Met Ala His
5785                5790                5795

gag aag aag gaa cag gca aaa gtg tta att acc gat tgt gaa cgt 37814
Glu Lys Lys Glu Gln Ala Lys Val Leu Ile Thr Asp Cys Glu Arg
5800                5805                5810

gca cat aaa caa caa acg act cgg gtt ttg tat gag cgt tgg aca 37859
Ala His Lys Gln Gln Thr Thr Arg Val Leu Tyr Glu Arg Trp Thr
5815                5820                5825

cgt gat att ata gca tgt ctg gag gca atg gaa acg cgc cat ata 37904
Arg Asp Ile Ile Ala Cys Leu Glu Ala Met Glu Thr Arg His Ile
5830                5835                5840

ttt aac ggg aca gaa ctg gca cgg ttg cga gat atg gcc gct gcg 37949
Phe Asn Gly Thr Glu Leu Ala Arg Leu Arg Asp Met Ala Ala Ala
5845                5850                5855

gga ggg ttt gat ata cac gca gtt tac cca caa gca cgt cag gtt 37994
Gly Gly Phe Asp Ile His Ala Val Tyr Pro Gln Ala Arg Gln Val
5860                5865                5870

gta gcg gca tgt gaa act aca gcc gtt acg gca tta gat act gtg 38039
Val Ala Ala Cys Glu Thr Thr Ala Val Thr Ala Leu Asp Thr Val
5875                5880                5885

ttt cgc cac aat cca tat acc ccc gaa aat aca aat att cca cca 38084
Phe Arg His Asn Pro Tyr Thr Pro Glu Asn Thr Asn Ile Pro Pro
5890                5895                5900

cct ttg gct ttg tta aga ggg tta aca tgg ttt gat gat ttt tcg 38129
Pro Leu Ala Leu Leu Arg Gly Leu Thr Trp Phe Asp Asp Phe Ser
5905                5910                5915

att acg gct ccc gta ttc acc gtt atg ttt cca ggt gtt agt att 38174
Ile Thr Ala Pro Val Phe Thr Val Met Phe Pro Gly Val Ser Ile
5920                5925                5930

gag gga ctc ctt ctg ctt atg cgt att cgc gcg gtt gtg tta tta 38219
Glu Gly Leu Leu Leu Leu Met Arg Ile Arg Ala Val Val Leu Leu
5935                5940                5945

tcc gcc gat acg tct att aat gga ata cct aac tac cga gat atg 38264
Ser Ala Asp Thr Ser Ile Asn Gly Ile Pro Asn Tyr Arg Asp Met
5950                5955                5960

ata tta cga acc tcg ggg gat cta tta caa ata ccc gca ttg gct 38309
Ile Leu Arg Thr Ser Gly Asp Leu Leu Gln Ile Pro Ala Leu Ala
5965                5970                5975

ggg tat gtt gat ttt tac aca cgg tct tat gat cag ttt ata acc 38354
Gly Tyr Val Asp Phe Tyr Thr Arg Ser Tyr Asp Gln Phe Ile Thr
5980                5985                5990

gaa agt gta acg tta agt gaa ctt aga gca gac atc aga cag gct 38399
Glu Ser Val Thr Leu Ser Glu Leu Arg Ala Asp Ile Arg Gln Ala
5995                6000                6005

gcc ggg gct aaa ctt aca gaa gca aat aag gct ttg gag gaa gta 38444
Ala Gly Ala Lys Leu Thr Glu Ala Asn Lys Ala Leu Glu Glu Val
6010                6015                6020

act cat gtt cgg gca cac gaa acg gct aaa ctt gca ctt aaa gaa 38489
Thr His Val Arg Ala His Glu Thr Ala Lys Leu Ala Leu Lys Glu
6025                6030                6035

ggt gtc ttc att aca tta cca agc gaa ggt tta ttg att cgg gct 38534
Gly Val Phe Ile Thr Leu Pro Ser Glu Gly Leu Leu Ile Arg Ala
6040                6045                6050

ata gag tat ttt aca act ttc gat cat aaa cga ttt ata gga acg 38579
Ile Glu Tyr Phe Thr Thr Phe Asp His Lys Arg Phe Ile Gly Thr
6055                6060                6065

gca tat gaa aga gtt tta caa aca atg gta gac cgc gat cta aag 38624
Ala Tyr Glu Arg Val Leu Gln Thr Met Val Asp Arg Asp Leu Lys
6070                6075                6080

gag gcc aac gca gag ctt gca cag ttt cgt atg gtg tgt cag gca 38669
Glu Ala Asn Ala Glu Leu Ala Gln Phe Arg Met Val Cys Gln Ala
6085                6090                6095

aca aag aac cgt gca ata caa att tta caa aac att gtt gat acg 38714
Thr Lys Asn Arg Ala Ile Gln Ile Leu Gln Asn Ile Val Asp Thr
6100                6105                6110

gcc aat gcc act gag caa caa gaa gac gtg gat ttc act aac ctg 38759
Ala Asn Ala Thr Glu Gln Gln Glu Asp Val Asp Phe Thr Asn Leu
6115                6120                6125

aag acg tta tta aaa cta acc ccc cct ccc aaa aca att gca ttg 38804
Lys Thr Leu Leu Lys Leu Thr Pro Pro Pro Lys Thr Ile Ala Leu
6130                6135                6140

gcc att gat aga tct act tcc gtt cag gac att gtc acg cag ttt 38849
Ala Ile Asp Arg Ser Thr Ser Val Gln Asp Ile Val Thr Gln Phe
6145                6150                6155

gca ttg ctg tta ggg cgt ctg gaa gaa gaa act ggt acg ttg gac 38894
Ala Leu Leu Leu Gly Arg Leu Glu Glu Glu Thr Gly Thr Leu Asp
6160                6165                6170

att cag gcg gtt gac tgg atg tac caa gct cgc aat att att gac 38939
Ile Gln Ala Val Asp Trp Met Tyr Gln Ala Arg Asn Ile Ile Asp
6175                6180                6185

tcc cat cca cta agt gtg cgt ata gac ggt acc ggc ccc ctg cat 38984
Ser His Pro Leu Ser Val Arg Ile Asp Gly Thr Gly Pro Leu His
6190                6195                6200

act tat aaa gat agg gtg gat aaa ctt tat gcg tta cga act aaa 39029
Thr Tyr Lys Asp Arg Val Asp Lys Leu Tyr Ala Leu Arg Thr Lys
6205                6210                6215

tta gat ctc cta cga cga cga ata gaa acc ggt gag gtt acg tgg 39074
Leu Asp Leu Leu Arg Arg Arg Ile Glu Thr Gly Glu Val Thr Trp
6220                6225                6230

gac gat gca tgg aca aca ttt aaa aga gaa acg ggg gat atg ttg 39119
Asp Asp Ala Trp Thr Thr Phe Lys Arg Glu Thr Gly Asp Met Leu
6235                6240                6245

gca tcg ggg gac acg tac gct act tcc gta gat agt ata aag gca 39164
Ala Ser Gly Asp Thr Tyr Ala Thr Ser Val Asp Ser Ile Lys Ala
6250                6255                6260

ctc cag gca tcg gcg tct gtg gtt gac atg ctt tgt tcc gaa ccc 39209
Leu Gln Ala Ser Ala Ser Val Val Asp Met Leu Cys Ser Glu Pro
6265                6270                6275

gaa ttt ttt tta ttg cct gtg gaa acg aaa aac cgt ctc caa aaa 39254
Glu Phe Phe Leu Leu Pro Val Glu Thr Lys Asn Arg Leu Gln Lys
6280                6285                6290

aag caa cag gaa cgt aaa acg gcg ttg gat gtt gtg ttg caa aaa 39299
Lys Gln Gln Glu Arg Lys Thr Ala Leu Asp Val Val Leu Gln Lys
6295                6300                6305

caa aga cag ttt gaa gag acc gcg tct cgc tta cga gct tta att 39344
Gln Arg Gln Phe Glu Glu Thr Ala Ser Arg Leu Arg Ala Leu Ile
6310                6315                6320

gaa cgt att cca acg gag agt gac cat gac gtt ctt cgt atg tta 39389
Glu Arg Ile Pro Thr Glu Ser Asp His Asp Val Leu Arg Met Leu
6325                6330                6335

tta cgt gat ttc gat caa ttt aca cat ttg cct ata tgg ata aaa 39434
Leu Arg Asp Phe Asp Gln Phe Thr His Leu Pro Ile Trp Ile Lys
6340                6345                6350

aca cag tat atg aca ttt cga aat tta ctc atg gta cgg tta ggc 39479
Thr Gln Tyr Met Thr Phe Arg Asn Leu Leu Met Val Arg Leu Gly
6355                6360                6365

ttg tat gca agt tat gct gag att ttt cca ccc gcg tct cca aac 39524
Leu Tyr Ala Ser Tyr Ala Glu Ile Phe Pro Pro Ala Ser Pro Asn
6370                6375                6380

gga gta ttt gct cct att ccc gcc atg tcg ggt gta tgt cta gaa 39569
Gly Val Phe Ala Pro Ile Pro Ala Met Ser Gly Val Cys Leu Glu
6385                6390                6395

gac caa tcc cga tgc att cgc gcg cgg gtg gcc gcg ttt atg ggg 39614
Asp Gln Ser Arg Cys Ile Arg Ala Arg Val Ala Ala Phe Met Gly
6400                6405                6410

gag gcg tct gtg gtg caa acg ttt agg gaa gcc aga tct tct ata 39659
Glu Ala Ser Val Val Gln Thr Phe Arg Glu Ala Arg Ser Ser Ile
6415                6420                6425

gac gct ttg ttt gga aaa aat tta acc ttt tac ttg gat act gat 39704
Asp Ala Leu Phe Gly Lys Asn Leu Thr Phe Tyr Leu Asp Thr Asp
6430                6435                6440

ggg gtt cca ctt cga tat aga gtg tgt tat aaa tca gtt ggg gtt 39749
Gly Val Pro Leu Arg Tyr Arg Val Cys Tyr Lys Ser Val Gly Val
6445                6450                6455

aaa ctt gga acc atg cta tgc agt cag ggt gga tta tct tta cga 39794
Lys Leu Gly Thr Met Leu Cys Ser Gln Gly Gly Leu Ser Leu Arg
6460                6465                6470

ccg gca ctt ccc gat gaa ggt att gtg gaa gaa act aca cta tcg 39839
Pro Ala Leu Pro Asp Glu Gly Ile Val Glu Glu Thr Thr Leu Ser
6475                6480                6485

gca tta cgc gtg gcc aat gag gtc aat gag cta cgc att gaa tac 39884
Ala Leu Arg Val Ala Asn Glu Val Asn Glu Leu Arg Ile Glu Tyr
6490                6495                6500

gaa tcc gct ata aaa tcc ggg ttt tct gcc ttt tcc acc ttt gtt 39929
Glu Ser Ala Ile Lys Ser Gly Phe Ser Ala Phe Ser Thr Phe Val
6505                6510                6515

agg cat cgc cac gcc gaa tgg ggt aaa acc aac gca cgc aga gcc 39974
Arg His Arg His Ala Glu Trp Gly Lys Thr Asn Ala Arg Arg Ala
6520                6525                6530

att gca gag ata tac gcc ggc ctt ata aca aca aca ttg aca cga 40019
Ile Ala Glu Ile Tyr Ala Gly Leu Ile Thr Thr Thr Leu Thr Arg
6535                6540                6545

caa tac ggg gtt cat tgg gac aag ctt att tat tct ttt gaa aaa 40064
Gln Tyr Gly Val His Trp Asp Lys Leu Ile Tyr Ser Phe Glu Lys
6550                6555                6560

cac cac cta act tct gta atg ggc aat gga cta act aaa cca atc 40109
His His Leu Thr Ser Val Met Gly Asn Gly Leu Thr Lys Pro Ile
6565                6570                6575

cag aga agg ggt gat gta cgc gta tta gag tta acc cta tct gat 40154
Gln Arg Arg Gly Asp Val Arg Val Leu Glu Leu Thr Leu Ser Asp
6580                6585                6590

att gta act att ttg gtt gcc aca acc ccg gta cat ctt ctc aat 40199
Ile Val Thr Ile Leu Val Ala Thr Thr Pro Val His Leu Leu Asn
6595                6600                6605

ttt gct aga ttg gat tta att aaa cag cat gag tat atg gcc cgt 40244
Phe Ala Arg Leu Asp Leu Ile Lys Gln His Glu Tyr Met Ala Arg
6610                6615                6620

acc ctc aga ccc gta atc gag gcc gca ttt aga ggt cgt tta ctc 40289
Thr Leu Arg Pro Val Ile Glu Ala Ala Phe Arg Gly Arg Leu Leu
6625                6630                6635

gtt cgc tca ttg gat gga gac ccg aaa ggc aat gcc cgg gcc ttt 40334
Val Arg Ser Leu Asp Gly Asp Pro Lys Gly Asn Ala Arg Ala Phe
6640                6645                6650

ttt aat gcc gcc cca tcc aaa cat aaa ctc ccg tta gct ctt gga 40379
Phe Asn Ala Ala Pro Ser Lys His Lys Leu Pro Leu Ala Leu Gly
6655                6660                6665

tca aac caa gat cct acc ggc ggg aga ata ttt gca ttt cgg atg 40424
Ser Asn Gln Asp Pro Thr Gly Gly Arg Ile Phe Ala Phe Arg Met
6670                6675                6680

gca gat tgg aaa ctt gtt aaa atg cca cag aaa ata acg gat cct 40469
Ala Asp Trp Lys Leu Val Lys Met Pro Gln Lys Ile Thr Asp Pro
6685                6690                6695

ttt gcg cca tgg caa ctt tcc ccc ccc ccc ggg gta aag gcc aat 40514
Phe Ala Pro Trp Gln Leu Ser Pro Pro Pro Gly Val Lys Ala Asn
6700                6705                6710

gtc gat gca gtt acc cgt ata atg gca aca gat cgt ctt gcg acc 40559
Val Asp Ala Val Thr Arg Ile Met Ala Thr Asp Arg Leu Ala Thr
6715                6720                6725

att act gta ctt ggg cgc atg tgt ctc ccg cca att tcc tta gtg 40604
Ile Thr Val Leu Gly Arg Met Cys Leu Pro Pro Ile Ser Leu Val
6730                6735                6740

tca atg tgg aat acg ctg caa ccg gag gaa ttc gca tac aga aca 40649
Ser Met Trp Asn Thr Leu Gln Pro Glu Glu Phe Ala Tyr Arg Thr
6745                6750                6755

caa gat gat gtg gac att ata gtt gat gcg aga ctg gat ttg tca 40694
Gln Asp Asp Val Asp Ile Ile Val Asp Ala Arg Leu Asp Leu Ser
6760                6765                6770

tcc acg ctt aat gca aga ttt gat acc gct ccc agc aat acc acg 40739
Ser Thr Leu Asn Ala Arg Phe Asp Thr Ala Pro Ser Asn Thr Thr
6775                6780                6785

tta gag tgg aat aca gac cgt aaa gta att aca gat gct tat att 40784
Leu Glu Trp Asn Thr Asp Arg Lys Val Ile Thr Asp Ala Tyr Ile
6790                6795                6800

caa acc ggg gca acg aca gtt ttt aca gta acg ggg gcg gca cca 40829
Gln Thr Gly Ala Thr Thr Val Phe Thr Val Thr Gly Ala Ala Pro
6805                6810                6815

act cac gtt tct aat gta aca gcg ttt gac ata gca act acg gct 40874
Thr His Val Ser Asn Val Thr Ala Phe Asp Ile Ala Thr Thr Ala
6820                6825                6830

att tta ttt ggg gct cct ttg gtt att gcc atg gaa ctt aca tcc 40919
Ile Leu Phe Gly Ala Pro Leu Val Ile Ala Met Glu Leu Thr Ser
6835                6840                6845

gtt  ttt tca caa aat tcc  gga ctt act ttg ggg  tta aaa tta ttc     40964
Val  Phe Ser Gln Asn Ser  Gly Leu Thr Leu Gly  Leu Lys Leu Phe
6850                 6855                 6860

gat  tcc cgg cat atg gct  aca gat tcg ggt ata  tcc tca gcc gta     41009
Asp  Ser Arg His Met Ala  Thr Asp Ser Gly Ile  Ser Ser Ala Val
6865                 6870                 6875

tct  ccc gat att gtt tct  tgg ggg tta cgt tta  ctg cat atg gat     41054
Ser  Pro Asp Ile Val Ser  Trp Gly Leu Arg Leu  Leu His Met Asp
6880                 6885                 6890

cct  cac cca att gaa aat  gca tgt tta att gtc  caa cta gaa aaa     41099
Pro  His Pro Ile Glu Asn  Ala Cys Leu Ile Val  Gln Leu Glu Lys
6895                 6900                 6905

ctg  tcc gcg ctc att gca  aac aaa cct ctt aca  aac aat ccc ccg     41144
Leu  Ser Ala Leu Ile Ala  Asn Lys Pro Leu Thr  Asn Asn Pro Pro
6910                 6915                 6920

tgt  tta ctg cta ttg gac  gaa cat atg aat ccc  tct tat gtt tta     41189
Cys  Leu Leu Leu Leu Asp  Glu His Met Asn Pro  Ser Tyr Val Leu
6925                 6930                 6935

tgg  gaa cga aaa gac tcg  att cca gct ccg gat  tat gtg gtc ttt     41234
Trp  Glu Arg Lys Asp Ser  Ile Pro Ala Pro Asp  Tyr Val Val Phe
6940                 6945                 6950

tgg  ggg cca gaa tct ctt  att gat ttg ccg tac  atc gac tcc gat     41279
Trp  Gly Pro Glu Ser Leu  Ile Asp Leu Pro Tyr  Ile Asp Ser Asp
6955                 6960                 6965

gag  gac tct ttc ccc tcg  tgt ccc gat gat cca  ttt tac tcg caa     41324
Glu  Asp Ser Phe Pro Ser  Cys Pro Asp Asp Pro  Phe Tyr Ser Gln
6970                 6975                 6980

att  att gcc ggt tat gcg  ccc caa ggc ccc cca  aac ctc gac aca     41369
Ile  Ile Ala Gly Tyr Ala  Pro Gln Gly Pro Pro  Asn Leu Asp Thr
6985                 6990                 6995

act  gat ttt tac cca acg  gag cca cta ttt aag  tct ccc gtt caa     41414
Thr  Asp Phe Tyr Pro Thr  Glu Pro Leu Phe Lys  Ser Pro Val Gln
7000                 7005                 7010

gtt  gtt aga agt tcc aaa  tgt aaa aaa atg ccc  gtc cgg ccc gcg     41459
Val  Val Arg Ser Ser Lys  Cys Lys Lys Met Pro  Val Arg Pro Ala
7015                 7020                 7025

cag  ccc gcg cag ccc gcg  cag ccc gcg cag ccc  gcg cag acc gtc     41504
Gln  Pro Ala Gln Pro Ala  Gln Pro Ala Gln Pro  Ala Gln Thr Val
7030                 7035                 7040

cag  ccc gcg cag ccc ata  gaa ccg ggc aca caa  ata gtg gta caa     41549
Gln  Pro Ala Gln Pro Ile  Glu Pro Gly Thr Gln  Ile Val Val Gln
7045                 7050                 7055

aat  ttt aag aaa ccc caa  agc gta aaa aca acc  ctt agc caa aaa     41594
Asn  Phe Lys Lys Pro Gln  Ser Val Lys Thr Thr  Leu Ser Gln Lys
7060                 7065                 7070

gat  att ccc ttg tat gtg  gaa acc gaa tca gaa  acg gct gtg ctt     41639
Asp  Ile Pro Leu Tyr Val  Glu Thr Glu Ser Glu  Thr Ala Val Leu
7075                 7080                 7085

ata  cct aag caa tta acc  acc tcc att aaa aca  acc gtt tgt aaa     41684
Ile  Pro Lys Gln Leu Thr  Thr Ser Ile Lys Thr  Thr Val Cys Lys
7090                 7095                 7100

agt att acc cca cca aat aac caa ttg tcg gat tgg aaa aat aat 41729
Ser Ile Thr Pro Pro Asn Asn Gln Leu Ser Asp Trp Lys Asn Asn
7105                7110                7115

cca cag caa aac caa acg tta aac caa gcg ttc agt aaa cca ata 41774
Pro Gln Gln Asn Gln Thr Leu Asn Gln Ala Phe Ser Lys Pro Ile
7120                7125                7130

ctt gag att acc tcc att ccg aca gat gac tcg ata tct tac cgg 41819
Leu Glu Ile Thr Ser Ile Pro Thr Asp Asp Ser Ile Ser Tyr Arg
7135                7140                7145

act tgg att gaa aaa tca aat caa aca caa aaa cgg cat caa aat 41864
Thr Trp Ile Glu Lys Ser Asn Gln Thr Gln Lys Arg His Gln Asn
7150                7155                7160

gac cct cga atg tat aac tcc aaa aca gta ttc cac cct gta aat 41909
Asp Pro Arg Met Tyr Asn Ser Lys Thr Val Phe His Pro Val Asn
7165                7170                7175

aac caa tta cct tct tgg gtt gac acg gca gcc gat gcc ccc caa 41954
Asn Gln Leu Pro Ser Trp Val Asp Thr Ala Ala Asp Ala Pro Gln
7180                7185                7190

acg gac cta ttg aca aac tat aaa aca aga cag ccg tcg cca aac 41999
Thr Asp Leu Leu Thr Asn Tyr Lys Thr Arg Gln Pro Ser Pro Asn
7195                7200                7205

ttt ccg cgg gac gta cac aca tgg ggc gta tct tct aac ccg ttt 42044
Phe Pro Arg Asp Val His Thr Trp Gly Val Ser Ser Asn Pro Phe
7210                7215                7220

aac tca ccg aac aga gac cta tat caa agt gat ttt agt gaa cct 42089
Asn Ser Pro Asn Arg Asp Leu Tyr Gln Ser Asp Phe Ser Glu Pro
7225                7230                7235

tct gac ggc tat agc agt gag agt gaa aat tct atc gta cta agt 42134
Ser Asp Gly Tyr Ser Ser Glu Ser Glu Asn Ser Ile Val Leu Ser
7240                7245                7250

ctc gac gaa cat cgg tca tgt cgc gtt cct agg cac gta cgc gtt 42179
Leu Asp Glu His Arg Ser Cys Arg Val Pro Arg His Val Arg Val
7255                7260                7265

gtt aat gcc gat gta gtc acc ggt cga cgt tat gtc cga ggg acc 42224
Val Asn Ala Asp Val Val Thr Gly Arg Arg Tyr Val Arg Gly Thr
7270                7275                7280

gcc ttg gga gca ctg gca ctg tta agc cag gca tgt cgg cgt atg 42269
Ala Leu Gly Ala Leu Ala Leu Leu Ser Gln Ala Cys Arg Arg Met
7285                7290                7295

atc gac aac gtt aga tat aca cgt aaa ctt tta atg gac cac acg 42314
Ile Asp Asn Val Arg Tyr Thr Arg Lys Leu Leu Met Asp His Thr
7300                7305                7310

gaa gat ata ttt caa ggc ctg ggg tat gtt aaa ttg tta tta gat 42359
Glu Asp Ile Phe Gln Gly Leu Gly Tyr Val Lys Leu Leu Leu Asp
7315                7320                7325

gga aca tat ata taa agtagcgcct attaaagaaa aaaaaaaaac aacgattatt 42414
Gly Thr Tyr Ile
7330

ttctgtgtat ttttatttac accctacgac ttcttgaagc gtttccagat tgtcccgtgt 42474

gtgacaaggt ctgtccctta cccccctggg gggtattttg ggttgggggc ggggtagact 42534

gtggcacgcc ttgggccgcg ggcggtgatc cggttgttgg ctggacagtg cttgactgtg 42594

ctccctgttg cggttgttgt ccagaagacc ccgacaccac gtgttgctgt tgtccaacgg 42654

atgccgacgt cgtttgaggt ggggggtgtt gcggggatga tcccgaaaac gccaacgcgg 42714

cgggctgttg taaagcagac tgatcggcgc tctgtgtttt ttgcggcaat atagtaggcc 42774

ccgagattcc caaactcatg gatggatttg ggggttgtgg tcgtataata cgcgggttaa 42834

acgtacgttt taagccaacc gttggtctta accatgtcat agggtcagtc tcggcaaaca 42894

tggccgttcg gcgtatcgta tttgcattat ggttagcgcg tgcacgcgcg gcactggccg 42954

cggctcccac ggtgtaaatg cttctggcat cagcgatgtc cacacggtga ccaggttgca 43014

aaggtccact ggcgtttaaa agtcgtatta aagcaacggg ggtgtaagcc gcaattgctt 43074

ccaccgaaaa tgtggtgggg ttgctgggat caaagactac acgagacgat gcgggttgtg 43134

tcatcgttta ttagtttacg ggacaatcga taacagcata cacgtacatc tgcgcaggat 43194

atgtacggaa aggcaattta tttccagaaa agcaccgccc ctaatacaac taccagtaca 43254

attacaatga acagggcata tgtcacgtta gctacgggta gagcaagttt ccagacacgc 43314

gtagtttggg tatcgggtaa cgcaggttta atgtcacttt gcatttgaac agacgtgttt 43374

ggacttccgt tctcgggtgg ggatctgaat gaaggccgcc agcgtatata ttcatccaaa 43434

ttattgccag tttccttata catgtatgca tccgtggcgc gggccataag tttaatggtg 43494

cgagatggat cttccggtcc cataaaacga aaggataact gaacatatgg cattcgcaca 43554

aagcagttca cccacattaa agcctggaga ggtcggcggt caataccccc acctcgttta 43614

attgattcca aagcagatag gttgataccg gtacttaacg ttgaactaag aatcacgtta 43674

ttactgtcaa tggacacttc agccactggt gcgttagtcg gacgaaaaaa aaaaccttga 43734

aatagcacag acacccccgt attttgaatt tttatgtaag ggtcacaatc tacttgcgcc 43794

caattcgcca ttaaacgcat aatatactct accggaaagg cttcggatac gttgtcttcg 43854

ccgttaaact gaaaaacaca acgggcgggg gggcgttgtg gatcaaatat tggaagatcc 43914

ccatcgcaac attgaagagc gcttggtacc accaaccgaa tacgttgtaa aagattatct 43974

ccgcaacccc tcctgcgttc actccgtaca tacgttctcc gtgacatatt gatctaaggt 44034

tgcaaaccaa ggcacacgcg tgaagtattt agaccattta tcgtgggata taggaggagt 44094

ttggagtgat ccaccccctg acgacttatt aatgcgttta ttttccccat gtattaagca 44154

tccttcaata tttcatgcaa atctagaaat ttggccatga ctcccgcaaa gcgttcacgg 44214

cgacgggtca cgctggcact atgttcacat ggaacaacat aagcagattt ttctgaatcg 44274

ttactttctt tatgttttaa aacggacgcc aggcgactgg taaatgatat ataatttaat 44334

tgagcgtcag ttgtaggtag aattgcttct atttccgggg gaattaaatt ttcaaaccaa 44394

acggaaagag taaaggtgct atcagcagga aaatactttg actccagtgc atcgatattt 44454

aatagattaa catcggtgtc tgtaattaaa tcgcgggccc tcatcccaga g atg gat 44511
Met Asp
    7335

cgg gta gaa tca gaa gaa ccc atg gat gga ttc gaa tcg ccc gta 44556
Arg Val Glu Ser Glu Glu Pro Met Asp Gly Phe Glu Ser Pro Val
                7340                7345                7350

ttc tcc gaa aat aca tct tct aat tcc gga tgg tgt tcc gac gca 44601
Phe Ser Glu Asn Thr Ser Ser Asn Ser Gly Trp Cys Ser Asp Ala
                7355                7360                7365

ttt tcc gat tcg tac atc gct tat aat cca gcc ctt ctg cta aaa 44646
Phe Ser Asp Ser Tyr Ile Ala Tyr Asn Pro Ala Leu Leu Leu Lys
                7370                7375                7380

aac gat ttg tta ttt tca gaa ttg tta ttt gcc tcc cac tta ata 44691
Asn Asp Leu Leu Phe Ser Glu Leu Leu Phe Ala Ser His Leu Ile
                7385                7390                7395

aat gtt ccc cgt gca ata gaa aac aac gtc act tat gag gcc tct 44736
Asn Val Pro Arg Ala Ile Glu Asn Asn Val Thr Tyr Glu Ala Ser
                7400                7405                7410

tcg gcg gta ggt gtg gat aat gaa atg acc tca agt acc act gaa 44781
Ser Ala Val Gly Val Asp Asn Glu Met Thr Ser Ser Thr Thr Glu
                7415                7420                7425

ttt ata gaa gaa att gga gac gtt ttg gcg tta gac aga gcc tgt 44826
Phe Ile Glu Glu Ile Gly Asp Val Leu Ala Leu Asp Arg Ala Cys
                7430                7435                7440

ttg gtc tgc aga acg ctt gat ttg tat aaa cgt aaa ttt gga ctg 44871
Leu Val Cys Arg Thr Leu Asp Leu Tyr Lys Arg Lys Phe Gly Leu
                7445                7450                7455

aca ccg gaa tgg gtt gcg gac tac gcc atg tta tgt atg aaa agt 44916
Thr Pro Glu Trp Val Ala Asp Tyr Ala Met Leu Cys Met Lys Ser
                7460                7465                7470

ctg gca tcc ccg ccc tgt gca gtt gtc act ttt agc gct gcc ttt 44961
Leu Ala Ser Pro Pro Cys Ala Val Val Thr Phe Ser Ala Ala Phe
                7475                7480                7485

gaa ttt gtg tat ctt atg gat cgt tac tac ctg tgc cgt tat aac 45006
Glu Phe Val Tyr Leu Met Asp Arg Tyr Tyr Leu Cys Arg Tyr Asn
                7490                7495                7500

gtt act ttg gtt ggg tcc ttt gcc agg cgc acg ctt tcc ctg tta 45051
Val Thr Leu Val Gly Ser Phe Ala Arg Arg Thr Leu Ser Leu Leu
                7505                7510                7515

gat ata caa aga cat ttt ttt ttg cat gta tgt ttt cgt acc gat 45096
Asp Ile Gln Arg His Phe Phe Leu His Val Cys Phe Arg Thr Asp
                7520                7525                7530

gga ggg tta cca ggt ata cga ccg ccc ccc ggt aag gaa atg gcc 45141
Gly Gly Leu Pro Gly Ile Arg Pro Pro Pro Gly Lys Glu Met Ala
                7535                7540                7545

aac aaa gta aga tat tcc aat tac tcc ttt ttt gta cag gcg gta 45186
Asn Lys Val Arg Tyr Ser Asn Tyr Ser Phe Phe Val Gln Ala Val
                7550                7555                7560

gtt agg gct gca tta cta tcg atc agc acg tct cgt tta gac gaa 45231
Val Arg Ala Ala Leu Leu Ser Ile Ser Thr Ser Arg Leu Asp Glu
                7565                7570                7575

acc gaa acg cgt aag tca ttt tac ttt aat cag gac gga ctg act 45276
Thr Glu Thr Arg Lys Ser Phe Tyr Phe Asn Gln Asp Gly Leu Thr
                7580                7585                7590

gga ggc cct caa cct tta gcg gcc gcc ttg gct aat tgg aaa gat 45321
Gly Gly Pro Gln Pro Leu Ala Ala Ala Leu Ala Asn Trp Lys Asp
                7595                7600                7605

tgc gcg cgg atg gtt gac tgt tca tca tcg gaa cat cgc aca agt 45366
Cys Ala Arg Met Val Asp Cys Ser Ser Ser Glu His Arg Thr Ser
                7610                7615                7620

ggg atg att acc tgc gcg gaa cgt gca tta aaa gag gat ata gag 45411
Gly Met Ile Thr Cys Ala Glu Arg Ala Leu Lys Glu Asp Ile Glu
                7625                7630                7635

ttt gaa gat ata tta ata gac aaa ctt aaa aaa tcg tct tac gta 45456
Phe Glu Asp Ile Leu Ile Asp Lys Leu Lys Lys Ser Ser Tyr Val
                7640                7645                7650

gaa gca gct tgg ggt tac gca gac ttg gct tta tta tta ctg agt 45501
Glu Ala Ala Trp Gly Tyr Ala Asp Leu Ala Leu Leu Leu Leu Ser
                7655                7660                7665

ggg gtt gct act tgg aat gta gac gag cgt aca aat tgt gct ata 45546
Gly Val Ala Thr Trp Asn Val Asp Glu Arg Thr Asn Cys Ala Ile
                7670                7675                7680

gaa act cgc gtt gga tgt gtt aaa tca tac tgg cag gcg aac cgg 45591
Glu Thr Arg Val Gly Cys Val Lys Ser Tyr Trp Gln Ala Asn Arg
                7685                7690                7695

att gaa aac tcc agg gac gtt cca aaa caa ttt tcc aaa ttt acg 45636
Ile Glu Asn Ser Arg Asp Val Pro Lys Gln Phe Ser Lys Phe Thr
                7700                7705                7710

agc gag gat gcc tgt ccc gaa gta gca ttt ggg cct att ttg tta 45681
Ser Glu Asp Ala Cys Pro Glu Val Ala Phe Gly Pro Ile Leu Leu
                7715                7720                7725

act acc tta aaa aac gca aag tgc cgt ggt cgc acg aat acc gaa 45726
Thr Thr Leu Lys Asn Ala Lys Cys Arg Gly Arg Thr Asn Thr Glu
                7730                7735                7740

tgc atg tta tgt tgt tta tta acc ata ggg cac tat tgg atc gct 45771
Cys Met Leu Cys Cys Leu Leu Thr Ile Gly His Tyr Trp Ile Ala
                7745                7750                7755

ttg cgg cag ttt aaa agg gat ata tta gca tac tca gca aat aac 45816
Leu Arg Gln Phe Lys Arg Asp Ile Leu Ala Tyr Ser Ala Asn Asn
                7760                7765                7770

aca agt tta ttt gac tgt atc gaa cct gta atc aat gca tgg agc 45861
Thr Ser Leu Phe Asp Cys Ile Glu Pro Val Ile Asn Ala Trp Ser
                7775                7780                7785

cta gat aac ccc att aaa ctt aaa ttt cca ttt aat gat gag ggt 45906
Leu Asp Asn Pro Ile Lys Leu Lys Phe Pro Phe Asn Asp Glu Gly
                7790                7795                7800

cga ttc ata acc att gta aaa gca gca ggt tcc gag gcc gta tat 45951
Arg Phe Ile Thr Ile Val Lys Ala Ala Gly Ser Glu Ala Val Tyr
                7805                7810                7815

aaa cat tta ttt tgc gat ctc cta tgc gct ctc tcg gaa tta cag 45996
Lys His Leu Phe Cys Asp Leu Leu Cys Ala Leu Ser Glu Leu Gln
                7820                7825                7830

aca aac cct aaa att tta ttt gcc cat cct aca acc gcg gat aag 46041
Thr Asn Pro Lys Ile Leu Phe Ala His Pro Thr Thr Ala Asp Lys
                7835                7840                7845

gaa gtg ttg gag tta tat aaa gcc caa ctg gct gca caa aac aga 46086
Glu Val Leu Glu Leu Tyr Lys Ala Gln Leu Ala Ala Gln Asn Arg
                7850                7855                7860

ttt gaa ggt cgt gta tgt gct ggc ctg tgg aca ttg gcg tat gca 46131
Phe Glu Gly Arg Val Cys Ala Gly Leu Trp Thr Leu Ala Tyr Ala
                7865                7870                7875

ttt aaa gcc tac cag att ttt cca cgc aaa cca acc gcc aat gcc 46176
Phe Lys Ala Tyr Gln Ile Phe Pro Arg Lys Pro Thr Ala Asn Ala
                7880                7885                7890

gca ttc ata cga gat gga gga ctt atg ctt cga cga cat gca ata 46221
Ala Phe Ile Arg Asp Gly Gly Leu Met Leu Arg Arg His Ala Ile
                7895                7900                7905

tcg ctg gtc tcc ctc gaa cac acc cta tcg aag tat gtc tag 46263
Ser Leu Val Ser Leu Glu His Thr Leu Ser Lys Tyr Val
                7910                7915

gcgatataaa tccgtatctc ggagcgggcc ttcgatgcgt gtacgctcca gaacgccatg 46323

ccgccgtcaa accattcgag gaaaacttat gtcaaaggag cggtctgtgt accgccatta 46383

ttttaattac atcgcaaggt cccccccaga agaactagct accgttagag gcttaatcgt 46443

gccaattatt aagacgaccc ctgtcaccct tccgtttaac ttgggtcaga cagtggcgga 46503

taactgcctg tcgttatccg gaatgggtta tcatttaggt ctcggaggtt attgtccgac 46563

atgcactgca tctggagaac cgcgtctatg tcgaaccgat cgggcggctc tgatactagc 46623

atatgttcag cagcttaaca acatatacga atatcgtgtg tttcttgcat ccattttggc 46683

gctatcagac cgagccaaca tgcaagcagc gtccgctgaa cccctattgt cgagcgtatt 46743

ggcacaaccg gaattatttt ttatgtatca tattatgagg gaggggggca tgcgagatat 46803

acgcgtactt ttttatcgtg atggagatgc cggagggttt atgatgtatg ttatatttcc 46863

ggggaaatct gttcacctcc attacagact aatcgatcat atacaggccg cgtgtcgggg 46923

gtataaaata gtcgcacacg tttggcagac aacattttta ctgtcggtat gtcgcaaccc 46983

agaacaacaa acagagactg tggtgccatc cattggaaca tcggacgttt actgtaaaat 47043

gtgtgacctt aactttgatg gagaattgct tttggaatac aaaagactct acgcattatt 47103

tgatgacttt gttcctcctc ggtgatttca gcttcagtgt tcattttatt atcccagcac 47163

ggggcgtgta tacaaacaaa gcctgccgcc tgcaagcggt ttagcatttt aacgttaaca 47223

actcgtgtct ctggaataaa acgttttaaa agccgttctg tgagtttagt gtcgtttcca 47283

aataacgcct taaaagttac actcgccgtc ccaatgagat gagaaaaata atagtcaatg 47343

tttaaagaca gcccgtgtga tgttacgtga atgggatctt ccgctaagtc agatattatt 47403

aacttacgct ttgcttcccc acaccgttta cctgcggtat tctgtaaagg atctccacgt 47463

agcaaagcta cactttttgc atcagcctcc acttcgtctg tgggggccac aataacataa 47523

gggatgcgtt ctcgaacgtt tgggatttga ccctgtctca ttactaattt ataatatact 47583

gttaagtgag ccaagcgacg gtttatgtag gcggatggtg gacgactaag ctcggccgtc 47643

ataacaaact tattaatatc caatttgggt gatgtaatct ggcgatgtgc atctgcaatt 47703

atgcgtccaa acccggccat cccagacggc atggcccgtc tattccattc agcaatggaa 47763
acacacgacg cctccgccgc agcacgcgag acggtgtcgt catataacaa cagttctaca 47823
agtttgcggg cataatcgtt aataaattga cagttgtttt ttctaaccaa gtcgactccc 47883
ttcattaaaa cctttccgcc gtaaattacc ccaatgtact ttttctttgt tataagcaaa 47943
agttttataa aagttttttc acactccaac tttataggag gacaaaacag agccgttgaa 48003
attatatgtg ccattttctc gccgatttta gctatcccct caacactaac acccttgaat 48063
cggataaaca cagaatccgt atctccatat ataaccttta cctcgtacgc tttttgggag 48123
agaacgctac tttcaatgtc tggaaacgct gtaataaaac gttcaaatgc ggcccagtta 48183
ttatgaatat aatctctggt acttaataac atttgacggc caattgtagt gacagtggcc 48243
gctacgtata aacatggcag aaatccctgc gcaactccag taaaaccgta cacggaatta 48303
caaactactt ttatcgcggc ttgttgtttg tctaataaca ctgcttcatc tgaagaactt 48363
ccgggtatgc gcgctctaat agccttgcgc atagccaacc agtcttttaa aagaacaccc 48423
agcagacttt ctcgaacgtt agagcgcaca aaaaaaagac gttttcctcc aactgtaaag 48483
gtggcataat cggatggatt caaacgttta accgtctcaa aatttaacgt tagcgtggta 48543
aaacataagt tatgggcctg aattatactt ggatataaac ttgcaaaatc caatacgacc 48603
accggatcga tataaaatcc cgtatcaggg tcaaaaaccc tggctccttt atatcctaca 48663
tttcgcccac ttgacgtacc agtgggagaa acgctctcgt cttcatccat ctcttcctca 48723
acatccccga catcgggaat aacatcctta tattcaaaag tagctgggta tcccccatcg 48783
ggtaaaataa atcctcgaga cgaagccagt cctaataaac aggtgtaaat cctaacctgc 48843
tgtccgtcgt aaatagcctt ggttaaagta attctagcta gccttgcaac cgcggataac 48903
tcaaggtgtg gtaaatattt aaaaaacagt ttccccacaa gagccgagtc ttgtatacaa 48963
tattcaccaa taattcctcg tgtattcggt ccactagcgt aatatcccgg aatgtctttg 49023
tagggcaaat ctctcttgga ctcatttaga gcttcacgtg caaccgaatc taatttataa 49083
ctcgagagtt ttaatttttc agttgcaatt gcatacatat ccagagatat gagaccgttg 49143
atctttacct tgcttcgtcg ctgaaatccg gatttgccaa catcccatat cttaaacaga 49203
cccccacggt ttatactgcc ataaccatca agcttgagac tgtatataga attaagtttc 49263
tccataataa acgcccaatc aaaattaaca atgttataac ctgtggcaaa ctcgggagcg 49323
tactgtttta cgagggtcat aaatgcaatt aatagctcga attcactatc aaactccagc 49383
acagtcggct ccggtaaccc cgcgtccttc atttcttgta catacctttg tggtaagtca 49443
caagagccaa gggaaaacag taaaatgtgt tctaaagact gtcgagggat tgaatataat 49503
agacaagaaa tttggattac aagatcctcc agatgtgttg catcgggaaa cgccagctca 49563
ttagatcctc ctgatttaca ttcaatatcg aaacataaca acttgtagtc aggccatgag 49623
tcatcgtttg gtatagcctg cagattatcc gacatgcagt caatttcaac gtcgcttaac 49683
gttaattggc gacttgccgg tcgaactcga acacgttccc catcaactcc aggttttagt 49743

tgataccaac caaaactaac aaagccggga ttatccatta gaaaacgagt ggtagcgtct 49803

acccgacctt catacttttt caactccggg tgaaagttat cacaaagata atttgtaaat 49863

ttagatgagg gagaatacac cctgtaaaac gcacatggct gtgtatcgta gtaataaaca 49923

tctgtgcgct caataacctc aacgcgaaag ctttctggag atgcgctttt aaacgaggta 49983

ccatgaaaag cgttcttgtc tccatttaac gttgcatcat tttgtgttat catagaactg 50043

cgtaaacact cggcaagtaa tacagataac tcgctaccgg aacgtatgcc acaagcggta 50103

tccacctcgg ctttgtttat ataaaaatat tgacagatgc cgtatacatg aactgccacc 50163

ctttttccac atcgggacat gccaagtaaa gtaataacgg taccaagcgg tcgtgttgca 50223

gttgcaaacc gggatacatc tccattagac gcggcttctg ttgtttcgac aatatcatat 50283

acatggaatg tgttaaagcg ggggtcaaac ttatccccac gaaagtcgat ttccccccaa 50343

atattcacgc gtctaggcca ggggctggaa caacgaaaat ccagaatcgg aacttctttt 50403

ccattacagt aaactttagg cggtcgacta agtgtaccga cgtgaacccc ctttcgttct 50463

tccatgggca catcttcatc taaacattta ggggccaaaa attgaaacga tgacatggta 50523

gttttgtaac tatgaagaaa ttctctgtta ctaccgcgcc cggttcttgg gttatattta 50583

atccctgatg cttgggttaa aaagggatta caaaaccccg ttctgatcgc cattttatgt 50643

taacgattga taatcttgta aaaagccagt gttactgagt aacacaaccc cacgcccttc 50703

taatacataa agtgtaatca cgtgatttgt tgtggtttcc gcatatgtaa tacccgttta 50763

aaagcctctc ttcttaatgt atcgacagac tgggttttgg gtggtcattt gaccctgcca 50823

acaaccccc attattacga gtacttcacc aaa atg gaa aat act cag aag 50874
Met Glu Asn Thr Gln Lys
    7920

act gtg aca gtg ccc acg ggg ccc ctg ggt tac gtt tat gcg tgc 50919
Thr Val Thr Val Pro Thr Gly Pro Leu Gly Tyr Val Tyr Ala Cys
7925                7930                7935

cgg gtt gaa gat ttg gat ctg gag gaa att tca ttt ttg gcc gct 50964
Arg Val Glu Asp Leu Asp Leu Glu Glu Ile Ser Phe Leu Ala Ala
7940                7945                7950

cgt agc acg gac tct gat ttg gct tta tta cct ttg atg cgt aat 51009
Arg Ser Thr Asp Ser Asp Leu Ala Leu Leu Pro Leu Met Arg Asn
7955                7960                7965

ttg acc gtg gaa aaa act ttt aca tcc agc ctg gcg gtg gtt tct 51054
Leu Thr Val Glu Lys Thr Phe Thr Ser Ser Leu Ala Val Val Ser
7970                7975                7980

gga gca cgc act acg ggt ctt gcc gga gct ggt att acc tta aaa 51099
Gly Ala Arg Thr Thr Gly Leu Ala Gly Ala Gly Ile Thr Leu Lys
7985                7990                7995

ctc act acc agt cat ttc tat cca tct gtc ttt gtc ttt cac gga 51144
Leu Thr Thr Ser His Phe Tyr Pro Ser Val Phe Val Phe His Gly
8000                8005                8010

ggc aaa cac gtt tta ccc agc tcc gcg gcc cca aat ctc aca cgc 51189
Gly Lys His Val Leu Pro Ser Ser Ala Ala Pro Asn Leu Thr Arg
8015                8020                8025

gcg tgt aac gcg gct cga gaa cgg ttt ggg ttt tca cgc tgc caa 51234
Ala Cys Asn Ala Ala Arg Glu Arg Phe Gly Phe Ser Arg Cys Gln
8030                8035                8040

ggg cct cct gtt gac ggt gct gtt gag acg acc ggc gct gag ata 51279
Gly Pro Pro Val Asp Gly Ala Val Glu Thr Thr Gly Ala Glu Ile
8045                8050                8055

tgc acc cgc ctt gga tta gag cca gaa aat aca ata tta tac ttg 51324
Cys Thr Arg Leu Gly Leu Glu Pro Glu Asn Thr Ile Leu Tyr Leu
8060                8065                8070

gtg gtc acg gca ttg ttt aag gaa gcc gta ttt atg tgc aac gtg 51369
Val Val Thr Ala Leu Phe Lys Glu Ala Val Phe Met Cys Asn Val
8075                8080                8085

ttt ctg cat tat gga gga ctc gat att gtt cat att aac cat ggg 51414
Phe Leu His Tyr Gly Gly Leu Asp Ile Val His Ile Asn His Gly
8090                8095                8100

gat gtt ata cgt ata ccg tta ttt ccg gta caa ctt ttc atg ccc 51459
Asp Val Ile Arg Ile Pro Leu Phe Pro Val Gln Leu Phe Met Pro
8105                8110                8115

gat gtt aac cgt ctg gta ccc gac cca ttc aac act cat cac agg 51504
Asp Val Asn Arg Leu Val Pro Asp Pro Phe Asn Thr His His Arg
8120                8125                8130

tct atc gga gag ggt ttt gta tac cca aca ccc ttt tat aac acc 51549
Ser Ile Gly Glu Gly Phe Val Tyr Pro Thr Pro Phe Tyr Asn Thr
8135                8140                8145

ggg ttg tgc cat tta ata cat gac tgt gtt att gct ccc atg gcc 51594
Gly Leu Cys His Leu Ile His Asp Cys Val Ile Ala Pro Met Ala
8150                8155                8160

gtt gcc ttg cgc gtc aga aat gta act gcc gtc gcc cga gga gcg 51639
Val Ala Leu Arg Val Arg Asn Val Thr Ala Val Ala Arg Gly Ala
8165                8170                8175

gcc cac ctt gct ttt gat gaa aat cac gag ggg gca gta ctc ccc 51684
Ala His Leu Ala Phe Asp Glu Asn His Glu Gly Ala Val Leu Pro
8180                8185                8190

cct gac att acg tac acg tat ttt cag tcc tct tca agt gga acc 51729
Pro Asp Ile Thr Tyr Thr Tyr Phe Gln Ser Ser Ser Ser Gly Thr
8195                8200                8205

act acc gcc cgt gga gcg cgt cga aac gat gtc aac tcc acg tct 51774
Thr Thr Ala Arg Gly Ala Arg Arg Asn Asp Val Asn Ser Thr Ser
8210                8215                8220

aag cct agc cca tcg ggg ggg ttt gaa aga cgg ttg gcg tct att 51819
Lys Pro Ser Pro Ser Gly Gly Phe Glu Arg Arg Leu Ala Ser Ile
8225                8230                8235

atg gcc gct gac aca gcc ttg cac gca gaa gtt ata ttc aac act 51864
Met Ala Ala Asp Thr Ala Leu His Ala Glu Val Ile Phe Asn Thr
8240                8245                8250

gga att tac gaa gaa act cca aca gat atc aaa gaa tgg cca atg 51909
Gly Ile Tyr Glu Glu Thr Pro Thr Asp Ile Lys Glu Trp Pro Met
8255                8260                8265

ttt ata ggc atg gag ggc act ttg cca agg cta aac gct ctg ggg 51954
Phe Ile Gly Met Glu Gly Thr Leu Pro Arg Leu Asn Ala Leu Gly
8270                8275                8280

tca tat acc gct cgt gtg gcc ggg gtc att ggt gcg atg gtt ttc 51999
Ser Tyr Thr Ala Arg Val Ala Gly Val Ile Gly Ala Met Val Phe
8285                8290                8295

agc cca aat tct gcg ttg tat cta act gag gtg gag gat agc ggg 52044
Ser Pro Asn Ser Ala Leu Tyr Leu Thr Glu Val Glu Asp Ser Gly
8300                8305                8310

atg acc gaa gcc aag gat ggg gga ccg ggt cca tca ttt aat cga 52089
Met Thr Glu Ala Lys Asp Gly Gly Pro Gly Pro Ser Phe Asn Arg
8315                8320                8325

ttt tac cag ttt gcc gga cct cat tta gct gcg aat ccc caa aca 52134
Phe Tyr Gln Phe Ala Gly Pro His Leu Ala Ala Asn Pro Gln Thr
8330                8335                8340

gat cga gat ggc cac gtt cta tcc agt cag tct acg ggt tca tca 52179
Asp Arg Asp Gly His Val Leu Ser Ser Gln Ser Thr Gly Ser Ser
8345                8350                8355

aac aca gag ttt agc gtg gat tat ttg gca ctc att tgt gga ttt 52224
Asn Thr Glu Phe Ser Val Asp Tyr Leu Ala Leu Ile Cys Gly Phe
8360                8365                8370

gga gca ccc ctg ttg gcg cga ctg ctt ttt tat cta gaa cgc tgt 52269
Gly Ala Pro Leu Leu Ala Arg Leu Leu Phe Tyr Leu Glu Arg Cys
8375                8380                8385

gac gct ggt gcg ttt aca ggg ggt cac ggg gat gcg tta aaa tat 52314
Asp Ala Gly Ala Phe Thr Gly Gly His Gly Asp Ala Leu Lys Tyr
8390                8395                8400

gtt acg ggg acc ttt gac tct gaa att cca tgt agt tta tgt gaa 52359
Val Thr Gly Thr Phe Asp Ser Glu Ile Pro Cys Ser Leu Cys Glu
8405                8410                8415

aaa cac acg cgg ccg gta tgc gct cac aca aca gta cac cga ctt 52404
Lys His Thr Arg Pro Val Cys Ala His Thr Thr Val His Arg Leu
8420                8425                8430

aga caa cgc atg ccg cga ttt gga caa gcc acc cgt caa cct att 52449
Arg Gln Arg Met Pro Arg Phe Gly Gln Ala Thr Arg Gln Pro Ile
8435                8440                8445

ggg gtg ttt gga aca atg aac agc caa tat agc gac tgc gat cct 52494
Gly Val Phe Gly Thr Met Asn Ser Gln Tyr Ser Asp Cys Asp Pro
8450                8455                8460

cta gga aac tat gct cca tat tta atc ctt cga aaa ccc ggg gat 52539
Leu Gly Asn Tyr Ala Pro Tyr Leu Ile Leu Arg Lys Pro Gly Asp
8465                8470                8475

caa acg gaa gca gca aag gca acc atg cag gac act tat agg gct 52584
Gln Thr Glu Ala Ala Lys Ala Thr Met Gln Asp Thr Tyr Arg Ala
8480                8485                8490

aca cta gaa cgc ttg ttt atc gat cta gaa caa gag cga cta ctg 52629
Thr Leu Glu Arg Leu Phe Ile Asp Leu Glu Gln Glu Arg Leu Leu
8495                8500                8505

gat cgc ggt gcc cca tgt tct tcc gag gga cta tcg tct gtc att 52674
Asp Arg Gly Ala Pro Cys Ser Ser Glu Gly Leu Ser Ser Val Ile
8510                8515                8520

gtg gat cat cca acg ttt cgt cgc ata tta gac aca ctg cgt gcg 52719
Val Asp His Pro Thr Phe Arg Arg Ile Leu Asp Thr Leu Arg Ala
8525                8530                8535

cgt ata gaa cag aca aca aca caa ttt atg aaa gtg ttg gtt gag 52764
Arg Ile Glu Gln Thr Thr Thr Gln Phe Met Lys Val Leu Val Glu
8540                8545                8550

acc cgc gat tat aag atc cgt gaa gga tta tcc gaa gcc acc cat 52809
Thr Arg Asp Tyr Lys Ile Arg Glu Gly Leu Ser Glu Ala Thr His
8555                8560                8565

tca atg gcg tta acg ttt gat cca tac tca gga gca ttt tgt ccc 52854
Ser Met Ala Leu Thr Phe Asp Pro Tyr Ser Gly Ala Phe Cys Pro
8570                8575                8580

att acc aat ttt tta gtt aaa cga aca cac cta gcc gtg gta caa 52899
Ile Thr Asn Phe Leu Val Lys Arg Thr His Leu Ala Val Val Gln
8585                8590                8595

gac tta gca tta agc caa tgt cat tgt gta ttt tac gga cag caa 52944
Asp Leu Ala Leu Ser Gln Cys His Cys Val Phe Tyr Gly Gln Gln
8600                8605                8610

gtt gag ggg cgg aac ttt cgt aac caa ttc caa cct gtt ttg cgg 52989
Val Glu Gly Arg Asn Phe Arg Asn Gln Phe Gln Pro Val Leu Arg
8615                8620                8625

cgg cgt ttt gtt gac ctg ttt aat ggg ggg ttt ata tca aca cgc 53034
Arg Arg Phe Val Asp Leu Phe Asn Gly Gly Phe Ile Ser Thr Arg
8630                8635                8640

tct ata acc gta aca tta tct gaa ggt cct gta tcc gcc cca aat 53079
Ser Ile Thr Val Thr Leu Ser Glu Gly Pro Val Ser Ala Pro Asn
8645                8650                8655

ccg aca ttg gga caa gac gcg ccc gcg ggg cgt acc ttt gat ggg 53124
Pro Thr Leu Gly Gln Asp Ala Pro Ala Gly Arg Thr Phe Asp Gly
8660                8665                8670

gat tta gcg cgc gta agc gtg gaa gtt att cgg gat ata cga gtt 53169
Asp Leu Ala Arg Val Ser Val Glu Val Ile Arg Asp Ile Arg Val
8675                8680                8685

aaa aat agg gtc gtt ttt tca ggt aac tgt aca aat ctc tct gag 53214
Lys Asn Arg Val Val Phe Ser Gly Asn Cys Thr Asn Leu Ser Glu
8690                8695                8700

gca gcc cgg gca agg ctt gta ggc ctt gca agt gcg tac caa cgc 53259
Ala Ala Arg Ala Arg Leu Val Gly Leu Ala Ser Ala Tyr Gln Arg
8705                8710                8715

caa gaa aaa aga gtg gat atg tta cac ggg gcc cta ggg ttt ttg 53304
Gln Glu Lys Arg Val Asp Met Leu His Gly Ala Leu Gly Phe Leu
8720                8725                8730

ctt aaa cag ttt cac ggc ctg tta ttt cct cgg ggt atg cca cca 53349
Leu Lys Gln Phe His Gly Leu Leu Phe Pro Arg Gly Met Pro Pro
8735                8740                8745

aac agt aaa tcc ccc aac ccg cag tgg ttt tgg acc ctg tta caa 53394
Asn Ser Lys Ser Pro Asn Pro Gln Trp Phe Trp Thr Leu Leu Gln
8750                8755                8760

cgc aac cag atg ccg gca gat aaa ctt aca cac gaa gag att acc 53439
Arg Asn Gln Met Pro Ala Asp Lys Leu Thr His Glu Glu Ile Thr
8765                8770                8775

act att gca gct gtt aaa cgg ttt acc gag gaa tat gca gca ata 53484
Thr Ile Ala Ala Val Lys Arg Phe Thr Glu Glu Tyr Ala Ala Ile
8780                8785                8790

aac ttt att aat cta ccc cca acc tgc ata gga gaa tta gcc cag 53529
Asn Phe Ile Asn Leu Pro Pro Thr Cys Ile Gly Glu Leu Ala Gln
8795                8800                8805

ttt tat atg gca aat ctt att ctt aaa tac tgc gat cat tca cag 53574
Phe Tyr Met Ala Asn Leu Ile Leu Lys Tyr Cys Asp His Ser Gln
8810                8815                8820

tac ctt ata aat acc tta act tct ata att acg ggt gcc agg cgc 53619
Tyr Leu Ile Asn Thr Leu Thr Ser Ile Ile Thr Gly Ala Arg Arg
8825                8830                8835

ccg cgt gac cca tca tcc gtt ttg cat tgg att cgt aaa gat gtc 53664
Pro Arg Asp Pro Ser Ser Val Leu His Trp Ile Arg Lys Asp Val
8840                8845                8850

acg tcc gcc gcg gac ata gaa acc caa gca aag gcg ctt ctt gaa 53709
Thr Ser Ala Ala Asp Ile Glu Thr Gln Ala Lys Ala Leu Leu Glu
8855                8860                8865

aaa acg gaa aac tta ccg gaa tta tgg act acg gct ttt act tca 53754
Lys Thr Glu Asn Leu Pro Glu Leu Trp Thr Thr Ala Phe Thr Ser
8870                8875                8880

act cat tta gtc cgc gcg gcc atg aat caa cgt ccc atg gtc gtt 53799
Thr His Leu Val Arg Ala Ala Met Asn Gln Arg Pro Met Val Val
8885                8890                8895

tta gga ata agc att agt aaa tat cac gga gcg gca gga aac aac 53844
Leu Gly Ile Ser Ile Ser Lys Tyr His Gly Ala Ala Gly Asn Asn
8900                8905                8910

cgc gtc ttt cag gca ggg aat tgg agc ggt tta aac ggg ggt aaa 53889
Arg Val Phe Gln Ala Gly Asn Trp Ser Gly Leu Asn Gly Gly Lys
8915                8920                8925

aat gta tgc ccg cta ttt aca ttt gat cgc act cgc cgt ttt ata 53934
Asn Val Cys Pro Leu Phe Thr Phe Asp Arg Thr Arg Arg Phe Ile
8930                8935                8940

ata gca tgt cct aga gga ggt ttt atc tgc ccc gta aca ggt ccc 53979
Ile Ala Cys Pro Arg Gly Gly Phe Ile Cys Pro Val Thr Gly Pro
8945                8950                8955

tcg tcg gga aat cga gaa acc acc cta tcc gac caa gtt cgc ggt 54024
Ser Ser Gly Asn Arg Glu Thr Thr Leu Ser Asp Gln Val Arg Gly
8960                8965                8970

ata att gtc agt ggc ggg gcc atg gtt caa tta gcc ata tac gcc 54069
Ile Ile Val Ser Gly Gly Ala Met Val Gln Leu Ala Ile Tyr Ala
8975                8980                8985

acg gtt gtg cgt gca gtg ggc gct cga gca caa cat atg gca ttt 54114
Thr Val Val Arg Ala Val Gly Ala Arg Ala Gln His Met Ala Phe
8990                8995                9000

gac gac tgg tta agt ctt aca gac gat gag ttt tta gcc aga gac 54159
Asp Asp Trp Leu Ser Leu Thr Asp Asp Glu Phe Leu Ala Arg Asp
9005                9010                9015

ttg gag gag tta cac gac cag att atc caa acc ctg gaa acg ccc 54204
Leu Glu Glu Leu His Asp Gln Ile Ile Gln Thr Leu Glu Thr Pro
9020                9025                9030

tgg acc gta gaa ggc gct cta gaa gca gta aag att cta gat gaa 54249
Trp Thr Val Glu Gly Ala Leu Glu Ala Val Lys Ile Leu Asp Glu
9035                9040                9045

aaa acg aca gcg gga gat ggg gaa acc ccc aca aac cta gca ttt 54294
Lys Thr Thr Ala Gly Asp Gly Glu Thr Pro Thr Asn Leu Ala Phe
9050                9055                9060

aat ttt gat tct tgt gaa cca agc cat gac acc aca tct aac gta 54339
Asn Phe Asp Ser Cys Glu Pro Ser His Asp Thr Thr Ser Asn Val
9065                9070                9075

tta aac att tca ggg tca aac att tca ggg tca act gtc cct ggt 54384
Leu Asn Ile Ser Gly Ser Asn Ile Ser Gly Ser Thr Val Pro Gly
9080                9085                9090

ctt aaa cga ccc ccc gaa gat gac gaa ctc ttt gat ctt agt ggt 54429
Leu Lys Arg Pro Pro Glu Asp Asp Glu Leu Phe Asp Leu Ser Gly
9095                9100                9105

att ccc ata aaa cat ggg aac att aca atg gaa atg att taa 54471
Ile Pro Ile Lys His Gly Asn Ile Thr Met Glu Met Ile
9110                9115                9120

cctccctctt tatccaatta aagcccacac gcgggtgagt gtacgtaata aacaagtcaa 54531

tattacatat tctgttgtgt tttctttttt tgtgtgtagt ccttacccat atgacctgta 54591

atatagtgtg tctccaacca ttcagcttac agtccagtgg acagtaacag cccgataac 54650

atg gaa ttg gat att aat cga aca ttg ttg gtt cta ctg ggt caa 54695
Met Glu Leu Asp Ile Asn Arg Thr Leu Leu Val Leu Leu Gly Gln
        9125                9130                9135

gtt tat acg tac atc ttt cag gtt gaa ctg cta cgt cga tgt gat 54740
Val Tyr Thr Tyr Ile Phe Gln Val Glu Leu Leu Arg Arg Cys Asp
        9140                9145                9150

cca agg gtg gcg tgt cgc ttt tta tat cgg tta gcg gct aac tgt 54785
Pro Arg Val Ala Cys Arg Phe Leu Tyr Arg Leu Ala Ala Asn Cys
        9155                9160                9165

ttg aca gtt cgt tat tta tta aag ctg ttt ctc cgg gga ttt aat 54830
Leu Thr Val Arg Tyr Leu Leu Lys Leu Phe Leu Arg Gly Phe Asn
        9170                9175                9180

acc cag cta aaa ttt gga aac act ccc acg gtt tgt gca ctg cat 54875
Thr Gln Leu Lys Phe Gly Asn Thr Pro Thr Val Cys Ala Leu His
        9185                9190                9195

tgg gca tta tgt tat gta aag gga gaa ggt gag cgt ttg ttt gag 54920
Trp Ala Leu Cys Tyr Val Lys Gly Glu Gly Glu Arg Leu Phe Glu
        9200                9205                9210

ttg cta caa cat ttt aaa acg cgt ttt gtt tat ggt gag act aaa 54965
Leu Leu Gln His Phe Lys Thr Arg Phe Val Tyr Gly Glu Thr Lys
        9215                9220                9225

gac tca aac tgt atc aaa gat tac ttt gtc tca gcg ttt aac tta 55010
Asp Ser Asn Cys Ile Lys Asp Tyr Phe Val Ser Ala Phe Asn Leu
        9230                9235                9240

aaa acc tgc caa tat cac cat gag ctg tcg tta aca aca tac gga 55055
Lys Thr Cys Gln Tyr His His Glu Leu Ser Leu Thr Thr Tyr Gly
        9245                9250                9255

ggt tac gta tcg agt gaa att cag ttt tta cac gac att gag aat 55100
Gly Tyr Val Ser Ser Glu Ile Gln Phe Leu His Asp Ile Glu Asn
        9260                9265                9270

ttt tta aaa cag ctt aat tac tgc tat att atc acg tct tct cgt 55145

Phe Leu Lys Gln Leu Asn Tyr Cys Tyr Ile Ile Thr Ser Ser Arg
        9275                9280                9285

gag gcg cta aac aca ttg gaa acc gtg acg cgg ttt atg aca gat 55190
Glu Ala Leu Asn Thr Leu Glu Thr Val Thr Arg Phe Met Thr Asp
        9290                9295                9300

act ata gga agc ggt cta ata cca ccc gtg gag ttg ttt gat ccg 55235
Thr Ile Gly Ser Gly Leu Ile Pro Pro Val Glu Leu Phe Asp Pro
        9305                9310                9315

gcg cat cca tgt gct ata tgt ttt gaa gaa tta tgt ata aca gct 55280
Ala His Pro Cys Ala Ile Cys Phe Glu Glu Leu Cys Ile Thr Ala
        9320                9325                9330

aac caa ggt gag acc tta cat cgt aga tta tta gga tgt atc tgc 55325
Asn Gln Gly Glu Thr Leu His Arg Arg Leu Leu Gly Cys Ile Cys
        9335                9340                9345

gat cac gtt act aag caa gtt cgg gtt aac gtg gat gtt gac gat 55370
Asp His Val Thr Lys Gln Val Arg Val Asn Val Asp Val Asp Asp
        9350                9355                9360

att att cgg tgt tta cca tat atc cct gat gta ccg gat atc aaa 55415
Ile Ile Arg Cys Leu Pro Tyr Ile Pro Asp Val Pro Asp Ile Lys
        9365                9370                9375

cgt caa tcc gcc gtt gaa gcg tta cga aca ctt caa acc aag acg 55460
Arg Gln Ser Ala Val Glu Ala Leu Arg Thr Leu Gln Thr Lys Thr
        9380                9385                9390

gta gtc aat ccc atg gga gca aag aac gat acg ttt gac caa aca 55505
Val Val Asn Pro Met Gly Ala Lys Asn Asp Thr Phe Asp Gln Thr
        9395                9400                9405

tac gaa att gcg agc acc atg ctt gat tct tat aat gtt ttt aaa 55550
Tyr Glu Ile Ala Ser Thr Met Leu Asp Ser Tyr Asn Val Phe Lys
        9410                9415                9420

cct gcc cct cgg tgt atg tac gcc atc agc gag ctt aaa ttc tgg 55595
Pro Ala Pro Arg Cys Met Tyr Ala Ile Ser Glu Leu Lys Phe Trp
        9425                9430                9435

tta acg tct aat tcc act gaa gga ccc caa cgt act tta gac gtg 55640
Leu Thr Ser Asn Ser Thr Glu Gly Pro Gln Arg Thr Leu Asp Val
        9440                9445                9450

ttt gtt gat aat ttg gat gta tta aac gaa cat gaa aaa cac gca 55685
Phe Val Asp Asn Leu Asp Val Leu Asn Glu His Glu Lys His Ala
        9455                9460                9465

gaa ctt aca gcc gta acg gtt gag ttg gcg tta ttt gga aaa act 55730
Glu Leu Thr Ala Val Thr Val Glu Leu Ala Leu Phe Gly Lys Thr
        9470                9475                9480

ccc ata cac ttt gat agg gcg ttt tct gaa gaa ctc gga tct ctg 55775
Pro Ile His Phe Asp Arg Ala Phe Ser Glu Glu Leu Gly Ser Leu
        9485                9490                9495

gat gca att gat agt att ttg gtt ggc aat cgc tca tcc tca cca 55820
Asp Ala Ile Asp Ser Ile Leu Val Gly Asn Arg Ser Ser Ser Pro
        9500                9505                9510

gac agt cag ata gaa gca tta att aaa gcc tgt tat gcc cat cat 55865
Asp Ser Gln Ile Glu Ala Leu Ile Lys Ala Cys Tyr Ala His His
        9515                9520                9525

cta tcg tcg cct ctc atg cgt cac att tct aac ccg agt cat gat 55910

Leu Ser Ser Pro Leu Met Arg His Ile Ser Asn Pro Ser His Asp
        9530                9535                9540

aac gaa gcc gcc tta cgc caa ctt tta gaa aga gtt ggg tgt gag 55955
Asn Glu Ala Ala Leu Arg Gln Leu Leu Glu Arg Val Gly Cys Glu
        9545                9550                9555

gat gat tta acc aaa gag gcg agt gac agc gct aca gca tcc gaa 56000
Asp Asp Leu Thr Lys Glu Ala Ser Asp Ser Ala Thr Ala Ser Glu
        9560                9565                9570

tgt gat ctg aac gat gat agt agc ata act ttt gct gtt cat gga 56045
Cys Asp Leu Asn Asp Asp Ser Ser Ile Thr Phe Ala Val His Gly
        9575                9580                9585

tgg gaa aac ctg tta tcc aaa gca aaa att gac gct gcg gaa aga 56090
Trp Glu Asn Leu Leu Ser Lys Ala Lys Ile Asp Ala Ala Glu Arg
        9590                9595                9600

aaa cga gta tat ctt gaa cat ctg tct aag cgc tct cta acc agc 56135
Lys Arg Val Tyr Leu Glu His Leu Ser Lys Arg Ser Leu Thr Ser
        9605                9610                9615

ctc ggt aga tgt atc cgc gaa cag cgc caa gag cta gaa aaa aca 56180
Leu Gly Arg Cys Ile Arg Glu Gln Arg Gln Glu Leu Glu Lys Thr
        9620                9625                9630

ctc agg gta aac gtt tat gga gag gcc tta ttg cag aca ttt gtt 56225
Leu Arg Val Asn Val Tyr Gly Glu Ala Leu Leu Gln Thr Phe Val
        9635                9640                9645

tcg atg caa aat ggg ttt ggg gca cga aac gtg ttt tta gct aag 56270
Ser Met Gln Asn Gly Phe Gly Ala Arg Asn Val Phe Leu Ala Lys
        9650                9655                9660

gtt tcc cag gca ggg tgt att atc gac aat cgc att cag gaa gcg 56315
Val Ser Gln Ala Gly Cys Ile Ile Asp Asn Arg Ile Gln Glu Ala
        9665                9670                9675

gcc ttt gat gca cat aga ttt ata agg aat acc tta gtt cga cat 56360
Ala Phe Asp Ala His Arg Phe Ile Arg Asn Thr Leu Val Arg His
        9680                9685                9690

aca gta gat gcg gct atg tta cct gca ctt aca cat aaa ttt ttt 56405
Thr Val Asp Ala Ala Met Leu Pro Ala Leu Thr His Lys Phe Phe
        9695                9700                9705

gag ttg gtc aac ggc cca ttg ttt aat cac gat gaa cac cgt ttt 56450
Glu Leu Val Asn Gly Pro Leu Phe Asn His Asp Glu His Arg Phe
        9710                9715                9720

gca caa ccc cct aac acc gcc tta ttt ttt acc gtg gaa aac gtt 56495
Ala Gln Pro Pro Asn Thr Ala Leu Phe Phe Thr Val Glu Asn Val
        9725                9730                9735

ggc cta ttt ccg cac tta aaa gag gaa ttg gca aag ttt atg ggc 56540
Gly Leu Phe Pro His Leu Lys Glu Glu Leu Ala Lys Phe Met Gly
        9740                9745                9750

ggt gtc gtt ggt tcc aac tgg ctt ctc agt cca ttt agg ggc ttt 56585
Gly Val Val Gly Ser Asn Trp Leu Leu Ser Pro Phe Arg Gly Phe
        9755                9760                9765

tat tgc ttt tct ggg gta gaa ggc gtt act ttt gca cag aga ctt 56630
Tyr Cys Phe Ser Gly Val Glu Gly Val Thr Phe Ala Gln Arg Leu
        9770                9775                9780

gcc tgg aaa tat att agg gag ctt gtg ttt gca acc aca cta ttc 56675

Ala Trp Lys Tyr Ile Arg Glu Leu Val Phe Ala Thr Thr Leu Phe
        9785                9790                9795

acc tct gtt ttc cat tgt ggg gag gtg cgg tta tgt cgc gtt gac 56720
Thr Ser Val Phe His Cys Gly Glu Val Arg Leu Cys Arg Val Asp
        9800                9805                9810

cgt cta ggt aag gat cca cgc ggg tgc acg tct caa cct aaa ggt 56765
Arg Leu Gly Lys Asp Pro Arg Gly Cys Thr Ser Gln Pro Lys Gly
        9815                9820                9825

ata ggc agt tcc cac gga ccc tta gac ggc att tat tta acg tac 56810
Ile Gly Ser Ser His Gly Pro Leu Asp Gly Ile Tyr Leu Thr Tyr
        9830                9835                9840

gaa gaa aca tgt ccc ctt gtg gct att att caa agt gga gaa aca 56855
Glu Glu Thr Cys Pro Leu Val Ala Ile Ile Gln Ser Gly Glu Thr
        9845                9850                9855

ggg atc gac cag aat acc gtc gta atc tac gat tca gac gtt ttt 56900
Gly Ile Asp Gln Asn Thr Val Val Ile Tyr Asp Ser Asp Val Phe
        9860                9865                9870

tct ctt cta tac acc cta atg cag cgg ctg gct ccg gat tca acg 56945
Ser Leu Leu Tyr Thr Leu Met Gln Arg Leu Ala Pro Asp Ser Thr
        9875                9880                9885

gac ccg gcg ttt tca taa cctccgttac gggggtgtgg ttatgctttt 56993
Asp Pro Ala Phe Ser
        9890

tatgcatatt ttct atg ttt gtt acg gcg gtt gtg tcg gtc tct cca agc 57043
Met Phe Val Thr Ala Val Val Ser Val Ser Pro Ser
        9895                9900

tcg ttt tat gag agt tta caa gta gag ccc aca caa tca gaa gat 57088
Ser Phe Tyr Glu Ser Leu Gln Val Glu Pro Thr Gln Ser Glu Asp
9905                9910                9915

ata acc cgg tct gct cat ctg ggc gat ggt gat gaa atc aga gaa 57133
Ile Thr Arg Ser Ala His Leu Gly Asp Gly Asp Glu Ile Arg Glu
9920                9925                9930

gct ata cac aag tcc cag gac gcc gaa aca aaa ccc acg ttt tac 57178
Ala Ile His Lys Ser Gln Asp Ala Glu Thr Lys Pro Thr Phe Tyr
9935                9940                9945

gtc tgc cca ccg cca aca ggc tcc aca atc gta cga tta gaa cca 57223
Val Cys Pro Pro Pro Thr Gly Ser Thr Ile Val Arg Leu Glu Pro
9950                9955                9960

act cgg aca tgt ccg gat tat cac ctt ggt aaa aac ttt aca gag 57268
Thr Arg Thr Cys Pro Asp Tyr His Leu Gly Lys Asn Phe Thr Glu
9965                9970                9975

ggt att gct gtt gtt tat aaa gaa aac att gca gcg tac aag ttt 57313
Gly Ile Ala Val Val Tyr Lys Glu Asn Ile Ala Ala Tyr Lys Phe
9980                9985                9990

aag gcg acg gta tat tac aaa gat gtt atc gtt agc acg gcg tgg 57358
Lys Ala Thr Val Tyr Tyr Lys Asp Val Ile Val Ser Thr Ala Trp
9995                10000               10005

gcc gga agt tct tat acg caa att act aat aga tat gcg gat agg 57403
Ala Gly Ser Ser Tyr Thr Gln Ile Thr Asn Arg Tyr Ala Asp Arg
10010               10015               10020

gta cca att ccc gtt tca gag atc acg gac acc att gat aag ttt 57448

Val Pro Ile Pro Val Ser Glu Ile Thr Asp Thr Ile Asp Lys Phe
10025               10030               10035

ggc aag tgt tct tct aaa gca acg tac gta cga aat aac cac aaa 57493
Gly Lys Cys Ser Ser Lys Ala Thr Tyr Val Arg Asn Asn His Lys
10040               10045               10050

gtt gaa gcc ttt aat gag gat aaa aat cca cag gat atg cct cta 57538
Val Glu Ala Phe Asn Glu Asp Lys Asn Pro Gln Asp Met Pro Leu
10055               10060               10065

atc gca tca aaa tat aat tct gtg gga tcc aaa gca tgg cat act 57583
Ile Ala Ser Lys Tyr Asn Ser Val Gly Ser Lys Ala Trp His Thr
10070               10075               10080

acc aat gac acg tac atg gtt gcc gga acc ccc gga aca tat agg 57628
Thr Asn Asp Thr Tyr Met Val Ala Gly Thr Pro Gly Thr Tyr Arg
10085               10090               10095

acg ggc acg tcg gtg aat tgc atc att gag gaa gtt gaa gcc aga 57673
Thr Gly Thr Ser Val Asn Cys Ile Ile Glu Glu Val Glu Ala Arg
10100               10105               10110

tca ata ttc cct tat gat agt ttt gga ctt tcc acg gga gat ata 57718
Ser Ile Phe Pro Tyr Asp Ser Phe Gly Leu Ser Thr Gly Asp Ile
10115               10120               10125

ata tac atg tcc ccg ttt ttt ggc cta cgg gat ggt gca tac aga 57763
Ile Tyr Met Ser Pro Phe Phe Gly Leu Arg Asp Gly Ala Tyr Arg
10130               10135               10140

gaa cat tcc aat tat gca atg gat cgt ttt cac cag ttt gag ggt 57808
Glu His Ser Asn Tyr Ala Met Asp Arg Phe His Gln Phe Glu Gly
10145               10150               10155

tat aga caa agg gat ctt gac act aga gca tta ctg gaa cct gca 57853
Tyr Arg Gln Arg Asp Leu Asp Thr Arg Ala Leu Leu Glu Pro Ala
10160               10165               10170

gcg cgg aac ttt tta gtc acg cct cat tta acg gtt ggt tgg aac 57898
Ala Arg Asn Phe Leu Val Thr Pro His Leu Thr Val Gly Trp Asn
10175               10180               10185

tgg aag cca aaa cga acg gaa gtt tgt tcg ctt gtc aag tgg cgt 57943
Trp Lys Pro Lys Arg Thr Glu Val Cys Ser Leu Val Lys Trp Arg
10190               10195               10200

gag gtt gaa gac gta gtt cgc gat gag tat gca cac aat ttt cgc 57988
Glu Val Glu Asp Val Val Arg Asp Glu Tyr Ala His Asn Phe Arg
10205               10210               10215

ttt aca atg aaa aca ctt tct acc acg ttt ata agt gaa aca aac 58033
Phe Thr Met Lys Thr Leu Ser Thr Thr Phe Ile Ser Glu Thr Asn
10220               10225               10230

gag ttt aat ctt aac caa atc cat ctc agt caa tgt gta aag gag 58078
Glu Phe Asn Leu Asn Gln Ile His Leu Ser Gln Cys Val Lys Glu
10235               10240               10245

gaa gcc cgg gct att att aac cgg atc tat aca acc aga tac aac 58123
Glu Ala Arg Ala Ile Ile Asn Arg Ile Tyr Thr Thr Arg Tyr Asn
10250               10255               10260

tca tct cat gtt aga acc ggg gat atc cag acc tac ctt gcc aga 58168
Ser Ser His Val Arg Thr Gly Asp Ile Gln Thr Tyr Leu Ala Arg
10265               10270               10275

ggg ggg ttt gtt gtg gtg ttt caa ccc ctg ctg agc aat tcc ctc 58213

Gly Gly Phe Val Val Val Phe Gln Pro Leu Leu Ser Asn Ser Leu
10280               10285               10290

gcc cgt ctc tat ctc caa gaa ttg gtc cgt gaa aac act aat cat 58258
Ala Arg Leu Tyr Leu Gln Glu Leu Val Arg Glu Asn Thr Asn His
10295               10300               10305

tca cca caa aaa cac ccg act cga aat acc aga tcc cga cga agc 58303
Ser Pro Gln Lys His Pro Thr Arg Asn Thr Arg Ser Arg Arg Ser
10310               10315               10320

gtg cca gtt gag ttg cgt gcc aat aga aca ata aca acc acc tca 58348
Val Pro Val Glu Leu Arg Ala Asn Arg Thr Ile Thr Thr Thr Ser
10325               10330               10335

tcg gtg gaa ttt gct atg ctc cag ttt aca tat gac cac att caa 58393
Ser Val Glu Phe Ala Met Leu Gln Phe Thr Tyr Asp His Ile Gln
10340               10345               10350

gag cat gtt aat gaa atg ttg gca cgt atc tcc tcg tcg tgg tgc 58438
Glu His Val Asn Glu Met Leu Ala Arg Ile Ser Ser Ser Trp Cys
10355               10360               10365

cag cta caa aat cgc gaa cgc gcc ctt tgg agc gga cta ttt cca 58483
Gln Leu Gln Asn Arg Glu Arg Ala Leu Trp Ser Gly Leu Phe Pro
10370               10375               10380

att aac cca agt gct tta gcg agc acc att ttg gat caa cgt gtt 58528
Ile Asn Pro Ser Ala Leu Ala Ser Thr Ile Leu Asp Gln Arg Val
10385               10390               10395

aaa gct cgt att ctc ggc gac gtt atc tcc gtt tct aat tgt cca 58573
Lys Ala Arg Ile Leu Gly Asp Val Ile Ser Val Ser Asn Cys Pro
10400               10405               10410

gaa ctg gga tca gat aca cgc att ata ctt caa aac tct atg agg 58618
Glu Leu Gly Ser Asp Thr Arg Ile Ile Leu Gln Asn Ser Met Arg
10415               10420               10425

gta tct ggt agt act acg cgt tgt tat agc cgt cct tta att tca 58663
Val Ser Gly Ser Thr Thr Arg Cys Tyr Ser Arg Pro Leu Ile Ser
10430               10435               10440

ata gtt agt tta aat ggg tcc ggg acg gtg gag ggc cag ctt gga 58708
Ile Val Ser Leu Asn Gly Ser Gly Thr Val Glu Gly Gln Leu Gly
10445               10450               10455

aca gat aac gag tta att atg tcc aga gat ctg tta gaa cca tgc 58753
Thr Asp Asn Glu Leu Ile Met Ser Arg Asp Leu Leu Glu Pro Cys
10460               10465               10470

gtg gct aat cac aag cga tat ttt cta ttt ggg cat cac tac gta 58798
Val Ala Asn His Lys Arg Tyr Phe Leu Phe Gly His His Tyr Val
10475               10480               10485

tat tat gag gat tat cgt tac gtc cgt gaa atc gca gtc cat gat 58843
Tyr Tyr Glu Asp Tyr Arg Tyr Val Arg Glu Ile Ala Val His Asp
10490               10495               10500

gtg gga atg att agc act tac gta gat tta aac tta aca ctt ctt 58888
Val Gly Met Ile Ser Thr Tyr Val Asp Leu Asn Leu Thr Leu Leu
10505               10510               10515

aaa gat aga gag ttt atg ccg ctg caa gta tat aca aga gac gag 58933
Lys Asp Arg Glu Phe Met Pro Leu Gln Val Tyr Thr Arg Asp Glu
10520               10525               10530

ctg cgg gat aca gga tta cta gac tac agt gaa att caa cgc cga 58978

Leu Arg Asp Thr Gly Leu Leu Asp Tyr Ser Glu Ile Gln Arg Arg
10535               10540               10545

aat caa atg cat tcg ctg cgt ttt tat gac ata gac aag gtt gtg 59023
Asn Gln Met His Ser Leu Arg Phe Tyr Asp Ile Asp Lys Val Val
10550               10555               10560

caa tat gat agc gga acg gcc att atg cag ggc atg gct cag ttt 59068
Gln Tyr Asp Ser Gly Thr Ala Ile Met Gln Gly Met Ala Gln Phe
10565               10570               10575

ttc cag gga ctt ggg acc gcg ggc cag gcc gtt gga cat gtg gtt 59113
Phe Gln Gly Leu Gly Thr Ala Gly Gln Ala Val Gly His Val Val
10580               10585               10590

ctt ggg gcc acg gga gcg ctg ctt tcc acc gta cac gga ttt acc 59158
Leu Gly Ala Thr Gly Ala Leu Leu Ser Thr Val His Gly Phe Thr
10595               10600               10605

acg ttt tta tct aac cca ttt ggg gca ttg gcc gtg gga tta ttg 59203
Thr Phe Leu Ser Asn Pro Phe Gly Ala Leu Ala Val Gly Leu Leu
10610               10615               10620

gtt ttg gcg gga ctg gta gcg gcc ttt ttt gcg tac cgg tac gtg 59248
Val Leu Ala Gly Leu Val Ala Ala Phe Phe Ala Tyr Arg Tyr Val
10625               10630               10635

ctt aaa ctt aaa aca agc ccg atg aag gca tta tat cca ctc aca 59293
Leu Lys Leu Lys Thr Ser Pro Met Lys Ala Leu Tyr Pro Leu Thr
10640               10645               10650

acc aag ggg tta aaa cag tta ccg gaa gga atg gat ccc ttt gcc 59338
Thr Lys Gly Leu Lys Gln Leu Pro Glu Gly Met Asp Pro Phe Ala
10655               10660               10665

gag aaa ccc aac gct act gat acc cca ata gaa gaa att ggc gac 59383
Glu Lys Pro Asn Ala Thr Asp Thr Pro Ile Glu Glu Ile Gly Asp
10670               10675               10680

tca caa aac act gaa ccg tcg gta aat agc ggg ttt gat ccc gat 59428
Ser Gln Asn Thr Glu Pro Ser Val Asn Ser Gly Phe Asp Pro Asp
10685               10690               10695

aaa ttt cga gaa gcc cag gaa atg att aaa tat atg acg tta gta 59473
Lys Phe Arg Glu Ala Gln Glu Met Ile Lys Tyr Met Thr Leu Val
10700               10705               10710

tct gcg gct gag cgc caa gaa tct aaa gcc cgc aaa aaa aat aag 59518
Ser Ala Ala Glu Arg Gln Glu Ser Lys Ala Arg Lys Lys Asn Lys
10715               10720               10725

act agc gcc ctt tta act tca cgt ctt acc ggc ctt gct tta cga 59563
Thr Ser Ala Leu Leu Thr Ser Arg Leu Thr Gly Leu Ala Leu Arg
10730               10735               10740

aat cgc cga gga tac tcc cgt gtt cgc acc gag aat gta acg ggg 59608
Asn Arg Arg Gly Tyr Ser Arg Val Arg Thr Glu Asn Val Thr Gly
10745               10750               10755

gtg taa atagccaggg ggtttgtttt aatttattaa taaaaatgtg tattacgtta 59664
Val
10760

ctcatgtgtc tccattacgc atcacagggg gtatttatac ccgataatat acaaaacgcg 59724

ttttgtacct ctaccgcacc cgatatctta acggggttat t atg gaa tcg tct 59777
Met Glu Ser Ser

aac att aac gcg cta caa caa ccg tcg tct atc gca cat cat ccg 59822
Asn Ile Asn Ala Leu Gln Gln Pro Ser Ser Ile Ala His His Pro
10765               10770               10775

tcc aaa cag tgc gct tca agt ctc aat gaa aca gta aaa gat tct 59867
Ser Lys Gln Cys Ala Ser Ser Leu Asn Glu Thr Val Lys Asp Ser
10780               10785               10790

ccc ccc gcg att tat gaa gat agg tta gaa cac acg ccg gta caa 59912
Pro Pro Ala Ile Tyr Glu Asp Arg Leu Glu His Thr Pro Val Gln
10795               10800               10805

tta ccc cgc gac ggt aca ccc cga gac gta tgt tct gtg gga cag 59957
Leu Pro Arg Asp Gly Thr Pro Arg Asp Val Cys Ser Val Gly Gln
10810               10815               10820

cta acc tgt cga gca tgt gca acg aaa cct ttt cgc ctt aac cgc 60002
Leu Thr Cys Arg Ala Cys Ala Thr Lys Pro Phe Arg Leu Asn Arg
10825               10830               10835

gac agc caa tac gac tac tta aac aca tgt cca ggg ggc cgt cat 60047
Asp Ser Gln Tyr Asp Tyr Leu Asn Thr Cys Pro Gly Gly Arg His
10840               10845               10850

att tca ctg gca ctg gag att ata acg ggt cga tgg gtt tgc atc 60092
Ile Ser Leu Ala Leu Glu Ile Ile Thr Gly Arg Trp Val Cys Ile
10855               10860               10865

ccg cgt gtg ttt ccg gat acc cca gag gaa aaa tgg atg gcg cca 60137
Pro Arg Val Phe Pro Asp Thr Pro Glu Glu Lys Trp Met Ala Pro
10870               10875               10880

tat att att cca gac cga gaa caa cca tca tca ggg gat gaa gat 60182
Tyr Ile Ile Pro Asp Arg Glu Gln Pro Ser Ser Gly Asp Glu Asp
10885               10890               10895

tct gac acc gat taa atttaactta aataaaacct taccacccat aaaaacgcct 60237
Ser Asp Thr Asp
10900

tctgtttgtt taacacgaca ccgcttaaca aaaaaaaaaa aaccaaacac gccttttatg 60297

aatgtaatac ttttatttgt tggttaacac cgccccacca tcatctgatt tgcaaacata 60357

tcggcgtcgt ctgccgtgga cccctgtatt aaaggggcct tggaactcgc ctccactgca 60417

tttacatctt gtccaactgt atctgtatgt ggggtgcttg ttgtattttg ggatgagcat 60477

agacccgaaa cgctttgaag ctgttttaat aaaatcgata ttcgaggatc ccgtgtcccc 60537

tctggtatat ttgtatggtg cgacaaaggc atttgtgtcc cattttgtga ttttagctct 60597

gtaacctcct gttgcagttt tgccacaacc ccagcaagct cttcgtgctg accattagaa 60657

actctgtgtc tcctctgcca atatgatgga gaaactcgac gtctccgatg cgttatatac 60717

gttggttcac cgggaaaata tatatttgag ggaaactctc cgtccatttg agactcccca 60777

ctataaaaag aatccaattc cctttgatcc atgctcttga aatcccgttt tcctggacga 60837

cggacatcgg ttttgtctgg aaaatttaca cacggggtct gcaagtcaat accccgttcg 60897

gcggccaatg cgttcataaa tgcggacatt tgcatttcca aacgattggg tggtggatat 60957

cccggaaacc cgtacggtcc cccgaagtgt cccggagggc aaccataacc ccctgtatta 61017

ggtgggaagg caggcgggtg tggagatcca tatggcccga cgatatactg tccgttattt 61077

ggagctccaa ttgatacctg cggattttta gtctgcccgg ttaacagctg tgaataatac 61137
gcggtaggta tcagtacaaa ttcccctccg gttggaacgc ccgacggggg ctgtggtgag 61197
atattactag cgttacctgc tacagaagcc atatcgctgt cgttcctaca caactgcgta 61257
acctttaaat gcggaacagt cttttcacaa tcttcatttg attccccaac acccaacgcg 61317
agatcgtata tgggcccgcc ggggtggaat gtggcgttta taacacccgc gttgggtaat 61377
ttagactcca ccccattaac gttggttatc cgagcaagtc catatccggt gctagcctga 61437
agataaacgt gacccataat tccggcttcg cgtctacgtt ttgcaaccac gtcccatcta 61497
tctcttaaaa gcatattgtt cacggctgtg gataataaca ccttggcgag tttatcttcg 61557
ctaaccttcc atactttatt taaacccgcg tagtctttaa ccagcgacaa taaccgcgct 61617
ttactttcca tcgataaaac ccggaatggt tcaattgaag attccggggt acagtcataa 61677
ttgaccactg ttccaacgcg tcttccaaca acacataacg caacatgggt aaaaaaatta 61737
ccgtctggta tctcattcgg ggacaatcgt tttgaagaca gggatacgga gggtaagtaa 61797
tttgtgacca agtataacgc acgttctagc ggagataata cagaatctct atttccaaaa 61857
aaattcgaat gggccgcttc aaacagcacc gcatgtagtt gagggcatct aacgataccc 61917
aaaaaaaaag gtccgcgtat gtcctcaatg attgcgatta cttcacccac gacacagtct 61977
tttcgatgat cgatgtttat tggtatttta ctagtaggcg gcaaagcgga ccgcacaatc 62037
tctggggtaa tatttaattc cccttcgtcc tttgaatata aggctaaata cccagccacg 62097
tataacgctt cacagttctc ttcgtcagct tcagcagcca ttataaacac cccacggacc 62157
ggatagtgaa tactcacggt gtggaggcaa actgaggaat gacacccaaa cagacaaaat 62217
atagaagatc atagtcactg ttaacgttga actgcgcaag gcggcgactt tcttccaatg 62277
ccgcccttac acgcggttgg tgcattaaca ttccaagtcc ccgttcatat tgcaacataa 62337
cactgtcatg tattgatacc acggcggcta tgggtaggga tgtaacattt tgtcggcggt 62397
gttctaattc caatgcaatt aagcttatga gccgatcttg gtactgtcca gaagaaatat 62457
ctattacggt tcttcctaaa cttccacgac taagctgggt atgcgcgtct aaacaaagag 62517
caactaatcc aggaaacatt tcagtcagct ctgtggtccg atttaacgta tacagtggtg 62577
ctatatatcg ttcacataaa aattgaaagt tattattacc gcttttaaac ttcccatcaa 62637
accccgtcgc tccgcgcaag attacattgt tggtaggggt tcctgttgct tctgacacaa 62697
tcaaacccag ttgaaaatta ttttttagtt tatctccgta tacgttcccg ttccataata 62757
agcgccttaa taataataac gccgtaatcg tgtcaattgt taaccttaat agagtttggt 62817
cttccataag aaacacgttt tgggcccgtt ctaaatacgc cgcggccgcc tgttgaatct 62877
tgtccacata tgcggtatga ttgcgatcaa taatgtcatt aaccccagga ttaaactgtc 62937
caggtgcagg cggtaggacc tgcaaccgta taagcgcatc cataacagaa tgtgacgtta 62997
aggcgccttg atcataccgc cccccacgag catgaaactg gtcgcgtggt agacgatcat 63057
agcaaaattg ataactgttt ttattttcgt gtgttgtcat ataattcaca aatgtctcag 63117

tatattccgg taggtgctct ataaggttcc cgaaggacga aacttgaggt tcgtggacac 63177

tattagatgt cctatacatt aaatataaac ataataccgc acactcgaac gcggagtacg 63237

ctctatctcc aacatacatt ctcccggcgg actgtagaca tgttaccgtt gtgttcataa 63297

acgtacggga aatgcgcccg tctttacaat caactccgcg tgcagctacg ggcctatcta 63357

acacaagccg ttcctgcaga gtacgatacc atggcccgaa aacaatccct ggagagttat 63417

tgccccttgc ccttcccaag tacaccaggg tgataaaatc cacttgaaag tttgtatcgt 63477

actgcaacgg tgcatcattt ttggcaatct gtacctcggg gtgtatagac tcattgcgta 63537

ttatttctgt acgtgtacat tcctcagatt gtgcatctgc ttcttccgcc tcggcagcag 63597

ccgtctccag ggaatccaaa accttggcca tgcgcgttag ttgttcttcg aggggcttta 63657

aacgacgatc tatttccgtt ggtaacgtaa tcgtttcccc gcgaaggttg tctaatgcgg 63717

caacggccgc cgcatttttt aacgttaacg tatttttttc caaatcggga ttcatacgcc 63777

ctcttaactc aaacgcggga gccgtccagt agtgtatggg gaagttgggg gctataaagt 63837

tcttagtggt agacaaaaat atcccacatt tattcggaaa cgagatagat ccgaacccat 63897

atctcgccgt catggtgtct gcagcaaaca aagtcaactg gcgtgaatat aaaccggtac 63957

tgctttaaaa gctgttttct tacccatggg aaaacatccc ggttatactt tgtaaaattc 64017

caccacaagc acctaaagaa ggccttctaa ggggtaaatc caccccacaa gctgcatttt 64077

cttcaaactt tgttaaagcg gaacgatggc atgatttcgc acgcttttcc gcaagagaac 64137

atacgtgaat ttttcttttg catagacgtc ttcgctctct aacggacctt atcggggggg 64197

tatattccgc tacattctcc aaatgcgacg ctagcataac aaggtttcca tgaatcacct 64257

ttgggggtaa ccgagttacc tgtaacaggt tcagaccccg ttgagataca aacacaagga 64317

ggggggtcac cattatttca tcagatcccg tgggtgtggt ttcctttatt aaagccatgg 64377

tatccctcag ctggcgcata ccctcgcaaa actggtgata cttagtaggg gtatgtatat 64437

tagcgctaaa acggcaagat tttaattcca ctataaaaca aacggtcttt ccggcaccac 64497

tggattccgt ttgtataata caaacacaat cggggcgtcg gcgtcccaaa tttacttcaa 64557

acgacattga tatgcgtaca gccctttgaa catccacgtg ggataacggc gacaggagtt 64617

ttgccagcct cgggttgaac gcgtccgcga aacctcgacg tacgttatca atatcctttt 64677

tgagtacatc gtaaaaacga gtgtggcaac gttgtcccaa acgaaaacac ttggcccgaa 64737

ttcgactagc ggacatattt gaagttccgt cccagaagat aacctaagac gcgtttgtct 64797

acaataaac atg tca acg gat aaa acc gat gta aaa atg ggc gtt 64842
Met Ser Thr Asp Lys Thr Asp Val Lys Met Gly Val
    10905               10910               10915

ttg cgt att tat ttg gac ggg gcg tat gga att gga aaa aca acc 64887
Leu Arg Ile Tyr Leu Asp Gly Ala Tyr Gly Ile Gly Lys Thr Thr
                10920               10925               10930

gcc gcc gaa gaa ttt tta cac cac ttt gca ata aca cca aac cgg 64932
Ala Ala Glu Glu Phe Leu His His Phe Ala Ile Thr Pro Asn Arg
                10935               10940               10945

atc tta ctc att ggg gag ccc ctg tcg tat tgg cgt aac ctt gca 64977
Ile Leu Leu Ile Gly Glu Pro Leu Ser Tyr Trp Arg Asn Leu Ala
                10950               10955               10960

ggg gag gac gcc att tgc gga att tac gga aca caa act cgc cgt 65022
Gly Glu Asp Ala Ile Cys Gly Ile Tyr Gly Thr Gln Thr Arg Arg
                10965               10970               10975

ctt aat gga gac gtt tcg cct gaa gac gca caa cgc ctc acg gct 65067
Leu Asn Gly Asp Val Ser Pro Glu Asp Ala Gln Arg Leu Thr Ala
                10980               10985               10990

cat ttt cag agc ctg ttc tgt tct ccg cat gca att atg cat gcg 65112
His Phe Gln Ser Leu Phe Cys Ser Pro His Ala Ile Met His Ala
                10995               11000               11005

aaa atc tcg gca ttg atg gac aca agt aca tcg gat ctc gta caa 65157
Lys Ile Ser Ala Leu Met Asp Thr Ser Thr Ser Asp Leu Val Gln
                11010               11015               11020

gta aat aag gag ccg tat aaa att atg tta tcc gac cga cac cca 65202
Val Asn Lys Glu Pro Tyr Lys Ile Met Leu Ser Asp Arg His Pro
                11025               11030               11035

atc gcc tca act ata tgt ttt ccc ttg tcc aga tac tta gtg gga 65247
Ile Ala Ser Thr Ile Cys Phe Pro Leu Ser Arg Tyr Leu Val Gly
                11040               11045               11050

gat atg tcc cca gcg gcg ctt cct ggg tta ttg ttt acg ctt ccc 65292
Asp Met Ser Pro Ala Ala Leu Pro Gly Leu Leu Phe Thr Leu Pro
                11055               11060               11065

gct gaa ccc ccc ggg acc aac ttg gta gtt tgt acc gtt tca ctc 65337
Ala Glu Pro Pro Gly Thr Asn Leu Val Val Cys Thr Val Ser Leu
                11070               11075               11080

ccc agt cat tta tcc aga gta agc aaa cgg gcc aga ccg gga gaa 65382
Pro Ser His Leu Ser Arg Val Ser Lys Arg Ala Arg Pro Gly Glu
                11085               11090               11095

acg gtt aat ctg ccg ttt gtt atg gtt ctg aga aat gta tat ata 65427
Thr Val Asn Leu Pro Phe Val Met Val Leu Arg Asn Val Tyr Ile
                11100               11105               11110

atg ctt att aat aca att ata ttt ctt aaa act aac aac tgg cac 65472
Met Leu Ile Asn Thr Ile Ile Phe Leu Lys Thr Asn Asn Trp His
                11115               11120               11125

gcg ggc tgg aac aca ctg tca ttt tgt aat gat gta ttt aaa cag 65517
Ala Gly Trp Asn Thr Leu Ser Phe Cys Asn Asp Val Phe Lys Gln
                11130               11135               11140

aaa tta caa aaa tcc gag tgt ata aaa cta cgc gaa gta cct ggg 65562
Lys Leu Gln Lys Ser Glu Cys Ile Lys Leu Arg Glu Val Pro Gly
                11145               11150               11155

att gaa gac acg tta ttc gcc gtg ctt aaa ctt ccg gag ctt tgc 65607
Ile Glu Asp Thr Leu Phe Ala Val Leu Lys Leu Pro Glu Leu Cys
                11160               11165               11170

gga gag ttt gga aat att ctg ccg tta tgg gca tgg gga atg gag 65652
Gly Glu Phe Gly Asn Ile Leu Pro Leu Trp Ala Trp Gly Met Glu
                11175               11180               11185

acc ctt tca aac tgc tca cga agc atg tct ccg ttc gta tta tcg 65697
Thr Leu Ser Asn Cys Ser Arg Ser Met Ser Pro Phe Val Leu Ser
                11190               11195               11200

tta gaa cag aca ccc   cag cat gcg gca caa   gaa cta aaa act ctg   65742
Leu Glu Gln Thr Pro   Gln His Ala Ala Gln   Glu Leu Lys Thr Leu
                11205                 11210                 11215

cta ccc cag atg acc   ccg gca aac atg tcc   tcc ggt gca tgg aat   65787
Leu Pro Gln Met Thr   Pro Ala Asn Met Ser   Ser Gly Ala Trp Asn
                11220                 11225                 11230

ata ttg aaa gag ctt   gtt aat gcc gtt cag   gac aac act tcc taa   65832
Ile Leu Lys Glu Leu   Val Asn Ala Val Gln   Asp Asn Thr Ser
                11235                 11240

atatacctag tatttacgta tgtaccagta aaaagatgat acacattgtc atactcgcgt   65892

gtacgtgttt ttctttttta tatatgcgtc atttattacc acatccttta atcccgcctt   65952

tatctcccta aaacggagtg gtaatattaa aagccgccaa gcctgttggt gggtgaggag   66012

gggtaaaggc acgctgtgtg cataacgttg cggtgatatt gtagcgcaag taacagcgac   66072

t atg   ttt gcg cta gtt tta   gcg gtg gta att ctt   cct ctt tgg   66115
  Met   Phe Ala Leu Val Leu   Ala Val Val Ile Leu   Pro Leu Trp
  11245                 11250                 11255

acc acg   gct aat aaa tct tac   gta aca cca acc cct   gcg act cgc   66160
Thr Thr   Ala Asn Lys Ser Tyr   Val Thr Pro Thr Pro   Ala Thr Arg
    11260                 11265                 11270

tct atc   gga cat atg tct gct   ctt cta cga gaa tat   tcc gac cgt   66205
Ser Ile   Gly His Met Ser Ala   Leu Leu Arg Glu Tyr   Ser Asp Arg
    11275                 11280                 11285

aat atg   tct ctg aaa tta gaa   gcc ttt tat cct act   ggt ttc gat   66250
Asn Met   Ser Leu Lys Leu Glu   Ala Phe Tyr Pro Thr   Gly Phe Asp
    11290                 11295                 11300

gaa gaa   ctc att aaa tca ctt   cac tgg gga aat gat   aga aaa cac   66295
Glu Glu   Leu Ile Lys Ser Leu   His Trp Gly Asn Asp   Arg Lys His
    11305                 11310                 11315

gtt ttc   ttg gtt att gtt aag   gtt aac cct aca aca   cac gaa gga   66340
Val Phe   Leu Val Ile Val Lys   Val Asn Pro Thr Thr   His Glu Gly
    11320                 11325                 11330

gac gtc   ggg ctg gtt ata ttt   cca aaa tac ttg tta   tcg cca tac   66385
Asp Val   Gly Leu Val Ile Phe   Pro Lys Tyr Leu Leu   Ser Pro Tyr
    11335                 11340                 11345

cat ttc   aaa gca gaa cat cga   gca ccg ttt cct gct   gga cgt ttt   66430
His Phe   Lys Ala Glu His Arg   Ala Pro Phe Pro Ala   Gly Arg Phe
    11350                 11355                 11360

gga ttt   ctt agt cac cct gtg   aca ccc gac gtg agc   ttc ttt gac   66475
Gly Phe   Leu Ser His Pro Val   Thr Pro Asp Val Ser   Phe Phe Asp
    11365                 11370                 11375

agt tcg   ttt gcg ccg tat tta   act acg caa cat ctt   gtt gcg ttt   66520
Ser Ser   Phe Ala Pro Tyr Leu   Thr Thr Gln His Leu   Val Ala Phe
    11380                 11385                 11390

act acg   ttc cca cca aac ccc   ctt gta tgg cat ttg   gaa aga gct   66565
Thr Thr   Phe Pro Pro Asn Pro   Leu Val Trp His Leu   Glu Arg Ala
    11395                 11400                 11405

gag acc   gca gca act gca gaa   agg ccg ttt ggg gta   agt ctt tta   66610
Glu Thr   Ala Ala Thr Ala Glu   Arg Pro Phe Gly Val   Ser Leu Leu
    11410                 11415                 11420

ccc gct cgc cca aca gtc ccc aag aat act att ctg gaa cat aaa 66655
Pro Ala Arg Pro Thr Val Pro Lys Asn Thr Ile Leu Glu His Lys
    11425               11430               11435

gcg cat ttt gct aca tgg gat gcc ctt gcc cga cat act ttt ttt 66700
Ala His Phe Ala Thr Trp Asp Ala Leu Ala Arg His Thr Phe Phe
    11440               11445               11450

tct gcc gaa gca att atc acc aac tca acg ttg aga ata cac gtt 66745
Ser Ala Glu Ala Ile Ile Thr Asn Ser Thr Leu Arg Ile His Val
    11455               11460               11465

ccc ctt ttt ggg tcg gta tgg cca att cga tac tgg gcc acc ggt 66790
Pro Leu Phe Gly Ser Val Trp Pro Ile Arg Tyr Trp Ala Thr Gly
    11470               11475               11480

tcg gtg ctt ctc aca agc gac tcg ggt cgt gtg gaa gta aat att 66835
Ser Val Leu Leu Thr Ser Asp Ser Gly Arg Val Glu Val Asn Ile
    11485               11490               11495

ggt gta gga ttt atg agc tcg ctc att tct tta tcc tct gga cca 66880
Gly Val Gly Phe Met Ser Ser Leu Ile Ser Leu Ser Ser Gly Pro
    11500               11505               11510

ccg ata gaa tta att gtt gta cca cat aca gta aaa ctg aac gcg 66925
Pro Ile Glu Leu Ile Val Val Pro His Thr Val Lys Leu Asn Ala
    11515               11520               11525

gtt aca agc gac acc aca tgg ttc cag cta aat cca ccg ggt ccg 66970
Val Thr Ser Asp Thr Thr Trp Phe Gln Leu Asn Pro Pro Gly Pro
    11530               11535               11540

gat ccg ggg cca tct tat cga gtt tat tta ctt gga cgt ggg ttg 67015
Asp Pro Gly Pro Ser Tyr Arg Val Tyr Leu Leu Gly Arg Gly Leu
    11545               11550               11555

gat atg aat ttt tca aag cat gct acg gtc gat ata tgc gca tat 67060
Asp Met Asn Phe Ser Lys His Ala Thr Val Asp Ile Cys Ala Tyr
    11560               11565               11570

ccc gaa gag agt ttg gat tac cgc tat cat tta tcc atg gcc cac 67105
Pro Glu Glu Ser Leu Asp Tyr Arg Tyr His Leu Ser Met Ala His
    11575               11580               11585

acg gag gct ctg cgg atg aca acg aag gcg gat caa cat gac ata 67150
Thr Glu Ala Leu Arg Met Thr Thr Lys Ala Asp Gln His Asp Ile
    11590               11595               11600

aac gag gaa agc tat tac cat atc gcc gca aga ata gcc aca tca 67195
Asn Glu Glu Ser Tyr Tyr His Ile Ala Ala Arg Ile Ala Thr Ser
    11605               11610               11615

att ttt gcg ttg tcg gaa atg ggc cgt acc aca gaa tat ttt ctg 67240
Ile Phe Ala Leu Ser Glu Met Gly Arg Thr Thr Glu Tyr Phe Leu
    11620               11625               11630

tta gat gag atc gta gat gtt cag tat caa tta aaa ttc ctt aat 67285
Leu Asp Glu Ile Val Asp Val Gln Tyr Gln Leu Lys Phe Leu Asn
    11635               11640               11645

tac att tta atg cgg ata gga gca gga gct cat ccc aac act ata 67330
Tyr Ile Leu Met Arg Ile Gly Ala Gly Ala His Pro Asn Thr Ile
    11650               11655               11660

tcc gga acc tcg gat ctg atc ttt gcc gat cca tcg cag ctt cat 67375
Ser Gly Thr Ser Asp Leu Ile Phe Ala Asp Pro Ser Gln Leu His
    11665               11670               11675

gac gaa ctt tca ctt ctt ttt ggt cag gta aaa ccc gca aat gtc 67420
Asp Glu Leu Ser Leu Leu Phe Gly Gln Val Lys Pro Ala Asn Val
    11680               11685               11690

gat tat ttt att tca tat gat gaa gcc cgt gat caa cta aag acc 67465
Asp Tyr Phe Ile Ser Tyr Asp Glu Ala Arg Asp Gln Leu Lys Thr
    11695               11700               11705

gca tac gcg ctt tcc cgt ggt caa gac cat gtg aat gca ctt tct 67510
Ala Tyr Ala Leu Ser Arg Gly Gln Asp His Val Asn Ala Leu Ser
    11710               11715               11720

ctc gcc agg cgt gtt ata atg agc ata tac aag ggg ctg ctt gtg 67555
Leu Ala Arg Arg Val Ile Met Ser Ile Tyr Lys Gly Leu Leu Val
    11725               11730               11735

aag caa aat tta aat gct aca gag agg cag gct tta ttt ttt gcc 67600
Lys Gln Asn Leu Asn Ala Thr Glu Arg Gln Ala Leu Phe Phe Ala
    11740               11745               11750

tca atg att tta tta aat ttc cgc gaa gga cta gaa aat tca tct 67645
Ser Met Ile Leu Leu Asn Phe Arg Glu Gly Leu Glu Asn Ser Ser
    11755               11760               11765

cgg gta tta gac ggt cgc aca act ttg ctt tta atg aca tcc atg 67690
Arg Val Leu Asp Gly Arg Thr Thr Leu Leu Leu Met Thr Ser Met
    11770               11775               11780

tgt acg gca gct cac gcc acg caa gca gca ctt aac ata caa gaa 67735
Cys Thr Ala Ala His Ala Thr Gln Ala Ala Leu Asn Ile Gln Glu
    11785               11790               11795

ggc ctg gca tac tta aat cct tca aaa cac atg ttt aca ata cca 67780
Gly Leu Ala Tyr Leu Asn Pro Ser Lys His Met Phe Thr Ile Pro
    11800               11805               11810

aac gta tac agt cct tgt atg ggt tcc ctt cgt aca gac ctc acg 67825
Asn Val Tyr Ser Pro Cys Met Gly Ser Leu Arg Thr Asp Leu Thr
    11815               11820               11825

gaa gag att cat gtt atg aat ctc ctg tcg gca ata cca aca cgc 67870
Glu Glu Ile His Val Met Asn Leu Leu Ser Ala Ile Pro Thr Arg
    11830               11835               11840

cca gga ctt aac gag gta ttg cat acc caa cta gac gaa tct gaa 67915
Pro Gly Leu Asn Glu Val Leu His Thr Gln Leu Asp Glu Ser Glu
    11845               11850               11855

ata ttc gac gcg gca ttt aaa acc atg atg att ttt acc aca tgg 67960
Ile Phe Asp Ala Ala Phe Lys Thr Met Met Ile Phe Thr Thr Trp
    11860               11865               11870

act gcc aaa gat ttg cat ata ctc cac acc cat gta cca gaa gta 68005
Thr Ala Lys Asp Leu His Ile Leu His Thr His Val Pro Glu Val
    11875               11880               11885

ttt acg tgt caa gat gca gcc gcg cgt aac gga gaa tat gtg ctc 68050
Phe Thr Cys Gln Asp Ala Ala Ala Arg Asn Gly Glu Tyr Val Leu
    11890               11895               11900

att ctt cca gct gtc cag gga cac agt tat gtg att aca cga aac 68095
Ile Leu Pro Ala Val Gln Gly His Ser Tyr Val Ile Thr Arg Asn
    11905               11910               11915

aaa cct caa agg ggt ttg gta tat tcc ctg gca gat gtg gat gta 68140
Lys Pro Gln Arg Gly Leu Val Tyr Ser Leu Ala Asp Val Asp Val
    11920               11925               11930

tat aac ccc ata tcc gtt gtt tat tta agc agg gat act tgc gtg 68185
Tyr Asn Pro Ile Ser Val Val Tyr Leu Ser Arg Asp Thr Cys Val
    11935               11940               11945

tct gaa cat ggt gtc ata gag acg gtc gca ctg ccc cat ccg gac 68230
Ser Glu His Gly Val Ile Glu Thr Val Ala Leu Pro His Pro Asp
    11950               11955               11960

aat tta aaa gaa tgt ttg tat tgc gga agt gtt ttt ctt agg tat 68275
Asn Leu Lys Glu Cys Leu Tyr Cys Gly Ser Val Phe Leu Arg Tyr
    11965               11970               11975

cta acc acg ggg gcg att atg gat ata att att att gac agc aaa 68320
Leu Thr Thr Gly Ala Ile Met Asp Ile Ile Ile Ile Asp Ser Lys
    11980               11985               11990

gat aca gaa cga caa cta gcc gct atg gga aac tcc aca att cca 68365
Asp Thr Glu Arg Gln Leu Ala Ala Met Gly Asn Ser Thr Ile Pro
    11995               12000               12005

ccc ttc aat cca gac atg cac ggg gat gac tct aag gct gtg ttg 68410
Pro Phe Asn Pro Asp Met His Gly Asp Asp Ser Lys Ala Val Leu
    12010               12015               12020

ttg ttt cca aac gga act gtg gta acg ctt cta gga ttc gaa cga 68455
Leu Phe Pro Asn Gly Thr Val Val Thr Leu Leu Gly Phe Glu Arg
    12025               12030               12035

cga caa gcc ata cga atg tcg gga caa tac ctt ggg gcc tct tta 68500
Arg Gln Ala Ile Arg Met Ser Gly Gln Tyr Leu Gly Ala Ser Leu
    12040               12045               12050

gga ggg gcg ttt ctg gcg gta gtg ggg ttt ggt att atc gga tgg 68545
Gly Gly Ala Phe Leu Ala Val Val Gly Phe Gly Ile Ile Gly Trp
    12055               12060               12065

atg tta tgt gga aat tcc cgc ctt cga gaa tat aat aaa ata cct 68590
Met Leu Cys Gly Asn Ser Arg Leu Arg Glu Tyr Asn Lys Ile Pro
    12070               12075               12080

ctg aca taa aaaacatgta taataaaaag tcactataaa cgtattctct 68639
Leu Thr
    12085

acaatacttt attcgcgaat aatacacact acctttgggt ttttttcccg tccccaaatg 68699

gtgtttggtg cactctacca aaaaatagag cgcctaaata tgctatataa cgcctcccag 68759

caaaatacgg ttcaaaggca ttacccgata ttgtattgta gtacagggca atgggaattg 68819

atgatcccaa taaacggcat agacgcacag cgccgttata gcaggggtct ccagagtaca 68879

gggtatctaa gtaccgggat atctcatact catgcctttc cgtgacagaa acatcaaccg 68939

gaacagtatc cgataaacca actcctgttt ttgcaaggcg taaaattcgc acaccttcct 68999

tttttgcaag atgtgacgtt tccttgtaac agggaagctg ggggagtggt aagaacaaca 69059

aagtttcagc caacgtgcca ataaagccca cttccctcaa gaggctgttt gctgtatcca 69119

caatggtccg tattaaatct tgagcaactt gatccgtgtc atcatcactg ggtaacgcgt 69179

taacataact acgcgttaaa tcttcaataa cggcataaca attaaacgct tcccaccgag 69239

acagtatata ttgaacaatc acgaaccgtt gacaggacgt cagatcacgt ccgtaagcat 69299

gcccgaaaaa tggaagttcc ccccgttcgc catataccgc aacaactgca gtatatatcg 69359

tctcacgggc ttcattaagt tcatcttcaa gtccaggcca ttttctggct ttaaatataa 69419

cctcgtccgc aaaaaaaacc gcacatgata acgcgcggat acaatgagta gtggctttat 69479

ggcgaggatc ccaaatgtcc attacccggg ggatggtcct aatctgtaca aagttactta 69539

gtgtaatatg atcggacttc ttacgccgtc taggctgttt ctcagaatac ggttcacccg 69599

aaatcggcac atcatctgct tttacgtctt ccgtaaccac atcagcagcg cgccgactaa 69659

caattatact tgttttttca tcgtcgttac ttccgttaag cgcgtctcgt atctcgggcg 69719

tcccgtcgaa taatccactc actagctcct gcaaactttc tggtaactcc aacatacgca 69779

tatacaccaa tgaaaaactg gcttcgtttg gtacgtacat aaagccattt gtggtattaa 69839

tggcggtggg tgttggaaac aattttagct tattctcgcg cgtaacatct acccccgcca 69899

ccaatgttaa atgcgtcacg gggagggaca cgagataatc tgcgagcgta gggtcctcca 69959

cttcaacatc aaatgttccg caaaggtcgc gatccaccgc cccgatcccc gctgcaagta 70019

aggccactcg atccaaaaac acgcagttat tattggatga taccgcccat gtcttcccgg 70079

tgcgattgag ctcacttcga acgtaactgg caacagatct gtcaccgggt ccgaccccgc 70139

gaacaacatg tccaaatttt gcgatctcgc ctccatgttt gcggggtatg gaaattaagc 70199

atcccccgca tataaaatac gccctggtag cacgctcgtt aaaataaaac gttacgccgt 70259

tataagatac ggttgaatga tatggaaatt ccatattaaa gcgtttatcg gaacattaac 70319

ctcgaacttg ccgtcccgtg atcgtgtgat cgccaacctt aggtccacac cgaatatgag 70379

aaatatataa ctacacgcaa acattcaaaa caccgtggta tcattaacgt catatgaaaa 70439

gatccaatca atccaatcaa ccacacctcc taccgtttag cacgtcagct atgtgacatg 70499

ctccaaacat acgtaaacat ttagagaggg tgttataaca gtctgtcagg cggggtatat 70559

tctacataat acaaggatcg gctttaactt tgtcaacatt tttactttgg actataaact 70619

gcgactgaac gtt atg aac cca ccc caa gcc cgc gtc tcg gaa cag 70665
Met Asn Pro Pro Gln Ala Arg Val Ser Glu Gln
                12090               12095

aca aag gac ttg ctt agc gtt atg gtt aac cag cac ccc gaa gag 70710
Thr Lys Asp Leu Leu Ser Val Met Val Asn Gln His Pro Glu Glu
            12100               12105               12110

gac gca aaa gtg tgt aaa tcc agt gat aat tca ccg ctt tat aac 70755
Asp Ala Lys Val Cys Lys Ser Ser Asp Asn Ser Pro Leu Tyr Asn
            12115               12120               12125

acc atg gtt atg tta tcg tat ggg ggt gat acg gac tta cta tta 70800
Thr Met Val Met Leu Ser Tyr Gly Gly Asp Thr Asp Leu Leu Leu
            12130               12135               12140

agc tct gca tgt acc cgc aca tct acc gta aac agg tcg gcg ttt 70845
Ser Ser Ala Cys Thr Arg Thr Ser Thr Val Asn Arg Ser Ala Phe
            12145               12150               12155

acg caa cac tcc gtg ttt tat att ata tcc acg gtg ttg att caa 70890
Thr Gln His Ser Val Phe Tyr Ile Ile Ser Thr Val Leu Ile Gln
            12160               12165               12170

cca ata tgt tgt atc ttc ttt ttt ttt tac tat aaa gcg aca cgc 70935

Pro Ile Cys Cys Ile Phe Phe Phe Phe Tyr Tyr Lys Ala Thr Arg
            12175               12180               12185

tgt atg ctc tta ttc aca gcc ggg tta ctt ctg acg att cta cat 70980
Cys Met Leu Leu Phe Thr Ala Gly Leu Leu Leu Thr Ile Leu His
            12190               12195               12200

cac ttt cga ctt att att atg tta ttg tgt gtc tac aga aat ata 71025
His Phe Arg Leu Ile Ile Met Leu Leu Cys Val Tyr Arg Asn Ile
            12205               12210               12215

cga tca gac ctg cta ccc tta tct aca tcc cag caa ctg ctg ctt 71070
Arg Ser Asp Leu Leu Pro Leu Ser Thr Ser Gln Gln Leu Leu Leu
            12220               12225               12230

gga att att gtt gtg act cga aca atg cta ttt tgt att acg gcg 71115
Gly Ile Ile Val Val Thr Arg Thr Met Leu Phe Cys Ile Thr Ala
            12235               12240               12245

tat tat act ctt ttt ata gac acc cgg gtg ttc ttt ttg att acc 71160
Tyr Tyr Thr Leu Phe Ile Asp Thr Arg Val Phe Phe Leu Ile Thr
            12250               12255               12260

gga cac ttg caa agt gag gtt att ttt cca gat agc gtt tca aaa 71205
Gly His Leu Gln Ser Glu Val Ile Phe Pro Asp Ser Val Ser Lys
            12265               12270               12275

ata ctt cct gtg tcg tgg ggt cca agt cca gcc gtg tta ctg gta 71250
Ile Leu Pro Val Ser Trp Gly Pro Ser Pro Ala Val Leu Leu Val
            12280               12285               12290

atg gcg gca gtt att tac gct atg gac tgt ttg gtg gac acg gta 71295
Met Ala Ala Val Ile Tyr Ala Met Asp Cys Leu Val Asp Thr Val
            12295               12300               12305

tcc ttt att ggg cca agg gtg tgg gtc cgt gtt atg tta aaa aca 71340
Ser Phe Ile Gly Pro Arg Val Trp Val Arg Val Met Leu Lys Thr
            12310               12315               12320

tct att tcg ttt tag tccatttcaa taaatgtact ataattgttc agtctaaaaa 71395
Ser Ile Ser Phe
            12325

taatgttggg tatttataat taccgccccc gtgttacttg gaaacaccca tacatatgtt 71455

ccactctaca tcaaacttct cgcagttttc ttgttcccgc acacgtttac acgtccggat 71515

tcaagtcgca acgctgctga caaa atg aca acg gtt tca tgt ccc gct aac 71566
Met Thr Thr Val Ser Cys Pro Ala Asn
                12330

gtg att act aca acg gaa tct gat cgt att gct ggg tta ttt aac 71611
Val Ile Thr Thr Thr Glu Ser Asp Arg Ile Ala Gly Leu Phe Asn
12335               12340               12345

atc cca gcg ggg atc att cca act gga aat gtg ctg tca acc ata 71656
Ile Pro Ala Gly Ile Ile Pro Thr Gly Asn Val Leu Ser Thr Ile
12350               12355               12360

gag gtg tgt gca cac cgt tgc att ttt gat ttt ttt aaa caa ata 71701
Glu Val Cys Ala His Arg Cys Ile Phe Asp Phe Phe Lys Gln Ile
12365               12370               12375

cga tca gat gat aac agc ctt tac tcg gct caa ttc gat att ctt 71746
Arg Ser Asp Asp Asn Ser Leu Tyr Ser Ala Gln Phe Asp Ile Leu
12380               12385               12390

ttg ggg aca tac tgc aat aca tta aac ttt gtg cgt ttt cta gaa 71791

Leu Gly Thr Tyr Cys Asn Thr Leu Asn Phe Val Arg Phe Leu Glu
12395               12400               12405

ctt gga ctg tct gtc gct tgc atc tgt act aaa ttt ccg gag ctg 71836
Leu Gly Leu Ser Val Ala Cys Ile Cys Thr Lys Phe Pro Glu Leu
12410               12415               12420

gct tac gtg cga gat ggc gtt att caa ttt gag gta caa caa ccc 71881
Ala Tyr Val Arg Asp Gly Val Ile Gln Phe Glu Val Gln Gln Pro
12425               12430               12435

atg ata gca cgt gat ggc cca cat ccc gtc gat cag cct gtt cat 71926
Met Ile Ala Arg Asp Gly Pro His Pro Val Asp Gln Pro Val His
12440               12445               12450

aat tat atg gtt aag cgg ata cac aag cgt tcg tta agc gct gcg 71971
Asn Tyr Met Val Lys Arg Ile His Lys Arg Ser Leu Ser Ala Ala
12455               12460               12465

ttt gca att gca tcg gaa gcg ttg agt ttg tta agt aac aca tat 72016
Phe Ala Ile Ala Ser Glu Ala Leu Ser Leu Leu Ser Asn Thr Tyr
12470               12475               12480

gtc gat ggg aca gag att gac tca tcg tta cgt ata aga gct atc 72061
Val Asp Gly Thr Glu Ile Asp Ser Ser Leu Arg Ile Arg Ala Ile
12485               12490               12495

caa cag atg gct cgt aat tta cgc acc gtt ttg gac tca ttt gaa 72106
Gln Gln Met Ala Arg Asn Leu Arg Thr Val Leu Asp Ser Phe Glu
12500               12505               12510

cga ggc act gcc gat caa ctt ctt ggt gtt cta ttg gag aaa gcc 72151
Arg Gly Thr Ala Asp Gln Leu Leu Gly Val Leu Leu Glu Lys Ala
12515               12520               12525

cca ccg cta tcg ctg ctt tca cca att aat aaa ttc caa ccc gag 72196
Pro Pro Leu Ser Leu Leu Ser Pro Ile Asn Lys Phe Gln Pro Glu
12530               12535               12540

gga cat cta aat cgt gtt gca cgc gcg gcc cta ctt tcg gac ctc 72241
Gly His Leu Asn Arg Val Ala Arg Ala Ala Leu Leu Ser Asp Leu
12545               12550               12555

aaa cgt aga gtc tgt gcg gat atg ttt ttt atg acc cga cac gcc 72286
Lys Arg Arg Val Cys Ala Asp Met Phe Phe Met Thr Arg His Ala
12560               12565               12570

agg gaa cct agg ctg atc tct gcg tat ctg tcg gat atg gtt tcg 72331
Arg Glu Pro Arg Leu Ile Ser Ala Tyr Leu Ser Asp Met Val Ser
12575               12580               12585

tgc acc caa cca tcg gtg atg gta tca cga ata act cat aca aac 72376
Cys Thr Gln Pro Ser Val Met Val Ser Arg Ile Thr His Thr Asn
12590               12595               12600

act cgc gga cgg cag gtt gac ggt gtg ttg gta aca aca gca acc 72421
Thr Arg Gly Arg Gln Val Asp Gly Val Leu Val Thr Thr Ala Thr
12605               12610               12615

tta aaa cgg caa cta tta cag gga att tta caa att gac gac acc 72466
Leu Lys Arg Gln Leu Leu Gln Gly Ile Leu Gln Ile Asp Asp Thr
12620               12625               12630

gcc gct gac gta cca gta aca tat ggc gaa atg gtt cta cag ggg 72511
Ala Ala Asp Val Pro Val Thr Tyr Gly Glu Met Val Leu Gln Gly
12635               12640               12645

aca aac ttg gta acc gcc ctt gtg atg gga aag gcc gtc cgc gga 72556

Thr Asn Leu Val Thr Ala Leu Val Met Gly Lys Ala Val Arg Gly
12650               12655               12660

atg gat gat gta gcc cgc cat ctc ctt gat ata acc gac cct aac 72601
Met Asp Asp Val Ala Arg His Leu Leu Asp Ile Thr Asp Pro Asn
12665               12670               12675

acg tta aac ata ccg tct ata ccc cca caa tcc aac tcc gat tca 72646
Thr Leu Asn Ile Pro Ser Ile Pro Pro Gln Ser Asn Ser Asp Ser
12680               12685               12690

acg aca gct ggg ctt ccg gtt aac gcc cgt gtt cct gcg gat tta 72691
Thr Thr Ala Gly Leu Pro Val Asn Ala Arg Val Pro Ala Asp Leu
12695               12700               12705

gtg att gtt ggg gat aaa ctt gta ttc tta gaa gca tta gaa cgg 72736
Val Ile Val Gly Asp Lys Leu Val Phe Leu Glu Ala Leu Glu Arg
12710               12715               12720

cgg gtc tac caa gct acg cgc gtt gcc tac cct ctt att gga aat 72781
Arg Val Tyr Gln Ala Thr Arg Val Ala Tyr Pro Leu Ile Gly Asn
12725               12730               12735

ata gat att acg ttt atc atg cca atg gga gtg ttt cag gca aac 72826
Ile Asp Ile Thr Phe Ile Met Pro Met Gly Val Phe Gln Ala Asn
12740               12745               12750

tcc atg gac aga tat aca cga cac gcc ggc gat ttt tca act gta 72871
Ser Met Asp Arg Tyr Thr Arg His Ala Gly Asp Phe Ser Thr Val
12755               12760               12765

tcc gaa cag gat cca cgt caa ttt cca ccc caa ggg att ttt ttt 72916
Ser Glu Gln Asp Pro Arg Gln Phe Pro Pro Gln Gly Ile Phe Phe
12770               12775               12780

tat aat aaa gat ggg ata tta aca cag ttg act ctt cgt gat gca 72961
Tyr Asn Lys Asp Gly Ile Leu Thr Gln Leu Thr Leu Arg Asp Ala
12785               12790               12795

atg ggt acc atc tgc cac agt tca ttg ctt gat gtc gag gcc aca 73006
Met Gly Thr Ile Cys His Ser Ser Leu Leu Asp Val Glu Ala Thr
12800               12805               12810

ctt gtt gcc ctc cgc caa caa cat tta gat cgt cag tgt tat ttt 73051
Leu Val Ala Leu Arg Gln Gln His Leu Asp Arg Gln Cys Tyr Phe
12815               12820               12825

ggt gta tac gtg gcc gag ggt aca gag gac aca ttg gat gtt caa 73096
Gly Val Tyr Val Ala Glu Gly Thr Glu Asp Thr Leu Asp Val Gln
12830               12835               12840

atg ggg agg ttt atg gaa acg tgg gca gat atg atg cct cat cac 73141
Met Gly Arg Phe Met Glu Thr Trp Ala Asp Met Met Pro His His
12845               12850               12855

cct cat tgg gta aac gaa cat tta aca att cta cag ttt ata gct 73186
Pro His Trp Val Asn Glu His Leu Thr Ile Leu Gln Phe Ile Ala
12860               12865               12870

ccg agc aac ccg cgt cta agg ttt gaa tta aac ccc gcc ttt gat 73231
Pro Ser Asn Pro Arg Leu Arg Phe Glu Leu Asn Pro Ala Phe Asp
12875               12880               12885

ttt ttt gtt gca ccg ggg gac gta gac ctt ccc gga ccg cag cgt 73276
Phe Phe Val Ala Pro Gly Asp Val Asp Leu Pro Gly Pro Gln Arg
12890               12895               12900

ccc ccg gaa gcc atg cca acc gtt aac gca aca tta cgg att atc 73321

Pro Pro Glu Ala Met Pro Thr Val Asn Ala Thr Leu Arg Ile Ile
12905               12910               12915

aac gga aac att ccc gtg cct cta tgt ccc att tca ttt cga gac 73366
Asn Gly Asn Ile Pro Val Pro Leu Cys Pro Ile Ser Phe Arg Asp
12920               12925               12930

tgt cgc gga acc caa ctc ggt ttg gga aga cat aca atg acc ccg 73411
Cys Arg Gly Thr Gln Leu Gly Leu Gly Arg His Thr Met Thr Pro
12935               12940               12945

gca acc att aaa gcc gta aag gat aca ttt gaa gac cgc gca tac 73456
Ala Thr Ile Lys Ala Val Lys Asp Thr Phe Glu Asp Arg Ala Tyr
12950               12955               12960

cca act att ttc tac atg cta gag gct gtt att cat gga aac gaa 73501
Pro Thr Ile Phe Tyr Met Leu Glu Ala Val Ile His Gly Asn Glu
12965               12970               12975

aga aac ttc tgt gcg tta ctg cga ctg tta aca cag tgt att cgc 73546
Arg Asn Phe Cys Ala Leu Leu Arg Leu Leu Thr Gln Cys Ile Arg
12980               12985               12990

ggg tat tgg gag caa tcc cac agg gtg gca ttt gta aat aac ttt 73591
Gly Tyr Trp Glu Gln Ser His Arg Val Ala Phe Val Asn Asn Phe
12995               13000               13005

cac atg tta atg tac ata act aca tat ctc gga aac ggt gag ctt 73636
His Met Leu Met Tyr Ile Thr Thr Tyr Leu Gly Asn Gly Glu Leu
13010               13015               13020

ccc gaa gtc tgt att aat ata tat cgg gat tta ctg cag cat gta 73681
Pro Glu Val Cys Ile Asn Ile Tyr Arg Asp Leu Leu Gln His Val
13025               13030               13035

aga gca tta cgc caa act ata acc gat ttt aca ata caa gga gag 73726
Arg Ala Leu Arg Gln Thr Ile Thr Asp Phe Thr Ile Gln Gly Glu
13040               13045               13050

ggc cat aac ggc gag acc tcg gaa gcg cta aat aac atc ctt acg 73771
Gly His Asn Gly Glu Thr Ser Glu Ala Leu Asn Asn Ile Leu Thr
13055               13060               13065

gat gac acg ttt att gca cct att cta tgg gat tgt gat gcg tta 73816
Asp Asp Thr Phe Ile Ala Pro Ile Leu Trp Asp Cys Asp Ala Leu
13070               13075               13080

ata tac cgt gat gaa gcc gcc cga gac cga ctc ccc gca att cgt 73861
Ile Tyr Arg Asp Glu Ala Ala Arg Asp Arg Leu Pro Ala Ile Arg
13085               13090               13095

gta agc ggg cga aac gga tac caa gcc ctt cac ttt gtg gat atg 73906
Val Ser Gly Arg Asn Gly Tyr Gln Ala Leu His Phe Val Asp Met
13100               13105               13110

gcc ggg cat aac ttc caa cga cgc gat aat gtg tta atc cac ggg 73951
Ala Gly His Asn Phe Gln Arg Arg Asp Asn Val Leu Ile His Gly
13115               13120               13125

aga ccc gtt cgg gga gac acg ggt cag ggt att ccc att act cca 73996
Arg Pro Val Arg Gly Asp Thr Gly Gln Gly Ile Pro Ile Thr Pro
13130               13135               13140

cac cat gac cgt gaa tgg ggt att ctc tcc aag att tac tac tat 74041
His His Asp Arg Glu Trp Gly Ile Leu Ser Lys Ile Tyr Tyr Tyr
13145               13150               13155

att gtc att cct gca ttt tcc cgc ggt tcc tgt tgt aca atg ggc 74086

Ile Val Ile Pro Ala Phe Ser Arg Gly Ser Cys Cys Thr Met Gly
13160               13165               13170

gtg cgt tat gat cgc cta tac cct gcg tta cag gca gtt atc gtt 74131
Val Arg Tyr Asp Arg Leu Tyr Pro Ala Leu Gln Ala Val Ile Val
13175               13180               13185

ccg gaa att ccc gct gat gaa gaa gcc cca act acc cca gaa gat 74176
Pro Glu Ile Pro Ala Asp Glu Glu Ala Pro Thr Thr Pro Glu Asp
13190               13195               13200

cca aga cac cct ctt cac gca cac caa ctc gtt ccg aac tct ctt 74221
Pro Arg His Pro Leu His Ala His Gln Leu Val Pro Asn Ser Leu
13205               13210               13215

aac gtt tac ttc cat aat gca cac cta acc gtt gat ggt gat gca 74266
Asn Val Tyr Phe His Asn Ala His Leu Thr Val Asp Gly Asp Ala
13220               13225               13230

ttg ctc aca cta caa gag tta atg gga gat atg gct gaa cga acg 74311
Leu Leu Thr Leu Gln Glu Leu Met Gly Asp Met Ala Glu Arg Thr
13235               13240               13245

acg gcc att tta gta tca agc gcc ccc gat gcg gga gcc gcc acg 74356
Thr Ala Ile Leu Val Ser Ser Ala Pro Asp Ala Gly Ala Ala Thr
13250               13255               13260

gca aca acc aga aat atg aga ata tat gac gga gcg ctt tac cat 74401
Ala Thr Thr Arg Asn Met Arg Ile Tyr Asp Gly Ala Leu Tyr His
13265               13270               13275

ggc ctt att atg atg gca tat cag gcg tac gat gaa acc att gca 74446
Gly Leu Ile Met Met Ala Tyr Gln Ala Tyr Asp Glu Thr Ile Ala
13280               13285               13290

acg ggt act ttt ttt tat ccc gtt ccg gtc aac cct ctg ttt gca 74491
Thr Gly Thr Phe Phe Tyr Pro Val Pro Val Asn Pro Leu Phe Ala
13295               13300               13305

tgt ccg gaa cat ttg gca tca ttg cgt gga atg aca aat gct agg 74536
Cys Pro Glu His Leu Ala Ser Leu Arg Gly Met Thr Asn Ala Arg
13310               13315               13320

cgg gtt ttg gca aaa atg gta cca cca atc cct cct ttt ctg gga 74581
Arg Val Leu Ala Lys Met Val Pro Pro Ile Pro Pro Phe Leu Gly
13325               13330               13335

gcc aac cac cac gca act ata cgc caa ccc gtt gcc tac cat gta 74626
Ala Asn His His Ala Thr Ile Arg Gln Pro Val Ala Tyr His Val
13340               13345               13350

acg cat agt aag tcg gat ttt aat act ctt aca tat tct ctt ctt 74671
Thr His Ser Lys Ser Asp Phe Asn Thr Leu Thr Tyr Ser Leu Leu
13355               13360               13365

gga ggg tat ttt aag ttt aca cca ata tct ctt aca cat caa cta 74716
Gly Gly Tyr Phe Lys Phe Thr Pro Ile Ser Leu Thr His Gln Leu
13370               13375               13380

cga acg gga ttt cac ccc ggg att gcc ttt acc gta gtg cgc cag 74761
Arg Thr Gly Phe His Pro Gly Ile Ala Phe Thr Val Val Arg Gln
13385               13390               13395

gat cgc ttt gcc aca gag caa ctt tta tat gcc gag cgt gct tct 74806
Asp Arg Phe Ala Thr Glu Gln Leu Leu Tyr Ala Glu Arg Ala Ser
13400               13405               13410

gaa tcg tac ttt gtc gga caa atc caa gta cac cat cat gat gct 74851

Glu Ser Tyr Phe Val Gly Gln Ile Gln Val His His His Asp Ala
13415               13420               13425

att ggg ggg gta aac ttt acc cta acc caa ccc aga gct cac gtg 74896
Ile Gly Gly Val Asn Phe Thr Leu Thr Gln Pro Arg Ala His Val
13430               13435               13440

gac ctg gga gtc ggg tat aca gct gta tgt gcc aca gca gcc ctg 74941
Asp Leu Gly Val Gly Tyr Thr Ala Val Cys Ala Thr Ala Ala Leu
13445               13450               13455

cga tgc cct ctc acg gat atg ggc aat act gcc caa aat ctt ttt 74986
Arg Cys Pro Leu Thr Asp Met Gly Asn Thr Ala Gln Asn Leu Phe
13460               13465               13470

ttt tca cga gga gga gtg cca atg tta cat gat aac gtt acc gaa 75031
Phe Ser Arg Gly Gly Val Pro Met Leu His Asp Asn Val Thr Glu
13475               13480               13485

tcg ttg cgt cgt ata aca gca tcg ggg ggt cgc tta aat ccc acc 75076
Ser Leu Arg Arg Ile Thr Ala Ser Gly Gly Arg Leu Asn Pro Thr
13490               13495               13500

gaa ccc cta ccc atc ttc ggc gga cta cgt cct gct aca tcg gca 75121
Glu Pro Leu Pro Ile Phe Gly Gly Leu Arg Pro Ala Thr Ser Ala
13505               13510               13515

gga att gca cga ggg caa gcc tct gtg tgt gag ttt gtg gcc atg 75166
Gly Ile Ala Arg Gly Gln Ala Ser Val Cys Glu Phe Val Ala Met
13520               13525               13530

ccg gtg tcc act gac cta caa tat ttt aga act gca tgc aat cct 75211
Pro Val Ser Thr Asp Leu Gln Tyr Phe Arg Thr Ala Cys Asn Pro
13535               13540               13545

aga ggt cga gca tct gga atg tta tat atg ggt gac cgt gac gcc 75256
Arg Gly Arg Ala Ser Gly Met Leu Tyr Met Gly Asp Arg Asp Ala
13550               13555               13560

gac ata gag gct ata atg ttt gat cac aca caa tcg gat gtt gct 75301
Asp Ile Glu Ala Ile Met Phe Asp His Thr Gln Ser Asp Val Ala
13565               13570               13575

tat aca gat cga gca act ctt aac cca tgg gca tca caa aaa cat 75346
Tyr Thr Asp Arg Ala Thr Leu Asn Pro Trp Ala Ser Gln Lys His
13580               13585               13590

tca tac ggt gac agg cta tac aac gga aca tac aac ctt aca ggc 75391
Ser Tyr Gly Asp Arg Leu Tyr Asn Gly Thr Tyr Asn Leu Thr Gly
13595               13600               13605

gct tct cct atc tac agc cca tgc ttt aag ttt ttt aca cca gcg 75436
Ala Ser Pro Ile Tyr Ser Pro Cys Phe Lys Phe Phe Thr Pro Ala
13610               13615               13620

gag gtt aac act aat tgt aat aca ctg gat cgg ctt cta atg gag 75481
Glu Val Asn Thr Asn Cys Asn Thr Leu Asp Arg Leu Leu Met Glu
13625               13630               13635

gca aag gct gtg gcg tcg caa agc tcc acc gac act gaa tat caa 75526
Ala Lys Ala Val Ala Ser Gln Ser Ser Thr Asp Thr Glu Tyr Gln
13640               13645               13650

ttt aaa cgc cct ccc ggt tct acc gaa atg aca cag gat ccg tgt 75571
Phe Lys Arg Pro Pro Gly Ser Thr Glu Met Thr Gln Asp Pro Cys
13655               13660               13665

ggc ctt ttt caa gaa gca tat cca cca cta tgc tca agc gat gcg 75616

Gly Leu Phe Gln Glu Ala Tyr Pro Pro Leu Cys Ser Ser Asp Ala
13670               13675               13680

gcc atg tta cga acg gct cac gcg gga gaa acc ggg gca gat gaa 75661
Ala Met Leu Arg Thr Ala His Ala Gly Glu Thr Gly Ala Asp Glu
13685               13690               13695

gtt cac tta gcc caa tat ctg att cga gac gcg tcg ccc ctt agg 75706
Val His Leu Ala Gln Tyr Leu Ile Arg Asp Ala Ser Pro Leu Arg
13700               13705               13710

gga tgt ctt cct ctt ccg cga taa tttcaccacg cccacatacc 75750
Gly Cys Leu Pro Leu Pro Arg
13715               13720

cactcccaat aaaagccctg tagagcgcat tggcatctta cttgagattt ggatacgctc 75810

ggccgacttg gtctgtttca cgcttcctta aacaac atg gct atg cca ttt gag 75864
Met Ala Met Pro Phe Glu
            13725

ata gag gta ttg tta cca gga gaa cta tcc ccg gcg gaa aca tct 75909
Ile Glu Val Leu Leu Pro Gly Glu Leu Ser Pro Ala Glu Thr Ser
        13730               13735               13740

gca tta cag aaa tgt gag gga aaa att att acc ttc tca acc ctg 75954
Ala Leu Gln Lys Cys Glu Gly Lys Ile Ile Thr Phe Ser Thr Leu
        13745               13750               13755

cgt cat cga gct tca ctg gtg gat ata gcg ctg tcg tca tat tac 75999
Arg His Arg Ala Ser Leu Val Asp Ile Ala Leu Ser Ser Tyr Tyr
        13760               13765               13770

att aac ggt gct cca cca gac acg ctc tcg ctg tta gag gca tac 76044
Ile Asn Gly Ala Pro Pro Asp Thr Leu Ser Leu Leu Glu Ala Tyr
        13775               13780               13785

cga atg cga ttc gcg gca gtt ata aca cgg gtc atc ccg gga aag 76089
Arg Met Arg Phe Ala Ala Val Ile Thr Arg Val Ile Pro Gly Lys
        13790               13795               13800

ttg ttg gcg cat gcc att ggc gtg ggt act cct aca ccc ggg ttg 76134
Leu Leu Ala His Ala Ile Gly Val Gly Thr Pro Thr Pro Gly Leu
        13805               13810               13815

ttt att caa aat aca tcc ccc gtt gat ctt tgt aat ggc gat tac 76179
Phe Ile Gln Asn Thr Ser Pro Val Asp Leu Cys Asn Gly Asp Tyr
        13820               13825               13830

atc tgc tta ctt cct ccg gtt ttc ggg tcc gca gac tca att cgc 76224
Ile Cys Leu Leu Pro Pro Val Phe Gly Ser Ala Asp Ser Ile Arg
        13835               13840               13845

ttg gac tct gta gga ctg gaa att gtt ttc cct tta acc atc ccc 76269
Leu Asp Ser Val Gly Leu Glu Ile Val Phe Pro Leu Thr Ile Pro
        13850               13855               13860

cag acc tta atg cga gaa atc atc gcc aaa gtg gtt gca cgg gcc 76314
Gln Thr Leu Met Arg Glu Ile Ile Ala Lys Val Val Ala Arg Ala
        13865               13870               13875

gtt gag cgc acg gcc gcg ggt gct caa att tta ccc cac gaa gtt 76359
Val Glu Arg Thr Ala Ala Gly Ala Gln Ile Leu Pro His Glu Val
        13880               13885               13890

cta cga ggc gcg gat gtc att tgt tac aat gga agg cgt tat gaa 76404
Leu Arg Gly Ala Asp Val Ile Cys Tyr Asn Gly Arg Arg Tyr Glu
        13895               13900               13905

ctc gaa aca aat tta caa cat cgg gac gga tcg gat gcg gct att 76449
Leu Glu Thr Asn Leu Gln His Arg Asp Gly Ser Asp Ala Ala Ile
        13910               13915               13920

cgc aca ttg gtt tta aat cta atg ttt tcc ata aac gag gga tgt 76494
Arg Thr Leu Val Leu Asn Leu Met Phe Ser Ile Asn Glu Gly Cys
        13925               13930               13935

ctg ctt tta ttg gcg ctg att cca act ttg tta gtc caa gga gca 76539
Leu Leu Leu Leu Ala Leu Ile Pro Thr Leu Leu Val Gln Gly Ala
        13940               13945               13950

cac gac ggt tat gta aat tta ttg ata caa acg gcc aat tgc gtt 76584
His Asp Gly Tyr Val Asn Leu Leu Ile Gln Thr Ala Asn Cys Val
        13955               13960               13965

aga gaa acc ggc cag tta att aat ata ccg cca atg ccg cgg att 76629
Arg Glu Thr Gly Gln Leu Ile Asn Ile Pro Pro Met Pro Arg Ile
        13970               13975               13980

caa gac ggc cat cgc cga ttt ccc ata tat gaa act att tca tct 76674
Gln Asp Gly His Arg Arg Phe Pro Ile Tyr Glu Thr Ile Ser Ser
        13985               13990               13995

tgg ata tca aca tca tct aga ctg ggg gat acc ttg gga act cgc 76719
Trp Ile Ser Thr Ser Ser Arg Leu Gly Asp Thr Leu Gly Thr Arg
        14000               14005               14010

gca att tta cgc gtc tgt gtg ttt gat gga ccc tct act gtt cat 76764
Ala Ile Leu Arg Val Cys Val Phe Asp Gly Pro Ser Thr Val His
        14015               14020               14025

ccg gga gac cgc acg gcc gtg att caa gtg taa acaggtgtta 76807
Pro Gly Asp Arg Thr Ala Val Ile Gln Val
        14030               14035

ataaaaacac aaccagtcta gttacatttc acgcgtcttg tttttattta ataggcataa 76867

acacggaatc cggtatacat gaactgccaa tatacacgga cataattaat gcaaccatca 76927

gatcatctga cattgttccc gtggtacctt tacccgtgta agtttttgtg tctagattac 76987

ccataccgcc tttaattacc tctgtcaggt tatccaactg tttacataga tactccacgg 77047

ggtctacacc taactttact gttagggata caagctcctg tgaggctatt atatttccgg 77107

agttaaatcg tttaacaaaa tagtctacgg ccggcgtttt ttgtttttgt aataaaaaaa 77167

aagggtacgc cacgctacat ccgggaggta tggaatgata aaacagtaac actggagcgg 77227

aagatagcac gtttcccttt tcgaggacag caaactgttg tgctatagcc aacgatatgg 77287

caactgcaga atcctggctg ctgtttccct ctatagaaac gtgtacgttt gtaaatgtat 77347

tggggtgtaa agcgagtatg tggcctaagc attgagtaac gcaacgccct atctcactgg 77407

aagacgtgcc agttaaagct ctaagaaaaa agtgctccaa tccaaatata atccaatccg 77467

acttataacg accaacaatc gctacaccag taccagacgc tcgtgtattt gaggtaaatg 77527

cagggtctac gtaaacgtac aacactgacg ataatatagc acaattcgca acggttgacg 77587

gccgatataa aataaacctc tcacgggcag tttttgtaaa taatggccgg tcaaacccca 77647

cacccccaga attctgttta cgcccaccta caatttcctg cacgaaggag tcggccataa 77707

ataaatctgc agtgcgccgc atggctccat ccattgtgat gaaaaccggc ttatttaata 77767

cataacacga acaagctgtg acatcgctat gtgctaaaac acgcggcatg tgatcgtcgc 77827

atacatatgt aacaacgttt aacaactgat ccgacgatcc acgtaagtta tacaaaaaac 77887

ttgtacttgc ttttccggta tttgttgatg aaacaaaaat aattttacaa ttggtttgat 77947

ttaaaaatcc gactatagtt tgtacagcat caggtcgaat aaaattagct tcatccacaa 78007

acagaagatt aaaatcttga cctcggatac cctggaacga tagaaagata tatagttacc 78067

ccaccaaagt ttaaatgtat ccttaaatac cacgtacgta aaaaatgttt gaatacgtac 78127

atatttcttt ttttttcca gtacaaccat atccggtgta ta atg gaa gcc cat 78181
Met Glu Ala His
        14040

ttg gca aat gaa acc aaa cat gca ctt tgg cat aat gat cac aca 78226
Leu Ala Asn Glu Thr Lys His Ala Leu Trp His Asn Asp His Thr
            14045               14050               14055

aaa gga tta cta cac gtt gtg ata cct aac gcg ggg ctt att gcg 78271
Lys Gly Leu Leu His Val Val Ile Pro Asn Ala Gly Leu Ile Ala
            14060               14065               14070

gcc gga ata gat ccc gca tta ctg att tta aag aaa ccc gga caa 78316
Ala Gly Ile Asp Pro Ala Leu Leu Ile Leu Lys Lys Pro Gly Gln
            14075               14080               14085

cgc ttc aag gtt gaa gta caa aca aga tat cat gct aca ggt caa 78361
Arg Phe Lys Val Glu Val Gln Thr Arg Tyr His Ala Thr Gly Gln
            14090               14095               14100

tgc gaa ccg tgg tgt caa gtt ttc gcc gcg tac att ccc gat aac 78406
Cys Glu Pro Trp Cys Gln Val Phe Ala Ala Tyr Ile Pro Asp Asn
            14105               14110               14115

gcc tta aca aat ctc tta ata cca aaa acg gaa cca ttt gtt tca 78451
Ala Leu Thr Asn Leu Leu Ile Pro Lys Thr Glu Pro Phe Val Ser
            14120               14125               14130

cac gtt ttt tcg gcc acg cat aat tca ggg gga ttg att tta tca 78496
His Val Phe Ser Ala Thr His Asn Ser Gly Gly Leu Ile Leu Ser
            14135               14140               14145

ttg cct gtt tat ctt agc ccc ggt tta ttc ttt gat gca ttt aac 78541
Leu Pro Val Tyr Leu Ser Pro Gly Leu Phe Phe Asp Ala Phe Asn
            14150               14155               14160

gtt gta gcg ata cga ata aat act gga aac cgc aag cac cgt gat 78586
Val Val Ala Ile Arg Ile Asn Thr Gly Asn Arg Lys His Arg Asp
            14165               14170               14175

att tgt att atg tat gca gaa cta atc cca aac gga acg cgt tat 78631
Ile Cys Ile Met Tyr Ala Glu Leu Ile Pro Asn Gly Thr Arg Tyr
            14180               14185               14190

ttt gct gat gga caa cgg gta ctt tta tta tgc aaa cag ctg att 78676
Phe Ala Asp Gly Gln Arg Val Leu Leu Leu Cys Lys Gln Leu Ile
            14195               14200               14205

gcg tat atc cga tgc acc cct cgt ctt gca tcg tct ata aaa ata 78721
Ala Tyr Ile Arg Cys Thr Pro Arg Leu Ala Ser Ser Ile Lys Ile
            14210               14215               14220

tac gca gag cat atg gtg gca gcc atg ggt gaa tca cac acg tca 78766
Tyr Ala Glu His Met Val Ala Ala Met Gly Glu Ser His Thr Ser
            14225               14230               14235

aat ggg gac aat att gga ccc gtt tca tcc ata atc gat ctt gat 78811
Asn Gly Asp Asn Ile Gly Pro Val Ser Ser Ile Ile Asp Leu Asp
            14240               14245               14250

cga cag tta act tct gga ggt att gat gac tcc cct gct gaa aca 78856
Arg Gln Leu Thr Ser Gly Gly Ile Asp Asp Ser Pro Ala Glu Thr
            14255               14260               14265

cgc ata cag gaa aat aat cgg gac gtc ctt gag cta ata aaa cgg 78901
Arg Ile Gln Glu Asn Asn Arg Asp Val Leu Glu Leu Ile Lys Arg
            14270               14275               14280

gcc gta aac att gtt aac tcc agg cac ccc gtc cga cct tct agt 78946
Ala Val Asn Ile Val Asn Ser Arg His Pro Val Arg Pro Ser Ser
            14285               14290               14295

tcc cgc gtt gca tct ggg ttg ctt caa agt gca aag ggc cac gga 78991
Ser Arg Val Ala Ser Gly Leu Leu Gln Ser Ala Lys Gly His Gly
            14300               14305               14310

gcg caa act tcc aac aca gat ccg atc aat aac ggt tcc ttt gat 79036
Ala Gln Thr Ser Asn Thr Asp Pro Ile Asn Asn Gly Ser Phe Asp
            14315               14320               14325

ggc gtc ctt gag ccg cct gga caa ggg cga ttt acg gga aag aaa 79081
Gly Val Leu Glu Pro Pro Gly Gln Gly Arg Phe Thr Gly Lys Lys
            14330               14335               14340

aac aat tcg tcc gcc agc atc cca cct tta caa gac gtt cta ttg 79126
Asn Asn Ser Ser Ala Ser Ile Pro Pro Leu Gln Asp Val Leu Leu
            14345               14350               14355

ttt acc cca gct tcg aca gaa ccc caa agt ctt atg gaa tgg ttc 79171
Phe Thr Pro Ala Ser Thr Glu Pro Gln Ser Leu Met Glu Trp Phe
            14360               14365               14370

gac atc tgt tat gcc caa tta gtt agc ggg gac act cca gca gat 79216
Asp Ile Cys Tyr Ala Gln Leu Val Ser Gly Asp Thr Pro Ala Asp
            14375               14380               14385

ttc tgg aaa cgg cgt ccc cta tca att gta ccg cga cat tac gca 79261
Phe Trp Lys Arg Arg Pro Leu Ser Ile Val Pro Arg His Tyr Ala
            14390               14395               14400

gaa tcc ccc agt ccg ttg att gta gta tct tac aac gga tcc tct 79306
Glu Ser Pro Ser Pro Leu Ile Val Val Ser Tyr Asn Gly Ser Ser
            14405               14410               14415

gcc tgg gga gga cgt att acc gga agt cca att tta tat cac tct 79351
Ala Trp Gly Gly Arg Ile Thr Gly Ser Pro Ile Leu Tyr His Ser
            14420               14425               14430

gca cag gct att att gat gct gcg tgt ata aat gcc cgg gtt gac 79396
Ala Gln Ala Ile Ile Asp Ala Ala Cys Ile Asn Ala Arg Val Asp
            14435               14440               14445

aat ccc caa agc cta cat gtg aca gct cgc caa gag cta gtc gcg 79441
Asn Pro Gln Ser Leu His Val Thr Ala Arg Gln Glu Leu Val Ala
            14450               14455               14460

cgt tta ccg ttt ttg gct aac gtc cta aat aat caa acc ccc tta 79486
Arg Leu Pro Phe Leu Ala Asn Val Leu Asn Asn Gln Thr Pro Leu
            14465               14470               14475

ccc gcc ttt aaa cca ggc gcc gaa atg ttt tta aac cag gta ttt 79531
Pro Ala Phe Lys Pro Gly Ala Glu Met Phe Leu Asn Gln Val Phe
            14480               14485               14490

aaa caa gcg tgt gtg aca tcg cta acc caa ggt ctt ata acg gag 79576
Lys Gln Ala Cys Val Thr Ser Leu Thr Gln Gly Leu Ile Thr Glu
            14495               14500               14505

tta caa acg aac ccg act cta caa caa ctc atg gaa tat gat att 79621
Leu Gln Thr Asn Pro Thr Leu Gln Gln Leu Met Glu Tyr Asp Ile
            14510               14515               14520

gca gat tct tcc caa acg gtt att gat gaa att gta gcc cgc aca 79666
Ala Asp Ser Ser Gln Thr Val Ile Asp Glu Ile Val Ala Arg Thr
            14525               14530               14535

cca gac ctg att cag act ata gtt tcg gtg tta acg gaa atg tca 79711
Pro Asp Leu Ile Gln Thr Ile Val Ser Val Leu Thr Glu Met Ser
            14540               14545               14550

atg gat gcg ttt tat aac agc tcc ttg atg tat gcg gtt ttg gcg 79756
Met Asp Ala Phe Tyr Asn Ser Ser Leu Met Tyr Ala Val Leu Ala
            14555               14560               14565

tat ctg tca tct gta tat aca cga cca caa ggt ggg ggg tat ata 79801
Tyr Leu Ser Ser Val Tyr Thr Arg Pro Gln Gly Gly Gly Tyr Ile
            14570               14575               14580

ccc tac ctt cac gct tcc ttc cca tgc tgg tta ggt aat cgt tct 79846
Pro Tyr Leu His Ala Ser Phe Pro Cys Trp Leu Gly Asn Arg Ser
            14585               14590               14595

ata tat tta ttt gac tat tat aat tca gga ggg gaa ata ctt aag 79891
Ile Tyr Leu Phe Asp Tyr Tyr Asn Ser Gly Gly Glu Ile Leu Lys
            14600               14605               14610

ctt tcc aag gtc ccc gtt ccc gta gcc tta gaa aag gtt ggt att 79936
Leu Ser Lys Val Pro Val Pro Val Ala Leu Glu Lys Val Gly Ile
            14615               14620               14625

ggt aat tcc aca caa ctg agg ggt aaa ttt ata cgc agc gcg gat 79981
Gly Asn Ser Thr Gln Leu Arg Gly Lys Phe Ile Arg Ser Ala Asp
            14630               14635               14640

att gtt gat att gga att tgt tct aag tat tta ccc ggt caa tgt 80026
Ile Val Asp Ile Gly Ile Cys Ser Lys Tyr Leu Pro Gly Gln Cys
            14645               14650               14655

tac gcg tac att tgt cta gga ttt aac cag caa tta caa tcc att 80071
Tyr Ala Tyr Ile Cys Leu Gly Phe Asn Gln Gln Leu Gln Ser Ile
            14660               14665               14670

tta gtt tta ccg ggg gga ttt gcg gca tgt ttt tgt att acc gat 80116
Leu Val Leu Pro Gly Gly Phe Ala Ala Cys Phe Cys Ile Thr Asp
            14675               14680               14685

acc cta cag gca gca cta cct gca tcg tta atc gga cct att cta 80161
Thr Leu Gln Ala Ala Leu Pro Ala Ser Leu Ile Gly Pro Ile Leu
            14690               14695               14700

gac aga ttc tgc ttc tct att ccc aac ccc cat aaa taa 80200
Asp Arg Phe Cys Phe Ser Ile Pro Asn Pro His Lys
            14705               14710

attagtgtca ctataaaaac ataacaccag aatctcttca tatgtaattt tacgtcattt 80260

ctcccgtttc caccccctct taaaatataa aataaccggg tgggtggcat taaacccaca 80320

agtacccggg cggcaatccg ctagactgtt tttctgctc atg gaa tta caa cgc 80374
Met Glu Leu Gln Arg
    14715

ata ttt ccg ctg tac acc gct acg ggt gca gcg cgc aaa tta acc 80419
Ile Phe Pro Leu Tyr Thr Ala Thr Gly Ala Ala Arg Lys Leu Thr
    14720               14725               14730

ccc gag gca gtt cag aga ctc tgc gat gca tta acg ctg gat atg 80464
Pro Glu Ala Val Gln Arg Leu Cys Asp Ala Leu Thr Leu Asp Met
    14735               14740               14745

gga tta tgg aag tcc atc ctg acc gat ccc cgg gtg aaa ata atg 80509
Gly Leu Trp Lys Ser Ile Leu Thr Asp Pro Arg Val Lys Ile Met
    14750               14755               14760

cga tca act gct ttt ata act tta agg atc gct ccg ttt atc ccc 80554
Arg Ser Thr Ala Phe Ile Thr Leu Arg Ile Ala Pro Phe Ile Pro
    14765               14770               14775

ctt caa acg gat act act aat att gcc gtt gtt gta gcc aca att 80599
Leu Gln Thr Asp Thr Thr Asn Ile Ala Val Val Val Ala Thr Ile
    14780               14785               14790

tac atc acg cgc cca cgt cag atg aac tta cct ccg aag act ttt 80644
Tyr Ile Thr Arg Pro Arg Gln Met Asn Leu Pro Pro Lys Thr Phe
    14795               14800               14805

cat gta att gta aat ttt aat tac gag gtc tcg tac gca atg acg 80689
His Val Ile Val Asn Phe Asn Tyr Glu Val Ser Tyr Ala Met Thr
    14810               14815               14820

gcg act tta aga att tat ccg gtt gaa aac ata gac cat gtt ttt 80734
Ala Thr Leu Arg Ile Tyr Pro Val Glu Asn Ile Asp His Val Phe
    14825               14830               14835

gga gca acg ttt aag aac ccg atc gcg tac ccc ctt cca aca tct 80779
Gly Ala Thr Phe Lys Asn Pro Ile Ala Tyr Pro Leu Pro Thr Ser
    14840               14845               14850

att ccg gat cct cga gca gat ccc acc ccc gca gat ctt aca cca 80824
Ile Pro Asp Pro Arg Ala Asp Pro Thr Pro Ala Asp Leu Thr Pro
    14855               14860               14865

acg cca aac tta agc aac tac tta caa ccc ccg cgg ctt ccg aaa 80869
Thr Pro Asn Leu Ser Asn Tyr Leu Gln Pro Pro Arg Leu Pro Lys
    14870               14875               14880

aat cca tac gca tgt aaa gtt att tct ccg gga gtg tgg tgg tca 80914
Asn Pro Tyr Ala Cys Lys Val Ile Ser Pro Gly Val Trp Trp Ser
    14885               14890               14895

gac gaa cga agg cgt tta tat gta ctg gct atg gaa cct aat tta 80959
Asp Glu Arg Arg Arg Leu Tyr Val Leu Ala Met Glu Pro Asn Leu
    14900               14905               14910

ata ggg cta tgt ccc gcc gga tgg cat gct cgg ata ctt ggc tct 81004
Ile Gly Leu Cys Pro Ala Gly Trp His Ala Arg Ile Leu Gly Ser
    14915               14920               14925

gta tta aat cga ctc ctc agc cat gcg gac gga tgt gat gaa tgt 81049
Val Leu Asn Arg Leu Leu Ser His Ala Asp Gly Cys Asp Glu Cys
    14930               14935               14940

aat cat aga gtt cac gtg ggg gca ctg tat gcg tta ccc cat gtc 81094
Asn His Arg Val His Val Gly Ala Leu Tyr Ala Leu Pro His Val
    14945               14950               14955

aca aat cat gcg gaa ggt tgt gtg tgt tgg gct ccg tgt atg tgg 81139
Thr Asn His Ala Glu Gly Cys Val Cys Trp Ala Pro Cys Met Trp
    14960               14965               14970

aga aag gcc ggt cag cgg gaa tta aaa gtg gag gta gac att ggc 81184
Arg Lys Ala Gly Gln Arg Glu Leu Lys Val Glu Val Asp Ile Gly
    14975               14980               14985

gcc acg cag gtt ctt ttt gta gat gtc acc acc tgc att cga att 81229
Ala Thr Gln Val Leu Phe Val Asp Val Thr Thr Cys Ile Arg Ile
    14990               14995               15000

acg agt act aaa aat cct cgc att acc gca aat ctt ggc gac gtt 81274
Thr Ser Thr Lys Asn Pro Arg Ile Thr Ala Asn Leu Gly Asp Val
    15005               15010               15015

ata gcg gga acc aac gcc agt ggt ctc tct gta cca gta aat tca 81319
Ile Ala Gly Thr Asn Ala Ser Gly Leu Ser Val Pro Val Asn Ser
    15020               15025               15030

tct ggg tgg cag ctt tat atg ttt gga gaa aca tta agc cgg gct 81364
Ser Gly Trp Gln Leu Tyr Met Phe Gly Glu Thr Leu Ser Arg Ala
    15035               15040               15045

att att aac ggc tgt ggt ctg ctt cag cga att tgc ttc ccc gag 81409
Ile Ile Asn Gly Cys Gly Leu Leu Gln Arg Ile Cys Phe Pro Glu
    15050               15055               15060

aca caa aga tta tcg ggt gaa ccg gaa cct aca acc acc tag 81451
Thr Gln Arg Leu Ser Gly Glu Pro Glu Pro Thr Thr Thr
    15065               15070               15075

tataccttaa ctcaaccgcc gttgtggaaa ggtatatgtc aacatttaca gtaatatatt 81511

aaaggttaaa tttataaaac actcacgttt gtgttgtgac ttgacgcgaa caccgctgtg 81571

ctgtaagacc cgtcggtaaa tgaaaacgta atagattcgc cttttacatg atccacgtaa 81631

tttgccccaa accactgttc caggcgagac ttgataccct caaacacggg ttccgttgct 81691

ttgcgtatat gagccgtata acccacttta attcctctaa acgtggccat tactaaagct 81751

attaatggta caagaaacca tgttttccca tgtctacgtg gtaccaaaaa cacagttgat 81811

ttttgtttga agtgttctaa aacactgtca gaaacacttg gcgtgttaaa cactgtacgc 81871

agaaagcagt caactctgtc ggcatgatcg cccaatagca ccgatgaaat aaaatgcgtg 81931

gtgtgcatga ggatcatttt ttgaaacagt tccaacgtcc ccttatatct gccatagatt 81991

ggaacgtcaa cctttgcgcg tttgccatga cttccacact cttcaatact ctcaaaagat 82051

gtttccacaa ggtacgaaaa ccgttgtgta aaggtagaca actgacagaa actatccgac 82111

agagaaaacg cgcgaaatgt gttcataaca ccgctatacg catttcgatg aggtgctgct 82171

tcttccggtg aatattcata aaactgtaca ctactgacag ccttttttaa ttcagggctt 82231

acgtttgcat ttaccgaata tcgccatggt ttcaaaacta cattgggggt acagttgtac 82291

cctgttgacg atagaaacgc gccaaacatt gcccgtcgag cagtagccga gaacagtgga 82351

atatattcac aacagttgtg aagcgttcca attccgggaa taacggcctg atgacgtcgg 82411

gttacatcta tagcaaaatt cagaaacggg atttgggttg cgtttcccag agacccttgc 82471

cgcgtggaac acggggtagg ggactccaac gtcccaaagc gttcatccct acgacgcttt 82531

agacgttcaa aatatcttac agattcttca ccaagcgtac gaccaaacat tatcaatgac 82591

atttaacatc aattcacgga atccgcctca tctcttgtaa gcagtaaaac aggaagccgc 82651

gtcatcttac gtactcgtta cgtatatatc ataaacattt tcagggccgc attcattcac 82711

tttggtc atg tca ggc cac act cca acc tac gct tct cat agg cgt 82757
Met Ser Gly His Thr Pro Thr Tyr Ala Ser His Arg Arg
            15080               15085

aac cgt gtc aaa cta gtt gag gcg cat aac cgc gcg ggg tta ttt 82802
Asn Arg Val Lys Leu Val Glu Ala His Asn Arg Ala Gly Leu Phe
15090               15095               15100

aaa gaa cgg acc ctc gat cta atc cgt ggg ggt gcg agt gta caa 82847
Lys Glu Arg Thr Leu Asp Leu Ile Arg Gly Gly Ala Ser Val Gln
15105               15110               15115

gat cca gca ttt gtg tat gcc ttt act gct gca aaa gag gcc tgc 82892
Asp Pro Ala Phe Val Tyr Ala Phe Thr Ala Ala Lys Glu Ala Cys
15120               15125               15130

gcc gat tta aat aac cag ctc cgc tct gca gct cgc ata gct tca 82937
Ala Asp Leu Asn Asn Gln Leu Arg Ser Ala Ala Arg Ile Ala Ser
15135               15140               15145

gtt gaa cag aag att cgt gat ata caa tcc aag gtt gag gaa caa 82982
Val Glu Gln Lys Ile Arg Asp Ile Gln Ser Lys Val Glu Glu Gln
15150               15155               15160

aca agt att caa cag att tta aat aca aac aga cgc tat ata gca 83027
Thr Ser Ile Gln Gln Ile Leu Asn Thr Asn Arg Arg Tyr Ile Ala
15165               15170               15175

ccc gat ttt att cgc ggt ttg gat aaa aca gaa gac gat aat acc 83072
Pro Asp Phe Ile Arg Gly Leu Asp Lys Thr Glu Asp Asp Asn Thr
15180               15185               15190

gat aat ata gac aga ctg gaa gac gcg gta gga ccg aac atc gaa 83117
Asp Asn Ile Asp Arg Leu Glu Asp Ala Val Gly Pro Asn Ile Glu
15195               15200               15205

cac gaa aat cat act tgg ttt gga gaa gac gac gaa gcg tta ctt 83162
His Glu Asn His Thr Trp Phe Gly Glu Asp Asp Glu Ala Leu Leu
15210               15215               15220

aca caa tgg atg ctg acg aca cac ccc cca acc tcc aaa tat ctc 83207
Thr Gln Trp Met Leu Thr Thr His Pro Pro Thr Ser Lys Tyr Leu
15225               15230               15235

caa ctg cag gac ctt tgc gtt ccc acc aca ata ccg acg gac atg 83252
Gln Leu Gln Asp Leu Cys Val Pro Thr Thr Ile Pro Thr Asp Met
15240               15245               15250

aac caa atg caa ccg cag ccg atc agc aag aac gag aat cca cca 83297
Asn Gln Met Gln Pro Gln Pro Ile Ser Lys Asn Glu Asn Pro Pro
15255               15260               15265

acc cca cac acg gat gtg taa atcatccatg ggccaatccg tcaactgcaa 83348
Thr Pro His Thr Asp Val
15270               15275

catgcatgga atcaccagaa cgatcacaac agacaagctt atttttatta aagcacggct 83408

taacgagaga tccaatacat caacgcgaaa gggtggacgt ttttccacaa tttaacaaac 83468

ccccatgggt ttttagaatt tccaaattat cccgtttaat tgtacccatc ttcacgctca 83528

atgaacagtt atgtttttct aaattacaga ttcgagatag acccaggttt gcgggacggg 83588

gaacgtatgg gcgtgttcat atatacccat cgtcaaaaat agctgtaaaa accatggaca 83648

gtcgtgtttt taatagagag ttaattaacg cgattttagc gagtgagggt tctatacgag 83708

caggggaaag gctaggtatt tctagcatag tttgcctttt aggtttttcg ttacaaacca 83768

aacagctact gtttccggca tacgacatgg atatggatga atacattgtt cgcctgtcca 83828

gacggttgac aatacctgat cacatagaca gaaaaattgc ccatgtattt ttagatttgg 83888

ctcaagcgtt gacgttttta aatcgaacgt gcggcctgac ccacctagat gtgaaatgtg 83948

gcaatatttt tcttaacgtc gacaactttg cctcgttgga aataaccaca gcagtaatcg 84008

gagactatag cctagtaaca ttaaatacgt attccctttg tactcgagcg atatttgaag 84068

ttggaaatcc atcccacccg gagcacgtac tacgcgtacc ccgggatgca tcgcagatgt 84128

catttcgttt ggtgttgagt catggaacaa accaaccccc tgaaatcttg cttgattata 84188

ttaatggaac gggccttact aaatatactg gaaccttgcc ccaaagagtt ggacttgcga 84248

ttgatcttta tgcattgggc caagcactct tagaagttat cctgctagga cgtcttcccg 84308

gacaactgcc catttcagta catcggaccc cgcattatca ctactacggt cataagttat 84368

caccagattt ggcgcttgat acgctggcat atcgatgtgt cctggcgcca tatatactcc 84428

catctgacat ccccggggac ttaaattata atccctttat acacgccgga gagctgaaca 84488

cccgtatttc ccggaattct ttacgccgga tattccagtg tcacgcagtg cgttacggcg 84548

taacgcactc aaagcttttc gaaggcatac gcattccggc ctcattatac ccagccactg 84608

ttgttacatc gttgttgtgt cacgataatt cagaaatacg ctcggatcac cctttatt 84666

atg gca cga tcg gga ttg gat agg atc gac ata agc ccc cag cca 84711
Met Ala Arg Ser Gly Leu Asp Arg Ile Asp Ile Ser Pro Gln Pro
                15280               15285               15290

gcc aaa aaa att gcc cgt gtg gga ggt cta cag cac cct ttt gta 84756
Ala Lys Lys Ile Ala Arg Val Gly Gly Leu Gln His Pro Phe Val
                15295               15300               15305

aaa acg gat att aac acg att aac gtt gaa cac cat ttt ata gac 84801
Lys Thr Asp Ile Asn Thr Ile Asn Val Glu His His Phe Ile Asp
                15310               15315               15320

acg cta cag aag aca tca ccg aac atg gac tgt cgc ggg atg aca 84846
Thr Leu Gln Lys Thr Ser Pro Asn Met Asp Cys Arg Gly Met Thr
                15325               15330               15335

gcg ggt att ttt att cgt tta tcc cac atg tat aaa att cta aca 84891
Ala Gly Ile Phe Ile Arg Leu Ser His Met Tyr Lys Ile Leu Thr
                15340               15345               15350

act ctg gag tct cca aat gat gta acc tac aca aca ccc ggt tct 84936
Thr Leu Glu Ser Pro Asn Asp Val Thr Tyr Thr Thr Pro Gly Ser
                15355               15360               15365

acc aac gca ctg ttc ttt aag acg tcc aca cag cct cag gag ccg 84981
Thr Asn Ala Leu Phe Phe Lys Thr Ser Thr Gln Pro Gln Glu Pro
                15370               15375               15380

cgt ccg gaa gag tta gca tcc aaa tta acc caa gac gac att aaa 85026
Arg Pro Glu Glu Leu Ala Ser Lys Leu Thr Gln Asp Asp Ile Lys
                15385               15390               15395

cgt att cta tta aca ata gaa tcg gag act cgt ggt cag ggc gac 85071

Arg Ile Leu Leu Thr Ile Glu Ser Glu Thr Arg Gly Gln Gly Asp
                15400               15405               15410

aat gcc att tgg aca cta ctc aga cga aat tta atc acc gca tca 85116
Asn Ala Ile Trp Thr Leu Leu Arg Arg Asn Leu Ile Thr Ala Ser
                15415               15420               15425

act ctt aaa tgg agt gta tct gga ccc gtc att cca cct cag tgg 85161
Thr Leu Lys Trp Ser Val Ser Gly Pro Val Ile Pro Pro Gln Trp
                15430               15435               15440

ttt tac cac cat aac act aca gac aca tac ggt gat gcg gcg gca 85206
Phe Tyr His His Asn Thr Thr Asp Thr Tyr Gly Asp Ala Ala Ala
                15445               15450               15455

atg gcg ttt gga aaa acc aac gaa ccg gcg gca cga gcg ata gtt 85251
Met Ala Phe Gly Lys Thr Asn Glu Pro Ala Ala Arg Ala Ile Val
                15460               15465               15470

gaa gca ttg ttt ata gat ccg gct gat atc cgt act cct gat cat 85296
Glu Ala Leu Phe Ile Asp Pro Ala Asp Ile Arg Thr Pro Asp His
                15475               15480               15485

tta acg cca gaa gct aca act aag ttt ttt aat ttt gac atg ctc 85341
Leu Thr Pro Glu Ala Thr Thr Lys Phe Phe Asn Phe Asp Met Leu
                15490               15495               15500

aat acc aaa tct cca agt ctc ctt gtg ggt aca cca aga atc gga 85386
Asn Thr Lys Ser Pro Ser Leu Leu Val Gly Thr Pro Arg Ile Gly
                15505               15510               15515

acg tat gaa tgt gga ctt tta atc gac gtt cga acg gga ctt ata 85431
Thr Tyr Glu Cys Gly Leu Leu Ile Asp Val Arg Thr Gly Leu Ile
                15520               15525               15530

ggc gcg tcg ttg gac gtt ctt gta tgt gac agg gac cct tta act 85476
Gly Ala Ser Leu Asp Val Leu Val Cys Asp Arg Asp Pro Leu Thr
                15535               15540               15545

ggc acc cta aat ccc cac cct gca gaa acc gac att tca ttt ttt 85521
Gly Thr Leu Asn Pro His Pro Ala Glu Thr Asp Ile Ser Phe Phe
                15550               15555               15560

gaa att aaa tgt cgt gct aaa tac ctc ttt gat cca gat gac aaa 85566
Glu Ile Lys Cys Arg Ala Lys Tyr Leu Phe Asp Pro Asp Asp Lys
                15565               15570               15575

aat aac ccg ctc ggt cgg acg tac acc acg tta ata aat aga cct 85611
Asn Asn Pro Leu Gly Arg Thr Tyr Thr Thr Leu Ile Asn Arg Pro
                15580               15585               15590

aca atg gca aat cta cgg gac ttt tta tat act ata aaa aac cca 85656
Thr Met Ala Asn Leu Arg Asp Phe Leu Tyr Thr Ile Lys Asn Pro
                15595               15600               15605

tgt gta agc ttc ttt gga ccc tca gca aac cca agt aca cgc gag 85701
Cys Val Ser Phe Phe Gly Pro Ser Ala Asn Pro Ser Thr Arg Glu
                15610               15615               15620

gcc tta ata acg gat cac gtt gaa tgg aaa cgt tta gga ttt aaa 85746
Ala Leu Ile Thr Asp His Val Glu Trp Lys Arg Leu Gly Phe Lys
                15625               15630               15635

ggt ggg agg gcc ctt aca gaa ctc gac gcc cat cat ttg ggc ctc 85791
Gly Gly Arg Ala Leu Thr Glu Leu Asp Ala His His Leu Gly Leu
                15640               15645               15650

aat cgg aca atc tca tcc cga gtg tgg gta ttt aat gat ccg gac 85836

Asn Arg Thr Ile Ser Ser Arg Val Trp Val Phe Asn Asp Pro Asp
                15655               15660               15665

ata caa aag ggg aca att aca acc att gca tgg gcc act gga gat 85881
Ile Gln Lys Gly Thr Ile Thr Thr Ile Ala Trp Ala Thr Gly Asp
                15670               15675               15680

acg gct ctt caa att cct gta ttt gcc aat ccg cgg cac gct aac 85926
Thr Ala Leu Gln Ile Pro Val Phe Ala Asn Pro Arg His Ala Asn
                15685               15690               15695

ttt aaa caa att gcc gta caa acc tat gta tta tcc ggt tac ttt 85971
Phe Lys Gln Ile Ala Val Gln Thr Tyr Val Leu Ser Gly Tyr Phe
                15700               15705               15710

cca gcg cta aaa cta cgg ccc ttc ctt gtc acc ttt ata gga cgt 86016
Pro Ala Leu Lys Leu Arg Pro Phe Leu Val Thr Phe Ile Gly Arg
                15715               15720               15725

gtg cgc cga cca cac gag gtg gga gtc cca ttg cgc gtc gat aca 86061
Val Arg Arg Pro His Glu Val Gly Val Pro Leu Arg Val Asp Thr
                15730               15735               15740

caa gcg gct gcc att tac gaa tat aac tgg ccg act atc cca ccc 86106
Gln Ala Ala Ala Ile Tyr Glu Tyr Asn Trp Pro Thr Ile Pro Pro
                15745               15750               15755

cac tgt gcg gtt ccg gtt ata gcc gtt cta acg cct atc gaa gtt 86151
His Cys Ala Val Pro Val Ile Ala Val Leu Thr Pro Ile Glu Val
                15760               15765               15770

gat gtg cct aga gtg aca caa ata ctt aaa gac aca gga aac aac 86196
Asp Val Pro Arg Val Thr Gln Ile Leu Lys Asp Thr Gly Asn Asn
                15775               15780               15785

gcg att aca tca gca ttg cgg tca ttg cga tgg gac aat ctt cat 86241
Ala Ile Thr Ser Ala Leu Arg Ser Leu Arg Trp Asp Asn Leu His
                15790               15795               15800

cca gcg gtc gag gag gaa tct gtg gat tgt gca aac ggt aca acg 86286
Pro Ala Val Glu Glu Glu Ser Val Asp Cys Ala Asn Gly Thr Thr
                15805               15810               15815

agc ttg tta cgt gca acg gag aaa ccg ttg ctt tga actcagagtt 86332
Ser Leu Leu Arg Ala Thr Glu Lys Pro Leu Leu
                15820               15825

ctttgaagac tttgactttg atgagaatgt aacagaggac gccgataaat ccacacaacg 86392

ccgcccacga gtgatcgatg taacaccaaa acgaaaacct tcgggaaaga gctcccattc 86452

caaatgcgca aaatgttaaa ccctgataaa ccctgataaa cgttctaata aaaacatcaa 86512

atcatggttg gttactgtga atgtttgttt tattgcttgg gggtttacaa gtacaaccca 86572

cgctactccc acccactgtt tgatcgctcg tataacagct catcctcgcg gtccgtttca 86632

tatgttgagt cattttcata gacgtagccg tagccttgtg atgggtaatt tgtgcggcga 86692

gaatttctat gtgcaggttt tacttttcgt atgtatcccc gtacccgctc gggtactctt 86752

cttacggcac cgtagaaccg actgcgtttc tgtcgatgat acacatatgc acgcatcaat 86812

ctgagaagca acatgacaac ggaaaacacg gccaggcaag ccaaggttcc ccgagttgtg 86872

ggaattaacc gtggagattg aaccgatata gggtcatata atcggtccat atacgagtgc 86932

gcggcggttc ccaacgtagc acaggccacg agcgttccca gggacggtcc tattaacacg 86992

tgtatataat gcgccaaaat taattctgat actataagat atacaactga caatgtacta 87052

aatgtagaca tggccacgga caccgatgac cacagtcccg tatgtagatg attcgccacc 87112

acaagttcca gcattaatga tacaaatagg atacatatcg ccatcaacgc agccatcaaa 87172

ttcacgaaca ctgcgcgcgt aggccccgca aggcgatata aaaagacgct ctgctgtcgt 87232

aaatttgcga ccgcttttat gttcgtttcg tccaattttc cgcgtccaca aaaatacgtt 87292

gtaaatatta cacttgtcgc aaaatgtcca agatataatg tagcagccac gccgatttgc 87352

ttgtaagcta ataataacac aacggcgttt aataaccaca atgacaaaag accccaaaaa 87412

agtgttgtgg gatctacaac taaccatgca acaccggagc tttgccggac acgttgattt 87472

ttcgtttctc ggtgtataat cgcggccgtg atcagtgtat ataccgccat ggccattgcc 87532

gttaaagccg tgtagtaagt aaatgccaca acgctatgtg gttccaaaaa caaaaccggg 87592

gcgctgtatc cacctctatt tccggaccat acccccccat ctagggtggc gttaaataac 87652

tcataatcaa ctacggcagc ataaaaacaa gggatcccgg tatattcaga agaggcggca 87712

attaacgtag ccaggagcat taccgcaccc aaagtgaaca tcatcacctg aattatccaa 87772

attcgccaat taagcgtatc catttgatga tctaacgctt ccacctcggg tgtcgtggtg 87832

tcgtacggcg agactttttc agaacgcggc cccttctttt gagttccc atg tct ccc 87889
Met Ser Pro

aac acc ggg gag agc aac gcc gcc gtc tat gcg tcc agt aca cag 87934
Asn Thr Gly Glu Ser Asn Ala Ala Val Tyr Ala Ser Ser Thr Gln
15830               15835               15840

ctc gcg cgg gcg tta tat gga ggg gat ctg gtt tcg tgg att aaa 87979
Leu Ala Arg Ala Leu Tyr Gly Gly Asp Leu Val Ser Trp Ile Lys
15845               15850               15855

cac acc cac ccg gga att agc ctg gaa ctg caa ttg gat gtt cca 88024
His Thr His Pro Gly Ile Ser Leu Glu Leu Gln Leu Asp Val Pro
15860               15865               15870

gta aaa cta ata aaa cct ggt atg tca caa act cgc ccg gta acc 88069
Val Lys Leu Ile Lys Pro Gly Met Ser Gln Thr Arg Pro Val Thr
15875               15880               15885

gtc gta cgt gcc cct atg ggc tct ggt aaa aca aca gcc ttg ctt 88114
Val Val Arg Ala Pro Met Gly Ser Gly Lys Thr Thr Ala Leu Leu
15890               15895               15900

gag tgg ctt caa cac gcg tta aag gca gat att agc gta ctg gtt 88159
Glu Trp Leu Gln His Ala Leu Lys Ala Asp Ile Ser Val Leu Val
15905               15910               15915

gtc tca tgt cgc cgt agc ttt acc cag acg ttg att caa cgg ttt 88204
Val Ser Cys Arg Arg Ser Phe Thr Gln Thr Leu Ile Gln Arg Phe
15920               15925               15930

aac gat gca ggc ctc tcc gga ttc gta aca tat ttg aca tcc gag 88249
Asn Asp Ala Gly Leu Ser Gly Phe Val Thr Tyr Leu Thr Ser Glu
15935               15940               15945

aca tat att atg ggt ttt aaa cgt ttg att gtg caa ctt gaa agc 88294
Thr Tyr Ile Met Gly Phe Lys Arg Leu Ile Val Gln Leu Glu Ser
15950               15955               15960

cta cac cgc gta tcc agc gaa gct atc gac agc tac gac gta tta 88339
Leu His Arg Val Ser Ser Glu Ala Ile Asp Ser Tyr Asp Val Leu
15965               15970               15975

ata ctg gat gag gta atg tca gtg att gga caa tta tac tcc ccc 88384
Ile Leu Asp Glu Val Met Ser Val Ile Gly Gln Leu Tyr Ser Pro
15980               15985               15990

aca atg aga cgt ctt tcc gcg gtt gat agc cta tta tat cgt ctt 88429
Thr Met Arg Arg Leu Ser Ala Val Asp Ser Leu Leu Tyr Arg Leu
15995               16000               16005

tta aat cgc tgt tct caa att atc gcg atg gat gct aca gta aac 88474
Leu Asn Arg Cys Ser Gln Ile Ile Ala Met Asp Ala Thr Val Asn
16010               16015               16020

tcg cag ttt att gat tta atc tcc gga ttg cgt gga gat gaa aac 88519
Ser Gln Phe Ile Asp Leu Ile Ser Gly Leu Arg Gly Asp Glu Asn
16025               16030               16035

ata cac aca att gtg tgt aca tac gcg gga gtt ggg ttc tcc gga 88564
Ile His Thr Ile Val Cys Thr Tyr Ala Gly Val Gly Phe Ser Gly
16040               16045               16050

aga act tgc acg atc ctg cgt gat atg ggc atc gac acg ctt gtg 88609
Arg Thr Cys Thr Ile Leu Arg Asp Met Gly Ile Asp Thr Leu Val
16055               16060               16065

cga gtc att aaa cga tct cct gaa cac gag gat gta cgt acc ata 88654
Arg Val Ile Lys Arg Ser Pro Glu His Glu Asp Val Arg Thr Ile
16070               16075               16080

cac caa cta cgt gga aca ttt ttt gac gaa cta gca cta cga tta 88699
His Gln Leu Arg Gly Thr Phe Phe Asp Glu Leu Ala Leu Arg Leu
16085               16090               16095

caa tgt ggg cat aac atc tgt ata ttt tca tca act tta tcg ttt 88744
Gln Cys Gly His Asn Ile Cys Ile Phe Ser Ser Thr Leu Ser Phe
16100               16105               16110

tcg gag cta gtt gct cag ttt tgt gca ata ttt aca gac tct att 88789
Ser Glu Leu Val Ala Gln Phe Cys Ala Ile Phe Thr Asp Ser Ile
16115               16120               16125

ctt att tta aac tca act cgg ccc cta tgt aat gta aac gaa tgg 88834
Leu Ile Leu Asn Ser Thr Arg Pro Leu Cys Asn Val Asn Glu Trp
16130               16135               16140

aaa cat ttt cgc gtg ttg gtg tac act acc gtc gtg acc gtt gga 88879
Lys His Phe Arg Val Leu Val Tyr Thr Thr Val Val Thr Val Gly
16145               16150               16155

ttg agt ttt gac atg gct cat ttt cat agc atg ttt gct tac ata 88924
Leu Ser Phe Asp Met Ala His Phe His Ser Met Phe Ala Tyr Ile
16160               16165               16170

aag cca atg tca tat ggg ccg gat atg gta tcg gtc tac cag tca 88969
Lys Pro Met Ser Tyr Gly Pro Asp Met Val Ser Val Tyr Gln Ser
16175               16180               16185

tta ggg cgt gta cgt tta ttg cta ctt aat gaa gtt ttg atg tac 89014
Leu Gly Arg Val Arg Leu Leu Leu Leu Asn Glu Val Leu Met Tyr
16190               16195               16200

gtc gat ggc tca agg acc aga tgc gga ccc ctg ttc tcg cca atg 89059
Val Asp Gly Ser Arg Thr Arg Cys Gly Pro Leu Phe Ser Pro Met
16205               16210               16215

tta cta aac ttt acc atc gca aat aaa ttt caa tgg ttt cct aca 89104
Leu Leu Asn Phe Thr Ile Ala Asn Lys Phe Gln Trp Phe Pro Thr
16220               16225               16230

cac acc caa ata act aac aaa ctg tgc tgt gca ttt agg caa cga 89149
His Thr Gln Ile Thr Asn Lys Leu Cys Cys Ala Phe Arg Gln Arg
16235               16240               16245

tgt gca aat gca ttt aca cgc tcg aac acc cat ctc ttc tca aga 89194
Cys Ala Asn Ala Phe Thr Arg Ser Asn Thr His Leu Phe Ser Arg
16250               16255               16260

ttt aaa tac aaa cac ctt ttc gag aga tgc tct ctt tgg agt tta 89239
Phe Lys Tyr Lys His Leu Phe Glu Arg Cys Ser Leu Trp Ser Leu
16265               16270               16275

gcc gat agc att aat atc tta caa act ctt ttg gcc tct aac caa 89284
Ala Asp Ser Ile Asn Ile Leu Gln Thr Leu Leu Ala Ser Asn Gln
16280               16285               16290

att ttg gtt gta ttg gat ggc atg ggt cca ata acg gac gtt tcc 89329
Ile Leu Val Val Leu Asp Gly Met Gly Pro Ile Thr Asp Val Ser
16295               16300               16305

cca gtt caa ttt tgt gca ttt ata cac gat ctc aga cat agc gct 89374
Pro Val Gln Phe Cys Ala Phe Ile His Asp Leu Arg His Ser Ala
16310               16315               16320

aac gcc gta gct tcc tgt atg cgt tct ctt aga cag gac aat gac 89419
Asn Ala Val Ala Ser Cys Met Arg Ser Leu Arg Gln Asp Asn Asp
16325               16330               16335

agc tgc ttg acc gat ttt ggc cct tcc gga ttt atg gcc gat aac 89464
Ser Cys Leu Thr Asp Phe Gly Pro Ser Gly Phe Met Ala Asp Asn
16340               16345               16350

att acc gcg ttt atg gaa aag tat ctt atg gag tca att aat acc 89509
Ile Thr Ala Phe Met Glu Lys Tyr Leu Met Glu Ser Ile Asn Thr
16355               16360               16365

gaa gaa caa att aaa gta ttt aaa gcc ctt gca tgt cca ata gaa 89554
Glu Glu Gln Ile Lys Val Phe Lys Ala Leu Ala Cys Pro Ile Glu
16370               16375               16380

cag cct aga cta gtc aat acg gca ata ttg ggg gcg tgt ata cga 89599
Gln Pro Arg Leu Val Asn Thr Ala Ile Leu Gly Ala Cys Ile Arg
16385               16390               16395

ata cct gaa gcg ttg gaa gca ttt gac gta ttt caa aaa ata tac 89644
Ile Pro Glu Ala Leu Glu Ala Phe Asp Val Phe Gln Lys Ile Tyr
16400               16405               16410

acg cac tac gct tcc ggt tgg ttt ccc gtc ctg gac aaa acc ggg 89689
Thr His Tyr Ala Ser Gly Trp Phe Pro Val Leu Asp Lys Thr Gly
16415               16420               16425

gaa ttt agc atc gcg act ata act acc gcc cca aat tta acc aca 89734
Glu Phe Ser Ile Ala Thr Ile Thr Thr Ala Pro Asn Leu Thr Thr
16430               16435               16440

cat tgg gag ctg ttt cgc cgt tgt gcc tat att gca aaa aca ctc 89779
His Trp Glu Leu Phe Arg Arg Cys Ala Tyr Ile Ala Lys Thr Leu
16445               16450               16455

aag tgg aat ccg tcc acc gaa ggc tgt gta aca caa gtt ttg gat 89824
Lys Trp Asn Pro Ser Thr Glu Gly Cys Val Thr Gln Val Leu Asp
16460               16465               16470

acg gac att aat aca ctt ttc aat caa cac ggg gat tcg ctg gct 89869
Thr Asp Ile Asn Thr Leu Phe Asn Gln His Gly Asp Ser Leu Ala
16475               16480               16485

caa cta ata ttt gag gtt atg cgc tgt aac gtt act gac gct aag 89914
Gln Leu Ile Phe Glu Val Met Arg Cys Asn Val Thr Asp Ala Lys
16490               16495               16500

att ata tta aac cgc ccg gtt tgg cga aca acc gga ttc tta gat 89959
Ile Ile Leu Asn Arg Pro Val Trp Arg Thr Thr Gly Phe Leu Asp
16505               16510               16515

gga tgc cat aat caa tgc ttc cgt cca atc cct aca aaa cac gaa 90004
Gly Cys His Asn Gln Cys Phe Arg Pro Ile Pro Thr Lys His Glu
16520               16525               16530

tat aac att gct cta ttt cgt tta att tgg gaa caa tta ttt ggc 90049
Tyr Asn Ile Ala Leu Phe Arg Leu Ile Trp Glu Gln Leu Phe Gly
16535               16540               16545

gcc cgc gta act aaa agt acc cag acc ttt ccg gga agt act cgt 90094
Ala Arg Val Thr Lys Ser Thr Gln Thr Phe Pro Gly Ser Thr Arg
16550               16555               16560

gtg aaa aac cta aaa aaa aaa gat cta gaa act tta ctt gat tca 90139
Val Lys Asn Leu Lys Lys Lys Asp Leu Glu Thr Leu Leu Asp Ser
16565               16570               16575

att aac gtg gat cgt tct gca tgt cgt acc tac cgc cag ttg tat 90184
Ile Asn Val Asp Arg Ser Ala Cys Arg Thr Tyr Arg Gln Leu Tyr
16580               16585               16590

aac ctg ctt atg agc cag cgc cat tcg ttc tct caa cag cgt tac 90229
Asn Leu Leu Met Ser Gln Arg His Ser Phe Ser Gln Gln Arg Tyr
16595               16600               16605

aaa att act gcc ccc gct tgg gca cgc cac gtg tat ttt caa gca 90274
Lys Ile Thr Ala Pro Ala Trp Ala Arg His Val Tyr Phe Gln Ala
16610               16615               16620

cat caa atg cac ttg gcc ccg cat gcc gaa gcc atg cta caa tta 90319
His Gln Met His Leu Ala Pro His Ala Glu Ala Met Leu Gln Leu
16625               16630               16635

gcg cta tcg gaa ctg tcc ccg gga tcg tgg ccg cgg ata aac ggg 90364
Ala Leu Ser Glu Leu Ser Pro Gly Ser Trp Pro Arg Ile Asn Gly
16640               16645               16650

gcg gta aat ttt gaa agt tta taa cccgttaata ccatatatgg 90408
Ala Val Asn Phe Glu Ser Leu
16655               16660

acatccatag ggggggttac ataaatacta agcctctgta caacacaaag ggcctctaac 90468

aatgcactga accacaacca agct atg gac gca acg cag att acc ttg 90516
Met Asp Ala Thr Gln Ile Thr Leu
            16665

gtt aga gaa agc gga cac att tgt gcc gca agc ata tac aca tcc 90561
Val Arg Glu Ser Gly His Ile Cys Ala Ala Ser Ile Tyr Thr Ser
16670               16675               16680

tgg aca cag tcc gga caa tta aca cag aac ggt ctt tcc gtg tta 90606
Trp Thr Gln Ser Gly Gln Leu Thr Gln Asn Gly Leu Ser Val Leu
16685               16690               16695

tac tac tta tta tgc aaa aac tca tgt ggg aaa tac gtc cct aag 90651

Tyr Tyr Leu Leu Cys Lys Asn Ser Cys Gly Lys Tyr Val Pro Lys
16700               16705               16710

ttt gcc gaa att acc gta caa caa gag gat tta tgt cgc tac tcc 90696
Phe Ala Glu Ile Thr Val Gln Gln Glu Asp Leu Cys Arg Tyr Ser
16715               16720               16725

agg cat ggg ggg agt gtt tct gcg gca acg ttt gcg tct atc tgc 90741
Arg His Gly Gly Ser Val Ser Ala Ala Thr Phe Ala Ser Ile Cys
16730               16735               16740

agg gcg gcg tcc tcg gct gcg tta gac gcc tgg ccc ctt gaa cca 90786
Arg Ala Ala Ser Ser Ala Ala Leu Asp Ala Trp Pro Leu Glu Pro
16745               16750               16755

ctg ggt aac gca gac acc tgg cgt tgt ctc cat ggc act gcc ctg 90831
Leu Gly Asn Ala Asp Thr Trp Arg Cys Leu His Gly Thr Ala Leu
16760               16765               16770

gcc act tta cgg cgc gta tta ggg ttt aaa tcg ttt tat tcg cca 90876
Ala Thr Leu Arg Arg Val Leu Gly Phe Lys Ser Phe Tyr Ser Pro
16775               16780               16785

gta aca ttc gag act gat acg aat aca ggt ctt ctg tta aaa aca 90921
Val Thr Phe Glu Thr Asp Thr Asn Thr Gly Leu Leu Leu Lys Thr
16790               16795               16800

atc ccc gat gaa cac gcg ttg aat aat gac aac acg cca tct acc 90966
Ile Pro Asp Glu His Ala Leu Asn Asn Asp Asn Thr Pro Ser Thr
16805               16810               16815

gga gta ttg agg gct aat ttt ccc gtg gcc att gat gtt tca gca 91011
Gly Val Leu Arg Ala Asn Phe Pro Val Ala Ile Asp Val Ser Ala
16820               16825               16830

gtc agc gca tgt aac gcc cac acg caa ggt acg tcg cta gcc tac 91056
Val Ser Ala Cys Asn Ala His Thr Gln Gly Thr Ser Leu Ala Tyr
16835               16840               16845

gcc cgc ctg acc gca ctt aaa tct aac ggt gac acc cag caa caa 91101
Ala Arg Leu Thr Ala Leu Lys Ser Asn Gly Asp Thr Gln Gln Gln
16850               16855               16860

aca cct tta gac gtg gag gta att aca cca aag gcc tac ata cgt 91146
Thr Pro Leu Asp Val Glu Val Ile Thr Pro Lys Ala Tyr Ile Arg
16865               16870               16875

cgg aaa tat aag tct acg ttt tcc ccc cct ata gag cgg gaa ggc 91191
Arg Lys Tyr Lys Ser Thr Phe Ser Pro Pro Ile Glu Arg Glu Gly
16880               16885               16890

caa acc tcc gat ttg ttt aac ctt gaa gaa cgc cgc ttg gtt ctt 91236
Gln Thr Ser Asp Leu Phe Asn Leu Glu Glu Arg Arg Leu Val Leu
16895               16900               16905

agt ggc aat cgc gca att gtg gta agg gta ctc tta ccg tgt tat 91281
Ser Gly Asn Arg Ala Ile Val Val Arg Val Leu Leu Pro Cys Tyr
16910               16915               16920

ttt gac tgt tta aca acg gat tcc acc gtt aca tct tcc ctt tca 91326
Phe Asp Cys Leu Thr Thr Asp Ser Thr Val Thr Ser Ser Leu Ser
16925               16930               16935

ata tta gca aca tat aga ctg tgg tac gcg gcg gcg ttt gga aaa 91371
Ile Leu Ala Thr Tyr Arg Leu Trp Tyr Ala Ala Ala Phe Gly Lys
16940               16945               16950

ccc ggg gtt gtc cgt cca atc ttt gcg tat tta ggc ccg gaa ctc 91416

Pro Gly Val Val Arg Pro Ile Phe Ala Tyr Leu Gly Pro Glu Leu
16955               16960               16965

aat ccg aag ggt gaa gac aga gac tac ttt tgt act gtc gga ttt 91461
Asn Pro Lys Gly Glu Asp Arg Asp Tyr Phe Cys Thr Val Gly Phe
16970               16975               16980

ccc gga tgg acc act ctt cgg aca caa act cca gcc gtc gaa tct 91506
Pro Gly Trp Thr Thr Leu Arg Thr Gln Thr Pro Ala Val Glu Ser
16985               16990               16995

att cgc acg gct acg gag atg tac atg gaa acg gat ggg ttg tgg 91551
Ile Arg Thr Ala Thr Glu Met Tyr Met Glu Thr Asp Gly Leu Trp
17000               17005               17010

cca gta acc ggt att cag gcc ttt cat tat cta gcc ccc tgg gga 91596
Pro Val Thr Gly Ile Gln Ala Phe His Tyr Leu Ala Pro Trp Gly
17015               17020               17025

cag cat ccc ccc tta cct ccg cgg gtg cag gat ctt att ggg caa 91641
Gln His Pro Pro Leu Pro Pro Arg Val Gln Asp Leu Ile Gly Gln
17030               17035               17040

atc cct caa gat act gga cat gca gat gca act gtc aat tgg gac 91686
Ile Pro Gln Asp Thr Gly His Ala Asp Ala Thr Val Asn Trp Asp
17045               17050               17055

gcg ggc cgg ata tct acc gtc ttc aaa cag cct gta caa cta caa 91731
Ala Gly Arg Ile Ser Thr Val Phe Lys Gln Pro Val Gln Leu Gln
17060               17065               17070

gat cgt tgg atg gca aag ttt gat ttc agc gcc ttt ttt ccc acg 91776
Asp Arg Trp Met Ala Lys Phe Asp Phe Ser Ala Phe Phe Pro Thr
17075               17080               17085

ata tac tgc gct atg ttc ccc atg cat ttt aga tta ggc aaa atc 91821
Ile Tyr Cys Ala Met Phe Pro Met His Phe Arg Leu Gly Lys Ile
17090               17095               17100

gtc ctg gct aga atg cgt cga gga atg ggg tgc cta aaa ccc gcg 91866
Val Leu Ala Arg Met Arg Arg Gly Met Gly Cys Leu Lys Pro Ala
17105               17110               17115

ttg gtg tct ttt ttt ggg ggg tta cgg cac ata ctc ccg agt ata 91911
Leu Val Ser Phe Phe Gly Gly Leu Arg His Ile Leu Pro Ser Ile
17120               17125               17130

tac aaa gct att att ttt ata gcc aat gaa att agc ctt tgc gtc 91956
Tyr Lys Ala Ile Ile Phe Ile Ala Asn Glu Ile Ser Leu Cys Val
17135               17140               17145

gaa caa acg gcc ttg gaa cag ggc ttt gct ata tgt act tat ata 92001
Glu Gln Thr Ala Leu Glu Gln Gly Phe Ala Ile Cys Thr Tyr Ile
17150               17155               17160

aaa gat gga ttt tgg gga atc ttc acc gat tta cat acg cgc aat 92046
Lys Asp Gly Phe Trp Gly Ile Phe Thr Asp Leu His Thr Arg Asn
17165               17170               17175

gta tgt tca gat cag gca cgt tgt tcg gcc tta aat tta gcg gcc 92091
Val Cys Ser Asp Gln Ala Arg Cys Ser Ala Leu Asn Leu Ala Ala
17180               17185               17190

acc tgc gaa aga gca gtc acg ggc tta tta cga att caa cta ggt 92136
Thr Cys Glu Arg Ala Val Thr Gly Leu Leu Arg Ile Gln Leu Gly
17195               17200               17205

ctt aac ttt aca ccc gcc atg gaa ccg gta ctc cgg gtc gag ggt 92181

Leu Asn Phe Thr Pro Ala Met Glu Pro Val Leu Arg Val Glu Gly
17210               17215               17220

gtg tac act cac gca ttt acc tgg tgt acc acg gga agc tgg ctg 92226
Val Tyr Thr His Ala Phe Thr Trp Cys Thr Thr Gly Ser Trp Leu
17225               17230               17235

tgg aat tta caa aca aac acg cct ccg gat tta gtt ggc gtg cca 92271
Trp Asn Leu Gln Thr Asn Thr Pro Pro Asp Leu Val Gly Val Pro
17240               17245               17250

tgg cga agt cag gcg gcg cga gat tta aag gag cgt ctt tca gga 92316
Trp Arg Ser Gln Ala Ala Arg Asp Leu Lys Glu Arg Leu Ser Gly
17255               17260               17265

ctc cta tgt acc gca aca aaa att cga gaa cgg ata cag gaa aat 92361
Leu Leu Cys Thr Ala Thr Lys Ile Arg Glu Arg Ile Gln Glu Asn
17270               17275               17280

tgc ata tgg gac cat gtc cta tac gac ata tgg gcc gga caa gtt 92406
Cys Ile Trp Asp His Val Leu Tyr Asp Ile Trp Ala Gly Gln Val
17285               17290               17295

gtg gag gct gcc aga aaa aca tac gtc gat ttt ttt gaa cat gtt 92451
Val Glu Ala Ala Arg Lys Thr Tyr Val Asp Phe Phe Glu His Val
17300               17305               17310

ttt gat cgc cgt tat act ccg gta tac tgg agt ctt cag gag caa 92496
Phe Asp Arg Arg Tyr Thr Pro Val Tyr Trp Ser Leu Gln Glu Gln
17315               17320               17325

aat tcg gaa aca aaa gca ata ccg gca tct tat ctg aca tac gga 92541
Asn Ser Glu Thr Lys Ala Ile Pro Ala Ser Tyr Leu Thr Tyr Gly
17330               17335               17340

cac atg caa gat aag gat tat aaa cca aga cag ata att atg gtt 92586
His Met Gln Asp Lys Asp Tyr Lys Pro Arg Gln Ile Ile Met Val
17345               17350               17355

cgt aat ccc aac cca cat gga cct cct act gtt gtt tac tgg gaa 92631
Arg Asn Pro Asn Pro His Gly Pro Pro Thr Val Val Tyr Trp Glu
17360               17365               17370

ttg cta cca tcg tgt gcc tgt att ccc ccc ata gac tgc gct gct 92676
Leu Leu Pro Ser Cys Ala Cys Ile Pro Pro Ile Asp Cys Ala Ala
17375               17380               17385

cat ctc aag ccc ctt ata cac acg ttt gtc act att att aac cat 92721
His Leu Lys Pro Leu Ile His Thr Phe Val Thr Ile Ile Asn His
17390               17395               17400

ctt cta gat gct cat aat gat ttt tca agt cca tca ttg aaa ttt 92766
Leu Leu Asp Ala His Asn Asp Phe Ser Ser Pro Ser Leu Lys Phe
17405               17410               17415

act gac gat ccc ctt gct tca tat aac ttc ttg ttt tta tga 92808
Thr Asp Asp Pro Leu Ala Ser Tyr Asn Phe Leu Phe Leu
17420               17425               17430

caaaaaaaca cgccgcaaca acccatcctt aaaataaaag gtttatttac tttacaaccc 92868

gtggtgaatt tttatacgtt tcaaataact gaacattttt cggtgttacc atggtgcgat 92928

ttaaccacca aaaatatacg ctcttctgat attccgaatc tcgtaaaggt ccatttaaca 92988

atcccggggg tacttgcacc acaccatctg gacagggggg ggttccgtgg ggcaggtcaa 93048

aacgctgacc caccccacat gaatatatag cctttataat attgggggcc gttccaggct 93108

gagggttcag taacttaaca aacatataat gcggcaatac gcgggttttt gtaaaggggt 93168
tgttatcaac gacatacatt agagtgttta acaaccataa aactccctca tataaaaacc 93228
gacgcatttt ttccaaaggt cctatttgac actcaacgcg tctaagatat acagacaatt 93288
gtacaaacag cgatggagat gccccggagg gcccaatgcc ttccagatac attaaaataa 93348
cacataaggt aaaatctagg acattatccg ggcggaatag agtcatccga tagattaaca 93408
ggcgcggagg cacccccacc gtatacaccc tatcttcaac cgcagttaat acggaaaaaa 93468
taaatccgcg gaacgctggt tgagtaacac actccatgta gtaacgatca caggacacct 93528
cacttgaatc accattcaac actactaaaa cggtctcttg gtgttccggt tttacgcgca 93588
gtgatacaac agagtttgcc aaaaagcgtg gcttcaaacc ggttacctcc cgcgcctcgc 93648
atacgaatct tggtattgct tgtattctaa gatcttcgat cacgtcgctc acatccaacc 93708
cctcttcggc tcgtgttagt aagttgtcga tcgttacgct gcaacctaaa atgctgggta 93768
tatttattcc ggacatccca tcggccatcc ccgcgcctcc ggtttgctcg aattttatcc 93828
agtaaggtcg aatccgctgc atttaccttg tgtacccgta acctctcagg ggggtgtcct 93888
ttcataaaat gggataggtt tttatatcca acatgcatgt attggttatt tattttattg 93948
ggttccggga ttctttcgtc atcttctgta gggtcaggca aaccccagga aggacttggt 94008
gttctccgtg ggccccgttt tattacctct gcgcgaacct gcatttcata taatattcgg 94068
atttgggata aataggactc tgttctcgcc tttttaaaaa tagcctggca taactcttcc 94128
tctgacctat gtacctcgct ttgagttacc aagaatccta atcgggtggc ccgtaatatg 94188
aatgaaaaat acggcgcaac tagtaatgag attgacgcat ttgaatatga tacagaaatt 94248
tcctggcctt gattattgtt tacccggtga agcttaaaac agcgaacaag ttcctgtttc 94308
catagctcag acaaacgttt tatatcatct ccataagggg ggatataacg agattgaaaa 94368
ctattggcaa tatatgcatc atcccctatt atgccggtaa gatctataac ctcgtgattt 94428
aaatcggcaa tacgtgtttc ttctgccatt gtaatatgtg accctttaga tggctttatt 94488
tttaccctct cttcccgtaa ccgtttcagc tctccttctt tgaactggag cctttcggtc 94548
agatcgctgt tcacatcctt gagaccctca atggttttga ataaattatt cacataaccc 94608
tcgagcatgc cgttgatact gttaaccacc gaagttttaa acgcactttg aacgtttgtt 94668
gttccggaca ttgccccccc gttaaaggat tggttggcct tgccaaaccc cggttgtgat 94728
gtgtccaccg atccacttcc ttccagaatg tgattgcccg tttcttctag ataggaacgt 94788
acggtttcgg taatatctcc aacatgtctc atgtttttta agttaactat tagctttaca 94848
agtctagacg cggccgatcc agcccgtgtt gtatcgttct cgcccattat acgatcaacc 94908
gcacgtgtgc tgtgagatct atcatcttca ttccggcgac ctattaacac gcgcaaaggg 94968
gctgtattta aaacttggca gacgcgagca tgttcacgta atgcataaca ggccaacacc 95028
tccccagaaa gccgctgtaa gggtgagtca aatactacac cctccccaca tacaacgggc 95088
ggccacacga ccaaacactc tcccttcatg cccgttacat catcctttgc cataattaat 95148

cttcggttat aattataata aagacgcgtc ctatcataat ccataatagc aacattttgc 95208

atacactcaa ctaggcttgt gacaaccgcc gctcctctgg ccaacgttgc atcggcaact 95268

tttaacatct gggacagttc tgccgcttga cccatatacg tatttaatgg tgcaggggtt 95328

ccattctgtt ctgatcgtac ctttcttaca acgggcacaa tacctacaca ggctatccag 95388

tccacgtatt tggcaaaacc gacccttcca tttaaaccac tggtatagag acaaccggtt 95448

attccacgca gaaactcaag taacgatgac tgtaatgttt gacgccaggt ttcaaaaacc 95508

tgatgtgcaa gccgtacggc ttctgattct ccacatagcc cataacgttc cgctagagcc 95568

ccggcatgca ggttacattg ttggatgtgg tgttcccaat ctgctgctag gtcctcatac 95628

cgagttgcat ccaacgcgtt catcaaaacg gttgcctgaa cttggcgaat tacagtttcc 95688

gtagaccgta cagcgctata tatgccttgt ccatcggtat atccaaagtc accggctagg 95748

atttttcgaa acaacatact ttgcgtggtt gggtgtatta acatccagcc atcttcctcc 95808

ggaaatgtac aaaaccctat atccggggcg tactcattcc agtatatatc gaacatgttc 95868

ttgtattggt catttgggtt acttccattc aagccctggt caatagaaac agaacttgct 95928

atcctttttt cttcactacc ggaactgtta ttaaaaagag acgttatttc ggccattgaa 95988

aaccacg atg aaa aga tca att tct gta gac agt tct tca ccc aaa 96034
Met Lys Arg Ser Ile Ser Val Asp Ser Ser Ser Pro Lys
        17435               17440               17445

aac gtt ttt aat cca gag acg ccc aat gga ttt gat gac agt gta 96079
Asn Val Phe Asn Pro Glu Thr Pro Asn Gly Phe Asp Asp Ser Val
                17450               17455               17460

tat tta aac ttc acc tct atg cat agc att caa cct atc ctc tca 96124
Tyr Leu Asn Phe Thr Ser Met His Ser Ile Gln Pro Ile Leu Ser
                17465               17470               17475

cgg att cga gaa ctt gcc gca att acg att cca aaa gaa cgt gtt 96169
Arg Ile Arg Glu Leu Ala Ala Ile Thr Ile Pro Lys Glu Arg Val
                17480               17485               17490

ccg cgg ttg tgt tgg ttt aaa cag tta ctc gaa ctg caa gcg cct 96214
Pro Arg Leu Cys Trp Phe Lys Gln Leu Leu Glu Leu Gln Ala Pro
                17495               17500               17505

cct gaa atg cag agg aat gag ctc ccc ttc tcc gtt tat tta att 96259
Pro Glu Met Gln Arg Asn Glu Leu Pro Phe Ser Val Tyr Leu Ile
                17510               17515               17520

agc gga aat gcc ggc tcc gga aaa agc acg tgt atc caa acg ctt 96304
Ser Gly Asn Ala Gly Ser Gly Lys Ser Thr Cys Ile Gln Thr Leu
                17525               17530               17535

aac gaa gct atc gat tgc att att acc gga tcc acc agg gtt gct 96349
Asn Glu Ala Ile Asp Cys Ile Ile Thr Gly Ser Thr Arg Val Ala
                17540               17545               17550

gcc caa aat gtt cat gct aag tta tca acg gct tat gcg agt cgt 96394
Ala Gln Asn Val His Ala Lys Leu Ser Thr Ala Tyr Ala Ser Arg
                17555               17560               17565

ccg ata aac aca atc ttt cat gaa ttt ggt ttt cgc gga aat cac 96439
Pro Ile Asn Thr Ile Phe His Glu Phe Gly Phe Arg Gly Asn His
                17570               17575               17580

att cag gct cag ctg ggc cgt tac gca tat aac tgg act acg acc 96484
Ile Gln Ala Gln Leu Gly Arg Tyr Ala Tyr Asn Trp Thr Thr Thr
                17585               17590               17595

ccc cct tct att gag gac ctg caa aaa aga gat att gta tac tac 96529
Pro Pro Ser Ile Glu Asp Leu Gln Lys Arg Asp Ile Val Tyr Tyr
                17600               17605               17610

tgg gaa gtt tta att gat ata aca aaa cga gtg ttt caa atg ggg 96574
Trp Glu Val Leu Ile Asp Ile Thr Lys Arg Val Phe Gln Met Gly
                17615               17620               17625

gac gac ggt cgc gga gga aca tcg aca ttt aaa acc ctg tgg gca 96619
Asp Asp Gly Arg Gly Gly Thr Ser Thr Phe Lys Thr Leu Trp Ala
                17630               17635               17640

att gaa cgt ttg ctt aat aaa cct aca ggc tca atg tcc gga acc 96664
Ile Glu Arg Leu Leu Asn Lys Pro Thr Gly Ser Met Ser Gly Thr
                17645               17650               17655

gcg ttt atc gca tgc ggt tcc ctt ccg gct ttt acc cgg agc aac 96709
Ala Phe Ile Ala Cys Gly Ser Leu Pro Ala Phe Thr Arg Ser Asn
                17660               17665               17670

gtt att gtt att gat gaa gca gga ttg cta ggg cgt cat att ctc 96754
Val Ile Val Ile Asp Glu Ala Gly Leu Leu Gly Arg His Ile Leu
                17675               17680               17685

acg gcc gtt gtt tac tgt tgg tgg ctt ttg aat gct ata tat caa 96799
Thr Ala Val Val Tyr Cys Trp Trp Leu Leu Asn Ala Ile Tyr Gln
                17690               17695               17700

agc cct cag tac ata aac ggt cga aaa ccg gtc ata gta tgc gtc 96844
Ser Pro Gln Tyr Ile Asn Gly Arg Lys Pro Val Ile Val Cys Val
                17705               17710               17715

ggt tcg ccc acc caa act gac tcg tta gaa tct cat ttt caa cat 96889
Gly Ser Pro Thr Gln Thr Asp Ser Leu Glu Ser His Phe Gln His
                17720               17725               17730

gac atg cag cgt tca cac gta act cct agt gaa aat ata ctc acg 96934
Asp Met Gln Arg Ser His Val Thr Pro Ser Glu Asn Ile Leu Thr
                17735               17740               17745

tat ata atc tgc aat caa act ctg cgt caa tat act aac atc tca 96979
Tyr Ile Ile Cys Asn Gln Thr Leu Arg Gln Tyr Thr Asn Ile Ser
                17750               17755               17760

cat aac tgg gca atc ttt att aat aac aaa cga tgt caa gag gac 97024
His Asn Trp Ala Ile Phe Ile Asn Asn Lys Arg Cys Gln Glu Asp
                17765               17770               17775

gat ttt gga aat ctt tta aaa acg ctt gag tac ggg cta cct att 97069
Asp Phe Gly Asn Leu Leu Lys Thr Leu Glu Tyr Gly Leu Pro Ile
                17780               17785               17790

acc gaa gca cat gcg cgt ctg gtc gat aca ttt gtt gta cct gca 97114
Thr Glu Ala His Ala Arg Leu Val Asp Thr Phe Val Val Pro Ala
                17795               17800               17805

tcc tat att aac aat cct gct aat ctt ccc gga tgg acg cgt ctg 97159
Ser Tyr Ile Asn Asn Pro Ala Asn Leu Pro Gly Trp Thr Arg Leu
                17810               17815               17820

tat tcg tcg cat aag gag gtg agc gcg tat atg agt aag tta cac 97204
Tyr Ser Ser His Lys Glu Val Ser Ala Tyr Met Ser Lys Leu His
                17825               17830               17835

gcg cat tta aaa cta tcg aaa aat gac cat ttt tct gtg ttt gcc 97249
Ala His Leu Lys Leu Ser Lys Asn Asp His Phe Ser Val Phe Ala
                17840               17845               17850

tta ccg act tat aca ttc atc cgg cta acg gca ttt gat gaa tac 97294
Leu Pro Thr Tyr Thr Phe Ile Arg Leu Thr Ala Phe Asp Glu Tyr
                17855               17860               17865

cgc aaa tta acg gga caa ccc gga ctt tct gtt gaa cat tgg ata 97339
Arg Lys Leu Thr Gly Gln Pro Gly Leu Ser Val Glu His Trp Ile
                17870               17875               17880

cgg gca aac tcc ggt cgt ttg cac aat tat tcc caa agc cga gat 97384
Arg Ala Asn Ser Gly Arg Leu His Asn Tyr Ser Gln Ser Arg Asp
                17885               17890               17895

cat gac atg gga aca gtt aaa tac gaa aca cat tca aat cgc gac 97429
His Asp Met Gly Thr Val Lys Tyr Glu Thr His Ser Asn Arg Asp
                17900               17905               17910

tta att gta gcc cgt aca gac atc act tac gtg cta aat agt ctc 97474
Leu Ile Val Ala Arg Thr Asp Ile Thr Tyr Val Leu Asn Ser Leu
                17915               17920               17925

gta gtt gta acc aca aga cta cgt aag tta gtt att gga ttc agt 97519
Val Val Val Thr Thr Arg Leu Arg Lys Leu Val Ile Gly Phe Ser
                17930               17935               17940

ggt aca ttt caa tcg ttt gca aag gtt tta cgt gac gac tcc ttt 97564
Gly Thr Phe Gln Ser Phe Ala Lys Val Leu Arg Asp Asp Ser Phe
                17945               17950               17955

gtg aag gct cga gga gag aca tcc atc gaa tat gct tac cgg ttt 97609
Val Lys Ala Arg Gly Glu Thr Ser Ile Glu Tyr Ala Tyr Arg Phe
                17960               17965               17970

ctg tca aac cta atc ttt gga ggc ttg att aac ttt tac aat ttt 97654
Leu Ser Asn Leu Ile Phe Gly Gly Leu Ile Asn Phe Tyr Asn Phe
                17975               17980               17985

ttg tta aat aaa aac cta cat ccc gat aag gta tcg tta gca tac 97699
Leu Leu Asn Lys Asn Leu His Pro Asp Lys Val Ser Leu Ala Tyr
                17990               17995               18000

aaa cgg tta gct gcc tta acc ctg gag tta ttg tct gga aca aac 97744
Lys Arg Leu Ala Ala Leu Thr Leu Glu Leu Leu Ser Gly Thr Asn
                18005               18010               18015

aaa gcc ccc tta cac gaa gca gcg gtt aat ggg gcg ggt gcc ggg 97789
Lys Ala Pro Leu His Glu Ala Ala Val Asn Gly Ala Gly Ala Gly
                18020               18025               18030

att gac tgt gat ggt gca gct act tct gcc gat aaa gcc ttc tgc 97834
Ile Asp Cys Asp Gly Ala Ala Thr Ser Ala Asp Lys Ala Phe Cys
                18035               18040               18045

ttt acc aaa gcc ccc gag tcc aaa gta acg gcc tcc ata ccc gaa 97879
Phe Thr Lys Ala Pro Glu Ser Lys Val Thr Ala Ser Ile Pro Glu
                18050               18055               18060

gac ccg gat gat gta att ttt acg gca ctt aac gac gag gtt att 97924
Asp Pro Asp Asp Val Ile Phe Thr Ala Leu Asn Asp Glu Val Ile
                18065               18070               18075

gac ttg gta tac tgc cag tac gaa ttt tcc tat ccc aaa tca tcc 97969
Asp Leu Val Tyr Cys Gln Tyr Glu Phe Ser Tyr Pro Lys Ser Ser
                18080               18085               18090

aat gag gtc cat gct cag ttt ctg tta atg aaa gct att tac gat 98014
Asn Glu Val His Ala Gln Phe Leu Leu Met Lys Ala Ile Tyr Asp
                18095               18100               18105

ggt cga tat gcc ata tta gca gag ctt ttc gaa agc agc ttt aca 98059
Gly Arg Tyr Ala Ile Leu Ala Glu Leu Phe Glu Ser Ser Phe Thr
                18110               18115               18120

acc gcc ccc ttt agc gcg tat gtc gat aat gtt aat ttc aac gga 98104
Thr Ala Pro Phe Ser Ala Tyr Val Asp Asn Val Asn Phe Asn Gly
                18125               18130               18135

agc gag ctt ttg atc ggc aat gtg cgg ggg ggg ctg tta tct ttg 98149
Ser Glu Leu Leu Ile Gly Asn Val Arg Gly Gly Leu Leu Ser Leu
                18140               18145               18150

gca tta caa aca gat acg tat acc ctt ttg ggg tat act ttt gca 98194
Ala Leu Gln Thr Asp Thr Tyr Thr Leu Leu Gly Tyr Thr Phe Ala
                18155               18160               18165

ccc gtg cca gtc ttt gta gag gaa ctg acc cga aaa aag ctg tac 98239
Pro Val Pro Val Phe Val Glu Glu Leu Thr Arg Lys Lys Leu Tyr
                18170               18175               18180

cgc gaa act acc gaa atg tta tat gct cta cac gta cct ctt atg 98284
Arg Glu Thr Thr Glu Met Leu Tyr Ala Leu His Val Pro Leu Met
                18185               18190               18195

gtc tta cag gat caa cat ggg ttt gtg tcc atc gta aac gct aac 98329
Val Leu Gln Asp Gln His Gly Phe Val Ser Ile Val Asn Ala Asn
                18200               18205               18210

gta tgt gaa ttt acc gag tct ata gag gat gca gaa ttg gca atg 98374
Val Cys Glu Phe Thr Glu Ser Ile Glu Asp Ala Glu Leu Ala Met
                18215               18220               18225

gcc acc acg gtg gac tat ggc ctt agt tct aaa cta gcc atg aca 98419
Ala Thr Thr Val Asp Tyr Gly Leu Ser Ser Lys Leu Ala Met Thr
                18230               18235               18240

att gca cgc tca cag ggt ctg agt tta gag aag gta gct atc tgt 98464
Ile Ala Arg Ser Gln Gly Leu Ser Leu Glu Lys Val Ala Ile Cys
                18245               18250               18255

ttt acg gcg gat aaa ctg cgc cta aat agt gtg tat gtt gcc atg 98509
Phe Thr Ala Asp Lys Leu Arg Leu Asn Ser Val Tyr Val Ala Met
                18260               18265               18270

tcg cgt acg gtc tcc tct agg ttc tta aaa atg aat cta aac cct 98554
Ser Arg Thr Val Ser Ser Arg Phe Leu Lys Met Asn Leu Asn Pro
                18275               18280               18285

cta cgg gaa cga tat gaa aaa tcc gca gaa att agc gat cac att 98599
Leu Arg Glu Arg Tyr Glu Lys Ser Ala Glu Ile Ser Asp His Ile
                18290               18295               18300

ctt gcc gct cta cgt gat ccc aac gta cac gtt gtg tat taa 98641
Leu Ala Ala Leu Arg Asp Pro Asn Val His Val Val Tyr
                18305               18310

agcattgtat aaaaacacgc atgcgggctt gctgttctca tttctaggtt ttgtcttaaa 98701

tacacccgcc atgagcatct ctggaccccc aacgacgttt attttatata ggttacatgg 98761

ggttaggcgg gttcttcact ggactttacc ggatcatgaa caaacactct acgcatttac 98821

gggtgggtca agatcaatgg cggtgaagac ggacgctcga tgtgatacaa tgagcggtgg 98881

tatgatcgtc cttcaacaca cccatacagt gaccctgcta accatagact gttctactga 98941
cttttcatca tacgcattta cgcaccggga tttccactta caggacaaac cccacgcaac 99001
atttgcgatg ccgtttatgt cctgggtcgg ttctgaccca acatctcagc tgtacagtaa 99061
tgtggggggg gtactatccg taataacgga agatgaccta tccatgtgta tctcaattgt 99121
tatatacggt ttacgggtaa acagacctga cgatcagacc acaccaacac caaccccgca 99181
ccagtataca tcgcaaaggc ggcagcctga aaccaactgt ccttcttcac cacaaccggc 99241
ctttttcaca tcagacgacg acgttctttc gttaatatta cgggacgccg caaacgcgta 99301
aagacagatt caagactaac atttatccca actgattaca tttcatacgc gaataaacga 99361
cacaaaaaat ttatatttaa cggcttttaa tttgaagaca cctatcctct taacgttgat 99421
gagccttgca ggttgggtgc cgcgcttcac cggtattata cataaccgat ttaccgtgtt 99481
tacggcagtc tgaccattta ccagtgtatg tctgtaatac gacgttgttg tgtcccgaca 99541
aaattaactc gcgtacaaat ttctgatgtt cccccggcgt ggcaacgctg gcatttccaa 99601
acacattacg ttctcgtacg tccatgaccg ctattttcag tattaattgg ttggtcggtc 99661
aaagtatttt ccttatgtaa aaggacacga tctaaagccg taaactcata cacaaacact 99721
ggtaccaacg gacgcgattt tccgtccgtt gagcgggtgt aatatcggcg aggtcttctt 99781
gcacgaatac tctcgtacag taggtttctg acacggggtg catgggtttt ttgacacaac 99841
acaaacattt gcaggctctt atgactggat ggattgaatt tatttttaga tagggtcacg 99901
tgtttttgtc gtgacacgcc tcgaccagaa aaggctgcgg ttttcgtaca cgcgaccgtt 99961
atttcacagg cgttcataac caagctgcgg cggatggtgt cggttaattg tctccgccca 100021
agttcgtcaa tagatgatac catgaacaac gtatcaaatg gtacatagtc gtctttggtt 100081
ttctcaatac agcccgcgtg cccaatcgga aatttttcat ttgcatcaac gctattttct 100141
gtaaaatcgt tctgaacact gtgttggctg gctacctgtt taaaatttgg gatcgaacac 100201
ggtccacgat gcaatcccca accccattga agcaatgccg tcggtacgga aggaggcaac 100261
tccgaaaaca ttatggtacg caagagggtc gattggagtg ttatataaca ctccaatcga 100321
tctcgggttc gcctttacgc gtaaaatact cattggcttg aacgaaatgt cgacaattcc 100381
gaaatggaac acgggacaat ggcgacggat gcgcgtgtgt tagcaccaga tgacatcttg 100441
aattcggttg ggttgtcttc tgtgcatgcg caccccacag cataaaaact aaccctgtac 100501
ggttctcgca taacctctgt agcacgcgtt gcaccagccg cccccagcct aagtatacat 100561
gcgaccccgg agtcccgcga cgaaccgtaa gcgtggtatt cagcaataac accccctgcc 100621
ttgcccaact ctccaggcat ccgtgagtgg gcggagtcat atttgggtat gattccatga 100681
gggccgcaaa aatattttta agactagacg gtggtgttat gccacgtttt acactaaacg 100741
ctagcccatg tgcatgtccc gcggtagggt atggatcttg accaataatt acaacgcgaa 100801
tgctctgggg tccgcaaaat cgcgtccatg caaaaatatc gcctgtagat ggaagtattt 100861
cttcccctga atttaaaaga cgattgtatt ctaaaaaaat acctttcgcg tacggctctt 100921

taagttcgtc cgacaacagg tcataccact caggggaaat gttaaacttg ctgaaaactt 100981
caaccgaatc cagttgcgaa gagacggggg tgaacgtttc cgtgtcgtaa tgatgtgaca 101041
tgttatttaa cttgaaggtt ggggggtcta gcttaacccc caaaggcagc ccgcggggtc 101101
gcttgcgggt ttttttggta accggatggg ccaaaacata aatgtccttt gaatccgata 101161
gtttcatttc attggcatac gcgttggaac aaacggtcgg ctccccagac acatccattt 101221
tccgggatat ttgtggaaga tggagtagag tctacccata caccggaaag ggcatccaac 101281
aaagcatcgc gtatgtcccc gcttttatgt tcttcaccaa cagattgtgc cagccccttt 101341
aaggtgacgt atggatttgt ccagtacgcc atttgtttgt ctttaaacca aagtataact 101401
tccggtactg gacattttgt cttaaccacg attcccgata gcgcctcgct gaggtttgat 101461
accgggggtg ccgcatagtc ccacgcctca tataccgatg acacgcacgg ttccgttata 101521
atcaaactca catccgatag cggtttggct ccaaaaaaca acggagtgtc gtcttggaga 101581
tgaagacaat acgcgattgt gatagttttt aaaaaaacta tctgcagtaa ccatttatgt 101641
gatgccatga cgcttgtgtt ttcccttcac tacgacgttg tcgtatcctt tgaaaaactt 101701
gaccactcta atggaagcat ggacaagtat gagttttata tatacagttg gcctttagtt 101761
aaactcttgg tgtcatatct cattttccta aaaagggcga tcttaatatg tcaaacgtca 101821
cggcgtgccg acaaagcgaa tttccatgca agatttggat gtagtattta tacacccaat 101881
cacatgtcac gtattaagct ttacagtccc ccgttatctg atataatcac ttttcttaac 101941
acgtcatcgg gaaaacagat gtttatatta tacctctcgc ggtcatttac ggcaaatact 102001
tagaccgttt tcaagcggac tgaaaacgct caaattgcct tttggaggcc tgcccaacgg 102061
ccattatccc ttggatctaa gattgatttg cggtaacgtt tgccaatcaa gctttaaaaa 102121
cgtaccccaa acttaaaacg ctcaaattgc cttttggagg cctgcccaac ggccattatc 102181
ccttggatct gagattgatt tacggtaacg tttgccaaac ccacgcattt cagtttaaat 102241
atttctaagc attcttagtg cgtacttggc agcgtgctta aaatatcaac caatatccat 102301
tatgctacac gtttccttct atccgtttca atccattaaa agtccattaa caaaaatgat 102361
gcatcatacc taattcacct aaaaacctga ctcattgcag cagcgtttcc tccttgcaga 102421
ctatccagtt ggcattttaa acgggtccgg ctgcctaaac cgaaaacacc gttgccttta 102481
ctgtaagtac aaaactaaaa tttatatttg cgtgcgtatt ttgtaacata tatgcctttt 102541
atccccccgc aagtttgctt taccctcgcc ttcaccaccc ccgccacctt ccggccattt 102601
taataacttt aattgctata agacataccc aaaccggatg atttttgccg ctggaaaaac 102661
agcttctaat tttcccgtct caactcggcc ttggttgcat ctccaagtat acctttagtt 102721
tgctcccgta gaggtgtata aatacaaacg gtgacaagta ttgagcgtaa tctcaaattt 102781
ttgtaattta gggcggagcg cttacgacag cacatgcgta ctgttagact gttatgttta 102841
ttgtatttgc agagcaggat gccccggtta ctccgagacc ggattgcggg cattccgaat 102901
cgtgtacgga cttaccaggg ggcagtattt acaccttggg ttccagatat accaaccctt 102961

acgaccaata gcaacactca ggtattttta aaatgcacgt ttaatgatca taatttacat 103021
acagttggta ataaagcaga ctgtggatgt ttaaggcatt tccttccccc tcccaacaaa 103081
ctaggacttc ttcatcttgt ttggaatacc tttacccgct ttaccggcag agcttttttt 103141
ggtaaggtgt ttcagtgaac ctgatgttga tccggaggtg gagggggtat tggactcccc 103201
ctgtggagag gcaactttgc gggttttact tcccttacat gccgaatcag actcagatgt 103261
caggtctatt gttaagcatc gtttaacgtc tctgccggta tgaaataaac ggcgcttagc 103321
accccttgcg cttcccggtt taatccccgg taacacagaa aaaagcctga ctttttgggg 103381
tgtatttacc aatcgggtat ccctttcatc gccacgagag gtctccccgg ttgaggtggt 103441
ttctggtctt acaattggac ctgtaattag ttggatggct gtatctttcc aggtccaggt 103501
ttgcatggtt aggcgggttg gatcggtaca tcgatccaac aagaataaca tgtttgttac 103561
aaacggtcct gttgaatcat gcaaaagaca acgcagggat gtttttaatc ccgcctcatc 103621
acgcccgtaa atacctatat agtttaatat caacattttt gtaggctcta caatttcggg 103681
ttgatacagt tccgcaagtt gatcatcaag ccatccgagt aaaggttgca tgtaacacgg 103741
gaatctcgcg tttccctctg ttcctctatc cgtggctcga aaaggcagtc tgtccatggt 103801
tcgtgggtct tgattaattc ccacagatac tggacgatca cggtagtcct gccccccggt 103861
ccggggttgc tgtgcagatt caatcgagcc atacaccacc ggggtcgccg atcgaacagc 103921
aggttggtct ttaaaaaata ccttccgtaa aaatgatgcg gtagagcatg ttttggttac 103981
accagggctc gagtctcggg tcggtggttg tatagaatcc tgttgagagt cacttggtga 104041
ctctgctgtg ggctctctag ccgacgattg aaggggccca gggtttggtg attgaatggg 104101
ctcccgactc gatcttgatg ttggctgttg gatggactcc cgactcggtc ctgggcttgg 104161
tggcagaaga tctatgacat ctcccggtag gatgtcgatg gaatcttcaa atgacggctc 104221
agaaaaacca tcgtcgtcgg atgggtgcac ttcatattcc ttgtaacttg tatcacttac 104281
gatcttatgc aggatggatt gcactggaca ccggcagaga ggacactgga cgctggtgga 104341
ggtccatgcc cgaatacaaa caaagcagaa gtcgtgcaaa cacggcatgg tttttccgag 104401
atcggaaacg gtgctcatgc atatggtgca ggtattatcc gaagcgtcgg aggtgccgct 104461
accgcccgct aatatggtat ccatggtaac aactggctgt attctaatgt ccgggcatcc 104521
aaacacgtag cagaactgcc atgcgttcta aattgtgagt tgtggcgagt acatttttat 104581
aattggtacc aacgaagaca cacccctata tccctccacc catttctttt aagtcccacc 104641
cactaaaacg tgggtataaa atgtgtattg gggtaggcgg acagtcccaa caaacaggga 104701
agttgattgg tataaccttg ggccgggtat acagctaagt gacattttag attctgtctt 104761
tatttagata aagagcgata cgaagacatt tctccacccc cctgtaatac ccgtaaataa 104821
aggtaagtcc acaaacaaaa gcactgtata taggaagtcg ggtgtattgg gacagttact 104881
ccattagagg cgtacaaaca atactgggat agggtaatgc aagtcccccc cgatggtcgc 104941
cccgcaaacg cgcggggagg tggggtcgct tttttttttc tctctcgagg gggccgcgag 105001

agggctggcc tcctctcccg gggtccgccg ggcgcccaga aaccgggggg gggttatttt 105061
cggggggggg tccgaccagc ccgcccgtcg cccgcccgca cagacagaca gacacttttt 105121
tcataaaaac cgttccgctt ttattaacaa caaacagtcc gcgcgccagt ggcgctcacg 105181
agaaaaggag gggactccgt cacccccgac tctgcggggg gctcctcccc ccgcgccctc 105241
cccacacatc gtcctcgtcc tcggaggacg aggacgagga caacagctcc accttgaccg 105301
ccgggcgcaa acccacccgg cggtctcgca gcacacccgg ggccaccgac acgatgctca 105361
ccccaaagga tgaccccggt gcgtccccgt cgtccccgcc cccctcctcg ctgtcccacg 105421
cgtcttcaca ccccacctcc caatcgtcca gctccaaagc gtgttctctg tcgtctgcgg 105481
tgcgccgctg tcgccccgcc tgggtttctg acggccgttc cgagcccccg tggtgtccga 105541
acacgaaccg tgttccgtcg ctcccctcca acaccgtctc cgcggcccca aaaccgggcg 105601
gccacattac tctgggaatc gggggggagg cattccgagc ctcgtccgcc gacgcataca 105661
gcgccaccga ccgaccggcc acgggtggaa gcacgagtgg ttctgcggca gggtcgggtt 105721
ccagcagggc gtggcggcaa aacaccctcg cccaggtggg tacgtcgccg gcctccggcc 105781
cggcggcccc cggtctccgt ccctcgggaa ggaagacggg tcgaagcgcg gcacccaggc 105841
cccatcggtt tgctgcgcgg tggctatgtg ccgcctcgtc cacaaagtcg gctgccccga 105901
gccccagacc ccgagactgt cgcgcgaggt ccttgcaacc gtcaaaaccc ggcagcacgt 105961
actgccggta ttcacggggc gacaggggga cgcgggtctt ggggcccgcg cgggtacaca 106021
cggtgtatgc gacgttccca ccgcggcaca aacacagggg ttgttcgccc gggtacaggt 106081
tggcaaacgc agtctcgata cgagcaaaac tcgctggccc aaaggtgcgc gacgatgcaa 106141
acacggcccg ggcgagtcct tctgtgaccg ccgagtctgg ccatcggacg acggcctggg 106201
cgtccggtcg cgccggggcc cggacgtaca cgtgatactg agacaaagcg ggtccatccc 106261
tgggccacct ctcgagggcc accgcgtcca acaccagcaa ccggcgccgg gcagaggcca 106321
accgcgagcc tagatactcg acggccccgg caaaggccag gtctcgggtc gacagtaata 106381
aaacgccccg ggcgttcaaa gcggacacgt ccggcgggcc ggtccagttc ccggcccagg 106441
catgagtgct cggcaggcac aaccggttac tcagggctgc caggaccaca gacagtcccc 106501
ctcgggatgg actccatgac ggtcccggat ctgtcgcgag ggtgctctcg agggggccgt 106561
tgatgtcctc tccgggcaac ggatcgtaga tgatcagaag cctcacatcc tccgggtctg 106621
ggatctgccg catccaggcg cacctccgtc gcagcgcctc cactccgctg ggtggaccaa 106681
accgtcggtc tcctccgccc ggacgccgag cggcgatttc cgccaaggcg ccgggatcaa 106741
agcttagcgc agggcgccag gccgtgggaa acaatgggtc gtcgaccaga cgggcgatgg 106801
tttcgggggt acagtacgcc ttgcgagcct ggtccgacgg gaccggggta tgcagggccc 106861
cccggggaat acgccgaaat cccccgtttg gggccggtcc gtcaagtggc atcgttatta 106921
cggcgggggg atccaccaca gggcccgagg tgatggtcac gggctcggat acccgcctct 106981
tggccttgga aaccacatga tcgtctgcaa cccgggcgtc cgcgacgggt gtctccctaa 107041

tcttgtcgag gaggcttctg ctctcgactg gctgggactt gcgcttgcgc ggagttcgta 107101
aacgatcatc cggtggacac acagaaagag agcgtgcggc ggccgacggc tgagggtcgg 107161
gagcctgtgt ggccggggtt gttggagaag ggtgaccgcg ggagatccgc gccgccggac 107221
tggagcccgt tgcctcgggg tatgccatgc tggcaaaggc tctgcggaga ctctgtagga 107281
taaagtgttt ttgggcccgg tcgtatcgac ggctcatagc cacggccgcg gccgcgtggg 107341
ggagagccca gagggcctcc cccgtggcca tggcttcgcc tacatgcgga acgggagacg 107401
ctacgctccc cgtaacggcg gtacccgccc gtcccggtgg caacagcttt tggtagaact 107461
ggttcagggc cgagttgaca ccggtcagct tggggttctg gagccatgct atagggtctc 107521
tgtctggaca gtagatcagg ttaatcagcg cgcggtactg tctagccgga tctcccaact 107581
ccggcacgta aagcggcacg ggttccgttg aggcctcgta acgagcccgc gccgctctca 107641
cagcctcatc ctcccagtga ccctctctgg tctccccgga cggtccaaac cgcaccctgt 107701
tggatgggag gggtgccgat ccgggccaag ggcttccgtc gggcatcatg agcggccccg 107761
acaccggggg aattatcggg gttctggatc gcggcaggga aaatgatttc tgtctctggc 107821
gccccggttc ccccgcaaga cgtttggtct tacgaatcct cggatcggga ccgctgatgg 107881
atcgatatcc cggttggata ttttgtttcg tcgacccacc atcatttgag tccgaatcat 107941
ccgaatttga cggggaaggg gcgtgttcgc gtccggacct gctgcctgta gtttcacttc 108001
ccaccgaaac gcgccggggt tcatcgtctt catcctccga tgacgatccc cacgacgagg 108061
aagaggatga agacgaaaca aactcacgac tctttggctt tttctccact gggctgtcat 108121
cctcaatcgg gtctggtgcg tgggatcttc ccggcagggc caaaaacgct ctaggtttgc 108181
cccccgacga acgtccaggg acgcgaggtg ttataccccg ggcatcatgt ttccttgggc 108241
gggtatcatc ggtctcaaac ggcaggtccg cctttgcccc cttagcggga acgctgtccg 108301
aaaggacgtg gtacaattgc tcaaccgggc cgggtacagg tccaccgggt ttccgcgccg 108361
ggagtgggac cttaaccttc aaagtctttt tcttcgggct ctttccctga gcgggccgtt 108421
gagttttctg gagaactact ccgtcccccg atgcatgcgc atgacccgct tgctcatcgc 108481
ccggcttttt acccgagatg gactgagttt gtctgtctcg atggaccacc gacggcaaac 108541
ctggtgaatt tcctctcgtc gtttgtcggg gtatagaccg ctggtcttcc cgttgatcgt 108601
tcccggcggc gtctccaaca ggagacgcgg gggatacagg ggagaaggcc tgcgggaacg 108661
gaggggtcgt acctctgccc gtttccccat cgttcatcgg tggttttgga gacctagcaa 108721
gcttcgttcc gagagagact gtctcaaggg agcgatcggc tcctgttggt tctcgcgcgc 108781
cggcctccga gaatcgggtg tggaagacct cggccagcgg gattacaggc gagcccatta 108841
gatcctgacc gtcctcgcat acgtagtcgt cttgtgttag ctcttcgcca acatcttccg 108901
ttctgggttc tggttgaagt cccgatacgg agggaattga aacgatctcg tgttcccgtc 108961
ccaccatgac cccgttctct ccaaatagta gatcgtcagg ctgactcgag gtgaccaccc 109021
gggccctgtg ttcggcggcc gccgcggccg cgtccaacag gtccattaac tccaaagtat 109081

caggcgaccc cgcgcgttgg ggtgtagagc gctgcatcgg cggcgtatcc atcgcactgg 109141

ggtgaattta gacgtacccg agttttccaa acgctctcgc agccttcaaa ggattgcgat 109201

tgcggttggt gagggagttc caacagtact taaaacgtgt tgtgcccccc cctcgaccgc 109261

atatttcctc cccgtgtcgt caccgtgtaa atattcttaa tgataagacg atgtagtgat 109321

tggacgagac tcgaggcggg aagttcatgg accatagtat gcgtttaagg agagaccgct 109381

ggttggcgat gtacgcccgg tgtctatttc cgcatacctt acaacatcat aacaagggat 109441

accagacatg tgaatttcat ttacatatgt ttaaataaca accaatcatc gtgtgtctac 109501

agacgatata taatatacat aaacacaatt ggggttgtct cacatgcaaa acatcttata 109561

taacacgggt tgtttccacc catccggcat ctagttaatc aaatgcacgt cgacggtgtg 109621

tttgggtccc tctccgtcgt cattacgttc gcgcaatcaa caagcgtata caccaccacc 109681

cctcccaacg attatgtcag gcggcacgaa gcccgcgata acccataaaa tacacacggg 109741

gttgtggtgt tcacgtaacc ccccgccgat ggggaggggg cgcggtaccc cgccgatggg 109801

gagggggcgc ggtaccccgc cgatggggag ggggcgcggt accccgccga tggggagggg 109861

gcgcggtacc ccgccgatgg ggagggggcg cggtaccccg ccgatgttta taaccataat 109921

tctctaaacc gttgtagaaa atcacaaaaa aatttattca aaaacaagtc gaagaacttc 109981

atatctgagg catgtaaacc cgttcgcact tcctggggtg gaatggggtg gggtgggggg 110041

gtgaaaaagg ggggggttta aattgggcgt ccgcatgtct gtggtgtacg ccaatcggat 110101

acactctttt gatctgcatt cgcacttccc gttttttcac tgtatgggtt ttcatgtttt 110161

ggcatgtgtc caaccaccgt tcgcactttc tttctatata tatatatata tatatatata 110221

tatatagaga aagagagaga gtttcttgtt cgcgcgtgtt cccgcgatgt cgcggtttta 110281

tggggtgtgg gcgggctttt cacagaatat atatattcca aatggagcgg caggcttttt 110341

aaaatcgatt tgacgtgata aaaaaaaaca cacggggccc cccccttttt ttggtgttat 110401

aaaggcaacc caatcgaagg tctcccgccc cggaatcccc cattgccatt ttacccaagt 110461

agccttattc atagatgtaa acgtttgggt gtgtgttttg ttgtgcaggg ttcgtccgat 110521

tcataacgcg acagcgtcga gtcggtttta agggaaaagg ttactacggc cccaaggac 110580

atg ttt tgc acc tca ccg gct acg cgg ggc gac tcg tcc gag tca 110625
Met Phe Cys Thr Ser Pro Ala Thr Arg Gly Asp Ser Ser Glu Ser
    18315               18320               18325

aaa ccc ggg gca tcg gtt gat gtt aac gga aag atg gaa tat gga 110670
Lys Pro Gly Ala Ser Val Asp Val Asn Gly Lys Met Glu Tyr Gly
    18330               18335               18340

tct gca cca gga ccc ctg aac ggc cgg gat acg tcg cgg ggc ccc 110715
Ser Ala Pro Gly Pro Leu Asn Gly Arg Asp Thr Ser Arg Gly Pro
    18345               18350               18355

ggc gcg ttt tgt act ccg ggt tgg gag atc cac ccg gcc agg ctc 110760
Gly Ala Phe Cys Thr Pro Gly Trp Glu Ile His Pro Ala Arg Leu
    18360               18365               18370

gtt gag gac atc aac cgt gtt ttt tta tgt att gca cag tcg tcg 110805

Val Glu Asp Ile Asn Arg Val Phe Leu Cys Ile Ala Gln Ser Ser
    18375                   18380                   18385

gga cgc gtc acg cga gat tca cga aga ttg cgg cgc ata tgc ctc 110850
Gly Arg Val Thr Arg Asp Ser Arg Arg Leu Arg Arg Ile Cys Leu
    18390                   18395                   18400

gac ttt tat cta atg ggt cgc acc aga cag cgt ccc acg tta gcg 110895
Asp Phe Tyr Leu Met Gly Arg Thr Arg Gln Arg Pro Thr Leu Ala
    18405                   18410                   18415

tgc tgg gag gaa ttg tta cag ctt caa ccc acc cag acg cag tgc 110940
Cys Trp Glu Glu Leu Leu Gln Leu Gln Pro Thr Gln Thr Gln Cys
    18420                   18425                   18430

tta cgc gct act tta atg gaa gtg tcc cat cga ccc cct cgg ggg 110985
Leu Arg Ala Thr Leu Met Glu Val Ser His Arg Pro Pro Arg Gly
    18435                   18440                   18445

gaa gac ggg ttc att gag gcg ccg aat gtt cct ttg cat agg agc 111030
Glu Asp Gly Phe Ile Glu Ala Pro Asn Val Pro Leu His Arg Ser
    18450                   18455                   18460

gca ctg gaa tgt gac gta tct gat gat ggt ggt gaa gac gat agc 111075
Ala Leu Glu Cys Asp Val Ser Asp Asp Gly Gly Glu Asp Asp Ser
    18465                   18470                   18475

gac gat gat ggg tct acg cca tcg gat gta att gaa ttt cgg gat 111120
Asp Asp Asp Gly Ser Thr Pro Ser Asp Val Ile Glu Phe Arg Asp
    18480                   18485                   18490

tcc gac gcg gaa tca tcg gac ggg gaa gac ttt ata gtg gaa gaa 111165
Ser Asp Ala Glu Ser Ser Asp Gly Glu Asp Phe Ile Val Glu Glu
    18495                   18500                   18505

gaa tca gag gag agc acc gat tct tgt gaa cca gac ggg gta ccc 111210
Glu Ser Glu Glu Ser Thr Asp Ser Cys Glu Pro Asp Gly Val Pro
    18510                   18515                   18520

ggc gat tgt tat cga gac ggg gat ggg tgc aac acc ccg tcc cca 111255
Gly Asp Cys Tyr Arg Asp Gly Asp Gly Cys Asn Thr Pro Ser Pro
    18525                   18530                   18535

aag aga ccc cag cgt gcc atc gag cga tac gcg ggt gca gaa acc 111300
Lys Arg Pro Gln Arg Ala Ile Glu Arg Tyr Ala Gly Ala Glu Thr
    18540                   18545                   18550

gcg gaa tat aca gcc gcg aaa gcg ctc acc gcg ttg ggc gag ggg 111345
Ala Glu Tyr Thr Ala Ala Lys Ala Leu Thr Ala Leu Gly Glu Gly
    18555                   18560                   18565

ggt gta gat tgg aag cga cgt cga cac gaa gcc ccg cgc cgg cat 111390
Gly Val Asp Trp Lys Arg Arg Arg His Glu Ala Pro Arg Arg His
    18570                   18575                   18580

gat ata ccg ccc ccc cat ggc gtg tag tctttataaa taaatacaat 111437
Asp Ile Pro Pro Pro His Gly Val
    18585                   18590

ggtttggctc gtgtcttttt ttgatgtctg tctgtggggg agtggggtgt tgtggatatt 111497

agagggtaga gggtgctggt ttgaacgtct ccattaaccc acggggtccc cacacgggcc 111557

gtgtggt atg aat ctc tgc gga tcc cgc ggt gag cac ccg ggc ggt 111603
        Met Asn Leu Cys Gly Ser Arg Gly Glu His Pro Gly Gly
                    18595                   18600

gaa tat gcc gga ctt tac tgc aca cga cac gat acc ccc gcg cac 111648

Glu Tyr Ala Gly Leu Tyr Cys Thr Arg His Asp Thr Pro Ala His
18605               18610               18615

cag gct ctc atg aac gac gcc gaa cgg tac ttc gcc gcc gcg cta 111693
Gln Ala Leu Met Asn Asp Ala Glu Arg Tyr Phe Ala Ala Ala Leu
18620               18625               18630

tgc gcc ata tct acc gag gcc tac gag gct ttt ata cac agc ccc 111738
Cys Ala Ile Ser Thr Glu Ala Tyr Glu Ala Phe Ile His Ser Pro
18635               18640               18645

tcc gag aga ccg tgc gcg agt ttg tgg ggg agg gca aag gac gcc 111783
Ser Glu Arg Pro Cys Ala Ser Leu Trp Gly Arg Ala Lys Asp Ala
18650               18655               18660

ttc gga cgg atg tgc ggg gag ctc gca gcg gat aga caa cgt cca 111828
Phe Gly Arg Met Cys Gly Glu Leu Ala Ala Asp Arg Gln Arg Pro
18665               18670               18675

ccc tcg gtt ccg ccg atc cgc aga gcg gtg tta tcg tta tta cgc 111873
Pro Ser Val Pro Pro Ile Arg Arg Ala Val Leu Ser Leu Leu Arg
18680               18685               18690

gag caa tgc atg ccg gat cca caa tcg cat ctg gag ctc agc gag 111918
Glu Gln Cys Met Pro Asp Pro Gln Ser His Leu Glu Leu Ser Glu
18695               18700               18705

cgg ctg ata ttg atg gca tat tgg tgc tgt ttg gga cac gcc gga 111963
Arg Leu Ile Leu Met Ala Tyr Trp Cys Cys Leu Gly His Ala Gly
18710               18715               18720

ctt ccg act att gga ttg tcg ccc gat aat aaa tgc atc cgc gcc 112008
Leu Pro Thr Ile Gly Leu Ser Pro Asp Asn Lys Cys Ile Arg Ala
18725               18730               18735

gaa tta tat gac cgc ccc ggg gga att tgt cac agg ctt ttt gac 112053
Glu Leu Tyr Asp Arg Pro Gly Gly Ile Cys His Arg Leu Phe Asp
18740               18745               18750

gcg tac ctg ggc tgc ggg tcc ctt gga gtc cca aga acc tac gag 112098
Ala Tyr Leu Gly Cys Gly Ser Leu Gly Val Pro Arg Thr Tyr Glu
18755               18760               18765

aga tcc tga caccccatcc ctttatatag aaaaaaaaaa taaatttaaa 112147
Arg Ser
18770

acatacaccg gataaaagcg tactgttttt tatttaaatt tacacgctcg gcgttgcccc 112207

ggttcggtga tcaccgggtc ttatctatat acaccgtgta actcgaaccc ccgtgactcc 112267

ctccaatcgc gttaccaaac tcttcttccg tatccgtaga ttccgagtcc tcgaaatcgt 112327

ccacttatcc aacaaattgt gacgttatat atcccaaggc aaaggccgct cccgtcatag 112387

caaatacaaa gacaattatt agcgtaatat aacagaattt tttacgatga tatattttat 112447

gttgatattt tccaattcga cgcaaaaatt catctgccgt ttcattttcg ctatcactat 112507

aataacactt ttcagccgaa cggctcggtt gtatggctgt tatcgttgta ttatttggtt 112567

gcgctcgcgg ggttaccacc gcttccatca gtaaggccac ggcctcaccc tccatggtgt 112627

tttgtccggc catagaaatc cagattgtaa ggccagcagg ctagtttaaa agtgtttaat 112687

accacacctt ttgatattta tatacatgca agattctaga ttattcatca ataggtcgtt 112747

taaagcgcgt tttcataaac gttgtcagct ataccgacat tctcacaaag aggtaaagtt 112807

accttacgtt attattaaat aaaacatgta gacattatta ataatcctag gaacaatcaa 112867

atccatattt gtaagttatg tttaacccct cccctttttg tcattatctc cgccctctta 112927

taatcggatc actttataag tgtgtcggtg agtatatttt gtacagttgt tggacaacag 112987

gtttttggtt cattaacact atcaacataa gtcggggtat acaagtata atg aac gac 113045
Met Asn Asp

gtt gat gca aca gac acc ttt gtt gga caa gga aag ttc cgt ggc 113090
Val Asp Ala Thr Asp Thr Phe Val Gly Gln Gly Lys Phe Arg Gly
18775               18780               18785

gcc atc tca aca tca ccg tca cat att atg caa aca tgt ggg ttt 113135
Ala Ile Ser Thr Ser Pro Ser His Ile Met Gln Thr Cys Gly Phe
18790               18795               18800

ata caa cag atg ttt cca gtt gaa atg tcg ccc ggc ata gaa tct 113180
Ile Gln Gln Met Phe Pro Val Glu Met Ser Pro Gly Ile Glu Ser
18805               18810               18815

gag gat gat ccc aat tat gac gtt aac atg gat ata cag tct ttt 113225
Glu Asp Asp Pro Asn Tyr Asp Val Asn Met Asp Ile Gln Ser Phe
18820               18825               18830

aat ata ttt gat ggt gta cac gaa act gaa gcc gaa gcc tct gtg 113270
Asn Ile Phe Asp Gly Val His Glu Thr Glu Ala Glu Ala Ser Val
18835               18840               18845

gca ttg tgc gca gaa gca cgc gtt gga att aat aaa gcg gga ttt 113315
Ala Leu Cys Ala Glu Ala Arg Val Gly Ile Asn Lys Ala Gly Phe
18850               18855               18860

gta ata tta aaa acg ttt aca cca ggg gcg gaa ggt ttt gcg ttt 113360
Val Ile Leu Lys Thr Phe Thr Pro Gly Ala Glu Gly Phe Ala Phe
18865               18870               18875

gcg tgt atg gac agt aaa aca tgt gaa cat gtg gtc att aaa gcg 113405
Ala Cys Met Asp Ser Lys Thr Cys Glu His Val Val Ile Lys Ala
18880               18885               18890

ggt caa cgt caa gga acg gcc acc gag gca acc gtg tta aga gcg 113450
Gly Gln Arg Gln Gly Thr Ala Thr Glu Ala Thr Val Leu Arg Ala
18895               18900               18905

tta acc cac cca tcc gtt gta cag ctt aaa gga acg ttt acg tat 113495
Leu Thr His Pro Ser Val Val Gln Leu Lys Gly Thr Phe Thr Tyr
18910               18915               18920

aac aaa atg aca tgt ctt ata tta cca cgt tac cga aca gat tta 113540
Asn Lys Met Thr Cys Leu Ile Leu Pro Arg Tyr Arg Thr Asp Leu
18925               18930               18935

tac tgc tat cta gct gca aag cgc aac ctc ccc ata tgt gac att 113585
Tyr Cys Tyr Leu Ala Ala Lys Arg Asn Leu Pro Ile Cys Asp Ile
18940               18945               18950

tta gca att cag cga tct gta tta cgc gcg tta cag tat ctt cat 113630
Leu Ala Ile Gln Arg Ser Val Leu Arg Ala Leu Gln Tyr Leu His
18955               18960               18965

aat aac agt att att cac cgt gat ata aaa tct gaa aat ata ttt 113675
Asn Asn Ser Ile Ile His Arg Asp Ile Lys Ser Glu Asn Ile Phe
18970               18975               18980

att aac cac cca ggt gat gtt tgt gtg gga gac ttt gga gca gcg 113720

Ile    Asn His Pro Gly Asp    Val Cys Val Gly Asp    Phe Gly Ala Ala
18985                  18990                  18995

tgt    ttc ccc gtg gat att    aat gcc aac agg tat    tat ggc tgg gct 113765
Cys    Phe Pro Val Asp Ile    Asn Ala Asn Arg Tyr    Tyr Gly Trp Ala
19000                  19005                  19010

gga    aca atc gcc aca aac    tct cct gag tta ttg    gct aga gat cca 113810
Gly    Thr Ile Ala Thr Asn    Ser Pro Glu Leu Leu    Ala Arg Asp Pro
19015                  19020                  19025

tat    gga cct gcc gtg gac    ata tgg agt gcc ggg    att gta tta ttt 113855
Tyr    Gly Pro Ala Val Asp    Ile Trp Ser Ala Gly    Ile Val Leu Phe
19030                  19035                  19040

gaa    atg gct aca gga cag    aac tcg tta ttt gaa    cga gac ggt tta 113900
Glu    Met Ala Thr Gly Gln    Asn Ser Leu Phe Glu    Arg Asp Gly Leu
19045                  19050                  19055

gat    ggc aat tgt gac agt    gag cgt caa att aaa    ctt att ata cga 113945
Asp    Gly Asn Cys Asp Ser    Glu Arg Gln Ile Lys    Leu Ile Ile Arg
19060                  19065                  19070

cga    tct gga act cat ccc    aat gaa ttt ccc att    aac cct aca tca 113990
Arg    Ser Gly Thr His Pro    Asn Glu Phe Pro Ile    Asn Pro Thr Ser
19075                  19080                  19085

aat    ctt cgt cga caa tac    att ggt ttg gca aaa    cgg tct tct cga 114035
Asn    Leu Arg Arg Gln Tyr    Ile Gly Leu Ala Lys    Arg Ser Ser Arg
19090                  19095                  19100

aaa    ccc gga tcc agg cca    ttg tgg aca aat cta    tat gag ttg cca 114080
Lys    Pro Gly Ser Arg Pro    Leu Trp Thr Asn Leu    Tyr Glu Leu Pro
19105                  19110                  19115

att    gat ttg gag tat ttg    ata tgt aag atg tta    tcg ttt gac gca 114125
Ile    Asp Leu Glu Tyr Leu    Ile Cys Lys Met Leu    Ser Phe Asp Ala
19120                  19125                  19130

cgt    cat cga cca tca gca    gag gtg ttg ctt aac    cac tct gtt ttc 114170
Arg    His Arg Pro Ser Ala    Glu Val Leu Leu Asn    His Ser Val Phe
19135                  19140                  19145

caa    act ctt ccc gat cca    tat cca aat cca atg    gaa gtt gga gat 114215
Gln    Thr Leu Pro Asp Pro    Tyr Pro Asn Pro Met    Glu Val Gly Asp
19150                  19155                  19160

taa aattcattaa gcctgttaat aaaatattgt ataaattgtg tttataacgt        114268

ataacccgtt aaggcaaata gggtacaaac gcgcaatgtt ttgaaatact aatataaata 114328

acataaccaa tagaaactta atacagagtc acgccccatt acaacaagga taaaacacgg 114388

gatcattttc ttaacattgt agtagcgctg aaaagcgtcc cctcccccgg ctcacagagc 114448

tgctcttcgg tgtagttggg tatactggtg cgcctcattt aatcgcg atg    ttt tta 114504
                                                    Met    Phe Leu
                                                    19165

atc caa tgt    ttg ata tcg gcc gtt    ata ttt tac ata caa    gtg acc 114549
Ile Gln Cys    Leu Ile Ser Ala Val    Ile Phe Tyr Ile Gln    Val Thr
        19170                  19175                  19180

aac gct ttg    atc ttc aag ggc gac    cac gtg agc ttg caa    gtt aac 114594
Asn Ala Leu    Ile Phe Lys Gly Asp    His Val Ser Leu Gln    Val Asn
        19185                  19190                  19195

agc agt ctc    acg tct atc ctt att    ccc atg caa aat gat    aat tat 114639

Ser Ser Leu Thr Ser Ile Leu Ile Pro Met Gln Asn Asp Asn Tyr
        19200               19205               19210

aca gag ata aaa gga cag ctt gtc ttt att gga gag caa cta cct 114684
Thr Glu Ile Lys Gly Gln Leu Val Phe Ile Gly Glu Gln Leu Pro
        19215               19220               19225

acc ggg aca aac tat agc gga aca ctg gaa ctg tta tac gcg gat 114729
Thr Gly Thr Asn Tyr Ser Gly Thr Leu Glu Leu Leu Tyr Ala Asp
        19230               19235               19240

acg gtg gcg ttt tgt ttc cgg tca gta caa gta ata aga tac gac 114774
Thr Val Ala Phe Cys Phe Arg Ser Val Gln Val Ile Arg Tyr Asp
        19245               19250               19255

gga tgt ccc cgg att aga acg agc gct ttt att tcg tgt agg tac 114819
Gly Cys Pro Arg Ile Arg Thr Ser Ala Phe Ile Ser Cys Arg Tyr
        19260               19265               19270

aaa cat tcg tgg cat tat ggt aac tca acg gat cgg ata tca aca 114864
Lys His Ser Trp His Tyr Gly Asn Ser Thr Asp Arg Ile Ser Thr
        19275               19280               19285

gag ccg gat gct ggt gta atg ttg aaa att acc aaa ccg gga ata 114909
Glu Pro Asp Ala Gly Val Met Leu Lys Ile Thr Lys Pro Gly Ile
        19290               19295               19300

aat gat gct ggt gtg tat gta ctt ctt gtt cgg tta gac cat agc 114954
Asn Asp Ala Gly Val Tyr Val Leu Leu Val Arg Leu Asp His Ser
        19305               19310               19315

aga tcc acc gat ggt ttc att ctt ggt gta aat gta tat aca gcg 114999
Arg Ser Thr Asp Gly Phe Ile Leu Gly Val Asn Val Tyr Thr Ala
        19320               19325               19330

ggc tcg cat cac aac att cac ggg gtt atc tac act tct ccg tct 115044
Gly Ser His His Asn Ile His Gly Val Ile Tyr Thr Ser Pro Ser
        19335               19340               19345

cta cag aat gga tat tct aca aga gcc ctt ttt caa caa gct cgt 115089
Leu Gln Asn Gly Tyr Ser Thr Arg Ala Leu Phe Gln Gln Ala Arg
        19350               19355               19360

ttg tgt gat tta ccc gcg aca ccc aaa ggg tcc ggt acc tcc ctg 115134
Leu Cys Asp Leu Pro Ala Thr Pro Lys Gly Ser Gly Thr Ser Leu
        19365               19370               19375

ttt caa cat atg ctt gat ctt cgt gcc ggt aaa tcg tta gag gat 115179
Phe Gln His Met Leu Asp Leu Arg Ala Gly Lys Ser Leu Glu Asp
        19380               19385               19390

aac cct tgg tta cat gag gac gtt gtt acg aca gaa act aag tcc 115224
Asn Pro Trp Leu His Glu Asp Val Val Thr Thr Glu Thr Lys Ser
        19395               19400               19405

gtt gtt aag gag ggg ata gaa aat cac gta tat cca acg gat atg 115269
Val Val Lys Glu Gly Ile Glu Asn His Val Tyr Pro Thr Asp Met
        19410               19415               19420

tcc acg tta ccc gaa aag tcc ctt aat gat cct cca gaa aat cta 115314
Ser Thr Leu Pro Glu Lys Ser Leu Asn Asp Pro Pro Glu Asn Leu
        19425               19430               19435

ctt ata att att cct ata gta gcg tct gtc atg atc ctc acc gcc 115359
Leu Ile Ile Ile Pro Ile Val Ala Ser Val Met Ile Leu Thr Ala
        19440               19445               19450

atg gtt att gtt att gta ata agc gtt aag cga cgt aga att aaa 115404

Met Val Ile Val Ile Val Ile Ser Val Lys Arg Arg Arg Ile Lys
        19455               19460               19465

aaa cat cca att tat cgc cca aat aca aaa aca aga agg ggc ata 115449
Lys His Pro Ile Tyr Arg Pro Asn Thr Lys Thr Arg Arg Gly Ile
        19470               19475               19480

caa aat gcg aca cca gaa tcc gat gtg atg ttg gag gcc gcc att 115494
Gln Asn Ala Thr Pro Glu Ser Asp Val Met Leu Glu Ala Ala Ile
        19485               19490               19495

gca caa cta gca acg att cgc gaa gaa tcc ccc cca cat tcc gtt 115539
Ala Gln Leu Ala Thr Ile Arg Glu Glu Ser Pro Pro His Ser Val
        19500               19505               19510

gta aac ccg ttt gtt aaa tag aactaattat cccggatttt atattaaata 115590
Val Asn Pro Phe Val Lys
        19515

aactatatgc gttttattta gcgttttgat tacgcgttgt gatatgaggg gaaggattaa 115650

gaatctccta actataagtt aacacgccca catttgggcg gggatgtttt atgaagcctt 115710

aaaggccgag ctggtataca cgagagcagt ccatggtttt agacctcggg cgaattgcgt 115770

ggttttaagt gactatattc cgagggtcgc ctgtaat atg ggg aca gtt aat 115822
Met Gly Thr Val Asn
    19520

aaa cct gtg gtg ggg gta ttg atg ggg ttc gga att atc acg gga 115867
Lys Pro Val Val Gly Val Leu Met Gly Phe Gly Ile Ile Thr Gly
    19525               19530               19535

acg ttg cgt ata acg aat ccg gtc aga gca tcc gtc ttg cga tac 115912
Thr Leu Arg Ile Thr Asn Pro Val Arg Ala Ser Val Leu Arg Tyr
    19540               19545               19550

gat gat ttt cac acc gat gaa gac aaa ctg gat aca aac tcc gta 115957
Asp Asp Phe His Thr Asp Glu Asp Lys Leu Asp Thr Asn Ser Val
    19555               19560               19565

tat gag cct tac tac cat tca gat cat gcg gag tct tca tgg gta 116002
Tyr Glu Pro Tyr Tyr His Ser Asp His Ala Glu Ser Ser Trp Val
    19570               19575               19580

aat cgg gga gag tct tcg cga aaa gcg tac gat cat aac tca cct 116047
Asn Arg Gly Glu Ser Ser Arg Lys Ala Tyr Asp His Asn Ser Pro
    19585               19590               19595

tat ata tgg cca cgt aat gat tat gat gga ttt tta gag aac gca 116092
Tyr Ile Trp Pro Arg Asn Asp Tyr Asp Gly Phe Leu Glu Asn Ala
    19600               19605               19610

cac gaa cac cat ggg gtg tat aat cag ggc cgt ggt atc gat agc 116137
His Glu His His Gly Val Tyr Asn Gln Gly Arg Gly Ile Asp Ser
    19615               19620               19625

ggg gaa cgg tta atg caa ccc aca caa atg tct gca cag gag gat 116182
Gly Glu Arg Leu Met Gln Pro Thr Gln Met Ser Ala Gln Glu Asp
    19630               19635               19640

ctt ggg gac gat acg ggc atc cac gtt atc cct acg tta aac ggc 116227
Leu Gly Asp Asp Thr Gly Ile His Val Ile Pro Thr Leu Asn Gly
    19645               19650               19655

gat gac aga cat aaa att gta aat gtg gac caa cgt caa tac ggt 116272
Asp Asp Arg His Lys Ile Val Asn Val Asp Gln Arg Gln Tyr Gly
    19660               19665               19670

gac gtg ttt aaa gga gat ctt aat cca aaa ccc caa ggc caa aga 116317
Asp Val Phe Lys Gly Asp Leu Asn Pro Lys Pro Gln Gly Gln Arg
    19675               19680               19685

ctc att gag gtg tca gtg gaa gaa aat cac ccg ttt act tta cgc 116362
Leu Ile Glu Val Ser Val Glu Glu Asn His Pro Phe Thr Leu Arg
    19690               19695               19700

gca ccg att cag cgg att tat gga gtc cgg tac acc gag act tgg 116407
Ala Pro Ile Gln Arg Ile Tyr Gly Val Arg Tyr Thr Glu Thr Trp
    19705               19710               19715

agc ttt ttg ccg tca tta acc tgt acg gga gac gca gcg ccc gcc 116452
Ser Phe Leu Pro Ser Leu Thr Cys Thr Gly Asp Ala Ala Pro Ala
    19720               19725               19730

atc cag cat ata tgt tta aaa cat aca aca tgc ttt caa gac gtg 116497
Ile Gln His Ile Cys Leu Lys His Thr Thr Cys Phe Gln Asp Val
    19735               19740               19745

gtg gtg gat gtg gat tgc gcg gaa aat act aaa gag gat cag ttg 116542
Val Val Asp Val Asp Cys Ala Glu Asn Thr Lys Glu Asp Gln Leu
    19750               19755               19760

gcc gaa atc agt tac cgt ttt caa ggt aag aag gaa gcg gac caa 116587
Ala Glu Ile Ser Tyr Arg Phe Gln Gly Lys Lys Glu Ala Asp Gln
    19765               19770               19775

ccg tgg att gtt gta aac acg agc aca ctg ttt gat gaa ctc gaa 116632
Pro Trp Ile Val Val Asn Thr Ser Thr Leu Phe Asp Glu Leu Glu
    19780               19785               19790

tta gac ccc ccc gag att gaa ccg ggt gtc ttg aaa gta ctt cgg 116677
Leu Asp Pro Pro Glu Ile Glu Pro Gly Val Leu Lys Val Leu Arg
    19795               19800               19805

aca gaa aaa caa tac ttg ggt gtg tac att tgg aac atg cgc ggc 116722
Thr Glu Lys Gln Tyr Leu Gly Val Tyr Ile Trp Asn Met Arg Gly
    19810               19815               19820

tcc gat ggt acg tct acc tac gcc acg ttt ttg gtc acc tgg aaa 116767
Ser Asp Gly Thr Ser Thr Tyr Ala Thr Phe Leu Val Thr Trp Lys
    19825               19830               19835

ggg gat gaa aaa aca aga aac cct acg ccc gca gta act cct caa 116812
Gly Asp Glu Lys Thr Arg Asn Pro Thr Pro Ala Val Thr Pro Gln
    19840               19845               19850

cca aga ggg gct gag ttt cat atg tgg aat tac cac tcg cat gta 116857
Pro Arg Gly Ala Glu Phe His Met Trp Asn Tyr His Ser His Val
    19855               19860               19865

ttt tca gtt ggt gat acg ttt agc ttg gca atg cat ctt cag tat 116902
Phe Ser Val Gly Asp Thr Phe Ser Leu Ala Met His Leu Gln Tyr
    19870               19875               19880

aag ata cat gaa gcg cca ttt gat ttg ctg tta gag tgg ttg tat 116947
Lys Ile His Glu Ala Pro Phe Asp Leu Leu Leu Glu Trp Leu Tyr
    19885               19890               19895

gtc ccc atc gat cct aca tgt caa cca atg cgg tta tat tct acg 116992
Val Pro Ile Asp Pro Thr Cys Gln Pro Met Arg Leu Tyr Ser Thr
    19900               19905               19910

tgt ttg tat cat ccc aac gca ccc caa tgc ctc tct cat atg aat 117037
Cys Leu Tyr His Pro Asn Ala Pro Gln Cys Leu Ser His Met Asn
    19915               19920               19925

tcc ggt tgt aca ttt acc tcg cca cat tta gcc cag cgt gtt gca 117082
Ser Gly Cys Thr Phe Thr Ser Pro His Leu Ala Gln Arg Val Ala
    19930               19935               19940

agc aca gtg tat caa aat tgt gaa cat gca gat aac tac acc gca 117127
Ser Thr Val Tyr Gln Asn Cys Glu His Ala Asp Asn Tyr Thr Ala
    19945               19950               19955

tat tgt ctg gga ata tct cat atg gag cct agc ttt ggt cta atc 117172
Tyr Cys Leu Gly Ile Ser His Met Glu Pro Ser Phe Gly Leu Ile
    19960               19965               19970

tta cac gac ggg ggc acc acg tta aag ttt gta gat aca ccc gag 117217
Leu His Asp Gly Gly Thr Thr Leu Lys Phe Val Asp Thr Pro Glu
    19975               19980               19985

agt ttg tcg gga tta tac gtt ttt gtg gtg tat ttt aac ggg cat 117262
Ser Leu Ser Gly Leu Tyr Val Phe Val Val Tyr Phe Asn Gly His
    19990               19995               20000

gtt gaa gcc gta gca tac act gtt gta tcc aca gta gat cat ttt 117307
Val Glu Ala Val Ala Tyr Thr Val Val Ser Thr Val Asp His Phe
    20005               20010               20015

gta aac gca att gaa gag cgt gga ttt ccg cca acg gcc ggt cag 117352
Val Asn Ala Ile Glu Glu Arg Gly Phe Pro Pro Thr Ala Gly Gln
    20020               20025               20030

cca ccg gcg act act aaa ccc aag gaa att acc ccc gta aac ccc 117397
Pro Pro Ala Thr Thr Lys Pro Lys Glu Ile Thr Pro Val Asn Pro
    20035               20040               20045

gga acg tca cca ctt cta cga tat gcc gca tgg acc gga ggg ctt 117442
Gly Thr Ser Pro Leu Leu Arg Tyr Ala Ala Trp Thr Gly Gly Leu
    20050               20055               20060

gca gca gta gta ctt tta tgt ctc gta ata ttt tta atc tgt acg 117487
Ala Ala Val Val Leu Leu Cys Leu Val Ile Phe Leu Ile Cys Thr
    20065               20070               20075

gct aaa cga atg agg gtt aaa gcc tat agg gta gac aag tcc ccg 117532
Ala Lys Arg Met Arg Val Lys Ala Tyr Arg Val Asp Lys Ser Pro
    20080               20085               20090

tat aac caa agc atg tat tac gct ggc ctt cca gtg gac gat ttc 117577
Tyr Asn Gln Ser Met Tyr Tyr Ala Gly Leu Pro Val Asp Asp Phe
    20095               20100               20105

gag gac tcg gaa tct acg gat acg gaa gaa gag ttt ggt aac gcg 117622
Glu Asp Ser Glu Ser Thr Asp Thr Glu Glu Glu Phe Gly Asn Ala
    20110               20115               20120

att gga ggg agt cac ggg ggt tcg agt tac acg gtg tat ata gat 117667
Ile Gly Gly Ser His Gly Gly Ser Ser Tyr Thr Val Tyr Ile Asp
    20125               20130               20135

aag acc cgg tga tcaccgaacc ggggcaacgc cgagcgtgta aatttaaata 117719
Lys Thr Arg
    20140

aaaaacagta cgcttttatc cggtgtatgt tttaaattta tttttttttt ctatataaag 117779

ggatggggtg tcaggatctc tcgtaggttc ttgggactcc aagggacccg cagcccaggt 117839

acgcgtcaaa aagcctgtga caaattcccc cggggcggtc atataattcg gcgcggatgc 117899

atttattatc gggcgacaat ccaatagtcg gaagtccggc gtgtcccaaa cagcaccaat 117959

atgccatcaa tatcagccgc tcgctgagct ccagatgcga ttgtggatcc ggcatgcatt 118019
gctcgcgtaa taacgataac accgctctgc ggatcggcgg aaccgagggt ggacgttgtc 118079
tatccgctgc gagctccccg cacatccgtc cgaaggcgtc ctttgccctc ccccacaaac 118139
tcgcgcacgg tctctcggag gggctgtgta taaaagcctc gtaggcctcg gtagatatgg 118199
cgcatagcgc ggcggcgaag taccgttcgg cgtcgttcat gagagcctgg tgcgcggggg 118259
tatcgtgtcg tgtgcagtaa agtccggcat attcaccgcc cgggtgctca ccgcgggatc 118319
cgcagagatt cataccacac ggcccgtgtg gggaccccgt gggttaatgg agacgttcaa 118379
accagcaccc tctaccctct aatatccaca acaccccact cccccacaga cagacatcaa 118439
aaaaagacac gagccaaacc attgtattta tttataaaga ctacacgcca tgggggggcg 118499
gtatatcatg ccggcgcggg gcttcgtgtc gacgtcgctt ccaatctaca cccccctcgc 118559
ccaacgcggt gagcgctttc gcggctgtat attccgcggt ttctgcaccc gcgtatcgct 118619
cgatggcacg ctggggtctc tttggggacg gggtgttgca cccatccccg tctcgataac 118679
aatcgccggg taccccgtct ggttcacaag aatcggtgct ctcctctgat tcttcttcca 118739
ctataaagtc ttccccgtcc gatgattccg cgtcggaatc ccgaaattca attacatccg 118799
atggcgtaga cccatcatcg tcgctatcgt cttcaccacc atcatcagat acgtcacatt 118859
ccagtgcgct cctatgcaaa ggaacattcg gcgcctcaat gaacccgtct tccccccgag 118919
ggggtcgatg ggacacttcc attaaagtag cgcgtaagca ctgcgtctgg gtgggttgaa 118979
gctgtaacaa ttcctcccag cacgctaacg tgggacgctg tctggtgcga cccattagat 119039
aaaagtcgag gcatatgcgc cgcaatcttc gtgaatctcg cgtgacgcgt cccgacgact 119099
gtgcaataca taaaaaaaca cggttgatgt cctcaacgag cctggccggg tggatctccc 119159
aacccggagt acaaaacgcg ccggggcccc gcgacgtatc ccggccgttc aggggtcctg 119219
gtgcagatcc atattccatc tttccgttaa catcaaccga tgccccgggt tttgactcgg 119279
acgagtcgcc ccgcgtagcc ggtgaggtgc aaaacatgtc cttggggccg tagtaacctt 119339
ttcccttaaa accgactcga cgctgtcgcg ttatgaatcg gacgaaccct gcacaacaaa 119399
acacacaccc aaacgtttac atctatgaat aaggctactt gggtaaaatg gcaatggggg 119459
attccggggc gggagacctt cgattgggtt gcctttataa caccaaaaaa aggggggggc 119519
cccgtgtgtt tttttttatc acgtcaaatc gattttaaaa agcctgccgc tccatttgga 119579
atatatatat tctgtgaaaa gcccgcccac accccataaa accgcgacat cgcgggaaca 119639
cgcgcgaaca agaaactctc tctctttctc tatatatata tatatatata tatatatata 119699
tagaaagaaa gtgcgaacgg tggttggaca catgccaaaa catgaaaacc catacagtga 119759
aaaaacggga agtgcgaatg cagatcaaaa gagtgtatcc gattggcgta caccacagac 119819
atgcggacgc ccaatttaac cccccccctt tttcaccccc ccaccccacc ccattccacc 119879
ccaggaagtg cgaacgggtt tacatgcctc agatatgaag ttcttcgact tgtttttgaa 119939
taaatttttt tgtgattttc tacaacggtt tagagaatta tggttataaa catcggcggg 119999

gtaccgcgcc ccctccccat cggcggggta ccgcgccccc tccccatcgg cggggtaccg 120059

cgccccctcc ccatcggcgg ggtaccgcgc cccctcccca tcggcggggt accgcgcccc 120119

ctccccatcg gcggggggtt acgtgaacac cacaaccccg tgtgtatttt atgggttatc 120179

gcgggcttcg tgccgcctga cataatcgtt gggaggggtg gtggtgtata cgcttgttga 120239

ttgcgcgaac gtaatgacga cggagaggga cccaaacaca ccgtcgacgt gcatttgatt 120299

aactagatgc cggatgggtg gaaacaaccc gtgttatata agatgttttg catgtgagac 120359

aaccccaatt gtgtttatgt atattatata tcgtctgtag acacacgatg attggttgtt 120419

atttaaacat atgtaaatga aattcacatg tctggtatcc cttgttatga tgttgtaagg 120479

tatgcggaaa tagacaccgg gcgtacatcg ccaaccagcg gtctctcctt aaacgcatac 120539

tatggtccat gaacttcccg cctcgagtct cgtccaatca ctacatcgtc ttatcattaa 120599

gaatatttac acggtgacga cacggggagg aaatatgcgg tcgagggggg ggcacaacac 120659

gttttaagta ctgttggaac tccctcacca accgcaatcg caatcctttg aaggctgcga 120719

gagcgtttgg aaaactcggg tacgtctaaa ttcaccccag tgcg atg gat acg 120772
Met Asp Thr

ccg ccg atg cag cgc tct aca ccc caa cgc gcg ggg tcg cct gat 120817
Pro Pro Met Gln Arg Ser Thr Pro Gln Arg Ala Gly Ser Pro Asp
20145               20150               20155

act ttg gag tta atg gac ctg ttg gac gcg gcc gcg gcg gcc gcc 120862
Thr Leu Glu Leu Met Asp Leu Leu Asp Ala Ala Ala Ala Ala Ala
20160               20165               20170

gaa cac agg gcc cgg gtg gtc acc tcg agt cag cct gac gat cta 120907
Glu His Arg Ala Arg Val Val Thr Ser Ser Gln Pro Asp Asp Leu
20175               20180               20185

cta ttt gga gag aac ggg gtc atg gtg gga cgg gaa cac gag atc 120952
Leu Phe Gly Glu Asn Gly Val Met Val Gly Arg Glu His Glu Ile
20190               20195               20200

gtt tca att ccc tcc gta tcg gga ctt caa cca gaa ccc aga acg 120997
Val Ser Ile Pro Ser Val Ser Gly Leu Gln Pro Glu Pro Arg Thr
20205               20210               20215

gaa gat gtt ggc gaa gag cta aca caa gac gac tac gta tgc gag 121042
Glu Asp Val Gly Glu Glu Leu Thr Gln Asp Asp Tyr Val Cys Glu
20220               20225               20230

gac ggt cag gat cta atg ggc tcg cct gta atc ccg ctg gcc gag 121087
Asp Gly Gln Asp Leu Met Gly Ser Pro Val Ile Pro Leu Ala Glu
20235               20240               20245

gtc ttc cac acc cga ttc tcg gag gcc ggc gcg cga gaa cca aca 121132
Val Phe His Thr Arg Phe Ser Glu Ala Gly Ala Arg Glu Pro Thr
20250               20255               20260

gga gcc gat cgc tcc ctt gag aca gtc tct ctc gga acg aag ctt 121177
Gly Ala Asp Arg Ser Leu Glu Thr Val Ser Leu Gly Thr Lys Leu
20265               20270               20275

gct agg tct cca aaa cca ccg atg aac gat ggg gaa acg ggc aga 121222
Ala Arg Ser Pro Lys Pro Pro Met Asn Asp Gly Glu Thr Gly Arg
20280               20285               20290

ggt acg acc cct ccg ttc ccg cag gcc ttc tcc cct gta tcc ccc 121267
Gly Thr Thr Pro Pro Phe Pro Gln Ala Phe Ser Pro Val Ser Pro
20295               20300               20305

gcg tct cct gtt gga gac gcc gcc ggg aac gat caa cgg gaa gac 121312
Ala Ser Pro Val Gly Asp Ala Ala Gly Asn Asp Gln Arg Glu Asp
20310               20315               20320

cag cgg tct ata ccc cga caa acg acg aga gga aat tca cca ggt 121357
Gln Arg Ser Ile Pro Arg Gln Thr Thr Arg Gly Asn Ser Pro Gly
20325               20330               20335

ttg ccg tcg gtg gtc cat cga gac aga caa act cag tcc atc tcg 121402
Leu Pro Ser Val Val His Arg Asp Arg Gln Thr Gln Ser Ile Ser
20340               20345               20350

ggt aaa aag ccg ggc gat gag caa gcg ggt cat gcg cat gca tcg 121447
Gly Lys Lys Pro Gly Asp Glu Gln Ala Gly His Ala His Ala Ser
20355               20360               20365

ggg gac gga gta gtt ctc cag aaa act caa cgg ccc gct cag gga 121492
Gly Asp Gly Val Val Leu Gln Lys Thr Gln Arg Pro Ala Gln Gly
20370               20375               20380

aag agc ccg aag aaa aag act ttg aag gtt aag gtc cca ctc ccg 121537
Lys Ser Pro Lys Lys Lys Thr Leu Lys Val Lys Val Pro Leu Pro
20385               20390               20395

gcg cgg aaa ccc ggt gga cct gta ccc ggc ccg gtt gag caa ttg 121582
Ala Arg Lys Pro Gly Gly Pro Val Pro Gly Pro Val Glu Gln Leu
20400               20405               20410

tac cac gtc ctt tcg gac agc gtt ccc gct aag ggg gca aag gcg 121627
Tyr His Val Leu Ser Asp Ser Val Pro Ala Lys Gly Ala Lys Ala
20415               20420               20425

gac ctg ccg ttt gag acc gat gat acc cgc cca agg aaa cat gat 121672
Asp Leu Pro Phe Glu Thr Asp Asp Thr Arg Pro Arg Lys His Asp
20430               20435               20440

gcc cgg ggt ata aca cct cgc gtc cct gga cgt tcg tcg ggg ggc 121717
Ala Arg Gly Ile Thr Pro Arg Val Pro Gly Arg Ser Ser Gly Gly
20445               20450               20455

aaa cct aga gcg ttt ttg gcc ctg ccg gga aga tcc cac gca cca 121762
Lys Pro Arg Ala Phe Leu Ala Leu Pro Gly Arg Ser His Ala Pro
20460               20465               20470

gac ccg att gag gat gac agc cca gtg gag aaa aag cca aag agt 121807
Asp Pro Ile Glu Asp Asp Ser Pro Val Glu Lys Lys Pro Lys Ser
20475               20480               20485

cgt gag ttt gtt tcg tct tca tcc tct tcc tcg tcg tgg gga tcg 121852
Arg Glu Phe Val Ser Ser Ser Ser Ser Ser Ser Ser Trp Gly Ser
20490               20495               20500

tca tcg gag gat gaa gac gat gaa ccc cgg cgc gtt tcg gtg gga 121897
Ser Ser Glu Asp Glu Asp Asp Glu Pro Arg Arg Val Ser Val Gly
20505               20510               20515

agt gaa act aca ggc agc agg tcc gga cgc gaa cac gcc cct tcc 121942
Ser Glu Thr Thr Gly Ser Arg Ser Gly Arg Glu His Ala Pro Ser
20520               20525               20530

ccg tca aat tcg gat gat tcg gac tca aat gat ggt ggg tcg acg 121987
Pro Ser Asn Ser Asp Asp Ser Asp Ser Asn Asp Gly Gly Ser Thr
20535               20540               20545

aaa caa aat atc caa ccg gga tat cga tcc atc agc ggt ccc gat 122032
Lys Gln Asn Ile Gln Pro Gly Tyr Arg Ser Ile Ser Gly Pro Asp
20550               20555               20560

ccg agg att cgt aag acc aaa cgt ctt gcg ggg gaa ccg ggg cgc 122077
Pro Arg Ile Arg Lys Thr Lys Arg Leu Ala Gly Glu Pro Gly Arg
20565               20570               20575

cag aga cag aaa tca ttt tcc ctg ccg cga tcc aga acc ccg ata 122122
Gln Arg Gln Lys Ser Phe Ser Leu Pro Arg Ser Arg Thr Pro Ile
20580               20585               20590

att ccc ccg gtg tcg ggg ccg ctc atg atg ccc gac gga agc cct 122167
Ile Pro Pro Val Ser Gly Pro Leu Met Met Pro Asp Gly Ser Pro
20595               20600               20605

tgg ccc gga tcg gca ccc ctc cca tcc aac agg gtg cgg ttt gga 122212
Trp Pro Gly Ser Ala Pro Leu Pro Ser Asn Arg Val Arg Phe Gly
20610               20615               20620

ccg tcc ggg gag acc aga gag ggt cac tgg gag gat gag gct gtg 122257
Pro Ser Gly Glu Thr Arg Glu Gly His Trp Glu Asp Glu Ala Val
20625               20630               20635

aga gcg gcg cgg gct cgt tac gag gcc tca acg gaa ccc gtg ccg 122302
Arg Ala Ala Arg Ala Arg Tyr Glu Ala Ser Thr Glu Pro Val Pro
20640               20645               20650

ctt tac gtg ccg gag ttg gga gat ccg gct aga cag tac cgc gcg 122347
Leu Tyr Val Pro Glu Leu Gly Asp Pro Ala Arg Gln Tyr Arg Ala
20655               20660               20665

ctg att aac ctg atc tac tgt cca gac aga gac cct ata gca tgg 122392
Leu Ile Asn Leu Ile Tyr Cys Pro Asp Arg Asp Pro Ile Ala Trp
20670               20675               20680

ctc cag aac ccc aag ctg acc ggt gtc aac tcg gcc ctg aac cag 122437
Leu Gln Asn Pro Lys Leu Thr Gly Val Asn Ser Ala Leu Asn Gln
20685               20690               20695

ttc tac caa aag ctg ttg cca ccg gga cgg gcg ggt acc gcc gtt 122482
Phe Tyr Gln Lys Leu Leu Pro Pro Gly Arg Ala Gly Thr Ala Val
20700               20705               20710

acg ggg agc gta gcg tct ccc gtt ccg cat gta ggc gaa gcc atg 122527
Thr Gly Ser Val Ala Ser Pro Val Pro His Val Gly Glu Ala Met
20715               20720               20725

gcc acg ggg gag gcc ctc tgg gct ctc ccc cac gcg gcc gcg gcc 122572
Ala Thr Gly Glu Ala Leu Trp Ala Leu Pro His Ala Ala Ala Ala
20730               20735               20740

gtg gct atg agc cgt cga tac gac cgg gcc caa aaa cac ttt atc 122617
Val Ala Met Ser Arg Arg Tyr Asp Arg Ala Gln Lys His Phe Ile
20745               20750               20755

cta cag agt ctc cgc aga gcc ttt gcc agc atg gca tac ccc gag 122662
Leu Gln Ser Leu Arg Arg Ala Phe Ala Ser Met Ala Tyr Pro Glu
20760               20765               20770

gca acg ggc tcc agt ccg gcg gcg cgg atc tcc cgc ggt cac cct 122707
Ala Thr Gly Ser Ser Pro Ala Ala Arg Ile Ser Arg Gly His Pro
20775               20780               20785

tct cca aca acc ccg gcc aca cag gct ccc gac cct cag ccg tcg 122752
Ser Pro Thr Thr Pro Ala Thr Gln Ala Pro Asp Pro Gln Pro Ser
20790               20795               20800

gcc gcc gca cgc tct ctt tct gtg tgt cca ccg gat gat cgt tta 122797
Ala Ala Ala Arg Ser Leu Ser Val Cys Pro Pro Asp Asp Arg Leu
20805               20810               20815

cga act ccg cgc aag cgc aag tcc cag cca gtc gag agc aga agc 122842
Arg Thr Pro Arg Lys Arg Lys Ser Gln Pro Val Glu Ser Arg Ser
20820               20825               20830

ctc ctc gac aag att agg gag aca ccc gtc gcg gac gcc cgg gtt 122887
Leu Leu Asp Lys Ile Arg Glu Thr Pro Val Ala Asp Ala Arg Val
20835               20840               20845

gca gac gat cat gtg gtt tcc aag gcc aag agg cgg gta tcc gag 122932
Ala Asp Asp His Val Val Ser Lys Ala Lys Arg Arg Val Ser Glu
20850               20855               20860

ccc gtg acc atc acc tcg ggc cct gtg gtg gat ccc ccc gcc gta 122977
Pro Val Thr Ile Thr Ser Gly Pro Val Val Asp Pro Pro Ala Val
20865               20870               20875

ata acg atg cca ctt gac gga ccg gcc cca aac ggg gga ttt cgg 123022
Ile Thr Met Pro Leu Asp Gly Pro Ala Pro Asn Gly Gly Phe Arg
20880               20885               20890

cgt att ccc cgg ggg gcc ctg cat acc ccg gtc ccg tcg gac cag 123067
Arg Ile Pro Arg Gly Ala Leu His Thr Pro Val Pro Ser Asp Gln
20895               20900               20905

gct cgc aag gcg tac tgt acc ccc gaa acc atc gcc cgt ctg gtc 123112
Ala Arg Lys Ala Tyr Cys Thr Pro Glu Thr Ile Ala Arg Leu Val
20910               20915               20920

gac gac cca ttg ttt ccc acg gcc tgg cgc cct gcg cta agc ttt 123157
Asp Asp Pro Leu Phe Pro Thr Ala Trp Arg Pro Ala Leu Ser Phe
20925               20930               20935

gat ccc ggc gcc ttg gcg gaa atc gcc gct cgg cgt ccg ggc gga 123202
Asp Pro Gly Ala Leu Ala Glu Ile Ala Ala Arg Arg Pro Gly Gly
20940               20945               20950

gga gac cga cgg ttt ggt cca ccc agc gga gtg gag gcg ctg cga 123247
Gly Asp Arg Arg Phe Gly Pro Pro Ser Gly Val Glu Ala Leu Arg
20955               20960               20965

cgg agg tgc gcc tgg atg cgg cag atc cca gac ccg gag gat gtg 123292
Arg Arg Cys Ala Trp Met Arg Gln Ile Pro Asp Pro Glu Asp Val
20970               20975               20980

agg ctt ctg atc atc tac gat ccg ttg ccc gga gag gac atc aac 123337
Arg Leu Leu Ile Ile Tyr Asp Pro Leu Pro Gly Glu Asp Ile Asn
20985               20990               20995

ggc ccc ctc gag agc acc ctc gcg aca gat ccg gga ccg tca tgg 123382
Gly Pro Leu Glu Ser Thr Leu Ala Thr Asp Pro Gly Pro Ser Trp
21000               21005               21010

agt cca tcc cga ggg gga ctg tct gtg gtc ctg gca gcc ctg agt 123427
Ser Pro Ser Arg Gly Gly Leu Ser Val Val Leu Ala Ala Leu Ser
21015               21020               21025

aac cgg ttg tgc ctg ccg agc act cat gcc tgg gcc ggg aac tgg 123472
Asn Arg Leu Cys Leu Pro Ser Thr His Ala Trp Ala Gly Asn Trp
21030               21035               21040

acc ggc ccg ccg gac gtg tcc gct ttg aac gcc cgg ggc gtt tta 123517
Thr Gly Pro Pro Asp Val Ser Ala Leu Asn Ala Arg Gly Val Leu
21045               21050               21055

tta ctg tcg acc cga gac ctg gcc ttt gcc ggg gcc gtc gag tat 123562
Leu Leu Ser Thr Arg Asp Leu Ala Phe Ala Gly Ala Val Glu Tyr
21060               21065               21070

cta ggc tcg cgg ttg gcc tct gcc cgg cgc cgg ttg ctg gtg ttg 123607
Leu Gly Ser Arg Leu Ala Ser Ala Arg Arg Arg Leu Leu Val Leu
21075               21080               21085

gac gcg gtg gcc ctc gag agg tgg ccc agg gat gga ccc gct ttg 123652
Asp Ala Val Ala Leu Glu Arg Trp Pro Arg Asp Gly Pro Ala Leu
21090               21095               21100

tct cag tat cac gtg tac gtc cgg gcc ccg gcg cga ccg gac gcc 123697
Ser Gln Tyr His Val Tyr Val Arg Ala Pro Ala Arg Pro Asp Ala
21105               21110               21115

cag gcc gtc gtc cga tgg cca gac tcg gcg gtc aca gaa gga ctc 123742
Gln Ala Val Val Arg Trp Pro Asp Ser Ala Val Thr Glu Gly Leu
21120               21125               21130

gcc cgg gcc gtg ttt gca tcg tcg cgc acc ttt ggg cca gcg agt 123787
Ala Arg Ala Val Phe Ala Ser Ser Arg Thr Phe Gly Pro Ala Ser
21135               21140               21145

ttt gct cgt atc gag act gcg ttt gcc aac ctg tac ccg ggc gaa 123832
Phe Ala Arg Ile Glu Thr Ala Phe Ala Asn Leu Tyr Pro Gly Glu
21150               21155               21160

caa ccc ctg tgt ttg tgc cgc ggt ggg aac gtc gca tac acc gtg 123877
Gln Pro Leu Cys Leu Cys Arg Gly Gly Asn Val Ala Tyr Thr Val
21165               21170               21175

tgt acc cgc gcg ggc ccc aag acc cgc gtc ccc ctg tcg ccc cgt 123922
Cys Thr Arg Ala Gly Pro Lys Thr Arg Val Pro Leu Ser Pro Arg
21180               21185               21190

gaa tac cgg cag tac gtg ctg ccg ggt ttt gac ggt tgc aag gac 123967
Glu Tyr Arg Gln Tyr Val Leu Pro Gly Phe Asp Gly Cys Lys Asp
21195               21200               21205

ctc gcg cga cag tct cgg ggt ctg ggg ctc ggg gca gcc gac ttt 124012
Leu Ala Arg Gln Ser Arg Gly Leu Gly Leu Gly Ala Ala Asp Phe
21210               21215               21220

gtg gac gag gcg gca cat agc cac cgc gca gca aac cga tgg ggc 124057
Val Asp Glu Ala Ala His Ser His Arg Ala Ala Asn Arg Trp Gly
21225               21230               21235

ctg ggt gcc gcg ctt cga ccc gtc ttc ctt ccc gag gga cgg aga 124102
Leu Gly Ala Ala Leu Arg Pro Val Phe Leu Pro Glu Gly Arg Arg
21240               21245               21250

ccg ggg gcc gcc ggg ccg gag gcc ggc gac gta ccc acc tgg gcg 124147
Pro Gly Ala Ala Gly Pro Glu Ala Gly Asp Val Pro Thr Trp Ala
21255               21260               21265

agg gtg ttt tgc cgc cac gcc ctg ctg gaa ccc gac cct gcc gca 124192
Arg Val Phe Cys Arg His Ala Leu Leu Glu Pro Asp Pro Ala Ala
21270               21275               21280

gaa cca ctc gtg ctt cca ccc gtg gcc ggt cgg tcg gtg gcg ctg 124237
Glu Pro Leu Val Leu Pro Pro Val Ala Gly Arg Ser Val Ala Leu
21285               21290               21295

tat gcg tcg gcg gac gag gct cgg aat gcc ctc ccc ccg att ccc 124282
Tyr Ala Ser Ala Asp Glu Ala Arg Asn Ala Leu Pro Pro Ile Pro
21300               21305               21310

aga   gta atg tgg ccg ccc   ggt ttt ggg gcc gcg   gag acg gtg ttg 124327
Arg   Val Met Trp Pro Pro   Gly Phe Gly Ala Ala   Glu Thr Val Leu
21315                 21320                 21325

gag   ggg agc gac gga aca   cgg ttc gtg ttc gga   cac cac ggg ggc 124372
Glu   Gly Ser Asp Gly Thr   Arg Phe Val Phe Gly   His His Gly Gly
21330                 21335                 21340

tcg   gaa cgg ccg tca gaa   acc cag gcg ggg cga   cag cgg cgc acc 124417
Ser   Glu Arg Pro Ser Glu   Thr Gln Ala Gly Arg   Gln Arg Arg Thr
21345                 21350                 21355

gca   gac gac aga gaa cac   gct ttg gag ctg gac   gat tgg gag gtg 124462
Ala   Asp Asp Arg Glu His   Ala Leu Glu Leu Asp   Asp Trp Glu Val
21360                 21365                 21370

ggg   tgt gaa gac gcg tgg   gac agc gag gag ggg   ggc ggg gac gac 124507
Gly   Cys Glu Asp Ala Trp   Asp Ser Glu Glu Gly   Gly Gly Asp Asp
21375                 21380                 21385

ggg   gac gca ccg ggg tca   tcc ttt ggg gtg agc   atc gtg tcg gtg 124552
Gly   Asp Ala Pro Gly Ser   Ser Phe Gly Val Ser   Ile Val Ser Val
21390                 21395                 21400

gcc   ccg ggt gtg ctg cga   gac cgc cgg gtg ggt   ttg cgc ccg gcg 124597
Ala   Pro Gly Val Leu Arg   Asp Arg Arg Val Gly   Leu Arg Pro Ala
21405                 21410                 21415

gtc   aag gtg gag ctg ttg   tcc tcg tcc tcg tcc   tcc gag gac gag 124642
Val   Lys Val Glu Leu Leu   Ser Ser Ser Ser Ser   Ser Glu Asp Glu
21420                 21425                 21430

gac   gat gtg tgg gga ggg   cgc ggg ggg agg agc   ccc ccg cag agt 124687
Asp   Asp Val Trp Gly Gly   Arg Gly Gly Arg Ser   Pro Pro Gln Ser
21435                 21440                 21445

cgg   ggg tga cggagtcccc tccttttctc gtgagcgcca ctggcgcgcg         124736
Arg   Gly
21450

gactgtttgt tgttaataaa agcggaacgg tttttatgaa aaaagtgtct gtctgtctgt 124796

gcgggcgggc gacgggcggg ctggtcggac ccccccccga aaataacccc cccccggttt 124856

ctgggcgccc ggcggacccc gggagagg                                    124884

<210>  9
<211>  238
<212>  PRT
<213>  Varicella zoster

<400>  9

Met His Val Ile Ser Glu Thr Leu Ala Tyr Gly His Val Pro Ala Phe
1               5                   10                  15

Ile Met Gly Ser Thr Leu Val Arg Pro Ser Leu Asn Ala Thr Ala Glu
            20                  25                  30

Glu Asn Pro Ala Ser Glu Thr Arg Cys Leu Leu Arg Val Leu Ala Gly
        35                  40                  45

Arg Thr Val Asp Leu Pro Gly Gly Gly Thr Leu His Ile Thr Cys Thr

50                  55                  60

Lys Thr Tyr Val Ile Ile Gly Lys Tyr Ser Lys Pro Gly Glu Arg Leu
65                  70                  75                  80

Ser Leu Ala Arg Leu Ile Gly Arg Ala Met Thr Pro Gly Gly Ala Arg
                85                  90                  95

Thr Phe Ile Ile Leu Ala Met Lys Glu Lys Arg Ser Thr Thr Leu Gly
            100                 105                 110

Tyr Glu Cys Gly Thr Gly Leu His Leu Leu Ala Pro Ser Met Gly Thr
        115                 120                 125

Phe Leu Arg Thr His Gly Leu Ser Asn Arg Asp Leu Cys Leu Trp Arg
    130                 135                 140

Gly Asn Ile Tyr Asp Met His Met Gln Arg Leu Met Phe Trp Glu Asn
145                 150                 155                 160

Ile Ala Gln Asn Thr Thr Glu Thr Pro Cys Ile Thr Ser Thr Leu Thr
                165                 170                 175

Cys Asn Leu Thr Glu Asp Ser Gly Glu Ala Ala Leu Thr Thr Ser Asp
            180                 185                 190

Arg Pro Thr Leu Pro Thr Leu Thr Ala Gln Gly Arg Pro Thr Val Ser
        195                 200                 205

Asn Ile Arg Gly Ile Leu Lys Gly Ser Pro Arg Gln Gln Pro Val Cys
    210                 215                 220

His Arg Val Arg Phe Ala Glu Pro Thr Glu Gly Val Leu Met
225                 230                 235

<210>  10
<211>  259
<212>  PRT
<213>  Varicella zoster

<400>  10

Met Gln Thr Val Cys Ala Ser Leu Cys Gly Tyr Ala Arg Ile Pro Thr
1               5                   10                  15

Glu Glu Pro Ser Tyr Glu Glu Val Arg Val Asn Thr His Pro Gln Gly
            20                  25                  30

Ala Ala Leu Leu Arg Leu Gln Glu Ala Leu Thr Ala Val Asn Gly Leu
        35                  40                  45

Leu Pro Ala Pro Leu Thr Leu Glu Asp Val Val Ala Ser Ala Asp Asn
    50                  55                  60

Thr Arg Arg Leu Val Arg Ala Gln Ala Leu Ala Arg Thr Tyr Ala Ala
65                  70                  75                  80

Cys Ser Arg Asn Ile Glu Cys Leu Lys Gln His His Phe Thr Glu Asp
                85                  90                  95

Asn Pro Gly Leu Asn Ala Val Val Arg Ser His Met Glu Asn Ser Lys
            100                 105                 110

Arg Leu Ala Asp Met Cys Leu Ala Ala Ile Thr His Leu Tyr Leu Ser
        115                 120                 125

Val Gly Ala Val Asp Val Thr Thr Asp Asp Ile Val Asp Gln Thr Leu
    130                 135                 140

Arg Met Thr Ala Glu Ser Glu Val Val Met Ser Asp Val Val Leu Leu
145                 150                 155                 160

Glu Lys Thr Leu Gly Val Val Ala Lys Pro Gln Ala Ser Phe Asp Val
                165                 170                 175

Ser His Asn His Glu Leu Ser Ile Ala Lys Gly Glu Asn Val Gly Leu
            180                 185                 190

Lys Thr Ser Pro Ile Lys Ser Glu Ala Thr Gln Leu Ser Glu Ile Lys
        195                 200                 205

Pro Pro Leu Ile Glu Val Ser Asp Asn Asn Thr Ser Asn Leu Thr Lys
    210                 215                 220

Lys Thr Tyr Pro Thr Glu Thr Leu Gln Pro Val Leu Thr Pro Lys Gln
225                 230                 235                 240

Thr Gln Asp Val Gln Arg Thr Thr Pro Ala Ile Lys Lys Ser His Val
                245                 250                 255

Met Leu Val

<210>  11
<211>  87
<212>  PRT
<213>  Varicella zoster

<400>  11

Met Gly Ser Ile Thr Ala Ser Phe Ile Leu Ile Thr Met Gln Ile Leu
1               5                   10                  15

Phe Phe Cys Glu Asp Ser Ser Gly Glu Pro Asn Phe Ala Glu Arg Asn
            20                  25                  30

Phe Trp His Ala Ser Cys Ser Ala Arg Gly Val Tyr Ile Asp Gly Ser
        35                  40                  45

Met Ile Thr Thr Leu Phe Phe Tyr Ala Ser Leu Leu Gly Val Cys Val
    50                  55                  60

Ala Leu Ile Ser Leu Ala Tyr His Ala Cys Phe Arg Leu Phe Thr Arg
65                  70                  75                  80

Ser Val Leu Arg Ser Thr Trp
                85

<210>  12
<211>  302
<212>  PRT
<213>  Varicella zoster

<400>  12

Met Ala Ser Ser Asp Gly Asp Arg Leu Cys Arg Ser Asn Ala Val Arg
1               5                   10                  15

Arg Lys Thr Thr Pro Ser Tyr Ser Gly Gln Tyr Arg Thr Ala Arg Arg
            20                  25                  30

Ser Val Val Val Gly Pro Pro Asp Asp Ser Asp Asp Ser Leu Gly Tyr
        35                  40                  45

Ile Thr Thr Val Gly Ala Asp Ser Pro Ser Pro Val Tyr Ala Asp Leu
    50                  55                  60

Tyr Phe Glu His Lys Asn Thr Thr Pro Arg Val His Gln Pro Asn Asp
65                  70                  75                  80

Ser Ser Gly Ser Glu Asp Asp Phe Glu Asp Ile Asp Glu Val Val Ala
                85                  90                  95

Ala Phe Arg Glu Ala Arg Leu Arg His Glu Leu Val Glu Asp Ala Val
            100                 105                 110

Tyr Glu Asn Pro Leu Ser Val Glu Lys Pro Ser Arg Ser Phe Thr Lys
        115                 120                 125

Asn Ala Ala Val Lys Pro Lys Leu Glu Asp Ser Pro Lys Arg Ala Pro
    130                 135                 140

Pro Gly Ala Gly Ala Ile Ala Ser Gly Arg Pro Ile Ser Phe Ser Thr
145                 150                 155                 160

Ala Pro Lys Thr Ala Thr Ser Ser Trp Cys Gly Pro Thr Pro Ser Tyr
                165                 170                 175

Asn Lys Arg Val Phe Cys Glu Ala Val Arg Arg Val Ala Ala Met Gln
            180                 185                 190

Ala Gln Lys Ala Ala Glu Ala Ala Trp Asn Ser Asn Pro Pro Arg Asn
        195                 200                 205

Asn Ala Glu Leu Asp Arg Leu Leu Thr Gly Ala Val Ile Arg Ile Thr
    210                 215                 220

Val His Glu Gly Leu Asn Leu Ile Gln Ala Ala Asn Glu Ala Asp Leu
225                 230                 235                 240

Gly Glu Gly Ala Ser Val Ser Lys Arg Gly His Asn Arg Lys Thr Gly
                245                 250                 255

Asp Leu Gln Gly Gly Met Gly Asn Glu Pro Met Tyr Ala Gln Val Arg
            260                 265                 270

Lys Pro Lys Ser Arg Thr Asp Thr Gln Thr Thr Gly Arg Ile Thr Asn
        275                 280                 285

Arg Ser Arg Ala Arg Ser Ala Ser Arg Thr Asp Thr Arg Lys
    290                 295                 300

<210> 13
<211> 410
<212> PRT
<213> Varicella zoster

<400> 13

Met Glu Cys Asn Leu Gly Thr Glu His Pro Ser Thr Asp Thr Trp Asn
1               5                   10                  15

Arg Ser Lys Thr Glu Gln Ala Val Val Asp Ala Phe Asp Glu Ser Leu
            20                  25                  30

Phe Gly Asp Val Ala Ser Asp Ile Gly Phe Glu Thr Ser Leu Tyr Ser
        35                  40                  45

His Ala Val Lys Thr Ala Pro Ser Pro Pro Trp Val Ala Ser Pro Lys
    50                  55                  60

Ile Leu Tyr Gln Gln Leu Ile Arg Asp Leu Asp Phe Ser Glu Gly Pro
65                  70                  75                  80

Arg Leu Leu Ser Cys Leu Glu Thr Trp Asn Glu Asp Leu Phe Ser Cys
                85                  90                  95

Phe Pro Ile Asn Glu Asp Leu Tyr Ser Asp Met Met Val Leu Ser Pro
            100                 105                 110

Asp Pro Asp Asp Val Ile Ser Thr Val Ser Thr Lys Asp His Val Glu

115                 120                 125

Met Phe Asn Leu Thr Thr Arg Gly Ser Val Arg Leu Pro Ser Pro Pro
    130                 135                 140

Lys Gln Pro Thr Gly Leu Pro Ala Tyr Val Gln Glu Val Gln Asp Ser
145                 150                 155                 160

Phe Thr Val Glu Leu Arg Ala Arg Glu Glu Ala Tyr Thr Lys Leu Leu
                165                 170                 175

Val Thr Tyr Cys Lys Ser Ile Ile Arg Tyr Leu Gln Gly Thr Ala Lys
            180                 185                 190

Arg Thr Thr Ile Gly Leu Asn Ile Gln Asn Pro Asp Gln Lys Ala Tyr
        195                 200                 205

Thr Gln Leu Arg Gln Ser Ile Leu Leu Arg Tyr Tyr Arg Glu Val Ala
    210                 215                 220

Ser Leu Ala Arg Leu Leu Tyr Leu His Leu Tyr Leu Thr Val Thr Arg
225                 230                 235                 240

Glu Phe Ser Trp Arg Leu Tyr Ala Ser Gln Ser Ala His Pro Asp Val
                245                 250                 255

Phe Ala Ala Leu Lys Phe Thr Trp Thr Glu Arg Arg Gln Phe Thr Cys
            260                 265                 270

Ala Phe His Pro Val Leu Cys Asn His Gly Ile Val Leu Leu Glu Gly
        275                 280                 285

Lys Pro Leu Thr Ala Ser Ala Leu Arg Glu Ile Asn Tyr Arg Arg Arg
    290                 295                 300

Glu Leu Gly Leu Pro Leu Val Arg Cys Gly Leu Val Glu Glu Asn Lys
305                 310                 315                 320

Ser Pro Leu Val Gln Gln Pro Ser Phe Ser Val His Leu Pro Arg Ser
                325                 330                 335

Val Gly Phe Leu Thr His His Ile Lys Arg Lys Leu Asp Ala Tyr Ala
            340                 345                 350

Val Lys His Pro Gln Glu Pro Arg His Val Arg Ala Asp His Pro Tyr
        355                 360                 365

Ala Lys Val Val Glu Asn Arg Asn Tyr Gly Ser Ser Ile Glu Ala Met
    370                 375                 380

Ile Leu Ala Pro Pro Ser Pro Ser Glu Ile Leu Pro Gly Asp Pro Pro

385                 390                 395                 400

Arg Pro Pro Thr Cys Gly Phe Leu Thr Arg
                405                 410

<210>  14
<211>  819
<212>  PRT
<213>  Varicella zoster

<400>  14

Met Gln Ser Gly His Tyr Asn Arg Arg Gln Ser Arg Arg Gln Arg Ile
1               5                   10                  15

Ser Ser Asn Thr Thr Asp Ser Pro Arg His Thr His Gly Thr Arg Tyr
            20                  25                  30

Arg Ser Thr Asn Trp Tyr Thr His Pro Pro Gln Ile Leu Ser Asn Ser
        35                  40                  45

Glu Thr Leu Val Ala Val Gln Glu Leu Leu Asn Ser Glu Met Asp Gln
    50                  55                  60

Asp Ser Ser Ser Asp Ala Ser Asp Asp Phe Pro Gly Tyr Ala Leu His
65                  70                  75                  80

His Ser Thr Tyr Asn Gly Ser Glu Gln Asn Thr Ser Thr Ser Arg His
                85                  90                  95

Glu Asn Arg Ile Phe Lys Leu Thr Glu Arg Glu Ala Asn Glu Glu Ile
            100                 105                 110

Asn Ile Asn Thr Asp Ala Ile Asp Asp Glu Gly Glu Ala Glu Glu Gly
        115                 120                 125

Glu Ala Glu Glu Asp Ala Ile Asp Asp Glu Gly Glu Ala Glu Glu Gly
    130                 135                 140

Glu Ala Glu Glu Asp Ala Ile Asp Asp Glu Gly Glu Ala Glu Glu Gly
145                 150                 155                 160

Glu Ala Glu Glu Asp Ala Ile Asp Asp Glu Gly Glu Ala Glu Glu Gly
                165                 170                 175

Glu Ala Glu Glu Gly Glu Ala Glu Glu Gly Glu Ala Glu Glu Asp Ala
            180                 185                 190

Ile Asp Asp Glu Gly Glu Ala Glu Glu Asp Ala Ala Glu Glu Asp Ala
        195                 200                 205

Ile Asp Asp Glu Gly Glu Ala Glu Glu Asp Tyr Phe Ser Val Ser Gln
    210                 215                 220

Val Cys Ser Arg Asp Ala Asp Glu Val Tyr Phe Thr Leu Asp Pro Glu
225                 230                 235                 240

Ile Ser Tyr Ser Thr Asp Leu Arg Ile Ala Lys Val Met Glu Pro Ala
                245                 250                 255

Val Ser Lys Glu Leu Asn Val Ser Lys Arg Cys Val Glu Pro Val Thr
            260                 265                 270

Leu Thr Gly Ser Met Leu Ala His Asn Gly Phe Asp Glu Ser Trp Phe
        275                 280                 285

Ala Met Arg Glu Cys Thr Arg Arg Glu Tyr Ile Thr Val Gln Gly Leu
    290                 295                 300

Tyr Asp Pro Ile His Leu Arg Tyr Gln Phe Asp Thr Ser Arg Met Thr
305                 310                 315                 320

Pro Pro Gln Ile Leu Arg Thr Ile Pro Ala Leu Pro Asn Met Thr Leu
                325                 330                 335

Gly Glu Leu Leu Leu Ile Phe Pro Ile Glu Phe Met Ala Gln Pro Ile
            340                 345                 350

Ser Ile Glu Arg Ile Leu Val Glu Asp Val Phe Leu Asp Arg Arg Ala
        355                 360                 365

Ser Ser Lys Thr His Lys Tyr Gly Pro Arg Trp Asn Ser Val Tyr Ala
    370                 375                 380

Leu Pro Tyr Asn Ala Gly Lys Met Tyr Val Gln His Ile Pro Gly Phe
385                 390                 395                 400

Tyr Asp Val Ser Leu Arg Ala Val Gly Gln Gly Thr Ala Ile Trp His
                405                 410                 415

His Met Ile Leu Ser Thr Ala Ala Cys Ala Ile Ser Asn Arg Ile Ser
            420                 425                 430

His Gly Asp Gly Leu Gly Phe Leu Leu Asp Ala Ala Ile Arg Ile Ser
        435                 440                 445

Ala Asn Cys Ile Phe Leu Gly Arg Asn Asp Asn Phe Gly Val Gly Asp
    450                 455                 460

Pro Cys Trp Leu Glu Asp His Leu Ala Gly Leu Pro Arg Glu Ala Val
465                 470                 475                 480

Pro Asp Val Leu Gln Val Thr Gln Leu Val Leu Pro Asn Arg Gly Pro
                485                 490                 495

Thr Val Ala Ile Met Arg Gly Phe Phe Gly Ala Leu Ala Tyr Trp Pro
            500                 505                 510

Glu Leu Arg Ile Ala Ile Ser Glu Pro Ser Thr Ser Leu Val Arg Tyr
        515                 520                 525

Ala Thr Gly His Met Glu Leu Ala Glu Trp Phe Leu Phe Ser Arg Thr
    530                 535                 540

His Ser Leu Lys Pro Gln Phe Thr Pro Thr Glu Arg Glu Met Leu Ala
545                 550                 555                 560

Ser Phe Phe Thr Leu Tyr Val Thr Leu Gly Gly Gly Met Leu Asn Trp
                565                 570                 575

Ile Cys Arg Ala Thr Ala Met Tyr Leu Ala Ala Pro Tyr His Ser Arg
            580                 585                 590

Ser Ala Tyr Ile Ala Val Cys Glu Ser Leu Pro Tyr Tyr Tyr Ile Pro
        595                 600                 605

Val Asn Ser Asp Leu Leu Cys Asp Leu Glu Val Leu Leu Leu Gly Glu
    610                 615                 620

Val Asp Leu Pro Thr Val Cys Glu Ser Tyr Ala Thr Ile Ala His Glu
625                 630                 635                 640

Leu Thr Gly Tyr Glu Ala Val Arg Thr Ala Ala Thr Asn Phe Met Ile
                645                 650                 655

Glu Phe Ala Asp Cys Tyr Lys Glu Ser Glu Thr Asp Leu Met Val Ser
            660                 665                 670

Ala Tyr Leu Gly Ala Val Leu Leu Leu Gln Arg Val Leu Gly His Ala
        675                 680                 685

Asn Leu Leu Leu Leu Leu Leu Ser Gly Ala Ala Leu Tyr Gly Gly Cys
    690                 695                 700

Ser Ile Tyr Ile Pro Arg Gly Ile Leu Asp Ala Tyr Asn Thr Leu Met
705                 710                 715                 720

Leu Ala Ala Ser Pro Leu Tyr Ala His Gln Thr Leu Thr Ser Phe Trp
                725                 730                 735

Lys Asp Arg Asp Asp Ala Met Gln Thr Leu Gly Ile Arg Pro Thr Thr
            740                 745                 750

Asp Val Leu Pro Lys Glu Gln Asp Arg Ile Val Gln Ala Ser Pro Ile
        755                 760                 765

Glu Met Asn Phe Arg Phe Val Gly Leu Glu Thr Ile Tyr Pro Arg Glu
    770                 775                 780

Gln Pro Ile Pro Ser Val Asp Leu Ala Glu Asn Leu Met Gln Tyr Arg
785                 790                 795                 800

Asn Glu Ile Leu Gly Leu Asp Trp Lys Ser Val Ala Met His Leu Leu
                805                 810                 815

Arg Lys Tyr

<210>  15
<211>  661
<212>  PRT
<213>  Varicella zoster

<400>  15

Met Phe Ser Arg Phe Ala Arg Ser Phe Ser Ser Asp Asp Arg Thr Arg
1               5                   10                  15

Lys Ser Tyr Asp Gly Ser Tyr Gln Ser Phe Asn Ala Gly Glu Arg Asp
            20                  25                  30

Leu Pro Thr Pro Thr Arg Asp Trp Cys Ser Ile Ser Gln Arg Ile Thr
        35                  40                  45

Ser Glu Arg Val Arg Asp Gly Cys Leu Ile Pro Thr Pro Gly Glu Ala
    50                  55                  60

Leu Glu Thr Ala Val Lys Ala Leu Ser Glu Lys Thr Asp Ser Leu Thr
65                  70                  75                  80

Ser Pro Val Leu Gln Ser Thr Glu Arg His Ser Val Leu Leu Gly Leu
                85                  90                  95

His His Asn Asn Val Pro Glu Ser Leu Val Val Ser Cys Met Ser Asn
            100                 105                 110

Asp Val His Asp Gly Phe Met Gln Arg Tyr Met Glu Thr Ile Gln Arg
        115                 120                 125

Cys Leu Asp Asp Leu Lys Leu Ser Gly Asp Gly Leu Trp Trp Val Tyr
    130                 135                 140

Glu Asn Thr Tyr Trp Gln Tyr Leu Lys Tyr Thr Thr Gly Ala Glu Val
145                 150                 155                 160

Pro Val Thr Ser Glu Lys Val Asn Lys Lys Ser Lys Ser Thr Val Leu
                165                 170                 175

Leu Phe Ser Ser Val Val Ala Asn Lys Pro Ile Ser Arg His Pro Phe
            180                 185                 190

Lys Ser Lys Val Ile Asn Ser Asp Tyr Arg Gly Ile Cys Gln Glu Leu
        195                 200                 205

Arg Glu Ala Leu Gly Ala Val Gln Lys Tyr Met Tyr Phe Met Arg Pro
    210                 215                 220

Asp Asp Pro Thr Asn Pro Ser Pro Asp Thr Arg Ile Arg Val Gln Glu
225                 230                 235                 240

Ile Ala Ala Tyr Thr Ala Thr Gly Tyr Gly Trp Met Leu Trp Phe Leu
                245                 250                 255

Asp Val Val Asp Ala Arg Val Cys Arg His Leu Lys Leu Gln Phe Arg
            260                 265                 270

Arg Ile Arg Gly Pro Arg Ala Ser Val Ile Pro Asp Asp Leu Leu Arg
        275                 280                 285

Arg His Leu Lys Thr Gly Pro Ala Val Ser Ala Gly Thr Gly Val Ala
    290                 295                 300

Phe Ile Leu Ala Ala Thr Thr Ala Ser Ala Leu Thr Ala Leu Leu Arg
305                 310                 315                 320

Ile Ser Val Leu Trp Arg Lys Glu Glu Trp Arg Asp Gly Leu Asn Gly
                325                 330                 335

Thr Ala Ala Ala Ile Val Ala Ala Val Glu Leu Ile Thr Leu Leu His
            340                 345                 350

His His Phe Gln Tyr Leu Ile Asn Met Met Leu Ile Gly Tyr Ala Cys
        355                 360                 365

Trp Gly Asp Gly Gly Leu Asn Asp Pro Tyr Ile Leu Lys Ala Leu Arg
    370                 375                 380

Ala Gln Gly Arg Phe Leu Tyr Phe Ala Gly Gln Leu Val Arg Thr Met
385                 390                 395                 400

Ser Thr His Ser Trp Val Val Leu Glu Thr Ser Thr His Met Trp Phe
                405                 410                 415

Ser Arg Ala Val Ala Gln Ser Ile Leu Ala His Gly Gly Lys Pro Thr
            420                 425                 430

Lys Tyr Tyr Ala Gln Val Leu Ala Ala Ser Lys Arg Tyr Thr Pro Leu
        435                 440                 445

His Leu Arg Arg Ile Ser Glu Pro Ser Ser Val Ser Asp Gln Pro Tyr
    450                 455                 460

Ile Arg Phe Asn Arg Leu Gly Ser Pro Ile Gly Thr Gly Ile Gly Asn
465                 470                 475                 480

Leu Glu Cys Val Cys Leu Thr Gly Asn Tyr Leu Ser Asp Asp Val Asn
                485                 490                 495

Ala Ser Ser His Val Ile Asn Thr Glu Ala Pro Leu Asn Ser Ile Ala
            500                 505                 510

Pro Asp Thr Asn Arg Gln Arg Thr Ser Arg Val Leu Val Arg Pro Asp
        515                 520                 525

Thr Gly Leu Asp Val Thr Val Arg Lys Asn His Cys Leu Asp Ile Gly
    530                 535                 540

His Thr Asp Gly Ser Pro Val Asp Pro Thr Tyr Pro Asp His Tyr Thr
545                 550                 555                 560

Arg Ile Lys Ala Glu Tyr Glu Gly Pro Val Arg Asp Glu Ser Asn Thr
                565                 570                 575

Met Phe Asp Gln Arg Ser Asp Leu Arg His Ile Glu Thr Gln Ala Ser
            580                 585                 590

Leu Asn Asp His Val Tyr Glu Asn Ile Pro Pro Lys Glu Val Gly Phe
        595                 600                 605

Asn Ser Ser Ser Asp Leu Asp Val Asp Ser Leu Asn Gly Tyr Thr Ser
    610                 615                 620

Gly Asp Met His Thr Asp Asp Asp Leu Ser Pro Asp Phe Ile Pro Asn
625                 630                 635                 640

Asp Val Pro Val Arg Cys Lys Thr Thr Val Thr Phe Arg Lys Asn Thr
                645                 650                 655

Pro Lys Ser His His
            660

<210>  16
<211>  301
<212>  PRT
<213>  Varicella zoster

<400>  16

Met Gly Asp Leu Ser Cys Trp Thr Lys Val Pro Gly Phe Thr Leu Thr
1               5                   10                  15

Gly Glu Leu Gln Tyr Leu Lys Gln Val Asp Asp Ile Leu Arg Tyr Gly
            20                  25                  30

Val Arg Lys Arg Asp Arg Thr Gly Ile Gly Thr Leu Ser Leu Phe Gly
        35                  40                  45

Met Gln Ala Arg Tyr Asn Leu Arg Asn Glu Phe Pro Leu Leu Thr Thr
    50                  55                  60

Lys Arg Val Phe Trp Arg Ala Val Val Glu Glu Leu Leu Trp Phe Ile
65                  70                  75                  80

Arg Gly Ser Thr Asp Ser Lys Glu Leu Ala Ala Lys Asp Ile His Ile
                85                  90                  95

Trp Asp Ile Tyr Gly Ser Ser Lys Phe Leu Asn Arg Asn Gly Phe His
            100                 105                 110

Lys Arg His Thr Gly Asp Leu Gly Pro Ile Tyr Gly Phe Gln Trp Arg
        115                 120                 125

His Phe Gly Ala Glu Tyr Lys Asp Cys Gln Ser Asn Tyr Leu Gln Gln
    130                 135                 140

Gly Ile Asp Gln Leu Gln Thr Val Ile Asp Thr Ile Lys Thr Asn Pro
145                 150                 155                 160

Glu Ser Arg Arg Met Ile Ile Ser Ser Trp Asn Pro Lys Asp Ile Pro
                165                 170                 175

Leu Met Val Leu Pro Pro Cys His Thr Leu Cys Gln Phe Tyr Val Ala
            180                 185                 190

Asn Gly Glu Leu Ser Cys Gln Val Tyr Gln Arg Ser Gly Asp Met Gly
        195                 200                 205

Leu Gly Val Pro Phe Asn Ile Ala Gly Tyr Ala Leu Leu Thr Tyr Ile
    210                 215                 220

Val Ala His Val Thr Gly Leu Lys Thr Gly Asp Leu Ile His Thr Met
225                 230                 235                 240

Gly Asp Ala His Ile Tyr Leu Asn His Ile Asp Ala Leu Lys Val Gln
                245                 250                 255

Leu Ala Arg Ser Pro Lys Pro Phe Pro Cys Leu Lys Ile Ile Arg Asn
            260                 265                 270

Val Thr Asp Ile Asn Asp Phe Lys Trp Asp Asp Phe Gln Leu Asp Gly
        275                 280                 285

Tyr Asn Pro His Pro Pro Leu Lys Met Glu Met Ala Leu
    290                 295                 300

<210>  17
<211>  455
<212>  PRT
<213>  Varicella zoster

<400>  17

Met Gly Leu Phe Gly Leu Thr Arg Phe Ile His Glu His Lys Leu Val
1               5                   10                  15

Lys Pro Ser Ile Ile Ser Thr Pro Pro Gly Val Leu Thr Pro Val Ala
            20                  25                  30

Val Asp Val Trp Asn Val Met Tyr Thr Leu Leu Glu Arg Leu Tyr Pro
        35                  40                  45

Val Gly Lys Arg Glu Asn Leu His Gly Pro Ser Val Thr Ile His Cys
    50                  55                  60

Leu Gly Val Leu Leu Arg Leu Leu Thr Gln Arg Ser Tyr Tyr Pro Ile
65                  70                  75                  80

Phe Val Leu Glu Arg Cys Thr Asp Gly Pro Leu Ser Arg Gly Ala Lys
                85                  90                  95

Ala Ile Met Ser Arg Ala Met Asn His Asp Glu Arg Gly Thr Ser Asp
            100                 105                 110

Leu Thr Arg Val Leu Leu Ser Ser Asn Thr Ser Cys Ser Ile Lys Tyr
        115                 120                 125

Asn Lys Thr Ser Glu Thr Tyr Asp Ser Val Phe Arg Asn Ser Ser Thr
    130                 135                 140

Ser Cys Ile Pro Ser Glu Glu Asn Lys Ser Gln Asp Met Phe Leu Asp
145                 150                 155                 160

Gly Cys Pro Arg Gln Thr Asp Lys Thr Ile Cys Leu Arg Asp Gln Asn
                165                 170                 175

Val Cys Ser Leu Thr Ser Thr Met Pro Ser Arg Gly His Pro Asn His
            180                 185                 190

Arg Leu Tyr His Lys Leu Cys Ala Ser Leu Ile Arg Trp Met Gly Tyr
        195                 200                 205

Ala Tyr Val Glu Ala Val Asp Ile Glu Ala Asp Glu Ala Cys Ala Asn
    210                 215                 220

Leu Phe His Thr Arg Thr Val Ala Leu Val Tyr Thr Thr Asp Thr Asp

225                 230                 235                 240

Leu Leu Phe Met Gly Cys Asp Ile Leu Leu Asp Ala Ile Pro Met Phe
                245                 250                 255

Ala Pro Val Val Arg Cys Arg Asp Leu Leu Gln Tyr Leu Gly Ile Thr
            260                 265                 270

Tyr Pro Glu Phe Leu Val Ala Phe Val Arg Cys Gln Thr Asp Leu His
        275                 280                 285

Thr Ser Asp Asn Leu Lys Ser Val Gln Gln Val Ile Gln Asp Thr Gly
    290                 295                 300

Leu Lys Val Pro His Gln Met Asp Thr Ser Thr Arg Ser Pro Thr Tyr
305                 310                 315                 320

Asp Ser Trp Arg His Gly Glu Val Phe Lys Ser Leu Thr Val Ala Thr
                325                 330                 335

Ser Gly Lys Thr Glu Asn Gly Val Ser Val Ser Lys Tyr Ala Ser Asn
            340                 345                 350

Arg Ser Glu Val Thr Val Asp Ala Ser Trp Ala Leu Asn Leu Leu Pro
        355                 360                 365

Pro Ser Ser Ser Pro Leu Asp Asn Leu Glu Arg Ala Phe Val Glu His
    370                 375                 380

Ile Ile Ala Val Val Thr Pro Leu Thr Arg Gly Arg Leu Lys Leu Met
385                 390                 395                 400

Lys Arg Val Asn Ile Met Gln Asn Thr Ala Asp Pro Tyr Met Val Ile
                405                 410                 415

Asn Thr Leu Tyr His Asn Leu Lys Gly Glu Lys Met Ala Arg Gln Tyr
            420                 425                 430

Ala Arg Ile Phe Lys Gln Phe Ile Pro Thr Pro Leu Pro Leu Asn Thr
        435                 440                 445

Val Leu Thr Lys Tyr Trp Asn
    450                 455

<210>  18
<211>  1038
<212>  PRT
<213>  Varicella zoster

<400>  18

Met Glu Glu Pro Ile Cys Tyr Asp Thr Gln Lys Leu Leu Asp Asp Leu
1               5                   10                  15

Ser Asn Leu Lys Val Gln Glu Ala Asp Asn Glu Arg Pro Trp Ser Pro
            20                  25                  30

Glu Lys Thr Glu Ile Ala Arg Val Lys Val Val Lys Phe Leu Arg Ser
        35                  40                  45

Thr Gln Lys Ile Pro Ala Lys His Phe Ile Gln Ile Trp Glu Pro Leu
    50                  55                  60

His Ser Asn Ile Cys Phe Val Tyr Ser Asn Thr Phe Leu Ala Glu Ala
65                  70                  75                  80

Ala Phe Thr Ala Glu Asn Leu Pro Gly Leu Leu Phe Trp Arg Leu Asp
                85                  90                  95

Leu Asp Trp Thr Ile Glu Glu Pro Gly Asn Ser Leu Lys Ile Leu Thr
            100                 105                 110

Gln Leu Ser Ser Val Val Gln Asp Ser Glu Thr Leu His Arg Leu Ser
        115                 120                 125

Ala Asn Lys Leu Arg Thr Ser Ser Lys Phe Gly Pro Val Ser Ile His
    130                 135                 140

Phe Ile Ile Thr Asp Trp Ile Asn Met Tyr Glu Val Ala Leu Lys Asp
145                 150                 155                 160

Ala Thr Thr Ala Ile Glu Ser Pro Phe Thr His Ala Arg Ile Gly Met
                165                 170                 175

Leu Glu Ser Ala Ile Ala Ala Leu Thr Gln His Lys Phe Ala Ile Ile
            180                 185                 190

Tyr Asp Met Pro Phe Val Gln Glu Gly Ile Arg Val Leu Thr Gln Tyr
        195                 200                 205

Ala Gly Trp Leu Leu Pro Phe Asn Val Met Trp Asn Gln Ile Gln Asn
    210                 215                 220

Ser Ser Leu Thr Pro Leu Thr Arg Ala Leu Phe Ile Ile Cys Met Ile
225                 230                 235                 240

Asp Glu Tyr Leu Thr Glu Thr Pro Val His Ser Ile Ser Glu Leu Phe
                245                 250                 255

Ala Asp Thr Val Asn Leu Ile Lys Asp Glu Ala Phe Val Ser Ile Glu
            260                 265                 270

Glu Ala Val Thr Asn Pro Arg Thr Val His Glu Ser Arg Ile Ser Ser
        275                 280                 285

Ala Leu Ala Tyr Arg Asp Pro Tyr Val Phe Glu Thr Ser Pro Gly Met
    290                 295                 300

Leu Ala Arg Arg Leu Arg Leu Asp Asn Gly Ile Trp Glu Ser Asn Leu
305                 310                 315                 320

Leu Ser Leu Ser Thr Pro Gly Ile His Ile Glu Ala Leu Leu His Leu
                325                 330                 335

Leu Asn Ser Asp Pro Glu Ala Glu Thr Thr Ser Gly Ser Asn Val Ala
            340                 345                 350

Glu His Thr Arg Gly Ile Trp Glu Lys Val Gln Ala Ser Thr Ser Pro
        355                 360                 365

Ser Met Leu Ile Ser Thr Leu Ala Glu Ser Gly Phe Thr Arg Phe Ser
    370                 375                 380

Cys Lys Leu Leu Arg Arg Phe Ile Ala His His Thr Leu Ala Gly Phe
385                 390                 395                 400

Ile His Gly Ser Val Val Ala Asp Glu His Ile Thr Asp Phe Gln Gln
                405                 410                 415

Thr Leu Gly Cys Leu Ala Leu Val Gly Gly Leu Ala Tyr Gln Leu Val
            420                 425                 430

Glu Thr Tyr Ala Pro Thr Thr Glu Tyr Val Leu Thr Tyr Thr Arg Thr
        435                 440                 445

Val Asn Glu Thr Glu Lys Arg Tyr Glu Thr Leu Leu Pro Ala Leu Gly
    450                 455                 460

Leu Pro Pro Gly Gly Leu Gly Gln Ile Met Arg Arg Cys Phe Ala Pro
465                 470                 475                 480

Arg Pro Leu Ile Glu Ser Ile Gln Ala Thr Arg Val Ile Leu Leu Asn
                485                 490                 495

Glu Ile Ser His Ala Glu Ala Arg Glu Thr Thr Tyr Phe Lys Gln Thr
            500                 505                 510

His Asn Gln Ser Ser Gly Ala Leu Leu Pro Gln Ala Gly Gln Ser Ala
        515                 520                 525

Val Arg Glu Ala Val Leu Thr Trp Phe Asp Leu Arg Met Asp Ser Arg
    530                 535                 540

Trp Gly Ile Thr Pro Pro Val Asp Val Gly Met Thr Pro Pro Ile Cys
545                 550                 555                 560

Val Asp Pro Pro Ala Thr Gly Leu Glu Ala Val Met Ile Thr Glu Ala
                565                 570                 575

Leu Lys Ile Ala Tyr Pro Thr Glu Tyr Asn Arg Ser Ser Val Phe Val
            580                 585                 590

Glu Pro Ser Phe Val Pro Tyr Ile Ile Ala Thr Ser Thr Leu Asp Ala
        595                 600                 605

Leu Ser Ala Thr Ile Ala Leu Ser Phe Asp Thr Arg Gly Ile Gln Gln
    610                 615                 620

Ala Leu Ser Ile Leu Gln Trp Ala Arg Asp Tyr Gly Ser Gly Thr Val
625                 630                 635                 640

Pro Asn Ala Asp Gly Tyr Arg Thr Lys Leu Ser Ala Leu Ile Thr Ile
                645                 650                 655

Leu Glu Pro Phe Thr Arg Thr His Pro Pro Val Leu Leu Pro Ser His
            660                 665                 670

Val Ser Thr Ile Asp Ser Leu Ile Cys Glu Leu His Arg Thr Val Gly
        675                 680                 685

Ile Ala Val Asp Leu Leu Pro Gln His Val Arg Pro Leu Val Pro Asp
    690                 695                 700

Arg Pro Ser Ile Thr Asn Ser Val Phe Leu Ala Thr Leu Tyr Tyr Asp
705                 710                 715                 720

Glu Leu Tyr Gly Arg Trp Thr Arg Leu Asp Lys Thr Ser Gln Ala Leu
                725                 730                 735

Val Glu Asn Phe Thr Ser Asn Ala Leu Val Val Ser Arg Tyr Met Leu
            740                 745                 750

Met Leu Gln Lys Phe Phe Ala Cys Arg Phe Tyr Pro Thr Pro Asp Leu
        755                 760                 765

Gln Ala Val Gly Ile Cys Asn Pro Lys Val Glu Arg Asp Glu Gln Phe
    770                 775                 780

Gly Val Trp Arg Leu Asn Asp Leu Ala Asp Ala Val Gly His Ile Val
785                 790                 795                 800

Gly Thr Ile Gln Gly Ile Arg Thr Gln Met Arg Val Gly Ile Ser Ser
                805                 810                 815

Leu Arg Thr Ile Met Ala Asp Ala Ser Ser Ala Leu Arg Glu Cys Glu
            820                 825                 830

Asn Leu Met Thr Lys Thr Ser Thr Ser Ala Ile Gly Pro Leu Phe Ser
        835                 840                 845

Thr Met Ala Ser Arg Tyr Ala Arg Phe Thr Gln Asp Gln Met Asp Ile
    850                 855                 860

Leu Met Arg Val Asp Lys Leu Thr Thr Gly Glu Asn Ile Pro Gly Leu
865                 870                 875                 880

Ala Asn Val Glu Ile Phe Leu Asn Arg Trp Glu Arg Ile Ala Thr Ala
                885                 890                 895

Cys Arg His Ala Thr Ala Val Pro Ser Ala Glu Ser Ile Ala Thr Val
            900                 905                 910

Cys Asn Glu Leu Arg Arg Gly Leu Lys Asn Ile Gln Glu Asp Arg Val
        915                 920                 925

Asn Ala Pro Thr Ser Tyr Met Ser His Ala Arg Asn Leu Glu Asp His
    930                 935                 940

Lys Ala Ala Val Ser Phe Val Met Asp Ser Arg Gln Gln Phe Ile Val
945                 950                 955                 960

Asp Ser Gly Pro Gln Met Gly Ala Val Leu Thr Ser Gln Cys Asn Ile
                965                 970                 975

Gly Thr Trp Glu Asn Val Asn Ala Thr Phe Leu His Asp Asn Val Lys
            980                 985                 990

Ile Thr Thr Thr Val Arg Asp Val  Ile Ser Glu Ala Pro  Thr Leu Ile
        995                 1000                 1005

Ile Gly  Gln Arg Trp Leu Arg  Pro Asp Glu Ile Leu  Ser Asn Val
    1010                 1015                 1020

Asp Leu  Arg Leu Gly Val Pro  Gly Asn Thr Ser Gly  Ser Asp Pro
    1025                 1030                 1035

<210>  19
<211>  2763
<212>  PRT
<213>  Varicella zoster

<400>  19

Met Asp Ile Ile Pro Pro Ile Ala Val Thr Val Ala Gly Val Gly Ser
1               5                   10                  15

Arg Asn Gln Phe Asp Gly Ala Leu Gly Pro Ala Ser Gly Leu Ser Cys
            20                  25                  30

Leu Arg Thr Ser Leu Ser Phe Leu His Met Thr Tyr Ala His Gly Ile
        35                  40                  45

Asn Ala Thr Leu Ser Ser Asp Met Ile Asp Gly Cys Leu Gln Glu Gly
    50                  55                  60

Ala Ala Trp Thr Thr Asp Leu Ser Asn Met Gly Arg Gly Val Pro Asp
65                  70                  75                  80

Met Cys Ala Leu Val Asp Leu Pro Asn Arg Ile Ser Tyr Ile Lys Leu
                85                  90                  95

Gly Asp Thr Thr Ser Thr Cys Cys Val Leu Ser Arg Ile Tyr Gly Asp
            100                 105                 110

Ser His Phe Phe Thr Val Pro Asp Glu Gly Phe Met Cys Thr Gln Ile
        115                 120                 125

Pro Ala Arg Ala Phe Phe Asp Asp Val Trp Met Gly Arg Glu Glu Ser
    130                 135                 140

Tyr Thr Ile Ile Thr Val Asp Ser Thr Gly Met Ala Ile Tyr Arg Gln
145                 150                 155                 160

Gly Asn Ile Ser Phe Ile Phe Asp Pro His Gly His Gly Thr Ile Gly
                165                 170                 175

Gln Ala Val Val Val Arg Val Asn Thr Thr Asp Val Tyr Ser Tyr Ile
            180                 185                 190

Ala Ser Glu Tyr Thr His Arg Pro Asp Asn Val Glu Ser Gln Trp Ala
        195                 200                 205

Ala Ala Leu Val Phe Phe Val Thr Ala Asn Asp Gly Pro Val Ser Glu
    210                 215                 220

Glu Ala Leu Ser Ser Ala Val Thr Leu Ile Tyr Gly Ser Cys Asp Thr
225                 230                 235                 240

Tyr Phe Thr Asp Glu Gln Tyr Cys Glu Lys Leu Val Thr Ala Gln His
                245                 250                 255

Pro Leu Leu Leu Ser Pro Pro Asn Ser Thr Thr Ile Val Leu Asn Lys
            260                 265                 270

Ser Ser Ile Val Pro Leu His Gln Asn Val Gly Glu Ser Val Ser Leu
        275                 280                 285

Glu Ala Thr Leu His Ser Thr Leu Thr Asn Thr Val Ala Leu Asp Pro
    290                 295                 300

Arg Cys Ser Tyr Ser Glu Val Asp Pro Trp His Ala Val Leu Glu Thr
305                 310                 315                 320

Thr Ser Thr Gly Ser Gly Val Leu Asp Cys Arg Arg Arg Arg Arg Pro
                325                 330                 335

Ser Trp Thr Pro Pro Ser Ser Glu Glu Asn Leu Ala Cys Ile Asp Asp
            340                 345                 350

Gly Leu Val Asn Asn Thr His Ser Thr Asp Asn Leu His Lys Pro Ala
        355                 360                 365

Lys Lys Val Leu Lys Phe Lys Pro Thr Val Asp Val Pro Asp Lys Thr
    370                 375                 380

Gln Val Ala His Val Leu Pro Arg Leu Arg Glu Val Ala Asn Thr Pro
385                 390                 395                 400

Asp Val Val Leu Asn Val Ser Asn Val Asp Thr Pro Glu Ser Ser Pro
                405                 410                 415

Thr Phe Ser Arg Asn Met Asn Val Gly Ser Ser Leu Lys Asp Arg Lys
            420                 425                 430

Pro Phe Leu Phe Glu Gln Ser Gly Asp Val Asn Met Val Val Glu Lys
        435                 440                 445

Leu Leu Gln His Gly His Glu Ile Ser Asn Gly Tyr Val Gln Asn Ala
    450                 455                 460

Val Gly Thr Leu Asp Thr Val Ile Thr Gly His Thr Asn Val Pro Ile
465                 470                 475                 480

Trp Val Thr Arg Pro Leu Val Met Pro Asp Glu Lys Asp Pro Leu Glu
                485                 490                 495

Leu Phe Ile Asn Leu Thr Ile Leu Arg Leu Thr Gly Phe Val Val Glu
            500                 505                 510

Asn Gly Thr Arg Thr His His Gly Ala Thr Ser Val Val Ser Asp Phe
        515                 520                 525

Ile Gly Pro Leu Gly Glu Ile Leu Thr Gly Phe Pro Ser Ala Ala Glu
    530                 535                 540

Leu Ile Arg Val Thr Ser Leu Ile Leu Thr Asn Met Pro Gly Ala Glu
545                 550                 555                 560

Tyr Ala Ile Lys Thr Val Leu Arg Lys Lys Cys Thr Ile Gly Met Leu
                565                 570                 575

Ile Ile Ala Lys Phe Gly Leu Val Ala Met Arg Val Gln Asp Thr Thr
            580                 585                 590

Gly Ala Leu His Ala Glu Leu Asp Val Leu Glu Ala Asp Leu Gly Gly
        595                 600                 605

Ser Ser Pro Ile Asp Leu Tyr Ser Arg Leu Ser Thr Gly Leu Ile Ser
    610                 615                 620

Ile Leu Asn Ser Pro Ile Ile Ser His Pro Gly Leu Phe Ala Glu Leu
625                 630                 635                 640

Ile Pro Thr Arg Thr Gly Ser Leu Ser Glu Arg Ile Arg Leu Leu Cys
                645                 650                 655

Glu Leu Val Ser Ala Arg Glu Thr Arg Tyr Met Arg Glu His Thr Ala
            660                 665                 670

Leu Val Ser Ser Val Lys Ala Leu Glu Asn Ala Leu Arg Ser Thr Arg
        675                 680                 685

Asn Lys Ile Asp Ala Ile Gln Ile Pro Glu Val Pro Gln Glu Pro Pro
    690                 695                 700

Glu Glu Thr Asp Ile Pro Pro Glu Glu Leu Ile Arg Arg Val Tyr Glu
705                 710                 715                 720

Ile Arg Ser Glu Val Thr Met Leu Leu Thr Ser Ala Val Thr Glu Tyr
                725                 730                 735

Phe Thr Arg Gly Val Leu Tyr Ser Thr Arg Ala Leu Ile Ala Glu Gln
            740                 745                 750

Ser Pro Arg Arg Phe Arg Val Ala Thr Ala Ser Thr Ala Pro Ile Gln
        755                 760                 765

Arg Leu Leu Asp Ser Leu Pro Glu Phe Asp Ala Lys Leu Thr Ala Ile
    770                 775                 780

Ile Ser Ser Leu Ser Ile His Pro Pro Pro Glu Thr Ile Gln Asn Leu
785                 790                 795                 800

Pro Val Val Ser Leu Leu Lys Glu Leu Ile Lys Glu Gly Glu Asp Leu
                805                 810                 815

Asn Thr Asp Thr Ala Leu Val Ser Trp Leu Ser Val Val Gly Glu Ala
            820                 825                 830

Gln Thr Ala Gly Tyr Leu Ser Arg Arg Glu Phe Asp Glu Leu Ser Arg
        835                 840                 845

Thr Ile Lys Thr Ile Asn Thr Arg Ala Thr Gln Arg Ala Ser Ala Glu
    850                 855                 860

Ala Glu Leu Ser Cys Phe Asn Thr Leu Ser Ala Ala Val Asp Gln Ala
865                 870                 875                 880

Val Lys Asp Tyr Glu Thr Tyr Asn Asn Gly Glu Val Lys Tyr Pro Glu
                885                 890                 895

Ile Thr Arg Asp Asp Leu Leu Ala Thr Ile Val Arg Ala Thr Asp Asp
            900                 905                 910

Leu Val Arg Gln Ile Lys Ile Leu Ser Asp Pro Met Ile Gln Ser Gly
        915                 920                 925

Leu Gln Pro Ser Ile Lys Arg Arg Leu Glu Thr Arg Leu Lys Glu Val
    930                 935                 940

Gln Thr Tyr Ala Asn Glu Ala Arg Thr Thr Gln Asp Thr Ile Lys Ser
945                 950                 955                 960

Arg Lys Gln Ala Ala Tyr Asn Lys Leu Gly Gly Leu Leu Arg Pro Val
                965                 970                 975

Thr Gly Phe Val Gly Leu Arg Ala Ala Val Asp Leu Leu Pro Glu Leu
            980                 985                 990

Ala Ser Glu Leu Asp Val Gln Gly  Ala Leu Val Asn Leu  Arg Thr Lys
        995                 1000                1005

Val Leu  Glu Ala Pro Val Glu  Ile Arg Ser Gln Leu  Thr Gly Asp
    1010                1015                1020

Phe Trp  Ala Leu Phe Asn Gln  Tyr Arg Asp Ile Leu  Glu His Pro
    1025                1030                1035

Gly Asn  Ala Arg Thr Ser Val  Leu Gly Gly Leu Gly  Ala Cys Phe
    1040                1045                1050

Thr Ala  Ile Ile Glu Ile Val  Pro Ile Pro Thr Glu  Tyr Arg Pro
    1055                1060                1065

Ser Leu  Leu Ala Phe Phe Gly  Asp Val Ala Asp Val  Leu Ala Ser
    1070                1075                1080

Asp Ile  Ala Thr Val Ser Thr  Asn Pro Glu Ser Glu  Ser Ala Ile
    1085                1090                1095

Asn Ala  Val Val Ala Thr Leu  Ser Lys Ala Thr Leu  Val Ser Ser
    1100                1105                1110

Thr Val Pro Ala Leu Ser Phe Val Leu Ser Leu Tyr Lys Lys Tyr
    1115                1120                1125

Gln Ala Leu Gln Gln Glu Ile Thr Asn Thr His Lys Leu Thr Glu
    1130                1135                1140

Leu Gln Lys Gln Leu Gly Asp Asp Phe Ser Thr Leu Ala Val Ser
    1145                1150                1155

Ser Gly His Leu Lys Phe Ile Ser Ser Ser Asn Val Asp Asp Tyr
    1160                1165                1170

Glu Ile Asn Asp Ala Ile Leu Ser Ile Gln Thr Asn Val His Ala
    1175                1180                1185

Leu Met Asp Thr Val Lys Leu Val Glu Val Glu Leu Gln Lys Leu
    1190                1195                1200

Pro Pro His Cys Ile Ala Gly Thr Ser Thr Leu Ser Arg Val Val
    1205                1210                1215

Lys Asp Leu His Lys Leu Val Thr Met Ala His Glu Lys Lys Glu
    1220                1225                1230

Gln Ala Lys Val Leu Ile Thr Asp Cys Glu Arg Ala His Lys Gln
    1235                1240                1245

Gln Thr Thr Arg Val Leu Tyr Glu Arg Trp Thr Arg Asp Ile Ile
    1250                1255                1260

Ala Cys Leu Glu Ala Met Glu Thr Arg His Ile Phe Asn Gly Thr
    1265                1270                1275

Glu Leu Ala Arg Leu Arg Asp Met Ala Ala Ala Gly Gly Phe Asp
    1280                1285                1290

Ile His Ala Val Tyr Pro Gln Ala Arg Gln Val Val Ala Ala Cys
    1295                1300                1305

Glu Thr Thr Ala Val Thr Ala Leu Asp Thr Val Phe Arg His Asn
    1310                1315                1320

Pro Tyr Thr Pro Glu Asn Thr Asn Ile Pro Pro Pro Leu Ala Leu
    1325                1330                1335

Leu Arg Gly Leu Thr Trp Phe Asp Asp Phe Ser Ile Thr Ala Pro
    1340                1345                1350

Val Phe Thr Val Met Phe Pro Gly Val Ser Ile Glu Gly Leu Leu
    1355                1360                1365

Leu Leu Met Arg Ile Arg Ala Val Val Leu Leu Ser Ala Asp Thr
    1370                1375                1380

Ser Ile Asn Gly Ile Pro Asn Tyr Arg Asp Met Ile Leu Arg Thr
    1385                1390                1395

Ser Gly Asp Leu Leu Gln Ile Pro Ala Leu Ala Gly Tyr Val Asp
    1400                1405                1410

Phe Tyr Thr Arg Ser Tyr Asp Gln Phe Ile Thr Glu Ser Val Thr
    1415                1420                1425

Leu Ser Glu Leu Arg Ala Asp Ile Arg Gln Ala Ala Gly Ala Lys
    1430                1435                1440

Leu Thr Glu Ala Asn Lys Ala Leu Glu Glu Val Thr His Val Arg
    1445                1450                1455

Ala His Glu Thr Ala Lys Leu Ala Leu Lys Glu Gly Val Phe Ile
    1460                1465                1470

Thr Leu Pro Ser Glu Gly Leu Leu Ile Arg Ala Ile Glu Tyr Phe
    1475                1480                1485

Thr Thr Phe Asp His Lys Arg Phe Ile Gly Thr Ala Tyr Glu Arg
    1490                1495                1500

Val Leu Gln Thr Met Val Asp Arg Asp Leu Lys Glu Ala Asn Ala
    1505                1510                1515

Glu Leu Ala Gln Phe Arg Met Val Cys Gln Ala Thr Lys Asn Arg
    1520                1525                1530

Ala Ile Gln Ile Leu Gln Asn Ile Val Asp Thr Ala Asn Ala Thr
    1535                1540                1545

Glu Gln Gln Glu Asp Val Asp Phe Thr Asn Leu Lys Thr Leu Leu
    1550                1555                1560

Lys Leu Thr Pro Pro Pro Lys Thr Ile Ala Leu Ala Ile Asp Arg
    1565                1570                1575

Ser Thr Ser Val Gln Asp Ile Val Thr Gln Phe Ala Leu Leu Leu
    1580                1585                1590

Gly Arg Leu Glu Glu Glu Thr Gly Thr Leu Asp Ile Gln Ala Val
    1595                1600                1605

Asp Trp Met Tyr Gln Ala Arg Asn Ile Ile Asp Ser His Pro Leu
    1610                1615                1620

Ser Val Arg Ile Asp Gly Thr Gly Pro Leu His Thr Tyr Lys Asp
    1625                1630                1635

Arg Val Asp Lys Leu Tyr Ala Leu Arg Thr Lys Leu Asp Leu Leu
    1640                1645                1650

Arg Arg Arg Ile Glu Thr Gly Glu Val Thr Trp Asp Asp Ala Trp
    1655                1660                1665

Thr Thr Phe Lys Arg Glu Thr Gly Asp Met Leu Ala Ser Gly Asp
    1670                1675                1680

Thr Tyr Ala Thr Ser Val Asp Ser Ile Lys Ala Leu Gln Ala Ser
    1685                1690                1695

Ala Ser Val Val Asp Met Leu Cys Ser Glu Pro Glu Phe Phe Leu
    1700                1705                1710

Leu Pro Val Glu Thr Lys Asn Arg Leu Gln Lys Lys Gln Gln Glu
    1715                1720                1725

Arg Lys Thr Ala Leu Asp Val Val Leu Gln Lys Gln Arg Gln Phe
    1730                1735                1740

Glu Glu Thr Ala Ser Arg Leu Arg Ala Leu Ile Glu Arg Ile Pro
    1745                1750                1755

Thr Glu Ser Asp His Asp Val Leu Arg Met Leu Leu Arg Asp Phe
    1760                1765                1770

Asp Gln Phe Thr His Leu Pro Ile Trp Ile Lys Thr Gln Tyr Met
    1775                1780                1785

Thr Phe Arg Asn Leu Leu Met Val Arg Leu Gly Leu Tyr Ala Ser
    1790                1795                1800

Tyr Ala Glu Ile Phe Pro Pro Ala Ser Pro Asn Gly Val Phe Ala
    1805                1810                1815

Pro Ile Pro Ala Met Ser Gly Val Cys Leu Glu Asp Gln Ser Arg
    1820                1825                1830

Cys Ile Arg Ala Arg Val Ala Ala Phe Met Gly Glu Ala Ser Val
    1835                1840                1845

Val Gln Thr Phe Arg Glu Ala Arg Ser Ser Ile Asp Ala Leu Phe
    1850                1855                1860

Gly Lys Asn Leu Thr Phe Tyr Leu Asp Thr Asp Gly Val Pro Leu
    1865                1870                1875

Arg Tyr Arg Val Cys Tyr Lys Ser Val Gly Val Lys Leu Gly Thr
    1880                1885                1890

Met Leu Cys Ser Gln Gly Gly Leu Ser Leu Arg Pro Ala Leu Pro
    1895                1900                1905

Asp Glu Gly Ile Val Glu Glu Thr Thr Leu Ser Ala Leu Arg Val
    1910                1915                1920

Ala Asn Glu Val Asn Glu Leu Arg Ile Glu Tyr Glu Ser Ala Ile
    1925                1930                1935

Lys Ser Gly Phe Ser Ala Phe Ser Thr Phe Val Arg His Arg His
    1940                1945                1950

Ala Glu Trp Gly Lys Thr Asn Ala Arg Arg Ala Ile Ala Glu Ile
    1955                1960                1965

Tyr Ala Gly Leu Ile Thr Thr Thr Leu Thr Arg Gln Tyr Gly Val
    1970                1975                1980

His Trp Asp Lys Leu Ile Tyr Ser Phe Glu Lys His His Leu Thr
    1985                1990                1995

Ser Val Met Gly Asn Gly Leu Thr Lys Pro Ile Gln Arg Arg Gly
    2000                2005                2010

Asp Val Arg Val Leu Glu Leu Thr Leu Ser Asp Ile Val Thr Ile
    2015                2020                2025

Leu Val Ala Thr Thr Pro Val His Leu Leu Asn Phe Ala Arg Leu
    2030                2035                2040

Asp Leu Ile Lys Gln His Glu Tyr Met Ala Arg Thr Leu Arg Pro
    2045                2050                2055

Val Ile Glu Ala Ala Phe Arg Gly Arg Leu Leu Val Arg Ser Leu
    2060                2065                2070

Asp Gly Asp Pro Lys Gly Asn Ala Arg Ala Phe Phe Asn Ala Ala
    2075                2080                2085

Pro Ser Lys His Lys Leu Pro Leu Ala Leu Gly Ser Asn Gln Asp
    2090                2095                2100

Pro Thr Gly Gly Arg Ile Phe Ala Phe Arg Met Ala Asp Trp Lys
    2105                2110                2115

Leu Val Lys Met Pro Gln Lys Ile Thr Asp Pro Phe Ala Pro Trp
    2120                2125                2130

Gln Leu Ser Pro Pro Pro Gly Val Lys Ala Asn Val Asp Ala Val
    2135                2140                2145

Thr Arg Ile Met Ala Thr Asp Arg Leu Ala Thr Ile Thr Val Leu
    2150                2155                2160

Gly Arg Met Cys Leu Pro Pro Ile Ser Leu Val Ser Met Trp Asn
    2165                2170                2175

Thr Leu Gln Pro Glu Glu Phe Ala Tyr Arg Thr Gln Asp Asp Val
    2180                2185                2190

Asp Ile Ile Val Asp Ala Arg Leu Asp Leu Ser Ser Thr Leu Asn
    2195                2200                2205

Ala Arg Phe Asp Thr Ala Pro Ser Asn Thr Thr Leu Glu Trp Asn
    2210                2215                2220

Thr Asp Arg Lys Val Ile Thr Asp Ala Tyr Ile Gln Thr Gly Ala
    2225                2230                2235

Thr Thr Val Phe Thr Val Thr Gly Ala Ala Pro Thr His Val Ser
    2240                2245                2250

Asn Val Thr Ala Phe Asp Ile Ala Thr Thr Ala Ile Leu Phe Gly
    2255                2260                2265

Ala Pro Leu Val Ile Ala Met Glu Leu Thr Ser Val Phe Ser Gln
    2270                2275                2280

Asn Ser Gly Leu Thr Leu Gly Leu Lys Leu Phe Asp Ser Arg His
    2285                2290                2295

Met Ala Thr Asp Ser Gly Ile Ser Ser Ala Val Ser Pro Asp Ile
    2300                2305                2310

Val Ser Trp Gly Leu Arg Leu Leu His Met Asp Pro His Pro Ile
    2315                2320                2325

Glu Asn Ala Cys Leu Ile Val Gln Leu Glu Lys Leu Ser Ala Leu
    2330                2335                2340

Ile Ala Asn Lys Pro Leu Thr Asn Asn Pro Pro Cys Leu Leu Leu
    2345                2350                2355

Leu Asp Glu His Met Asn Pro Ser Tyr Val Leu Trp Glu Arg Lys
    2360                2365                2370

Asp Ser Ile Pro Ala Pro Asp Tyr Val Val Phe Trp Gly Pro Glu
    2375                2380                2385

Ser Leu Ile Asp Leu Pro Tyr Ile Asp Ser Asp Glu Asp Ser Phe
    2390                2395                2400

Pro Ser Cys Pro Asp Asp Pro Phe Tyr Ser Gln Ile Ile Ala Gly
    2405                2410                2415

Tyr Ala Pro Gln Gly Pro Pro Asn Leu Asp Thr Thr Asp Phe Tyr
    2420                2425                2430

Pro Thr Glu Pro Leu Phe Lys Ser Pro Val Gln Val Val Arg Ser
    2435                2440                2445

Ser Lys Cys Lys Lys Met Pro Val Arg Pro Ala Gln Pro Ala Gln
    2450                2455                2460

Pro Ala Gln Pro Ala Gln Pro Ala Gln Thr Val Gln Pro Ala Gln
    2465                2470                2475

Pro Ile Glu Pro Gly Thr Gln Ile Val Val Gln Asn Phe Lys Lys
    2480                2485                2490

Pro Gln Ser Val Lys Thr Thr Leu Ser Gln Lys Asp Ile Pro Leu
    2495                2500                2505

Tyr Val Glu Thr Glu Ser Glu Thr Ala Val Leu Ile Pro Lys Gln
    2510                2515                2520

Leu Thr Thr Ser Ile Lys Thr Thr Val Cys Lys Ser Ile Thr Pro
    2525                2530                2535

Pro Asn Asn Gln Leu Ser Asp Trp Lys Asn Asn Pro Gln Gln Asn
    2540                2545                2550

Gln Thr Leu Asn Gln Ala Phe Ser Lys Pro Ile Leu Glu Ile Thr
    2555                2560                2565

Ser Ile Pro Thr Asp Asp Ser Ile Ser Tyr Arg Thr Trp Ile Glu
    2570                2575                2580

Lys Ser Asn Gln Thr Gln Lys Arg His Gln Asn Asp Pro Arg Met
    2585                2590                2595

Tyr Asn Ser Lys Thr Val Phe His Pro Val Asn Asn Gln Leu Pro
    2600                2605                2610

Ser Trp Val Asp Thr Ala Ala Asp Ala Pro Gln Thr Asp Leu Leu
    2615                2620                2625

Thr Asn Tyr Lys Thr Arg Gln Pro Ser Pro Asn Phe Pro Arg Asp
    2630                2635                2640

Val His Thr Trp Gly Val Ser Ser Asn Pro Phe Asn Ser Pro Asn
    2645                2650                2655

Arg Asp Leu Tyr Gln Ser Asp Phe Ser Glu Pro Ser Asp Gly Tyr
    2660                2665                2670

Ser Ser Glu Ser Glu Asn Ser Ile Val Leu Ser Leu Asp Glu His
    2675                2680                2685

Arg Ser Cys Arg Val Pro Arg His Val Arg Val Val Asn Ala Asp
    2690                2695                2700

Val Val Thr Gly Arg Arg Tyr Val Arg Gly Thr Ala Leu Gly Ala
    2705                2710                2715

Leu Ala Leu Leu Ser Gln Ala Cys Arg Arg Met Ile Asp Asn Val
    2720                2725                2730

Arg Tyr Thr Arg Lys Leu Leu Met Asp His Thr Glu Asp Ile Phe
    2735                2740                2745

Gln Gly Leu Gly Tyr Val Lys Leu Leu Leu Asp Gly Thr Tyr Ile
    2750                2755                2760

<210>  20
<211>  585
<212>  PRT
<213>  Varicella zoster

<400>  20

Met Asp Arg Val Glu Ser Glu Glu Pro Met Asp Gly Phe Glu Ser Pro
1               5                   10                  15

Val Phe Ser Glu Asn Thr Ser Ser Asn Ser Gly Trp Cys Ser Asp Ala
            20                  25                  30

Phe Ser Asp Ser Tyr Ile Ala Tyr Asn Pro Ala Leu Leu Leu Lys Asn
        35                  40                  45

Asp Leu Leu Phe Ser Glu Leu Leu Phe Ala Ser His Leu Ile Asn Val
    50                  55                  60

Pro Arg Ala Ile Glu Asn Asn Val Thr Tyr Glu Ala Ser Ser Ala Val
65                  70                  75                  80

Gly Val Asp Asn Glu Met Thr Ser Ser Thr Thr Glu Phe Ile Glu Glu
                85                  90                  95

Ile Gly Asp Val Leu Ala Leu Asp Arg Ala Cys Leu Val Cys Arg Thr
            100                 105                 110

Leu Asp Leu Tyr Lys Arg Lys Phe Gly Leu Thr Pro Glu Trp Val Ala
        115                 120                 125

Asp Tyr Ala Met Leu Cys Met Lys Ser Leu Ala Ser Pro Pro Cys Ala
    130                 135                 140

Val Val Thr Phe Ser Ala Ala Phe Glu Phe Val Tyr Leu Met Asp Arg
145                 150                 155                 160

Tyr Tyr Leu Cys Arg Tyr Asn Val Thr Leu Val Gly Ser Phe Ala Arg
                165                 170                 175

Arg Thr Leu Ser Leu Leu Asp Ile Gln Arg His Phe Phe Leu His Val
            180                 185                 190

Cys Phe Arg Thr Asp Gly Gly Leu Pro Gly Ile Arg Pro Pro Pro Gly
        195                 200                 205

Lys Glu Met Ala Asn Lys Val Arg Tyr Ser Asn Tyr Ser Phe Phe Val
    210                 215                 220

Gln Ala Val Val Arg Ala Ala Leu Leu Ser Ile Ser Thr Ser Arg Leu
225                 230                 235                 240

Asp Glu Thr Glu Thr Arg Lys Ser Phe Tyr Phe Asn Gln Asp Gly Leu
                245                 250                 255

Thr Gly Gly Pro Gln Pro Leu Ala Ala Ala Leu Ala Asn Trp Lys Asp
            260                 265                 270

Cys Ala Arg Met Val Asp Cys Ser Ser Ser Glu His Arg Thr Ser Gly
        275                 280                 285

Met Ile Thr Cys Ala Glu Arg Ala Leu Lys Glu Asp Ile Glu Phe Glu
    290                 295                 300

Asp Ile Leu Ile Asp Lys Leu Lys Lys Ser Ser Tyr Val Glu Ala Ala
305                 310                 315                 320

Trp Gly Tyr Ala Asp Leu Ala Leu Leu Leu Leu Ser Gly Val Ala Thr
                325                 330                 335

Trp Asn Val Asp Glu Arg Thr Asn Cys Ala Ile Glu Thr Arg Val Gly
            340                 345                 350

Cys Val Lys Ser Tyr Trp Gln Ala Asn Arg Ile Glu Asn Ser Arg Asp
        355                 360                 365

Val Pro Lys Gln Phe Ser Lys Phe Thr Ser Glu Asp Ala Cys Pro Glu
    370                 375                 380

Val Ala Phe Gly Pro Ile Leu Leu Thr Thr Leu Lys Asn Ala Lys Cys
385                 390                 395                 400

Arg Gly Arg Thr Asn Thr Glu Cys Met Leu Cys Cys Leu Leu Thr Ile
                405                 410                 415

Gly His Tyr Trp Ile Ala Leu Arg Gln Phe Lys Arg Asp Ile Leu Ala
            420                 425                 430

Tyr Ser Ala Asn Asn Thr Ser Leu Phe Asp Cys Ile Glu Pro Val Ile
        435                 440                 445

Asn Ala Trp Ser Leu Asp Asn Pro Ile Lys Leu Lys Phe Pro Phe Asn
    450                 455                 460

Asp Glu Gly Arg Phe Ile Thr Ile Val Lys Ala Ala Gly Ser Glu Ala
465                 470                 475                 480

Val Tyr Lys His Leu Phe Cys Asp Leu Leu Cys Ala Leu Ser Glu Leu
                485                 490                 495

Gln Thr Asn Pro Lys Ile Leu Phe Ala His Pro Thr Thr Ala Asp Lys
            500                 505                 510

Glu Val Leu Glu Leu Tyr Lys Ala Gln Leu Ala Ala Gln Asn Arg Phe
        515                 520                 525

Glu Gly Arg Val Cys Ala Gly Leu Trp Thr Leu Ala Tyr Ala Phe Lys
    530                 535                 540

Ala Tyr Gln Ile Phe Pro Arg Lys Pro Thr Ala Asn Ala Ala Phe Ile
545                 550                 555                 560

Arg Asp Gly Gly Leu Met Leu Arg Arg His Ala Ile Ser Leu Val Ser
                565                 570                 575

Leu Glu His Thr Leu Ser Lys Tyr Val
            580                 585

<210>  21
<211>  1204
<212>  PRT
<213>  Varicella zoster

<400>  21

Met Glu Asn Thr Gln Lys Thr Val Thr Val Pro Thr Gly Pro Leu Gly
1               5                   10                  15

Tyr Val Tyr Ala Cys Arg Val Glu Asp Leu Asp Leu Glu Glu Ile Ser
            20                  25                  30

Phe Leu Ala Ala Arg Ser Thr Asp Ser Asp Leu Ala Leu Leu Pro Leu

35                  40                  45

Met Arg Asn Leu Thr Val Glu Lys Thr Phe Thr Ser Ser Leu Ala Val
    50                  55                  60

Val Ser Gly Ala Arg Thr Thr Gly Leu Ala Gly Ala Gly Ile Thr Leu
65                  70                  75                  80

Lys Leu Thr Thr Ser His Phe Tyr Pro Ser Val Phe Val Phe His Gly
                85                  90                  95

Gly Lys His Val Leu Pro Ser Ser Ala Ala Pro Asn Leu Thr Arg Ala
            100                 105                 110

Cys Asn Ala Ala Arg Glu Arg Phe Gly Phe Ser Arg Cys Gln Gly Pro
        115                 120                 125

Pro Val Asp Gly Ala Val Glu Thr Thr Gly Ala Glu Ile Cys Thr Arg
    130                 135                 140

Leu Gly Leu Glu Pro Glu Asn Thr Ile Leu Tyr Leu Val Val Thr Ala
145                 150                 155                 160

Leu Phe Lys Glu Ala Val Phe Met Cys Asn Val Phe Leu His Tyr Gly
                165                 170                 175

Gly Leu Asp Ile Val His Ile Asn His Gly Asp Val Ile Arg Ile Pro
            180                 185                 190

Leu Phe Pro Val Gln Leu Phe Met Pro Asp Val Asn Arg Leu Val Pro
        195                 200                 205

Asp Pro Phe Asn Thr His His Arg Ser Ile Gly Glu Gly Phe Val Tyr
    210                 215                 220

Pro Thr Pro Phe Tyr Asn Thr Gly Leu Cys His Leu Ile His Asp Cys
225                 230                 235                 240

Val Ile Ala Pro Met Ala Val Ala Leu Arg Val Arg Asn Val Thr Ala
                245                 250                 255

Val Ala Arg Gly Ala Ala His Leu Ala Phe Asp Glu Asn His Glu Gly
            260                 265                 270

Ala Val Leu Pro Pro Asp Ile Thr Tyr Thr Tyr Phe Gln Ser Ser Ser
        275                 280                 285

Ser Gly Thr Thr Thr Ala Arg Gly Ala Arg Arg Asn Asp Val Asn Ser
    290                 295                 300

Thr Ser Lys Pro Ser Pro Ser Gly Gly Phe Glu Arg Arg Leu Ala Ser

305                 310                 315                 320

Ile Met Ala Ala Asp Thr Ala Leu His Ala Glu Val Ile Phe Asn Thr
                325                 330                 335

Gly Ile Tyr Glu Glu Thr Pro Thr Asp Ile Lys Glu Trp Pro Met Phe
            340                 345                 350

Ile Gly Met Glu Gly Thr Leu Pro Arg Leu Asn Ala Leu Gly Ser Tyr
        355                 360                 365

Thr Ala Arg Val Ala Gly Val Ile Gly Ala Met Val Phe Ser Pro Asn
    370                 375                 380

Ser Ala Leu Tyr Leu Thr Glu Val Glu Asp Ser Gly Met Thr Glu Ala
385                 390                 395                 400

Lys Asp Gly Gly Pro Gly Pro Ser Phe Asn Arg Phe Tyr Gln Phe Ala
                405                 410                 415

Gly Pro His Leu Ala Ala Asn Pro Gln Thr Asp Arg Asp Gly His Val
            420                 425                 430

Leu Ser Ser Gln Ser Thr Gly Ser Ser Asn Thr Glu Phe Ser Val Asp
        435                 440                 445

Tyr Leu Ala Leu Ile Cys Gly Phe Gly Ala Pro Leu Leu Ala Arg Leu
    450                 455                 460

Leu Phe Tyr Leu Glu Arg Cys Asp Ala Gly Ala Phe Thr Gly Gly His
465                 470                 475                 480

Gly Asp Ala Leu Lys Tyr Val Thr Gly Thr Phe Asp Ser Glu Ile Pro
                485                 490                 495

Cys Ser Leu Cys Glu Lys His Thr Arg Pro Val Cys Ala His Thr Thr
            500                 505                 510

Val His Arg Leu Arg Gln Arg Met Pro Arg Phe Gly Gln Ala Thr Arg
        515                 520                 525

Gln Pro Ile Gly Val Phe Gly Thr Met Asn Ser Gln Tyr Ser Asp Cys
    530                 535                 540

Asp Pro Leu Gly Asn Tyr Ala Pro Tyr Leu Ile Leu Arg Lys Pro Gly
545                 550                 555                 560

Asp Gln Thr Glu Ala Ala Lys Ala Thr Met Gln Asp Thr Tyr Arg Ala
                565                 570                 575

Thr Leu Glu Arg Leu Phe Ile Asp Leu Glu Gln Glu Arg Leu Leu Asp

580                 585                 590

Arg Gly Ala Pro Cys Ser Ser Glu Gly Leu Ser Ser Val Ile Val Asp
        595                 600                 605

His Pro Thr Phe Arg Arg Ile Leu Asp Thr Leu Arg Ala Arg Ile Glu
    610                 615                 620

Gln Thr Thr Thr Gln Phe Met Lys Val Leu Val Glu Thr Arg Asp Tyr
625                 630                 635                 640

Lys Ile Arg Glu Gly Leu Ser Glu Ala Thr His Ser Met Ala Leu Thr
                645                 650                 655

Phe Asp Pro Tyr Ser Gly Ala Phe Cys Pro Ile Thr Asn Phe Leu Val
            660                 665                 670

Lys Arg Thr His Leu Ala Val Val Gln Asp Leu Ala Leu Ser Gln Cys
        675                 680                 685

His Cys Val Phe Tyr Gly Gln Gln Val Glu Gly Arg Asn Phe Arg Asn
    690                 695                 700

Gln Phe Gln Pro Val Leu Arg Arg Arg Phe Val Asp Leu Phe Asn Gly
705                 710                 715                 720

Gly Phe Ile Ser Thr Arg Ser Ile Thr Val Thr Leu Ser Glu Gly Pro
                725                 730                 735

Val Ser Ala Pro Asn Pro Thr Leu Gly Gln Asp Ala Pro Ala Gly Arg
            740                 745                 750

Thr Phe Asp Gly Asp Leu Ala Arg Val Ser Val Glu Val Ile Arg Asp
        755                 760                 765

Ile Arg Val Lys Asn Arg Val Val Phe Ser Gly Asn Cys Thr Asn Leu
    770                 775                 780

Ser Glu Ala Ala Arg Ala Arg Leu Val Gly Leu Ala Ser Ala Tyr Gln
785                 790                 795                 800

Arg Gln Glu Lys Arg Val Asp Met Leu His Gly Ala Leu Gly Phe Leu
                805                 810                 815

Leu Lys Gln Phe His Gly Leu Leu Phe Pro Arg Gly Met Pro Pro Asn
            820                 825                 830

Ser Lys Ser Pro Asn Pro Gln Trp Phe Trp Thr Leu Leu Gln Arg Asn
        835                 840                 845

Gln Met Pro Ala Asp Lys Leu Thr His Glu Glu Ile Thr Thr Ile Ala

850                 855                 860

Ala Val Lys Arg Phe Thr Glu Glu Tyr Ala Ala Ile Asn Phe Ile Asn
865                 870                 875                 880

Leu Pro Pro Thr Cys Ile Gly Glu Leu Ala Gln Phe Tyr Met Ala Asn
                885                 890                 895

Leu Ile Leu Lys Tyr Cys Asp His Ser Gln Tyr Leu Ile Asn Thr Leu
            900                 905                 910

Thr Ser Ile Ile Thr Gly Ala Arg Arg Pro Arg Asp Pro Ser Ser Val
        915                 920                 925

Leu His Trp Ile Arg Lys Asp Val Thr Ser Ala Ala Asp Ile Glu Thr
    930                 935                 940

Gln Ala Lys Ala Leu Leu Glu Lys Thr Glu Asn Leu Pro Glu Leu Trp
945                 950                 955                 960

Thr Thr Ala Phe Thr Ser Thr His Leu Val Arg Ala Ala Met Asn Gln
                965                 970                 975

Arg Pro Met Val Val Leu Gly Ile Ser Ile Ser Lys Tyr His Gly Ala
            980                 985                 990

Ala Gly Asn Asn Arg Val Phe Gln  Ala Gly Asn Trp Ser  Gly Leu Asn
        995                 1000                 1005

Gly Gly  Lys Asn Val Cys Pro  Leu Phe Thr Phe Asp  Arg Thr Arg
    1010                 1015                 1020

Arg Phe  Ile Ile Ala Cys Pro  Arg Gly Gly Phe Ile  Cys Pro Val
    1025                 1030                 1035

Thr Gly  Pro Ser Ser Gly Asn  Arg Glu Thr Thr Leu  Ser Asp Gln
    1040                 1045                 1050

Val Arg  Gly Ile Ile Val Ser  Gly Gly Ala Met Val  Gln Leu Ala
    1055                 1060                 1065

Ile Tyr  Ala Thr Val Val Arg  Ala Val Gly Ala Arg  Ala Gln His
    1070                 1075                 1080

Met Ala  Phe Asp Asp Trp Leu  Ser Leu Thr Asp Asp  Glu Phe Leu
    1085                 1090                 1095

Ala Arg  Asp Leu Glu Glu Leu  His Asp Gln Ile Ile  Gln Thr Leu
    1100                 1105                 1110

Glu Thr  Pro Trp Thr Val Glu  Gly Ala Leu Glu Ala  Val Lys Ile

1115                    1120                    1125

Leu Asp  Glu Lys Thr Thr Ala  Gly Asp Gly Glu Thr  Pro Thr Asn
    1130                 1135                 1140

Leu Ala  Phe Asn Phe Asp Ser  Cys Glu Pro Ser His  Asp Thr Thr
    1145                 1150                 1155

Ser Asn  Val Leu Asn Ile Ser  Gly Ser Asn Ile Ser  Gly Ser Thr
    1160                 1165                 1170

Val Pro  Gly Leu Lys Arg Pro  Pro Glu Asp Asp Glu  Leu Phe Asp
    1175                 1180                 1185

Leu Ser  Gly Ile Pro Ile Lys  His Gly Asn Ile Thr  Met Glu Met
    1190                 1195                 1200

Ile

<210>  22
<211>  770
<212>  PRT
<213>  Varicella zoster

<400>  22

Met Glu Leu Asp Ile Asn Arg Thr Leu Leu Val Leu Leu Gly Gln Val
1               5                   10                  15

Tyr Thr Tyr Ile Phe Gln Val Glu Leu Leu Arg Arg Cys Asp Pro Arg
            20                  25                  30

Val Ala Cys Arg Phe Leu Tyr Arg Leu Ala Ala Asn Cys Leu Thr Val
        35                  40                  45

Arg Tyr Leu Leu Lys Leu Phe Leu Arg Gly Phe Asn Thr Gln Leu Lys
    50                  55                  60

Phe Gly Asn Thr Pro Thr Val Cys Ala Leu His Trp Ala Leu Cys Tyr
65                  70                  75                  80

Val Lys Gly Glu Gly Glu Arg Leu Phe Glu Leu Leu Gln His Phe Lys
                85                  90                  95

Thr Arg Phe Val Tyr Gly Glu Thr Lys Asp Ser Asn Cys Ile Lys Asp
            100                 105                 110

Tyr Phe Val Ser Ala Phe Asn Leu Lys Thr Cys Gln Tyr His His Glu
        115                 120                 125

Leu Ser Leu Thr Thr Tyr Gly Gly Tyr Val Ser Ser Glu Ile Gln Phe
    130                 135                 140

Leu His Asp Ile Glu Asn Phe Leu Lys Gln Leu Asn Tyr Cys Tyr Ile
145                 150                 155                 160

Ile Thr Ser Ser Arg Glu Ala Leu Asn Thr Leu Glu Thr Val Thr Arg
                165                 170                 175

Phe Met Thr Asp Thr Ile Gly Ser Gly Leu Ile Pro Pro Val Glu Leu
            180                 185                 190

Phe Asp Pro Ala His Pro Cys Ala Ile Cys Phe Glu Glu Leu Cys Ile
        195                 200                 205

Thr Ala Asn Gln Gly Glu Thr Leu His Arg Arg Leu Leu Gly Cys Ile
    210                 215                 220

Cys Asp His Val Thr Lys Gln Val Arg Val Asn Val Asp Val Asp Asp
225                 230                 235                 240

Ile Ile Arg Cys Leu Pro Tyr Ile Pro Asp Val Pro Asp Ile Lys Arg
                245                 250                 255

Gln Ser Ala Val Glu Ala Leu Arg Thr Leu Gln Thr Lys Thr Val Val
            260                 265                 270

Asn Pro Met Gly Ala Lys Asn Asp Thr Phe Asp Gln Thr Tyr Glu Ile
        275                 280                 285

Ala Ser Thr Met Leu Asp Ser Tyr Asn Val Phe Lys Pro Ala Pro Arg
    290                 295                 300

Cys Met Tyr Ala Ile Ser Glu Leu Lys Phe Trp Leu Thr Ser Asn Ser
305                 310                 315                 320

Thr Glu Gly Pro Gln Arg Thr Leu Asp Val Phe Val Asp Asn Leu Asp
                325                 330                 335

Val Leu Asn Glu His Glu Lys His Ala Glu Leu Thr Ala Val Thr Val
            340                 345                 350

Glu Leu Ala Leu Phe Gly Lys Thr Pro Ile His Phe Asp Arg Ala Phe
        355                 360                 365

Ser Glu Glu Leu Gly Ser Leu Asp Ala Ile Asp Ser Ile Leu Val Gly
    370                 375                 380

Asn Arg Ser Ser Ser Pro Asp Ser Gln Ile Glu Ala Leu Ile Lys Ala
385                 390                 395                 400

Cys Tyr Ala His His Leu Ser Ser Pro Leu Met Arg His Ile Ser Asn
                405                 410                 415

Pro Ser His Asp Asn Glu Ala Ala Leu Arg Gln Leu Leu Glu Arg Val
            420                 425                 430

Gly Cys Glu Asp Asp Leu Thr Lys Glu Ala Ser Asp Ser Ala Thr Ala
        435                 440                 445

Ser Glu Cys Asp Leu Asn Asp Asp Ser Ser Ile Thr Phe Ala Val His
    450                 455                 460

Gly Trp Glu Asn Leu Leu Ser Lys Ala Lys Ile Asp Ala Ala Glu Arg
465                 470                 475                 480

Lys Arg Val Tyr Leu Glu His Leu Ser Lys Arg Ser Leu Thr Ser Leu
                485                 490                 495

Gly Arg Cys Ile Arg Glu Gln Arg Gln Glu Leu Glu Lys Thr Leu Arg
            500                 505                 510

Val Asn Val Tyr Gly Glu Ala Leu Leu Gln Thr Phe Val Ser Met Gln
        515                 520                 525

Asn Gly Phe Gly Ala Arg Asn Val Phe Leu Ala Lys Val Ser Gln Ala
    530                 535                 540

Gly Cys Ile Ile Asp Asn Arg Ile Gln Glu Ala Ala Phe Asp Ala His
545                 550                 555                 560

Arg Phe Ile Arg Asn Thr Leu Val Arg His Thr Val Asp Ala Ala Met
                565                 570                 575

Leu Pro Ala Leu Thr His Lys Phe Phe Glu Leu Val Asn Gly Pro Leu
            580                 585                 590

Phe Asn His Asp Glu His Arg Phe Ala Gln Pro Pro Asn Thr Ala Leu
        595                 600                 605

Phe Phe Thr Val Glu Asn Val Gly Leu Phe Pro His Leu Lys Glu Glu
    610                 615                 620

Leu Ala Lys Phe Met Gly Gly Val Val Gly Ser Asn Trp Leu Leu Ser
625                 630                 635                 640

Pro Phe Arg Gly Phe Tyr Cys Phe Ser Gly Val Glu Gly Val Thr Phe
                645                 650                 655

Ala Gln Arg Leu Ala Trp Lys Tyr Ile Arg Glu Leu Val Phe Ala Thr
            660                 665                 670

Thr Leu Phe Thr Ser Val Phe His Cys Gly Glu Val Arg Leu Cys Arg
        675                 680                 685

Val Asp Arg Leu Gly Lys Asp Pro Arg Gly Cys Thr Ser Gln Pro Lys
    690                 695                 700

Gly Ile Gly Ser Ser His Gly Pro Leu Asp Gly Ile Tyr Leu Thr Tyr
705                 710                 715                 720

Glu Glu Thr Cys Pro Leu Val Ala Ile Ile Gln Ser Gly Glu Thr Gly
                725                 730                 735

Ile Asp Gln Asn Thr Val Val Ile Tyr Asp Ser Asp Val Phe Ser Leu
            740                 745                 750

Leu Tyr Thr Leu Met Gln Arg Leu Ala Pro Asp Ser Thr Asp Pro Ala
        755                 760                 765

Phe Ser
    770

<210>  23
<211>  868
<212>  PRT
<213>  Varicella zoster

<400>  23

Met Phe Val Thr Ala Val Val Ser Val Ser Pro Ser Ser Phe Tyr Glu
1               5                   10                  15

Ser Leu Gln Val Glu Pro Thr Gln Ser Glu Asp Ile Thr Arg Ser Ala
            20                  25                  30

His Leu Gly Asp Gly Asp Glu Ile Arg Glu Ala Ile His Lys Ser Gln
        35                  40                  45

Asp Ala Glu Thr Lys Pro Thr Phe Tyr Val Cys Pro Pro Pro Thr Gly
    50                  55                  60

Ser Thr Ile Val Arg Leu Glu Pro Thr Arg Thr Cys Pro Asp Tyr His
65                  70                  75                  80

Leu Gly Lys Asn Phe Thr Glu Gly Ile Ala Val Val Tyr Lys Glu Asn
                85                  90                  95

Ile Ala Ala Tyr Lys Phe Lys Ala Thr Val Tyr Tyr Lys Asp Val Ile
            100                 105                 110

Val Ser Thr Ala Trp Ala Gly Ser Ser Tyr Thr Gln Ile Thr Asn Arg
        115                 120                 125

Tyr Ala Asp Arg Val Pro Ile Pro Val Ser Glu Ile Thr Asp Thr Ile
    130                 135                 140

Asp Lys Phe Gly Lys Cys Ser Ser Lys Ala Thr Tyr Val Arg Asn Asn
145                 150                 155                 160

His Lys Val Glu Ala Phe Asn Glu Asp Lys Asn Pro Gln Asp Met Pro
                165                 170                 175

Leu Ile Ala Ser Lys Tyr Asn Ser Val Gly Ser Lys Ala Trp His Thr
            180                 185                 190

Thr Asn Asp Thr Tyr Met Val Ala Gly Thr Pro Gly Thr Tyr Arg Thr
        195                 200                 205

Gly Thr Ser Val Asn Cys Ile Ile Glu Glu Val Glu Ala Arg Ser Ile
    210                 215                 220

Phe Pro Tyr Asp Ser Phe Gly Leu Ser Thr Gly Asp Ile Ile Tyr Met
225                 230                 235                 240

Ser Pro Phe Phe Gly Leu Arg Asp Gly Ala Tyr Arg Glu His Ser Asn
                245                 250                 255

Tyr Ala Met Asp Arg Phe His Gln Phe Glu Gly Tyr Arg Gln Arg Asp
            260                 265                 270

Leu Asp Thr Arg Ala Leu Leu Glu Pro Ala Ala Arg Asn Phe Leu Val
        275                 280                 285

Thr Pro His Leu Thr Val Gly Trp Asn Trp Lys Pro Lys Arg Thr Glu
    290                 295                 300

Val Cys Ser Leu Val Lys Trp Arg Glu Val Glu Asp Val Val Arg Asp
305                 310                 315                 320

Glu Tyr Ala His Asn Phe Arg Phe Thr Met Lys Thr Leu Ser Thr Thr
                325                 330                 335

Phe Ile Ser Glu Thr Asn Glu Phe Asn Leu Asn Gln Ile His Leu Ser
            340                 345                 350

Gln Cys Val Lys Glu Glu Ala Arg Ala Ile Ile Asn Arg Ile Tyr Thr
        355                 360                 365

Thr Arg Tyr Asn Ser Ser His Val Arg Thr Gly Asp Ile Gln Thr Tyr
    370                 375                 380

Leu Ala Arg Gly Gly Phe Val Val Val Phe Gln Pro Leu Leu Ser Asn
385                 390                 395                 400

Ser Leu Ala Arg Leu Tyr Leu Gln Glu Leu Val Arg Glu Asn Thr Asn
                405                 410                 415

His Ser Pro Gln Lys His Pro Thr Arg Asn Thr Arg Ser Arg Arg Ser
            420                 425                 430

Val Pro Val Glu Leu Arg Ala Asn Arg Thr Ile Thr Thr Thr Ser Ser
        435                 440                 445

Val Glu Phe Ala Met Leu Gln Phe Thr Tyr Asp His Ile Gln Glu His
    450                 455                 460

Val Asn Glu Met Leu Ala Arg Ile Ser Ser Ser Trp Cys Gln Leu Gln
465                 470                 475                 480

Asn Arg Glu Arg Ala Leu Trp Ser Gly Leu Phe Pro Ile Asn Pro Ser
                485                 490                 495

Ala Leu Ala Ser Thr Ile Leu Asp Gln Arg Val Lys Ala Arg Ile Leu
            500                 505                 510

Gly Asp Val Ile Ser Val Ser Asn Cys Pro Glu Leu Gly Ser Asp Thr
        515                 520                 525

Arg Ile Ile Leu Gln Asn Ser Met Arg Val Ser Gly Ser Thr Thr Arg
    530                 535                 540

Cys Tyr Ser Arg Pro Leu Ile Ser Ile Val Ser Leu Asn Gly Ser Gly
545                 550                 555                 560

Thr Val Glu Gly Gln Leu Gly Thr Asp Asn Glu Leu Ile Met Ser Arg
                565                 570                 575

Asp Leu Leu Glu Pro Cys Val Ala Asn His Lys Arg Tyr Phe Leu Phe
            580                 585                 590

Gly His His Tyr Val Tyr Tyr Glu Asp Tyr Arg Tyr Val Arg Glu Ile
        595                 600                 605

Ala Val His Asp Val Gly Met Ile Ser Thr Tyr Val Asp Leu Asn Leu
    610                 615                 620

Thr Leu Leu Lys Asp Arg Glu Phe Met Pro Leu Gln Val Tyr Thr Arg
625                 630                 635                 640

Asp Glu Leu Arg Asp Thr Gly Leu Leu Asp Tyr Ser Glu Ile Gln Arg
                645                 650                 655

Arg Asn Gln Met His Ser Leu Arg Phe Tyr Asp Ile Asp Lys Val Val
            660                 665                 670

Gln Tyr Asp Ser Gly Thr Ala Ile Met Gln Gly Met Ala Gln Phe Phe
        675                 680                 685

Gln Gly Leu Gly Thr Ala Gly Gln Ala Val Gly His Val Val Leu Gly
    690                 695                 700

Ala Thr Gly Ala Leu Leu Ser Thr Val His Gly Phe Thr Thr Phe Leu
705                 710                 715                 720

Ser Asn Pro Phe Gly Ala Leu Ala Val Gly Leu Leu Val Leu Ala Gly
                725                 730                 735

Leu Val Ala Ala Phe Phe Ala Tyr Arg Tyr Val Leu Lys Leu Lys Thr
            740                 745                 750

Ser Pro Met Lys Ala Leu Tyr Pro Leu Thr Thr Lys Gly Leu Lys Gln
        755                 760                 765

Leu Pro Glu Gly Met Asp Pro Phe Ala Glu Lys Pro Asn Ala Thr Asp
    770                 775                 780

Thr Pro Ile Glu Glu Ile Gly Asp Ser Gln Asn Thr Glu Pro Ser Val
785                 790                 795                 800

Asn Ser Gly Phe Asp Pro Asp Lys Phe Arg Glu Ala Gln Glu Met Ile
                805                 810                 815

Lys Tyr Met Thr Leu Val Ser Ala Ala Glu Arg Gln Glu Ser Lys Ala
            820                 825                 830

Arg Lys Lys Asn Lys Thr Ser Ala Leu Leu Thr Ser Arg Leu Thr Gly
        835                 840                 845

Leu Ala Leu Arg Asn Arg Arg Gly Tyr Ser Arg Val Arg Thr Glu Asn
    850                 855                 860

Val Thr Gly Val
865

<210>  24
<211>  143
<212>  PRT
<213>  Varicella zoster

<400>  24

Met Glu Ser Ser Asn Ile Asn Ala Leu Gln Gln Pro Ser Ser Ile Ala
1               5                   10                  15

His His Pro Ser Lys Gln Cys Ala Ser Ser Leu Asn Glu Thr Val Lys
            20                  25                  30

Asp Ser Pro Pro Ala Ile Tyr Glu Asp Arg Leu Glu His Thr Pro Val
        35                  40                  45

Gln Leu Pro Arg Asp Gly Thr Pro Arg Asp Val Cys Ser Val Gly Gln
    50                  55                  60

Leu Thr Cys Arg Ala Cys Ala Thr Lys Pro Phe Arg Leu Asn Arg Asp
65                  70                  75                  80

Ser Gln Tyr Asp Tyr Leu Asn Thr Cys Pro Gly Gly Arg His Ile Ser
                85                  90                  95

Leu Ala Leu Glu Ile Ile Thr Gly Arg Trp Val Cys Ile Pro Arg Val
            100                 105                 110

Phe Pro Asp Thr Pro Glu Glu Lys Trp Met Ala Pro Tyr Ile Ile Pro
        115                 120                 125

Asp Arg Glu Gln Pro Ser Ser Gly Asp Glu Asp Ser Asp Thr Asp
    130                 135                 140


<210>  25
<211>  341
<212>  PRT
<213>  Varicella zoster

<400>  25

Met Ser Thr Asp Lys Thr Asp Val Lys Met Gly Val Leu Arg Ile Tyr
1               5                   10                  15

Leu Asp Gly Ala Tyr Gly Ile Gly Lys Thr Thr Ala Ala Glu Glu Phe
            20                  25                  30

Leu His His Phe Ala Ile Thr Pro Asn Arg Ile Leu Leu Ile Gly Glu
        35                  40                  45

Pro Leu Ser Tyr Trp Arg Asn Leu Ala Gly Glu Asp Ala Ile Cys Gly
    50                  55                  60

Ile Tyr Gly Thr Gln Thr Arg Arg Leu Asn Gly Asp Val Ser Pro Glu
65                  70                  75                  80

Asp Ala Gln Arg Leu Thr Ala His Phe Gln Ser Leu Phe Cys Ser Pro
                85                  90                  95

His Ala Ile Met His Ala Lys Ile Ser Ala Leu Met Asp Thr Ser Thr
            100                 105                 110

Ser Asp Leu Val Gln Val Asn Lys Glu Pro Tyr Lys Ile Met Leu Ser
        115                 120                 125

Asp Arg His Pro Ile Ala Ser Thr Ile Cys Phe Pro Leu Ser Arg Tyr
    130                 135                 140

Leu Val Gly Asp Met Ser Pro Ala Ala Leu Pro Gly Leu Leu Phe Thr

145                 150                 155                 160

Leu Pro Ala Glu Pro Pro Gly Thr Asn Leu Val Val Cys Thr Val Ser
                165                 170                 175

Leu Pro Ser His Leu Ser Arg Val Ser Lys Arg Ala Arg Pro Gly Glu
            180                 185                 190

Thr Val Asn Leu Pro Phe Val Met Val Leu Arg Asn Val Tyr Ile Met
        195                 200                 205

Leu Ile Asn Thr Ile Ile Phe Leu Lys Thr Asn Asn Trp His Ala Gly
    210                 215                 220

Trp Asn Thr Leu Ser Phe Cys Asn Asp Val Phe Lys Gln Lys Leu Gln
225                 230                 235                 240

Lys Ser Glu Cys Ile Lys Leu Arg Glu Val Pro Gly Ile Glu Asp Thr
                245                 250                 255

Leu Phe Ala Val Leu Lys Leu Pro Glu Leu Cys Gly Glu Phe Gly Asn
            260                 265                 270

Ile Leu Pro Leu Trp Ala Trp Gly Met Glu Thr Leu Ser Asn Cys Ser
        275                 280                 285

Arg Ser Met Ser Pro Phe Val Leu Ser Leu Glu Gln Thr Pro Gln His
    290                 295                 300

Ala Ala Gln Glu Leu Lys Thr Leu Leu Pro Gln Met Thr Pro Ala Asn
305                 310                 315                 320

Met Ser Ser Gly Ala Trp Asn Ile Leu Lys Glu Leu Val Asn Ala Val
                325                 330                 335

Gln Asp Asn Thr Ser
            340

<210>  26
<211>  841
<212>  PRT
<213>  Varicella zoster

<400>  26

Met Phe Ala Leu Val Leu Ala Val Val Ile Leu Pro Leu Trp Thr Thr
1               5                   10                  15

Ala Asn Lys Ser Tyr Val Thr Pro Thr Pro Ala Thr Arg Ser Ile Gly
            20                  25                  30

His Met Ser Ala Leu Leu Arg Glu Tyr Ser Asp Arg Asn Met Ser Leu
        35                  40                  45

Lys Leu Glu Ala Phe Tyr Pro Thr Gly Phe Asp Glu Glu Leu Ile Lys
    50                  55                  60

Ser Leu His Trp Gly Asn Asp Arg Lys His Val Phe Leu Val Ile Val
65                  70                  75                  80

Lys Val Asn Pro Thr Thr His Glu Gly Asp Val Gly Leu Val Ile Phe
                85                  90                  95

Pro Lys Tyr Leu Leu Ser Pro Tyr His Phe Lys Ala Glu His Arg Ala
            100                 105                 110

Pro Phe Pro Ala Gly Arg Phe Gly Phe Leu Ser His Pro Val Thr Pro
        115                 120                 125

Asp Val Ser Phe Phe Asp Ser Ser Phe Ala Pro Tyr Leu Thr Thr Gln
    130                 135                 140

His Leu Val Ala Phe Thr Thr Phe Pro Pro Asn Pro Leu Val Trp His
145                 150                 155                 160

Leu Glu Arg Ala Glu Thr Ala Ala Thr Ala Glu Arg Pro Phe Gly Val
                165                 170                 175

Ser Leu Leu Pro Ala Arg Pro Thr Val Pro Lys Asn Thr Ile Leu Glu
            180                 185                 190

His Lys Ala His Phe Ala Thr Trp Asp Ala Leu Ala Arg His Thr Phe
        195                 200                 205

Phe Ser Ala Glu Ala Ile Ile Thr Asn Ser Thr Leu Arg Ile His Val
    210                 215                 220

Pro Leu Phe Gly Ser Val Trp Pro Ile Arg Tyr Trp Ala Thr Gly Ser
225                 230                 235                 240

Val Leu Leu Thr Ser Asp Ser Gly Arg Val Glu Val Asn Ile Gly Val
                245                 250                 255

Gly Phe Met Ser Ser Leu Ile Ser Leu Ser Ser Gly Pro Pro Ile Glu
            260                 265                 270

Leu Ile Val Val Pro His Thr Val Lys Leu Asn Ala Val Thr Ser Asp
        275                 280                 285

Thr Thr Trp Phe Gln Leu Asn Pro Pro Gly Pro Asp Pro Gly Pro Ser
    290                 295                 300

Tyr Arg Val Tyr Leu Leu Gly Arg Gly Leu Asp Met Asn Phe Ser Lys
305                 310                 315                 320

His Ala Thr Val Asp Ile Cys Ala Tyr Pro Glu Glu Ser Leu Asp Tyr
                325                 330                 335

Arg Tyr His Leu Ser Met Ala His Thr Glu Ala Leu Arg Met Thr Thr
            340                 345                 350

Lys Ala Asp Gln His Asp Ile Asn Glu Glu Ser Tyr Tyr His Ile Ala
        355                 360                 365

Ala Arg Ile Ala Thr Ser Ile Phe Ala Leu Ser Glu Met Gly Arg Thr
    370                 375                 380

Thr Glu Tyr Phe Leu Leu Asp Glu Ile Val Asp Val Gln Tyr Gln Leu
385                 390                 395                 400

Lys Phe Leu Asn Tyr Ile Leu Met Arg Ile Gly Ala Gly Ala His Pro
                405                 410                 415

Asn Thr Ile Ser Gly Thr Ser Asp Leu Ile Phe Ala Asp Pro Ser Gln
            420                 425                 430

Leu His Asp Glu Leu Ser Leu Leu Phe Gly Gln Val Lys Pro Ala Asn
        435                 440                 445

Val Asp Tyr Phe Ile Ser Tyr Asp Glu Ala Arg Asp Gln Leu Lys Thr
    450                 455                 460

Ala Tyr Ala Leu Ser Arg Gly Gln Asp His Val Asn Ala Leu Ser Leu
465                 470                 475                 480

Ala Arg Arg Val Ile Met Ser Ile Tyr Lys Gly Leu Leu Val Lys Gln
                485                 490                 495

Asn Leu Asn Ala Thr Glu Arg Gln Ala Leu Phe Phe Ala Ser Met Ile
            500                 505                 510

Leu Leu Asn Phe Arg Glu Gly Leu Glu Asn Ser Ser Arg Val Leu Asp
        515                 520                 525

Gly Arg Thr Thr Leu Leu Leu Met Thr Ser Met Cys Thr Ala Ala His
    530                 535                 540

Ala Thr Gln Ala Ala Leu Asn Ile Gln Glu Gly Leu Ala Tyr Leu Asn
545                 550                 555                 560

Pro Ser Lys His Met Phe Thr Ile Pro Asn Val Tyr Ser Pro Cys Met
                565                 570                 575

Gly Ser Leu Arg Thr Asp Leu Thr Glu Glu Ile His Val Met Asn Leu
            580                 585                 590

Leu Ser Ala Ile Pro Thr Arg Pro Gly Leu Asn Glu Val Leu His Thr
        595                 600                 605

Gln Leu Asp Glu Ser Glu Ile Phe Asp Ala Ala Phe Lys Thr Met Met
    610                 615                 620

Ile Phe Thr Thr Trp Thr Ala Lys Asp Leu His Ile Leu His Thr His
625                 630                 635                 640

Val Pro Glu Val Phe Thr Cys Gln Asp Ala Ala Ala Arg Asn Gly Glu
                645                 650                 655

Tyr Val Leu Ile Leu Pro Ala Val Gln Gly His Ser Tyr Val Ile Thr
            660                 665                 670

Arg Asn Lys Pro Gln Arg Gly Leu Val Tyr Ser Leu Ala Asp Val Asp
        675                 680                 685

Val Tyr Asn Pro Ile Ser Val Val Tyr Leu Ser Arg Asp Thr Cys Val
    690                 695                 700

Ser Glu His Gly Val Ile Glu Thr Val Ala Leu Pro His Pro Asp Asn
705                 710                 715                 720

Leu Lys Glu Cys Leu Tyr Cys Gly Ser Val Phe Leu Arg Tyr Leu Thr
                725                 730                 735

Thr Gly Ala Ile Met Asp Ile Ile Ile Ile Asp Ser Lys Asp Thr Glu
            740                 745                 750

Arg Gln Leu Ala Ala Met Gly Asn Ser Thr Ile Pro Pro Phe Asn Pro
        755                 760                 765

Asp Met His Gly Asp Asp Ser Lys Ala Val Leu Leu Phe Pro Asn Gly
    770                 775                 780

Thr Val Val Thr Leu Leu Gly Phe Glu Arg Arg Gln Ala Ile Arg Met
785                 790                 795                 800

Ser Gly Gln Tyr Leu Gly Ala Ser Leu Gly Gly Ala Phe Leu Ala Val
                805                 810                 815

Val Gly Phe Gly Ile Ile Gly Trp Met Leu Cys Gly Asn Ser Arg Leu
            820                 825                 830

Arg Glu Tyr Asn Lys Ile Pro Leu Thr
        835                 840

<210>  27
<211>  240

<212>  PRT
<213>  Varicella zoster

<400>  27

Met Asn Pro Pro Gln Ala Arg Val Ser Glu Gln Thr Lys Asp Leu Leu
1               5                   10                  15

Ser Val Met Val Asn Gln His Pro Glu Glu Asp Ala Lys Val Cys Lys
            20                  25                  30

Ser Ser Asp Asn Ser Pro Leu Tyr Asn Thr Met Val Met Leu Ser Tyr
        35                  40                  45

Gly Gly Asp Thr Asp Leu Leu Leu Ser Ser Ala Cys Thr Arg Thr Ser
    50                  55                  60

Thr Val Asn Arg Ser Ala Phe Thr Gln His Ser Val Phe Tyr Ile Ile
65                  70                  75                  80

Ser Thr Val Leu Ile Gln Pro Ile Cys Cys Ile Phe Phe Phe Phe Tyr
                85                  90                  95

Tyr Lys Ala Thr Arg Cys Met Leu Leu Phe Thr Ala Gly Leu Leu Leu
            100                 105                 110

Thr Ile Leu His His Phe Arg Leu Ile Ile Met Leu Leu Cys Val Tyr
        115                 120                 125

Arg Asn Ile Arg Ser Asp Leu Leu Pro Leu Ser Thr Ser Gln Gln Leu
    130                 135                 140

Leu Leu Gly Ile Ile Val Val Thr Arg Thr Met Leu Phe Cys Ile Thr
145                 150                 155                 160

Ala Tyr Tyr Thr Leu Phe Ile Asp Thr Arg Val Phe Phe Leu Ile Thr
                165                 170                 175

Gly His Leu Gln Ser Glu Val Ile Phe Pro Asp Ser Val Ser Lys Ile
            180                 185                 190

Leu Pro Val Ser Trp Gly Pro Ser Pro Ala Val Leu Leu Val Met Ala
        195                 200                 205

Ala Val Ile Tyr Ala Met Asp Cys Leu Val Asp Thr Val Ser Phe Ile
    210                 215                 220

Gly Pro Arg Val Trp Val Arg Val Met Leu Lys Thr Ser Ile Ser Phe
225                 230                 235                 240

<210>  28
<211>  1396
<212>  PRT

<213>  Varicella zoster

<400>  28

Met Thr Thr Val Ser Cys Pro Ala Asn Val Ile Thr Thr Thr Glu Ser
1               5                   10                  15

Asp Arg Ile Ala Gly Leu Phe Asn Ile Pro Ala Gly Ile Ile Pro Thr
            20                  25                  30

Gly Asn Val Leu Ser Thr Ile Glu Val Cys Ala His Arg Cys Ile Phe
        35                  40                  45

Asp Phe Phe Lys Gln Ile Arg Ser Asp Asp Asn Ser Leu Tyr Ser Ala
    50                  55                  60

Gln Phe Asp Ile Leu Leu Gly Thr Tyr Cys Asn Thr Leu Asn Phe Val
65                  70                  75                  80

Arg Phe Leu Glu Leu Gly Leu Ser Val Ala Cys Ile Cys Thr Lys Phe
                85                  90                  95

Pro Glu Leu Ala Tyr Val Arg Asp Gly Val Ile Gln Phe Glu Val Gln
            100                 105                 110

Gln Pro Met Ile Ala Arg Asp Gly Pro His Pro Val Asp Gln Pro Val
        115                 120                 125

His Asn Tyr Met Val Lys Arg Ile His Lys Arg Ser Leu Ser Ala Ala
    130                 135                 140

Phe Ala Ile Ala Ser Glu Ala Leu Ser Leu Leu Ser Asn Thr Tyr Val
145                 150                 155                 160

Asp Gly Thr Glu Ile Asp Ser Ser Leu Arg Ile Arg Ala Ile Gln Gln
                165                 170                 175

Met Ala Arg Asn Leu Arg Thr Val Leu Asp Ser Phe Glu Arg Gly Thr
            180                 185                 190

Ala Asp Gln Leu Leu Gly Val Leu Leu Glu Lys Ala Pro Pro Leu Ser
        195                 200                 205

Leu Leu Ser Pro Ile Asn Lys Phe Gln Pro Glu Gly His Leu Asn Arg
    210                 215                 220

Val Ala Arg Ala Ala Leu Leu Ser Asp Leu Lys Arg Arg Val Cys Ala
225                 230                 235                 240

Asp Met Phe Phe Met Thr Arg His Ala Arg Glu Pro Arg Leu Ile Ser
                245                 250                 255

Ala Tyr Leu Ser Asp Met Val Ser Cys Thr Gln Pro Ser Val Met Val
            260                 265                 270

Ser Arg Ile Thr His Thr Asn Thr Arg Gly Arg Gln Val Asp Gly Val
        275                 280                 285

Leu Val Thr Thr Ala Thr Leu Lys Arg Gln Leu Leu Gln Gly Ile Leu
    290                 295                 300

Gln Ile Asp Asp Thr Ala Ala Asp Val Pro Val Thr Tyr Gly Glu Met
305                 310                 315                 320

Val Leu Gln Gly Thr Asn Leu Val Thr Ala Leu Val Met Gly Lys Ala
                325                 330                 335

Val Arg Gly Met Asp Asp Val Ala Arg His Leu Leu Asp Ile Thr Asp
            340                 345                 350

Pro Asn Thr Leu Asn Ile Pro Ser Ile Pro Pro Gln Ser Asn Ser Asp
        355                 360                 365

Ser Thr Thr Ala Gly Leu Pro Val Asn Ala Arg Val Pro Ala Asp Leu
    370                 375                 380

Val Ile Val Gly Asp Lys Leu Val Phe Leu Glu Ala Leu Glu Arg Arg
385                 390                 395                 400

Val Tyr Gln Ala Thr Arg Val Ala Tyr Pro Leu Ile Gly Asn Ile Asp
                405                 410                 415

Ile Thr Phe Ile Met Pro Met Gly Val Phe Gln Ala Asn Ser Met Asp
            420                 425                 430

Arg Tyr Thr Arg His Ala Gly Asp Phe Ser Thr Val Ser Glu Gln Asp
        435                 440                 445

Pro Arg Gln Phe Pro Pro Gln Gly Ile Phe Phe Tyr Asn Lys Asp Gly
    450                 455                 460

Ile Leu Thr Gln Leu Thr Leu Arg Asp Ala Met Gly Thr Ile Cys His
465                 470                 475                 480

Ser Ser Leu Leu Asp Val Glu Ala Thr Leu Val Ala Leu Arg Gln Gln
                485                 490                 495

His Leu Asp Arg Gln Cys Tyr Phe Gly Val Tyr Val Ala Glu Gly Thr
            500                 505                 510

Glu Asp Thr Leu Asp Val Gln Met Gly Arg Phe Met Glu Thr Trp Ala
        515                 520                 525

Asp Met Met Pro His His Pro His Trp Val Asn Glu His Leu Thr Ile
    530                 535                 540

Leu Gln Phe Ile Ala Pro Ser Asn Pro Arg Leu Arg Phe Glu Leu Asn
545                 550                 555                 560

Pro Ala Phe Asp Phe Phe Val Ala Pro Gly Asp Val Asp Leu Pro Gly
                565                 570                 575

Pro Gln Arg Pro Pro Glu Ala Met Pro Thr Val Asn Ala Thr Leu Arg
            580                 585                 590

Ile Ile Asn Gly Asn Ile Pro Val Pro Leu Cys Pro Ile Ser Phe Arg
        595                 600                 605

Asp Cys Arg Gly Thr Gln Leu Gly Leu Gly Arg His Thr Met Thr Pro
    610                 615                 620

Ala Thr Ile Lys Ala Val Lys Asp Thr Phe Glu Asp Arg Ala Tyr Pro
625                 630                 635                 640

Thr Ile Phe Tyr Met Leu Glu Ala Val Ile His Gly Asn Glu Arg Asn
                645                 650                 655

Phe Cys Ala Leu Leu Arg Leu Leu Thr Gln Cys Ile Arg Gly Tyr Trp
            660                 665                 670

Glu Gln Ser His Arg Val Ala Phe Val Asn Asn Phe His Met Leu Met
        675                 680                 685

Tyr Ile Thr Thr Tyr Leu Gly Asn Gly Glu Leu Pro Glu Val Cys Ile
    690                 695                 700

Asn Ile Tyr Arg Asp Leu Leu Gln His Val Arg Ala Leu Arg Gln Thr
705                 710                 715                 720

Ile Thr Asp Phe Thr Ile Gln Gly Glu Gly His Asn Gly Glu Thr Ser
                725                 730                 735

Glu Ala Leu Asn Asn Ile Leu Thr Asp Asp Thr Phe Ile Ala Pro Ile
            740                 745                 750

Leu Trp Asp Cys Asp Ala Leu Ile Tyr Arg Asp Glu Ala Ala Arg Asp
        755                 760                 765

Arg Leu Pro Ala Ile Arg Val Ser Gly Arg Asn Gly Tyr Gln Ala Leu
    770                 775                 780

His Phe Val Asp Met Ala Gly His Asn Phe Gln Arg Arg Asp Asn Val
785                 790                 795                 800

Leu Ile His Gly Arg Pro Val Arg Gly Asp Thr Gly Gln Gly Ile Pro
                805                 810                 815

Ile Thr Pro His His Asp Arg Glu Trp Gly Ile Leu Ser Lys Ile Tyr
            820                 825                 830

Tyr Tyr Ile Val Ile Pro Ala Phe Ser Arg Gly Ser Cys Cys Thr Met
        835                 840                 845

Gly Val Arg Tyr Asp Arg Leu Tyr Pro Ala Leu Gln Ala Val Ile Val
    850                 855                 860

Pro Glu Ile Pro Ala Asp Glu Glu Ala Pro Thr Thr Pro Glu Asp Pro
865                 870                 875                 880

Arg His Pro Leu His Ala His Gln Leu Val Pro Asn Ser Leu Asn Val
                885                 890                 895

Tyr Phe His Asn Ala His Leu Thr Val Asp Gly Asp Ala Leu Leu Thr
            900                 905                 910

Leu Gln Glu Leu Met Gly Asp Met Ala Glu Arg Thr Thr Ala Ile Leu
        915                 920                 925

Val Ser Ser Ala Pro Asp Ala Gly Ala Ala Thr Ala Thr Thr Arg Asn
    930                 935                 940

Met Arg Ile Tyr Asp Gly Ala Leu Tyr His Gly Leu Ile Met Met Ala
945                 950                 955                 960

Tyr Gln Ala Tyr Asp Glu Thr Ile Ala Thr Gly Thr Phe Phe Tyr Pro
                965                 970                 975

Val Pro Val Asn Pro Leu Phe Ala Cys Pro Glu His Leu Ala Ser Leu
            980                 985                 990

Arg Gly Met Thr Asn Ala Arg Arg  Val Leu Ala Lys Met  Val Pro Pro
        995                 1000                1005

Ile Pro  Pro Phe Leu Gly Ala  Asn His His Ala Thr  Ile Arg Gln
    1010                1015                1020

Pro Val  Ala Tyr His Val Thr  His Ser Lys Ser Asp  Phe Asn Thr
    1025                1030                1035

Leu Thr  Tyr Ser Leu Leu Gly  Gly Tyr Phe Lys Phe  Thr Pro Ile
    1040                1045                1050

Ser Leu  Thr His Gln Leu Arg  Thr Gly Phe His Pro  Gly Ile Ala
    1055                1060                1065

Phe Thr Val Val Arg Gln Asp Arg Phe Ala Thr Glu Gln Leu Leu
    1070                1075                1080

Tyr Ala Glu Arg Ala Ser Glu Ser Tyr Phe Val Gly Gln Ile Gln
    1085                1090                1095

Val His His His Asp Ala Ile Gly Gly Val Asn Phe Thr Leu Thr
    1100                1105                1110

Gln Pro Arg Ala His Val Asp Leu Gly Val Gly Tyr Thr Ala Val
    1115                1120                1125

Cys Ala Thr Ala Ala Leu Arg Cys Pro Leu Thr Asp Met Gly Asn
    1130                1135                1140

Thr Ala Gln Asn Leu Phe Phe Ser Arg Gly Gly Val Pro Met Leu
    1145                1150                1155

His Asp Asn Val Thr Glu Ser Leu Arg Arg Ile Thr Ala Ser Gly
    1160                1165                1170

Gly Arg Leu Asn Pro Thr Glu Pro Leu Pro Ile Phe Gly Gly Leu
    1175                1180                1185

Arg Pro Ala Thr Ser Ala Gly Ile Ala Arg Gly Gln Ala Ser Val
    1190                1195                1200

Cys Glu Phe Val Ala Met Pro Val Ser Thr Asp Leu Gln Tyr Phe
    1205                1210                1215

Arg Thr Ala Cys Asn Pro Arg Gly Arg Ala Ser Gly Met Leu Tyr
    1220                1225                1230

Met Gly Asp Arg Asp Ala Asp Ile Glu Ala Ile Met Phe Asp His
    1235                1240                1245

Thr Gln Ser Asp Val Ala Tyr Thr Asp Arg Ala Thr Leu Asn Pro
    1250                1255                1260

Trp Ala Ser Gln Lys His Ser Tyr Gly Asp Arg Leu Tyr Asn Gly
    1265                1270                1275

Thr Tyr Asn Leu Thr Gly Ala Ser Pro Ile Tyr Ser Pro Cys Phe
    1280                1285                1290

Lys Phe Phe Thr Pro Ala Glu Val Asn Thr Asn Cys Asn Thr Leu
    1295                1300                1305

Asp Arg Leu Leu Met Glu Ala Lys Ala Val Ala Ser Gln Ser Ser
    1310                1315                1320

Thr Asp Thr Glu Tyr Gln Phe Lys Arg Pro Pro Gly Ser Thr Glu
    1325                1330                1335

Met Thr Gln Asp Pro Cys Gly Leu Phe Gln Glu Ala Tyr Pro Pro
    1340                1345                1350

Leu Cys Ser Ser Asp Ala Ala Met Leu Arg Thr Ala His Ala Gly
    1355                1360                1365

Glu Thr Gly Ala Asp Glu Val His Leu Ala Gln Tyr Leu Ile Arg
    1370                1375                1380

Asp Ala Ser Pro Leu Arg Gly Cys Leu Pro Leu Pro Arg
    1385                1390                1395

<210>  29
<211>  316
<212>  PRT
<213>  Varicella zoster

<400>  29

Met Ala Met Pro Phe Glu Ile Glu Val Leu Leu Pro Gly Glu Leu Ser
1               5                   10                  15

Pro Ala Glu Thr Ser Ala Leu Gln Lys Cys Glu Gly Lys Ile Ile Thr
            20                  25                  30

Phe Ser Thr Leu Arg His Arg Ala Ser Leu Val Asp Ile Ala Leu Ser
        35                  40                  45

Ser Tyr Tyr Ile Asn Gly Ala Pro Pro Asp Thr Leu Ser Leu Leu Glu
    50                  55                  60

Ala Tyr Arg Met Arg Phe Ala Ala Val Ile Thr Arg Val Ile Pro Gly
65                  70                  75                  80

Lys Leu Leu Ala His Ala Ile Gly Val Gly Thr Pro Thr Pro Gly Leu
                85                  90                  95

Phe Ile Gln Asn Thr Ser Pro Val Asp Leu Cys Asn Gly Asp Tyr Ile
            100                 105                 110

Cys Leu Leu Pro Pro Val Phe Gly Ser Ala Asp Ser Ile Arg Leu Asp
        115                 120                 125

Ser Val Gly Leu Glu Ile Val Phe Pro Leu Thr Ile Pro Gln Thr Leu
    130                 135                 140

Met Arg Glu Ile Ile Ala Lys Val Val Ala Arg Ala Val Glu Arg Thr
145                 150                 155                 160

Ala Ala Gly Ala Gln Ile Leu Pro His Glu Val Leu Arg Gly Ala Asp

165                 170                 175

Val Ile Cys Tyr Asn Gly Arg Arg Tyr Glu Leu Glu Thr Asn Leu Gln
            180                 185                 190

His Arg Asp Gly Ser Asp Ala Ala Ile Arg Thr Leu Val Leu Asn Leu
        195                 200                 205

Met Phe Ser Ile Asn Glu Gly Cys Leu Leu Leu Leu Ala Leu Ile Pro
    210                 215                 220

Thr Leu Leu Val Gln Gly Ala His Asp Gly Tyr Val Asn Leu Leu Ile
225                 230                 235                 240

Gln Thr Ala Asn Cys Val Arg Glu Thr Gly Gln Leu Ile Asn Ile Pro
                245                 250                 255

Pro Met Pro Arg Ile Gln Asp Gly His Arg Arg Phe Pro Ile Tyr Glu
            260                 265                 270

Thr Ile Ser Ser Trp Ile Ser Thr Ser Ser Arg Leu Gly Asp Thr Leu
        275                 280                 285

Gly Thr Arg Ala Ile Leu Arg Val Cys Val Phe Asp Gly Pro Ser Thr
    290                 295                 300

Val His Pro Gly Asp Arg Thr Ala Val Ile Gln Val
305                 310                 315

<210>  30
<211>  676
<212>  PRT
<213>  Varicella zoster

<400>  30

Met Glu Ala His Leu Ala Asn Glu Thr Lys His Ala Leu Trp His Asn
1               5                   10                  15

Asp His Thr Lys Gly Leu Leu His Val Val Ile Pro Asn Ala Gly Leu
            20                  25                  30

Ile Ala Ala Gly Ile Asp Pro Ala Leu Leu Ile Leu Lys Lys Pro Gly
        35                  40                  45

Gln Arg Phe Lys Val Glu Val Gln Thr Arg Tyr His Ala Thr Gly Gln
    50                  55                  60

Cys Glu Pro Trp Cys Gln Val Phe Ala Ala Tyr Ile Pro Asp Asn Ala
65                  70                  75                  80

Leu Thr Asn Leu Leu Ile Pro Lys Thr Glu Pro Phe Val Ser His Val
                85                  90                  95

Phe Ser Ala Thr His Asn Ser Gly Gly Leu Ile Leu Ser Leu Pro Val
            100                 105                 110

Tyr Leu Ser Pro Gly Leu Phe Phe Asp Ala Phe Asn Val Val Ala Ile
        115                 120                 125

Arg Ile Asn Thr Gly Asn Arg Lys His Arg Asp Ile Cys Ile Met Tyr
    130                 135                 140

Ala Glu Leu Ile Pro Asn Gly Thr Arg Tyr Phe Ala Asp Gly Gln Arg
145                 150                 155                 160

Val Leu Leu Leu Cys Lys Gln Leu Ile Ala Tyr Ile Arg Cys Thr Pro
                165                 170                 175

Arg Leu Ala Ser Ser Ile Lys Ile Tyr Ala Glu His Met Val Ala Ala
            180                 185                 190

Met Gly Glu Ser His Thr Ser Asn Gly Asp Asn Ile Gly Pro Val Ser
        195                 200                 205

Ser Ile Ile Asp Leu Asp Arg Gln Leu Thr Ser Gly Gly Ile Asp Asp
    210                 215                 220

Ser Pro Ala Glu Thr Arg Ile Gln Glu Asn Asn Arg Asp Val Leu Glu
225                 230                 235                 240

Leu Ile Lys Arg Ala Val Asn Ile Val Asn Ser Arg His Pro Val Arg
                245                 250                 255

Pro Ser Ser Ser Arg Val Ala Ser Gly Leu Leu Gln Ser Ala Lys Gly
            260                 265                 270

His Gly Ala Gln Thr Ser Asn Thr Asp Pro Ile Asn Asn Gly Ser Phe
        275                 280                 285

Asp Gly Val Leu Glu Pro Pro Gly Gln Gly Arg Phe Thr Gly Lys Lys
    290                 295                 300

Asn Asn Ser Ser Ala Ser Ile Pro Pro Leu Gln Asp Val Leu Leu Phe
305                 310                 315                 320

Thr Pro Ala Ser Thr Glu Pro Gln Ser Leu Met Glu Trp Phe Asp Ile
                325                 330                 335

Cys Tyr Ala Gln Leu Val Ser Gly Asp Thr Pro Ala Asp Phe Trp Lys
            340                 345                 350

Arg Arg Pro Leu Ser Ile Val Pro Arg His Tyr Ala Glu Ser Pro Ser
        355                 360                 365

Pro Leu Ile Val Val Ser Tyr Asn Gly Ser Ser Ala Trp Gly Gly Arg
    370                 375                 380

Ile Thr Gly Ser Pro Ile Leu Tyr His Ser Ala Gln Ala Ile Ile Asp
385                 390                 395                 400

Ala Ala Cys Ile Asn Ala Arg Val Asp Asn Pro Gln Ser Leu His Val
                405                 410                 415

Thr Ala Arg Gln Glu Leu Val Ala Arg Leu Pro Phe Leu Ala Asn Val
            420                 425                 430

Leu Asn Asn Gln Thr Pro Leu Pro Ala Phe Lys Pro Gly Ala Glu Met
        435                 440                 445

Phe Leu Asn Gln Val Phe Lys Gln Ala Cys Val Thr Ser Leu Thr Gln
    450                 455                 460

Gly Leu Ile Thr Glu Leu Gln Thr Asn Pro Thr Leu Gln Gln Leu Met
465                 470                 475                 480

Glu Tyr Asp Ile Ala Asp Ser Ser Gln Thr Val Ile Asp Glu Ile Val
                485                 490                 495

Ala Arg Thr Pro Asp Leu Ile Gln Thr Ile Val Ser Val Leu Thr Glu
            500                 505                 510

Met Ser Met Asp Ala Phe Tyr Asn Ser Ser Leu Met Tyr Ala Val Leu
        515                 520                 525

Ala Tyr Leu Ser Ser Val Tyr Thr Arg Pro Gln Gly Gly Gly Tyr Ile
    530                 535                 540

Pro Tyr Leu His Ala Ser Phe Pro Cys Trp Leu Gly Asn Arg Ser Ile
545                 550                 555                 560

Tyr Leu Phe Asp Tyr Tyr Asn Ser Gly Gly Glu Ile Leu Lys Leu Ser
                565                 570                 575

Lys Val Pro Val Pro Val Ala Leu Glu Lys Val Gly Ile Gly Asn Ser
            580                 585                 590

Thr Gln Leu Arg Gly Lys Phe Ile Arg Ser Ala Asp Ile Val Asp Ile
        595                 600                 605

Gly Ile Cys Ser Lys Tyr Leu Pro Gly Gln Cys Tyr Ala Tyr Ile Cys
    610                 615                 620

Leu Gly Phe Asn Gln Gln Leu Gln Ser Ile Leu Val Leu Pro Gly Gly
625                 630                 635                 640

Phe Ala Ala Cys Phe Cys Ile Thr Asp Thr Leu Gln Ala Ala Leu Pro
                645                 650                 655

Ala Ser Leu Ile Gly Pro Ile Leu Asp Arg Phe Cys Phe Ser Ile Pro
            660                 665                 670

Asn Pro His Lys
        675

<210>  31
<211>  363
<212>  PRT
<213>  Varicella zoster

<400>  31

Met Glu Leu Gln Arg Ile Phe Pro Leu Tyr Thr Ala Thr Gly Ala Ala
1               5                   10                  15

Arg Lys Leu Thr Pro Glu Ala Val Gln Arg Leu Cys Asp Ala Leu Thr
            20                  25                  30

Leu Asp Met Gly Leu Trp Lys Ser Ile Leu Thr Asp Pro Arg Val Lys
        35                  40                  45

Ile Met Arg Ser Thr Ala Phe Ile Thr Leu Arg Ile Ala Pro Phe Ile
    50                  55                  60

Pro Leu Gln Thr Asp Thr Thr Asn Ile Ala Val Val Val Ala Thr Ile
65                  70                  75                  80

Tyr Ile Thr Arg Pro Arg Gln Met Asn Leu Pro Pro Lys Thr Phe His
                85                  90                  95

Val Ile Val Asn Phe Asn Tyr Glu Val Ser Tyr Ala Met Thr Ala Thr
            100                 105                 110

Leu Arg Ile Tyr Pro Val Glu Asn Ile Asp His Val Phe Gly Ala Thr
        115                 120                 125

Phe Lys Asn Pro Ile Ala Tyr Pro Leu Pro Thr Ser Ile Pro Asp Pro
    130                 135                 140

Arg Ala Asp Pro Thr Pro Ala Asp Leu Thr Pro Thr Pro Asn Leu Ser
145                 150                 155                 160

Asn Tyr Leu Gln Pro Pro Arg Leu Pro Lys Asn Pro Tyr Ala Cys Lys
                165                 170                 175

Val Ile Ser Pro Gly Val Trp Trp Ser Asp Glu Arg Arg Arg Leu Tyr
            180                 185                 190

Val Leu Ala Met Glu Pro Asn Leu Ile Gly Leu Cys Pro Ala Gly Trp
        195                 200                 205

His Ala Arg Ile Leu Gly Ser Val Leu Asn Arg Leu Leu Ser His Ala
    210                 215                 220

Asp Gly Cys Asp Glu Cys Asn His Arg Val His Val Gly Ala Leu Tyr
225                 230                 235                 240

Ala Leu Pro His Val Thr Asn His Ala Glu Gly Cys Val Cys Trp Ala
                245                 250                 255

Pro Cys Met Trp Arg Lys Ala Gly Gln Arg Glu Leu Lys Val Glu Val
            260                 265                 270

Asp Ile Gly Ala Thr Gln Val Leu Phe Val Asp Val Thr Thr Cys Ile
        275                 280                 285

Arg Ile Thr Ser Thr Lys Asn Pro Arg Ile Thr Ala Asn Leu Gly Asp
    290                 295                 300

Val Ile Ala Gly Thr Asn Ala Ser Gly Leu Ser Val Pro Val Asn Ser
305                 310                 315                 320

Ser Gly Trp Gln Leu Tyr Met Phe Gly Glu Thr Leu Ser Arg Ala Ile
                325                 330                 335

Ile Asn Gly Cys Gly Leu Leu Gln Arg Ile Cys Phe Pro Glu Thr Gln
            340                 345                 350

Arg Leu Ser Gly Glu Pro Glu Pro Thr Thr Thr
        355                 360

<210>  32
<211>  199
<212>  PRT
<213>  Varicella zoster

<400>  32

Met Ser Gly His Thr Pro Thr Tyr Ala Ser His Arg Arg Asn Arg Val
1               5                   10                  15

Lys Leu Val Glu Ala His Asn Arg Ala Gly Leu Phe Lys Glu Arg Thr
            20                  25                  30

Leu Asp Leu Ile Arg Gly Gly Ala Ser Val Gln Asp Pro Ala Phe Val
        35                  40                  45

Tyr Ala Phe Thr Ala Ala Lys Glu Ala Cys Ala Asp Leu Asn Asn Gln
    50                  55                  60

Leu Arg Ser Ala Ala Arg Ile Ala Ser Val Glu Gln Lys Ile Arg Asp
65                  70                  75                  80

Ile Gln Ser Lys Val Glu Glu Gln Thr Ser Ile Gln Gln Ile Leu Asn
                85                  90                  95

Thr Asn Arg Arg Tyr Ile Ala Pro Asp Phe Ile Arg Gly Leu Asp Lys
            100                 105                 110

Thr Glu Asp Asp Asn Thr Asp Asn Ile Asp Arg Leu Glu Asp Ala Val
        115                 120                 125

Gly Pro Asn Ile Glu His Glu Asn His Thr Trp Phe Gly Glu Asp Asp
    130                 135                 140

Glu Ala Leu Leu Thr Gln Trp Met Leu Thr Thr His Pro Pro Thr Ser
145                 150                 155                 160

Lys Tyr Leu Gln Leu Gln Asp Leu Cys Val Pro Thr Thr Ile Pro Thr
                165                 170                 175

Asp Met Asn Gln Met Gln Pro Gln Pro Ile Ser Lys Asn Glu Asn Pro
            180                 185                 190

Pro Thr Pro His Thr Asp Val
        195

<210>  33
<211>  551
<212>  PRT
<213>  Varicella zoster

<400>  33

Met Ala Arg Ser Gly Leu Asp Arg Ile Asp Ile Ser Pro Gln Pro Ala
1               5                   10                  15

Lys Lys Ile Ala Arg Val Gly Gly Leu Gln His Pro Phe Val Lys Thr
            20                  25                  30

Asp Ile Asn Thr Ile Asn Val Glu His His Phe Ile Asp Thr Leu Gln
        35                  40                  45

Lys Thr Ser Pro Asn Met Asp Cys Arg Gly Met Thr Ala Gly Ile Phe
    50                  55                  60

Ile Arg Leu Ser His Met Tyr Lys Ile Leu Thr Thr Leu Glu Ser Pro
65                  70                  75                  80

Asn Asp Val Thr Tyr Thr Thr Pro Gly Ser Thr Asn Ala Leu Phe Phe
                85                  90                  95

Lys Thr Ser Thr Gln Pro Gln Glu Pro Arg Pro Glu Glu Leu Ala Ser

            100                 105                 110

Lys Leu Thr Gln Asp Asp Ile Lys Arg Ile Leu Leu Thr Ile Glu Ser
        115                 120                 125

Glu Thr Arg Gly Gln Gly Asp Asn Ala Ile Trp Thr Leu Leu Arg Arg
    130                 135                 140

Asn Leu Ile Thr Ala Ser Thr Leu Lys Trp Ser Val Ser Gly Pro Val
145                 150                 155                 160

Ile Pro Pro Gln Trp Phe Tyr His His Asn Thr Thr Asp Thr Tyr Gly
                165                 170                 175

Asp Ala Ala Ala Met Ala Phe Gly Lys Thr Asn Glu Pro Ala Ala Arg
            180                 185                 190

Ala Ile Val Glu Ala Leu Phe Ile Asp Pro Ala Asp Ile Arg Thr Pro
        195                 200                 205

Asp His Leu Thr Pro Glu Ala Thr Thr Lys Phe Phe Asn Phe Asp Met
    210                 215                 220

Leu Asn Thr Lys Ser Pro Ser Leu Leu Val Gly Thr Pro Arg Ile Gly
225                 230                 235                 240

Thr Tyr Glu Cys Gly Leu Leu Ile Asp Val Arg Thr Gly Leu Ile Gly
                245                 250                 255

Ala Ser Leu Asp Val Leu Val Cys Asp Arg Asp Pro Leu Thr Gly Thr
            260                 265                 270

Leu Asn Pro His Pro Ala Glu Thr Asp Ile Ser Phe Phe Glu Ile Lys
        275                 280                 285

Cys Arg Ala Lys Tyr Leu Phe Asp Pro Asp Asp Lys Asn Asn Pro Leu
    290                 295                 300

Gly Arg Thr Tyr Thr Thr Leu Ile Asn Arg Pro Thr Met Ala Asn Leu
305                 310                 315                 320

Arg Asp Phe Leu Tyr Thr Ile Lys Asn Pro Cys Val Ser Phe Phe Gly
                325                 330                 335

Pro Ser Ala Asn Pro Ser Thr Arg Glu Ala Leu Ile Thr Asp His Val
            340                 345                 350

Glu Trp Lys Arg Leu Gly Phe Lys Gly Gly Arg Ala Leu Thr Glu Leu
        355                 360                 365

Asp Ala His His Leu Gly Leu Asn Arg Thr Ile Ser Ser Arg Val Trp

370                 375                 380

Val Phe Asn Asp Pro Asp Ile Gln Lys Gly Thr Ile Thr Thr Ile Ala
385                 390                 395                 400

Trp Ala Thr Gly Asp Thr Ala Leu Gln Ile Pro Val Phe Ala Asn Pro
                405                 410                 415

Arg His Ala Asn Phe Lys Gln Ile Ala Val Gln Thr Tyr Val Leu Ser
            420                 425                 430

Gly Tyr Phe Pro Ala Leu Lys Leu Arg Pro Phe Leu Val Thr Phe Ile
        435                 440                 445

Gly Arg Val Arg Arg Pro His Glu Val Gly Val Pro Leu Arg Val Asp
    450                 455                 460

Thr Gln Ala Ala Ala Ile Tyr Glu Tyr Asn Trp Pro Thr Ile Pro Pro
465                 470                 475                 480

His Cys Ala Val Pro Val Ile Ala Val Leu Thr Pro Ile Glu Val Asp
                485                 490                 495

Val Pro Arg Val Thr Gln Ile Leu Lys Asp Thr Gly Asn Asn Ala Ile
            500                 505                 510

Thr Ser Ala Leu Arg Ser Leu Arg Trp Asp Asn Leu His Pro Ala Val
        515                 520                 525

Glu Glu Glu Ser Val Asp Cys Ala Asn Gly Thr Thr Ser Leu Leu Arg
    530                 535                 540

Ala Thr Glu Lys Pro Leu Leu
545                 550

<210>  34
<211>  835
<212>  PRT
<213>  Varicella zoster

<400>  34

Met Ser Pro Asn Thr Gly Glu Ser Asn Ala Ala Val Tyr Ala Ser Ser
1               5                   10                  15

Thr Gln Leu Ala Arg Ala Leu Tyr Gly Gly Asp Leu Val Ser Trp Ile
            20                  25                  30

Lys His Thr His Pro Gly Ile Ser Leu Glu Leu Gln Leu Asp Val Pro
        35                  40                  45

Val Lys Leu Ile Lys Pro Gly Met Ser Gln Thr Arg Pro Val Thr Val
    50                  55                  60

Val Arg Ala Pro Met Gly Ser Gly Lys Thr Thr Ala Leu Leu Glu Trp
65                  70                  75                  80

Leu Gln His Ala Leu Lys Ala Asp Ile Ser Val Leu Val Val Ser Cys
                85                  90                  95

Arg Arg Ser Phe Thr Gln Thr Leu Ile Gln Arg Phe Asn Asp Ala Gly
            100                 105                 110

Leu Ser Gly Phe Val Thr Tyr Leu Thr Ser Glu Thr Tyr Ile Met Gly
        115                 120                 125

Phe Lys Arg Leu Ile Val Gln Leu Glu Ser Leu His Arg Val Ser Ser
    130                 135                 140

Glu Ala Ile Asp Ser Tyr Asp Val Leu Ile Leu Asp Glu Val Met Ser
145                 150                 155                 160

Val Ile Gly Gln Leu Tyr Ser Pro Thr Met Arg Arg Leu Ser Ala Val
                165                 170                 175

Asp Ser Leu Leu Tyr Arg Leu Leu Asn Arg Cys Ser Gln Ile Ile Ala
            180                 185                 190

Met Asp Ala Thr Val Asn Ser Gln Phe Ile Asp Leu Ile Ser Gly Leu
        195                 200                 205

Arg Gly Asp Glu Asn Ile His Thr Ile Val Cys Thr Tyr Ala Gly Val
    210                 215                 220

Gly Phe Ser Gly Arg Thr Cys Thr Ile Leu Arg Asp Met Gly Ile Asp
225                 230                 235                 240

Thr Leu Val Arg Val Ile Lys Arg Ser Pro Glu His Glu Asp Val Arg
                245                 250                 255

Thr Ile His Gln Leu Arg Gly Thr Phe Phe Asp Glu Leu Ala Leu Arg
            260                 265                 270

Leu Gln Cys Gly His Asn Ile Cys Ile Phe Ser Ser Thr Leu Ser Phe
        275                 280                 285

Ser Glu Leu Val Ala Gln Phe Cys Ala Ile Phe Thr Asp Ser Ile Leu
    290                 295                 300

Ile Leu Asn Ser Thr Arg Pro Leu Cys Asn Val Asn Glu Trp Lys His
305                 310                 315                 320

Phe Arg Val Leu Val Tyr Thr Thr Val Val Thr Val Gly Leu Ser Phe
                325                 330                 335

Asp Met Ala His Phe His Ser Met Phe Ala Tyr Ile Lys Pro Met Ser
            340                 345                 350

Tyr Gly Pro Asp Met Val Ser Val Tyr Gln Ser Leu Gly Arg Val Arg
        355                 360                 365

Leu Leu Leu Leu Asn Glu Val Leu Met Tyr Val Asp Gly Ser Arg Thr
    370                 375                 380

Arg Cys Gly Pro Leu Phe Ser Pro Met Leu Leu Asn Phe Thr Ile Ala
385                 390                 395                 400

Asn Lys Phe Gln Trp Phe Pro Thr His Thr Gln Ile Thr Asn Lys Leu
                405                 410                 415

Cys Cys Ala Phe Arg Gln Arg Cys Ala Asn Ala Phe Thr Arg Ser Asn
            420                 425                 430

Thr His Leu Phe Ser Arg Phe Lys Tyr Lys His Leu Phe Glu Arg Cys
        435                 440                 445

Ser Leu Trp Ser Leu Ala Asp Ser Ile Asn Ile Leu Gln Thr Leu Leu
    450                 455                 460

Ala Ser Asn Gln Ile Leu Val Val Leu Asp Gly Met Gly Pro Ile Thr
465                 470                 475                 480

Asp Val Ser Pro Val Gln Phe Cys Ala Phe Ile His Asp Leu Arg His
                485                 490                 495

Ser Ala Asn Ala Val Ala Ser Cys Met Arg Ser Leu Arg Gln Asp Asn
            500                 505                 510

Asp Ser Cys Leu Thr Asp Phe Gly Pro Ser Gly Phe Met Ala Asp Asn
        515                 520                 525

Ile Thr Ala Phe Met Glu Lys Tyr Leu Met Glu Ser Ile Asn Thr Glu
    530                 535                 540

Glu Gln Ile Lys Val Phe Lys Ala Leu Ala Cys Pro Ile Glu Gln Pro
545                 550                 555                 560

Arg Leu Val Asn Thr Ala Ile Leu Gly Ala Cys Ile Arg Ile Pro Glu
                565                 570                 575

Ala Leu Glu Ala Phe Asp Val Phe Gln Lys Ile Tyr Thr His Tyr Ala
            580                 585                 590

Ser Gly Trp Phe Pro Val Leu Asp Lys Thr Gly Glu Phe Ser Ile Ala
        595                 600                 605

Thr Ile Thr Thr Ala Pro Asn Leu Thr Thr His Trp Glu Leu Phe Arg
    610                 615                 620

Arg Cys Ala Tyr Ile Ala Lys Thr Leu Lys Trp Asn Pro Ser Thr Glu
625                 630                 635                 640

Gly Cys Val Thr Gln Val Leu Asp Thr Asp Ile Asn Thr Leu Phe Asn
                645                 650                 655

Gln His Gly Asp Ser Leu Ala Gln Leu Ile Phe Glu Val Met Arg Cys
            660                 665                 670

Asn Val Thr Asp Ala Lys Ile Ile Leu Asn Arg Pro Val Trp Arg Thr
        675                 680                 685

Thr Gly Phe Leu Asp Gly Cys His Asn Gln Cys Phe Arg Pro Ile Pro
    690                 695                 700

Thr Lys His Glu Tyr Asn Ile Ala Leu Phe Arg Leu Ile Trp Glu Gln
705                 710                 715                 720

Leu Phe Gly Ala Arg Val Thr Lys Ser Thr Gln Thr Phe Pro Gly Ser
                725                 730                 735

Thr Arg Val Lys Asn Leu Lys Lys Lys Asp Leu Glu Thr Leu Leu Asp
            740                 745                 750

Ser Ile Asn Val Asp Arg Ser Ala Cys Arg Thr Tyr Arg Gln Leu Tyr
        755                 760                 765

Asn Leu Leu Met Ser Gln Arg His Ser Phe Ser Gln Gln Arg Tyr Lys
    770                 775                 780

Ile Thr Ala Pro Ala Trp Ala Arg His Val Tyr Phe Gln Ala His Gln
785                 790                 795                 800

Met His Leu Ala Pro His Ala Glu Ala Met Leu Gln Leu Ala Leu Ser
                805                 810                 815

Glu Leu Ser Pro Gly Ser Trp Pro Arg Ile Asn Gly Ala Val Asn Phe
            820                 825                 830

Glu Ser Leu
        835

<210>  35
<211>  771
<212>  PRT
<213>  Varicella zoster

<400>  35

Met Asp Ala Thr Gln Ile Thr Leu Val Arg Glu Ser Gly His Ile Cys
1               5                   10                  15

Ala Ala Ser Ile Tyr Thr Ser Trp Thr Gln Ser Gly Gln Leu Thr Gln
            20                  25                  30

Asn Gly Leu Ser Val Leu Tyr Tyr Leu Leu Cys Lys Asn Ser Cys Gly
        35                  40                  45

Lys Tyr Val Pro Lys Phe Ala Glu Ile Thr Val Gln Gln Glu Asp Leu
    50                  55                  60

Cys Arg Tyr Ser Arg His Gly Gly Ser Val Ser Ala Ala Thr Phe Ala
65                  70                  75                  80

Ser Ile Cys Arg Ala Ala Ser Ser Ala Ala Leu Asp Ala Trp Pro Leu
                85                  90                  95

Glu Pro Leu Gly Asn Ala Asp Thr Trp Arg Cys Leu His Gly Thr Ala
            100                 105                 110

Leu Ala Thr Leu Arg Arg Val Leu Gly Phe Lys Ser Phe Tyr Ser Pro
        115                 120                 125

Val Thr Phe Glu Thr Asp Thr Asn Thr Gly Leu Leu Leu Lys Thr Ile
    130                 135                 140

Pro Asp Glu His Ala Leu Asn Asn Asp Asn Thr Pro Ser Thr Gly Val
145                 150                 155                 160

Leu Arg Ala Asn Phe Pro Val Ala Ile Asp Val Ser Ala Val Ser Ala
                165                 170                 175

Cys Asn Ala His Thr Gln Gly Thr Ser Leu Ala Tyr Ala Arg Leu Thr
            180                 185                 190

Ala Leu Lys Ser Asn Gly Asp Thr Gln Gln Gln Thr Pro Leu Asp Val
        195                 200                 205

Glu Val Ile Thr Pro Lys Ala Tyr Ile Arg Arg Lys Tyr Lys Ser Thr
    210                 215                 220

Phe Ser Pro Pro Ile Glu Arg Glu Gly Gln Thr Ser Asp Leu Phe Asn
225                 230                 235                 240

Leu Glu Glu Arg Arg Leu Val Leu Ser Gly Asn Arg Ala Ile Val Val
                245                 250                 255

Arg Val Leu Leu Pro Cys Tyr Phe Asp Cys Leu Thr Thr Asp Ser Thr
            260                 265                 270

Val Thr Ser Ser Leu Ser Ile Leu Ala Thr Tyr Arg Leu Trp Tyr Ala
        275                 280                 285

Ala Ala Phe Gly Lys Pro Gly Val Val Arg Pro Ile Phe Ala Tyr Leu
    290                 295                 300

Gly Pro Glu Leu Asn Pro Lys Gly Glu Asp Arg Asp Tyr Phe Cys Thr
305                 310                 315                 320

Val Gly Phe Pro Gly Trp Thr Thr Leu Arg Thr Gln Thr Pro Ala Val
                325                 330                 335

Glu Ser Ile Arg Thr Ala Thr Glu Met Tyr Met Glu Thr Asp Gly Leu
            340                 345                 350

Trp Pro Val Thr Gly Ile Gln Ala Phe His Tyr Leu Ala Pro Trp Gly
        355                 360                 365

Gln His Pro Pro Leu Pro Pro Arg Val Gln Asp Leu Ile Gly Gln Ile
    370                 375                 380

Pro Gln Asp Thr Gly His Ala Asp Ala Thr Val Asn Trp Asp Ala Gly
385                 390                 395                 400

Arg Ile Ser Thr Val Phe Lys Gln Pro Val Gln Leu Gln Asp Arg Trp
                405                 410                 415

Met Ala Lys Phe Asp Phe Ser Ala Phe Phe Pro Thr Ile Tyr Cys Ala
            420                 425                 430

Met Phe Pro Met His Phe Arg Leu Gly Lys Ile Val Leu Ala Arg Met
        435                 440                 445

Arg Arg Gly Met Gly Cys Leu Lys Pro Ala Leu Val Ser Phe Phe Gly
    450                 455                 460

Gly Leu Arg His Ile Leu Pro Ser Ile Tyr Lys Ala Ile Ile Phe Ile
465                 470                 475                 480

Ala Asn Glu Ile Ser Leu Cys Val Glu Gln Thr Ala Leu Glu Gln Gly
                485                 490                 495

Phe Ala Ile Cys Thr Tyr Ile Lys Asp Gly Phe Trp Gly Ile Phe Thr
            500                 505                 510

Asp Leu His Thr Arg Asn Val Cys Ser Asp Gln Ala Arg Cys Ser Ala
        515                 520                 525

Leu Asn Leu Ala Ala Thr Cys Glu Arg Ala Val Thr Gly Leu Leu Arg
    530                 535                 540

Ile Gln Leu Gly Leu Asn Phe Thr Pro Ala Met Glu Pro Val Leu Arg
545                 550                 555                 560

Val Glu Gly Val Tyr Thr His Ala Phe Thr Trp Cys Thr Thr Gly Ser
                565                 570                 575

Trp Leu Trp Asn Leu Gln Thr Asn Thr Pro Pro Asp Leu Val Gly Val
            580                 585                 590

Pro Trp Arg Ser Gln Ala Ala Arg Asp Leu Lys Glu Arg Leu Ser Gly
        595                 600                 605

Leu Leu Cys Thr Ala Thr Lys Ile Arg Glu Arg Ile Gln Glu Asn Cys
    610                 615                 620

Ile Trp Asp His Val Leu Tyr Asp Ile Trp Ala Gly Gln Val Val Glu
625                 630                 635                 640

Ala Ala Arg Lys Thr Tyr Val Asp Phe Phe Glu His Val Phe Asp Arg
                645                 650                 655

Arg Tyr Thr Pro Val Tyr Trp Ser Leu Gln Glu Gln Asn Ser Glu Thr
            660                 665                 670

Lys Ala Ile Pro Ala Ser Tyr Leu Thr Tyr Gly His Met Gln Asp Lys
        675                 680                 685

Asp Tyr Lys Pro Arg Gln Ile Ile Met Val Arg Asn Pro Asn Pro His
    690                 695                 700

Gly Pro Pro Thr Val Val Tyr Trp Glu Leu Leu Pro Ser Cys Ala Cys
705                 710                 715                 720

Ile Pro Pro Ile Asp Cys Ala Ala His Leu Lys Pro Leu Ile His Thr
                725                 730                 735

Phe Val Thr Ile Ile Asn His Leu Leu Asp Ala His Asn Asp Phe Ser
            740                 745                 750

Ser Pro Ser Leu Lys Phe Thr Asp Asp Pro Leu Ala Ser Tyr Asn Phe
        755                 760                 765

Leu Phe Leu
    770

<210>  36
<211>  881
<212>  PRT
<213>  Varicella zoster

<400>  36

Met Lys Arg Ser Ile Ser Val Asp Ser Ser Ser Pro Lys Asn Val Phe
1               5                   10                  15

Asn Pro Glu Thr Pro Asn Gly Phe Asp Asp Ser Val Tyr Leu Asn Phe
            20                  25                  30

Thr Ser Met His Ser Ile Gln Pro Ile Leu Ser Arg Ile Arg Glu Leu
        35                  40                  45

Ala Ala Ile Thr Ile Pro Lys Glu Arg Val Pro Arg Leu Cys Trp Phe
    50                  55                  60

Lys Gln Leu Leu Glu Leu Gln Ala Pro Pro Glu Met Gln Arg Asn Glu
65                  70                  75                  80

Leu Pro Phe Ser Val Tyr Leu Ile Ser Gly Asn Ala Gly Ser Gly Lys
                85                  90                  95

Ser Thr Cys Ile Gln Thr Leu Asn Glu Ala Ile Asp Cys Ile Ile Thr
            100                 105                 110

Gly Ser Thr Arg Val Ala Ala Gln Asn Val His Ala Lys Leu Ser Thr
        115                 120                 125

Ala Tyr Ala Ser Arg Pro Ile Asn Thr Ile Phe His Glu Phe Gly Phe
    130                 135                 140

Arg Gly Asn His Ile Gln Ala Gln Leu Gly Arg Tyr Ala Tyr Asn Trp
145                 150                 155                 160

Thr Thr Thr Pro Pro Ser Ile Glu Asp Leu Gln Lys Arg Asp Ile Val
                165                 170                 175

Tyr Tyr Trp Glu Val Leu Ile Asp Ile Thr Lys Arg Val Phe Gln Met
            180                 185                 190

Gly Asp Asp Gly Arg Gly Gly Thr Ser Thr Phe Lys Thr Leu Trp Ala
        195                 200                 205

Ile Glu Arg Leu Leu Asn Lys Pro Thr Gly Ser Met Ser Gly Thr Ala
    210                 215                 220

Phe Ile Ala Cys Gly Ser Leu Pro Ala Phe Thr Arg Ser Asn Val Ile
225                 230                 235                 240

Val Ile Asp Glu Ala Gly Leu Leu Gly Arg His Ile Leu Thr Ala Val
                245                 250                 255

Val Tyr Cys Trp Trp Leu Leu Asn Ala Ile Tyr Gln Ser Pro Gln Tyr
            260                 265                 270

Ile Asn Gly Arg Lys Pro Val Ile Val Cys Val Gly Ser Pro Thr Gln
        275                 280                 285

Thr Asp Ser Leu Glu Ser His Phe Gln His Asp Met Gln Arg Ser His
    290                 295                 300

Val Thr Pro Ser Glu Asn Ile Leu Thr Tyr Ile Ile Cys Asn Gln Thr
305                 310                 315                 320

Leu Arg Gln Tyr Thr Asn Ile Ser His Asn Trp Ala Ile Phe Ile Asn
                325                 330                 335

Asn Lys Arg Cys Gln Glu Asp Asp Phe Gly Asn Leu Leu Lys Thr Leu
            340                 345                 350

Glu Tyr Gly Leu Pro Ile Thr Glu Ala His Ala Arg Leu Val Asp Thr
        355                 360                 365

Phe Val Val Pro Ala Ser Tyr Ile Asn Asn Pro Ala Asn Leu Pro Gly
    370                 375                 380

Trp Thr Arg Leu Tyr Ser Ser His Lys Glu Val Ser Ala Tyr Met Ser
385                 390                 395                 400

Lys Leu His Ala His Leu Lys Leu Ser Lys Asn Asp His Phe Ser Val
                405                 410                 415

Phe Ala Leu Pro Thr Tyr Thr Phe Ile Arg Leu Thr Ala Phe Asp Glu
            420                 425                 430

Tyr Arg Lys Leu Thr Gly Gln Pro Gly Leu Ser Val Glu His Trp Ile
        435                 440                 445

Arg Ala Asn Ser Gly Arg Leu His Asn Tyr Ser Gln Ser Arg Asp His
    450                 455                 460

Asp Met Gly Thr Val Lys Tyr Glu Thr His Ser Asn Arg Asp Leu Ile
465                 470                 475                 480

Val Ala Arg Thr Asp Ile Thr Tyr Val Leu Asn Ser Leu Val Val Val
                485                 490                 495

Thr Thr Arg Leu Arg Lys Leu Val Ile Gly Phe Ser Gly Thr Phe Gln
            500                 505                 510

Ser Phe Ala Lys Val Leu Arg Asp Asp Ser Phe Val Lys Ala Arg Gly
        515                 520                 525

Glu Thr Ser Ile Glu Tyr Ala Tyr Arg Phe Leu Ser Asn Leu Ile Phe
    530                 535                 540

Gly Gly Leu Ile Asn Phe Tyr Asn Phe Leu Leu Asn Lys Asn Leu His
545                 550                 555                 560

Pro Asp Lys Val Ser Leu Ala Tyr Lys Arg Leu Ala Ala Leu Thr Leu
                565                 570                 575

Glu Leu Leu Ser Gly Thr Asn Lys Ala Pro Leu His Glu Ala Ala Val
            580                 585                 590

Asn Gly Ala Gly Ala Gly Ile Asp Cys Asp Gly Ala Ala Thr Ser Ala
        595                 600                 605

Asp Lys Ala Phe Cys Phe Thr Lys Ala Pro Glu Ser Lys Val Thr Ala
    610                 615                 620

Ser Ile Pro Glu Asp Pro Asp Asp Val Ile Phe Thr Ala Leu Asn Asp
625                 630                 635                 640

Glu Val Ile Asp Leu Val Tyr Cys Gln Tyr Glu Phe Ser Tyr Pro Lys
                645                 650                 655

Ser Ser Asn Glu Val His Ala Gln Phe Leu Leu Met Lys Ala Ile Tyr
            660                 665                 670

Asp Gly Arg Tyr Ala Ile Leu Ala Glu Leu Phe Glu Ser Ser Phe Thr
        675                 680                 685

Thr Ala Pro Phe Ser Ala Tyr Val Asp Asn Val Asn Phe Asn Gly Ser
    690                 695                 700

Glu Leu Leu Ile Gly Asn Val Arg Gly Gly Leu Leu Ser Leu Ala Leu
705                 710                 715                 720

Gln Thr Asp Thr Tyr Thr Leu Leu Gly Tyr Thr Phe Ala Pro Val Pro
                725                 730                 735

Val Phe Val Glu Glu Leu Thr Arg Lys Lys Leu Tyr Arg Glu Thr Thr
            740                 745                 750

Glu Met Leu Tyr Ala Leu His Val Pro Leu Met Val Leu Gln Asp Gln
        755                 760                 765

His Gly Phe Val Ser Ile Val Asn Ala Asn Val Cys Glu Phe Thr Glu
    770                 775                 780

Ser Ile Glu Asp Ala Glu Leu Ala Met Ala Thr Thr Val Asp Tyr Gly
785                 790                 795                 800

Leu Ser Ser Lys Leu Ala Met Thr Ile Ala Arg Ser Gln Gly Leu Ser
                805                 810                 815

Leu Glu Lys Val Ala Ile Cys Phe Thr Ala Asp Lys Leu Arg Leu Asn
            820                 825                 830

Ser Val Tyr Val Ala Met Ser Arg Thr Val Ser Ser Arg Phe Leu Lys
        835                 840                 845

Met Asn Leu Asn Pro Leu Arg Glu Arg Tyr Glu Lys Ser Ala Glu Ile
    850                 855                 860

Ser Asp His Ile Leu Ala Ala Leu Arg Asp Pro Asn Val His Val Val
865                 870                 875                 880

Tyr

<210>  37
<211>  278
<212>  PRT
<213>  Varicella zoster

<400>  37

Met Phe Cys Thr Ser Pro Ala Thr Arg Gly Asp Ser Ser Glu Ser Lys
1               5                   10                  15

Pro Gly Ala Ser Val Asp Val Asn Gly Lys Met Glu Tyr Gly Ser Ala
            20                  25                  30

Pro Gly Pro Leu Asn Gly Arg Asp Thr Ser Arg Gly Pro Gly Ala Phe
        35                  40                  45

Cys Thr Pro Gly Trp Glu Ile His Pro Ala Arg Leu Val Glu Asp Ile
    50                  55                  60

Asn Arg Val Phe Leu Cys Ile Ala Gln Ser Ser Gly Arg Val Thr Arg
65                  70                  75                  80

Asp Ser Arg Arg Leu Arg Arg Ile Cys Leu Asp Phe Tyr Leu Met Gly
                85                  90                  95

Arg Thr Arg Gln Arg Pro Thr Leu Ala Cys Trp Glu Glu Leu Leu Gln
            100                 105                 110

Leu Gln Pro Thr Gln Thr Gln Cys Leu Arg Ala Thr Leu Met Glu Val
        115                 120                 125

Ser His Arg Pro Pro Arg Gly Glu Asp Gly Phe Ile Glu Ala Pro Asn
    130                 135                 140

Val Pro Leu His Arg Ser Ala Leu Glu Cys Asp Val Ser Asp Asp Gly
145                 150                 155                 160

Gly Glu Asp Asp Ser Asp Asp Asp Gly Ser Thr Pro Ser Asp Val Ile

165                 170                 175

Glu Phe Arg Asp Ser Asp Ala Glu Ser Ser Asp Gly Glu Asp Phe Ile
            180                 185                 190

Val Glu Glu Glu Ser Glu Glu Ser Thr Asp Ser Cys Glu Pro Asp Gly
        195                 200                 205

Val Pro Gly Asp Cys Tyr Arg Asp Gly Asp Gly Cys Asn Thr Pro Ser
    210                 215                 220

Pro Lys Arg Pro Gln Arg Ala Ile Glu Arg Tyr Ala Gly Ala Glu Thr
225                 230                 235                 240

Ala Glu Tyr Thr Ala Ala Lys Ala Leu Thr Ala Leu Gly Glu Gly Gly
                245                 250                 255

Val Asp Trp Lys Arg Arg Arg His Glu Ala Pro Arg Arg His Asp Ile
            260                 265                 270

Pro Pro Pro His Gly Val
        275

<210>  38
<211>  180
<212>  PRT
<213>  Varicella zoster

<400>  38

Met Asn Leu Cys Gly Ser Arg Gly Glu His Pro Gly Gly Glu Tyr Ala
1               5                   10                  15

Gly Leu Tyr Cys Thr Arg His Asp Thr Pro Ala His Gln Ala Leu Met
            20                  25                  30

Asn Asp Ala Glu Arg Tyr Phe Ala Ala Ala Leu Cys Ala Ile Ser Thr
        35                  40                  45

Glu Ala Tyr Glu Ala Phe Ile His Ser Pro Ser Glu Arg Pro Cys Ala
    50                  55                  60

Ser Leu Trp Gly Arg Ala Lys Asp Ala Phe Gly Arg Met Cys Gly Glu
65                  70                  75                  80

Leu Ala Ala Asp Arg Gln Arg Pro Pro Ser Val Pro Pro Ile Arg Arg
                85                  90                  95

Ala Val Leu Ser Leu Leu Arg Glu Gln Cys Met Pro Asp Pro Gln Ser
            100                 105                 110

His Leu Glu Leu Ser Glu Arg Leu Ile Leu Met Ala Tyr Trp Cys Cys
        115                 120                 125

Leu Gly His Ala Gly Leu Pro Thr Ile Gly Leu Ser Pro Asp Asn Lys
    130                 135                 140

Cys Ile Arg Ala Glu Leu Tyr Asp Arg Pro Gly Gly Ile Cys His Arg
145                 150                 155                 160

Leu Phe Asp Ala Tyr Leu Gly Cys Gly Ser Leu Gly Val Pro Arg Thr
                165                 170                 175

Tyr Glu Arg Ser
            180

<210>  39
<211>  393
<212>  PRT
<213>  Varicella zoster

<400>  39

Met Asn Asp Val Asp Ala Thr Asp Thr Phe Val Gly Gln Gly Lys Phe
1               5                   10                  15

Arg Gly Ala Ile Ser Thr Ser Pro Ser His Ile Met Gln Thr Cys Gly
            20                  25                  30

Phe Ile Gln Gln Met Phe Pro Val Glu Met Ser Pro Gly Ile Glu Ser
        35                  40                  45

Glu Asp Asp Pro Asn Tyr Asp Val Asn Met Asp Ile Gln Ser Phe Asn
    50                  55                  60

Ile Phe Asp Gly Val His Glu Thr Glu Ala Glu Ala Ser Val Ala Leu
65                  70                  75                  80

Cys Ala Glu Ala Arg Val Gly Ile Asn Lys Ala Gly Phe Val Ile Leu
                85                  90                  95

Lys Thr Phe Thr Pro Gly Ala Glu Gly Phe Ala Phe Ala Cys Met Asp
            100                 105                 110

Ser Lys Thr Cys Glu His Val Val Ile Lys Ala Gly Gln Arg Gln Gly
        115                 120                 125

Thr Ala Thr Glu Ala Thr Val Leu Arg Ala Leu Thr His Pro Ser Val
    130                 135                 140

Val Gln Leu Lys Gly Thr Phe Thr Tyr Asn Lys Met Thr Cys Leu Ile
145                 150                 155                 160

Leu Pro Arg Tyr Arg Thr Asp Leu Tyr Cys Tyr Leu Ala Ala Lys Arg
                165                 170                 175

Asn Leu Pro Ile Cys Asp Ile Leu Ala Ile Gln Arg Ser Val Leu Arg
            180                 185                 190

Ala Leu Gln Tyr Leu His Asn Asn Ser Ile Ile His Arg Asp Ile Lys
        195                 200                 205

Ser Glu Asn Ile Phe Ile Asn His Pro Gly Asp Val Cys Val Gly Asp
    210                 215                 220

Phe Gly Ala Ala Cys Phe Pro Val Asp Ile Asn Ala Asn Arg Tyr Tyr
225                 230                 235                 240

Gly Trp Ala Gly Thr Ile Ala Thr Asn Ser Pro Glu Leu Leu Ala Arg
                245                 250                 255

Asp Pro Tyr Gly Pro Ala Val Asp Ile Trp Ser Ala Gly Ile Val Leu
            260                 265                 270

Phe Glu Met Ala Thr Gly Gln Asn Ser Leu Phe Glu Arg Asp Gly Leu
        275                 280                 285

Asp Gly Asn Cys Asp Ser Glu Arg Gln Ile Lys Leu Ile Ile Arg Arg
    290                 295                 300

Ser Gly Thr His Pro Asn Glu Phe Pro Ile Asn Pro Thr Ser Asn Leu
305                 310                 315                 320

Arg Arg Gln Tyr Ile Gly Leu Ala Lys Arg Ser Ser Arg Lys Pro Gly
                325                 330                 335

Ser Arg Pro Leu Trp Thr Asn Leu Tyr Glu Leu Pro Ile Asp Leu Glu
            340                 345                 350

Tyr Leu Ile Cys Lys Met Leu Ser Phe Asp Ala Arg His Arg Pro Ser
        355                 360                 365

Ala Glu Val Leu Leu Asn His Ser Val Phe Gln Thr Leu Pro Asp Pro
    370                 375                 380

Tyr Pro Asn Pro Met Glu Val Gly Asp
385                 390

<210>  40
<211>  354
<212>  PRT
<213>  Varicella zoster

<400>  40

Met Phe Leu Ile Gln Cys Leu Ile Ser Ala Val Ile Phe Tyr Ile Gln
1               5                   10                  15

Val Thr Asn Ala Leu Ile Phe Lys Gly Asp His Val Ser Leu Gln Val
            20                  25                  30

Asn Ser Ser Leu Thr Ser Ile Leu Ile Pro Met Gln Asn Asp Asn Tyr
        35                  40                  45

Thr Glu Ile Lys Gly Gln Leu Val Phe Ile Gly Glu Gln Leu Pro Thr
    50                  55                  60

Gly Thr Asn Tyr Ser Gly Thr Leu Glu Leu Leu Tyr Ala Asp Thr Val
65                  70                  75                  80

Ala Phe Cys Phe Arg Ser Val Gln Val Ile Arg Tyr Asp Gly Cys Pro
                85                  90                  95

Arg Ile Arg Thr Ser Ala Phe Ile Ser Cys Arg Tyr Lys His Ser Trp
            100                 105                 110

His Tyr Gly Asn Ser Thr Asp Arg Ile Ser Thr Glu Pro Asp Ala Gly
        115                 120                 125

Val Met Leu Lys Ile Thr Lys Pro Gly Ile Asn Asp Ala Gly Val Tyr
    130                 135                 140

Val Leu Leu Val Arg Leu Asp His Ser Arg Ser Thr Asp Gly Phe Ile
145                 150                 155                 160

Leu Gly Val Asn Val Tyr Thr Ala Gly Ser His His Asn Ile His Gly
                165                 170                 175

Val Ile Tyr Thr Ser Pro Ser Leu Gln Asn Gly Tyr Ser Thr Arg Ala
            180                 185                 190

Leu Phe Gln Gln Ala Arg Leu Cys Asp Leu Pro Ala Thr Pro Lys Gly
        195                 200                 205

Ser Gly Thr Ser Leu Phe Gln His Met Leu Asp Leu Arg Ala Gly Lys
    210                 215                 220

Ser Leu Glu Asp Asn Pro Trp Leu His Glu Asp Val Val Thr Thr Glu
225                 230                 235                 240

Thr Lys Ser Val Val Lys Glu Gly Ile Glu Asn His Val Tyr Pro Thr
                245                 250                 255

Asp Met Ser Thr Leu Pro Glu Lys Ser Leu Asn Asp Pro Pro Glu Asn
            260                 265                 270

Leu Leu Ile Ile Ile Pro Ile Val Ala Ser Val Met Ile Leu Thr Ala
        275                 280                 285

Met Val Ile Val Ile Val Ile Ser Val Lys Arg Arg Arg Ile Lys Lys
    290                 295                 300

His Pro Ile Tyr Arg Pro Asn Thr Lys Thr Arg Arg Gly Ile Gln Asn
305                 310                 315                 320

Ala Thr Pro Glu Ser Asp Val Met Leu Glu Ala Ala Ile Ala Gln Leu
                325                 330                 335

Ala Thr Ile Arg Glu Glu Ser Pro Pro His Ser Val Val Asn Pro Phe
            340                 345                 350

Val Lys

<210>  41
<211>  623
<212>  PRT
<213>  Varicella zoster

<400>  41

Met Gly Thr Val Asn Lys Pro Val Val Gly Val Leu Met Gly Phe Gly
1               5                   10                  15

Ile Ile Thr Gly Thr Leu Arg Ile Thr Asn Pro Val Arg Ala Ser Val
            20                  25                  30

Leu Arg Tyr Asp Asp Phe His Thr Asp Glu Asp Lys Leu Asp Thr Asn
        35                  40                  45

Ser Val Tyr Glu Pro Tyr Tyr His Ser Asp His Ala Glu Ser Ser Trp
    50                  55                  60

Val Asn Arg Gly Glu Ser Ser Arg Lys Ala Tyr Asp His Asn Ser Pro
65                  70                  75                  80

Tyr Ile Trp Pro Arg Asn Asp Tyr Asp Gly Phe Leu Glu Asn Ala His
                85                  90                  95

Glu His His Gly Val Tyr Asn Gln Gly Arg Gly Ile Asp Ser Gly Glu
            100                 105                 110

Arg Leu Met Gln Pro Thr Gln Met Ser Ala Gln Glu Asp Leu Gly Asp
        115                 120                 125

Asp Thr Gly Ile His Val Ile Pro Thr Leu Asn Gly Asp Asp Arg His
    130                 135                 140

Lys Ile Val Asn Val Asp Gln Arg Gln Tyr Gly Asp Val Phe Lys Gly
145                 150                 155                 160

Asp Leu Asn Pro Lys Pro Gln Gly Gln Arg Leu Ile Glu Val Ser Val

                165                 170                 175

Glu Glu Asn His Pro Phe Thr Leu Arg Ala Pro Ile Gln Arg Ile Tyr
            180                 185                 190

Gly Val Arg Tyr Thr Glu Thr Trp Ser Phe Leu Pro Ser Leu Thr Cys
        195                 200                 205

Thr Gly Asp Ala Ala Pro Ala Ile Gln His Ile Cys Leu Lys His Thr
    210                 215                 220

Thr Cys Phe Gln Asp Val Val Val Asp Val Asp Cys Ala Glu Asn Thr
225                 230                 235                 240

Lys Glu Asp Gln Leu Ala Glu Ile Ser Tyr Arg Phe Gln Gly Lys Lys
                245                 250                 255

Glu Ala Asp Gln Pro Trp Ile Val Val Asn Thr Ser Thr Leu Phe Asp
            260                 265                 270

Glu Leu Glu Leu Asp Pro Pro Glu Ile Glu Pro Gly Val Leu Lys Val
        275                 280                 285

Leu Arg Thr Glu Lys Gln Tyr Leu Gly Val Tyr Ile Trp Asn Met Arg
    290                 295                 300

Gly Ser Asp Gly Thr Ser Thr Tyr Ala Thr Phe Leu Val Thr Trp Lys
305                 310                 315                 320

Gly Asp Glu Lys Thr Arg Asn Pro Thr Pro Ala Val Thr Pro Gln Pro
                325                 330                 335

Arg Gly Ala Glu Phe His Met Trp Asn Tyr His Ser His Val Phe Ser
            340                 345                 350

Val Gly Asp Thr Phe Ser Leu Ala Met His Leu Gln Tyr Lys Ile His
        355                 360                 365

Glu Ala Pro Phe Asp Leu Leu Leu Glu Trp Leu Tyr Val Pro Ile Asp
    370                 375                 380

Pro Thr Cys Gln Pro Met Arg Leu Tyr Ser Thr Cys Leu Tyr His Pro
385                 390                 395                 400

Asn Ala Pro Gln Cys Leu Ser His Met Asn Ser Gly Cys Thr Phe Thr
                405                 410                 415

Ser Pro His Leu Ala Gln Arg Val Ala Ser Thr Val Tyr Gln Asn Cys
            420                 425                 430

Glu His Ala Asp Asn Tyr Thr Ala Tyr Cys Leu Gly Ile Ser His Met

435                 440                 445

Glu Pro Ser Phe Gly Leu Ile Leu His Asp Gly Gly Thr Thr Leu Lys
    450                 455                 460

Phe Val Asp Thr Pro Glu Ser Leu Ser Gly Leu Tyr Val Phe Val Val
465                 470                 475                 480

Tyr Phe Asn Gly His Val Glu Ala Val Ala Tyr Thr Val Val Ser Thr
                485                 490                 495

Val Asp His Phe Val Asn Ala Ile Glu Glu Arg Gly Phe Pro Pro Thr
            500                 505                 510

Ala Gly Gln Pro Pro Ala Thr Thr Lys Pro Lys Glu Ile Thr Pro Val
        515                 520                 525

Asn Pro Gly Thr Ser Pro Leu Leu Arg Tyr Ala Ala Trp Thr Gly Gly
    530                 535                 540

Leu Ala Ala Val Val Leu Leu Cys Leu Val Ile Phe Leu Ile Cys Thr
545                 550                 555                 560

Ala Lys Arg Met Arg Val Lys Ala Tyr Arg Val Asp Lys Ser Pro Tyr
                565                 570                 575

Asn Gln Ser Met Tyr Tyr Ala Gly Leu Pro Val Asp Asp Phe Glu Asp
            580                 585                 590

Ser Glu Ser Thr Asp Thr Glu Glu Glu Phe Gly Asn Ala Ile Gly Gly
        595                 600                 605

Ser His Gly Gly Ser Ser Tyr Thr Val Tyr Ile Asp Lys Thr Arg
    610                 615                 620

<210>  42
<211>  1310
<212>  PRT
<213>  Varicella zoster

<400>  42

Met Asp Thr Pro Pro Met Gln Arg Ser Thr Pro Gln Arg Ala Gly Ser
1               5                   10                  15

Pro Asp Thr Leu Glu Leu Met Asp Leu Leu Asp Ala Ala Ala Ala Ala
            20                  25                  30

Ala Glu His Arg Ala Arg Val Val Thr Ser Ser Gln Pro Asp Asp Leu
        35                  40                  45

Leu Phe Gly Glu Asn Gly Val Met Val Gly Arg Glu His Glu Ile Val
    50                  55                  60

Ser Ile Pro Ser Val Ser Gly Leu Gln Pro Glu Pro Arg Thr Glu Asp
65                  70                  75                  80

Val Gly Glu Glu Leu Thr Gln Asp Asp Tyr Val Cys Glu Asp Gly Gln
                85                  90                  95

Asp Leu Met Gly Ser Pro Val Ile Pro Leu Ala Glu Val Phe His Thr
            100                 105                 110

Arg Phe Ser Glu Ala Gly Ala Arg Glu Pro Thr Gly Ala Asp Arg Ser
        115                 120                 125

Leu Glu Thr Val Ser Leu Gly Thr Lys Leu Ala Arg Ser Pro Lys Pro
    130                 135                 140

Pro Met Asn Asp Gly Glu Thr Gly Arg Gly Thr Thr Pro Pro Phe Pro
145                 150                 155                 160

Gln Ala Phe Ser Pro Val Ser Pro Ala Ser Pro Val Gly Asp Ala Ala
                165                 170                 175

Gly Asn Asp Gln Arg Glu Asp Gln Arg Ser Ile Pro Arg Gln Thr Thr
            180                 185                 190

Arg Gly Asn Ser Pro Gly Leu Pro Ser Val Val His Arg Asp Arg Gln
        195                 200                 205

Thr Gln Ser Ile Ser Gly Lys Lys Pro Gly Asp Glu Gln Ala Gly His
    210                 215                 220

Ala His Ala Ser Gly Asp Gly Val Val Leu Gln Lys Thr Gln Arg Pro
225                 230                 235                 240

Ala Gln Gly Lys Ser Pro Lys Lys Lys Thr Leu Lys Val Lys Val Pro
                245                 250                 255

Leu Pro Ala Arg Lys Pro Gly Gly Pro Val Pro Gly Pro Val Glu Gln
            260                 265                 270

Leu Tyr His Val Leu Ser Asp Ser Val Pro Ala Lys Gly Ala Lys Ala
        275                 280                 285

Asp Leu Pro Phe Glu Thr Asp Asp Thr Arg Pro Arg Lys His Asp Ala
    290                 295                 300

Arg Gly Ile Thr Pro Arg Val Pro Gly Arg Ser Ser Gly Gly Lys Pro
305                 310                 315                 320

Arg Ala Phe Leu Ala Leu Pro Gly Arg Ser His Ala Pro Asp Pro Ile
                325                 330                 335

Glu Asp Asp Ser Pro Val Glu Lys Lys Pro Lys Ser Arg Glu Phe Val
            340                 345                 350

Ser Ser Ser Ser Ser Ser Ser Ser Trp Gly Ser Ser Ser Glu Asp Glu
        355                 360                 365

Asp Asp Glu Pro Arg Arg Val Ser Val Gly Ser Glu Thr Thr Gly Ser
    370                 375                 380

Arg Ser Gly Arg Glu His Ala Pro Ser Pro Ser Asn Ser Asp Asp Ser
385                 390                 395                 400

Asp Ser Asn Asp Gly Gly Ser Thr Lys Gln Asn Ile Gln Pro Gly Tyr
                405                 410                 415

Arg Ser Ile Ser Gly Pro Asp Pro Arg Ile Arg Lys Thr Lys Arg Leu
            420                 425                 430

Ala Gly Glu Pro Gly Arg Gln Arg Gln Lys Ser Phe Ser Leu Pro Arg
        435                 440                 445

Ser Arg Thr Pro Ile Ile Pro Pro Val Ser Gly Pro Leu Met Met Pro
    450                 455                 460

Asp Gly Ser Pro Trp Pro Gly Ser Ala Pro Leu Pro Ser Asn Arg Val
465                 470                 475                 480

Arg Phe Gly Pro Ser Gly Glu Thr Arg Glu Gly His Trp Glu Asp Glu
                485                 490                 495

Ala Val Arg Ala Ala Arg Ala Arg Tyr Glu Ala Ser Thr Glu Pro Val
            500                 505                 510

Pro Leu Tyr Val Pro Glu Leu Gly Asp Pro Ala Arg Gln Tyr Arg Ala
        515                 520                 525

Leu Ile Asn Leu Ile Tyr Cys Pro Asp Arg Asp Pro Ile Ala Trp Leu
    530                 535                 540

Gln Asn Pro Lys Leu Thr Gly Val Asn Ser Ala Leu Asn Gln Phe Tyr
545                 550                 555                 560

Gln Lys Leu Leu Pro Pro Gly Arg Ala Gly Thr Ala Val Thr Gly Ser
                565                 570                 575

Val Ala Ser Pro Val Pro His Val Gly Glu Ala Met Ala Thr Gly Glu
            580                 585                 590

Ala Leu Trp Ala Leu Pro His Ala Ala Ala Ala Val Ala Met Ser Arg
        595                 600                 605

Arg Tyr Asp Arg Ala Gln Lys His Phe Ile Leu Gln Ser Leu Arg Arg
    610                 615                 620

Ala Phe Ala Ser Met Ala Tyr Pro Glu Ala Thr Gly Ser Ser Pro Ala
625                 630                 635                 640

Ala Arg Ile Ser Arg Gly His Pro Ser Pro Thr Thr Pro Ala Thr Gln
                645                 650                 655

Ala Pro Asp Pro Gln Pro Ser Ala Ala Ala Arg Ser Leu Ser Val Cys
            660                 665                 670

Pro Pro Asp Asp Arg Leu Arg Thr Pro Arg Lys Arg Lys Ser Gln Pro
        675                 680                 685

Val Glu Ser Arg Ser Leu Leu Asp Lys Ile Arg Glu Thr Pro Val Ala
    690                 695                 700

Asp Ala Arg Val Ala Asp Asp His Val Val Ser Lys Ala Lys Arg Arg
705                 710                 715                 720

Val Ser Glu Pro Val Thr Ile Thr Ser Gly Pro Val Val Asp Pro Pro
                725                 730                 735

Ala Val Ile Thr Met Pro Leu Asp Gly Pro Ala Pro Asn Gly Gly Phe
            740                 745                 750

Arg Arg Ile Pro Arg Gly Ala Leu His Thr Pro Val Pro Ser Asp Gln
        755                 760                 765

Ala Arg Lys Ala Tyr Cys Thr Pro Glu Thr Ile Ala Arg Leu Val Asp
    770                 775                 780

Asp Pro Leu Phe Pro Thr Ala Trp Arg Pro Ala Leu Ser Phe Asp Pro
785                 790                 795                 800

Gly Ala Leu Ala Glu Ile Ala Ala Arg Arg Pro Gly Gly Gly Asp Arg
                805                 810                 815

Arg Phe Gly Pro Pro Ser Gly Val Glu Ala Leu Arg Arg Arg Cys Ala
            820                 825                 830

Trp Met Arg Gln Ile Pro Asp Pro Glu Asp Val Arg Leu Leu Ile Ile
        835                 840                 845

Tyr Asp Pro Leu Pro Gly Glu Asp Ile Asn Gly Pro Leu Glu Ser Thr
    850                 855                 860

Leu Ala Thr Asp Pro Gly Pro Ser Trp Ser Pro Ser Arg Gly Gly Leu
865                 870                 875                 880

Ser Val Val Leu Ala Ala Leu Ser Asn Arg Leu Cys Leu Pro Ser Thr
                885                 890                 895

His Ala Trp Ala Gly Asn Trp Thr Gly Pro Pro Asp Val Ser Ala Leu
            900                 905                 910

Asn Ala Arg Gly Val Leu Leu Leu Ser Thr Arg Asp Leu Ala Phe Ala
        915                 920                 925

Gly Ala Val Glu Tyr Leu Gly Ser Arg Leu Ala Ser Ala Arg Arg Arg
    930                 935                 940

Leu Leu Val Leu Asp Ala Val Ala Leu Glu Arg Trp Pro Arg Asp Gly
945                 950                 955                 960

Pro Ala Leu Ser Gln Tyr His Val Tyr Val Arg Ala Pro Ala Arg Pro
                965                 970                 975

Asp Ala Gln Ala Val Val Arg Trp Pro Asp Ser Ala Val Thr Glu Gly
            980                 985                 990

Leu Ala Arg Ala Val Phe Ala Ser  Ser Arg Thr Phe Gly  Pro Ala Ser
        995                 1000                1005

Phe Ala  Arg Ile Glu Thr Ala  Phe Ala Asn Leu Tyr  Pro Gly Glu
    1010                1015                1020

Gln Pro  Leu Cys Leu Cys Arg  Gly Gly Asn Val Ala  Tyr Thr Val
    1025                1030                1035

Cys Thr  Arg Ala Gly Pro Lys  Thr Arg Val Pro Leu  Ser Pro Arg
    1040                1045                1050

Glu Tyr  Arg Gln Tyr Val Leu  Pro Gly Phe Asp Gly  Cys Lys Asp
    1055                1060                1065

Leu Ala  Arg Gln Ser Arg Gly  Leu Gly Leu Gly Ala  Ala Asp Phe
    1070                1075                1080

Val Asp  Glu Ala Ala His Ser  His Arg Ala Ala Asn  Arg Trp Gly
    1085                1090                1095

Leu Gly  Ala Ala Leu Arg Pro  Val Phe Leu Pro Glu  Gly Arg Arg
    1100                1105                1110

Pro Gly  Ala Ala Gly Pro Glu  Ala Gly Asp Val Pro  Thr Trp Ala
    1115                1120                1125

Arg Val  Phe Cys Arg His Ala  Leu Leu Glu Pro Asp  Pro Ala Ala
    1130                1135                1140

Glu Pro Leu Val Leu Pro Pro Val Ala Gly Arg Ser Val Ala Leu
    1145                    1150                    1155

Tyr Ala Ser Ala Asp Glu Ala Arg Asn Ala Leu Pro Pro Ile Pro
    1160                    1165                    1170

Arg Val Met Trp Pro Pro Gly Phe Gly Ala Ala Glu Thr Val Leu
    1175                    1180                    1185

Glu Gly Ser Asp Gly Thr Arg Phe Val Phe Gly His His Gly Gly
    1190                    1195                    1200

Ser Glu Arg Pro Ser Glu Thr Gln Ala Gly Arg Gln Arg Arg Thr
    1205                    1210                    1215

Ala Asp Asp Arg Glu His Ala Leu Glu Leu Asp Asp Trp Glu Val
    1220                    1225                    1230

Gly Cys Glu Asp Ala Trp Asp Ser Glu Glu Gly Gly Gly Asp Asp
    1235                    1240                    1245

Gly Asp Ala Pro Gly Ser Ser Phe Gly Val Ser Ile Val Ser Val
    1250                    1255                    1260

Ala Pro Gly Val Leu Arg Asp Arg Arg Val Gly Leu Arg Pro Ala
    1265                    1270                    1275

Val Lys Val Glu Leu Leu Ser Ser Ser Ser Ser Ser Glu Asp Glu
    1280                    1285                    1290

Asp Asp Val Trp Gly Gly Arg Gly Gly Arg Ser Pro Pro Gln Ser
    1295                    1300                    1305

Arg Gly
    1310

<210>  43
<211>  1002
<212>  DNA
<213>  Varicella zoster

<220>
<221>  CDS
<222>  (1)..(1002)

<400>  43
atg cat tta aag cct acc aga ttt ttc cac gca aac caa ccg cca atg        48
Met His Leu Lys Pro Thr Arg Phe Phe His Ala Asn Gln Pro Pro Met
1               5                   10                  15

ccg cat tca tac gag atg gag gac tta tgc ttc gac gac atg caa tat        96
Pro His Ser Tyr Glu Met Glu Asp Leu Cys Phe Asp Asp Met Gln Tyr
            20                  25                  30

cgc tgg tct ccc tcg aac aca ccc tat cga agt atg tct agg cga tat 144
Arg Trp Ser Pro Ser Asn Thr Pro Tyr Arg Ser Met Ser Arg Arg Tyr
        35                  40                  45

aaa tcc gta tct cgg agc ggg cct tcg atg cgt gta cgc tcc aga acg 192
Lys Ser Val Ser Arg Ser Gly Pro Ser Met Arg Val Arg Ser Arg Thr
    50                  55                  60

cca tgc cgc cgt caa acc att cga gga aaa ctt atg tca aag gag cgg 240
Pro Cys Arg Arg Gln Thr Ile Arg Gly Lys Leu Met Ser Lys Glu Arg
65                  70                  75                  80

tct gtg tac cgc cat tat ttt aat tac atc gca agg tcc ccc cca gaa 288
Ser Val Tyr Arg His Tyr Phe Asn Tyr Ile Ala Arg Ser Pro Pro Glu
                85                  90                  95

gaa cta gct acc gtt aga ggc tta atc gtg cca att att aag acg acc 336
Glu Leu Ala Thr Val Arg Gly Leu Ile Val Pro Ile Ile Lys Thr Thr
            100                 105                 110

cct gtc acc ctt ccg ttt aac ttg ggt cag aca gtg gcg gat aac tgc 384
Pro Val Thr Leu Pro Phe Asn Leu Gly Gln Thr Val Ala Asp Asn Cys
        115                 120                 125

ctg tcg tta tcc gga atg ggt tat cat tta ggt ctc gga ggt tat tgt 432
Leu Ser Leu Ser Gly Met Gly Tyr His Leu Gly Leu Gly Gly Tyr Cys
    130                 135                 140

ccg aca tgc act gca tct gga gaa ccg cgt cta tgt cga acc gat cgg 480
Pro Thr Cys Thr Ala Ser Gly Glu Pro Arg Leu Cys Arg Thr Asp Arg
145                 150                 155                 160

gcg gct ctg ata cta gca tat gtt cag cag ctt aac aac ata tac gaa 528
Ala Ala Leu Ile Leu Ala Tyr Val Gln Gln Leu Asn Asn Ile Tyr Glu
                165                 170                 175

tat cgt gtg ttt ctt gca tcc att ttg gcg cta tca gac cga gcc aac 576
Tyr Arg Val Phe Leu Ala Ser Ile Leu Ala Leu Ser Asp Arg Ala Asn
            180                 185                 190

atg caa gca gcg tcc gct gaa ccc cta ttg tcg agc gta ttg gca caa 624
Met Gln Ala Ala Ser Ala Glu Pro Leu Leu Ser Ser Val Leu Ala Gln
        195                 200                 205

ccg gaa tta ttt ttt atg tat cat att atg agg gag ggg ggc atg cga 672
Pro Glu Leu Phe Phe Met Tyr His Ile Met Arg Glu Gly Gly Met Arg
    210                 215                 220

gat ata cgc gta ctt ttt tat cgt gat gga gat gcc gga ggg ttt atg 720
Asp Ile Arg Val Leu Phe Tyr Arg Asp Gly Asp Ala Gly Gly Phe Met
225                 230                 235                 240

atg tat gtt ata ttt ccg ggg aaa tct gtt cac ctc cat tac aga cta 768
Met Tyr Val Ile Phe Pro Gly Lys Ser Val His Leu His Tyr Arg Leu
                245                 250                 255

atc gat cat ata cag gcc gcg tgt cgg ggg tat aaa ata gtc gca cac 816
Ile Asp His Ile Gln Ala Ala Cys Arg Gly Tyr Lys Ile Val Ala His
            260                 265                 270

gtt tgg cag aca aca ttt tta ctg tcg gta tgt cgc aac cca gaa caa 864
Val Trp Gln Thr Thr Phe Leu Leu Ser Val Cys Arg Asn Pro Glu Gln
        275                 280                 285

caa aca gag act gtg gtg cca tcc att gga aca tcg gac gtt tac tgt 912
Gln Thr Glu Thr Val Val Pro Ser Ile Gly Thr Ser Asp Val Tyr Cys
    290                 295                 300

aaa atg tgt gac ctt aac ttt gat gga gaa ttg ctt ttg gaa tac aaa          960
Lys Met Cys Asp Leu Asn Phe Asp Gly Glu Leu Leu Leu Glu Tyr Lys
305                 310                 315                 320

aga ctc tac gca tta ttt gat gac ttt gtt cct cct cgg tga                  1002
Arg Leu Tyr Ala Leu Phe Asp Asp Phe Val Pro Pro Arg
                325                 330

<210>  44
<211>  333
<212>  PRT
<213>  Varicella zoster

<400>  44

Met His Leu Lys Pro Thr Arg Phe Phe His Ala Asn Gln Pro Pro Met
1               5                   10                  15

Pro His Ser Tyr Glu Met Glu Asp Leu Cys Phe Asp Asp Met Gln Tyr
            20                  25                  30

Arg Trp Ser Pro Ser Asn Thr Pro Tyr Arg Ser Met Ser Arg Arg Tyr
        35                  40                  45

Lys Ser Val Ser Arg Ser Gly Pro Ser Met Arg Val Arg Ser Arg Thr
    50                  55                  60

Pro Cys Arg Arg Gln Thr Ile Arg Gly Lys Leu Met Ser Lys Glu Arg
65                  70                  75                  80

Ser Val Tyr Arg His Tyr Phe Asn Tyr Ile Ala Arg Ser Pro Pro Glu
                85                  90                  95

Glu Leu Ala Thr Val Arg Gly Leu Ile Val Pro Ile Ile Lys Thr Thr
            100                 105                 110

Pro Val Thr Leu Pro Phe Asn Leu Gly Gln Thr Val Ala Asp Asn Cys
        115                 120                 125

Leu Ser Leu Ser Gly Met Gly Tyr His Leu Gly Leu Gly Gly Tyr Cys
    130                 135                 140

Pro Thr Cys Thr Ala Ser Gly Glu Pro Arg Leu Cys Arg Thr Asp Arg
145                 150                 155                 160

Ala Ala Leu Ile Leu Ala Tyr Val Gln Gln Leu Asn Asn Ile Tyr Glu
                165                 170                 175

Tyr Arg Val Phe Leu Ala Ser Ile Leu Ala Leu Ser Asp Arg Ala Asn
            180                 185                 190

Met Gln Ala Ala Ser Ala Glu Pro Leu Leu Ser Ser Val Leu Ala Gln
        195                 200                 205

Pro Glu Leu Phe Phe Met Tyr His Ile Met Arg Glu Gly Gly Met Arg
    210                 215                 220

Asp Ile Arg Val Leu Phe Tyr Arg Asp Gly Asp Ala Gly Gly Phe Met
225                 230                 235                 240

Met Tyr Val Ile Phe Pro Gly Lys Ser Val His Leu His Tyr Arg Leu
                245                 250                 255

Ile Asp His Ile Gln Ala Ala Cys Arg Gly Tyr Lys Ile Val Ala His
            260                 265                 270

Val Trp Gln Thr Thr Phe Leu Leu Ser Val Cys Arg Asn Pro Glu Gln
        275                 280                 285

Gln Thr Glu Thr Val Val Pro Ser Ile Gly Thr Ser Asp Val Tyr Cys
    290                 295                 300

Lys Met Cys Asp Leu Asn Phe Asp Gly Glu Leu Leu Leu Glu Tyr Lys
305                 310                 315                 320

Arg Leu Tyr Ala Leu Phe Asp Asp Phe Val Pro Pro Arg
                325                 330

<210>  45
<211>  1533
<212>  DNA
<213>  Varicella zoster

<220>
<221>  CDS
<222>  (1)..(1533)

<400>  45
atg gat gct gac gac aca ccc ccc aac ctc caa ata tct cca act gca      48
Met Asp Ala Asp Asp Thr Pro Pro Asn Leu Gln Ile Ser Pro Thr Ala
1               5                   10                  15

gga cct ttg cgt tcc cac cac aat acc gac gga cat gaa cca aat gca      96
Gly Pro Leu Arg Ser His His Asn Thr Asp Gly His Glu Pro Asn Ala
            20                  25                  30

acc gca gcc gat cag caa gaa cga gaa tcc acc aac ccc aca cac gga      144
Thr Ala Ala Asp Gln Gln Glu Arg Glu Ser Thr Asn Pro Thr His Gly
        35                  40                  45

tgt gta aat cat cca tgg gcc aat ccg tca act gca aca tgc atg gaa      192
Cys Val Asn His Pro Trp Ala Asn Pro Ser Thr Ala Thr Cys Met Glu
    50                  55                  60

tca cca gaa cga tca caa cag aca agc tta ttt tta tta aag cac ggc      240
Ser Pro Glu Arg Ser Gln Gln Thr Ser Leu Phe Leu Leu Lys His Gly
65                  70                  75                  80

tta acg aga gat cca ata cat caa cgc gaa agg gtg gac gtt ttt cca      288
Leu Thr Arg Asp Pro Ile His Gln Arg Glu Arg Val Asp Val Phe Pro
                85                  90                  95

caa ttt aac aaa ccc cca tgg gtt ttt aga att tcc aaa tta tcc cgt     336
Gln Phe Asn Lys Pro Pro Trp Val Phe Arg Ile Ser Lys Leu Ser Arg
            100                 105                 110

tta att gta ccc atc ttc acg ctc aat gaa cag tta tgt ttt tct aaa     384
Leu Ile Val Pro Ile Phe Thr Leu Asn Glu Gln Leu Cys Phe Ser Lys
        115                 120                 125

tta cag att cga gat aga ccc agg ttt gcg gga cgg gga acg tat ggg     432
Leu Gln Ile Arg Asp Arg Pro Arg Phe Ala Gly Arg Gly Thr Tyr Gly
    130                 135                 140

cgt gtt cat ata tac cca tcg tca aaa ata gct gta aaa acc atg gac     480
Arg Val His Ile Tyr Pro Ser Ser Lys Ile Ala Val Lys Thr Met Asp
145                 150                 155                 160

agt cgt gtt ttt aat aga gag tta att aac gcg att tta gcg agt gag     528
Ser Arg Val Phe Asn Arg Glu Leu Ile Asn Ala Ile Leu Ala Ser Glu
                165                 170                 175

ggt tct ata cga gca ggg gaa agg cta ggt att tct agc ata gtt tgc     576
Gly Ser Ile Arg Ala Gly Glu Arg Leu Gly Ile Ser Ser Ile Val Cys
            180                 185                 190

ctt tta ggt ttt tcg tta caa acc aaa cag cta ctg ttt ccg gca tac     624
Leu Leu Gly Phe Ser Leu Gln Thr Lys Gln Leu Leu Phe Pro Ala Tyr
        195                 200                 205

gac atg gat atg gat gaa tac att gtt cgc ctg tcc aga cgg ttg aca     672
Asp Met Asp Met Asp Glu Tyr Ile Val Arg Leu Ser Arg Arg Leu Thr
    210                 215                 220

ata cct gat cac ata gac aga aaa att gcc cat gta ttt tta gat ttg     720
Ile Pro Asp His Ile Asp Arg Lys Ile Ala His Val Phe Leu Asp Leu
225                 230                 235                 240

gct caa gcg ttg acg ttt tta aat cga acg tgc ggc ctg acc cac cta     768
Ala Gln Ala Leu Thr Phe Leu Asn Arg Thr Cys Gly Leu Thr His Leu
                245                 250                 255

gat gtg aaa tgt ggc aat att ttt ctt aac gtc gac aac ttt gcc tcg     816
Asp Val Lys Cys Gly Asn Ile Phe Leu Asn Val Asp Asn Phe Ala Ser
            260                 265                 270

ttg gaa ata acc aca gca gta atc gga gac tat agc cta gta aca tta     864
Leu Glu Ile Thr Thr Ala Val Ile Gly Asp Tyr Ser Leu Val Thr Leu
        275                 280                 285

aat acg tat tcc ctt tgt act cga gcg ata ttt gaa gtt gga aat cca     912
Asn Thr Tyr Ser Leu Cys Thr Arg Ala Ile Phe Glu Val Gly Asn Pro
    290                 295                 300

tcc cac ccg gag cac gta cta cgc gta ccc cgg gat gca tcg cag atg     960
Ser His Pro Glu His Val Leu Arg Val Pro Arg Asp Ala Ser Gln Met
305                 310                 315                 320

tca ttt cgt ttg gtg ttg agt cat gga aca aac caa ccc cct gaa atc    1008
Ser Phe Arg Leu Val Leu Ser His Gly Thr Asn Gln Pro Pro Glu Ile
                325                 330                 335

ttg ctt gat tat att aat gga acg ggc ctt act aaa tat act gga acc    1056
Leu Leu Asp Tyr Ile Asn Gly Thr Gly Leu Thr Lys Tyr Thr Gly Thr
            340                 345                 350

ttg ccc caa aga gtt gga ctt gcg att gat ctt tat gca ttg ggc caa    1104
Leu Pro Gln Arg Val Gly Leu Ala Ile Asp Leu Tyr Ala Leu Gly Gln
        355                 360                 365

gca ctc tta gaa gtt atc ctg cta gga cgt ctt ccc gga caa ctg ccc 1152
Ala Leu Leu Glu Val Ile Leu Leu Gly Arg Leu Pro Gly Gln Leu Pro
    370                 375                 380

att tca gta cat cgg acc ccg cat tat cac tac tac ggt cat aag tta 1200
Ile Ser Val His Arg Thr Pro His Tyr His Tyr Tyr Gly His Lys Leu
385                 390                 395                 400

tca cca gat ttg gcg ctt gat acg ctg gca tat cga tgt gtc ctg gcg 1248
Ser Pro Asp Leu Ala Leu Asp Thr Leu Ala Tyr Arg Cys Val Leu Ala
                405                 410                 415

cca tat ata ctc cca tct gac atc ccc ggg gac tta aat tat aat ccc 1296
Pro Tyr Ile Leu Pro Ser Asp Ile Pro Gly Asp Leu Asn Tyr Asn Pro
            420                 425                 430

ttt ata cac gcc gga gag ctg aac acc cgt att tcc cgg aat tct tta 1344
Phe Ile His Ala Gly Glu Leu Asn Thr Arg Ile Ser Arg Asn Ser Leu
        435                 440                 445

cgc cgg ata ttc cag tgt cac gca gtg cgt tac ggc gta acg cac tca 1392
Arg Arg Ile Phe Gln Cys His Ala Val Arg Tyr Gly Val Thr His Ser
    450                 455                 460

aag ctt ttc gaa ggc ata cgc att ccg gcc tca tta tac cca gcc act 1440
Lys Leu Phe Glu Gly Ile Arg Ile Pro Ala Ser Leu Tyr Pro Ala Thr
465                 470                 475                 480

gtt gtt aca tcg ttg ttg tgt cac gat aat tca gaa ata cgc tcg gat 1488
Val Val Thr Ser Leu Leu Cys His Asp Asn Ser Glu Ile Arg Ser Asp
                485                 490                 495

cac cct tta tta tgg cac gat cgg gat tgg ata gga tcg aca taa 1533
His Pro Leu Leu Trp His Asp Arg Asp Trp Ile Gly Ser Thr
            500                 505                 510

<210> 46
<211> 510
<212> PRT
<213> Varicella zoster

<400> 46

Met Asp Ala Asp Asp Thr Pro Pro Asn Leu Gln Ile Ser Pro Thr Ala
1               5                   10                  15

Gly Pro Leu Arg Ser His His Asn Thr Asp Gly His Glu Pro Asn Ala
            20                  25                  30

Thr Ala Ala Asp Gln Gln Glu Arg Glu Ser Thr Asn Pro Thr His Gly
        35                  40                  45

Cys Val Asn His Pro Trp Ala Asn Pro Ser Thr Ala Thr Cys Met Glu
    50                  55                  60

Ser Pro Glu Arg Ser Gln Gln Thr Ser Leu Phe Leu Leu Lys His Gly
65                  70                  75                  80

Leu Thr Arg Asp Pro Ile His Gln Arg Glu Arg Val Asp Val Phe Pro
                85                  90                  95

Gln Phe Asn Lys Pro Pro Trp Val Phe Arg Ile Ser Lys Leu Ser Arg
            100                 105                 110

Leu Ile Val Pro Ile Phe Thr Leu Asn Glu Gln Leu Cys Phe Ser Lys
        115                 120                 125

Leu Gln Ile Arg Asp Arg Pro Arg Phe Ala Gly Arg Gly Thr Tyr Gly
    130                 135                 140

Arg Val His Ile Tyr Pro Ser Ser Lys Ile Ala Val Lys Thr Met Asp
145                 150                 155                 160

Ser Arg Val Phe Asn Arg Glu Leu Ile Asn Ala Ile Leu Ala Ser Glu
                165                 170                 175

Gly Ser Ile Arg Ala Gly Glu Arg Leu Gly Ile Ser Ser Ile Val Cys
            180                 185                 190

Leu Leu Gly Phe Ser Leu Gln Thr Lys Gln Leu Leu Phe Pro Ala Tyr
        195                 200                 205

Asp Met Asp Met Asp Glu Tyr Ile Val Arg Leu Ser Arg Arg Leu Thr
    210                 215                 220

Ile Pro Asp His Ile Asp Arg Lys Ile Ala His Val Phe Leu Asp Leu
225                 230                 235                 240

Ala Gln Ala Leu Thr Phe Leu Asn Arg Thr Cys Gly Leu Thr His Leu
                245                 250                 255

Asp Val Lys Cys Gly Asn Ile Phe Leu Asn Val Asp Asn Phe Ala Ser
            260                 265                 270

Leu Glu Ile Thr Thr Ala Val Ile Gly Asp Tyr Ser Leu Val Thr Leu
        275                 280                 285

Asn Thr Tyr Ser Leu Cys Thr Arg Ala Ile Phe Glu Val Gly Asn Pro
    290                 295                 300

Ser His Pro Glu His Val Leu Arg Val Pro Arg Asp Ala Ser Gln Met
305                 310                 315                 320

Ser Phe Arg Leu Val Leu Ser His Gly Thr Asn Gln Pro Pro Glu Ile
                325                 330                 335

Leu Leu Asp Tyr Ile Asn Gly Thr Gly Leu Thr Lys Tyr Thr Gly Thr
            340                 345                 350

Leu Pro Gln Arg Val Gly Leu Ala Ile Asp Leu Tyr Ala Leu Gly Gln
        355                 360                 365

Ala Leu Leu Glu Val Ile Leu Leu Gly Arg Leu Pro Gly Gln Leu Pro
    370                 375                 380

Ile Ser Val His Arg Thr Pro His Tyr His Tyr Tyr Gly His Lys Leu
385                 390                 395                 400

Ser Pro Asp Leu Ala Leu Asp Thr Leu Ala Tyr Arg Cys Val Leu Ala
                405                 410                 415

Pro Tyr Ile Leu Pro Ser Asp Ile Pro Gly Asp Leu Asn Tyr Asn Pro
            420                 425                 430

Phe Ile His Ala Gly Glu Leu Asn Thr Arg Ile Ser Arg Asn Ser Leu
        435                 440                 445

Arg Arg Ile Phe Gln Cys His Ala Val Arg Tyr Gly Val Thr His Ser
    450                 455                 460

Lys Leu Phe Glu Gly Ile Arg Ile Pro Ala Ser Leu Tyr Pro Ala Thr
465                 470                 475                 480

Val Val Thr Ser Leu Leu Cys His Asp Asn Ser Glu Ile Arg Ser Asp
                485                 490                 495

His Pro Leu Leu Trp His Asp Arg Asp Trp Ile Gly Ser Thr
            500                 505                 510

<210>  47
<211>  246
<212>  DNA
<213>  Varicella zoster

<220>
<221>  CDS
<222>  (1)..(246)

<400>  47
atg gga caa tct tca tcc agc ggt cga gga gga atc tgt gga ttg tgc      48
Met Gly Gln Ser Ser Ser Ser Gly Arg Gly Gly Ile Cys Gly Leu Cys
1               5                   10                  15

aaa cgg tac aac gag ctt gtt acg tgc aac gga gaa acc gtt gct ttg      96
Lys Arg Tyr Asn Glu Leu Val Thr Cys Asn Gly Glu Thr Val Ala Leu
            20                  25                  30

aac tca gag ttc ttt gaa gac ttt gac ttt gat gag aat gta aca gag      144
Asn Ser Glu Phe Phe Glu Asp Phe Asp Phe Asp Glu Asn Val Thr Glu
        35                  40                  45

gac gcc gat aaa tcc aca caa cgc cgc cca cga gtg atc gat gta aca      192
Asp Ala Asp Lys Ser Thr Gln Arg Arg Pro Arg Val Ile Asp Val Thr
    50                  55                  60

cca aaa cga aaa cct tcg gga aag agc tcc cat tcc aaa tgc gca aaa      240
Pro Lys Arg Lys Pro Ser Gly Lys Ser Ser His Ser Lys Cys Ala Lys
65                  70                  75                  80

tgt taa                                                              246

Cys

<210> 48
<211> 81
<212> PRT
<213> Varicella zoster

<400> 48

Met Gly Gln Ser Ser Ser Ser Gly Arg Gly Gly Ile Cys Gly Leu Cys
1               5                   10                  15

Lys Arg Tyr Asn Glu Leu Val Thr Cys Asn Gly Glu Thr Val Ala Leu
            20                  25                  30

Asn Ser Glu Phe Phe Glu Asp Phe Asp Phe Asp Glu Asn Val Thr Glu
        35                  40                  45

Asp Ala Asp Lys Ser Thr Gln Arg Arg Pro Arg Val Ile Asp Val Thr
    50                  55                  60

Pro Lys Arg Lys Pro Ser Gly Lys Ser Ser His Ser Lys Cys Ala Lys
65                  70                  75                  80

Cys

<210> 49
<211> 735
<212> DNA
<213> Varicella zoster

<220>
<221> CDS
<222> (1)..(735)

<400> 49
atg aaa aat ccg cag aaa tta gcg atc aca ttc ttg ccg ctc tac gtg 48
Met Lys Asn Pro Gln Lys Leu Ala Ile Thr Phe Leu Pro Leu Tyr Val
1               5                   10                  15

atc cca acg tac acg ttg tgt att aaa gca ttg tat aaa aac acg cat 96
Ile Pro Thr Tyr Thr Leu Cys Ile Lys Ala Leu Tyr Lys Asn Thr His
            20                  25                  30

gcg ggc ttg ctg ttc tca ttt cta ggt ttt gtc tta aat aca ccc gcc 144
Ala Gly Leu Leu Phe Ser Phe Leu Gly Phe Val Leu Asn Thr Pro Ala
        35                  40                  45

atg agc atc tct gga ccc cca acg acg ttt att tta tat agg tta cat 192
Met Ser Ile Ser Gly Pro Pro Thr Thr Phe Ile Leu Tyr Arg Leu His
    50                  55                  60

ggg gtt agg cgg gtt ctt cac tgg act tta ccg gat cat gaa caa aca 240
Gly Val Arg Arg Val Leu His Trp Thr Leu Pro Asp His Glu Gln Thr
65                  70                  75                  80

ctc tac gca ttt acg ggt ggg tca aga tca atg gcg gtg aag acg gac 288
Leu Tyr Ala Phe Thr Gly Gly Ser Arg Ser Met Ala Val Lys Thr Asp

                    85                  90                  95

gct cga tgt gat aca atg agc ggt ggt atg atc gtc ctt caa cac acc        336
Ala Arg Cys Asp Thr Met Ser Gly Gly Met Ile Val Leu Gln His Thr
            100                 105                 110

cat aca gtg acc ctg cta acc ata gac tgt tct act gac ttt tca tca        384
His Thr Val Thr Leu Leu Thr Ile Asp Cys Ser Thr Asp Phe Ser Ser
        115                 120                 125

tac gca ttt acg cac cgg gat ttc cac tta cag gac aaa ccc cac gca        432
Tyr Ala Phe Thr His Arg Asp Phe His Leu Gln Asp Lys Pro His Ala
    130                 135                 140

aca ttt gcg atg ccg ttt atg tcc tgg gtc ggt tct gac cca aca tct        480
Thr Phe Ala Met Pro Phe Met Ser Trp Val Gly Ser Asp Pro Thr Ser
145                 150                 155                 160

cag ctg tac agt aat gtg ggg ggg gta cta tcc gta ata acg gaa gat        528
Gln Leu Tyr Ser Asn Val Gly Gly Val Leu Ser Val Ile Thr Glu Asp
                165                 170                 175

gac cta tcc atg tgt atc tca att gtt ata tac ggt tta cgg gta aac        576
Asp Leu Ser Met Cys Ile Ser Ile Val Ile Tyr Gly Leu Arg Val Asn
            180                 185                 190

aga cct gac gat cag acc aca cca aca cca acc ccg cac cag tat aca        624
Arg Pro Asp Asp Gln Thr Thr Pro Thr Pro Thr Pro His Gln Tyr Thr
        195                 200                 205

tcg caa agg cgg cag cct gaa acc aac tgt cct tct tca cca caa ccg        672
Ser Gln Arg Arg Gln Pro Glu Thr Asn Cys Pro Ser Ser Pro Gln Pro
    210                 215                 220

gcc ttt ttc aca tca gac gac gac gtt ctt tcg tta ata tta cgg gac        720
Ala Phe Phe Thr Ser Asp Asp Asp Val Leu Ser Leu Ile Leu Arg Asp
225                 230                 235                 240

gcc gca aac gcg taa                                                    735
Ala Ala Asn Ala

<210>  50
<211>  244
<212>  PRT
<213>  Varicella zoster

<400>  50

Met Lys Asn Pro Gln Lys Leu Ala Ile Thr Phe Leu Pro Leu Tyr Val
1               5                   10                  15

Ile Pro Thr Tyr Thr Leu Cys Ile Lys Ala Leu Tyr Lys Asn Thr His
            20                  25                  30

Ala Gly Leu Leu Phe Ser Phe Leu Gly Phe Val Leu Asn Thr Pro Ala
        35                  40                  45

Met Ser Ile Ser Gly Pro Pro Thr Thr Phe Ile Leu Tyr Arg Leu His
    50                  55                  60

Gly Val Arg Arg Val Leu His Trp Thr Leu Pro Asp His Glu Gln Thr
65                  70                  75                  80

Leu Tyr Ala Phe Thr Gly Gly Ser Arg Ser Met Ala Val Lys Thr Asp
                85                  90                  95

Ala Arg Cys Asp Thr Met Ser Gly Gly Met Ile Val Leu Gln His Thr
            100                 105                 110

His Thr Val Thr Leu Leu Thr Ile Asp Cys Ser Thr Asp Phe Ser Ser
        115                 120                 125

Tyr Ala Phe Thr His Arg Asp Phe His Leu Gln Asp Lys Pro His Ala
    130                 135                 140

Thr Phe Ala Met Pro Phe Met Ser Trp Val Gly Ser Asp Pro Thr Ser
145                 150                 155                 160

Gln Leu Tyr Ser Asn Val Gly Gly Val Leu Ser Val Ile Thr Glu Asp
                165                 170                 175

Asp Leu Ser Met Cys Ile Ser Ile Val Ile Tyr Gly Leu Arg Val Asn
            180                 185                 190

Arg Pro Asp Asp Gln Thr Thr Pro Thr Pro Thr Pro His Gln Tyr Thr
        195                 200                 205

Ser Gln Arg Arg Gln Pro Glu Thr Asn Cys Pro Ser Ser Pro Gln Pro
    210                 215                 220

Ala Phe Phe Thr Ser Asp Asp Asp Val Leu Ser Leu Ile Leu Arg Asp
225                 230                 235                 240

Ala Ala Asn Ala

<210>  51
<211>  124884
<212>  DNA
<213>  Varicella zoster

<220>
<221>  CDS
<222>  (5569)..(6405)

<220>
<221>  CDS
<222>  (6553)..(7095)

<220>
<221>  CDS
<222>  (12245)..(12553)

<220>
<221>  CDS
<222>  (15752)..(19684)

<220>
<221>  CDS
<222>  (20400)..(21803)

<220>
<221>  CDS
<222>  (23666)..(24583)

<220>
<221>  CDS
<222>  (25259)..(25474)

<220>
<221>  CDS
<222>  (31035)..(32030)

<220>
<221>  CDS
<222>  (54592)..(56217)

<220>
<221>  CDS
<222>  (60132)..(60908)

<220>
<221>  CDS
<222>  (60975)..(62714)

<220>
<221>  CDS
<222>  (62747)..(64564)

<220>
<221>  CDS
<222>  (74249)..(77833)

<220>
<221>  CDS
<222>  (80267)..(80737)

<220>
<221>  CDS
<222>  (80864)..(81673)

<220>
<221>  CDS
<222>  (81747)..(82454)

<220>
<221>  CDS
<222>  (94410)..(95861)

<220>
<221>  CDS
<222>  (96040)..(98367)

<220>
<221>  CDS
<222>  (98392)..(99312)

<220>
<221>  CDS
<222>  (101091)..(102317)

<220>
<221>  CDS
<222>  (102407)..(103627)

<220>
<221>  CDS
<222>  (103772)..(105454)

<220>
<221>  CDS
<222>  (114218)..(115408)

<220>
<221>  CDS
<222>  (116308)..(119559)

<220>
<221>  CDS
<222>  (119611)..(120633)

<220>
<221>  CDS
<222>  (120744)..(122102)

<220>
<221>  CDS
<222>  (122438)..(122977)

<220>
<221>  CDS
<222>  (123970)..(124296)

<400>  51
   cctctcccgg ggtccgccgg gcgcccagaa accggggggg ggttattttc gggggggggt      60
   ccgaccagcc cgcccgtcgc ccgcccgcac agacagacag acactttttt cataaaaacc     120
   gttccgcttt tattaacaac aaacagtccg cgcgccagtg gcgctcacga gaaaaggagg     180
   ggactccgtc acccccgact ctgcgggggg ctcctccccc cgcgccctcc ccacacatcg     240
   tcctcgtcct cggaggacga ggacgaggac aacagctcca ccttgaccgc cgggcgcaaa     300
   cccacccggc ggtctcgcag cacacccggg gccaccgaca cgatgctcac cccaaaggat     360
   gaccccggtg cgtccccgtc gtccccgccc ccctcctcgc tgtcccacgc gtcttcacac     420
   cccacctccc aatcgtccag ctccaaagcg tgttctctgt cgtctgcggt gcgccgctgt     480
   cgccccgcct gggtttctga cggccgttcc gagcccccgt ggtgtccgaa cacgaaccgt     540
   gttccgtcgc tcccctccaa caccgtctcc gcggccccaa aaccgggcgg ccacattact     600
   ctgggaatcg gggggagggc attccgagcc tcgtccgccg acgcatacag cgccaccgac     660
   cgaccggcca cgggtggaag cacgagtggt tctgcggcag ggtcgggttc cagcagggcg     720
   tggcggcaaa acaccctcgc ccaggtgggt acgtcgccgg cctccggccc ggcggccccc     780
   ggtctccgtc cctcgggaag gaagacgggt cgaagcgcgg cacccaggcc ccatcggttt     840
   gctgcgcggt ggctatgtgc cgcctcgtcc acaaagtcgg ctgccccgag ccccagaccc     900
   cgagactgtc gcgcgaggtc cttgcaaccg tcaaaacccg gcagcacgta ctgccggtat     960
   tcacggggcg acagggggac gcgggtcttg gggcccgcgc gggtacacac ggtgtatgcg    1020
   acgttcccac cgcggcacaa acacaggggt tgttcgcccg ggtacaggtt ggcaaacgca    1080
   gtctcgatac gagcaaaact cgctggccca aaggtgcgcg acgatgcaaa cacggcccgg    1140
   gcgagtcctt ctgtgaccgc cgagtctggc catcggacga cggcctgggc gtccggtcgc    1200
   gccggggccc ggacgtacac gtgatactga gacaaagcgg gtccatccct gggccacctc 1260
   tcgagggcca ccgcgtccaa caccagcaac cggcgccggg cagaggccaa ccgcgagcct 1320
   agatactcga cggccccggc aaaggccagg tctcgggtcg acagtaataa aacgccccgg 1380
   gcgttcaaag cggacacgtc cggcgggccg gtccagttcc cggcccaggc atgagtgctc 1440
   ggcaggcaca accggttact cagggctgcc aggaccacag acagtccccc tcgggatgga 1500
   ctccatgacg gtcccggatc tgtcgcgagg gtgctctcga gggggccgtt gatgtcctct 1560
   ccgggcaacg gatcgtagat gatcagaagc ctcacatcct ccgggtctgg gatctgccgc 1620
   atccaggcgc acctccgtcg cagcgcctcc actccgctgg gtggaccaaa ccgtcggtct 1680
   cctccgcccg gacgccgagc ggcgatttcc gccaaggcgc cgggatcaaa gcttagcgca 1740
   gggcgccagg ccgtgggaaa caatgggtcg tcgaccagac gggcgatggt ttcgggggta 1800
   cagtacgcct tgcgagcctg gtccgacggg accggggtat gcagggcccc ccggggaata 1860
   cgccgaaatc cccgtttggg ggccggtccg tcaagtggca tcgttattac ggcgggggga 1920
   tccaccacag ggcccgaggt gatggtcacg ggctcggata cccgcctctt ggccttggaa 1980
   accacatgat cgtctgcaac ccgggcgtcc gcgacgggtg tctccctaat cttgtcgagg 2040
   aggcttctgc tctcgactgg ctgggacttg cgcttgcgcg gagttcgtaa acgatcatcc 2100
   ggtggacaca cagaaagaga gcgtgcggcg gccgacggct gagggtcggg agcctgtgtg 2160
   gccggggttg ttggagaagg gtgaccgcgg gagatccgcg ccgccggact ggagcccgtt 2220
   gcctcggggt atgccatgct ggcaaaggct ctgcggagac tctgtaggat aaagtgtttt 2280
   tgggcccggt cgtatcgacg gctcatagcc acggccgcgg ccgcgtgggg gagagcccag 2340
   agggcctccc ccgtggccat ggcttcgcct acatgcggaa cgggagacgc tacgctcccc 2400
   gtaacggcgg tacccgcccg tcccggtggc aacagctttt ggtagaactg gttcagggcc 2460
   gagttgacac cggtcagctt ggggttctgg agccatgcta tagggtctct gtctggacag 2520
   tagatcaggt taatcagcgc gcggtactgt ctagccggat ctcccaactc cggcacgtaa 2580
   agcggcacgg gttccgttga ggcctcgtaa cgagcccgcg ccgctctcac agcctcatcc 2640
   tcccagtgac cctctctggt ctccccggac ggtccaaacc gcaccctgtt ggatgggagg 2700
   ggtgccgatc cgggccaagg gcttccgtcg ggcatcatga gcggccccga caccggggga 2760
   attatcgggg ttctggatcg cggcagggaa aatgatttct gtctctggcg ccccggttcc 2820
   cccgcaagac gtttggtctt acgaatcctc ggatcgggac cgctgatgga tcgatatccc 2880
   ggttggatat tttgtttcgt cgacccacca tcatttgagt ccgaatcatc cgaatttgac 2940
   ggggaagggg cgtgttcgcg tccggacctg ctgcctgtag tttcacttcc caccgaaacg 3000
   cgccggggtt catcgtcttc atcctccgat gacgatcccc acgacgagga agaggatgaa 3060
   gacgaaacaa actcacgact ctttggcttt ttctccactg ggctgtcatc ctcaatcggg 3120
   tctggtgcgt gggatcttcc cggcagggcc aaaaacgctc taggtttgcc ccccgacgaa 3180
   cgtccaggga cgcgaggtgt tataccccgg gcatcatgtt tccttgggcg ggtatcatcg 3240
   gtctcaaacg gcaggtccgc ctttgccccc ttagcgggaa cgctgtccga aaggacgtgg 3300
   tacaattgct caaccgggcc gggtacaggt ccaccgggtt tccgcgccgg gagtgggacc 3360
   ttaaccttca aagtcttttt cttcgggctc tttccctgag cgggccgttg agttttctgg 3420
   agaactactc cgtcccccga tgcatgcgca tgacccgctt gctcatcgcc cggcttttta 3480
   cccgagatgg actgagtttg tctgtctcga tggaccaccg acggcaaacc tggtgaattt 3540
   cctctcgtcg tttgtcgggg tatagaccgc tggtcttccc gttgatcgtt cccggcggcg 3600
   tctccaacag gagacgcggg ggatacaggg gagaaggcct gcgggaacgg aggggtcgta 3660
   cctctgcccg tttccccatc gttcatcggt ggttttggag acctagcaag cttcgttccg 3720
   agagagactg tctcaaggga gcgatcggct cctgttggtt ctcgcgcgcc ggcctccgag 3780
   aatcgggtgt ggaagacctc ggccagcggg attacaggcg agcccattag atcctgaccg 3840
   tcctcgcata cgtagtcgtc ttgtgttagc tcttcgccaa catcttccgt tctgggttct 3900
   ggttgaagtc ccgatacgga gggaattgaa acgatctcgt gttcccgtcc caccatgacc 3960
   ccgttctctc caaatagtag atcgtcaggc tgactcgagg tgaccacccg ggccctgtgt 4020
   tcggcggccg ccgcggccgc gtccaacagg tccattaact ccaaagtatc aggcgacccc 4080
   gcgcgttggg gtgtagagcg ctgcatcggc ggcgtatcca tcgcactggg gtgaatttag 4140
   acgtacccga gttttccaaa cgctctcgca gccttcaaag gattgcgatt gcggttggtg 4200
   agggagttcc aacagtactt aaaacgtgtt gtgccccccc ctcgaccgca tatttcctcc 4260
   ccgtgtcgtc accgtgtaaa tattcttaat gataagacga tgtagtgatt ggacgagact 4320
   cgaggcggga agttcatgga ccatagtatg cgtttaagga gagaccgctg gttggcgatg 4380
   tacgcccggt gtctatttcc gcatacctta caacatcata acaagggata ccagacatgt 4440
   gaatttcatt tacatatgtt taaataacaa ccaatcatcg tgtgtctaca gacgatatat 4500
   aatatacata aacacaattg gggttgtctc acatgcaaaa catcttatat aacacgggtt 4560
   gtttccaccc atccggcatc tagttaatca aatgcacgtc gacggtgtgt ttgggtccct 4620
   ctccgtcgtc attacgttcg cgcaatcaac aagcgtatac accaccaccc ctcccaacga 4680
   ttatgtcagg cggcacgaag cccgcgataa cccataaaat acacacgggg ttgtggtgtt 4740
   cacgtaaccc cccgccgatg gggagggggc gcggtacccc gccgatgggg agggggcgcg 4800
   gtaccccgcc gatggggagg gggcgcggta ccccgccgat ggggaggggg cgcggtaccc 4860
   cgccgatggg gagggggcgc ggtaccccgc cgatgtttat aaccataatt ctctaaaccg 4920
   ttgtagaaaa tcacaaaaaa atttattcaa aaacaagtcg aagaacttca tatctgaggc 4980
   atgtaaaccc gttcgcactt cctggggtgg aatggggtgg ggtggggggg tgaaaaaggg 5040
   ggggggttaa attgggcgtc cgcatgtctg tggtgtacgc caatcggata cactcttttg 5100
   atctgcattc gcacttcccg ttttttcact gtatgggttt tcatgttttg gcatgtgtcc 5160
   aaccaccgtt cgcactttct ttctatatat atatatatat atatatatat atatagagaa 5220
   agagagagag tttcttgttc gcgcgtgttc ccgcgatgtc gcggttttat ggggtgtggg 5280
   cgggcttttc acagaatata tatattccaa atggagcggc aggcttttta aaatcgattt 5340
   gacgtgataa aaaaaaacac acggggcccc cccctttttt tggtgttata aaggcaaccc 5400
   aatcgaaggt ctcccgcccc ggaatccccc attgccattt tacccaagta gccttattca 5460
   tagatgtaaa cgtttgggtg tgtgttttgt tgtgcagggt tcgtccgatt cataacgcga 5520
   cagcgtcgag tcggttttaa gggaaaaggt tactacggcc ccaaggac atg ttt tgc 5577
   Met Phe Cys
   1
   acc tca ccg gct acg cgg ggc gac tcg tcc gag tca aaa ccc ggg gca 5625
   Thr Ser Pro Ala Thr Arg Gly Asp Ser Ser Glu Ser Lys Pro Gly Ala
   5 10 15
   tcg gtt gat gtt aac gga aag atg gaa tat gga tct gca cca gga ccc 5673
   Ser Val Asp Val Asn Gly Lys Met Glu Tyr Gly Ser Ala Pro Gly Pro
   20 25 30 35
   ctg aac ggc cgg gat acg tcg cgg ggc ccc ggc gcg ttt tgt act ccg 5721
   Leu Asn Gly Arg Asp Thr Ser Arg Gly Pro Gly Ala Phe Cys Thr Pro
   40 45 50
   ggt tgg gag atc cac ccg gcc agg ctc gtt gag gac atc aac cgt gtt 5769
   Gly Trp Glu Ile His Pro Ala Arg Leu Val Glu Asp Ile Asn Arg Val
   55 60 65
   ttt tta tgt att gca cag tcg tcg gga cgc gtc acg cga gat tca cga 5817
   Phe Leu Cys Ile Ala Gln Ser Ser Gly Arg Val Thr Arg Asp Ser Arg
   70 75 80
   aga ttg cgg cgc ata tgc ctc gac ttt tat cta atg ggt cgc acc aga 5865
   Arg Leu Arg Arg Ile Cys Leu Asp Phe Tyr Leu Met Gly Arg Thr Arg
   85 90 95
   cag cgt ccc acg tta gcg tgc tgg gag gaa ttg tta cag ctt caa ccc 5913
   Gln Arg Pro Thr Leu Ala Cys Trp Glu Glu Leu Leu Gln Leu Gln Pro
   100 105 110 115
   acc cag acg cag tgc tta cgc gct act tta atg gaa gtg tcc cat cga 5961
   Thr Gln Thr Gln Cys Leu Arg Ala Thr Leu Met Glu Val Ser His Arg
   120 125 130
   ccc cct cgg ggg gaa gac ggg ttc att gag gcg ccg aat gtt cct ttg 6009
   Pro Pro Arg Gly Glu Asp Gly Phe Ile Glu Ala Pro Asn Val Pro Leu
   135 140 145
   cat agg agc gca ctg gaa tgt gac gta tct gat gat ggt ggt gaa gac 6057
   His Arg Ser Ala Leu Glu Cys Asp Val Ser Asp Asp Gly Gly Glu Asp
   150 155 160
   gat agc gac gat gat ggg tct acg cca tcg gat gta att gaa ttt cgg 6105
   Asp Ser Asp Asp Asp Gly Ser Thr Pro Ser Asp Val Ile Glu Phe Arg
   165 170 175
   gat tcc gac gcg gaa tca tcg gac ggg gaa gac ttt ata gtg gaa gaa 6153
   Asp Ser Asp Ala Glu Ser Ser Asp Gly Glu Asp Phe Ile Val Glu Glu
   180 185 190 195
   gaa tca gag gag agc acc gat tct tgt gaa cca gac ggg gta ccc ggc 6201
   Glu Ser Glu Glu Ser Thr Asp Ser Cys Glu Pro Asp Gly Val Pro Gly
   200 205 210
   gat tgt tat cga gac ggg gat ggg tgc aac acc ccg tcc cca aag aga 6249
   Asp Cys Tyr Arg Asp Gly Asp Gly Cys Asn Thr Pro Ser Pro Lys Arg
   215 220 225
   ccc cag cgt gcc atc gag cga tac gcg ggt gca gaa acc gcg gaa tat 6297
   Pro Gln Arg Ala Ile Glu Arg Tyr Ala Gly Ala Glu Thr Ala Glu Tyr
   230 235 240
   aca gcc gcg aaa gcg ctc acc gcg ttg ggc gag ggg ggt gta gat tgg 6345
   Thr Ala Ala Lys Ala Leu Thr Ala Leu Gly Glu Gly Gly Val Asp Trp
   245 250 255
   aag cga cgt cga cac gaa gcc ccg cgc cgg cat gat ata ccg ccc ccc 6393
   Lys Arg Arg Arg His Glu Ala Pro Arg Arg His Asp Ile Pro Pro Pro
   260 265 270 275
   cat ggc gtg tag tctttataaa taaatacaat ggtttggctc gtgtcttttt 6445
   His Gly Val
   ttgatgtctg tctgtggggg agtggggtgt tgtggatatt agagggtaga gggtgctggt 6505
   ttgaacgtct ccattaaccc acggggtccc cacacgggcc gtgtggt atg aat ctc 6561
   Met Asn Leu
   280
   tgc gga tcc cgc ggt gag cac ccg ggc ggt gaa tat gcc gga ctt tac 6609
   Cys Gly Ser Arg Gly Glu His Pro Gly Gly Glu Tyr Ala Gly Leu Tyr
   285 290 295
   tgc aca cga cac gat acc ccc gcg cac cag gct ctc atg aac gac gcc 6657
   Cys Thr Arg His Asp Thr Pro Ala His Gln Ala Leu Met Asn Asp Ala
   300 305 310
   gaa cgg tac ttc gcc gcc gcg cta tgc gcc ata tct acc gag gcc tac 6705
   Glu Arg Tyr Phe Ala Ala Ala Leu Cys Ala Ile Ser Thr Glu Ala Tyr
   315 320 325
   gag gct ttt ata cac agc ccc tcc gag aga ccg tgc gcg agt ttg tgg 6753
   Glu Ala Phe Ile His Ser Pro Ser Glu Arg Pro Cys Ala Ser Leu Trp
   330 335 340 345
   ggg agg gca aag gac gcc ttc gga cgg atg tgc ggg gag ctc gca gcg 6801
   Gly Arg Ala Lys Asp Ala Phe Gly Arg Met Cys Gly Glu Leu Ala Ala
   350 355 360
   gat aga caa cgt cca ccc tcg gtt ccg ccg atc cgc aga gcg gtg tta 6849
   Asp Arg Gln Arg Pro Pro Ser Val Pro Pro Ile Arg Arg Ala Val Leu
   365 370 375
   tcg tta tta cgc gag caa tgc atg ccg gat cca caa tcg cat ctg gag 6897
   Ser Leu Leu Arg Glu Gln Cys Met Pro Asp Pro Gln Ser His Leu Glu
   380 385 390
   ctc agc gag cgg ctg ata ttg atg gca tat tgg tgc tgt ttg gga cac 6945
   Leu Ser Glu Arg Leu Ile Leu Met Ala Tyr Trp Cys Cys Leu Gly His
   395 400 405
   gcc gga ctt ccg act att gga ttg tcg ccc gat aat aaa tgc atc cgc 6993
   Ala Gly Leu Pro Thr Ile Gly Leu Ser Pro Asp Asn Lys Cys Ile Arg
   410 415 420 425
   gcc gaa tta tat gac cgc ccc ggg gga att tgt cac agg ctt ttt gac 7041
   Ala Glu Leu Tyr Asp Arg Pro Gly Gly Ile Cys His Arg Leu Phe Asp
   430 435 440
   gcg tac ctg ggc tgc ggg tcc ctt gga gtc cca aga acc tac gag aga 7089
   Ala Tyr Leu Gly Cys Gly Ser Leu Gly Val Pro Arg Thr Tyr Glu Arg
   445 450 455
   tcc tga caccccatcc ctttatatag aaaaaaaaaa taaatttaaa acatacaccg 7145
   Ser
   gataaaagcg tactgttttt tatttaaatt tacacgctcg gcgttgcccc ggttcggtga 7205
   tcaccgggtc ttatctatat acaccgtgta actcgaaccc ccgtgactcc ctccaatcgc 7265
   gttaccaaac tcttcttccg tatccgtaga ttccgagtcc tcgaaatcgt ccactggaag 7325
   gccagcgtaa tacatgcttt ggttatacgg ggacttgtct accctatagg ctttaaccct 7385
   cattcgttta gccgtacaga ttaaaaatat tacgagacat aaaagtacta ctgctgcaag 7445
   ccctccggtc catgcggcat atcgtagaag tggtgacgtt ccggggttta cgggggtaat 7505
   ttccttgggt ttagtagtcg ccggtggctg accggccgtt ggcggaaatc cacgctcttc 7565
   aattgcgttt acaaaatgat ctactgtgga tacaacagtg tatgctacgg cttcaacatg 7625
   cccgttaaaa tacaccacaa aaacgtataa tcccgacaaa ctctcgggtg tatctacaaa 7685
   ctttaacgtg gtgcccccgt cgtgtaagat tagaccaaag ctaggctcca tatgagatat 7745
   tcccagacaa tatgcggtgt agttatctgc atgttcacaa ttttgataca ctgtgcttgc 7805
   aacacgctgg gctaaatgtg gcgaggtaaa tgtacaaccg gaattcatat gagagaggca 7865
   ttggggtgcg ttgggatgat acaaacacgt agaatataac cgcattggtt gacatgtagg 7925
   atcgatgggg acatacaacc actctaacag caaatcaaat ggcgcttcat gtatcttata 7985
   ctgaagatgc attgccaagc taaacgtatc accaactgaa aatacatgcg agtggtaatt 8045
   ccacatatga aactcagccc ctcttggttg aggagttact gcgggcgtag ggtttcttgt 8105
   tttttcatcc cctttccagg tgaccaaaaa cgtggcgtag gtagacgtac catcggagcc 8165
   gcgcatgttc caaatgtaca cacccaagta ttgtttttct gtccgaagta ctttcaagac 8225
   acccggttca atctcggggg ggtctaattc gagttcatca aacagtgtgc tcgtgtttac 8285
   aacaatccac ggttggtccg cttccttctt accttgaaaa cggtaactga tttcggccaa 8345
   ctgatcctct ttagtatttt ccgcgcaatc cacatccacc accacgtctt gaaagcatgt 8405
   tgtatgtttt aaacatatat gctggatggc gggcgctgcg tctcccgtac aggttaatga 8465
   cggcaaaaag ctccaagtct cggtgtaccg gactccataa atccgctgaa tcggtgcgcg 8525
   taaagtaaac gggtgatttt cttccactga cacctcaatg agtctttggc cttggggttt 8585
   tggattaaga tctcctttaa acacgtcacc gtattgacgt tggtccacat ttacaatttt 8645
   atgtctgtca tcgccgttta acgtagggat aacgtggatg cccgtatcgt ccccaagatc 8705
   ctcctgtgca gacatttgtg tgggttgcat taaccgttcc ccgctatcga taccacggcc 8765
   ctgattatac accccatggt gttcgtgtgc gttctctaaa aatccatcat aatcattacg 8825
   tggccatata taaggtgagt tatgatcgta cgcttttcgc gaagactctc cccgatttac 8885
   ccatgaagac tccgcatgat ctgaatggta gtaaggctca tatacggagt ttgtatccag 8945
   tttgtcttca tcggtgtgaa aatcatcgta tcgcaagacg gatgctctga ccggattcgt 9005
   tatacgcaac gttcccgtga taattccgaa ccccatcaat acccccacca caggtttatt 9065
   aactgtcccc atattacagg cgaccctcgg aatatagtca cttaaaacca cgcaattcgc 9125
   ccgaggtcta aaaccatgga ctgctctcgt gtataccagc tcggccttta aggcttcata 9185
   aaacatcccc gcccaaatgt gggcgtgtta acttatagtt aggagattct taatccttcc 9245
   cctcatatca caacgcgtaa tcaaaacgct aaataaaacg catatagttt atttaatata 9305
   aaatccggga taattagttc tatttaacaa acgggtttac aacggaatgt gggggggatt 9365
   cttcgcgaat cgttgctagt tgtgcaatgg cggcctccaa catcacatcg gattctggtg 9425
   tcgcattttg tatgcccctt cttgtttttg tatttgggcg ataaattgga tgttttttaa 9485
   ttctacgtcg cttaacgctt attacaataa caataaccat ggcggtgagg atcatgacag 9545
   acgctactat aggaataatt ataagtagat tttctggagg atcattaagg gacttttcgg 9605
   gtaacgtgga catatccgtt ggatatacgt gattttctat cccctcctta acaacggact 9665
   tagtttctgt cgtaacaacg tcctcatgta accaagggtt atcctctaac gatttaccgg 9725
   cacgaagatc aagcatatgt tgaaacaggg aggtaccgga ccctttgggt gtcgcgggta 9785
   aatcacacaa acgagcttgt tgaaaaaggg ctcttgtaga atatccattc tgtagagacg 9845
   gagaagtgta gataaccccg tgaatgttgt gatgcgagcc cgctgtatat acatttacac 9905
   caagaatgaa accatcggtg gatctgctat ggtctaaccg aacaagaagt acatacacac 9965
   cagcatcatt tattcccggt ttggtaattt tcaacattac accagcatcc ggctctgttg 10025
   atatccgatc cgttgagtta ccataatgcc acgaatgttt gtacctacac gaaataaaag 10085
   cgctcgttct aatccgggga catccgtcgt atcttattac ttgtactgac cggaaacaaa 10145
   acgccaccgt atccgcgtat aacagttcca gtgttccgct atagtttgtc ccggtaggta 10205
   gttgctctcc aataaagaca agctgtcctt ttatctctgt ataattatca ttttgcatgg 10265
   gaataaggat agacgtgaga ctgctgttaa cttgcaagct cacgtggtcg cccttgaaga 10325
   tcaaagcgtt ggtcacttgt atgtaaaata taacggccga tatcaaacat tggattaaaa 10385
   acatcgcgat taaatgaggc gcaccagtat acccaactac accgaagagc agctctgtga 10445
   gccgggggag gggacgcttt tcagcgctac tacaatgtta agaaaatgat cccgtgtttt 10505
   atccttgttg taatggggcg tgactctgta ttaagtttct attggttatg ttatttatat 10565
   tagtatttca aaacattgcg cgtttgtacc ctatttgcct taacgggtta tacgttataa 10625
   acacaattta tacaatattt tattaacagg cttaatgaat tttaatctcc aacttccatt 10685
   ggatttggat atggatcggg aagagtttgg aaaacagagt ggttaagcaa cacctctgct 10745
   gatggtcgat gacgtgcgtc aaacgataac atcttacata tcaaatactc caaatcaatt 10805
   ggcaactcat atagatttgt ccacaatggc ctggatccgg gttttcgaga agaccgtttt 10865
   gccaaaccaa tgtattgtcg acgaagattt gatgtagggt taatggaaaa ttcattggga 10925
   tgagttccag atcgtcgtat aataagttta atttgacgct cactgtcaca attgccatct 10985
   aaaccgtctc gttcaaataa cgagttctgt cctgtagcca tttcaaataa tacaatcccg 11045
   gcactccata tgtccacggc aggtccatat ggatctctag ccaataactc aggagagttt 11105
   gtggcgattg ttccagccca gccataatac ctgttggcat taatatccac ggggaaacac 11165
   gctgctccaa agtctcccac acaaacatca cctgggtggt taataaatat attttcagat 11225
   tttatatcac ggtgaataat actgttatta tgaagatact gtaacgcgcg taatacagat 11285
   cgctgaattg ctaaaatgtc acatatgggg aggttgcgct ttgcagctag atagcagtat 11345
   aaatctgttc ggtaacgtgg taatataaga catgtcattt tgttatacgt aaacgttcct 11405
   ttaagctgta caacggatgg gtgggttaac gctcttaaca cggttgcctc ggtggccgtt 11465
   ccttgacgtt gacccgcttt aatgaccaca tgttcacatg ttttactgtc catacacgca 11525
   aacgcaaaac cttccgcccc tggtgtaaac gtttttaata ttacaaatcc cgctttatta 11585
   attccaacgc gtgcttctgc gcacaatgcc acagaggctt cggcttcagt ttcgtgtaca 11645
   ccatcaaata tattaaaaga ctgtatatcc atgttaacgt cataattggg atcatcctca 11705
   gattctatgc cgggcgacat ttcaactgga aacatctgtt gtataaaccc acatgtttgc 11765
   ataatatgtg acggtgatgt tgagatggcg ccacggaact ttccttgtcc aacaaaggtg 11825
   tctgttgcat caacgtcgtt cattatactt gtataccccg acttatgttg atagtgttaa 11885
   tgaaccaaaa acctgttgtc caacaactgt acaaaatata ctcaccgaca cacttataaa 11945
   gtgatccgat tataagaggg cggagataat gacaaaaagg ggaggggtta aacataactt 12005
   acaaatatgg atttgattgt tcctaggatt attaataatg tctacatgtt ttatttaata 12065
   ataacgtaag gtaactttac ctctttgtga gaatgtcggt atagctgaca acgtttatga 12125
   aaacgcgctt taaacgacct attgatgaat aatctagaat cttgcatgta tataaatatc 12185
   aaaaggtgtg gtattaaaca cttttaaact agcctgctgg ccttacaatc tggatttct 12244
   atg gcc gga caa aac acc atg gag ggt gag gcc gtg gcc tta ctg atg 12292
   Met Ala Gly Gln Asn Thr Met Glu Gly Glu Ala Val Ala Leu Leu Met
   460 465 470
   gaa gcg gtg gta acc ccg cga gcg caa cca aat aat aca acg ata aca 12340
   Glu Ala Val Val Thr Pro Arg Ala Gln Pro Asn Asn Thr Thr Ile Thr
   475 480 485 490
   gcc ata caa ccg agc cgt tcg gct gaa aag tgt tat tat agt gat agc 12388
   Ala Ile Gln Pro Ser Arg Ser Ala Glu Lys Cys Tyr Tyr Ser Asp Ser
   495 500 505
   gaa aat gaa acg gca gat gaa ttt ttg cgt cga att gga aaa tat caa 12436
   Glu Asn Glu Thr Ala Asp Glu Phe Leu Arg Arg Ile Gly Lys Tyr Gln
   510 515 520
   cat aaa ata tat cat cgt aaa aaa ttc tgt tat att acg cta ata att 12484
   His Lys Ile Tyr His Arg Lys Lys Phe Cys Tyr Ile Thr Leu Ile Ile
   525 530 535
   gtc ttt gta ttt gct atg acg gga gcg gcc ttt gcc ttg gga tat ata 12532
   Val Phe Val Phe Ala Met Thr Gly Ala Ala Phe Ala Leu Gly Tyr Ile
   540 545 550
   acg tca caa ttt gtt gga taa gtggacgatt tcgaggactc ggaatctacg 12583
   Thr Ser Gln Phe Val Gly
   555 560
   gatacggaag aagagtttgg taacgcgatt ggagggagtc acgggggttc gagttacacg 12643
   gtgtatatag ataagacccg gtgatcaccg aaccggggca acgccgagcg tgtaaattta 12703
   aataaaaaac agtacgcttt tatccggtgt atgttttaaa tttatttttt ttttctatat 12763
   aaagggatgg ggtgtcagga tctctcgtag gttcttggga ctccaaggga cccgcagccc 12823
   aggtacgcgt caaaaagcct gtgacaaatt cccccggggc ggtcatataa ttcggcgcgg 12883
   atgcatttat tatcgggcga caatccaata gtcggaagtc cggcgtgtcc caaacagcac 12943
   caatatgcca tcaatatcag ccgctcgctg agctccagat gcgattgtgg atccggcatg 13003
   cattgctcgc gtaataacga taacaccgct ctgcggatcg gcggaaccga gggtggacgt 13063
   tgtctatccg ctgcgagctc cccgcacatc cgtccgaagg cgtcctttgc cctcccccac 13123
   aaactcgcgc acggtctctc ggaggggctg tgtataaaag cctcgtaggc ctcggtagat 13183
   atggcgcata gcgcggcggc gaagtaccgt tcggcgtcgt tcatgagagc ctggtgcgcg 13243
   ggggtatcgt gtcgtgtgca gtaaagtccg gcatattcac cgcccgggtg ctcaccgcgg 13303
   gatccgcaga gattcatacc acacggcccg tgtggggacc ccgtgggtta atggagacgt 13363
   tcaaaccagc accctctacc ctctaatatc cacaacaccc cactccccca cagacagaca 13423
   tcaaaaaaag acacgagcca aaccattgta tttatttata aagactacac gccatggggg 13483
   ggcggtatat catgccggcg cggggcttcg tgtcgacgtc gcttccaatc tacacccccc 13543
   tcgcccaacg cggtgagcgc tttcgcggct gtatattccg cggtttctgc acccgcgtat 13603
   cgctcgatgg cacgctgggg tctctttggg gacggggtgt tgcacccatc cccgtctcga 13663
   taacaatcgc cgggtacccc gtctggttca caagaatcgg tgctctcctc tgattcttct 13723
   tccactataa agtcttcccc gtccgatgat tccgcgtcgg aatcccgaaa ttcaattaca 13783
   tccgatggcg tagacccatc atcgtcgcta tcgtcttcac caccatcatc agatacgtca 13843
   cattccagtg cgctcctatg caaaggaaca ttcggcgcct caatgaaccc gtcttccccc 13903
   cgagggggtc gatgggacac ttccattaaa gtagcgcgta agcactgcgt ctgggtgggt 13963
   tgaagctgta acaattcctc ccagcacgct aacgtgggac gctgtctggt gcgacccatt 14023
   agataaaagt cgaggcatat gcgccgcaat cttcgtgaat ctcgcgtgac gcgtcccgac 14083
   gactgtgcaa tacataaaaa aacacggttg atgtcctcaa cgagcctggc cgggtggatc 14143
   tcccaacccg gagtacaaaa cgcgccgggg ccccgcgacg tatcccggcc gttcaggggt 14203
   cctggtgcag atccatattc catctttccg ttaacatcaa ccgatgcccc gggttttgac 14263
   tcggacgagt cgccccgcgt agccggtgag gtgcaaaaca tgtccttggg gccgtagtaa 14323
   ccttttccct taaaaccgac tcgacgctgt cgcgttatga atcggacgaa ccctgcacaa 14383
   caaaacacac acccaaacgt ttacatctat gaataaggct acttgggtaa aatggcaatg 14443
   ggggattccg gggcgggaga ccttcgattg ggttgccttt ataacaccaa aaaaaggggg 14503
   gggccccgtg tgtttttttt tatcacgtca aatcgatttt aaaaagcctg ccgctccatt 14563
   tggaatatat atattctgtg aaaagcccgc ccacacccca taaaaccgcg acatcgcggg 14623
   aacacgcgcg aacaagaaac tctctctctt tctctatata tatatatata tatatatata 14683
   tatatagaaa gaaagtgcga acggtggttg gacacatgcc aaaacatgaa aacccataca 14743
   gtgaaaaaac gggaagtgcg aatgcagatc aaaagagtgt atccgattgg cgtacaccac 14803
   agacatgcgg acgcccaatt taaccccccc cctttttcac ccccccaccc caccccattc 14863
   caccccagga agtgcgaacg ggtttacatg cctcagatat gaagttcttc gacttgtttt 14923
   tgaataaatt tttttgtgat tttctacaac ggtttagaga attatggtta taaacatcgg 14983
   cggggtaccg cgccccctcc ccatcggcgg ggtaccgcgc cccctcccca tcggcggggt 15043
   accgcgcccc ctccccatcg gcggggtacc gcgccccctc cccatcggcg gggtaccgcg 15103
   ccccctcccc atcggcgggg ggttacgtga acaccacaac cccgtgtgta ttttatgggt 15163
   tatcgcgggc ttcgtgccgc ctgacataat cgttgggagg ggtggtggtg tatacgcttg 15223
   ttgattgcgc gaacgtaatg acgacggaga gggacccaaa cacaccgtcg acgtgcattt 15283
   gattaactag atgccggatg ggtggaaaca acccgtgtta tataagatgt tttgcatgtg 15343
   agacaacccc aattgtgttt atgtatatta tatatcgtct gtagacacac gatgattggt 15403
   tgttatttaa acatatgtaa atgaaattca catgtctggt atcccttgtt atgatgttgt 15463
   aaggtatgcg gaaatagaca ccgggcgtac atcgccaacc agcggtctct ccttaaacgc 15523
   atactatggt ccatgaactt cccgcctcga gtctcgtcca atcactacat cgtcttatca 15583
   ttaagaatat ttacacggtg acgacacggg gaggaaatat gcggtcgagg ggggggcaca 15643
   acacgtttta agtactgttg gaactccctc accaaccgca atcgcaatcc tttgaaggct 15703
   gcgagagcgt ttggaaaact cgggtacgtc taaattcacc ccagtgcg atg gat acg 15760
   Met Asp Thr
   ccg ccg atg cag cgc tct aca ccc caa cgc gcg ggg tcg cct gat act 15808
   Pro Pro Met Gln Arg Ser Thr Pro Gln Arg Ala Gly Ser Pro Asp Thr
   565 570 575
   ttg gag tta atg gac ctg ttg gac gcg gcc gcg gcg gcc gcc gaa cac 15856
   Leu Glu Leu Met Asp Leu Leu Asp Ala Ala Ala Ala Ala Ala Glu His
   580 585 590 595
   agg gcc cgg gtg gtc acc tcg agt cag cct gac gat cta cta ttt gga 15904
   Arg Ala Arg Val Val Thr Ser Ser Gln Pro Asp Asp Leu Leu Phe Gly
   600 605 610
   gag aac ggg gtc atg gtg gga cgg gaa cac gag atc gtt tca att ccc 15952
   Glu Asn Gly Val Met Val Gly Arg Glu His Glu Ile Val Ser Ile Pro
   615 620 625
   tcc gta tcg gga ctt caa cca gaa ccc aga acg gaa gat gtt ggc gaa 16000
   Ser Val Ser Gly Leu Gln Pro Glu Pro Arg Thr Glu Asp Val Gly Glu
   630 635 640
   gag cta aca caa gac gac tac gta tgc gag gac ggt cag gat cta atg 16048
   Glu Leu Thr Gln Asp Asp Tyr Val Cys Glu Asp Gly Gln Asp Leu Met
   645 650 655
   ggc tcg cct gta atc ccg ctg gcc gag gtc ttc cac acc cga ttc tcg 16096
   Gly Ser Pro Val Ile Pro Leu Ala Glu Val Phe His Thr Arg Phe Ser
   660 665 670 675
   gag gcc ggc gcg cga gaa cca aca gga gcc gat cgc tcc ctt gag aca 16144
   Glu Ala Gly Ala Arg Glu Pro Thr Gly Ala Asp Arg Ser Leu Glu Thr
   680 685 690
   gtc tct ctc gga acg aag ctt gct agg tct cca aaa cca ccg atg aac     16192
   Val Ser Leu Gly Thr Lys Leu Ala Arg Ser Pro Lys Pro Pro Met Asn
               695                 700                 705
   gat ggg gaa acg ggc aga ggt acg acc cct ccg ttc ccg cag gcc ttc     16240
   Asp Gly Glu Thr Gly Arg Gly Thr Thr Pro Pro Phe Pro Gln Ala Phe
           710                 715                 720
   tcc cct gta tcc ccc gcg tct cct gtt gga gac gcc gcc ggg aac gat     16288
   Ser Pro Val Ser Pro Ala Ser Pro Val Gly Asp Ala Ala Gly Asn Asp
       725                 730                 735
   caa cgg gaa gac cag cgg tct ata ccc cga caa acg acg aga gga aat     16336
   Gln Arg Glu Asp Gln Arg Ser Ile Pro Arg Gln Thr Thr Arg Gly Asn
   740                 745                 750                 755
   tca cca ggt ttg ccg tcg gtg gtc cat cga gac aga caa act cag tcc     16384
   Ser Pro Gly Leu Pro Ser Val Val His Arg Asp Arg Gln Thr Gln Ser
                   760                 765                 770
   atc tcg ggt aaa aag ccg ggc gat gag caa gcg ggt cat gcg cat gca     16432
   Ile Ser Gly Lys Lys Pro Gly Asp Glu Gln Ala Gly His Ala His Ala
               775                 780                 785
   tcg ggg gac gga gta gtt ctc cag aaa act caa cgg ccc gct cag gga     16480
   Ser Gly Asp Gly Val Val Leu Gln Lys Thr Gln Arg Pro Ala Gln Gly
           790                 795                 800
   aag agc ccg aag aaa aag act ttg aag gtt aag gtc cca ctc ccg gcg     16528
   Lys Ser Pro Lys Lys Lys Thr Leu Lys Val Lys Val Pro Leu Pro Ala
       805                 810                 815
   cgg aaa ccc ggt gga cct gta ccc ggc ccg gtt gag caa ttg tac cac     16576
   Arg Lys Pro Gly Gly Pro Val Pro Gly Pro Val Glu Gln Leu Tyr His
   820                 825                 830                 835
   gtc ctt tcg gac agc gtt ccc gct aag ggg gca aag gcg gac ctg ccg     16624
   Val Leu Ser Asp Ser Val Pro Ala Lys Gly Ala Lys Ala Asp Leu Pro
                   840                 845                 850
   ttt gag acc gat gat acc cgc cca agg aaa cat gat gcc cgg ggt ata     16672
   Phe Glu Thr Asp Asp Thr Arg Pro Arg Lys His Asp Ala Arg Gly Ile
               855                 860                 865
   aca cct cgc gtc cct gga cgt tcg tcg ggg ggc aaa cct aga gcg ttt     16720
   Thr Pro Arg Val Pro Gly Arg Ser Ser Gly Gly Lys Pro Arg Ala Phe
           870                 875                 880
   ttg gcc ctg ccg gga aga tcc cac gca cca gac ccg att gag gat gac     16768
   Leu Ala Leu Pro Gly Arg Ser His Ala Pro Asp Pro Ile Glu Asp Asp
       885                 890                 895
   agc cca gtg gag aaa aag cca aag agt cgt gag ttt gtt tcg tct tca     16816
   Ser Pro Val Glu Lys Lys Pro Lys Ser Arg Glu Phe Val Ser Ser Ser
   900                 905                 910                 915
   tcc tct tcc tcg tcg tgg gga tcg tca tcg gag gat gaa gac gat gaa     16864
   Ser Ser Ser Ser Ser Trp Gly Ser Ser Ser Glu Asp Glu Asp Asp Glu
                   920                 925                 930
   ccc cgg cgc gtt tcg gtg gga agt gaa act aca ggc agc agg tcc gga     16912
   Pro Arg Arg Val Ser Val Gly Ser Glu Thr Thr Gly Ser Arg Ser Gly
               935                 940                 945
   cgc gaa cac gcc cct tcc ccg tca aat tcg gat gat tcg gac tca aat     16960
   Arg Glu His Ala Pro Ser Pro Ser Asn Ser Asp Asp Ser Asp Ser Asn
           950                 955                 960
   gat ggt ggg tcg acg aaa caa aat atc caa ccg gga tat cga tcc atc     17008
   Asp Gly Gly Ser Thr Lys Gln Asn Ile Gln Pro Gly Tyr Arg Ser Ile
       965                 970                 975
   agc ggt ccc gat ccg agg att cgt aag acc aaa cgt ctt gcg ggg gaa     17056
   Ser Gly Pro Asp Pro Arg Ile Arg Lys Thr Lys Arg Leu Ala Gly Glu
   980                 985                 990                 995
   ccg ggg cgc cag aga  cag aaa tca ttt tcc  ctg ccg cga tcc aga     17101
   Pro Gly Arg Gln Arg  Gln Lys Ser Phe Ser  Leu Pro Arg Ser Arg
                   1000                 1005                 1010
   acc ccg ata att ccc  ccg gtg tcg ggg ccg  ctc atg atg ccc gac     17146
   Thr Pro Ile Ile Pro  Pro Val Ser Gly Pro  Leu Met Met Pro Asp
                   1015                 1020                 1025
   gga agc cct tgg ccc  gga tcg gca ccc ctc  cca tcc aac agg gtg     17191
   Gly Ser Pro Trp Pro  Gly Ser Ala Pro Leu  Pro Ser Asn Arg Val
                   1030                 1035                 1040
   cgg ttt gga ccg tcc  ggg gag acc aga gag  ggt cac tgg gag gat     17236
   Arg Phe Gly Pro Ser  Gly Glu Thr Arg Glu  Gly His Trp Glu Asp
                   1045                 1050                 1055
   gag gct gtg aga gcg  gcg cgg gct cgt tac  gag gcc tca acg gaa     17281
   Glu Ala Val Arg Ala  Ala Arg Ala Arg Tyr  Glu Ala Ser Thr Glu
                   1060                 1065                 1070
   ccc gtg ccg ctt tac  gtg ccg gag ttg gga  gat ccg gct aga cag     17326
   Pro Val Pro Leu Tyr  Val Pro Glu Leu Gly  Asp Pro Ala Arg Gln
                   1075                 1080                 1085
   tac cgc gcg ctg att  aac ctg atc tac tgt  cca gac aga gac cct     17371
   Tyr Arg Ala Leu Ile  Asn Leu Ile Tyr Cys  Pro Asp Arg Asp Pro
                   1090                 1095                 1100
   ata gca tgg ctc cag  aac ccc aag ctg acc  ggt gtc aac tcg gcc     17416
   Ile Ala Trp Leu Gln  Asn Pro Lys Leu Thr  Gly Val Asn Ser Ala
                   1105                 1110                 1115
   ctg aac cag ttc tac  caa aag ctg ttg cca  ccg gga cgg gcg ggt     17461
   Leu Asn Gln Phe Tyr  Gln Lys Leu Leu Pro  Pro Gly Arg Ala Gly
                   1120                 1125                 1130
   acc gcc gtt acg ggg  agc gta gcg tct ccc  gtt ccg cat gta ggc     17506
   Thr Ala Val Thr Gly  Ser Val Ala Ser Pro  Val Pro His Val Gly
                   1135                 1140                 1145
   gaa gcc atg gcc acg  ggg gag gcc ctc tgg  gct ctc ccc cac gcg     17551
   Glu Ala Met Ala Thr  Gly Glu Ala Leu Trp  Ala Leu Pro His Ala
                   1150                 1155                 1160
   gcc gcg gcc gtg gct  atg agc cgt cga tac  gac cgg gcc caa aaa     17596
   Ala Ala Ala Val Ala  Met Ser Arg Arg Tyr  Asp Arg Ala Gln Lys
                   1165                 1170                 1175
   cac ttt atc cta cag  agt ctc cgc aga gcc  ttt gcc agc atg gca     17641
   His Phe Ile Leu Gln  Ser Leu Arg Arg Ala  Phe Ala Ser Met Ala
                   1180                 1185                 1190
   tac ccc gag gca acg  ggc tcc agt ccg gcg  gcg cgg atc tcc cgc     17686
   Tyr Pro Glu Ala Thr  Gly Ser Ser Pro Ala  Ala Arg Ile Ser Arg
                   1195                 1200                 1205
   ggt cac cct tct cca  aca acc ccg gcc aca  cag gct ccc gac cct     17731
   Gly His Pro Ser Pro  Thr Thr Pro Ala Thr  Gln Ala Pro Asp Pro
                   1210                 1215                 1220
   cag ccg tcg gcc gcc gca cgc tct ctt tct gtg tgt cca ccg gat 17776
   Gln Pro Ser Ala Ala Ala Arg Ser Leu Ser Val Cys Pro Pro Asp
   1225 1230 1235
   gat cgt tta cga act ccg cgc aag cgc aag tcc cag cca gtc gag 17821
   Asp Arg Leu Arg Thr Pro Arg Lys Arg Lys Ser Gln Pro Val Glu
   1240 1245 1250
   agc aga agc ctc ctc gac aag att agg gag aca ccc gtc gcg gac 17866
   Ser Arg Ser Leu Leu Asp Lys Ile Arg Glu Thr Pro Val Ala Asp
   1255 1260 1265
   gcc cgg gtt gca gac gat cat gtg gtt tcc aag gcc aag agg cgg 17911
   Ala Arg Val Ala Asp Asp His Val Val Ser Lys Ala Lys Arg Arg
   1270 1275 1280
   gta tcc gag ccc gtg acc atc acc tcg ggc cct gtg gtg gat ccc 17956
   Val Ser Glu Pro Val Thr Ile Thr Ser Gly Pro Val Val Asp Pro
   1285 1290 1295
   ccc gcc gta ata acg atg cca ctt gac gga ccg gcc cca aac ggg 18001
   Pro Ala Val Ile Thr Met Pro Leu Asp Gly Pro Ala Pro Asn Gly
   1300 1305 1310
   gga ttt cgg cgt att ccc cgg ggg gcc ctg cat acc ccg gtc ccg 18046
   Gly Phe Arg Arg Ile Pro Arg Gly Ala Leu His Thr Pro Val Pro
   1315 1320 1325
   tcg gac cag gct cgc aag gcg tac tgt acc ccc gaa acc atc gcc 18091
   Ser Asp Gln Ala Arg Lys Ala Tyr Cys Thr Pro Glu Thr Ile Ala
   1330 1335 1340
   cgt ctg gtc gac gac cca ttg ttt ccc acg gcc tgg cgc cct gcg 18136
   Arg Leu Val Asp Asp Pro Leu Phe Pro Thr Ala Trp Arg Pro Ala
   1345 1350 1355
   cta agc ttt gat ccc ggc gcc ttg gcg gaa atc gcc gct cgg cgt 18181
   Leu Ser Phe Asp Pro Gly Ala Leu Ala Glu Ile Ala Ala Arg Arg
   1360 1365 1370
   ccg ggc gga gga gac cga cgg ttt ggt cca ccc agc gga gtg gag 18226
   Pro Gly Gly Gly Asp Arg Arg Phe Gly Pro Pro Ser Gly Val Glu
   1375 1380 1385
   gcg ctg cga cgg agg tgc gcc tgg atg cgg cag atc cca gac ccg 18271
   Ala Leu Arg Arg Arg Cys Ala Trp Met Arg Gln Ile Pro Asp Pro
   1390 1395 1400
   gag gat gtg agg ctt ctg atc atc tac gat ccg ttg ccc gga gag 18316
   Glu Asp Val Arg Leu Leu Ile Ile Tyr Asp Pro Leu Pro Gly Glu
   1405 1410 1415
   gac atc aac ggc ccc ctc gag agc acc ctc gcg aca gat ccg gga 18361
   Asp Ile Asn Gly Pro Leu Glu Ser Thr Leu Ala Thr Asp Pro Gly
   1420 1425 1430
   ccg tca tgg agt cca tcc cga ggg gga ctg tct gtg gtc ctg gca 18406
   Pro Ser Trp Ser Pro Ser Arg Gly Gly Leu Ser Val Val Leu Ala
   1435 1440 1445
   gcc ctg agt aac cgg ttg tgc ctg ccg agc act cat gcc tgg gcc 18451
   Ala Leu Ser Asn Arg Leu Cys Leu Pro Ser Thr His Ala Trp Ala
   1450 1455 1460
   ggg aac tgg acc ggc ccg ccg gac gtg tcc gct ttg aac gcc cgg 18496
   Gly Asn Trp Thr Gly Pro Pro Asp Val Ser Ala Leu Asn Ala Arg
   1465 1470 1475
   ggc gtt tta tta ctg tcg acc cga gac ctg gcc ttt gcc ggg gcc 18541
   Gly Val Leu Leu Leu Ser Thr Arg Asp Leu Ala Phe Ala Gly Ala
   1480 1485 1490
   gtc gag tat cta ggc tcg cgg ttg gcc tct gcc cgg cgc cgg ttg 18586
   Val Glu Tyr Leu Gly Ser Arg Leu Ala Ser Ala Arg Arg Arg Leu
   1495 1500 1505
   ctg gtg ttg gac gcg gtg gcc ctc gag agg tgg ccc agg gat gga 18631
   Leu Val Leu Asp Ala Val Ala Leu Glu Arg Trp Pro Arg Asp Gly
   1510 1515 1520
   ccc gct ttg tct cag tat cac gtg tac gtc cgg gcc ccg gcg cga 18676
   Pro Ala Leu Ser Gln Tyr His Val Tyr Val Arg Ala Pro Ala Arg
   1525 1530 1535
   ccg gac gcc cag gcc gtc gtc cga tgg cca gac tcg gcg gtc aca 18721
   Pro Asp Ala Gln Ala Val Val Arg Trp Pro Asp Ser Ala Val Thr
   1540 1545 1550
   gaa gga ctc gcc cgg gcc gtg ttt gca tcg tcg cgc acc ttt ggg 18766
   Glu Gly Leu Ala Arg Ala Val Phe Ala Ser Ser Arg Thr Phe Gly
   1555 1560 1565
   cca gcg agt ttt gct cgt atc gag act gcg ttt gcc aac ctg tac 18811
   Pro Ala Ser Phe Ala Arg Ile Glu Thr Ala Phe Ala Asn Leu Tyr
   1570 1575 1580
   ccg ggc gaa caa ccc ctg tgt ttg tgc cgc ggt ggg aac gtc gca 18856
   Pro Gly Glu Gln Pro Leu Cys Leu Cys Arg Gly Gly Asn Val Ala
   1585 1590 1595
   tac acc gtg tgt acc cgc gcg ggc ccc aag acc cgc gtc ccc ctg 18901
   Tyr Thr Val Cys Thr Arg Ala Gly Pro Lys Thr Arg Val Pro Leu
   1600 1605 1610
   tcg ccc cgt gaa tac cgg cag tac gtg ctg ccg ggt ttt gac ggt 18946
   Ser Pro Arg Glu Tyr Arg Gln Tyr Val Leu Pro Gly Phe Asp Gly
   1615 1620 1625
   tgc aag gac ctc gcg cga cag tct cgg ggt ctg ggg ctc ggg gca 18991
   Cys Lys Asp Leu Ala Arg Gln Ser Arg Gly Leu Gly Leu Gly Ala
   1630 1635 1640
   gcc gac ttt gtg gac gag gcg gca cat agc cac cgc gca gca aac 19036
   Ala Asp Phe Val Asp Glu Ala Ala His Ser His Arg Ala Ala Asn
   1645 1650 1655
   cga tgg ggc ctg ggt gcc gcg ctt cga ccc gtc ttc ctt ccc gag 19081
   Arg Trp Gly Leu Gly Ala Ala Leu Arg Pro Val Phe Leu Pro Glu
   1660 1665 1670
   gga cgg aga ccg ggg gcc gcc ggg ccg gag gcc ggc gac gta ccc 19126
   Gly Arg Arg Pro Gly Ala Ala Gly Pro Glu Ala Gly Asp Val Pro
   1675 1680 1685
   acc tgg gcg agg gtg ttt tgc cgc cac gcc ctg ctg gaa ccc gac 19171
   Thr Trp Ala Arg Val Phe Cys Arg His Ala Leu Leu Glu Pro Asp
   1690 1695 1700
   cct gcc gca gaa cca ctc gtg ctt cca ccc gtg gcc ggt cgg tcg 19216
   Pro Ala Ala Glu Pro Leu Val Leu Pro Pro Val Ala Gly Arg Ser
   1705 1710 1715
   gtg gcg ctg tat gcg tcg gcg gac gag gct cgg aat gcc ctc ccc 19261
   Val Ala Leu Tyr Ala Ser Ala Asp Glu Ala Arg Asn Ala Leu Pro
   1720 1725 1730
   ccg att ccc aga gta atg tgg ccg ccc ggt ttt ggg gcc gcg gag 19306
   Pro Ile Pro Arg Val Met Trp Pro Pro Gly Phe Gly Ala Ala Glu
   1735 1740 1745
   acg gtg ttg gag ggg agc gac gga aca cgg ttc gtg ttc gga cac 19351
   Thr Val Leu Glu Gly Ser Asp Gly Thr Arg Phe Val Phe Gly His
   1750 1755 1760
   cac ggg ggc tcg gaa cgg ccg tca gaa acc cag gcg ggg cga cag 19396
   His Gly Gly Ser Glu Arg Pro Ser Glu Thr Gln Ala Gly Arg Gln
   1765 1770 1775
   cgg cgc acc gca gac gac aga gaa cac gct ttg gag ctg gac gat 19441
   Arg Arg Thr Ala Asp Asp Arg Glu His Ala Leu Glu Leu Asp Asp
   1780 1785 1790
   tgg gag gtg ggg tgt gaa gac gcg tgg gac agc gag gag ggg ggc 19486
   Trp Glu Val Gly Cys Glu Asp Ala Trp Asp Ser Glu Glu Gly Gly
   1795 1800 1805
   ggg gac gac ggg gac gca ccg ggg tca tcc ttt ggg gtg agc atc 19531
   Gly Asp Asp Gly Asp Ala Pro Gly Ser Ser Phe Gly Val Ser Ile
   1810 1815 1820
   gtg tcg gtg gcc ccg ggt gtg ctg cga gac cgc cgg gtg ggt ttg 19576
   Val Ser Val Ala Pro Gly Val Leu Arg Asp Arg Arg Val Gly Leu
   1825 1830 1835
   cgc ccg gcg gtc aag gtg gag ctg ttg tcc tcg tcc tcg tcc tcc 19621
   Arg Pro Ala Val Lys Val Glu Leu Leu Ser Ser Ser Ser Ser Ser
   1840 1845 1850
   gag gac gag gac gat gtg tgg gga ggg cgc ggg ggg agg agc ccc 19666
   Glu Asp Glu Asp Asp Val Trp Gly Gly Arg Gly Gly Arg Ser Pro
   1855 1860 1865
   ccg cag agt cgg ggg tga cggagtcccc tccttttctc gtgagcgcca 19714
   Pro Gln Ser Arg Gly
   1870
   ctggcgcgcg gactgtttgt tgttaataaa agcggaacgg tttttatgaa aaaagtgtct 19774
   gtctgtctgt gcgggcgggc gacgggcggg ctggtcggac ccccccccga aaataacccc 19834
   cccccggttt ctgggcgccc ggcggacccc gggagaggag gccagccctc tcgcggcccc 19894
   ctcgagagag aaaaaaaaaa gcgaccccac ctccccgcgc gtttgcgggg cgaccatcgg 19954
   gggggacttg cattacccta tcccagtatt gtttgtacgc ctctaatgga gtaactgtcc 20014
   caatacaccc gacttcctat atacagtgct tttgtttgtg gacttacctt tatttacggg 20074
   tattacaggg gggtggagaa atgtcttcgt atcgctcttt atctaaataa agacagaatc 20134
   taaaatgtca cttagctgta tacccggccc aaggttatac caatcaactt ccctgtttgt 20194
   tgggactgtc cgcctacccc aatacacatt ttatacccac gttttagtgg gtgggactta 20254
   aaagaaatgg gtggagggat ataggggtgt gtcttcgttg gtaccaatta taaaaatgta 20314
   ctcgccacaa ctcacaattt agaacgcatg gcagttctgc tacgtgtttg gatgcccgga 20374
   cattagaata cagccagttg ttacc atg gat acc ata tta gcg ggc ggt agc 20426
   Met Asp Thr Ile Leu Ala Gly Gly Ser
   1875
   ggc acc tcc gac gct tcg gat aat acc tgc acc ata tgc atg agc 20471
   Gly Thr Ser Asp Ala Ser Asp Asn Thr Cys Thr Ile Cys Met Ser
   1880 1885 1890
   acc gtt tcc gat ctc gga aaa acc atg ccg tgt ttg cac gac ttc 20516
   Thr Val Ser Asp Leu Gly Lys Thr Met Pro Cys Leu His Asp Phe
   1895 1900 1905
   tgc ttt gtt tgt att cgg gca tgg acc tcc acc agc gtc cag tgt 20561
   Cys Phe Val Cys Ile Arg Ala Trp Thr Ser Thr Ser Val Gln Cys
   1910 1915 1920
   cct ctc tgc cgg tgt cca gtg caa tcc atc ctg cat aag atc gta 20606
   Pro Leu Cys Arg Cys Pro Val Gln Ser Ile Leu His Lys Ile Val
   1925 1930 1935
   agt gat aca agt tac aag gaa tat gaa gtg cac cca tcc gac gac 20651
   Ser Asp Thr Ser Tyr Lys Glu Tyr Glu Val His Pro Ser Asp Asp
   1940 1945 1950
   gat ggt ttt tct gag ccg tca ttt gaa gat tcc atc gac atc cta 20696
   Asp Gly Phe Ser Glu Pro Ser Phe Glu Asp Ser Ile Asp Ile Leu
   1955 1960 1965
   ccg gga gat gtc ata gat ctt ctg cca cca agc cca gga ccg agt 20741
   Pro Gly Asp Val Ile Asp Leu Leu Pro Pro Ser Pro Gly Pro Ser
   1970 1975 1980
   cgg gag tcc atc caa cag cca aca tca aga tcg agt cgg gag ccc 20786
   Arg Glu Ser Ile Gln Gln Pro Thr Ser Arg Ser Ser Arg Glu Pro
   1985 1990 1995
   att caa tca cca aac cct ggg ccc ctt caa tcg tcg gct aga gag 20831
   Ile Gln Ser Pro Asn Pro Gly Pro Leu Gln Ser Ser Ala Arg Glu
   2000 2005 2010
   ccc aca gca gag tca cca agt gac tct caa cag gat tct ata caa 20876
   Pro Thr Ala Glu Ser Pro Ser Asp Ser Gln Gln Asp Ser Ile Gln
   2015 2020 2025
   cca ccg acc cga gac tcg agc cct ggt gta acc aaa aca tgc tct 20921
   Pro Pro Thr Arg Asp Ser Ser Pro Gly Val Thr Lys Thr Cys Ser
   2030 2035 2040
   acc gca tca ttt tta cgg aag gta ttt ttt aaa gac caa cct gct 20966
   Thr Ala Ser Phe Leu Arg Lys Val Phe Phe Lys Asp Gln Pro Ala
   2045 2050 2055
   gtt cga tcg gcg acc ccg gtg gtg tat ggc tcg att gaa tct gca 21011
   Val Arg Ser Ala Thr Pro Val Val Tyr Gly Ser Ile Glu Ser Ala
   2060 2065 2070
   cag caa ccc cgg acc ggg ggg cag gac tac cgt gat cgt cca gta 21056
   Gln Gln Pro Arg Thr Gly Gly Gln Asp Tyr Arg Asp Arg Pro Val
   2075 2080 2085
   tct gtg gga att aat caa gac cca cga acc atg gac aga ctg cct 21101
   Ser Val Gly Ile Asn Gln Asp Pro Arg Thr Met Asp Arg Leu Pro
   2090 2095 2100
   ttt cga gcc acg gat aga gga aca gag gga aac gcg aga ttc ccg 21146
   Phe Arg Ala Thr Asp Arg Gly Thr Glu Gly Asn Ala Arg Phe Pro
   2105 2110 2115
   tgt tac atg caa cct tta ctc gga tgg ctt gat gat caa ctt gcg 21191
   Cys Tyr Met Gln Pro Leu Leu Gly Trp Leu Asp Asp Gln Leu Ala
   2120 2125 2130
   gaa ctg tat caa ccc gaa att gta gag cct aca aaa atg ttg ata 21236
   Glu Leu Tyr Gln Pro Glu Ile Val Glu Pro Thr Lys Met Leu Ile
   2135 2140 2145
   tta aac tat ata ggt att tac ggg cgt gat gag gcg gga tta aaa 21281
   Leu Asn Tyr Ile Gly Ile Tyr Gly Arg Asp Glu Ala Gly Leu Lys
   2150 2155 2160
   aca tcc ctg cgt tgt ctt ttg cat gat tca aca gga ccg ttt gta 21326
   Thr Ser Leu Arg Cys Leu Leu His Asp Ser Thr Gly Pro Phe Val
   2165 2170 2175
   aca aac atg tta ttc ttg ttg gat cga tgt acc gat cca acc cgc 21371
   Thr Asn Met Leu Phe Leu Leu Asp Arg Cys Thr Asp Pro Thr Arg
   2180 2185 2190
   cta acc atg caa acc tgg acc tgg aaa gat aca gcc atc caa cta 21416
   Leu Thr Met Gln Thr Trp Thr Trp Lys Asp Thr Ala Ile Gln Leu
   2195 2200 2205
   att aca ggt cca att gta aga cca gaa acc acc tca acc ggg gag 21461
   Ile Thr Gly Pro Ile Val Arg Pro Glu Thr Thr Ser Thr Gly Glu
   2210 2215 2220
   acc tct cgt ggc gat gaa agg gat acc cga ttg gta aat aca ccc 21506
   Thr Ser Arg Gly Asp Glu Arg Asp Thr Arg Leu Val Asn Thr Pro
   2225 2230 2235
   caa aaa gtc agg ctt ttt tct gtg tta ccg ggg att aaa ccg gga 21551
   Gln Lys Val Arg Leu Phe Ser Val Leu Pro Gly Ile Lys Pro Gly
   2240 2245 2250
   agc gca agg ggt gct aag cgc cgt tta ttt cat acc ggc aga gac 21596
   Ser Ala Arg Gly Ala Lys Arg Arg Leu Phe His Thr Gly Arg Asp
   2255 2260 2265
   gtt aaa cga tgc tta aca ata gac ctg aca tct gag tct gat tcg 21641
   Val Lys Arg Cys Leu Thr Ile Asp Leu Thr Ser Glu Ser Asp Ser
   2270 2275 2280
   gca tgt aag gga agt aaa acc cgc aaa gtt gcc tct cca cag ggg 21686
   Ala Cys Lys Gly Ser Lys Thr Arg Lys Val Ala Ser Pro Gln Gly
   2285 2290 2295
   gag tcc aat acc ccc tcc acc tcc gga tca aca tca ggt tca ctg 21731
   Glu Ser Asn Thr Pro Ser Thr Ser Gly Ser Thr Ser Gly Ser Leu
   2300 2305 2310
   aaa cac ctt acc aaa aaa agc tct gcc ggt aaa gcg ggt aaa ggt 21776
   Lys His Leu Thr Lys Lys Ser Ser Ala Gly Lys Ala Gly Lys Gly
   2315 2320 2325
   att cca aac aag atg aag aag tcc tag tttgttggga gggggaagga 21823
   Ile Pro Asn Lys Met Lys Lys Ser
   2330 2335
   aatgccttaa acatccacag tctgctttat taccaactgt atgtaaatta tgatcattaa 21883
   acgtgcattt taaaaatacc tgagtgttgc tattggtcgt aagggttggt atatctggaa 21943
   cccaaggtgt aaatactgcc ccctggtaag tccgtacacg attcggaatg cccgcaatcc 22003
   ggtctcggag taaccggggc atcctgctct gcaaatacaa taaacataac agtctaacag 22063
   tacgcatgtg ctgtcgtaag cgctccgccc taaattacaa aaatttgaga ttacgctcaa 22123
   tacttgtcac cgtttgtatt tatacacctc tacgggagca aactaaaggt atacttggag 22183
   atgcaaccaa ggccgagttg agacgggaaa attagaagct gtttttccag cggcaaaaat 22243
   catccggttt gggtatgtct tatagcaatt aaagttatta aaatggccgg aaggtggcgg 22303
   gggtggtgaa ggcgagggta aagcaaactt gcggggggat aaaaggcata tatgttacaa 22363
   aatacgcacg caaatataaa ttttagtttt gtacttacag taaaggcaac ggtgttttcg 22423
   gtttaggcag ccggacccgt ttaaaatgcc aactggatag tctgcaagga ggaaacgctg 22483
   ctgcaatgag tcaggttttt aggtgaatta ggtatgatgc atcatttttg ttaatggact 22543
   tttaatggat tgaaacggat agaaggaaac gtgtagcata atggatattg gttgatattt 22603
   taagcacgct gccaagtacg cactaagaat gcttagaaat atttaaactg aaatgcgtgg 22663
   gtttggcaaa cgttaccgta aatcaatctc agatccaagg gataatggcc gttgggcagg 22723
   cctccaaaag gcaatttgag cgttttaagt ttggggtacg tttttaaagc ttgattggca 22783
   aacgttaccg caaatcaatc ttagatccaa gggataatgg ccgttgggca ggcctccaaa 22843
   aggcaatttg agcgttttca gtccgcttga aaacggtcta agtatttgcc gtaaatgacc 22903
   gcgagaggta taatataaac atctgttttc ccgatgacgt gttaagaaaa gtgattatat 22963
   cagataacgg gggactgtaa agcttaatac gtgacatgtg attgggtgta taaatactac 23023
   atccaaatct tgcatggaaa ttcgctttgt cggcacgccg tgacgtttga catattaaga 23083
   tcgccctttt taggaaaatg agatatgaca ccaagagttt aactaaaggc caactgtata 23143
   tataaaactc atacttgtcc atgcttccat tagagtggtc aagtttttca aaggatacga 23203
   caacgtcgta gtgaagggaa aacacaagcg tcatggcatc acataaatgg ttactgcaga 23263
   tagttttttt aaaaactatc acaatcgcgt attgtcttca tctccaagac gacactccgt 23323
   tgttttttgg agccaaaccg ctatcggatg tgagtttgat tataacggaa ccgtgcgtgt 23383
   catcggtata tgaggcgtgg gactatgcgg cacccccggt atcaaacctc agcgaggcgc 23443
   tatcgggaat cgtggttaag acaaaatgtc cagtaccgga agttatactt tggtttaaag 23503
   acaaacaaat ggcgtactgg acaaatccat acgtcacctt aaaggggctg gcacaatctg 23563
   ttggtgaaga acataaaagc ggggacatac gcgatgcttt gttggatgcc ctttccggtg 23623
   tatgggtaga ctctactcca tcttccacaa atatcccgga aa atg gat gtg tct 23677
   Met Asp Val Ser
   2340
   ggg gag ccg acc gtt tgt tcc aac gcg tat gcc aat gaa atg aaa 23722
   Gly Glu Pro Thr Val Cys Ser Asn Ala Tyr Ala Asn Glu Met Lys
   2345 2350 2355
   cta tcg gat tca aag gac att tat gtt ttg gcc cat ccg gtt acc 23767
   Leu Ser Asp Ser Lys Asp Ile Tyr Val Leu Ala His Pro Val Thr
   2360 2365 2370
   aaa aaa acc cgc aag cga ccc cgc ggg ctg cct ttg ggg gtt aag 23812
   Lys Lys Thr Arg Lys Arg Pro Arg Gly Leu Pro Leu Gly Val Lys
   2375 2380 2385
   cta gac ccc cca acc ttc aag tta aat aac atg tca cat cat tac 23857
   Leu Asp Pro Pro Thr Phe Lys Leu Asn Asn Met Ser His His Tyr
   2390 2395 2400
   gac acg gaa acg ttc acc ccc gtc tct tcg caa ctg gat tcg gtt 23902
   Asp Thr Glu Thr Phe Thr Pro Val Ser Ser Gln Leu Asp Ser Val
   2405 2410 2415
   gaa gtt ttc agc aag ttt aac att tcc cct gag tgg tat gac ctg 23947
   Glu Val Phe Ser Lys Phe Asn Ile Ser Pro Glu Trp Tyr Asp Leu
   2420 2425 2430
   ttg tcg gac gaa ctt aaa gag ccg tac gcg aaa ggt att ttt tta 23992
   Leu Ser Asp Glu Leu Lys Glu Pro Tyr Ala Lys Gly Ile Phe Leu
   2435 2440 2445
   gaa tac aat cgt ctt tta aat tca ggg gaa gaa ata ctt cca tct 24037
   Glu Tyr Asn Arg Leu Leu Asn Ser Gly Glu Glu Ile Leu Pro Ser
   2450 2455 2460
   aca ggc gat att ttt gca tgg acg cga ttt tgc gga ccc cag agc 24082
   Thr Gly Asp Ile Phe Ala Trp Thr Arg Phe Cys Gly Pro Gln Ser
   2465 2470 2475
   att cgc gtt gta att att ggt caa gat cca tac cct acc gcg gga 24127
   Ile Arg Val Val Ile Ile Gly Gln Asp Pro Tyr Pro Thr Ala Gly
   2480 2485 2490
   cat gca cat ggg cta gcg ttt agt gta aaa cgt ggc ata aca cca 24172
   His Ala His Gly Leu Ala Phe Ser Val Lys Arg Gly Ile Thr Pro
   2495 2500 2505
   ccg tct agt ctt aaa aat att ttt gcg gcc ctc atg gaa tca tac 24217
   Pro Ser Ser Leu Lys Asn Ile Phe Ala Ala Leu Met Glu Ser Tyr
   2510 2515 2520
   cca aat atg act ccg ccc act cac gga tgc ctg gag agt tgg gca 24262
   Pro Asn Met Thr Pro Pro Thr His Gly Cys Leu Glu Ser Trp Ala
   2525 2530 2535
   agg cag ggg gtg tta ttg ctg aat acc acg ctt acg gtt cgt cgc 24307
   Arg Gln Gly Val Leu Leu Leu Asn Thr Thr Leu Thr Val Arg Arg
   2540 2545 2550
   ggg act ccg ggg tcg cat gta tac tta ggc tgg ggg cgg ctg gtg 24352
   Gly Thr Pro Gly Ser His Val Tyr Leu Gly Trp Gly Arg Leu Val
   2555 2560 2565
   caa cgc gtg cta cag agg tta tgc gag aac cgt aca ggg tta gtt 24397
   Gln Arg Val Leu Gln Arg Leu Cys Glu Asn Arg Thr Gly Leu Val
   2570 2575 2580
   ttt atg ctg tgg ggt gcg cat gca cag aag aca acc caa ccg aat 24442
   Phe Met Leu Trp Gly Ala His Ala Gln Lys Thr Thr Gln Pro Asn
   2585 2590 2595
   tca aga tgt cat ctg gtg cta aca cac gcg cat ccg tcg cca ttg 24487
   Ser Arg Cys His Leu Val Leu Thr His Ala His Pro Ser Pro Leu
   2600 2605 2610
   tcc cgt gtt cca ttt cgg aat tgt cga cat ttc gtt caa gcc aat 24532
   Ser Arg Val Pro Phe Arg Asn Cys Arg His Phe Val Gln Ala Asn
   2615 2620 2625
   gag tat ttt acg cgt aaa ggc gaa ccc gag atc gat tgg agt gtt 24577
   Glu Tyr Phe Thr Arg Lys Gly Glu Pro Glu Ile Asp Trp Ser Val
   2630 2635 2640
   ata taa cactccaatc gaccctcttg cgtaccataa tgttttcgga gttgcctcct 24633
   Ile
   tccgtaccga cggcattgct tcaatggggt tggggattgc atcgtggacc gtgttcgatc 24693
   ccaaatttta aacaggtagc cagccaacac agtgttcaga acgattttac agaaaatagc 24753
   gttgatgcaa atgaaaaatt tccgattggg cacgcgggct gtattgagaa aaccaaagac 24813
   gactatgtac catttgatac gttgttcatg gtatcatcta ttgacgaact tgggcggaga 24873
   caattaaccg acaccatccg ccgcagcttg gttatgaacg cctgtgaaat aacggtcgcg 24933
   tgtacgaaaa ccgcagcctt ttctggtcga ggcgtgtcac gacaaaaaca cgtgacccta 24993
   tctaaaaata aattcaatcc atccagtcat aagagcctgc aaatgtttgt gttgtgtcaa 25053
   aaaacccatg caccccgtgt cagaaaccta ctgtacgaga gtattcgtgc aagaagacct 25113
   cgccgatatt acacccgctc aacggacgga aaatcgcgtc cgttggtacc agtgtttgtg 25173
   tatgagttta cggctttaga tcgtgtcctt ttacataagg aaaatacttt gaccgaccaa 25233
   ccaattaata ctgaaaatag cggtc atg gac gta cga gaa cgt aat gtg ttt 25285
   Met Asp Val Arg Glu Arg Asn Val Phe
   2645 2650
   gga aat gcc agc gtt gcc acg ccg ggg gaa cat cag aaa ttt gta 25330
   Gly Asn Ala Ser Val Ala Thr Pro Gly Glu His Gln Lys Phe Val
   2655 2660 2665
   cgc gag tta att ttg tcg gga cac aac aac gtc gta tta cag aca 25375
   Arg Glu Leu Ile Leu Ser Gly His Asn Asn Val Val Leu Gln Thr
   2670 2675 2680
   tac act ggt aaa tgg tca gac tgc cgt aaa cac ggt aaa tcg gtt 25420
   Tyr Thr Gly Lys Trp Ser Asp Cys Arg Lys His Gly Lys Ser Val
   2685 2690 2695
   atg tat aat acc ggt gaa gcg cgg cac cca acc tgc aag gct cat 25465
   Met Tyr Asn Thr Gly Glu Ala Arg His Pro Thr Cys Lys Ala His
   2700 2705 2710
   caa cgt taa gaggataggt gtcttcaaat taaaagccgt taaatataaa 25514
   Gln Arg
   ttttttgtgt cgtttattcg cgtatgaaat gtaatcagtt gggataaatg ttagtcttga 25574
   atctgtcttt acgcgtttgc ggcgtcccgt aatattaacg aaagaacgtc gtcgtctgat 25634
   gtgaaaaagg ccggttgtgg tgaagaagga cagttggttt caggctgccg cctttgcgat 25694
   gtatactggt gcggggttgg tgttggtgtg gtctgatcgt caggtctgtt tacccgtaaa 25754
   ccgtatataa caattgagat acacatggat aggtcatctt ccgttattac ggatagtacc 25814
   ccccccacat tactgtacag ctgagatgtt gggtcagaac cgacccagga cataaacggc 25874
   atcgcaaatg ttgcgtgggg tttgtcctgt aagtggaaat cccggtgcgt aaatgcgtat 25934
   gatgaaaagt cagtagaaca gtctatggtt agcagggtca ctgtatgggt gtgttgaagg 25994
   acgatcatac caccgctcat tgtatcacat cgagcgtccg tcttcaccgc cattgatctt 26054
   gacccacccg taaatgcgta gagtgtttgt tcatgatccg gtaaagtcca gtgaagaacc 26114
   cgcctaaccc catgtaacct atataaaata aacgtcgttg ggggtccaga gatgctcatg 26174
   gcgggtgtat ttaagacaaa acctagaaat gagaacagca agcccgcatg cgtgttttta 26234
   tacaatgctt taatacacaa cgtgtacgtt gggatcacgt agagcggcaa gaatgtgatc 26294
   gctaatttct gcggattttt catatcgttc ccgtagaggg tttagattca tttttaagaa 26354
   cctagaggag accgtacgcg acatggcaac atacacacta tttaggcgca gtttatccgc 26414
   cgtaaaacag atagctacct tctctaaact cagaccctgt gagcgtgcaa ttgtcatggc 26474
   tagtttagaa ctaaggccat agtccaccgt ggtggccatt gccaattctg catcctctat 26534
   agactcggta aattcacata cgttagcgtt tacgatggac acaaacccat gttgatcctg 26594
   taagaccata agaggtacgt gtagagcata taacatttcg gtagtttcgc ggtacagctt 26654
   ttttcgggtc agttcctcta caaagactgg cacgggtgca aaagtatacc ccaaaagggt 26714
   atacgtatct gtttgtaatg ccaaagataa cagccccccc cgcacattgc cgatcaaaag 26774
   ctcgcttccg ttgaaattaa cattatcgac atacgcgcta aagggggcgg ttgtaaagct 26834
   gctttcgaaa agctctgcta atatggcata tcgaccatcg taaatagctt tcattaacag 26894
   aaactgagca tggacctcat tggatgattt gggataggaa aattcgtact ggcagtatac 26954
   caagtcaata acctcgtcgt taagtgccgt aaaaattaca tcatccgggt cttcgggtat 27014
   ggaggccgtt actttggact cgggggcttt ggtaaagcag aaggctttat cggcagaagt 27074
   agctgcacca tcacagtcaa tcccggcacc cgccccatta accgctgctt cgtgtaaggg 27134
   ggctttgttt gttccagaca ataactccag ggttaaggca gctaaccgtt tgtatgctaa 27194
   cgatacctta tcgggatgta ggtttttatt taacaaaaaa ttgtaaaagt taatcaagcc 27254
   tccaaagatt aggtttgaca gaaaccggta agcatattcg atggatgtct ctcctcgagc 27314
   cttcacaaag gagtcgtcac gtaaaacctt tgcaaacgat tgaaatgtac cactgaatcc 27374
   aataactaac ttacgtagtc ttgtggttac aactacgaga ctatttagca cgtaagtgat 27434
   gtctgtacgg gctacaatta agtcgcgatt tgaatgtgtt tcgtatttaa ctgttcccat 27494
   gtcatgatct cggctttggg aataattgtg caaacgaccg gagtttgccc gtatccaatg 27554
   ttcaacagaa agtccgggtt gtcccgttaa tttgcggtat tcatcaaatg ccgttagccg 27614
   gatgaatgta taagtcggta aggcaaacac agaaaaatgg tcatttttcg atagttttaa 27674
   atgcgcgtgt aacttactca tatacgcgct cacctcctta tgcgacgaat acagacgcgt 27734
   ccatccggga agattagcag gattgttaat ataggatgca ggtacaacaa atgtatcgac 27794
   cagacgcgca tgtgcttcgg taataggtag cccgtactca agcgttttta aaagatttcc 27854
   aaaatcgtcc tcttgacatc gtttgttatt aataaagatt gcccagttat gtgagatgtt 27914
   agtatattga cgcagagttt gattgcagat tatatacgtg agtatatttt cactaggagt 27974
   tacgtgtgaa cgctgcatgt catgttgaaa atgagattct aacgagtcag tttgggtggg 28034
   cgaaccgacg catactatga ccggttttcg accgtttatg tactgagggc tttgatatat 28094
   agcattcaaa agccaccaac agtaaacaac ggccgtgaga atatgacgcc ctagcaatcc 28154
   tgcttcatca ataacaataa cgttgctccg ggtaaaagcc ggaagggaac cgcatgcgat 28214
   aaacgcggtt ccggacattg agcctgtagg tttattaagc aaacgttcaa ttgcccacag 28274
   ggttttaaat gtcgatgttc ctccgcgacc gtcgtccccc atttgaaaca ctcgttttgt 28334
   tatatcaatt aaaacttccc agtagtatac aatatctctt ttttgcaggt cctcaataga 28394
   agggggggtc gtagtccagt tatatgcgta acggcccagc tgagcctgaa tgtgatttcc 28454
   gcgaaaacca aattcatgaa agattgtgtt tatcggacga ctcgcataag ccgttgataa 28514
   cttagcatga acattttggg cagcaaccct ggtggatccg gtaataatgc aatcgatagc 28574
   ttcgttaagc gtttggatac acgtgctttt tccggagccg gcatttccgc taattaaata 28634
   aacggagaag gggagctcat tcctctgcat ttcaggaggc gcttgcagtt cgagtaactg 28694
   tttaaaccaa cacaaccgcg gaacacgttc ttttggaatc gtaattgcgg caagttctcg 28754
   aatccgtgag aggataggtt gaatgctatg catagaggtg aagtttaaat atacactgtc 28814
   atcaaatcca ttgggcgtct ctggattaaa aacgtttttg ggtgaagaac tgtctacaga 28874
   aattgatctt ttcatcgtgg ttttcaatgg ccgaaataac gtctcttttt aataacagtt 28934
   ccggtagtga agaaaaaagg atagcaagtt ctgtttctat tgaccagggc ttgaatggaa 28994
   gtaacccaaa tgaccaatac aagaacatgt tcgatatata ctggaatgag tacgccccgg 29054
   atatagggtt ttgtacattt ccggaggaag atggctggat gttaatacac ccaaccacgc 29114
   aaagtatgtt gtttcgaaaa atcctagccg gtgactttgg atataccgat ggacaaggca 29174
   tatatagcgc tgtacggtct acggaaactg taattcgcca agttcaggca accgttttga 29234
   tgaacgcgtt ggatgcaact cggtatgagg acctagcagc agattgggaa caccacatcc 29294
   aacaatgtaa cctgcatgcc ggggctctag cggaacgtta tgggctatgt ggagaatcag 29354
   aagccgtacg gcttgcacat caggtttttg aaacctggcg tcaaacatta cagtcatcgt 29414
   tacttgagtt tctgcgtgga ataaccggtt gtctctatac cagtggttta aatggaaggg 29474
   tcggttttgc caaatacgtg gactggatag cctgtgtagg tattgtgccc gttgtaagaa 29534
   aggtacgatc agaacagaat ggaacccctg caccattaaa tacgtatatg ggtcaagcgg 29594
   cagaactgtc ccagatgtta aaagttgccg atgcaacgtt ggccagagga gcggcggttg 29654
   tcacaagcct agttgagtgt atgcaaaatg ttgctattat ggattatgat aggacgcgtc 29714
   tttattataa ttataaccga agattaatta tggcaaagga tgatgtaacg ggcatgaagg 29774
   gagagtgttt ggtcgtgtgg ccgcccgttg tatgtgggga gggtgtagta tttgactcac 29834
   ccttacagcg gctttctggg gaggtgttgg cctgttatgc attacgtgaa catgctcgcg 29894
   tctgccaagt tttaaataca gcccctttgc gcgtgttaat aggtcgccgg aatgaagatg 29954
   atagatctca cagcacacgt gcggttgatc gtataatggg cgagaacgat acaacacggg 30014
   ctggatcggc cgcgtctaga cttgtaaagc taatagttaa cttaaaaaac atgagacatg 30074
   ttggagatat taccgaaacc gtacgttcct atctagaaga aacgggcaat cacattctgg 30134
   aaggaagtgg atcggtggac acatcacaac cggggtttgg caaggccaac caatccttta 30194
   acgggggggc aatgtccgga acaacaaacg ttcaaagtgc gtttaaaact tcggtggtta 30254
   acagtatcaa cggcatgctc gagggttatg tgaataattt attcaaaacc attgagggtc 30314
   tcaaggatgt gaacagcgat ctgaccgaaa ggctccagtt caaagaagga gagctgaaac 30374
   ggttacggga agagagggta aaaataaagc catctaaagg gtcacatatt acaatggcag 30434
   aagaaacacg tattgccgat ttaaatcacg aggttataga tcttaccggc ataatagggg 30494
   atgatgcata tattgccaat agttttcaat ctcgttatat ccccccttat ggagatgata 30554
   taaaacgttt gtctgagcta tggaaacagg aacttgttcg ctgttttaag cttcaccggg 30614
   taaacaataa tcaaggccag gaaatttctg tatcatattc aaatgcgtca atctcattac 30674
   tagttgcgcc gtatttttca ttcatattac gggccacccg attaggattc ttggtaactc 30734
   aaagcgaggt acataggtca gaggaagagt tatgccaggc tatttttaaa aaggcgagaa 30794
   cagagtccta tttatcccaa atccgaatat tatatgaaat gcaggttcgc gcagaggtaa 30854
   taaaacgggg cccacggaga acaccaagtc cttcctgggg tttgcctgac cctacagaag 30914
   atgacgaaag aatcccggaa cccaataaaa taaataacca atacatgcat gttggatata 30974
   aaaacctatc ccattttatg aaaggacacc cccctgagag gttacgggta cacaaggtaa 31034
   atg cag cgg att cga cct tac tgg ata aaa ttc gag caa acc gga 31079
   Met Gln Arg Ile Arg Pro Tyr Trp Ile Lys Phe Glu Gln Thr Gly
   2715 2720 2725
   ggc gcg ggg atg gcc gat ggg atg tcc gga ata aat ata ccc agc 31124
   Gly Ala Gly Met Ala Asp Gly Met Ser Gly Ile Asn Ile Pro Ser
   2730 2735 2740
   att tta ggt tgc agc gta acg atc gac aac tta cta aca cga gcc 31169
   Ile Leu Gly Cys Ser Val Thr Ile Asp Asn Leu Leu Thr Arg Ala
   2745 2750 2755
   gaa gag ggg ttg gat gtg agc gac gtg atc gaa gat ctt aga ata 31214
   Glu Glu Gly Leu Asp Val Ser Asp Val Ile Glu Asp Leu Arg Ile
   2760 2765 2770
   caa gca ata cca aga ttc gta tgc gag gcg cgg gag gta acc ggt 31259
   Gln Ala Ile Pro Arg Phe Val Cys Glu Ala Arg Glu Val Thr Gly
   2775 2780 2785
   ttg aag cca cgc ttt ttg gca aac tct gtt gta tca ctg cgc gta 31304
   Leu Lys Pro Arg Phe Leu Ala Asn Ser Val Val Ser Leu Arg Val
   2790 2795 2800
   aaa ccg gaa cac caa gag acc gtt tta gta gtg ttg aat ggt gat 31349
   Lys Pro Glu His Gln Glu Thr Val Leu Val Val Leu Asn Gly Asp
   2805 2810 2815
   tca agt gag gtg tcc tgt gat cgt tac tac atg gag tgt gtt act 31394
   Ser Ser Glu Val Ser Cys Asp Arg Tyr Tyr Met Glu Cys Val Thr
   2820 2825 2830
   caa cca gcg ttc cgc gga ttt att ttt tcc gta tta act gcg gtt 31439
   Gln Pro Ala Phe Arg Gly Phe Ile Phe Ser Val Leu Thr Ala Val
   2835 2840 2845
   gaa gat agg gtg tat acg gtg ggg gtg cct ccg cgc ctg tta atc 31484
   Glu Asp Arg Val Tyr Thr Val Gly Val Pro Pro Arg Leu Leu Ile
   2850 2855 2860
   tat cgg atg act cta ttc cgc ccg gat aat gtc cta gat ttt acc 31529
   Tyr Arg Met Thr Leu Phe Arg Pro Asp Asn Val Leu Asp Phe Thr
   2865 2870 2875
   tta tgt gtt att tta atg tat ctg gaa ggc att ggg ccc tcc ggg 31574
   Leu Cys Val Ile Leu Met Tyr Leu Glu Gly Ile Gly Pro Ser Gly
       2880                2885                2890
   gca tct cca tcg ctg ttt gta caa ttg tct gta tat ctt aga cgc     31619
   Ala Ser Pro Ser Leu Phe Val Gln Leu Ser Val Tyr Leu Arg Arg
       2895                2900                2905
   gtt gag tgt caa ata gga cct ttg gaa aaa atg cgt cgg ttt tta     31664
   Val Glu Cys Gln Ile Gly Pro Leu Glu Lys Met Arg Arg Phe Leu
       2910                2915                2920
   tat gag gga gtt tta tgg ttg tta aac act cta atg tat gtc gtt     31709
   Tyr Glu Gly Val Leu Trp Leu Leu Asn Thr Leu Met Tyr Val Val
       2925                2930                2935
   gat aac aac ccc ttt aca aaa acc cgc gta ttg ccg cat tat atg     31754
   Asp Asn Asn Pro Phe Thr Lys Thr Arg Val Leu Pro His Tyr Met
       2940                2945                2950
   ttt gtt aag tta ctg aac cct cag cct gga acg gcc ccc aat att     31799
   Phe Val Lys Leu Leu Asn Pro Gln Pro Gly Thr Ala Pro Asn Ile
       2955                2960                2965
   ata aag gct ata tat tca tgt ggg gtg ggt cag cgt ttt gac ctg     31844
   Ile Lys Ala Ile Tyr Ser Cys Gly Val Gly Gln Arg Phe Asp Leu
       2970                2975                2980
   ccc cac gga acc ccc ccc tgt cca gat ggt gtg gtg caa gta ccc     31889
   Pro His Gly Thr Pro Pro Cys Pro Asp Gly Val Val Gln Val Pro
       2985                2990                2995
   ccg gga ttg tta aat gga cct tta cga gat tcg gaa tat cag aag     31934
   Pro Gly Leu Leu Asn Gly Pro Leu Arg Asp Ser Glu Tyr Gln Lys
       3000                3005                3010
   agc gta tat ttt tgg tgg tta aat cgc acc atg gta aca ccg aaa     31979
   Ser Val Tyr Phe Trp Trp Leu Asn Arg Thr Met Val Thr Pro Lys
       3015                3020                3025
   aat gtt cag tta ttt gaa acg tat aaa aat tca cca cgg gtt gta     32024
   Asn Val Gln Leu Phe Glu Thr Tyr Lys Asn Ser Pro Arg Val Val
       3030                3035                3040
   aag taa ataaaccttt tattttaagg atgggttgtt gcggcgtgtt tttttgtcat     32080
   Lys
   aaaaacaaga agttatatga agcaagggga tcgtcagtaa atttcaatga tggacttgaa     32140
   aaatcattat gagcatctag aagatggtta ataatagtga caaacgtgtg tataaggggc     32200
   ttgagatgag cagcgcagtc tatgggggga atacaggcac acgatggtag caattcccag     32260
   taaacaacag taggaggtcc atgtgggttg ggattacgaa ccataattat ctgtcttggt     32320
   ttataatcct tatcttgcat gtgtccgtat gtcagataag atgccggtat tgcttttgtt     32380
   tccgaatttt gctcctgaag actccagtat accggagtat aacggcgatc aaaaacatgt     32440
   tcaaaaaaat cgacgtatgt ttttctggca gcctccacaa cttgtccggc ccatatgtcg     32500
   tataggacat ggtcccatat gcaattttcc tgtatccgtt ctcgaatttt tgttgcggta     32560
   cataggagtc ctgaaagacg ctcctttaaa tctcgcgccg cctgacttcg ccatggcacg     32620
   ccaactaaat ccggaggcgt gtttgtttgt aaattccaca gccagcttcc cgtggtacac     32680
   caggtaaatg cgtgagtgta cacaccctcg acccggagta ccggttccat ggcgggtgta     32740
   aagttaagac ctagttgaat tcgtaataag cccgtgactg ctctttcgca ggtggccgct 32800
   aaatttaagg ccgaacaacg tgcctgatct gaacatacat tgcgcgtatg taaatcggtg 32860
   aagattcccc aaaatccatc ttttatataa gtacatatag caaagccctg ttccaaggcc 32920
   gtttgttcga cgcaaaggct aatttcattg gctataaaaa taatagcttt gtatatactc 32980
   gggagtatgt gccgtaaccc cccaaaaaaa gacaccaacg cgggttttag gcaccccatt 33040
   cctcgacgca ttctagccag gacgattttg cctaatctaa aatgcatggg gaacatagcg 33100
   cagtatatcg tgggaaaaaa ggcgctgaaa tcaaactttg ccatccaacg atcttgtagt 33160
   tgtacaggct gtttgaagac ggtagatatc cggcccgcgt cccaattgac agttgcatct 33220
   gcatgtccag tatcttgagg gatttgccca ataagatcct gcacccgcgg aggtaagggg 33280
   ggatgctgtc cccagggggc tagataatga aaggcctgaa taccggttac tggccacaac 33340
   ccatccgttt ccatgtacat ctccgtagcc gtgcgaatag attcgacggc tggagtttgt 33400
   gtccgaagag tggtccatcc gggaaatccg acagtacaaa agtagtctct gtcttcaccc 33460
   ttcggattga gttccgggcc taaatacgca aagattggac ggacaacccc gggttttcca 33520
   aacgccgccg cgtaccacag tctatatgtt gctaatattg aaagggaaga tgtaacggtg 33580
   gaatccgttg ttaaacagtc aaaataacac ggtaagagta cccttaccac aattgcgcga 33640
   ttgccactaa gaaccaagcg gcgttcttca aggttaaaca aatcggaggt ttggccttcc 33700
   cgctctatag ggggggaaaa cgtagactta tatttccgac gtatgtaggc ctttggtgta 33760
   attacctcca cgtctaaagg tgtttgttgc tgggtgtcac cgttagattt aagtgcggtc 33820
   aggcgggcgt aggctagcga cgtaccttgc gtgtgggcgt tacatgcgct gactgctgaa 33880
   acatcaatgg ccacgggaaa attagccctc aatactccgg tagatggcgt gttgtcatta 33940
   ttcaacgcgt gttcatcggg gattgttttt aacagaagac ctgtattcgt atcagtctcg 34000
   aatgttactg gcgaataaaa cgatttaaac cctaatacgc gccgtaaagt ggccagggca 34060
   gtgccatgga gacaacgcca ggtgtctgcg ttacccagtg gttcaagggg ccaggcgtct 34120
   aacgcagccg aggacgccgc cctgcagata gacgcaaacg ttgccgcaga aacactcccc 34180
   ccatgcctgg agtagcgaca taaatcctct tgttgtacgg taatttcggc aaacttaggg 34240
   acgtatttcc cacatgagtt tttgcataat aagtagtata acacggaaag accgttctgt 34300
   gttaattgtc cggactgtgt ccaggatgtg tatatgcttg cggcacaaat gtgtccgctt 34360
   tctctaacca aggtaatctg cgttgcgtcc atagcttggt tgtggttcag tgcattgtta 34420
   gaggcccttt gtgttgtaca gaggcttagt atttatgtaa ccccccctat ggatgtccat 34480
   atatggtatt aacgggttat aaactttcaa aatttaccgc cccgtttatc cgcggccacg 34540
   atcccgggga cagttccgat agcgctaatt gtagcatggc ttcggcatgc ggggccaagt 34600
   gcatttgatg tgcttgaaaa tacacgtggc gtgcccaagc gggggcagta attttgtaac 34660
   gctgttgaga gaacgaatgg cgctggctca taagcaggtt atacaactgg cggtaggtac 34720
   gacatgcaga acgatccacg ttaattgaat caagtaaagt ttctagatct ttttttttta 34780
   ggtttttcac acgagtactt cccggaaagg tctgggtact tttagttacg cgggcgccaa 34840
   ataattgttc ccaaattaaa cgaaatagag caatgttata ttcgtgtttt gtagggattg 34900
   gacggaagca ttgattatgg catccatcta agaatccggt tgttcgccaa accgggcggt 34960
   ttaatataat cttagcgtca gtaacgttac agcgcataac ctcaaatatt agttgagcca 35020
   gcgaatcccc gtgttgattg aaaagtgtat taatgtccgt atccaaaact tgtgttacac 35080
   agccttcggt ggacggattc cacttgagtg tttttgcaat ataggcacaa cggcgaaaca 35140
   gctcccaatg tgtggttaaa tttggggcgg tagttatagt cgcgatgcta aattccccgg 35200
   ttttgtccag gacgggaaac caaccggaag cgtagtgcgt gtatattttt tgaaatacgt 35260
   caaatgcttc caacgcttca ggtattcgta tacacgcccc caatattgcc gtattgacta 35320
   gtctaggctg ttctattgga catgcaaggg ctttaaatac tttaatttgt tcttcggtat 35380
   taattgactc cataagatac ttttccataa acgcggtaat gttatcggcc ataaatccgg 35440
   aagggccaaa atcggtcaag cagctgtcat tgtcctgtct aagagaacgc atacaggaag 35500
   ctacggcgtt agcgctatgt ctgagatcgt gtataaatgc acaaaattga actggggaaa 35560
   cgtccgttat tggacccatg ccatccaata caaccaaaat ttggttagag gccaaaagag 35620
   tttgtaagat attaatgcta tcggctaaac tccaaagaga gcatctctcg aaaaggtgtt 35680
   tgtatttaaa tcttgagaag agatgggtgt tcgagcgtgt aaatgcattt gcacatcgtt 35740
   gcctaaatgc acagcacagt ttgttagtta tttgggtgtg tgtaggaaac cattgaaatt 35800
   tatttgcgat ggtaaagttt agtaacattg gcgagaacag gggtccgcat ctggtccttg 35860
   agccatcgac gtacatcaaa acttcattaa gtagcaataa acgtacacgc cctaatgact 35920
   ggtagaccga taccatatcc ggcccatatg acattggctt tatgtaagca aacatgctat 35980
   gaaaatgagc catgtcaaaa ctcaatccaa cggtcacgac ggtagtgtac accaacacgc 36040
   gaaaatgttt ccattcgttt acattacata ggggccgagt tgagtttaaa ataagaatag 36100
   agtctgtaaa tattgcacaa aactgagcaa ctagctccga aaacgataaa gttgatgaaa 36160
   atatacagat gttatgccca cattgtaatc gtagtgctag ttcgtcaaaa aatgttccac 36220
   gtagttggtg tatggtacgt acatcctcgt gttcaggaga tcgtttaatg actcgcacaa 36280
   gcgtgtcgat gcccatatca cgcaggatcg tgcaagttct tccggagaac ccaactcccg 36340
   cgtatgtaca cacaattgtg tgtatgtttt catctccacg caatccggag attaaatcaa 36400
   taaactgcga gtttactgta gcatccatcg cgataatttg agaacagcga tttaaaagac 36460
   gatataatag gctatcaacc gcggaaagac gtctcattgt gggggagtat aattgtccaa 36520
   tcactgacat tacctcatcc agtattaata cgtcgtagct gtcgatagct tcgctggata 36580
   cgcggtgtag gctttcaagt tgcacaatca aacgtttaaa acccataata tatgtctcgg 36640
   atgtcaaata tgttacgaat ccggagaggc ctgcatcgtt aaaccgttga atcaacgtct 36700
   gggtaaagct acggcgacat gagacaacca gtacgctaat atctgccttt aacgcgtgtt 36760
   gaagccactc aagcaaggct gttgttttac cagagcccat aggggcacgt acgacggtta 36820
   ccgggcgagt ttgtgacata ccaggtttta ttagttttac tggaacatcc aattgcagtt 36880
   ccaggctaat tcccgggtgg gtgtgtttaa tccacgaaac cagatcccct ccatataacg 36940
   cccgcgcgag ctgtgtactg gacgcataga cggcggcgtt gctctccccg gtgttgggag 37000
   acatgggaac tcaaaagaag gggccgcgtt ctgaaaaagt ctcgccgtac gacaccacga 37060
   cacccgaggt ggaagcgtta gatcatcaaa tggatacgct taattggcga atttggataa 37120
   ttcaggtgat gatgttcact ttgggtgcgg taatgctcct ggctacgtta attgccgcct 37180
   cttctgaata taccgggatc ccttgttttt atgctgccgt agttgattat gagttattta 37240
   acgccaccct agatgggggg gtatggtccg gaaatagagg tggatacagc gccccggttt 37300
   tgtttttgga accacatagc gttgtggcat ttacttacta cacggcttta acggcaatgg 37360
   ccatggcggt atatacactg atcacggccg cgattataca ccgagaaacg aaaaatcaac 37420
   gtgtccggca aagctccggt gttgcatggt tagttgtaga tcccacaaca cttttttggg 37480
   gtcttttgtc attgtggtta ttaaacgccg ttgtgttatt attagcttac aagcaaatcg 37540
   gcgtggctgc tacattatat cttggacatt ttgcgacaag tgtaatattt acaacgtatt 37600
   tttgtggacg cggaaaattg gacgaaacga acataaaagc ggtcgcaaat ttacgacagc 37660
   agagcgtctt tttatatcgc cttgcggggc ctacgcgcgc agtgttcgtg aatttgatgg 37720
   ctgcgttgat ggcgatatgt atcctatttg tatcattaat gctggaactt gtggtggcga 37780
   atcatctaca tacgggactg tggtcatcgg tgtccgtggc catgtctaca tttagtacat 37840
   tgtcagttgt atatcttata gtatcagaat taattttggc gcattatata cacgtgttaa 37900
   taggaccgtc cctgggaacg ctcgtggcct gtgctacgtt gggaaccgcc gcgcactcgt 37960
   atatggaccg attatatgac cctatatcgg ttcaatctcc acggttaatt cccacaactc 38020
   ggggaacctt ggcttgcctg gccgtgtttt ccgttgtcat gttgcttctc agattgatgc 38080
   gtgcatatgt gtatcatcga cagaaacgca gtcggttcta cggtgccgta agaagagtac 38140
   ccgagcgggt acggggatac atacgaaaag taaaacctgc acatagaaat tctcgccgca 38200
   caaattaccc atcacaaggc tacggctacg tctatgaaaa tgactcaaca tatgaaacgg 38260
   accgcgagga tgagctgtta tacgagcgat caaacagtgg gtgggagtag cgtgggttgt 38320
   acttgtaaac ccccaagcaa taaaacaaac attcacagta accaaccatg atttgatgtt 38380
   tttattagaa cgtttatcag ggtttatcag ggtttaacat tttgcgcatt tggaatggga 38440
   gctctttccc gaaggttttc gttttggtgt tacatcgatc actcgtgggc ggcgttgtgt 38500
   ggatttatcg gcgtcctctg ttacattctc atcaaagtca aagtcttcaa agaactctga 38560
   gttcaaagca acggtttctc cgttgcacgt aacaagctcg ttgtaccgtt tgcacaatcc 38620
   acagattcct cctcgaccgc tggatgaaga ttgtcccatc gcaatgaccg caatgctgat 38680
   gtaatcgcgt tgtttcctgt gtctttaagt atttgtgtca ctctaggcac atcaacttcg 38740
   ataggcgtta gaacggctat aaccggaacc gcacagtggg gtgggatagt cggccagtta 38800
   tattcgtaaa tggcagccgc ttgtgtatcg acgcgcaatg ggactcccac ctcgtgtggt 38860
   cggcgcacac gtcctataaa ggtgacaagg aagggccgta gttttagcgc tggaaagtaa 38920
   ccggataata catagggttg tacggcaatt tgtttaaagt tagcgtgccg cggattggca 38980
   aatacaggaa tttgaagagc cgtatctcca gtggcccatg caatggttgt aattgtcccc 39040
   ttttgtatgt ccggatcatt aaatacccac actcgggatg agattgtccg attgaggccc 39100
   aaatgatggg cgtcgagttc tgtaagggcc ctcccacctt taaatcctaa acgtttccat 39160
   tcaacgtgat ccgttattaa ggcctcgcgt gtacttgggt ttgctgaggg tccaaagaag 39220
   cttacacatg ggttttttat agtatataaa aagtcccgta gatttgccat tgtaggtcta 39280
   tttattaacg tggtgtacgt ccgaccgagc gggttatttt tgtcatctgg atcaaagagg 39340
   tatttagcac gacatttaat ttcaaaaaat gaaatgtcgg tttctgcagg gtggggattt 39400
   agggtgccag ttaaagggtc cctgtcacat acaagaacgt ccaacgacgc gcctataagt 39460
   cccgttcgaa cgtcgattaa aagtccacat tcatacgttc cgattcttgg tgtacccaca 39520
   aggagacttg gagatttggt attgagcatg tcaaaattaa aaaacttagt tgtagcttct 39580
   ggcgttaaat gatcaggagt acggatatca gccggatcta taaacaatgc ttcaactatc 39640
   gctcgtgccg ccggttcgtt ggtttttcca aacgccattg ccgccgcatc accgtatgtg 39700
   tctgtagtgt tatggtggta aaaccactga ggtggaatga cgggtccaga tacactccat 39760
   ttaagagttg atgcggtgat taaatttcgt ctgagtagtg tccaaatggc attgtcgccc 39820
   tgaccacgag tctccgattc tattgttaat agaatacgtt taatgtcgtc ttgggttaat 39880
   ttggatgcta actcttccgg acgcggctcc tgaggctgtg tggacgtctt aaagaacagt 39940
   gcgttggtag aaccgggtgt tgtgtaggtt acatcatttg gagactccag agttgttaga 40000
   attttataca tgtgggataa acgaataaaa atacccgctg tcatcccgcg acagtccatg 40060
   ttcggtgatg tcttctgtag cgtgtctata aaatggtgtt caacgttaat cgtgttaata 40120
   tccgttttta caaaagggtg ctgtagacct cccacacggg caattttttt ggctggctgg 40180
   gggcttatgt cgatcctatc caatcccgat cgtgccataa taaagggtga tccgagcgta 40240
   tttctgaatt atcgtgacac aacaacgatg taacaacagt ggctgggtat aatgaggccg 40300
   gaatgcgtat gccttcgaaa agctttgagt gcgttacgcc gtaacgcact gcgtgacact 40360
   ggaatatccg gcgtaaagaa ttccgggaaa tacgggtgtt cagctctccg gcgtgtataa 40420
   agggattata atttaagtcc ccggggatgt cagatgggag tatatatggc gccaggacac 40480
   atcgatatgc cagcgtatca agcgccaaat ctggtgataa cttatgaccg tagtagtgat 40540
   aatgcggggt ccgatgtact gaaatgggca gttgtccggg aagacgtcct agcaggataa 40600
   cttctaagag tgcttggccc aatgcataaa gatcaatcgc aagtccaact ctttggggca 40660
   aggttccagt atatttagta aggcccgttc cattaatata atcaagcaag atttcagggg 40720
   gttggtttgt tccatgactc aacaccaaac gaaatgacat ctgcgatgca tcccggggta 40780
   cgcgtagtac gtgctccggg tgggatggat ttccaacttc aaatatcgct cgagtacaaa 40840
   gggaatacgt atttaatgtt actaggctat agtctccgat tactgctgtg gttatttcca 40900
   acgaggcaaa gttgtcgacg ttaagaaaaa tattgccaca tttcacatct aggtgggtca 40960
   ggccgcacgt tcgatttaaa aacgtcaacg cttgagccaa atctaaaaat acatgggcaa 41020
   tttttctgtc tatgtgatca ggtattgtca accgtctgga caggcgaaca atgtattcat 41080
   ccatatccat gtcgtatgcc ggaaacagta gctgtttggt ttgtaacgaa aaacctaaaa 41140
   ggcaaactat gctagaaata cctagccttt cccctgctcg tatagaaccc tcactcgcta 41200
   aaatcgcgtt aattaactct ctattaaaaa cacgactgtc catggttttt acagctattt 41260
   ttgacgatgg gtatatatga acacgcccat acgttccccg tcccgcaaac ctgggtctat 41320
   ctcgaatctg taatttagaa aaacataact gttcattgag cgtgaagatg ggtacaatta 41380
   aacgggataa tttggaaatt ctaaaaaccc atgggggttt gttaaattgt ggaaaaacgt 41440
   ccaccctttc gcgttgatgt attggatctc tcgttaagcc gtgctttaat aaaaataagc 41500
   ttgtctgttg tgatcgttct ggtgattcca tgcatgttgc agttgacgga ttggcccatg 41560
   gatgatttac acatccgtgt gtggggttgg tggattctcg ttcttgctga tcggctgcgg 41620
   ttgcatttgg ttcatgtccg tcggtattgt ggtgggaacg caaaggtcct gcagttggag 41680
   atatttggag gttggggggt gtgtcgtcag catccattgt gtaagtaacg cttcgtcgtc 41740
   ttctccaaac caagtatgat tttcgtgttc gatgttcggt cctaccgcgt cttccagtct 41800
   gtctatatta tcggtattat cgtcttctgt tttatccaaa ccgcgaataa aatcgggtgc 41860
   tatatagcgt ctgtttgtat ttaaaatctg ttgaatactt gtttgttcct caaccttgga 41920
   ttgtatatca cgaatcttct gttcaactga agctatgcga gctgcagagc ggagctggtt 41980
   atttaaatcg gcgcaggcct cttttgcagc agtaaaggca tacacaaatg ctggatcttg 42040
   tacactcgca cccccacgga ttagatcgag ggtccgttct ttaaataacc ccgcgcggtt 42100
   atgcgcctca actagtttga cacggttacg cctatgagaa gcgtaggttg gagtgtggcc 42160
   tgacatgacc aaagtgaatg aatgcggccc tgaaaatgtt tatgatatat acgtaacgag 42220
   tacgtaagat gacgcggctt cctgttttac tgcttacaag agatgaggcg gattccgtga 42280
   attgatgtta aatgtcattg ataatgtttg gtcgtacgct tggtgaagaa tctgtaagat 42340
   attttgaacg tctaaagcgt cgtagggatg aacgctttgg gacgttggag tcccctaccc 42400
   cgtgttccac gcggcaaggg tctctgggaa acgcaaccca aatcccgttt ctgaattttg 42460
   ctatagatgt aacccgacgt catcaggccg ttattcccgg aattggaacg cttcacaact 42520
   gttgtgaata tattccactg ttctcggcta ctgctcgacg ggcaatgttt ggcgcgtttc 42580
   tatcgtcaac agggtacaac tgtaccccca atgtagtttt gaaaccatgg cgatattcgg 42640
   taaatgcaaa cgtaagccct gaattaaaaa aggctgtcag tagtgtacag ttttatgaat 42700
   attcaccgga agaagcagca cctcatcgaa atgcgtatag cggtgttatg aacacatttc 42760
   gcgcgttttc tctgtcggat agtttctgtc agttgtctac ctttacacaa cggttttcgt 42820
   accttgtgga aacatctttt gagagtattg aagagtgtgg aagtcatggc aaacgcgcaa 42880
   aggttgacgt tccaatctat ggcagatata aggggacgtt ggaactgttt caaaaaatga 42940
   tcctcatgca caccacgcat tttatttcat cggtgctatt gggcgatcat gccgacagag 43000
   ttgactgctt tctgcgtaca gtgtttaaca cgccaagtgt ttctgacagt gttttagaac 43060
   acttcaaaca aaaatcaact gtgtttttgg taccacgtag acatgggaaa acatggtttc 43120
   ttgtaccatt aatagcttta gtaatggcca cgtttagagg aattaaagtg ggttatacgg 43180
   ctcatatacg caaagcaacg gaacccgtgt ttgagggtat caagtctcgc ctggaacagt 43240
   ggtttggggc aaattacgtg gatcatgtaa aaggcgaatc tattacgttt tcatttaccg 43300
   acgggtctta cagcacagcg gtgttcgcgt caagtcacaa cacaaacgtg agtgttttat 43360
   aaatttaacc tttaatatat tactgtaaat gttgacatat acctttccac aacggcggtt 43420
   gagttaaggt atactaggtg gttgtaggtt ccggttcacc cgataatctt tgtgtctcgg 43480
   ggaagcaaat tcgctgaagc agaccacagc cgttaataat agcccggctt aatgtttctc 43540
   caaacatata aagctgccac ccagatgaat ttactggtac agagagacca ctggcgttgg 43600
   ttcccgctat aacgtcgcca agatttgcgg taatgcgagg atttttagta ctcgtaattc 43660
   gaatgcaggt ggtgacatct acaaaaagaa cctgcgtggc gccaatgtct acctccactt 43720
   ttaattcccg ctgaccggcc tttctccaca tacacggagc ccaacacaca caaccttccg 43780
   catgatttgt gacatggggt aacgcataca gtgcccccac gtgaactcta tgattacatt 43840
   catcacatcc gtccgcatgg ctgaggagtc gatttaatac agagccaagt atccgagcat 43900
   gccatccggc gggacatagc cctattaaat taggttccat agccagtaca tataaacgcc 43960
   ttcgttcgtc tgaccaccac actcccggag aaataacttt acatgcgtat ggatttttcg 44020
   gaagccgcgg gggttgtaag tagttgctta agtttggcgt tggtgtaaga tctgcggggg 44080
   tgggatctgc tcgaggatcc ggaatagatg ttggaagggg gtacgcgatc gggttcttaa 44140
   acgttgctcc aaaaacatgg tctatgtttt caaccggata aattcttaaa gtcgccgtca 44200
   ttgcgtacga gacctcgtaa ttaaaattta caattacatg aaaagtcttc ggaggtaagt 44260
   tcatctgacg tgggcgcgtg atgtaaattg tggctacaac aacggcaata ttagtagtat 44320
   ccgtttgaag ggggataaac ggagcgatcc ttaaagttat aaaagcagtt gatcgcatta 44380
   ttttcacccg gggatcggtc aggatggact tccataatcc catatccagc gttaatgcat 44440
   cgcagagtct ctgaactgcc tcgggggtta atttgcgcgc tgcacccgta gcggtgtaca 44500
   gcggaaatat gcgttgtaat tccatgagca gaaaaacagt ctagcggatt gccgcccggg 44560
   tacttgtggg tttaatgcca cccacccggt tattttatat tttaagaggg ggtggaaacg 44620
   ggagaaatga cgtaaaatta catatgaaga gattctggtg ttatgttttt atagtgacac 44680
   taatttattt atgggggttg ggaatagaga agcagaatct gtctagaata ggtccgatta 44740
   acgatgcagg tagtgctgcc tgtagggtat cggtaataca aaaacatgcc gcaaatcccc 44800
   ccggtaaaac taaaatggat tgtaattgct ggttaaatcc tagacaaatg tacgcgtaac 44860
   attgaccggg taaatactta gaacaaattc caatatcaac aatatccgcg ctgcgtataa 44920
   atttacccct cagttgtgtg gaattaccaa taccaacctt ttctaaggct acgggaacgg 44980
   ggaccttgga aagcttaagt atttcccctc ctgaattata atagtcaaat aaatatatag 45040
   aacgattacc taaccagcat gggaaggaag cgtgaaggta gggtatatac cccccacctt 45100
   gtggtcgtgt atatacagat gacagatacg ccaaaaccgc atacatcaag gagctgttat 45160
   aaaacgcatc cattgacatt tccgttaaca ccgaaactat agtctgaatc aggtctggtg 45220
   tgcgggctac aatttcatca ataaccgttt gggaagaatc tgcaatatca tattccatga 45280
   gttgttgtag agtcgggttc gtttgtaact ccgttataag accttgggtt agcgatgtca 45340
   cacacgcttg tttaaatacc tggtttaaaa acatttcggc gcctggttta aaggcgggta 45400
   agggggtttg attatttagg acgttagcca aaaacggtaa acgcgcgact agctcttggc 45460
   gagctgtcac atgtaggctt tggggattgt caacccgggc atttatacac gcagcatcaa 45520
   taatagcctg tgcagagtga tataaaattg gacttccggt aatacgtcct ccccaggcag 45580
   aggatccgtt gtaagatact acaatcaacg gactggggga ttctgcgtaa tgtcgcggta 45640
   caattgatag gggacgccgt ttccagaaat ctgctggagt gtccccgcta actaattggg 45700
   cataacagat gtcgaaccat tccataagac tttggggttc tgtcgaagct ggggtaaaca 45760
   atagaacgtc ttgtaaaggt gggatgctgg cggacgaatt gtttttcttt cccgtaaatc 45820
   gcccttgtcc aggcggctca aggacgccat caaaggaacc gttattgatc ggatctgtgt 45880
   tggaagtttg cgctccgtgg ccctttgcac tttgaagcaa cccagatgca acgcgggaac 45940
   tagaaggtcg gacggggtgc ctggagttaa caatgtttac ggcccgtttt attagctcaa 46000
   ggacgtcccg attattttcc tgtatgcgtg tttcagcagg ggagtcatca atacctccag 46060
   aagttaactg tcgatcaaga tcgattatgg atgaaacggg tccaatattg tccccatttg 46120
   acgtgtgtga ttcacccatg gctgccacca tatgctctgc gtatattttt atagacgatg 46180
   caagacgagg ggtgcatcgg atatacgcaa tcagctgttt gcataataaa agtacccgtt 46240
   gtccatcagc aaaataacgc gttccgtttg ggattagttc tgcatacata atacaaatat 46300
   cacggtgctt gcggtttcca gtatttattc gtatcgctac aacgttaaat gcatcaaaga 46360
   ataaaccggg gctaagataa acaggcaatg ataaaatcaa tccccctgaa ttatgcgtgg 46420
   ccgaaaaaac gtgtgaaaca aatggttccg tttttggtat taagagattt gttaaggcgt 46480
   tatcgggaat gtacgcggcg aaaacttgac accacggttc gcattgacct gtagcatgat 46540
   atcttgtttg tacttcaacc ttgaagcgtt gtccgggttt ctttaaaatc agtaatgcgg 46600
   gatctattcc ggccgcaata agccccgcgt taggtatcac aacgtgtagt aatccttttg 46660
   tgtgatcatt atgccaaagt gcatgtttgg tttcatttgc caaatgggct tccattatac 46720
   accggatatg gttgtactgg aaaaaaaaaa gaaatatgta cgtattcaaa cattttttac 46780
   gtacgtggta tttaaggata catttaaact ttggtggggt aactatatat ctttctatcg 46840
   ttccagggta tccgaggtca agattttaat cttctgtttg tggatgaagc taattttatt 46900
   cgacctgatg ctgtacaaac tatagtcgga tttttaaatc aaaccaattg taaaattatt 46960
   tttgtttcat caacaaatac cggaaaagca agtacaagtt ttttgtataa cttacgtgga 47020
   tcgtcggatc agttgttaaa cgttgttaca tatgtatgcg acgatcacat gccgcgtgtt 47080
   ttagcacata gcgatgtcac agcttgttcg tgttatgtat taaataagcc ggttttcatc 47140
   acaatggatg gagccatgcg gcgcactgca gatttattta tggccgactc cttcgtgcag 47200
   gaaattgtag gtgggcgtaa acagaattct gggggtgtgg ggtttgaccg gccattattt 47260
   acaaaaactg cccgtgagag gtttatttta tatcggccgt caaccgttgc gaattgtgct 47320
   atattatcgt cagtgttgta cgtttacgta gaccctgcat ttacctcaaa tacacgagcg 47380
   tctggtactg gtgtagcgat tgttggtcgt tataagtcgg attggattat atttggattg 47440
   gagcactttt ttcttagagc tttaactggc acgtcttcca gtgagatagg gcgttgcgtt 47500
   actcaatgct taggccacat actcgcttta caccccaata catttacaaa cgtacacgtt 47560
   tctatagagg gaaacagcag ccaggattct gcagttgcca tatcgttggc tatagcacaa 47620
   cagtttgctg tcctcgaaaa gggaaacgtg ctatcttccg ctccagtgtt actgttttat 47680
   cattccatac ctcccggatg tagcgtggcg tacccttttt ttttattaca aaaacaaaaa 47740
   acgccggccg tagactattt tgttaaacga tttaactccg gaaatataat agcctcacag 47800
   gagcttgtat ccctaacagt aaagttaggt gtagaccccg tggagtatct atgtaaacag 47860
   ttggataacc tgacagaggt aattaaaggc ggtatgggta atctagacac aaaaacttac 47920
   acgggtaaag gtaccacggg aacaatgtca gatgatctga tggttgcatt aattatgtcc 47980
   gtgtatattg gcagttcatg tataccggat tccgtgttta tgcctattaa ataaaaacaa 48040
   gacgcgtgaa atgtaactag actggttgtg tttttattaa cacctgttta cacttgaatc 48100
   acggccgtgc ggtctcccgg atgaacagta gagggtccat caaacacaca gacgcgtaaa 48160
   attgcgcgag ttcccaaggt atcccccagt ctagatgatg ttgatatcca agatgaaata 48220
   gtttcatata tgggaaatcg gcgatggccg tcttgaatcc gcggcattgg cggtatatta 48280
   attaactggc cggtttctct aacgcaattg gccgtttgta tcaataaatt tacataaccg 48340
   tcgtgtgctc cttggactaa caaagttgga atcagcgcca ataaaagcag acatccctcg 48400
   tttatggaaa acattagatt taaaaccaat gtgcgaatag ccgcatccga tccgtcccga 48460
   tgttgtaaat ttgtttcgag ttcataacgc cttccattgt aacaaatgac atccgcgcct 48520
   cgtagaactt cgtggggtaa aatttgagca cccgcggccg tgcgctcaac ggcccgtgca 48580
   accactttgg cgatgatttc tcgcattaag gtctggggga tggttaaagg gaaaacaatt 48640
   tccagtccta cagagtccaa gcgaattgag tctgcggacc cgaaaaccgg aggaagtaag 48700
   cagatgtaat cgccattaca aagatcaacg ggggatgtat tttgaataaa caacccgggt 48760
   gtaggagtac ccacgccaat ggcatgcgcc aacaactttc ccgggatgac ccgtgttata 48820
   actgccgcga atcgcattcg gtatgcctct aacagcgaga gcgtgtctgg tggagcaccg 48880
   ttaatgtaat atgacgacag cgctatatcc accagtgaag ctcgatgacg cagggttgag 48940
   aaggtaataa tttttccctc acatttctgt aatgcagatg tttccgccgg ggatagttct 49000
   cctggtaaca atacctctat ctcaaatggc atagccatgt tgtttaagga agcgtgaaac 49060
   agaccaagtc ggccgagcgt atccaaatct caagtaagat gccaatgcgc tctacagggc 49120
   ttttattggg agtgggtatg tgggcgtggt gaaattatcg cggaagagga agacatcccc 49180
   taaggggcga cgcgtctcga atcagatatt gggctaagtg aacttcatct gccccggttt 49240
   ctcccgcgtg agccgttcgt aacatggccg catcgcttga gcatagtggt ggatatgctt 49300
   cttgaaaaag gccacacgga tcctgtgtca tttcggtaga accgggaggg cgtttaaatt 49360
   gatattcagt gtcggtggag ctttgcgacg ccacagcctt tgcctccatt agaagccgat 49420
   ccagtgtatt acaattagtg ttaacctccg ctggtgtaaa aaacttaaag catgggctgt 49480
   agataggaga agcgcctgta aggttgtatg ttccgttgta tagcctgtca ccgtatgaat 49540
   gtttttgtga tgcccatggg ttaagagttg ctcgatctgt ataagcaaca tccgattgtg 49600
   tgtgatcaaa cattatagcc tctatgtcgg cgtcacggtc acccatatat aacattccag 49660
   atgctcgacc tctaggattg catgcagttc taaaatattg taggtcagtg gacaccggca 49720
   tggccacaaa ctcacacaca gaggcttgcc ctcgtgcaat tcctgccgat gtagcaggac 49780
   gtagtccgcc gaagatgggt aggggttcgg tgggatttaa gcgacccccc gatgctgtta 49840
   tacgacgcaa cgattcggta acgttatcat gtaacattgg cactcctcct cgtgaaaaaa 49900
   aaagattttg ggcagtattg cccatatccg tgagagggca tcgcagggct gctgtggcac 49960
   atacagctgt atacccgact cccaggtcca cgtgagctct gggttgggtt agggtaaagt 50020
   ttaccccccc aatagcatca tgatggtgta cttggatttg tccgacaaag tacgattcag 50080
   aagcacgctc ggcatataaa agttgctctg tggcaaagcg atcctggcgc actacggtaa 50140
   aggcaatccc ggggtgaaat cccgttcgta gttgatgtgt aagagatatt ggtgtaaact 50200
   taaaataccc tccaagaaga gaatatgtaa gagtattaaa atccgactta ctatgcgtta 50260
   catggtaggc aacgggttgg cgtatagttg cgtggtggtt ggctcccaga aaaggaggga 50320
   ttggtggtac catttttgcc aaaacccgcc tagcatttgt cattccacgc aatgatgcca 50380
   aatgttccgg acatgcaaac agagggttga ccggaacggg ataaaaaaaa gtacccgttg 50440
   caatggtttc atcgtacgcc tgatatgcca tcataataag gccatggtaa agcgctccgt 50500
   catatattct catatttctg gttgttgccg tggcggctcc cgcatcgggg gcgcttgata 50560
   ctaaaatggc cgtcgttcgt tcagccatat ctcccattaa ctcttgtagt gtgagcaatg 50620
   catcaccatc aacggttagg tgtgcattat ggaagtaaac gttaagagag ttcggaacga 50680
   gttggtgtgc gtgaagaggg tgtcttggat cttctggggt agttggggct tcttcatcag 50740
   cgggaatttc cggaacgata actgcctgta acgcagggta taggcgatca taacgcacgc 50800
   ccattgtaca acaggaaccg cgggaaaatg caggaatgac aatatagtag taaatcttgg 50860
   agagaatacc ccattcacgg tcatggtgtg gagtaatggg aataccctga cccgtgtctc 50920
   cccgaacggg tctcccgtgg attaacacat tatcgcgtcg ttggaagtta tgcccggcca 50980
   tatccacaaa gtgaagggct tggtatccgt ttcgcccgct tacacgaatt gcggggagtc 51040
   ggtctcgggc ggcttcatca cggtatatta acgcatcaca atcccataga ataggtgcaa 51100
   taaacgtgtc atccgtaagg atgttattta gcgcttccga ggtctcgccg ttatggccct 51160
   ctccttgtat tgtaaaatcg gttatagttt ggcgtaatgc tcttacatgc tgcagtaaat 51220
   cccgatatat attaatacag acttcgggaa gctcaccgtt tccgagatat gtagttatgt 51280
   acattaacat gtgaaagtta tttacaaatg ccaccctgtg ggattgctcc caatacccgc 51340
   gaatacactg tgttaacagt cgcagtaacg cacagaagtt tctttcgttt ccatgaataa 51400
   cagcctctag catgtagaaa atagttgggt atgcgcggtc ttcaaatgta tcctttacgg 51460
   ctttaatggt tgccggggtc attgtatgtc ttcccaaacc gagttgggtt ccgcgacagt 51520
   ctcgaaatga aatgggacat agaggcacgg gaatgtttcc gttgataatc cgtaatgttg 51580
   cgttaacggt tggcatggct tccgggggac gctgcggtcc gggaaggtct acgtcccccg 51640
   gtgcaacaaa aaaatcaaag gcggggttta attcaaacct tagacgcggg ttgctcggag 51700
   ctataaactg tagaattgtt aaatgttcgt ttacccaatg agggtgatga ggcatcatat 51760
   ctgcccacgt ttccataaac ctccccattt gaacatccaa tgtgtcctct gtaccctcgg 51820
   ccacgtatac accaaaataa cactgacgat ctaaatgttg ttggcggagg gcaacaagtg 51880
   tggcctcgac atcaagcaat gaactgtggc agatggtacc cattgcatca cgaagagtca 51940
   actgtgttaa tatcccatct ttattataaa aaaaaatccc ttggggtgga aattgacgtg 52000
   gatcctgttc ggatacagtt gaaaaatcgc cggcgtgtcg tgtatatctg tccatggagt 52060
   ttgcctgaaa cactcccatt ggcatgataa acgtaatatc tatatttcca ataagagggt 52120
   aggcaacgcg cgtagcttgg tagacccgcc gttctaatgc ttctaagaat acaagtttat 52180
   ccccaacaat cactaaatcc gcaggaacac gggcgttaac cggaagccca gctgtcgttg 52240
   aatcggagtt ggattgtggg ggtatagacg gtatgtttaa cgtgttaggg tcggttatat 52300
   caaggagatg gcgggctaca tcatccattc cgcggacggc ctttcccatc acaagggcgg 52360
   ttaccaagtt tgtcccctgt agaaccattt cgccatatgt tactggtacg tcagcggcgg 52420
   tgtcgtcaat ttgtaaaatt ccctgtaata gttgccgttt taaggttgct gttgttacca 52480
   acacaccgtc aacctgccgt ccgcgagtgt ttgtatgagt tattcgtgat accatcaccg 52540
   atggttgggt gcacgaaacc atatccgaca gatacgcaga gatcagccta ggttccctgg 52600
   cgtgtcgggt cataaaaaac atatccgcac agactctacg tttgaggtcc gaaagtaggg 52660
   ccgcgcgtgc aacacgattt agatgtccct cgggttggaa tttattaatt ggtgaaagca 52720
   gcgatagcgg tggggctttc tccaatagaa caccaagaag ttgatcggca gtgcctcgtt 52780
   caaatgagtc caaaacggtg cgtaaattac gagccatctg ttggatagct cttatacgta 52840
   acgatgagtc aatctctgtc ccatcgacat atgtgttact taacaaactc aacgcttccg 52900
   atgcaattgc aaacgcagcg cttaacgaac gcttgtgtat ccgcttaacc atataattat 52960
   gaacaggctg atcgacggga tgtgggccat cacgtgctat catgggttgt tgtacctcaa 53020
   attgaataac gccatctcgc acgtaagcca gctccggaaa tttagtacag atgcaagcga 53080
   cagacagtcc aagttctaga aaacgcacaa agtttaatgt attgcagtat gtccccaaaa 53140
   gaatatcgaa ttgagccgag taaaggctgt tatcatctga tcgtatttgt ttaaaaaaat 53200
   caaaaatgca acggtgtgca cacacctcta tggttgacag cacatttcca gttggaatga 53260
   tccccgctgg gatgttaaat aacccagcaa tacgatcaga ttccgttgta gtaatcacgt 53320
   tagcgggaca tgaaaccgtt gtcattttgt cagcagcgtt gcgacttgaa tccggacgtg 53380
   taaacgtgtg cgggaacaag aaaactgcga gaagtttgat gtagagtgga acatatgtat 53440
   gggtgtttcc aagtaacacg ggggcggtaa ttataaatac ccaacattat ttttagactg 53500
   aacaattata gtacatttat tgaaatggac taaaacgaaa tagatgtttt taacataaca 53560
   cggacccaca cccttggccc aataaaggat accgtgtcca ccaaacagtc catagcgtaa 53620
   ataactgccg ccattaccag taacacggct ggacttggac cccacgacac aggaagtatt 53680
   tttgaaacgc tatctggaaa aataacctca ctttgcaagt gtccggtaat caaaaagaac 53740
   acccgggtgt ctataaaaag agtataatac gccgtaatac aaaatagcat tgttcgagtc 53800
   acaacaataa ttccaagcag cagttgctgg gatgtagata agggtagcag gtctgatcgt 53860
   atatttctgt agacacacaa taacataata ataagtcgaa agtgatgtag aatcgtcaga 53920
   agtaacccgg ctgtgaataa gagcatacag cgtgtcgctt tatagtaaaa aaaaaagaag 53980
   atacaacata ttggttgaat caacaccgtg gatataatat aaaacacgga gtgttgcgta 54040
   aacgccgacc tgtttacggt agatgtgcgg gtacatgcag agcttaatag taagtccgta 54100
   tcacccccat acgataacat aaccatggtg ttataaagcg gtgaattatc actggattta 54160
   cacacttttg cgtcctcttc ggggtgctgg ttaaccataa cgctaagcaa gtcctttgtc 54220
   tgttccgaga cgcgggcttg gggtgggttc ataacgttca gtcgcagttt atagtccaaa 54280
   gtaaaaatgt tgacaaagtt aaagccgatc cttgtattat gtagaatata ccccgcctga 54340
   cagactgtta taacaccctc tctaaatgtt tacgtatgtt tggagcatgt cacatagctg 54400
   acgtgctaaa cggtaggagg tgtggttgat tggattgatt ggatcttttc atatgacgtt 54460
   aatgatacca cggtgttttg aatgtttgcg tgtagttata tatttctcat attcggtgtg 54520
   gacctaaggt tggcgatcac acgatcacgg gacggcaagt tcgaggttaa tgttccgata 54580
   aacgctttaa t atg gaa ttt cca tat cat tca acc gta tct tat aac 54627
   Met Glu Phe Pro Tyr His Ser Thr Val Ser Tyr Asn
   3045 3050 3055
   ggc gta acg ttt tat ttt aac gag cgt gct acc agg gcg tat ttt 54672
   Gly Val Thr Phe Tyr Phe Asn Glu Arg Ala Thr Arg Ala Tyr Phe
   3060 3065 3070
   ata tgc ggg gga tgc tta att tcc ata ccc cgc aaa cat gga ggc 54717
   Ile Cys Gly Gly Cys Leu Ile Ser Ile Pro Arg Lys His Gly Gly
   3075 3080 3085
   gag atc gca aaa ttt gga cat gtt gtt cgc ggg gtc gga ccc ggt 54762
   Glu Ile Ala Lys Phe Gly His Val Val Arg Gly Val Gly Pro Gly
   3090 3095 3100
   gac aga tct gtt gcc agt tac gtt cga agt gag ctc aat cgc acc 54807
   Asp Arg Ser Val Ala Ser Tyr Val Arg Ser Glu Leu Asn Arg Thr
   3105 3110 3115
   ggg aag aca tgg gcg gta tca tcc aat aat aac tgc gtg ttt ttg 54852
   Gly Lys Thr Trp Ala Val Ser Ser Asn Asn Asn Cys Val Phe Leu
   3120 3125 3130
   gat cga gtg gcc tta ctt gca gcg gga tcg ggg gcg gtg gat cgc 54897
   Asp Arg Val Ala Leu Leu Ala Ala Gly Ser Gly Ala Val Asp Arg
   3135 3140 3145
   gac ctt tgc gga aca ttt gat gtt gaa gtg gag gac cct acg ctc 54942
   Asp Leu Cys Gly Thr Phe Asp Val Glu Val Glu Asp Pro Thr Leu
   3150 3155 3160
   gca gat tat ctc gtg tcc ctc ccc gtg acg cat tta aca ttg gtg 54987
   Ala Asp Tyr Leu Val Ser Leu Pro Val Thr His Leu Thr Leu Val
   3165 3170 3175
   gcg ggg gta gat gtt acg cgc gag aat aag cta aaa ttg ttt cca 55032
   Ala Gly Val Asp Val Thr Arg Glu Asn Lys Leu Lys Leu Phe Pro
   3180 3185 3190
   aca ccc acc gcc att aat acc aca aat ggc ttt atg tac gta cca 55077
   Thr Pro Thr Ala Ile Asn Thr Thr Asn Gly Phe Met Tyr Val Pro
   3195 3200 3205
   aac gaa gcc agt ttt tca ttg gtg tat atg cgt atg ttg gag tta 55122
   Asn Glu Ala Ser Phe Ser Leu Val Tyr Met Arg Met Leu Glu Leu
   3210 3215 3220
   cca gaa agt ttg cag gag cta gtg agt gga tta ttc gac ggg acg 55167
   Pro Glu Ser Leu Gln Glu Leu Val Ser Gly Leu Phe Asp Gly Thr
   3225 3230 3235
   ccc gag ata cga gac gcg ctt aac gga agt aac gac gat gaa aaa 55212
   Pro Glu Ile Arg Asp Ala Leu Asn Gly Ser Asn Asp Asp Glu Lys
   3240 3245 3250
   aca agt ata att gtt agt cgg cgc gct gct gat gtg gtt acg gaa 55257
   Thr Ser Ile Ile Val Ser Arg Arg Ala Ala Asp Val Val Thr Glu
   3255 3260 3265
   gac gta aaa gca gat gat gtg ccg att tcg ggt gaa ccg tat tct 55302
   Asp Val Lys Ala Asp Asp Val Pro Ile Ser Gly Glu Pro Tyr Ser
   3270 3275 3280
   gag aaa cag cct aga cgg cgt aag aag tcc gat cat att aca cta 55347
   Glu Lys Gln Pro Arg Arg Arg Lys Lys Ser Asp His Ile Thr Leu
   3285 3290 3295
   agt aac ttt gta cag att agg acc atc ccc cgg gta atg gac att 55392
   Ser Asn Phe Val Gln Ile Arg Thr Ile Pro Arg Val Met Asp Ile
   3300 3305 3310
   tgg gat cct cgc cat aaa gcc act act cat tgt atc cgc gcg tta 55437
   Trp Asp Pro Arg His Lys Ala Thr Thr His Cys Ile Arg Ala Leu
   3315 3320 3325
   tca tgt gcg gtt ttt ttt gcg gac gag gtt ata ttt aaa gcc aga 55482
   Ser Cys Ala Val Phe Phe Ala Asp Glu Val Ile Phe Lys Ala Arg
   3330 3335 3340
   aaa tgg cct gga ctt gaa gat gaa ctt aat gaa gcc cgt gag acg 55527
   Lys Trp Pro Gly Leu Glu Asp Glu Leu Asn Glu Ala Arg Glu Thr
   3345 3350 3355
   ata tat act gca gtt gtt gcg gta tat ggc gaa cgg ggg gaa ctt 55572
   Ile Tyr Thr Ala Val Val Ala Val Tyr Gly Glu Arg Gly Glu Leu
   3360 3365 3370
   cca ttt ttc ggg cat gct tac gga cgt gat ctg acg tcc tgt caa 55617
   Pro Phe Phe Gly His Ala Tyr Gly Arg Asp Leu Thr Ser Cys Gln
   3375 3380 3385
   cgg ttc gtg att gtt caa tat ata ctg tct cgg tgg gaa gcg ttt 55662
   Arg Phe Val Ile Val Gln Tyr Ile Leu Ser Arg Trp Glu Ala Phe
   3390 3395 3400
   aat tgt tat gcc gtt att gaa gat tta acg cgt agt tat gtt aac 55707
   Asn Cys Tyr Ala Val Ile Glu Asp Leu Thr Arg Ser Tyr Val Asn
   3405 3410 3415
   gcg tta ccc agt gat gat gac acg gat caa gtt gct caa gat tta 55752
   Ala Leu Pro Ser Asp Asp Asp Thr Asp Gln Val Ala Gln Asp Leu
   3420 3425 3430
   ata cgg acc att gtg gat aca gca aac agc ctc ttg agg gaa gtg 55797
   Ile Arg Thr Ile Val Asp Thr Ala Asn Ser Leu Leu Arg Glu Val
   3435 3440 3445
   ggc ttt att ggc acg ttg gct gaa act ttg ttg ttc tta cca ctc 55842
   Gly Phe Ile Gly Thr Leu Ala Glu Thr Leu Leu Phe Leu Pro Leu
   3450 3455 3460
   ccc cag ctt ccc tgt tac aag gaa acg tca cat ctt gca aaa aag 55887
   Pro Gln Leu Pro Cys Tyr Lys Glu Thr Ser His Leu Ala Lys Lys
   3465 3470 3475
   gaa ggt gtg cga att tta cgc ctt gca aaa aca gga gtt ggt tta 55932
   Glu Gly Val Arg Ile Leu Arg Leu Ala Lys Thr Gly Val Gly Leu
   3480 3485 3490
   tcg gat act gtt ccg gtt gat gtt tct gtc acg gaa agg cat gag 55977
   Ser Asp Thr Val Pro Val Asp Val Ser Val Thr Glu Arg His Glu
   3495 3500 3505
   tat gag ata tcc cgg tac tta gat acc ctg tac tct gga gac ccc 56022
   Tyr Glu Ile Ser Arg Tyr Leu Asp Thr Leu Tyr Ser Gly Asp Pro
   3510 3515 3520
   tgc tat aac ggc gct gtg cgt cta tgc cgt tta ttg gga tca tca 56067
   Cys Tyr Asn Gly Ala Val Arg Leu Cys Arg Leu Leu Gly Ser Ser
   3525 3530 3535
   att ccc att gcc ctg tac tac aat aca ata tcg ggt aat gcc ttt 56112
   Ile Pro Ile Ala Leu Tyr Tyr Asn Thr Ile Ser Gly Asn Ala Phe
   3540 3545 3550
   gaa ccg tat ttt gct ggg agg cgt tat ata gca tat tta ggc gct 56157
   Glu Pro Tyr Phe Ala Gly Arg Arg Tyr Ile Ala Tyr Leu Gly Ala
   3555 3560 3565
   cta ttt ttt ggt aga gtg cac caa aca cca ttt ggg gac ggg aaa 56202
   Leu Phe Phe Gly Arg Val His Gln Thr Pro Phe Gly Asp Gly Lys
   3570 3575 3580
   aaa acc caa agg tag tgtgtattat tcgcgaataa agtattgtag agaatacgtt 56257
   Lys Thr Gln Arg
   3585
   tatagtgact ttttattata catgtttttt atgtcagagg tattttatta tattctcgaa 56317
   ggcgggaatt tccacataac atccatccga taataccaaa ccccactacc gccagaaacg 56377
   cccctcctaa agaggcccca aggtattgtc ccgacattcg tatggcttgt cgtcgttcga 56437
   atcctagaag cgttaccaca gttccgtttg gaaacaacaa cacagcctta gagtcatccc 56497
   cgtgcatgtc tggattgaag ggtggaattg tggagtttcc catagcggct agttgtcgtt 56557
   ctgtatcttt gctgtcaata ataattatat ccataatcgc ccccgtggtt agatacctaa 56617
   gaaaaacact tccgcaatac aaacattctt ttaaattgtc cggatggggc agtgcgaccg 56677
   tctctatgac accatgttca gacacgcaag tatccctgct taaataaaca acggatatgg 56737
   ggttatatac atccacatct gccagggaat ataccaaacc cctttgaggt ttgtttcgtg 56797
   taatcacata actgtgtccc tggacagctg gaagaatgag cacatattct ccgttacgcg 56857
   cggctgcatc ttgacacgta aatacttctg gtacatgggt gtggagtata tgcaaatctt 56917
   tggcagtcca tgtggtaaaa atcatcatgg ttttaaatgc cgcgtcgaat atttcagatt 56977
   cgtctagttg ggtatgcaat acctcgttaa gtcctgggcg tgttggtatt gccgacagga 57037
   gattcataac atgaatctct tccgtgaggt ctgtacgaag ggaacccata caaggactgt 57097
   atacgtttgg tattgtaaac atgtgttttg aaggatttaa gtatgccagg ccttcttgta 57157
   tgttaagtgc tgcttgcgtg gcgtgagctg ccgtacacat ggatgtcatt aaaagcaaag 57217
   ttgtgcgacc gtctaatacc cgagatgaat tttctagtcc ttcgcggaaa tttaataaaa 57277
   tcattgaggc aaaaaataaa gcctgcctct ctgtagcatt taaattttgc ttcacaagca 57337
   gccccttgta tatgctcatt ataacacgcc tggcgagaga aagtgcattc acatggtctt 57397
   gaccacggga aagcgcgtat gcggtcttta gttgatcacg ggcttcatca tatgaaataa 57457
   aataatcgac atttgcgggt tttacctgac caaaaagaag tgaaagttcg tcatgaagct 57517
   gcgatggatc ggcaaagatc agatccgagg ttccggatat agtgttggga tgagctcctg 57577
   ctcctatccg cattaaaatg taattaagga attttaattg atactgaaca tctacgatct 57637
   catctaacag aaaatattct gtggtacggc ccatttccga caacgcaaaa attgatgtgg 57697
   ctattcttgc ggcgatatgg taatagcttt cctcgtttat gtcatgttga tccgccttcg 57757
   ttgtcatccg cagagcctcc gtgtgggcca tggataaatg atagcggtaa tccaaactct 57817
   cttcgggata tgcgcatata tcgaccgtag catgctttga aaaattcata tccaacccac 57877
   gtccaagtaa ataaactcga taagatggcc ccggatccgg acccggtgga tttagctgga 57937
   accatgtggt gtcgcttgta accgcgttca gttttactgt atgtggtaca acaattaatt 57997
   ctatcggtgg tccagaggat aaagaaatga gcgagctcat aaatcctaca ccaatattta 58057
   cttccacacg acccgagtcg cttgtgagaa gcaccgaacc ggtggcccag tatcgaattg 58117
   gccataccga cccaaaaagg ggaacgtgta ttctcaacgt tgagttggtg ataattgctt 58177
   cggcagaaaa aaaagtatgt cgggcaaggg catcccatgt agcaaaatgc gctttatgtt 58237
   ccagaatagt attcttgggg actgttgggc gagcgggtaa aagacttacc ccaaacggcc 58297
   tttctgcagt tgctgcggtc tcagctcttt ccaaatgcca tacaaggggg tttggtggga 58357
   acgtagtaaa cgcaacaaga tgttgcgtag ttaaatacgg cgcaaacgaa ctgtcaaaga 58417
   agctcacgtc gggtgtcaca gggtgactaa gaaatccaaa acgtccagca ggaaacggtg 58477
   ctcgatgttc tgctttgaaa tggtatggcg ataacaagta ttttggaaat ataaccagcc 58537
   cgacgtctcc ttcgtgtgtt gtagggttaa ccttaacaat aaccaagaaa acgtgttttc 58597
   tatcatttcc ccagtgaagt gatttaatga gttcttcatc gaaaccagta ggataaaagg 58657
   cttctaattt cagagacata ttacggtcgg aatattctcg tagaagagca gacatatgtc 58717
   cgatagagcg agtcgcaggg gttggtgtta cgtaagattt attagccgtg gtccaaagag 58777
   gaagaattac caccgctaaa actagcgcaa acatagtcgc tgttacttgc gctacaatat 58837
   caccgcaacg ttatgcacac agcgtgcctt tacccctcct cacccaccaa caggcttggc 58897
   ggcttttaat attaccactc cgttttaggg agataaaggc gggattaaag gatgtggtaa 58957
   taaatgacgc atatataaaa aagaaaaaca cgtacacgcg agtatgacaa tgtgtatcat 59017
   ctttttactg gtacatacgt aaatactagg tatatttagg aagtgttgtc ctgaacggca 59077
   ttaacaagct ctttcaatat attccatgca ccggaggaca tgtttgccgg ggtcatctgg 59137
   ggtagcagag tttttagttc ttgtgccgca tgctggggtg tctgttctaa cgataatacg 59197
   aacggagaca tgcttcgtga gcagtttgaa agggtctcca ttccccatgc ccataacggc 59257
   agaatatttc caaactctcc gcaaagctcc ggaagtttaa gcacggcgaa taacgtgtct 59317
   tcaatcccag gtacttcgcg tagttttata cactcggatt tttgtaattt ctgtttaaat 59377
   acatcattac aaaatgacag tgtgttccag cccgcgtgcc agttgttagt tttaagaaat 59437
   ataattgtat taataagcat tatatataca tttctcagaa ccataacaaa cggcagatta 59497
   accgtttctc ccggtctggc ccgtttgctt actctggata aatgactggg gagtgaaacg 59557
   gtacaaacta ccaagttggt cccggggggt tcagcgggaa gcgtaaacaa taacccagga 59617
   agcgccgctg gggacatatc tcccactaag tatctggaca agggaaaaca tatagttgag 59677
   gcgattgggt gtcggtcgga taacataatt ttatacggct ccttatttac ttgtacgaga 59737
   tccgatgtac ttgtgtccat caatgccgag attttcgcat gcataattgc atgcggagaa 59797
   cagaacaggc tctgaaaatg agccgtgagg cgttgtgcgt cttcaggcga aacgtctcca 59857
   ttaagacggc gagtttgtgt tccgtaaatt ccgcaaatgg cgtcctcccc tgcaaggtta 59917
   cgccaatacg acaggggctc cccaatgagt aagatccggt ttggtgttat tgcaaagtgg 59977
   tgtaaaaatt cttcggcggc ggttgttttt ccaattccat acgccccgtc caaataaata 60037
   cgcaaaacgc ccatttttac atcggtttta tccgttgaca tgtttattgt agacaaacgc 60097
   gtcttaggtt atcttctggg acggaacttc aaat atg tcc gct agt cga att 60149
   Met Ser Ala Ser Arg Ile
   3590
   cgg gcc aag tgt ttt cgt ttg gga caa cgt tgc cac act cgt ttt 60194
   Arg Ala Lys Cys Phe Arg Leu Gly Gln Arg Cys His Thr Arg Phe
   3595 3600 3605
   tac gat gta ctc aaa aag gat att gat aac gta cgt cga ggt ttc 60239
   Tyr Asp Val Leu Lys Lys Asp Ile Asp Asn Val Arg Arg Gly Phe
   3610 3615 3620
   gcg gac gcg ttc aac ccg agg ctg gca aaa ctc ctg tcg ccg tta 60284
   Ala Asp Ala Phe Asn Pro Arg Leu Ala Lys Leu Leu Ser Pro Leu
   3625 3630 3635
   tcc cac gtg gat gtt caa agg gct gta cgc ata tca atg tcg ttt 60329
   Ser His Val Asp Val Gln Arg Ala Val Arg Ile Ser Met Ser Phe
   3640 3645 3650
   gaa gta aat ttg gga cgc cga cgc ccc gat tgt gtt tgt att ata 60374
   Glu Val Asn Leu Gly Arg Arg Arg Pro Asp Cys Val Cys Ile Ile
   3655 3660 3665
   caa acg gaa tcc agt ggt gcc gga aag acc gtt tgt ttt ata gtg 60419
   Gln Thr Glu Ser Ser Gly Ala Gly Lys Thr Val Cys Phe Ile Val
   3670 3675 3680
   gaa tta aaa tct tgc cgt ttt agc gct aat ata cat acc cct act 60464
   Glu Leu Lys Ser Cys Arg Phe Ser Ala Asn Ile His Thr Pro Thr
   3685 3690 3695
   aag tat cac cag ttt tgc gag ggt atg cgc cag ctg agg gat acc 60509
   Lys Tyr His Gln Phe Cys Glu Gly Met Arg Gln Leu Arg Asp Thr
   3700 3705 3710
   atg gct tta ata aag gaa acc aca ccc acg gga tct gat gaa ata 60554
   Met Ala Leu Ile Lys Glu Thr Thr Pro Thr Gly Ser Asp Glu Ile
   3715 3720 3725
   atg gtg acc ccc ctc ctt gtg ttt gta tct caa cgg ggt ctg aac 60599
   Met Val Thr Pro Leu Leu Val Phe Val Ser Gln Arg Gly Leu Asn
   3730 3735 3740
   ctg tta cag gta act cgg tta ccc cca aag gtg att cat gga aac 60644
   Leu Leu Gln Val Thr Arg Leu Pro Pro Lys Val Ile His Gly Asn
   3745 3750 3755
   ctt gtt atg cta gcg tcg cat ttg gag aat gta gcg gaa tat acc 60689
   Leu Val Met Leu Ala Ser His Leu Glu Asn Val Ala Glu Tyr Thr
   3760 3765 3770
   ccc ccg ata agg tcc gtt aga gag cga aga cgt cta tgc aaa aag 60734
   Pro Pro Ile Arg Ser Val Arg Glu Arg Arg Arg Leu Cys Lys Lys
   3775 3780 3785
   aaa att cac gta tgt tct ctt gcg aaa aag cgt gcg aaa tca tgc 60779
   Lys Ile His Val Cys Ser Leu Ala Lys Lys Arg Ala Lys Ser Cys
   3790 3795 3800
   cat cgt tcc gct tta aca aag ttt gaa gaa aat gca gct tgt ggg 60824
   His Arg Ser Ala Leu Thr Lys Phe Glu Glu Asn Ala Ala Cys Gly
   3805 3810 3815
   gtg gat tta ccc ctt aga agg cct tct tta ggt gct tgt ggt gga 60869
   Val Asp Leu Pro Leu Arg Arg Pro Ser Leu Gly Ala Cys Gly Gly
   3820 3825 3830
   att tta caa agt ata acc ggg atg ttt tcc cat ggg taa gaaaacagct 60918
   Ile Leu Gln Ser Ile Thr Gly Met Phe Ser His Gly
   3835 3840
   tttaaagcag taccggttta tattcacgcc agttgacttt gtttgctgca gacacc atg 60977
   Met
   acg gcg aga tat ggg ttc gga tct atc tcg ttt ccg aat aaa tgt 61022
   Thr Ala Arg Tyr Gly Phe Gly Ser Ile Ser Phe Pro Asn Lys Cys
   3845 3850 3855
   ggg ata ttt ttg tct acc act aag aac ttt ata gcc ccc aac ttc 61067
   Gly Ile Phe Leu Ser Thr Thr Lys Asn Phe Ile Ala Pro Asn Phe
   3860 3865 3870
   ccc ata cac tac tgg acg gct ccc gcg ttt gag tta aga ggg cgt 61112
   Pro Ile His Tyr Trp Thr Ala Pro Ala Phe Glu Leu Arg Gly Arg
   3875 3880 3885
   atg aat ccc gat ttg gaa aaa aat acg tta acg tta aaa aat gcg 61157
   Met Asn Pro Asp Leu Glu Lys Asn Thr Leu Thr Leu Lys Asn Ala
   3890 3895 3900
   gcg gcc gtt gcc gca tta gac aac ctt cgc ggg gaa acg att acg 61202
   Ala Ala Val Ala Ala Leu Asp Asn Leu Arg Gly Glu Thr Ile Thr
   3905 3910 3915
   tta cca acg gaa ata gat cgt cgt tta aag ccc ctc gaa gaa caa 61247
   Leu Pro Thr Glu Ile Asp Arg Arg Leu Lys Pro Leu Glu Glu Gln
   3920 3925 3930
   cta acg cgc atg gcc aag gtt ttg gat tcc ctg gag acg gct gct 61292
   Leu Thr Arg Met Ala Lys Val Leu Asp Ser Leu Glu Thr Ala Ala
   3935 3940 3945
   gcc gag gcg gaa gaa gca gat gca caa tct gag gaa tgt aca cgt 61337
   Ala Glu Ala Glu Glu Ala Asp Ala Gln Ser Glu Glu Cys Thr Arg
   3950 3955 3960
   aca gaa ata ata cgc aat gag tct ata cac ccc gag gta cag att 61382
   Thr Glu Ile Ile Arg Asn Glu Ser Ile His Pro Glu Val Gln Ile
   3965 3970 3975
   gcc aaa aat gat gca ccg ttg cag tac gat aca aac ttt caa gtg 61427
   Ala Lys Asn Asp Ala Pro Leu Gln Tyr Asp Thr Asn Phe Gln Val
   3980 3985 3990
   gat ttt atc acc ctg gtg tac ttg gga agg gca agg ggc aat aac 61472
   Asp Phe Ile Thr Leu Val Tyr Leu Gly Arg Ala Arg Gly Asn Asn
   3995 4000 4005
   tct cca ggg att gtt ttc ggg cca tgg tat cgt act ctg cag gaa 61517
   Ser Pro Gly Ile Val Phe Gly Pro Trp Tyr Arg Thr Leu Gln Glu
   4010 4015 4020
   cgg ctt gtg tta gat agg ccc gta gct gca cgc gga gtt gat tgt 61562
   Arg Leu Val Leu Asp Arg Pro Val Ala Ala Arg Gly Val Asp Cys
   4025 4030 4035
   aaa gac ggg cgc att tcc cgt acg ttt atg aac aca acg gta aca 61607
   Lys Asp Gly Arg Ile Ser Arg Thr Phe Met Asn Thr Thr Val Thr
   4040 4045 4050
   tgt cta cag tcc gcc ggg aga atg tat gtt gga gat aga gcg tac 61652
   Cys Leu Gln Ser Ala Gly Arg Met Tyr Val Gly Asp Arg Ala Tyr
   4055 4060 4065
   tcc gcg ttc gag tgt gcg gta tta tgt tta tat tta atg tat agg 61697
   Ser Ala Phe Glu Cys Ala Val Leu Cys Leu Tyr Leu Met Tyr Arg
   4070 4075 4080
   aca tct aat agt gtc cac gaa cct caa gtt tcg tcc ttc ggg aac 61742
   Thr Ser Asn Ser Val His Glu Pro Gln Val Ser Ser Phe Gly Asn
   4085 4090 4095
   ctt ata gag cac cta ccg gaa tat act gag aca ttt gtg aat tat 61787
   Leu Ile Glu His Leu Pro Glu Tyr Thr Glu Thr Phe Val Asn Tyr
   4100 4105 4110
   atg aca aca cac gaa aat aaa aac agt tat caa ttt tgc tat gat 61832
   Met Thr Thr His Glu Asn Lys Asn Ser Tyr Gln Phe Cys Tyr Asp
   4115 4120 4125
   cgt cta cca cgc gac cag ttt cat gct cgt ggg ggg cgg tat gat 61877
   Arg Leu Pro Arg Asp Gln Phe His Ala Arg Gly Gly Arg Tyr Asp
   4130 4135 4140
   caa ggc gcc tta acg tca cat tct gtt atg gat gcg ctt ata cgg 61922
   Gln Gly Ala Leu Thr Ser His Ser Val Met Asp Ala Leu Ile Arg
   4145 4150 4155
   ttg cag gtc cta ccg cct gca cct gga cag ttt aat cct ggg gtt 61967
   Leu Gln Val Leu Pro Pro Ala Pro Gly Gln Phe Asn Pro Gly Val
   4160 4165 4170
   aat gac att att gat cgc aat cat acc gca tat gtg gac aag att 62012
   Asn Asp Ile Ile Asp Arg Asn His Thr Ala Tyr Val Asp Lys Ile
   4175 4180 4185
   caa cag gcg gcc gcg gcg tat tta gaa cgg gcc caa aac gtg ttt 62057
   Gln Gln Ala Ala Ala Ala Tyr Leu Glu Arg Ala Gln Asn Val Phe
   4190 4195 4200
   ctt atg gaa gac caa act cta tta agg tta aca att gac acg att 62102
   Leu Met Glu Asp Gln Thr Leu Leu Arg Leu Thr Ile Asp Thr Ile
   4205 4210 4215
   acg gcg tta tta tta tta agg cgc tta tta tgg aac ggg aac gta 62147
   Thr Ala Leu Leu Leu Leu Arg Arg Leu Leu Trp Asn Gly Asn Val
   4220 4225 4230
   tac gga gat aaa cta aaa aat aat ttt caa ctg ggt ttg att gtg 62192
   Tyr Gly Asp Lys Leu Lys Asn Asn Phe Gln Leu Gly Leu Ile Val
   4235 4240 4245
   tca gaa gca aca gga acc cct acc aac aat gta atc ttg cgc gga 62237
   Ser Glu Ala Thr Gly Thr Pro Thr Asn Asn Val Ile Leu Arg Gly
   4250 4255 4260
   gcg acg ggg ttt gat ggg aag ttt aaa agc ggt aat aat aac ttt 62282
   Ala Thr Gly Phe Asp Gly Lys Phe Lys Ser Gly Asn Asn Asn Phe
   4265 4270 4275
   caa ttt tta tgt gaa cga tat ata gca cca ctg tat acg tta aat 62327
   Gln Phe Leu Cys Glu Arg Tyr Ile Ala Pro Leu Tyr Thr Leu Asn
   4280 4285 4290
   cgg acc aca gag ctg act gaa atg ttt cct gga tta gtt gct ctt 62372
   Arg Thr Thr Glu Leu Thr Glu Met Phe Pro Gly Leu Val Ala Leu
   4295 4300 4305
   tgt tta gac gcg cat acc cag ctt agt cgt gga agt tta gga aga 62417
   Cys Leu Asp Ala His Thr Gln Leu Ser Arg Gly Ser Leu Gly Arg
   4310 4315 4320
   acc gta ata gat att tct tct gga cag tac caa gat cgg ctc ata 62462
   Thr Val Ile Asp Ile Ser Ser Gly Gln Tyr Gln Asp Arg Leu Ile
   4325 4330 4335
   agc tta att gca ttg gaa tta gaa cac cgc cga caa aat gtt aca 62507
   Ser Leu Ile Ala Leu Glu Leu Glu His Arg Arg Gln Asn Val Thr
   4340 4345 4350
   tcc cta ccc ata gcc gcc gtg gta tca ata cat gac agt gtt atg 62552
   Ser Leu Pro Ile Ala Ala Val Val Ser Ile His Asp Ser Val Met
   4355 4360 4365
   ttg caa tat gaa cgg gga ctt gga atg tta atg cac caa ccg cgt 62597
   Leu Gln Tyr Glu Arg Gly Leu Gly Met Leu Met His Gln Pro Arg
   4370 4375 4380
   gta  agg gcg gca ttg gaa  gaa agt cgc cgc ctt  gcg cag ttc aac      62642
   Val  Arg Ala Ala Leu Glu  Glu Ser Arg Arg Leu  Ala Gln Phe Asn
   4385                 4390                 4395
   gtt  aac agt gac tat gat  ctt cta tat ttt gtc  tgt ttg ggt gtc      62687
   Val  Asn Ser Asp Tyr Asp  Leu Leu Tyr Phe Val  Cys Leu Gly Val
   4400                 4405                 4410
   att  cct cag ttt gcc tcc  aca ccg tga gtattcacta tccggtccgt        62734
   Ile  Pro Gln Phe Ala Ser  Thr Pro
   4415                 4420
   ggggtgttta ta atg gct gct  gaa gct gac gaa gag  aac tgt gaa gcg     62782
                 Met Ala Ala  Glu Ala Asp Glu Glu  Asn Cys Glu Ala
                     4425                 4430
   tta  tac gtg gct ggg tat  tta gcc tta tat tca  aag gac gaa ggg      62827
   Leu  Tyr Val Ala Gly Tyr  Leu Ala Leu Tyr Ser  Lys Asp Glu Gly
   4435                 4440                 4445
   gaa  tta aat att acc cca  gag att gtg cgg tcc  gct ttg ccg cct      62872
   Glu  Leu Asn Ile Thr Pro  Glu Ile Val Arg Ser  Ala Leu Pro Pro
   4450                 4455                 4460
   act  agt aaa ata cca ata  aac atc gat cat cga  aaa gac tgt gtc      62917
   Thr  Ser Lys Ile Pro Ile  Asn Ile Asp His Arg  Lys Asp Cys Val
   4465                 4470                 4475
   gtg  ggt gaa gta atc gca  atc att gag gac ata  cgc gga cct ttt      62962
   Val  Gly Glu Val Ile Ala  Ile Ile Glu Asp Ile  Arg Gly Pro Phe
   4480                 4485                 4490
   ttt  ttg ggt atc gtt aga  tgc cct caa cta cat  gcg gtg ctg ttt      63007
   Phe  Leu Gly Ile Val Arg  Cys Pro Gln Leu His  Ala Val Leu Phe
   4495                 4500                 4505
   gaa  gcg gcc cat tcg aat  ttt ttt gga aat aga  gat tct gta tta      63052
   Glu  Ala Ala His Ser Asn  Phe Phe Gly Asn Arg  Asp Ser Val Leu
   4510                 4515                 4520
   tct  ccg cta gaa cgt gcg  tta tac ttg gtc aca  aat tac tta ccc      63097
   Ser  Pro Leu Glu Arg Ala  Leu Tyr Leu Val Thr  Asn Tyr Leu Pro
   4525                 4530                 4535
   tcc  gta tcc ctg tct tca  aaa cga ttg tcc ccg  aat gag ata cca      63142
   Ser  Val Ser Leu Ser Ser  Lys Arg Leu Ser Pro  Asn Glu Ile Pro
   4540                 4545                 4550
   gac  ggt aat ttt ttt acc  cat gtt gcg tta tgt  gtt gtt gga aga      63187
   Asp  Gly Asn Phe Phe Thr  His Val Ala Leu Cys  Val Val Gly Arg
   4555                 4560                 4565
   cgc  gtt gga aca gtg gtc  aat tat gac tgt acc  ccg gaa tct tca      63232
   Arg  Val Gly Thr Val Val  Asn Tyr Asp Cys Thr  Pro Glu Ser Ser
   4570                 4575                 4580
   att  gaa cca ttc cgg gtt  tta tcg atg gaa agt  aaa gcg cgg tta      63277
   Ile  Glu Pro Phe Arg Val  Leu Ser Met Glu Ser  Lys Ala Arg Leu
   4585                 4590                 4595
   ttg  tcg ctg gtt aaa gac  tac gcg ggt tta aat  aaa gta tgg aag      63322
   Leu  Ser Leu Val Lys Asp  Tyr Ala Gly Leu Asn  Lys Val Trp Lys
   4600                 4605                 4610
   gtt  agc gaa gat aaa ctc  gcc aag gtg tta tta  tcc aca gcc gtg      63367
   Val  Ser Glu Asp Lys Leu  Ala Lys Val Leu Leu  Ser Thr Ala Val
   4615                 4620                 4625
   aac aat atg ctt tta aga gat aga tgg gac gtg gtt gca aaa cgt 63412
   Asn Asn Met Leu Leu Arg Asp Arg Trp Asp Val Val Ala Lys Arg
   4630 4635 4640
   aga cgc gaa gcc gga att atg ggt cac gtt tat ctt cag gct agc 63457
   Arg Arg Glu Ala Gly Ile Met Gly His Val Tyr Leu Gln Ala Ser
   4645 4650 4655
   acc gga tat gga ctt gct cgg ata acc aac gtt aat ggg gtg gag 63502
   Thr Gly Tyr Gly Leu Ala Arg Ile Thr Asn Val Asn Gly Val Glu
   4660 4665 4670
   tct aaa tta ccc aac gcg ggt gtt ata aac gcc aca ttc cac ccc 63547
   Ser Lys Leu Pro Asn Ala Gly Val Ile Asn Ala Thr Phe His Pro
   4675 4680 4685
   ggc ggg ccc ata tac gat ctc gcg ttg ggt gtt ggg gaa tca aat 63592
   Gly Gly Pro Ile Tyr Asp Leu Ala Leu Gly Val Gly Glu Ser Asn
   4690 4695 4700
   gaa gat tgt gaa aag act gtt ccg cat tta aag gtt acg cag ttg 63637
   Glu Asp Cys Glu Lys Thr Val Pro His Leu Lys Val Thr Gln Leu
   4705 4710 4715
   tgt agg aac gac agc gat atg gct tct gta gca ggt aac gct agt 63682
   Cys Arg Asn Asp Ser Asp Met Ala Ser Val Ala Gly Asn Ala Ser
   4720 4725 4730
   aat atc tca cca cag ccc ccg tcg ggc gtt cca acc gga ggg gaa 63727
   Asn Ile Ser Pro Gln Pro Pro Ser Gly Val Pro Thr Gly Gly Glu
   4735 4740 4745
   ttt gta ctg ata cct acc gcg tat tat tca cag ctg tta acc ggg 63772
   Phe Val Leu Ile Pro Thr Ala Tyr Tyr Ser Gln Leu Leu Thr Gly
   4750 4755 4760
   cag act aaa aat ccg cag gta tca att gga gct cca aat aac gga 63817
   Gln Thr Lys Asn Pro Gln Val Ser Ile Gly Ala Pro Asn Asn Gly
   4765 4770 4775
   cag tat atc gtc ggg cca tat gga tct cca cac ccg cct gcc ttc 63862
   Gln Tyr Ile Val Gly Pro Tyr Gly Ser Pro His Pro Pro Ala Phe
   4780 4785 4790
   cca cct aat aca ggg ggt tat ggt tgc cct ccg gga cac ttc ggg 63907
   Pro Pro Asn Thr Gly Gly Tyr Gly Cys Pro Pro Gly His Phe Gly
   4795 4800 4805
   gga ccg tac ggg ttt ccg gga tat cca cca ccc aat cgt ttg gaa 63952
   Gly Pro Tyr Gly Phe Pro Gly Tyr Pro Pro Pro Asn Arg Leu Glu
   4810 4815 4820
   atg caa atg tcc gca ttt atg aac gca ttg gcc gcc gaa cgg ggt 63997
   Met Gln Met Ser Ala Phe Met Asn Ala Leu Ala Ala Glu Arg Gly
   4825 4830 4835
   att gac ttg cag acc ccg tgt gta aat ttt cca gac aaa acc gat 64042
   Ile Asp Leu Gln Thr Pro Cys Val Asn Phe Pro Asp Lys Thr Asp
   4840 4845 4850
   gtc cgt cgt cca gga aaa cgg gat ttc aag agc atg gat caa agg 64087
   Val Arg Arg Pro Gly Lys Arg Asp Phe Lys Ser Met Asp Gln Arg
   4855 4860 4865
   gaa ttg gat tct ttt tat agt ggg gag tct caa atg gac gga gag 64132
   Glu Leu Asp Ser Phe Tyr Ser Gly Glu Ser Gln Met Asp Gly Glu
   4870 4875 4880
   ttt ccc tca aat ata tat ttt ccc ggt gaa cca acg tat ata acg 64177
   Phe Pro Ser Asn Ile Tyr Phe Pro Gly Glu Pro Thr Tyr Ile Thr
   4885 4890 4895
   cat cgg aga cgt cga gtt tct cca tca tat tgg cag agg aga cac 64222
   His Arg Arg Arg Arg Val Ser Pro Ser Tyr Trp Gln Arg Arg His
   4900 4905 4910
   aga gtt tct aat ggt cag cac gaa gag ctt gct ggg gtt gtg gca 64267
   Arg Val Ser Asn Gly Gln His Glu Glu Leu Ala Gly Val Val Ala
   4915 4920 4925
   aaa ctg caa cag gag gtt aca gag cta aaa tca caa aat ggg aca 64312
   Lys Leu Gln Gln Glu Val Thr Glu Leu Lys Ser Gln Asn Gly Thr
   4930 4935 4940
   caa atg cct ttg tcg cac cat aca aat ata cca gag ggg aca cgg 64357
   Gln Met Pro Leu Ser His His Thr Asn Ile Pro Glu Gly Thr Arg
   4945 4950 4955
   gat cct cga ata tcg att tta tta aaa cag ctt caa agc gtt tcg 64402
   Asp Pro Arg Ile Ser Ile Leu Leu Lys Gln Leu Gln Ser Val Ser
   4960 4965 4970
   ggt cta tgc tca tcc caa aat aca aca agc acc cca cat aca gat 64447
   Gly Leu Cys Ser Ser Gln Asn Thr Thr Ser Thr Pro His Thr Asp
   4975 4980 4985
   aca gtt gga caa gat gta aat gca gtg gag gcg agt tcc aag gcc 64492
   Thr Val Gly Gln Asp Val Asn Ala Val Glu Ala Ser Ser Lys Ala
   4990 4995 5000
   cct tta ata cag ggg tcc acg gca gac gac gcc gat atg ttt gca 64537
   Pro Leu Ile Gln Gly Ser Thr Ala Asp Asp Ala Asp Met Phe Ala
   5005 5010 5015
   aat cag atg atg gtg ggg cgg tgt taa ccaacaaata aaagtattac 64584
   Asn Gln Met Met Val Gly Arg Cys
   5020 5025
   attcataaaa ggcgtgtttg gttttttttt ttttgttaag cggtgtcgtg ttaaacaaac 64644
   agaaggcgtt tttatgggtg gtaaggtttt atttaagtta aatttaatcg gtgtcagaat 64704
   cttcatcccc tgatgatggt tgttctcggt ctggaataat atatggcgcc atccattttt 64764
   cctctggggt atccggaaac acacgcggga tgcaaaccca tcgacccgtt ataatctcca 64824
   gtgccagtga aatatgacgg ccccctggac atgtgtttaa gtagtcgtat tggctgtcgc 64884
   ggttaaggcg aaaaggtttc gttgcacatg ctcgacaggt tagctgtccc acagaacata 64944
   cgtctcgggg tgtaccgtcg cggggtaatt gtaccggcgt gtgttctaac ctatcttcat 65004
   aaatcgcggg gggagaatct tttactgttt cattgagact tgaagcgcac tgtttggacg 65064
   gatgatgtgc gatagacgac ggttgttgta gcgcgttaat gttagacgat tccataataa 65124
   ccccgttaag atatcgggtg cggtagaggt acaaaacgcg ttttgtatat tatcgggtat 65184
   aaataccccc tgtgatgcgt aatggagaca catgagtaac gtaatacaca tttttattaa 65244
   taaattaaaa caaaccccct ggctatttac acccccgtta cattctcggt gcgaacacgg 65304
   gagtatcctc ggcgatttcg taaagcaagg ccggtaagac gtgaagttaa aagggcgcta 65364
   gtcttatttt ttttgcgggc tttagattct tggcgctcag ccgcagatac taacgtcata 65424
   tatttaatca tttcctgggc ttctcgaaat ttatcgggat caaacccgct atttaccgac 65484
   ggttcagtgt tttgtgagtc gccaatttct tctattgggg tatcagtagc gttgggtttc 65544
   tcggcaaagg gatccattcc ttccggtaac tgttttaacc ccttggttgt gagtggatat 65604
   aatgccttca tcgggcttgt tttaagttta agcacgtacc ggtacgcaaa aaaggccgct 65664
   accagtcccg ccaaaaccaa taatcccacg gccaatgccc caaatgggtt agataaaaac 65724
   gtggtaaatc cgtgtacggt ggaaagcagc gctcccgtgg ccccaagaac cacatgtcca 65784
   acggcctggc ccgcggtccc aagtccctgg aaaaactgag ccatgccctg cataatggcc 65844
   gttccgctat catattgcac aaccttgtct atgtcataaa aacgcagcga atgcatttga 65904
   tttcggcgtt gaatttcact gtagtctagt aatcctgtat cccgcagctc gtctcttgta 65964
   tatacttgca gcggcataaa ctctctatct ttaagaagtg ttaagtttaa atctacgtaa 66024
   gtgctaatca ttcccacatc atggactgcg atttcacgga cgtaacgata atcctcataa 66084
   tatacgtagt gatgcccaaa tagaaaatat cgcttgtgat tagccacgca tggttctaac 66144
   agatctctgg acataattaa ctcgttatct gttccaagct ggccctccac cgtcccggac 66204
   ccatttaaac taactattga aattaaagga cggctataac aacgcgtagt actaccagat 66264
   accctcatag agttttgaag tataatgcgt gtatctgatc ccagttctgg acaattagaa 66324
   acggagataa cgtcgccgag aatacgagct ttaacacgtt gatccaaaat ggtgctcgct 66384
   aaagcacttg ggttaattgg aaatagtccg ctccaaaggg cgcgttcgcg attttgtagc 66444
   tggcaccacg acgaggagat acgtgccaac atttcattaa catgctcttg aatgtggtca 66504
   tatgtaaact ggagcatagc aaattccacc gatgaggtgg ttgttattgt tctattggca 66564
   cgcaactcaa ctggcacgct tcgtcgggat ctggtatttc gagtcgggtg tttttgtggt 66624
   gaatgattag tgttttcacg gaccaattct tggagataga gacgggcgag ggaattgctc 66684
   agcaggggtt gaaacaccac aacaaacccc cctctggcaa ggtaggtctg gatatccccg 66744
   gttctaacat gagatgagtt gtatctggtt gtatagatcc ggttaataat agcccgggct 66804
   tcctccttta cacattgact gagatggatt tggttaagat taaactcgtt tgtttcactt 66864
   ataaacgtgg tagaaagtgt tttcattgta aagcgaaaat tgtgtgcata ctcatcgcga 66924
   actacgtctt caacctcacg ccacttgaca agcgaacaaa cttccgttcg ttttggcttc 66984
   cagttccaac caaccgttaa atgaggcgtg actaaaaagt tccgcgctgc aggttccagt 67044
   aatgctctag tgtcaagatc cctttgtcta taaccctcaa actggtgaaa acgatccatt 67104
   gcataattgg aatgttctct gtatgcacca tcccgtaggc caaaaaacgg ggacatgtat 67164
   attatatctc ccgtggaaag tccaaaacta tcataaggga atattgatct ggcttcaact 67224
   tcctcaatga tgcaattcac cgacgtgccc gtcctatatg ttccgggggt tccggcaacc 67284
   atgtacgtgt cattggtagt atgccatgct ttggatccca cagaattata ttttgatgcg 67344
   attagaggca tatcctgtgg atttttatcc tcattaaagg cttcaacttt gtggttattt 67404
   cgtacgtacg ttgctttaga agaacacttg ccaaacttat caatggtgtc cgtgatctct 67464
   gaaacgggaa ttggtaccct atccgcatat ctattagtaa tttgcgtata agaacttccg 67524
   gcccacgccg tgctaacgat aacatctttg taatataccg tcgccttaaa cttgtacgct 67584
   gcaatgtttt ctttataaac aacagcaata ccctctgtaa agtttttacc aaggtgataa 67644
   tccggacatg tccgagttgg ttctaatcgt acgattgtgg agcctgttgg cggtgggcag 67704
   acgtaaaacg tgggttttgt ttcggcgtcc tgggacttgt gtatagcttc tctgatttca 67764
   tcaccatcgc ccagatgagc agaccgggtt atatcttctg attgtgtggg ctctacttgt 67824
   aaactctcat aaaacgagct tggagagacc gacacaaccg ccgtaacaaa catagaaaat 67884
   atgcataaaa agcataacca cacccccgta acggaggtta tgaaaacgcc gggtccgttg 67944
   aatccggagc cagccgctgc attagggtgt atagaagaga aaaaacgtct gaatcgtaga 68004
   ttacgacggt attctggtcg atccctgttt ctccactttg aataatagcc acaaggggac 68064
   atgtttcttc gtacgttaaa taaatgccgt ctaagggtcc gtgggaactg cctatacctt 68124
   taggttgaga cgtgcacccg cgtggatcct tacctagacg gtcaacgcga cataaccgca 68184
   cctccccaca atggaaaaca gaggtgaata gtgtggttgc aaacacaagc tccctaatat 68244
   atttccaggc aagtctctgt gcaaaagtaa cgccttctac cccagaaaag caataaaagc 68304
   ccctaaatgg actgagaagc cagttggaac caacgacacc gcccataaac tttgccaatt 68364
   cctcttttaa gtgcggaaat aggccaacgt tttccacggt aaaaaataag gcggtgttag 68424
   ggggttgtgc aaaacggtgt tcatcgtgat taaacaatgg gccgttgacc aactcaaaaa 68484
   atttatgtgt aagtgcaggt aacatagccg catctactgt atgtcgaact aaggtattcc 68544
   ttataaatct atgtgcatca aaggccgctt cctgaatgcg attgtcgata atacaccctg 68604
   cctgggaaac cttagctaaa aacacgtttc gtgccccaaa cccattttgc atcgaaacaa 68664
   atgtctgcaa taaggcctct ccataaacgt ttaccctgag tgttttttct agctcttggc 68724
   gctgttcgcg gatacatcta ccgaggctgg ttagagagcg cttagacaga tgttcaagat 68784
   atactcgttt tctttccgca gcgtcaattt ttgctttgga taacaggttt tcccatccat 68844
   gaacagcaaa agttatgcta ctatcatcgt tcagatcaca ttcggatgct gtagcgctgt 68904
   cactcgcctc tttggttaaa tcatcctcac acccaactct ttctaaaagt tggcgtaagg 68964
   cggcttcgtt atcatgactc gggttagaaa tgtgacgcat gagaggcgac gatagatgat 69024
   gggcataaca ggctttaatt aatgcttcta tctgactgtc tggtgaggat gagcgattgc 69084
   caaccaaaat actatcaatt gcatccagag atccgagttc ttcagaaaac gccctatcaa 69144
   agtgtatggg agtttttcca aataacgcca actcaaccgt tacggctgta agttctgcgt 69204
   gtttttcatg ttcgtttaat acatccaaat tatcaacaaa cacgtctaaa gtacgttggg 69264
   gtccttcagt ggaattagac gttaaccaga atttaagctc gctgatggcg tacatacacc 69324
   gaggggcagg tttaaaaaca ttataagaat caagcatggt gctcgcaatt tcgtatgttt 69384
   ggtcaaacgt atcgttcttt gctcccatgg gattgactac cgtcttggtt tgaagtgttc 69444
   gtaacgcttc aacggcggat tgacgtttga tatccggtac atcagggata tatggtaaac 69504
   accgaataat atcgtcaaca tccacgttaa cccgaacttg cttagtaacg tgatcgcaga 69564
   tacatcctaa taatctacga tgtaaggtct caccttggtt agctgttata cataattctt 69624
   caaaacatat agcacatgga tgcgccggat caaacaactc cacgggtggt attagaccgc 69684
   ttcctatagt atctgtcata aaccgcgtca cggtttccaa tgtgtttagc gcctcacgag 69744
   aagacgtgat aatatagcag taattaagct gttttaaaaa attctcaatg tcgtgtaaaa 69804
   actgaatttc actcgatacg taacctccgt atgttgttaa cgacagctca tggtgatatt 69864
   ggcaggtttt taagttaaac gctgagacaa agtaatcttt gatacagttt gagtctttag 69924
   tctcaccata aacaaaacgc gttttaaaat gttgtagcaa ctcaaacaaa cgctcacctt 69984
   ctccctttac ataacataat gcccaatgca gtgcacaaac cgtgggagtg tttccaaatt 70044
   ttagctgggt attaaatccc cggagaaaca gctttaataa ataacgaact gtcaaacagt 70104
   tagccgctaa ccgatataaa aagcgacacg ccacccttgg atcacatcga cgtagcagtt 70164
   caacctgaaa gatgtacgta taaacttgac ccagtagaac caacaatgtt cgattaatat 70224
   ccaattccat gttatcgggc tgttactgtc cactggactg taagctgaat ggttggagac 70284
   acactatatt acaggtcata tgggtaagga ctacacacaa aaaaagaaaa cacaacagaa 70344
   tatgtaatat tgacttgttt attacgtaca ctcacccgcg tgtgggcttt aattggataa 70404
   agagggaggt taaatcattt ccattgtaat gttcccatgt tttatgggaa taccactaag 70464
   atcaaagagt tcgtcatctt cggggggtcg tttaagacca gggacagttg accctgaaat 70524
   gtttgaccct gaaatgttta atacgttaga tgtggtgtca tggcttggtt cacaagaatc 70584
   aaaattaaat gctaggtttg tgggggtttc cccatctccc gctgtcgttt tttcatctag 70644
   aatctttact gcttctagag cgccttctac ggtccagggc gtttccaggg tttggataat 70704
   ctggtcgtgt aactcctcca agtctctggc taaaaactca tcgtctgtaa gacttaacca 70764
   gtcgtcaaat gccatatgtt gtgctcgagc gcccactgca cgcacaaccg tggcgtatat 70824
   ggctaattga accatggccc cgccactgac aattataccg cgaacttggt cggatagggt 70884
   ggtttctcga tttcccgacg agggacctgt tacggggcag ataaaacctc ctctaggaca 70944
   tgctattata aaacggcgag tgcgatcaaa tgtaaatagc gggcatacat ttttaccccc 71004
   gtttaaaccg ctccaattcc ctgcctgaaa gacgcggttg tttcctgccg ctccgtgata 71064
   tttactaatg cttattccta aaacgaccat gggacgttga ttcatggccg cgcggactaa 71124
   atgagttgaa gtaaaagccg tagtccataa ttccggtaag ttttccgttt tttcaagaag 71184
   cgcctttgct tgggtttcta tgtccgcggc ggacgtgaca tctttacgaa tccaatgcaa 71244
   aacggatgat gggtcacgcg ggcgcctggc acccgtaatt atagaagtta aggtatttat 71304
   aaggtactgt gaatgatcgc agtatttaag aataagattt gccatataaa actgggctaa 71364
   ttctcctatg caggttgggg gtagattaat aaagtttatt gctgcatatt cctcggtaaa 71424
   ccgtttaaca gctgcaatag tggtaatctc ttcgtgtgta agtttatctg ccggcatctg 71484
   gttgcgttgt aacagggtcc aaaaccactg cgggttgggg gatttactgt ttggtggcat 71544
   accccgagga aataacaggc cgtgaaactg tttaagcaaa aaccctaggg ccccgtgtaa 71604
   catatccact cttttttctt ggcgttggta cgcacttgca aggcctacaa gccttgcccg 71664
   ggctgcctca gagagatttg tacagttacc tgaaaaaacg accctatttt taactcgtat 71724
   atcccgaata acttccacgc ttacgcgcgc taaatcccca tcaaaggtac gccccgcggg 71784
   cgcgtcttgt cccaatgtcg gatttggggc ggatacagga ccttcagata atgttacggt 71844
   tatagagcgt gttgatataa accccccatt aaacaggtca acaaaacgcc gccgcaaaac 71904
   aggttggaat tggttacgaa agttccgccc ctcaacttgc tgtccgtaaa atacacaatg 71964
   acattggctt aatgctaagt cttgtaccac ggctaggtgt gttcgtttaa ctaaaaaatt 72024
   ggtaatggga caaaatgctc ctgagtatgg atcaaacgtt aacgccattg aatgggtggc 72084
   ttcggataat ccttcacgga tcttataatc gcgggtctca accaacactt tcataaattg 72144
   tgttgttgtc tgttctatac gcgcacgcag tgtgtctaat atgcgacgaa acgttggatg 72204
   atccacaatg acagacgata gtccctcgga agaacatggg gcaccgcgat ccagtagtcg 72264
   ctcttgttct agatcgataa acaagcgttc tagtgtagcc ctataagtgt cctgcatggt 72324
   tgcctttgct gcttccgttt gatccccggg ttttcgaagg attaaatatg gagcatagtt 72384
   tcctagagga tcgcagtcgc tatattggct gttcattgtt ccaaacaccc caataggttg 72444
   acgggtggct tgtccaaatc gcggcatgcg ttgtctaagt cggtgtactg ttgtgtgagc 72504
   gcataccggc cgcgtgtgtt tttcacataa actacatgga atttcagagt caaaggtccc 72564
   cgtaacatat tttaacgcat ccccgtgacc ccctgtaaac gcaccagcgt cacagcgttc 72624
   tagataaaaa agcagtcgcg ccaacagggg tgctccaaat ccacaaatga gtgccaaata 72684
   atccacgcta aactctgtgt ttgatgaacc cgtagactga ctggatagaa cgtggccatc 72744
   tcgatctgtt tggggattcg cagctaaatg aggtccggca aactggtaaa atcgattaaa 72804
   tgatggaccc ggtcccccat ccttggcttc ggtcatcccg ctatcctcca cctcagttag 72864
   atacaacgca gaatttgggc tgaaaaccat cgcaccaatg accccggcca cacgagcggt 72924
   atatgacccc agagcgttta gccttggcaa agtgccctcc atgcctataa acattggcca 72984
   ttctttgata tctgttggag tttcttcgta aattccagtg ttgaatataa cttctgcgtg 73044
   caaggctgtg tcagcggcca taatagacgc caaccgtctt tcaaaccccc ccgatgggct 73104
   aggcttagac gtggagttga catcgtttcg acgcgctcca cgggcggtag tggttccact 73164
   tgaagaggac tgaaaatacg tgtacgtaat gtcagggggg agtactgccc cctcgtgatt 73224
   ttcatcaaaa gcaaggtggg ccgctcctcg ggcgacggca gttacatttc tgacgcgcaa 73284
   ggcaacggcc atgggagcaa taacacagtc atgtattaaa tggcacaacc cggtgttata 73344
   aaagggtgtt gggtatacaa aaccctctcc gatagacctg tgatgagtgt tgaatgggtc 73404
   gggtaccaga cggttaacat cgggcatgaa aagttgtacc ggaaataacg gtatacgtat 73464
   aacatcccca tggttaatat gaacaatatc gagtcctcca taatgcagaa acacgttgca 73524
   cataaatacg gcttccttaa acaatgccgt gaccaccaag tataatattg tattttctgg 73584
   ctctaatcca aggcgggtgc atatctcagc gccggtcgtc tcaacagcac cgtcaacagg 73644
   aggcccttgg cagcgtgaaa acccaaaccg ttctcgagcc gcgttacacg cgcgtgtgag 73704
   atttggggcc gcggagctgg gtaaaacgtg tttgcctccg tgaaagacaa agacagatgg 73764
   atagaaatga ctggtagtga gttttaaggt aataccagct ccggcaagac ccgtagtgcg 73824
   tgctccagaa accaccgcca ggctggatgt aaaagttttt tccacggtca aattacgcat 73884
   caaaggtaat aaagccaaat cagagtccgt gctacgagcg gccaaaaatg aaatttcctc 73944
   cagatccaaa tcttcaaccc ggcacgcata aacgtaaccc aggggccccg tgggcactgt 74004
   cacagtcttc tgagtatttt ccattttggt gaagtactcg taataatggg gggttgttgg 74064
   cagggtcaaa tgaccaccca aaacccagtc tgtcgataca ttaagaagag aggcttttaa 74124
   acgggtatta catatgcgga aaccacaaca aatcacgtga ttacacttta tgtattagaa 74184
   gggcgtgggg ttgtgttact cagtaacact ggctttttac aagattatca atcgttaaca 74244
   taaa atg gcg atc aga acg ggg ttt tgt aat ccc ttt tta acc caa 74290
   Met Ala Ile Arg Thr Gly Phe Cys Asn Pro Phe Leu Thr Gln
   5030 5035 5040
   gca tca ggg att aaa tat aac cca aga acc ggg cgc ggt agt aac 74335
   Ala Ser Gly Ile Lys Tyr Asn Pro Arg Thr Gly Arg Gly Ser Asn
   5045 5050 5055
   aga gaa ttt ctt cat agt tac aaa act acc atg tca tcg ttt caa 74380
   Arg Glu Phe Leu His Ser Tyr Lys Thr Thr Met Ser Ser Phe Gln
   5060 5065 5070
   ttt ttg gcc cct aaa tgt tta gat gaa gat gtg ccc atg gaa gaa 74425
   Phe Leu Ala Pro Lys Cys Leu Asp Glu Asp Val Pro Met Glu Glu
   5075 5080 5085
   cga aag ggg gtt cac gtc ggt aca ctt agt cga ccg cct aaa gtt 74470
   Arg Lys Gly Val His Val Gly Thr Leu Ser Arg Pro Pro Lys Val
   5090 5095 5100
   tac tgt aat gga aaa gaa gtt ccg att ctg gat ttt cgt tgt tcc 74515
   Tyr Cys Asn Gly Lys Glu Val Pro Ile Leu Asp Phe Arg Cys Ser
   5105 5110 5115
   agc ccc tgg cct aga cgc gtg aat att tgg ggg gaa atc gac ttt 74560
   Ser Pro Trp Pro Arg Arg Val Asn Ile Trp Gly Glu Ile Asp Phe
   5120 5125 5130
   cgt ggg gat aag ttt gac ccc cgc ttt aac aca ttc cat gta tat 74605
   Arg Gly Asp Lys Phe Asp Pro Arg Phe Asn Thr Phe His Val Tyr
   5135 5140 5145
   gat att gtc gaa aca aca gaa gcc gcg tct aat gga gat gta tcc 74650
   Asp Ile Val Glu Thr Thr Glu Ala Ala Ser Asn Gly Asp Val Ser
   5150 5155 5160
   cgg ttt gca act gca aca cga ccg ctt ggt acc gtt att act tta 74695
   Arg Phe Ala Thr Ala Thr Arg Pro Leu Gly Thr Val Ile Thr Leu
   5165 5170 5175
   ctt ggc atg tcc cga tgt gga aaa agg gtg gca gtt cat gta tac 74740
   Leu Gly Met Ser Arg Cys Gly Lys Arg Val Ala Val His Val Tyr
   5180 5185 5190
   ggc atc tgt caa tat ttt tat ata aac aaa gcc gag gtg gat acc 74785
   Gly Ile Cys Gln Tyr Phe Tyr Ile Asn Lys Ala Glu Val Asp Thr
   5195 5200 5205
   gct tgt ggc ata cgt tcc ggt agc gag tta tct gta tta ctt gcc 74830
   Ala Cys Gly Ile Arg Ser Gly Ser Glu Leu Ser Val Leu Leu Ala
   5210 5215 5220
   gag tgt tta cgc agt tct atg ata aca caa aat gat gca acg tta 74875
   Glu Cys Leu Arg Ser Ser Met Ile Thr Gln Asn Asp Ala Thr Leu
   5225 5230 5235
   aat gga gac aag aac gct ttt cat ggt acc tcg ttt aaa agc gca 74920
   Asn Gly Asp Lys Asn Ala Phe His Gly Thr Ser Phe Lys Ser Ala
   5240 5245 5250
   tct cca gaa agc ttt cgc gtt gag gtt att gag cgc aca gat gtt 74965
   Ser Pro Glu Ser Phe Arg Val Glu Val Ile Glu Arg Thr Asp Val
   5255 5260 5265
   tat tac tac gat aca cag cca tgt gcg ttt tac agg gtg tat tct 75010
   Tyr Tyr Tyr Asp Thr Gln Pro Cys Ala Phe Tyr Arg Val Tyr Ser
   5270 5275 5280
   ccc tca tct aaa ttt aca aat tat ctt tgt gat aac ttt cac ccg 75055
   Pro Ser Ser Lys Phe Thr Asn Tyr Leu Cys Asp Asn Phe His Pro
   5285 5290 5295
   gag ttg aaa aag tat gaa ggt cgg gta gac gct acc act cgt ttt 75100
   Glu Leu Lys Lys Tyr Glu Gly Arg Val Asp Ala Thr Thr Arg Phe
   5300 5305 5310
   cta atg gat aat ccc ggc ttt gtt agt ttt ggt tgg tat caa cta 75145
   Leu Met Asp Asn Pro Gly Phe Val Ser Phe Gly Trp Tyr Gln Leu
   5315 5320 5325
   aaa cct gga gtt gat ggg gaa cgt gtt cga gtt cga ccg gca agt 75190
   Lys Pro Gly Val Asp Gly Glu Arg Val Arg Val Arg Pro Ala Ser
   5330 5335 5340
   cgc caa tta acg tta agc gac gtt gaa att gac tgc atg tcg gat 75235
   Arg Gln Leu Thr Leu Ser Asp Val Glu Ile Asp Cys Met Ser Asp
   5345 5350 5355
   aat ctg cag gct ata cca aac gat gac tca tgg cct gac tac aag 75280
   Asn Leu Gln Ala Ile Pro Asn Asp Asp Ser Trp Pro Asp Tyr Lys
   5360 5365 5370
   ttg tta tgt ttc gat att gaa tgt aaa tca gga gga tct aat gag 75325
   Leu Leu Cys Phe Asp Ile Glu Cys Lys Ser Gly Gly Ser Asn Glu
   5375 5380 5385
   ctg gcg ttt ccc gat gca aca cat ctg gag gat ctt gta atc caa 75370
   Leu Ala Phe Pro Asp Ala Thr His Leu Glu Asp Leu Val Ile Gln
   5390 5395 5400
   att tct tgt cta tta tat tca atc cct cga cag tct tta gaa cac 75415
   Ile Ser Cys Leu Leu Tyr Ser Ile Pro Arg Gln Ser Leu Glu His
   5405 5410 5415
   att tta ctg ttt tcc ctt ggc tct tgt gac tta cca caa agg tat 75460
   Ile Leu Leu Phe Ser Leu Gly Ser Cys Asp Leu Pro Gln Arg Tyr
   5420 5425 5430
   gta caa gaa atg aag gac gcg ggg tta ccg gag ccg act gtg ctg 75505
   Val Gln Glu Met Lys Asp Ala Gly Leu Pro Glu Pro Thr Val Leu
   5435 5440 5445
   gag ttt gat agt gaa ttc gag cta tta att gca ttt atg acc ctc 75550
   Glu Phe Asp Ser Glu Phe Glu Leu Leu Ile Ala Phe Met Thr Leu
   5450 5455 5460
   gta aaa cag tac gct ccc gag ttt gcc aca ggt tat aac att gtt 75595
   Val Lys Gln Tyr Ala Pro Glu Phe Ala Thr Gly Tyr Asn Ile Val
   5465 5470 5475
   aat ttt gat tgg gcg ttt att atg gag aaa ctt aat tct ata tac 75640
   Asn Phe Asp Trp Ala Phe Ile Met Glu Lys Leu Asn Ser Ile Tyr
   5480 5485 5490
   agt ctc aag ctt gat ggt tat ggc agt ata aac cgt ggg ggt ctg 75685
   Ser Leu Lys Leu Asp Gly Tyr Gly Ser Ile Asn Arg Gly Gly Leu
   5495 5500 5505
   ttt aag ata tgg gat gtt ggc aaa tcc gga ttt cag cga cga agc 75730
   Phe Lys Ile Trp Asp Val Gly Lys Ser Gly Phe Gln Arg Arg Ser
   5510 5515 5520
   aag gta aag atc aac ggt ctc ata tct ctg gat atg tat gca att 75775
   Lys Val Lys Ile Asn Gly Leu Ile Ser Leu Asp Met Tyr Ala Ile
   5525 5530 5535
   gca act gaa aaa tta aaa ctc tcg agt tat aaa tta gat tcg gtt 75820
   Ala Thr Glu Lys Leu Lys Leu Ser Ser Tyr Lys Leu Asp Ser Val
   5540 5545 5550
   gca cgt gaa gct cta aat gag tcc aag aga gat ttg ccc tac aaa 75865
   Ala Arg Glu Ala Leu Asn Glu Ser Lys Arg Asp Leu Pro Tyr Lys
   5555 5560 5565
   gac att ccg gga tat tac gct agt gga ccg aat aca cga gga att 75910
   Asp Ile Pro Gly Tyr Tyr Ala Ser Gly Pro Asn Thr Arg Gly Ile
   5570 5575 5580
   att ggt gaa tat tgt ata caa gac tcg gct ctt gtg ggg aaa ctg 75955
   Ile Gly Glu Tyr Cys Ile Gln Asp Ser Ala Leu Val Gly Lys Leu
   5585 5590 5595
   ttt ttt aaa tat tta cca cac ctt gag tta tcc gcg gtt gca agg 76000
   Phe Phe Lys Tyr Leu Pro His Leu Glu Leu Ser Ala Val Ala Arg
   5600 5605 5610
   cta gct aga att act tta acc aag gct att tac gac gga cag cag 76045
   Leu Ala Arg Ile Thr Leu Thr Lys Ala Ile Tyr Asp Gly Gln Gln
   5615 5620 5625
   gtt agg att tac acc tgt tta tta gga ctg gct tcg tct cga gga 76090
   Val Arg Ile Tyr Thr Cys Leu Leu Gly Leu Ala Ser Ser Arg Gly
   5630 5635 5640
   ttt att tta ccc gat ggg gga tac cca gct act ttt gaa tat aag 76135
   Phe Ile Leu Pro Asp Gly Gly Tyr Pro Ala Thr Phe Glu Tyr Lys
   5645 5650 5655
   gat gtt att ccc gat gtc ggg gat gtt gag gaa gag atg gat gaa 76180
   Asp Val Ile Pro Asp Val Gly Asp Val Glu Glu Glu Met Asp Glu
   5660 5665 5670
   gac gag agc gtt tct ccc act ggt acg tca agt ggg cga aat gta 76225
   Asp Glu Ser Val Ser Pro Thr Gly Thr Ser Ser Gly Arg Asn Val
   5675 5680 5685
   gga tat aaa gga gcc agg gtt ttt gac cct gat acg gga ttt tat 76270
   Gly Tyr Lys Gly Ala Arg Val Phe Asp Pro Asp Thr Gly Phe Tyr
   5690 5695 5700
   atc gat ccg gtg gtc gta ttg gat ttt gca agt tta tat cca agt 76315
   Ile Asp Pro Val Val Val Leu Asp Phe Ala Ser Leu Tyr Pro Ser
   5705 5710 5715
   ata att cag gcc cat aac tta tgt ttt acc acg cta acg tta aat 76360
   Ile Ile Gln Ala His Asn Leu Cys Phe Thr Thr Leu Thr Leu Asn
   5720 5725 5730
   ttt gag acg gtt aaa cgt ttg aat cca tcc gat tat gcc acc ttt 76405
   Phe Glu Thr Val Lys Arg Leu Asn Pro Ser Asp Tyr Ala Thr Phe
   5735 5740 5745
   aca gtt gga gga aaa cgt ctt ttt ttt gtg cgc tct aac gtt cga 76450
   Thr Val Gly Gly Lys Arg Leu Phe Phe Val Arg Ser Asn Val Arg
   5750 5755 5760
   gaa agt ctg ctg ggt gtt ctt tta aaa gac tgg ttg gct atg cgc 76495
   Glu Ser Leu Leu Gly Val Leu Leu Lys Asp Trp Leu Ala Met Arg
   5765 5770 5775
   aag gct att aga gcg cgc ata ccc gga agt tct tca gat gaa gca 76540
   Lys Ala Ile Arg Ala Arg Ile Pro Gly Ser Ser Ser Asp Glu Ala
   5780 5785 5790
   gtg tta tta gac aaa caa caa gcc gcg ata aaa gta gtt tgt aat 76585
   Val Leu Leu Asp Lys Gln Gln Ala Ala Ile Lys Val Val Cys Asn
   5795 5800 5805
   tcc gtg tac ggt ttt act gga gtt gcg cag gga ttt ctg cca tgt 76630
   Ser Val Tyr Gly Phe Thr Gly Val Ala Gln Gly Phe Leu Pro Cys
   5810 5815 5820
   tta tac gta gcg gcc act gtc act aca att ggc cgt caa atg tta 76675
   Leu Tyr Val Ala Ala Thr Val Thr Thr Ile Gly Arg Gln Met Leu
   5825 5830 5835
   tta agt acc aga gat tat att cat aat aac tgg gcc gca ttt gaa 76720
   Leu Ser Thr Arg Asp Tyr Ile His Asn Asn Trp Ala Ala Phe Glu
   5840 5845 5850
   cgt ttt att aca gcg ttt cca gac att gaa agt agc gtt ctc tcc 76765
   Arg Phe Ile Thr Ala Phe Pro Asp Ile Glu Ser Ser Val Leu Ser
   5855 5860 5865
   caa aaa gcg tac gag gta aag gtt ata tat gga gat acg gat tct 76810
   Gln Lys Ala Tyr Glu Val Lys Val Ile Tyr Gly Asp Thr Asp Ser
   5870 5875 5880
   gtg ttt atc cga ttc aag ggt gtt agt gtt gag ggg ata gct aaa 76855
   Val Phe Ile Arg Phe Lys Gly Val Ser Val Glu Gly Ile Ala Lys
   5885 5890 5895
   atc ggc gag aaa atg gca cat ata att tca acg gct ctg ttt tgt 76900
   Ile Gly Glu Lys Met Ala His Ile Ile Ser Thr Ala Leu Phe Cys
   5900 5905 5910
   cct cct ata aag ttg gag tgt gaa aaa act ttt ata aaa ctt ttg 76945
   Pro Pro Ile Lys Leu Glu Cys Glu Lys Thr Phe Ile Lys Leu Leu
   5915 5920 5925
   ctt ata aca aag aaa aag tac att ggg gta att tac ggc gga aag 76990
   Leu Ile Thr Lys Lys Lys Tyr Ile Gly Val Ile Tyr Gly Gly Lys
   5930 5935 5940
   gtt tta atg aag gga gtc gac ttg gtt aga aaa aac aac tgt caa 77035
   Val Leu Met Lys Gly Val Asp Leu Val Arg Lys Asn Asn Cys Gln
   5945 5950 5955
   ttt att aac gat tat gcc cgc aaa ctt gta gaa ctg ttg tta tat 77080
   Phe Ile Asn Asp Tyr Ala Arg Lys Leu Val Glu Leu Leu Leu Tyr
   5960 5965 5970
   gac gac acc gtc tcg cgt gct gcg gcg gag gcg tcg tgt gtt tcc 77125
   Asp Asp Thr Val Ser Arg Ala Ala Ala Glu Ala Ser Cys Val Ser
   5975 5980 5985
   att gct gaa tgg aat aga cgg gcc atg ccg tct ggg atg gcc ggg 77170
   Ile Ala Glu Trp Asn Arg Arg Ala Met Pro Ser Gly Met Ala Gly
   5990 5995 6000
   ttt gga cgc ata att gca gat gca cat cgc cag att aca tca ccc 77215
   Phe Gly Arg Ile Ile Ala Asp Ala His Arg Gln Ile Thr Ser Pro
   6005 6010 6015
   aaa ttg gat att aat aag ttt gtt atg acg gcc gag ctt agt cgt 77260
   Lys Leu Asp Ile Asn Lys Phe Val Met Thr Ala Glu Leu Ser Arg
   6020 6025 6030
   cca cca tcc gcc tac ata aac cgt cgc ttg gct cac tta aca gta 77305
   Pro Pro Ser Ala Tyr Ile Asn Arg Arg Leu Ala His Leu Thr Val
   6035 6040 6045
   tat tat aaa tta gta atg aga cag ggt caa atc cca aac gtt cga 77350
   Tyr Tyr Lys Leu Val Met Arg Gln Gly Gln Ile Pro Asn Val Arg
   6050 6055 6060
   gaa cgc atc cct tat gtt att gtg gcc ccc aca gac gaa gtg gag 77395
   Glu Arg Ile Pro Tyr Val Ile Val Ala Pro Thr Asp Glu Val Glu
   6065 6070 6075
   gct gat gca aaa agt gta gct ttg cta cgt gga gat cct tta cag 77440
   Ala Asp Ala Lys Ser Val Ala Leu Leu Arg Gly Asp Pro Leu Gln
   6080 6085 6090
   aat acc gca ggt aaa cgg tgt ggg gaa gca aag cgt aag tta ata 77485
   Asn Thr Ala Gly Lys Arg Cys Gly Glu Ala Lys Arg Lys Leu Ile
   6095 6100 6105
   ata tct gac tta gcg gaa gat ccc att cac gta aca tca cac ggg 77530
   Ile Ser Asp Leu Ala Glu Asp Pro Ile His Val Thr Ser His Gly
   6110 6115 6120
   ctg tct tta aac att gac tat tat ttt tct cat ctc att ggg acg 77575
   Leu Ser Leu Asn Ile Asp Tyr Tyr Phe Ser His Leu Ile Gly Thr
   6125 6130 6135
   gcg agt gta act ttt aag gcg tta ttt gga aac gac act aaa ctc 77620
   Ala Ser Val Thr Phe Lys Ala Leu Phe Gly Asn Asp Thr Lys Leu
   6140 6145 6150
   aca gaa cgg ctt tta aaa cgt ttt att cca gag aca cga gtt gtt 77665
   Thr Glu Arg Leu Leu Lys Arg Phe Ile Pro Glu Thr Arg Val Val
   6155 6160 6165
   aac gtt aaa atg cta aac cgc ttg cag gcg gca ggc ttt gtt tgt 77710
   Asn Val Lys Met Leu Asn Arg Leu Gln Ala Ala Gly Phe Val Cys
   6170 6175 6180
   ata cac gcc ccg tgc tgg gat aat aaa atg aac act gaa gct gaa 77755
   Ile His Ala Pro Cys Trp Asp Asn Lys Met Asn Thr Glu Ala Glu
   6185 6190 6195
   atc acc gag gag gaa caa agt cat caa ata atg cgt aga gtc ttt 77800
   Ile Thr Glu Glu Glu Gln Ser His Gln Ile Met Arg Arg Val Phe
   6200 6205 6210
   tgt att cca aaa gca att ctc cat caa agt taa ggtcacacat 77843
   Cys Ile Pro Lys Ala Ile Leu His Gln Ser
   6215 6220
   tttacagtaa acgtccgatg ttccaatgga tggcaccaca gtctctgttt gttgttctgg 77903
   gttgcgacat accgacagta aaaatgttgt ctgccaaacg tgtgcgacta ttttataccc 77963
   ccgacacgcg gcctgtatat gatcgattag tctgtaatgg aggtgaacag atttccccgg 78023
   aaatataaca tacatcataa accctccggc atctccatca cgataaaaaa gtacgcgtat 78083
   atctcgcatg cccccctccc tcataatatg atacataaaa aataattccg gttgtgccaa 78143
   tacgctcgac aataggggtt cagcggacgc tgcttgcatg ttggctcggt ctgatagcgc 78203
   caaaatggat gcaagaaaca cacgatattc gtatatgttg ttaagctgct gaacatatgc 78263
   tagtatcaga gccgcccgat cggttcgaca tagacgcggt tctccagatg cagtgcatgt 78323
   cggacaataa cctccgagac ctaaatgata acccattccg gataacgaca ggcagttatc 78383
   cgccactgtc tgacccaagt taaacggaag ggtgacaggg gtcgtcttaa taattggcac 78443
   gattaagcct ctaacggtag ctagttcttc tgggggggac cttgcgatgt aattaaaata 78503
   atggcggtac acagaccgct cctttgacat aagttttcct cgaatggttt gacggcggca 78563
   tggcgttctg gagcgtacac gcatcgaagg cccgctccga gatacggatt tatatcgcct 78623
   agacatactt cgatagggtg tgttcgaggg agaccagcga tattgcatgt cgtcgaagca 78683
   taagtcctcc atctcgtatg aatgcggcat tggcggttgg tttgcgtgga aaaatctggt 78743
   aggctttaaa tgcatacgcc aatgtccaca ggccagcaca tacacgacct tcaaatctgt 78803
   tttgtgcagc cagttgggct ttatataact ccaacacttc cttatccgcg gttgtaggat 78863
   gggcaaataa aattttaggg tttgtctgta attccgagag agcgcatagg agatcgcaaa 78923
   ataaatgttt atatacggcc tcggaacctg ctgcttttac aatggttatg aatcgaccct 78983
   catcattaaa tggaaattta agtttaatgg ggttatctag gctccatgca ttgattacag 79043
   gttcgataca gtcaaataaa cttgtgttat ttgctgagta tgctaatata tcccttttaa 79103
   actgccgcaa agcgatccaa tagtgcccta tggttaataa acaacataac atgcattcgg 79163
   tattcgtgcg accacggcac tttgcgtttt ttaaggtagt taacaaaata ggcccaaatg 79223
   ctacttcggg acaggcatcc tcgctcgtaa atttggaaaa ttgttttgga acgtccctgg 79283
   agttttcaat ccggttcgcc tgccagtatg atttaacaca tccaacgcga gtttctatag 79343
   cacaatttgt acgctcgtct acattccaag tagcaacccc actcagtaat aataaagcca 79403
   agtctgcgta accccaagct gcttctacgt aagacgattt tttaagtttg tctattaata 79463
   tatcttcaaa ctctatatcc tcttttaatg cacgttccgc gcaggtaatc atcccacttg 79523
   tgcgatgttc cgatgatgaa cagtcaacca tccgcgcgca atctttccaa ttagccaagg 79583
   cggccgctaa aggttgaggg cctccagtca gtccgtcctg attaaagtaa aatgacttac 79643
   gcgtttcggt ttcgtctaaa cgagacgtgc tgatcgatag taatgcagcc ctaactaccg 79703
   cctgtacaaa aaaggagtaa ttggaatatc ttactttgtt ggccatttcc ttaccggggg 79763
   gcggtcgtat acctggtaac cctccatcgg tacgaaaaca tacatgcaaa aaaaaatgtc 79823
   tttgtatatc taacagggaa agcgtgcgcc tggcaaagga cccaaccaaa gtaacgttat 79883
   aacggcacag gtagtaacga tccataagat acacaaattc aaaggcagcg ctaaaagtga 79943
   caactgcaca gggcggggat gccagacttt tcatacataa catggcgtag tccgcaaccc 80003
   attccggtgt cagtccaaat ttacgtttat acaaatcaag cgttctgcag accaaacagg 80063
   ctctgtctaa cgccaaaacg tctccaattt cttctataaa ttcagtggta cttgaggtca 80123
   tttcattatc cacacctacc gccgaagagg cctcataagt gacgttgttt tctattgcac 80183
   ggggaacatt tattaagtgg gaggcaaata acaattctga aaataacaaa tcgtttttta 80243
   gcagaagggc tggattataa gcg atg tac gaa tcg gaa aat gcg tcg gaa 80293
   Met Tyr Glu Ser Glu Asn Ala Ser Glu
   6225 6230
   cac cat ccg gaa tta gaa gat gta ttt tcg gag aat acg ggc gat 80338
   His His Pro Glu Leu Glu Asp Val Phe Ser Glu Asn Thr Gly Asp
   6235 6240 6245
   tcg aat cca tcc atg ggt tct tct gat tct acc cga tcc atc tct 80383
   Ser Asn Pro Ser Met Gly Ser Ser Asp Ser Thr Arg Ser Ile Ser
   6250 6255 6260
   ggg atg agg gcc cgc gat tta att aca gac acc gat gtt aat cta 80428
   Gly Met Arg Ala Arg Asp Leu Ile Thr Asp Thr Asp Val Asn Leu
   6265 6270 6275
   tta aat atc gat gca ctg gag tca aag tat ttt cct gct gat agc 80473
   Leu Asn Ile Asp Ala Leu Glu Ser Lys Tyr Phe Pro Ala Asp Ser
   6280 6285 6290
   acc ttt act ctt tcc gtt tgg ttt gaa aat tta att ccc ccg gaa 80518
   Thr Phe Thr Leu Ser Val Trp Phe Glu Asn Leu Ile Pro Pro Glu
   6295 6300 6305
   ata gaa gca att cta cct aca act gac gct caa tta aat tat ata 80563
   Ile Glu Ala Ile Leu Pro Thr Thr Asp Ala Gln Leu Asn Tyr Ile
   6310 6315 6320
   tca ttt acc agt cgc ctg gcg tcc gtt tta aaa cat aaa gaa agt 80608
   Ser Phe Thr Ser Arg Leu Ala Ser Val Leu Lys His Lys Glu Ser
   6325 6330 6335
   aac gat tca gaa aaa tct gct tat gtt gtt cca tgt gaa cat agt 80653
   Asn Asp Ser Glu Lys Ser Ala Tyr Val Val Pro Cys Glu His Ser
   6340 6345 6350
   gcc agc gtg acc cgt cgc cgt gaa cgc ttt gcg gga gtc atg gcc 80698
   Ala Ser Val Thr Arg Arg Arg Glu Arg Phe Ala Gly Val Met Ala
   6355 6360 6365
   aaa ttt cta gat ttg cat gaa ata ttg aag gat gct taa tacatgggga 80747
   Lys Phe Leu Asp Leu His Glu Ile Leu Lys Asp Ala
   6370 6375
   aaataaacgc attaataagt cgtcaggggg tggatcactc caaactcctc ctatatccca 80807
   cgataaatgg tctaaatact tcacgcgtgt gccttggttt gcaaccttag atcaat atg 80866
   Met
   tca cgg aga acg tat gta cgg agt gaa cgc agg agg ggt tgc gga 80911
   Ser Arg Arg Thr Tyr Val Arg Ser Glu Arg Arg Arg Gly Cys Gly
   6380 6385 6390
   gat aat ctt tta caa cgt att cgg ttg gtg gta cca agc gct ctt 80956
   Asp Asn Leu Leu Gln Arg Ile Arg Leu Val Val Pro Ser Ala Leu
   6395 6400 6405
   caa tgt tgc gat ggg gat ctt cca ata ttt gat cca caa cgc ccc 81001
   Gln Cys Cys Asp Gly Asp Leu Pro Ile Phe Asp Pro Gln Arg Pro
   6410 6415 6420
   ccc gcc cgt tgt gtt ttt cag ttt aac ggc gaa gac aac gta tcc 81046
   Pro Ala Arg Cys Val Phe Gln Phe Asn Gly Glu Asp Asn Val Ser
   6425 6430 6435
   gaa gcc ttt ccg gta gag tat att atg cgt tta atg gcg aat tgg 81091
   Glu Ala Phe Pro Val Glu Tyr Ile Met Arg Leu Met Ala Asn Trp
   6440 6445 6450
   gcg caa gta gat tgt gac cct tac ata aaa att caa aat acg ggg 81136
   Ala Gln Val Asp Cys Asp Pro Tyr Ile Lys Ile Gln Asn Thr Gly
   6455 6460 6465
   gtg tct gtg cta ttt caa ggt ttt ttt ttt cgt ccg act aac gca 81181
   Val Ser Val Leu Phe Gln Gly Phe Phe Phe Arg Pro Thr Asn Ala
   6470 6475 6480
   cca gtg gct gaa gtg tcc att gac agt aat aac gtg att ctt agt 81226
   Pro Val Ala Glu Val Ser Ile Asp Ser Asn Asn Val Ile Leu Ser
   6485 6490 6495
   tca acg tta agt acc ggt atc aac cta tct gct ttg gaa tca att 81271
   Ser Thr Leu Ser Thr Gly Ile Asn Leu Ser Ala Leu Glu Ser Ile
   6500 6505 6510
   aaa cga ggt ggg ggt att gac cgc cga cct ctc cag gct tta atg 81316
   Lys Arg Gly Gly Gly Ile Asp Arg Arg Pro Leu Gln Ala Leu Met
   6515 6520 6525
   tgg gtg aac tgc ttt gtg cga atg cca tat gtt cag tta tcc ttt 81361
   Trp Val Asn Cys Phe Val Arg Met Pro Tyr Val Gln Leu Ser Phe
   6530 6535 6540
   cgt ttt atg gga ccg gaa gat cca tct cgc acc att aaa ctt atg 81406
   Arg Phe Met Gly Pro Glu Asp Pro Ser Arg Thr Ile Lys Leu Met
   6545 6550 6555
   gcc cgc gcc acg gat gca tac atg tat aag gaa act ggc aat aat 81451
   Ala Arg Ala Thr Asp Ala Tyr Met Tyr Lys Glu Thr Gly Asn Asn
   6560 6565 6570
   ttg gat gaa tat ata cgc tgg cgg cct tca ttc aga tcc cca ccc 81496
   Leu Asp Glu Tyr Ile Arg Trp Arg Pro Ser Phe Arg Ser Pro Pro
   6575 6580 6585
   gag aac gga agt cca aac acg tct gtt caa atg caa agt gac att 81541
   Glu Asn Gly Ser Pro Asn Thr Ser Val Gln Met Gln Ser Asp Ile
   6590 6595 6600
   aaa cct gcg tta ccc gat acc caa act acg cgt gtc tgg aaa ctt 81586
   Lys Pro Ala Leu Pro Asp Thr Gln Thr Thr Arg Val Trp Lys Leu
   6605 6610 6615
   gct cta ccc gta gct aac gtg aca tat gcc ctg ttc att gta att 81631
   Ala Leu Pro Val Ala Asn Val Thr Tyr Ala Leu Phe Ile Val Ile
   6620 6625 6630
   gta ctg gta gtt gta tta ggg gcg gtg ctt ttc tgg aaa taa 81673
   Val Leu Val Val Val Leu Gly Ala Val Leu Phe Trp Lys
   6635 6640 6645
   attgcctttc cgtacatatc ctgcgcagat gtacgtgtat gctgttatcg attgtcccgt 81733
   aaactaataa acg atg aca caa ccc gca tcg tct cgt gta gtc ttt gat 81782
   Met Thr Gln Pro Ala Ser Ser Arg Val Val Phe Asp
   6650 6655
   ccc agc aac ccc acc aca ttt tcg gtg gaa gca att gcg gct tac 81827
   Pro Ser Asn Pro Thr Thr Phe Ser Val Glu Ala Ile Ala Ala Tyr
   6660 6665 6670
   acc ccc gtt gct tta ata cga ctt tta aac gcc agt gga cct ttg 81872
   Thr Pro Val Ala Leu Ile Arg Leu Leu Asn Ala Ser Gly Pro Leu
   6675 6680 6685
   caa cct ggt cac cgt gtg gac atc gct gat gcc aga agc att tac 81917
   Gln Pro Gly His Arg Val Asp Ile Ala Asp Ala Arg Ser Ile Tyr
   6690 6695 6700
   acc gtg gga gcc gcg gcc agt gcc gcg cgt gca cgc gct aac cat 81962
   Thr Val Gly Ala Ala Ala Ser Ala Ala Arg Ala Arg Ala Asn His
   6705 6710 6715
   aat gca aat acg ata cgc cga acg gcc atg ttt gcc gag act gac 82007
   Asn Ala Asn Thr Ile Arg Arg Thr Ala Met Phe Ala Glu Thr Asp
   6720 6725 6730
   cct atg aca tgg tta aga cca acg gtt ggc tta aaa cgt acg ttt 82052
   Pro Met Thr Trp Leu Arg Pro Thr Val Gly Leu Lys Arg Thr Phe
   6735 6740 6745
   aac ccg cgt att ata cga cca caa ccc cca aat cca tcc atg agt 82097
   Asn Pro Arg Ile Ile Arg Pro Gln Pro Pro Asn Pro Ser Met Ser
   6750 6755 6760
   ttg gga atc tcg ggg cct act ata ttg ccg caa aaa aca cag agc 82142
   Leu Gly Ile Ser Gly Pro Thr Ile Leu Pro Gln Lys Thr Gln Ser
   6765 6770 6775
   gcc gat cag tct gct tta caa cag ccc gcc gcg ttg gcg ttt tcg 82187
   Ala Asp Gln Ser Ala Leu Gln Gln Pro Ala Ala Leu Ala Phe Ser
   6780 6785 6790
   gga tca tcc ccg caa cac ccc cca cct caa acg acg tcg gca tcc 82232
   Gly Ser Ser Pro Gln His Pro Pro Pro Gln Thr Thr Ser Ala Ser
   6795 6800 6805
   gtt gga caa cag caa cac gtg gtg tcg ggg tct tct gga caa caa 82277
   Val Gly Gln Gln Gln His Val Val Ser Gly Ser Ser Gly Gln Gln
   6810 6815 6820
   ccg caa cag gga gca cag tca agc act gtc cag cca aca acc gga 82322
   Pro Gln Gln Gly Ala Gln Ser Ser Thr Val Gln Pro Thr Thr Gly
   6825 6830 6835
   tca ccg ccc gcg gcc caa ggc gtg cca cag tct acc ccg ccc cca 82367
   Ser Pro Pro Ala Ala Gln Gly Val Pro Gln Ser Thr Pro Pro Pro
   6840 6845 6850
   acc caa aat acc ccc cag ggg ggt aag gga cag acc ttg tca cac 82412
   Thr Gln Asn Thr Pro Gln Gly Gly Lys Gly Gln Thr Leu Ser His
   6855 6860 6865
   acg gga caa tct gga aac gct tca aga agt cgt agg gtg taa 82454
   Thr Gly Gln Ser Gly Asn Ala Ser Arg Ser Arg Arg Val
   6870 6875 6880
   ataaaaatac acagaaaata atcgttgttt tttttttttc tttaataggc gctactttat 82514
   atatatgttc catctaataa caatttaaca taccccaggc cttgaaatat atcttccgtg 82574
   tggtccatta aaagtttacg tgtatatcta acgttgtcga tcatacgccg acatgcctgg 82634
   cttaacagtg ccagtgctcc caaggcggtc cctcggacat aacgtcgacc ggtgactaca 82694
   tcggcattaa caacgcgtac gtgcctagga acgcgacatg accgatgttc gtcgagactt 82754
   agtacgatag aattttcact ctcactgcta tagccgtcag aaggttcact aaaatcactt 82814
   tgatataggt ctctgttcgg tgagttaaac gggttagaag atacgcccca tgtgtgtacg 82874
   tcccgcggaa agtttggcga cggctgtctt gttttatagt ttgtcaatag gtccgtttgg 82934
   ggggcatcgg ctgccgtgtc aacccaagaa ggtaattggt tatttacagg gtggaatact 82994
   gttttggagt tatacattcg agggtcattt tgatgccgtt tttgtgtttg atttgatttt 83054
   tcaatccaag tccggtaaga tatcgagtca tctgtcggaa tggaggtaat ctcaagtatt 83114
   ggtttactga acgcttggtt taacgtttgg ttttgctgtg gattattttt ccaatccgac 83174
   aattggttat ttggtggggt aatactttta caaacggttg ttttaatgga ggtggttaat 83234
   tgcttaggta taagcacagc cgtttctgat tcggtttcca catacaaggg aatatctttt 83294
   tggctaaggg ttgtttttac gctttggggt ttcttaaaat tttgtaccac tatttgtgtg 83354
   cccggttcta tgggctgcgc gggctggacg gtctgcgcgg gctgcgcggg ctgcgcgggc 83414
   tgcgcgggct gcgcgggccg gacgggcatt tttttacatt tggaacttct aacaacttga 83474
   acgggagact taaatagtgg ctccgttggg taaaaatcag ttgtgtcgag gtttgggggg 83534
   ccttggggcg cataaccggc aataatttgc gagtaaaatg gatcatcggg acacgagggg 83594
   aaagagtcct catcggagtc gatgtacggc aaatcaataa gagattctgg cccccaaaag 83654
   accacataat ccggagctgg aatcgagtct tttcgttccc ataaaacata agagggattc 83714
   atatgttcgt ccaatagcag taaacacggg ggattgtttg taagaggttt gtttgcaatg 83774
   agcgcggaca gtttttctag ttggacaatt aaacatgcat tttcaattgg gtgaggatcc 83834
   atatgcagta aacgtaaccc ccaagaaaca atatcgggag atacggctga ggatataccc 83894
   gaatctgtag ccatatgccg ggaatcgaat aattttaacc ccaaagtaag tccggaattt 83954
   tgtgaaaaaa cggatgtaag ttccatggca ataaccaaag gagccccaaa taaaatagcc 84014
   gtagttgcta tgtcaaacgc tgttacatta gaaacgtgag ttggtgccgc ccccgttact 84074
   gtaaaaactg tcgttgcccc ggtttgaata taagcatctg taattacttt acggtctgta 84134
   ttccactcta acgtggtatt gctgggagcg gtatcaaatc ttgcattaag cgtggatgac 84194
   aaatccagtc tcgcatcaac tataatgtcc acatcatctt gtgttctgta tgcgaattcc 84254
   tccggttgca gcgtattcca cattgacact aaggaaattg gcgggagaca catgcgccca 84314
   agtacagtaa tggtcgcaag acgatctgtt gccattatac gggtaactgc atcgacattg 84374
   gcctttaccc cggggggggg ggaaagttgc catggcgcaa aaggatccgt tattttctgt 84434
   ggcattttaa caagtttcca atctgccatc cgaaatgcaa atattctccc gccggtagga 84494
   tcttggtttg atccaagagc taacgggagt ttatgtttgg atggggcggc attaaaaaag 84554
   gcccgggcat tgcctttcgg gtctccatcc aatgagcgaa cgagtaaacg acctctaaat 84614
   gcggcctcga ttacgggtct gagggtacgg gccatatact catgctgttt aattaaatcc 84674
   aatctagcaa aattgagaag atgtaccggg gttgtggcaa ccaaaatagt tacaatatca 84734
   gatagggtta actctaatac gcgtacatca ccccttctct ggattggttt agttagtcca 84794
   ttgcccatta cagaagttag gtggtgtttt tcaaaagaat aaataagctt gtcccaatga 84854
   accccgtatt gtcgtgtcaa tgttgttgtt ataaggccgg cgtatatctc tgcaatggct 84914
   ctgcgtgcgt tggttttacc ccattcggcg tggcgatgcc taacaaaggt ggaaaaggca 84974
   gaaaacccgg attttatagc ggattcgtat tcaatgcgta gctcattgac ctcattggcc 85034
   acgcgtaatg ccgatagtgt agtttcttcc acaatacctt catcgggaag tgccggtcgt 85094
   aaagataatc caccctgact gcatagcatg gttccaagtt taaccccaac tgatttataa 85154
   cacactctat atcgaagtgg aaccccatca gtatccaagt aaaaggttaa attttttcca 85214
   aacaaagcgt ctatagaaga tctggcttcc ctaaacgttt gcaccacaga cgcctccccc 85274
   ataaacgcgg ccacccgcgc gcgaatgcat cgggattggt cttctagaca tacacccgac 85334
   atggcgggaa taggagcaaa tactccgttt ggagacgcgg gtggaaaaat ctcagcataa 85394
   cttgcataca agcctaaccg taccatgagt aaatttcgaa atgtcatata ctgtgttttt 85454
   atccatatag gcaaatgtgt aaattgatcg aaatcacgta ataacatacg aagaacgtca 85514
   tggtcactct ccgttggaat acgttcaatt aaagctcgta agcgagacgc ggtctcttca 85574
   aactgtcttt gtttttgcaa cacaacatcc aacgccgttt tacgttcctg ttgctttttt 85634
   tggagacggt ttttcgtttc cacaggcaat aaaaaaaatt cgggttcgga acaaagcatg 85694
   tcaaccacag acgccgatgc ctggagtgcc tttatactat ctacggaagt agcgtacgtg 85754
   tcccccgatg ccaacatatc ccccgtttct cttttaaatg ttgtccatgc atcgtcccac 85814
   gtaacctcac cggtttctat tcgtcgtcgt aggagatcta atttagttcg taacgcataa 85874
   agtttatcca ccctatcttt ataagtatgc agggggccgg taccgtctat acgcacactt 85934
   agtggatggg agtcaataat attgcgagct tggtacatcc agtcaaccgc ctgaatgtcc 85994
   aacgtaccag tttcttcttc cagacgccct aacagcaatg caaactgcgt gacaatgtcc 86054
   tgaacggaag tagatctatc aatggccaat gcaattgttt tgggaggggg ggttagtttt 86114
   aataacgtct tcaggttagt gaaatccacg tcttcttgtt gctcagtggc attggccgta 86174
   tcaacaatgt tttgtaaaat ttgtattgca cggttctttg ttgcctgaca caccatacga 86234
   aactgtgcaa gctctgcgtt ggcctccttt agatcgcggt ctaccattgt ttgtaaaact 86294
   ctttcatatg ccgttcctat aaatcgttta tgatcgaaag ttgtaaaata ctctatagcc 86354
   cgaatcaata aaccttcgct tggtaatgta atgaagacac cttctttaag tgcaagttta 86414
   gccgtttcgt gtgcccgaac atgagttact tcctccaaag ccttatttgc ttctgtaagt 86474
   ttagccccgg cagcctgtct gatgtctgct ctaagttcac ttaacgttac actttcggtt 86534
   ataaactgat cataagaccg tgtgtaaaaa tcaacatacc cagccaatgc gggtatttgt 86594
   aatagatccc ccgaggttcg taatatcata tctcggtagt taggtattcc attaatagac 86654
   gtatcggcgg ataataacac aaccgcgcga atacgcataa gcagaaggag tccctcaata 86714
   ctaacacctg gaaacataac ggtgaatacg ggagccgtaa tcgaaaaatc atcaaaccat 86774
   gttaaccctc ttaacaaagc caaaggtggt ggaatatttg tattttcggg ggtatatgga 86834
   ttgtggcgaa acacagtatc taatgccgta acggctgtag tttcacatgc cgctacaacc 86894
   tgacgtgctt gtgggtaaac tgcgtgtata tcaaaccctc ccgcagcggc catatctcgc 86954
   aaccgtgcca gttctgtccc gttaaatata tggcgcgttt ccattgcctc cagacatgct 87014
   ataatatcac gtgtccaacg ctcatacaaa acccgagtcg tttgttgttt atgtgcacgt 87074
   tcacaatcgg taattaacac ttttgcctgt tccttcttct catgtgccat tgtgacgagt 87134
   ttatgaagat cctttactac tcgagataag gtagatgtcc cagcaataca atgggggggt 87194
   agcttttgca gttcaacttc aacaagttta accgtatcca ttagggcgtg cacatttgtt 87254
   tgtattgata atatcgcatc gtttatttca taatcatcta catttgaaga tgatataaac 87314
   ttcaagtgtc cagatgagac agctagggtg gagaagtcat ctccaagttg tttttgtaat 87374
   tcagtcaact tatgggtatt cgtaatttct tgttgtaaag cctgatattt tttatataac 87434
   gacaacacaa aggataaggc tggcactgta gatgaaacta acgtcgcttt actaagagtt 87494
   gcaacaacag cgtttatggc ggactcactt tccgggttag tagatacggt cgcgatgtcg 87554
   gatgcaagca catctgccac gtcaccaaaa aacgcaagca atgatggtct atactccgta 87614
   ggtatcggca caatttcgat aatagctgta aaacaagctc ccagtcctcc taagacagat 87674
   gtgcgtgcgt ttccgggatg ttctaaaatg tctcgatatt ggttaaataa cgcccagaaa 87734
   tcacccgtaa gttgagaacg gatctctacc ggcgcctcta agactttggt cctgagattt 87794
   accagggctc cttggacatc taactcagaa gcaagttccg gtaataaatc tactgcagcc 87854
   ctaagtccca caaaaccggt taccgggcga agtaaccccc cgagtttatt atatgccgcc 87914
   tgttttcgac tctttattgt gtcctgtgtg gttcgggcct cgtttgcata cgtctgaacc 87974
   tctttaagcc ttgtttccaa tcgtctttta atcgaaggtt gtaaaccgga ttggatcatt 88034
   ggatcactta aaatttttat ctgtcgcacc aaatcgtctg tagcacgtac aattgttgct 88094
   aataaatcat cccgtgttat ttcaggatac ttgacctcac cattgttata tgtttcatag 88154
   tcctttacgg cttggtctac ggccgcgctt agcgtattaa agcaagacaa ctctgcttcc 88214
   gcggaagccc gttgcgttgc gcgtgtatta atggttttaa ttgtacgtga taattcatcg 88274
   aactctcgtc tggataagta acctgcggtt tgagcttccc cgactacaga taaccacgat 88334
   acgagagccg tgtctgtgtt taaatcttcc ccttctttaa taagctcttt taacagagat 88394
   acgacgggga gattttgtat agtctcagga ggagggtgta tagacaggga cgatatgatt 88454
   gccgttaatt tagcgtcgaa ttccggaaga gaatctaaaa gccgttgaat gggtgccgta 88514
   cttgcggtcg cgacccgaaa acgcctaggg gattgttcag cgatcaaggc ccgtgtgcta 88574
   tataacactc cgcgggtgaa gtattctgta acagccgagg tcaatagcat tgtaacttcg 88634
   gatcgtatct catatacacg ccgaattaac tcttcgggtg gaatgtcggt ttcttccggg 88694
   ggttcctggg gaacttctgg tatttgaatg gcatcaattt tattgcgggt agaccgtaat 88754
   gcattctcta aagcctttac actagaaaca agcgcggtgt gttcacgcat atagcgtgtc 88814
   tcccgggccg agactaattc acaaagaaga cgtattcgtt cagacaggga ccctgtacgg 88874
   gttggaataa gctcggcaaa aagtccggga tgagaaataa taggcgaatt tagtatactt 88934
   ataagacctg tcgacagtct agaatagagg tctatgggcg acgaacctcc tagatccgct 88994
   tctaacacat ctagttcggc atgtaaagcg ccggttgtat cctgaacccg catggcaact 89054
   agaccaaact tagcgataat gagcatgcca attgtacatt ttttccggag aacagtttta 89114
   atagcatatt ccgcccccgg catgtttgtt aatatcaaac ttgtaacgcg tataagttcc 89174
   gcggcggagg gaaatcctgt taaaatttcc ccaaggggac ctataaagtc tgatacaacg 89234
   cttgtagcac catgatgtgt acgtgttcca ttttccacca caaatcccgt taaacgcaaa 89294
   atggtgaggt taataaaaag ctccaatgga tccttttcgt ctggcataac caagggcctt 89354
   gttacccaaa tgggaacatt tgtatgaccg gtaataacag tatccaacgt acccaccgca 89414
   ttttgtacgt atccattgct aatttcatgc ccatgttgta gtagtttttc gacaaccatg 89474
   ttgacatcac cactctgttc aaatagaaat ggcttccgat ctttcaaact gcttcctaca 89534
   ttcatgttcc gtgaaaaagt gggactggat tcaggcgtat ctacattgga tacatttaac 89594
   acaacgtctg gggtgttagc aacttctcgt aggcggggta atacatgtgc cacttgtgtt 89654
   ttatccggca cgtctacagt tggtttaaat ttgagaacct ttttagcggg tttatgtaaa 89714
   ttatccgtgg aatgtgtatt atttaccaag ccatcgtcga tacaagctaa attttcctcg 89774
   cttgaaggag gagtccatga aggacggcgt ctacgacgac aatccaaaac gccagaccca 89834
   gtcgaggttg tttctagaac cgcatgccaa ggatcaacct cgctgtaact acatctaggg 89894
   tccagtgcaa ccgtgttggt taacgttgaa tgtagggttg cttccaagga tacactttca 89954
   ccaacgtttt ggtgaagagg tactatagac gatttattaa gcacaattgt cgtggaatta 90014
   ggaggtgaaa gaagcaacgg atgttgagct gtaaccagtt tttcgcaata ttgttcatct 90074
   gtaaaatatg tatcacagct tccgtatata agcgttactg ccgaagatag cgcttcttcg 90134
   cttacgggac cgtcgtttgc ggtgacaaaa aaaactaatg cagcggccca ttgggattct 90194
   acgttatcgg ggcggtgggt atactccgat gcgatataag agtacacatc cgtggtattc 90254
   acccgaacaa ctacagcctg tcctatagtc ccatggccat gtggatcaaa aataaaagat 90314
   atgtttccct gacgatagat ggccattccc gttgagtcta cagttataat tgtatacgac 90374
   tcttcacgtc ccatccacac atcatcgaaa aacgctctag cgggaatttg tgtgcacata 90434
   aaaccctcgt ctggaacggt aaaaaaatgg ctatcgccgt atattctaga caaaacgcag 90494
   cacgtactgg tagtgtcccc cagtttaata tatgaaattc gattggggag atcaacaaga 90554
   gcacacatat ctgggacacc cctccccata ttagacagat ccgtagtcca tgctgcaccc 90614
   tcttgtaaac atccatcaat catgtctgat gacagggttg cattaattcc atgcgcatat 90674
   gtcatatgca aaaacgataa agatgttctt aaacatgaca gacctgacgc cggtcccagg 90734
   gcaccgtcaa attgattacg gcttcccact cccgcaacag tgacagctat aggcggaatt 90794
   atatccattt tattgcttac gaaaattacg cgctcaactg gtagggacgc tacgtttacg 90854
   atgattcaaa tcccaaagag cgttgaataa gcacgtgtag ctccaaaaac ctaggaggtt 90914
   atgaaaatac tagcaacaac agcgttatac aaacgaaacg cccagtcaat aaaaaccaca 90974
   aatactttaa tgtacataaa cacgcctgtt ttatattaag ggtcactccc acttgtattc 91034
   ccgggtacgc caagacgcaa atctacatta gataaaatct catctggacg aagccatctt 91094
   tgtcctatta tcagcgtcgg agcctctgaa attacgtctc tgaccgttgt cgttatttta 91154
   acattatcat gtaaaaacgt tgcatttaca ttctcccatg ttcctatatt acattgtgaa 91214
   gttaaaaccg cgcccatctg aggtccagaa tccacaataa actgttgcct ggagtccata 91274
   acgaatgaaa ctgctgcctt gtgatcttcc agatttcggg cgtgactcat atatgaggtt 91334
   ggggcattta cacgatcctc ttgtatattt tttaaaccgc gcctcaattc attacacacg 91394
   gttgcaatag attcggccga cgggactgcc gtggcatgcc tacaagctgt tgctattcgt 91454
   tcccacctat ttaaaaaaat ctctacattt gcaagaccgg gtatattttc tcctgttgtt 91514
   agtttgtcaa cacgcattaa aatgtccatt tgatcctgtg taaaccgtgc ataccgggaa 91574
   gccatcgttg aaaaaagagg cccaatagca gaagtggagg ttttagtcat taaattttca 91634
   cattccctaa gggctgagga agcatcggcc ataattgtgc gcaggctgga tattcccact 91694
   ctcatttgcg ttcggattcc ttgtattgtc ccaacaatat gaccaaccgc atcagcaaga 91754
   tcgtttaaac gccatacccc aaattgttca tcgcgttcaa cctttgggtt acagatacca 91814
   acagcctgaa gatctggcgt tggataaaaa cgacacgcaa aaaatttttg taacattaac 91874
   atgtaccgag aaaccactaa cgcgttggat gtaaaatttt caaccaacgc ctgcgatgtt 91934
   ttatccagtc gggtccaacg accgtaaagt tcatcataat agagagttgc taaaaaaacg 91994
   ctatttgtaa tagaaggacg gtcaggaacc aaaggacgga cgtgctgggg aagcaggtca 92054
   acggcaatgc caacagtccg atgaagttcg catataaggg aatctatagt agaaacgtga 92114
   gatggtaaaa gtactggggg gtgtgtacgg gtaaaaggtt ctaatattgt tataagagca 92174
   gatagttttg tgcgatatcc atctgcattg ggcacggttc cggatccata atcgcgagcc 92234
   cactgaagaa tagacaaggc ttgctgtatt ccccgtgtat caaaagacaa agctattgtt 92294
   gccgaaaggg catcaagcgt gcttgttgca ataatataag gcacaaacga cggttccaca 92354
   aacacgctag agcgattata ttcggtagga tatgcaatct ttagtgcttc tgttatcatg 92414
   acagcttcca accctgtagc cggtggatca acacaaatag gaggtgtcat acccacatcc 92474
   accgggggag taatacccca tcttgaatcc atacgtaggt caaaccaggt tagtacggct 92534
   tcgcgtacgg cactttgtcc tgcttgtggt aataacgcac ctgaggattg attatgtgtt 92594
   tgcttaaaat atgttgtctc tctagcttct gcatgtgaaa tttcattaag tagtattacg 92654
   cgtgtcgctt gtatactttc aataaggggt cgtggagcaa aacagcgccg cataatttgt 92714
   cccaggcctc ccggtggtaa tcctaaggcg ggtaatagcg tttcataccg tttttcggtc 92774
   tcgtttactg tccgtgtata tgttaacaca tactcggtag taggagcgta cgtttccact 92834
   aattggtatg ccagtccacc cactaaagcg agacatccta gtgtttgttg gaaatctgta 92894
   atatgctcgt ctgctacaac gcttccgtga ataaaaccgg cgagtgtgtg gtgagcaata 92954
   aaccgacgta gcaatttgca tgaaaatctt gtaaacccgg attcggcaag ggtgcttatt 93014
   aacatactag gcgatgtact agcctgaacc ttttcccaaa tgccacgggt gtgttctgct 93074
   acattacttc cagatgtggt ttccgcttcc gggtcggagt ttagtaaatg taacagcgcc 93134
   tcaatatgaa ttccgggggt ggacaacgat aagaggttgc tttcccatat accattgtct 93194
   aatctaagtc tcctagcaag cattcccggg gatgtctcaa aaacataagg gtctcgataa 93254
   gccagagctg aggaaattcg tgactcgtgc accgttcgtg gattcgttac cgcttcttcg 93314
   atggatacga acgcctcatc tttaattaaa tttacagtat ctgcaaataa ttctgatatg 93374
   ctatgtactg gcgtttccgt gagatattca tcaatcatac agattataaa aagggctcgt 93434
   gttagaggag tgagtgagct attttgaatc tgattccaca taacattaaa cggaagaagc 93494
   catcctgcat attgtgttaa aacacgaatc ccctcttgaa caaatggcat atcgtaaatg 93554
   atcgcaaatt tatgttgtgt taaagctgca atggcgcttt ccaacattcc aatacgagcg 93614
   tgagtgaatg gtgattcaat ggctgttgtt gcatccttta aggcgacctc gtacatattt 93674
   atccagtccg ttataatgaa gtgtatcgaa acgggtccaa atttagacga ggttcgtaat 93734
   ttattggccg ataaacgatg taacgtctcg gaatcttgta ctacacttga tagctgggtt 93794
   aaaattttta agctattacc tggctcctct atcgtccagt ctagatctag tctccaaaac 93854
   aacagtccgg gtaaattttc ggccgtgaaa gcagcctccg ccaaaaatgt attggaatat 93914
   acaaaacaga tattagaatg caggggttcc catatctgaa taaaatgttt agctggaatt 93974
   ttctgggtag atcgtaaaaa cttaactacc ttaactctgg cgatttctgt tttctctggt 94034
   gaccatggtc tttcgttgtc cgcttcttgt actttcaagt tacttaaatc atccaaaagt 94094
   ttttgtgtat cataacaaat tggttcttcc atggtatatt ctacgctgac ttaactttat 94154
   aacgtgatat gaaaaagcta catccgtccg gaatgaaaag acataaatgt aacatcaata 94214
   aaaacgcatg ttaataaaaa ggacgtcacg gtaagttgaa gaacctttta acgccaatca 94274
   aaatattttt ataggctcct cctacctaac attatataaa cggtactacg actgtataat 94334
   gtgtacacat accccaggca acatttcaga tagtaccacg tcacgattgc attgtgtgaa 94394
   tttaacccct cagct atg ggg agt caa cca acc aac tcg cat ttt act 94442
   Met Gly Ser Gln Pro Thr Asn Ser His Phe Thr
   6885 6890
   tta aac gaa caa acg cta tgt gga act aat atc agt ctt tta gga 94487
   Leu Asn Glu Gln Thr Leu Cys Gly Thr Asn Ile Ser Leu Leu Gly
   6895 6900 6905
   aat aac cgt ttt att caa ata ggg aac ggg ctt cat atg act tat 94532
   Asn Asn Arg Phe Ile Gln Ile Gly Asn Gly Leu His Met Thr Tyr
   6910 6915 6920
   gct ccg ggc ttc ttc gga aat tgg agt cgc gat tta aca att ggc 94577
   Ala Pro Gly Phe Phe Gly Asn Trp Ser Arg Asp Leu Thr Ile Gly
   6925 6930 6935
   cct cgc ttt gga ggt ctg aac aaa caa ccg ata cat gta cca cca 94622
   Pro Arg Phe Gly Gly Leu Asn Lys Gln Pro Ile His Val Pro Pro
   6940 6945 6950
   aaa cgt aca gaa acc gcg tct att caa gta acc ccc cgt tca att 94667
   Lys Arg Thr Glu Thr Ala Ser Ile Gln Val Thr Pro Arg Ser Ile
   6955 6960 6965
   gtt att aat cgt atg aac aac att caa ata aat cca act tca att 94712
   Val Ile Asn Arg Met Asn Asn Ile Gln Ile Asn Pro Thr Ser Ile
   6970 6975 6980
   ggt aac ccg caa gtt acc att aga ctc ccg tta aat aat ttt aaa 94757
   Gly Asn Pro Gln Val Thr Ile Arg Leu Pro Leu Asn Asn Phe Lys
   6985 6990 6995
   tca acg aca cag cta atc caa caa gtg tca tta acc gat ttt ttt 94802
   Ser Thr Thr Gln Leu Ile Gln Gln Val Ser Leu Thr Asp Phe Phe
   7000 7005 7010
   cgt ccg gac att gag cat gct ggg tca atc gtc tta atc ctt cgt 94847
   Arg Pro Asp Ile Glu His Ala Gly Ser Ile Val Leu Ile Leu Arg
   7015 7020 7025
   cat cca tct gac atg att gga gaa gct aat aca ctt aca cag gct 94892
   His Pro Ser Asp Met Ile Gly Glu Ala Asn Thr Leu Thr Gln Ala
   7030 7035 7040
   gga cgt gac ccc gat gta cta cta gag ggt tta cga aac cta ttc 94937
   Gly Arg Asp Pro Asp Val Leu Leu Glu Gly Leu Arg Asn Leu Phe
   7045 7050 7055
   aat gcc tgc acg gct cct tgg acc gtt gga gaa ggt ggg ggg ctt 94982
   Asn Ala Cys Thr Ala Pro Trp Thr Val Gly Glu Gly Gly Gly Leu
   7060 7065 7070
   aga gca tat gta acg tca tta agt ttc atc gcc gca tgc cgg gca 95027
   Arg Ala Tyr Val Thr Ser Leu Ser Phe Ile Ala Ala Cys Arg Ala
   7075 7080 7085
   gaa gaa tat acg gat aaa cag gca gcg gat gcc aac aga aca gca 95072
   Glu Glu Tyr Thr Asp Lys Gln Ala Ala Asp Ala Asn Arg Thr Ala
   7090 7095 7100
   att gtt tct gcc tat gga tgc agt cgt atg gaa acg cgg ctc ata 95117
   Ile Val Ser Ala Tyr Gly Cys Ser Arg Met Glu Thr Arg Leu Ile
   7105 7110 7115
   agg ttt tcg gag tgt tta cgt gcg atg gta caa tgt cat gta ttt 95162
   Arg Phe Ser Glu Cys Leu Arg Ala Met Val Gln Cys His Val Phe
   7120 7125 7130
   cca cat cga ttt ata agt ttt ttt ggg tcc ctg ctg gaa tat acc 95207
   Pro His Arg Phe Ile Ser Phe Phe Gly Ser Leu Leu Glu Tyr Thr
   7135 7140 7145
   att cag gat aat tta tgc aat ata acc gcc gtg gcc aaa ggt ccc 95252
   Ile Gln Asp Asn Leu Cys Asn Ile Thr Ala Val Ala Lys Gly Pro
   7150 7155 7160
   caa gaa gct gca cgt aca gac aaa act tca act cgc agg gtc aca 95297
   Gln Glu Ala Ala Arg Thr Asp Lys Thr Ser Thr Arg Arg Val Thr
   7165 7170 7175
   gcc aac atc ccg gcc tgc gta ttt tgg gac gtt gac aaa gat tta 95342
   Ala Asn Ile Pro Ala Cys Val Phe Trp Asp Val Asp Lys Asp Leu
   7180 7185 7190
   cat ctt tcc gcg gac gga ctg aag cat gtg ttc ttg gtt ttt gta 95387
   His Leu Ser Ala Asp Gly Leu Lys His Val Phe Leu Val Phe Val
   7195 7200 7205
   tat aca cag cga cgc caa cga gaa ggt gta aga ctg cat ctt gca 95432
   Tyr Thr Gln Arg Arg Gln Arg Glu Gly Val Arg Leu His Leu Ala
   7210 7215 7220
   tta agc caa cta aac gaa caa tgt ttt ggt cgt ggt att ggc ttc 95477
   Leu Ser Gln Leu Asn Glu Gln Cys Phe Gly Arg Gly Ile Gly Phe
   7225 7230 7235
   ctg tta gga cgc ata cga gct gaa aat gcc gcc tgg ggg act gaa 95522
   Leu Leu Gly Arg Ile Arg Ala Glu Asn Ala Ala Trp Gly Thr Glu
   7240 7245 7250
   ggg gtt gca aat acc cac cag cca tat aac aca agg gcg ttg ccg 95567
   Gly Val Ala Asn Thr His Gln Pro Tyr Asn Thr Arg Ala Leu Pro
   7255 7260 7265
   ctt gtg cag tta tcc aat gac ccg aca agc cct cga tgt agt att 95612
   Leu Val Gln Leu Ser Asn Asp Pro Thr Ser Pro Arg Cys Ser Ile
   7270 7275 7280
   ggc gaa att aca gga gta aat tgg aac ttg gct aga cag cga ttg 95657
   Gly Glu Ile Thr Gly Val Asn Trp Asn Leu Ala Arg Gln Arg Leu
   7285 7290 7295
   tat caa tgg acc ggc gat ttt cgg gga ctt ccc aca caa tta tcc 95702
   Tyr Gln Trp Thr Gly Asp Phe Arg Gly Leu Pro Thr Gln Leu Ser
   7300 7305 7310
   tgc atg tat gcg gca tat acg tta att gga aca att cca tca gag 95747
   Cys Met Tyr Ala Ala Tyr Thr Leu Ile Gly Thr Ile Pro Ser Glu
   7315 7320 7325
   tct gtg cgt tat aca aga cgc atg gaa cgg ttc gga ggt tat aac 95792
   Ser Val Arg Tyr Thr Arg Arg Met Glu Arg Phe Gly Gly Tyr Asn
   7330 7335 7340
   gtg cca act att tgg tta gag ggg gtt gtg tgg ggg ggt aca aat 95837
   Val Pro Thr Ile Trp Leu Glu Gly Val Val Trp Gly Gly Thr Asn
   7345 7350 7355
   aca tgg aac gaa tgt tat tat taa agcatgtatg taaaataaac tgaatttaac 95891
   Thr Trp Asn Glu Cys Tyr Tyr
   7360
   atagcgtggg ttttgcgtga tattatatac tggggagggg caggctgtac gtaaccatat 95951
   ataagggagt ctacaatatt gtagaactaa ctcagctgtg agtttagggt ttaaaggttt 96011
   attccggagc ctaaatacgt tatccgtt atg gag ttc aaa aga att ttt aat 96063
   Met Glu Phe Lys Arg Ile Phe Asn
   7365 7370
   acg gtt cat gac att ata aac cga tta tgt caa cat ggc tac aag 96108
   Thr Val His Asp Ile Ile Asn Arg Leu Cys Gln His Gly Tyr Lys
   7375 7380 7385
   gaa tac atc att ccg ccc gaa tca acc aca ccg gtg gaa tta atg 96153
   Glu Tyr Ile Ile Pro Pro Glu Ser Thr Thr Pro Val Glu Leu Met
   7390 7395 7400
   gag tat att agc act atc gtc tca aaa ctt aag gcg gtg acg cga 96198
   Glu Tyr Ile Ser Thr Ile Val Ser Lys Leu Lys Ala Val Thr Arg
   7405 7410 7415
   caa gat gag cga gtg tac cga tgt tgt gga gaa ctt atc cat tgc 96243
   Gln Asp Glu Arg Val Tyr Arg Cys Cys Gly Glu Leu Ile His Cys
   7420 7425 7430
   cgt att aac cta cga tcc gtt tcc atg gaa acg tgg ttg act tcc 96288
   Arg Ile Asn Leu Arg Ser Val Ser Met Glu Thr Trp Leu Thr Ser
   7435 7440 7445
   cca att ctc tgt tta act ccc cga gtc cgc caa gca att gaa ggg 96333
   Pro Ile Leu Cys Leu Thr Pro Arg Val Arg Gln Ala Ile Glu Gly
   7450 7455 7460
   cgg agg gac gaa att cgt cgg gct ata tta gaa ccg ttt ttg aaa 96378
   Arg Arg Asp Glu Ile Arg Arg Ala Ile Leu Glu Pro Phe Leu Lys
   7465 7470 7475
   gat caa tac ccc gct tta gct acc ctt gga cta cag tct gct tta 96423
   Asp Gln Tyr Pro Ala Leu Ala Thr Leu Gly Leu Gln Ser Ala Leu
   7480 7485 7490
   aag tac gaa gac ttt tat tta act aag tta gag gaa ggt aaa tta 96468
   Lys Tyr Glu Asp Phe Tyr Leu Thr Lys Leu Glu Glu Gly Lys Leu
   7495 7500 7505
   gag tcg ctt tgc caa ttc ttt tta aga ctg gcg gcc acc gtg aca 96513
   Glu Ser Leu Cys Gln Phe Phe Leu Arg Leu Ala Ala Thr Val Thr
   7510 7515 7520
   aca gaa atc gta aac ctg cct aaa atc gca act ctt att ccc gga 96558
   Thr Glu Ile Val Asn Leu Pro Lys Ile Ala Thr Leu Ile Pro Gly
   7525 7530 7535
   ata aat gat ggt tat aca tgg act gat gtc tgt cgg gta ttt ttc 96603
   Ile Asn Asp Gly Tyr Thr Trp Thr Asp Val Cys Arg Val Phe Phe
   7540 7545 7550
   aca gcg ttg gca tgt cag aaa att gtc ccg gct aca ccg gtt atg 96648
   Thr Ala Leu Ala Cys Gln Lys Ile Val Pro Ala Thr Pro Val Met
   7555 7560 7565
   atg ttt tta ggt cga gag acc ggg gca acg gcc agt tgt tat tta 96693
   Met Phe Leu Gly Arg Glu Thr Gly Ala Thr Ala Ser Cys Tyr Leu
   7570 7575 7580
   atg gac ccg gaa tcc atc act gtt ggg aga gct gtt cga gct atc 96738
   Met Asp Pro Glu Ser Ile Thr Val Gly Arg Ala Val Arg Ala Ile
   7585 7590 7595
   aca ggc gat gtg gga acg gta tta caa agt cga ggt gga gtg gga 96783
   Thr Gly Asp Val Gly Thr Val Leu Gln Ser Arg Gly Gly Val Gly
   7600 7605 7610
   att tct cta cag agt ctg aat tta ata cct acg gaa aat caa acg 96828
   Ile Ser Leu Gln Ser Leu Asn Leu Ile Pro Thr Glu Asn Gln Thr
   7615 7620 7625
   aaa ggt ctt ctt gca gtt tta aaa ctt tta gat tgc atg gtt atg 96873
   Lys Gly Leu Leu Ala Val Leu Lys Leu Leu Asp Cys Met Val Met
   7630 7635 7640
   gca att aac agt gat tgt gaa cga cca act gga gtt tgt gtt tac 96918
   Ala Ile Asn Ser Asp Cys Glu Arg Pro Thr Gly Val Cys Val Tyr
   7645 7650 7655
   ata gaa cca tgg cac gtc gat cta caa act gtt ttg gcc aca cgt 96963
   Ile Glu Pro Trp His Val Asp Leu Gln Thr Val Leu Ala Thr Arg
   7660 7665 7670
   gga atg ttg gtt cgt gat gaa ata ttt cga tgt gat aac ata ttt 97008
   Gly Met Leu Val Arg Asp Glu Ile Phe Arg Cys Asp Asn Ile Phe
   7675 7680 7685
   tgt tgt tta tgg acc cca gat tta ttt ttt gaa aga tac cta agc 97053
   Cys Cys Leu Trp Thr Pro Asp Leu Phe Phe Glu Arg Tyr Leu Ser
   7690 7695 7700
   tat cta aaa ggg gct agt aat gtt cag tgg act ctt ttt gat aac 97098
   Tyr Leu Lys Gly Ala Ser Asn Val Gln Trp Thr Leu Phe Asp Asn
   7705 7710 7715
   aga gcc gat atc ctt cga aca tta cac ggg gag gca ttc act tca 97143
   Arg Ala Asp Ile Leu Arg Thr Leu His Gly Glu Ala Phe Thr Ser
   7720 7725 7730
   acc tat tta cgt tta gag aga gaa gga tta ggc gtt tct tct gtt 97188
   Thr Tyr Leu Arg Leu Glu Arg Glu Gly Leu Gly Val Ser Ser Val
   7735 7740 7745
   ccc att caa gat atc gca ttc aca atc ata cgc agt gct gct gta 97233
   Pro Ile Gln Asp Ile Ala Phe Thr Ile Ile Arg Ser Ala Ala Val
   7750 7755 7760
   aca gga agc ccc ttt tta atg ttc aaa gat gcc tgt aat cgt aat 97278
   Thr Gly Ser Pro Phe Leu Met Phe Lys Asp Ala Cys Asn Arg Asn
   7765 7770 7775
   tat cat atg aat acc caa gga aat gct atc acg ggg tca aat ttg 97323
   Tyr His Met Asn Thr Gln Gly Asn Ala Ile Thr Gly Ser Asn Leu
   7780 7785 7790
   tgt acg gaa att gtt caa aag gca gac gct cat caa cat ggc gta 97368
   Cys Thr Glu Ile Val Gln Lys Ala Asp Ala His Gln His Gly Val
   7795 7800 7805
   tgt aat ctt gcc agc ata aat ctt aca acg tgc tta tcc aaa ggc 97413
   Cys Asn Leu Ala Ser Ile Asn Leu Thr Thr Cys Leu Ser Lys Gly
   7810 7815 7820
   cca gtg tca ttt aat tta aac gac ctt caa ttg aca gca aga acg 97458
   Pro Val Ser Phe Asn Leu Asn Asp Leu Gln Leu Thr Ala Arg Thr
   7825 7830 7835
   act gtt att ttt tta aac ggg gtc ctg gcg gct ggg aac ttt cca 97503
   Thr Val Ile Phe Leu Asn Gly Val Leu Ala Ala Gly Asn Phe Pro
   7840 7845 7850
   tgt aaa aaa tca tgt aaa ggt gta aaa aac aac cga tca ctt ggc 97548
   Cys Lys Lys Ser Cys Lys Gly Val Lys Asn Asn Arg Ser Leu Gly
   7855 7860 7865
   att ggc ata caa ggg tta cat aca act tgt ctc cgc tta gga ttt 97593
   Ile Gly Ile Gln Gly Leu His Thr Thr Cys Leu Arg Leu Gly Phe
   7870 7875 7880
   gat tta act tcc caa cca gct aga cgg tta aat gta caa ata gcg 97638
   Asp Leu Thr Ser Gln Pro Ala Arg Arg Leu Asn Val Gln Ile Ala
   7885 7890 7895
   gag tta atg ttg tat gag aca atg aaa aca agc atg gaa atg tgt 97683
   Glu Leu Met Leu Tyr Glu Thr Met Lys Thr Ser Met Glu Met Cys
   7900 7905 7910
   aag att ggc ggc tta gcc ccg ttt aag ggt ttt acc gaa agt aaa 97728
   Lys Ile Gly Gly Leu Ala Pro Phe Lys Gly Phe Thr Glu Ser Lys
   7915 7920 7925
   tat gct aag gga tgg tta cac caa gat ggg ttt tct acg ata agt 97773
   Tyr Ala Lys Gly Trp Leu His Gln Asp Gly Phe Ser Thr Ile Ser
   7930 7935 7940
   tat tta gat tta cca tgg tgt acc ctg cga gat gat att tgc gct 97818
   Tyr Leu Asp Leu Pro Trp Cys Thr Leu Arg Asp Asp Ile Cys Ala
   7945 7950 7955
   tat ggg tta tac aac tcg cag ttc tta gcg tta atg ccc aca gtt 97863
   Tyr Gly Leu Tyr Asn Ser Gln Phe Leu Ala Leu Met Pro Thr Val
   7960 7965 7970
   tca tct gca cag gta acg gag tgc agt gag ggt ttc tct cca att 97908
   Ser Ser Ala Gln Val Thr Glu Cys Ser Glu Gly Phe Ser Pro Ile
   7975 7980 7985
   tat aat aat atg ttt agt aag gtc acc acc tcg ggt gag tta ctt 97953
   Tyr Asn Asn Met Phe Ser Lys Val Thr Thr Ser Gly Glu Leu Leu
   7990 7995 8000
   aga ccc aac tta gac ctt atg gac gaa cta aga gat atg tat tca 97998
   Arg Pro Asn Leu Asp Leu Met Asp Glu Leu Arg Asp Met Tyr Ser
   8005 8010 8015
   tgt gaa gaa aaa cga ctg gaa gtt ata aac ata ctt gag aaa aac 98043
   Cys Glu Glu Lys Arg Leu Glu Val Ile Asn Ile Leu Glu Lys Asn
   8020 8025 8030
   caa tgg tca gta ata cgt tcg ttt ggc tgt tta tct aat agt cac 98088
   Gln Trp Ser Val Ile Arg Ser Phe Gly Cys Leu Ser Asn Ser His
   8035 8040 8045
   cca ctc tta aaa tat aaa aca gcg ttt gaa tat gag caa gag gat 98133
   Pro Leu Leu Lys Tyr Lys Thr Ala Phe Glu Tyr Glu Gln Glu Asp
   8050 8055 8060
   ctc gtt gat atg tgt gca gaa agg gcg cca ttt att gac caa agt 98178
   Leu Val Asp Met Cys Ala Glu Arg Ala Pro Phe Ile Asp Gln Ser
   8065 8070 8075
   caa tca atg act tta ttt att gag gaa cgc cca gac ggg aca att 98223
   Gln Ser Met Thr Leu Phe Ile Glu Glu Arg Pro Asp Gly Thr Ile
   8080 8085 8090
   ccc gcc tcc aaa ata atg aat ttg ctt ata cgt gcc tat aaa gcc 98268
   Pro Ala Ser Lys Ile Met Asn Leu Leu Ile Arg Ala Tyr Lys Ala
   8095 8100 8105
   ggc ctt aaa acg ggt atg tac tac tgt aaa att cgt aaa gct acg 98313
   Gly Leu Lys Thr Gly Met Tyr Tyr Cys Lys Ile Arg Lys Ala Thr
   8110 8115 8120
   aac agc gga ctg ttt gcg gga ggc gaa tta acc tgt acc agt tgt 98358
   Asn Ser Gly Leu Phe Ala Gly Gly Glu Leu Thr Cys Thr Ser Cys
   8125 8130 8135
   gct tta taa atttacacgg gaaactattc caaa atg gat cag aaa gat tgc 98409
   Ala Leu Met Asp Gln Lys Asp Cys
   8140 8145
   agt cat ttt ttt tac agg ccg gag tgt cca gat ata aac aat tta 98454
   Ser His Phe Phe Tyr Arg Pro Glu Cys Pro Asp Ile Asn Asn Leu
   8150 8155 8160
   cgt gcc ctg agc att tcg aat cgt tgg tta gaa agc gat ttt atc 98499
   Arg Ala Leu Ser Ile Ser Asn Arg Trp Leu Glu Ser Asp Phe Ile
   8165 8170 8175
   att gaa gat gat tat caa tac ttg gac tgt tta acg gaa gat gaa 98544
   Ile Glu Asp Asp Tyr Gln Tyr Leu Asp Cys Leu Thr Glu Asp Glu
   8180 8185 8190
   cta ata ttc tac aga ttt att ttt aca ttt tta tcg gcg gca gat 98589
   Leu Ile Phe Tyr Arg Phe Ile Phe Thr Phe Leu Ser Ala Ala Asp
   8195 8200 8205
   gat ctg gta aat gtt aat ttg ggc tct cta acc caa ctc ttt tcc 98634
   Asp Leu Val Asn Val Asn Leu Gly Ser Leu Thr Gln Leu Phe Ser
   8210 8215 8220
   caa aag gat att cac cat tac tac att gaa caa gag tgc atc gag 98679
   Gln Lys Asp Ile His His Tyr Tyr Ile Glu Gln Glu Cys Ile Glu
   8225 8230 8235
   gtt gtc cac gcg cgt gtc tat agt caa att caa cta atg ttg ttc 98724
   Val Val His Ala Arg Val Tyr Ser Gln Ile Gln Leu Met Leu Phe
   8240 8245 8250
   aga ggg gat gaa tcg ttg cgg gta caa tac gta aat gtc act att 98769
   Arg Gly Asp Glu Ser Leu Arg Val Gln Tyr Val Asn Val Thr Ile
   8255 8260 8265
   aat aat ccg tcg atc caa caa aaa gta caa tgg ttg gaa gaa aag 98814
   Asn Asn Pro Ser Ile Gln Gln Lys Val Gln Trp Leu Glu Glu Lys
   8270 8275 8280
   gta cgg gac aac cca tcc gtt gca gaa aaa tat ata cta atg att 98859
   Val Arg Asp Asn Pro Ser Val Ala Glu Lys Tyr Ile Leu Met Ile
   8285 8290 8295
   ctt ata gag ggc att ttt ttt gta tca tcg ttc gcg gct att gca 98904
   Leu Ile Glu Gly Ile Phe Phe Val Ser Ser Phe Ala Ala Ile Ala
   8300 8305 8310
   tat tta cgc aat aac gga cta ttt gtt gta act tgt caa ttt aac 98949
   Tyr Leu Arg Asn Asn Gly Leu Phe Val Val Thr Cys Gln Phe Asn
   8315 8320 8325
   gac ctt ata agc cga gat gaa gcc ata cat acc agc gca tcg tgt 98994
   Asp Leu Ile Ser Arg Asp Glu Ala Ile His Thr Ser Ala Ser Cys
   8330 8335 8340
   tgt ata tac aat aac tat gta ccc gaa aaa ccc gct atc acc aga 99039
   Cys Ile Tyr Asn Asn Tyr Val Pro Glu Lys Pro Ala Ile Thr Arg
   8345 8350 8355
   ata cat caa ctg ttt tcg gaa gcc gtt gaa atc gag tgt gcg ttt 99084
   Ile His Gln Leu Phe Ser Glu Ala Val Glu Ile Glu Cys Ala Phe
   8360 8365 8370
   tta aaa tcc cat gca ccc aaa acc cgt ttg gtg aac gtc gat gca 99129
   Leu Lys Ser His Ala Pro Lys Thr Arg Leu Val Asn Val Asp Ala
   8375 8380 8385
   att aca caa tac gtg aaa ttc agc gcg gac agg ctt tta tca gcg 99174
   Ile Thr Gln Tyr Val Lys Phe Ser Ala Asp Arg Leu Leu Ser Ala
   8390 8395 8400
   att aat gta cca aaa cta ttt aac acc cca cct ccc gat tcg gac 99219
   Ile Asn Val Pro Lys Leu Phe Asn Thr Pro Pro Pro Asp Ser Asp
   8405 8410 8415
   ttt cca ctt gca ttt  atg att gca gat aaa  aac aca aat ttt ttt      99264
   Phe Pro Leu Ala Phe  Met Ile Ala Asp Lys  Asn Thr Asn Phe Phe
                   8420                 8425                 8430
   gag aga cac agt aca  tct tat gcg ggc aca  gtg ata aac gat tta      99309
   Glu Arg His Ser Thr  Ser Tyr Ala Gly Thr  Val Ile Asn Asp Leu
                   8435                 8440                 8445
   taa catgtatata cgagcaaaat aaaacaatga accattaagt cgctcttatg      99362
   tgtgttttaa ttccaatatt ttgttaatac agtgtttagt gggagtggag taggaataaa  99422
   ctgtttaaaa atacgtgcgt attggcgagc cattttttcc ccctttaagt tatgatataa  99482
   ggtgttaata accatatatg ggtctgccgt attttgcata atatttacac gtttcattaa  99542
   ctttaggcga ccgcgggtca atggagttac cacggcgatt atatgttcaa caaatgcgcg  99602
   ttccaaatta tccaatgggg aggatgaggg tggcagaagg tttaaagccc aactggcgtc  99662
   tactgtcacc tccgatcggt tagatgcata tttggaaacg gacactccgt tttctgtttt  99722
   acccgacgtg gctacggtaa gacttttgaa aacctcgcca tgtctccacg agtcgtaagt  99782
   gggggagcgc gttgaagtgt ccatttgatg tggaactttc aggccggtat cctgaataac  99842
   ttgctgaaca gattttaggt tgtcacttgt atgcaaatcg gtctgacagc gaacaaaggc  99902
   aaccaaaaat tcagggtatg taattcctaa atattgaagc aaatcgcgac atcgtactac  99962
   tggagcaaac ataggaattg catctaacaa aatatcacag cccatgaaga gtaaatcagt 100022
   atctgtcgta taaaccaaag ccactgtacg cgtatgaaat aagtttgcac atgcctcgtc 100082
   cgcctcaatg tcaaccgcct cgacgtatgc ataccccatc catctaataa gacttgcaca 100142
   caatttgtga tataatcgat ggttaggatg tcctcgggat ggcattgtag aggtaagact 100202
   gcatacgttt tggtcgcgca ggcagatcgt cttgtcagtt tgtcgtggac aaccgtccaa 100262
   aaacatatcc tgggatttgt tttcttcgct aggaatacaa ctcgtggaag agtttcgaaa 100322
   cacactgtca tatgtttccg atgttttgtt atacttgata gaacatgatg tgttggatga 100382
   tagtagaaca cgggttaagt ccgaggttcc cctttcatcg tggttcatgg cccgtgacat 100442
   aattgccttg gctccacgtg ataatgggcc gtctgtacaa cgttccaata caaatatcgg 100502
   atagtatgac cgttgtgtta atagccgcaa taagactcca agacaatgta tcgttacaga 100562
   tggtccgtgt aaattctcgc gtttacccac agggtataaa cgttccaaca atgtgtacat 100622
   gacgttccat acgtctaccg ccacgggggt taaaactccg ggtggcgttg aaatgatgct 100682
   gggtttaacc agtttatgtt catggataaa gcgtgtcagt ccaaagagcc ccatcaatac 100742
   acttaaccaa gtttatatcg tcttgtgaaa ggtttcacag cggcttcagt aattcttcga 100802
   tgtgatcttg tgttaattac agacgtatat aatgatgtac atatttttta tgacaaacat 100862
   tacatcaaat ctggtaactt cttacgtatt ttattgcaca gtccctatta aggaacaccc 100922
   cctgtattgg aacacgtgga aatgttccct cccatgggcc gtactattca aatatcatgc 100982
   ctgtttatgt acttaagtca ccggatcggg ttaaacaaac atattaacga aactcgtgtg 101042
   tttacatatg attacttttt ctatagtaaa cattttaagt agtaaatt atg gat ttg  101099
                                                           Met Asp Leu
   agg tcg cgt aca gac gat gct ttg gac atg gaa ttg cat gcg ggt     101144
   Arg Ser Arg Thr Asp Asp Ala Leu Asp Met Glu Leu His Ala Gly
       8450                8455                8460
   ttt gac gcc cca gaa atc gcc aga gct gtt tta acg gaa aaa acg     101189
   Phe Asp Ala Pro Glu Ile Ala Arg Ala Val Leu Thr Glu Lys Thr
       8465                8470                8475
   ctt act ggt tta att tcg tct ata tca cct ctg gtt aat aga cta     101234
   Leu Thr Gly Leu Ile Ser Ser Ile Ser Pro Leu Val Asn Arg Leu
       8480                8485                8490
   agg gat tct att tta ata ttc agc gac gaa gga tta att att cac     101279
   Arg Asp Ser Ile Leu Ile Phe Ser Asp Glu Gly Leu Ile Ile His
       8495                8500                8505
   tgt agt ttg gaa aca gaa caa ctg tat att cct ata ccg gca aat     101324
   Cys Ser Leu Glu Thr Glu Gln Leu Tyr Ile Pro Ile Pro Ala Asn
       8510                8515                8520
   atg ttt gac cag tat aat tgg act ggg ccg aga atg gtt gta ctc     101369
   Met Phe Asp Gln Tyr Asn Trp Thr Gly Pro Arg Met Val Val Leu
       8525                8530                8535
   gcg gca acg gag gga cgg tcc tcg ctt att gac gcg ttt cgc cat     101414
   Ala Ala Thr Glu Gly Arg Ser Ser Leu Ile Asp Ala Phe Arg His
       8540                8545                8550
   aca aaa gat ccg tcg acc cca aca cgg tta tat ttt aaa ttt acc     101459
   Thr Lys Asp Pro Ser Thr Pro Thr Arg Leu Tyr Phe Lys Phe Thr
       8555                8560                8565
   gga caa ccc ccc gag cgg agt att atc caa acg atg gta tgg caa     101504
   Gly Gln Pro Pro Glu Arg Ser Ile Ile Gln Thr Met Val Trp Gln
       8570                8575                8580
   cgc ccg ggt gat tgt ggt cca gat gat caa gta caa tgt tac aaa     101549
   Arg Pro Gly Asp Cys Gly Pro Asp Asp Gln Val Gln Cys Tyr Lys
       8585                8590                8595
   caa gtt gta aaa cgt gaa ctc gct tgt tat aca atg atg ttt cca     101594
   Gln Val Val Lys Arg Glu Leu Ala Cys Tyr Thr Met Met Phe Pro
       8600                8605                8610
   aat cta act cca gat ata agc att tgc tta aaa cgc gat caa ttc     101639
   Asn Leu Thr Pro Asp Ile Ser Ile Cys Leu Lys Arg Asp Gln Phe
       8615                8620                8625
   acc cgt tta cag cga cta ctt aaa act ttt ggg ttt aca aca tgc     101684
   Thr Arg Leu Gln Arg Leu Leu Lys Thr Phe Gly Phe Thr Thr Cys
       8630                8635                8640
   ttc att cta aca gcc acg gat atg tac atc cag acc gcc ggg ggt     101729
   Phe Ile Leu Thr Ala Thr Asp Met Tyr Ile Gln Thr Ala Gly Gly
       8645                8650                8655
   ggt ttt atc tca ttt aat gtt tcc ttg gat ata aac gga agc aag     101774
   Gly Phe Ile Ser Phe Asn Val Ser Leu Asp Ile Asn Gly Ser Lys
       8660                8665                8670
   cct aca cca tat aat tta ata cgc tca atc aca aat tca aaa agg     101819
   Pro Thr Pro Tyr Asn Leu Ile Arg Ser Ile Thr Asn Ser Lys Arg
       8675                8680                8685
   atc ctt aat aat gtt gtt tat ggc agc ggg agt atg cgt gaa ttt     101864
   Ile Leu Asn Asn Val Val Tyr Gly Ser Gly Ser Met Arg Glu Phe
       8690                    8695                    8700
   gga gta tta ttg gaa aca cac agt gga ttc cgt tct gcc gta caa     101909
   Gly Val Leu Leu Glu Thr His Ser Gly Phe Arg Ser Ala Val Gln
       8705                    8710                    8715
   aat ctt aag tta aca cgg gat gag acg tgt tat att aat ttt tat     101954
   Asn Leu Lys Leu Thr Arg Asp Glu Thr Cys Tyr Ile Asn Phe Tyr
       8720                    8725                    8730
   ctc gcc tta act aac tcc ccc atg gtt gga ttg tat atc caa cgt     101999
   Leu Ala Leu Thr Asn Ser Pro Met Val Gly Leu Tyr Ile Gln Arg
       8735                    8740                    8745
   tcc gca ccc gtg cat tct ttt ttt tat gca acg ttc tta agt ccc     102044
   Ser Ala Pro Val His Ser Phe Phe Tyr Ala Thr Phe Leu Ser Pro
       8750                    8755                    8760
   aaa gac ctt aaa gaa aaa tta acc tcg atg caa tta ttt gcg aac     102089
   Lys Asp Leu Lys Glu Lys Leu Thr Ser Met Gln Leu Phe Ala Asn
       8765                    8770                    8775
   atg gaa tct gtg aag gat gaa cca cca tta aaa aaa aga cgc aat     102134
   Met Glu Ser Val Lys Asp Glu Pro Pro Leu Lys Lys Arg Arg Asn
       8780                    8785                    8790
   tta tta aca aaa aga aac gaa aaa aat acc gga aat aaa atg ggg     102179
   Leu Leu Thr Lys Arg Asn Glu Lys Asn Thr Gly Asn Lys Met Gly
       8795                    8800                    8805
   ggg aaa ctc ccc gaa acc aca tgg cag gag gga atc gga att cgc     102224
   Gly Lys Leu Pro Glu Thr Thr Trp Gln Glu Gly Ile Gly Ile Arg
       8810                    8815                    8820
   gaa tat tgt gtg gct cct cca gtg gac cct gca gga acc ctg gat     102269
   Glu Tyr Cys Val Ala Pro Pro Val Asp Pro Ala Gly Thr Leu Asp
       8825                    8830                    8835
   tat tct gaa tta tca cgt gaa tct gac gta ata tgt aca gtt aaa     102314
   Tyr Ser Glu Leu Ser Arg Glu Ser Asp Val Ile Cys Thr Val Lys
       8840                    8845                    8850
   taa gtgcaacttt tgcttatatt ttacatacaa acttgtgtgt accatagatg     102367
   aacacatttt tatttgtttt gaattattaa acttaagac atg gcc gtg aat ggt     102421
                                              Met Ala Val Asn Gly
                                                  8855
   gaa aga gct gtc cat gat gaa aac ctg ggt gtg tta gac aga gaa     102466
   Glu Arg Ala Val His Asp Glu Asn Leu Gly Val Leu Asp Arg Glu
       8860                    8865                    8870
   tta atc cgc gct caa tca atc caa gga tgt gtc gga aac cct caa     102511
   Leu Ile Arg Ala Gln Ser Ile Gln Gly Cys Val Gly Asn Pro Gln
       8875                    8880                    8885
   gaa tgt aat tcg tgt gca ata acc tca gca tcg cgg ttg ttt ctc     102556
   Glu Cys Asn Ser Cys Ala Ile Thr Ser Ala Ser Arg Leu Phe Leu
       8890                    8895                    8900
   gtg gga cta caa gca agc gtt atc acg tcc ggg tta att tta caa     102601
   Val Gly Leu Gln Ala Ser Val Ile Thr Ser Gly Leu Ile Leu Gln
       8905                    8910                    8915
   tat cac gtc tgc gaa gct gcc gtc aat gca act att atg ggg ttg     102646
   Tyr His Val Cys Glu Ala Ala Val Asn Ala Thr Ile Met Gly Leu
       8920                    8925                    8930
   atc gtc gtt tcg ggg tta tgg cca aca tcc gtg aaa ttt cta cgc 102691
   Ile Val Val Ser Gly Leu Trp Pro Thr Ser Val Lys Phe Leu Arg
   8935 8940 8945
   aca tta gca aaa ttg gga cga tgt ttg cag acg gtg gtc gtg ttg 102736
   Thr Leu Ala Lys Leu Gly Arg Cys Leu Gln Thr Val Val Val Leu
   8950 8955 8960
   ggt ttt gct gtg tta tgg gcg gtt ggt tgc cca ata tcc cgg gat 102781
   Gly Phe Ala Val Leu Trp Ala Val Gly Cys Pro Ile Ser Arg Asp
   8965 8970 8975
   ctt cca ttt gta gaa tta ctg gga att tcc ata tcc gcg att acc 102826
   Leu Pro Phe Val Glu Leu Leu Gly Ile Ser Ile Ser Ala Ile Thr
   8980 8985 8990
   gga aca gtg gct gct gtg cat atc cat tac tac aac ttt gtt acg 102871
   Gly Thr Val Ala Ala Val His Ile His Tyr Tyr Asn Phe Val Thr
   8995 9000 9005
   aca ttc aat gga ccg cat att tat ttt tat gtt atg atg ttg gga 102916
   Thr Phe Asn Gly Pro His Ile Tyr Phe Tyr Val Met Met Leu Gly
   9010 9015 9020
   act ggg ttg gga ggt tta cta acc gtt att tta tat atg tat gtc 102961
   Thr Gly Leu Gly Gly Leu Leu Thr Val Ile Leu Tyr Met Tyr Val
   9025 9030 9035
   agt aaa tat gag gtt ctt att gga ttg tgt ata tct att gtc aca 103006
   Ser Lys Tyr Glu Val Leu Ile Gly Leu Cys Ile Ser Ile Val Thr
   9040 9045 9050
   cta gtt tca att gtc gat gcc gcc acc gat ttg caa gat acg tgt 103051
   Leu Val Ser Ile Val Asp Ala Ala Thr Asp Leu Gln Asp Thr Cys
   9055 9060 9065
   ata tat cgt aaa aat cgc cat aag caa tta aac act tat aca gat 103096
   Ile Tyr Arg Lys Asn Arg His Lys Gln Leu Asn Thr Tyr Thr Asp
   9070 9075 9080
   tta ggt ttt gcc gtt gta tat aca caa aat gac cgc ggg aga gta 103141
   Leu Gly Phe Ala Val Val Tyr Thr Gln Asn Asp Arg Gly Arg Val
   9085 9090 9095
   tgt gac cat cga gaa agt tcc cgg acc ctt aaa cgc gtg ttt aaa 103186
   Cys Asp His Arg Glu Ser Ser Arg Thr Leu Lys Arg Val Phe Lys
   9100 9105 9110
   gga att cgt ata atg tct gtt ata ccc ccg gtg tta tat ata gtt 103231
   Gly Ile Arg Ile Met Ser Val Ile Pro Pro Val Leu Tyr Ile Val
   9115 9120 9125
   acc cca tta atg tgg gca atc tca cat ata att aaa tta aat cat 103276
   Thr Pro Leu Met Trp Ala Ile Ser His Ile Ile Lys Leu Asn His
   9130 9135 9140
   ttt atc aaa ctt aca caa gta acg tta gca gtt tca ata gga ggt 103321
   Phe Ile Lys Leu Thr Gln Val Thr Leu Ala Val Ser Ile Gly Gly
   9145 9150 9155
   cat att ata gca ttt ggg tta cag ggt ttt gcc gtt tta tat caa 103366
   His Ile Ile Ala Phe Gly Leu Gln Gly Phe Ala Val Leu Tyr Gln
   9160 9165 9170
   gaa aaa aaa aac cta tgg gta att gta tta tat aca acg acc tcg 103411
   Glu Lys Lys Asn Leu Trp Val Ile Val Leu Tyr Thr Thr Thr Ser
   9175 9180 9185
   gtg acg ggt ata gct gta aca ttt gcc ggc att tca tgg gga gct 103456
   Val Thr Gly Ile Ala Val Thr Phe Ala Gly Ile Ser Trp Gly Ala
   9190 9195 9200
   att ata att cta aca tca aca gtt gcg gcg ggt ttg acg tgt att 103501
   Ile Ile Ile Leu Thr Ser Thr Val Ala Ala Gly Leu Thr Cys Ile
   9205 9210 9215
   cag atg atg aga cta agc gtt aaa cct att gac tgt ttt atg gca 103546
   Gln Met Met Arg Leu Ser Val Lys Pro Ile Asp Cys Phe Met Ala
   9220 9225 9230
   tct cat atc act aaa gta tat cac gtg tgt gtt tat att ata ata 103591
   Ser His Ile Thr Lys Val Tyr His Val Cys Val Tyr Ile Ile Ile
   9235 9240 9245
   aat cta tgc tat cta tgt ggt aca tat gta tcg taa tcgagataaa 103637
   Asn Leu Cys Tyr Leu Cys Gly Thr Tyr Val Ser
   9250 9255
   taaagttttt aaagttgcaa aagccgtttt tattattccc aatgtcgaaa aaaacgtttc 103697
   catcatttaa attccgcggt gggtgtttta atcttttatt taaggggagc gtggatgtgt 103757
   caataaaaac cagg atg aag cgg ata caa ata aat tta att tta acg 103804
   Met Lys Arg Ile Gln Ile Asn Leu Ile Leu Thr
   9260 9265 9270
   atc gcg tgt ata caa tta tcg act gaa tct caa ccc aca ccc gta 103849
   Ile Ala Cys Ile Gln Leu Ser Thr Glu Ser Gln Pro Thr Pro Val
   9275 9280 9285
   agt ata act gaa tta tat acc tcg gcc gct acc cga aag ccc gat 103894
   Ser Ile Thr Glu Leu Tyr Thr Ser Ala Ala Thr Arg Lys Pro Asp
   9290 9295 9300
   ccc gcc gtc gcg ccc acc tcg gcc gct tcc cga aag ccc gat ccc 103939
   Pro Ala Val Ala Pro Thr Ser Ala Ala Ser Arg Lys Pro Asp Pro
   9305 9310 9315
   gcc gtc gcg ccc acc tcg gcc gct tcc cga aag ccc gat ccc gcc 103984
   Ala Val Ala Pro Thr Ser Ala Ala Ser Arg Lys Pro Asp Pro Ala
   9320 9325 9330
   gtc gcg ccc acc tcg gcc gct tcc cga aag ccc gat ccc gcc gtc 104029
   Val Ala Pro Thr Ser Ala Ala Ser Arg Lys Pro Asp Pro Ala Val
   9335 9340 9345
   gcg ccc acc tcg gcc gct acc cga aag ccc gat ccc gcc gtc gcg 104074
   Ala Pro Thr Ser Ala Ala Thr Arg Lys Pro Asp Pro Ala Val Ala
   9350 9355 9360
   ccc acc tcg gcc gct tcc cga aag ccc gat ccc gcc gtc gcg ccc 104119
   Pro Thr Ser Ala Ala Ser Arg Lys Pro Asp Pro Ala Val Ala Pro
   9365 9370 9375
   acc tcg gcc gct acc cga aag ccc gat ccc gcc gtc gcg ccc acc 104164
   Thr Ser Ala Ala Thr Arg Lys Pro Asp Pro Ala Val Ala Pro Thr
   9380 9385 9390
   tcg gcc gct tcc cga aag ccc gat ccc gca gcc aac acc caa cat 104209
   Ser Ala Ala Ser Arg Lys Pro Asp Pro Ala Ala Asn Thr Gln His
   9395 9400 9405
   tca caa cca cct ttt cta tat gaa aat ata caa tgc gtt cac ggc 104254
   Ser Gln Pro Pro Phe Leu Tyr Glu Asn Ile Gln Cys Val His Gly
   9410 9415 9420
   gga ata caa tcc ata ccc tat ttt cac aca ttt atc atg cct tgt 104299
   Gly Ile Gln Ser Ile Pro Tyr Phe His Thr Phe Ile Met Pro Cys
   9425 9430 9435
   tac atg cgt cta acg acc gga caa cag gcg gcc ttt aag cag caa 104344
   Tyr Met Arg Leu Thr Thr Gly Gln Gln Ala Ala Phe Lys Gln Gln
   9440 9445 9450
   caa aaa aca tat gaa caa tat tct tta gat ccg gaa ggt tca aat 104389
   Gln Lys Thr Tyr Glu Gln Tyr Ser Leu Asp Pro Glu Gly Ser Asn
   9455 9460 9465
   ata aca agg tgg aag tcg ctt ata cgc ccc gat ctt cat att gaa 104434
   Ile Thr Arg Trp Lys Ser Leu Ile Arg Pro Asp Leu His Ile Glu
   9470 9475 9480
   gtt tgg ttt acg cgt cac ctt ata gat ccg cac cgt caa ctg ggc 104479
   Val Trp Phe Thr Arg His Leu Ile Asp Pro His Arg Gln Leu Gly
   9485 9490 9495
   aat gcg tta ata cgc atg cca gat tta ccg gtt atg tta tat agc 104524
   Asn Ala Leu Ile Arg Met Pro Asp Leu Pro Val Met Leu Tyr Ser
   9500 9505 9510
   aac agt gcc gat tta aac tta ata aac aac cct gag ata ttt aca 104569
   Asn Ser Ala Asp Leu Asn Leu Ile Asn Asn Pro Glu Ile Phe Thr
   9515 9520 9525
   cac gct aag gaa aat tat gta ata cca gat gtt aaa aca acg tct 104614
   His Ala Lys Glu Asn Tyr Val Ile Pro Asp Val Lys Thr Thr Ser
   9530 9535 9540
   gat ttt tct gta aca att tta tct atg gat gct acc acg gag gga 104659
   Asp Phe Ser Val Thr Ile Leu Ser Met Asp Ala Thr Thr Glu Gly
   9545 9550 9555
   acg tat att tgg cga gtc gtt aat aca aaa act aag aac gtc ata 104704
   Thr Tyr Ile Trp Arg Val Val Asn Thr Lys Thr Lys Asn Val Ile
   9560 9565 9570
   tcg gaa cac agt att aca gtt aca acg tat tat cgt cca aat att 104749
   Ser Glu His Ser Ile Thr Val Thr Thr Tyr Tyr Arg Pro Asn Ile
   9575 9580 9585
   acc gtt gtc ggc gat cca gtc tta acc gga cag aca tac gca gcc 104794
   Thr Val Val Gly Asp Pro Val Leu Thr Gly Gln Thr Tyr Ala Ala
   9590 9595 9600
   tac tgt aac gta tca aag tat tat cca ccg cac tcg gta cgt gtt 104839
   Tyr Cys Asn Val Ser Lys Tyr Tyr Pro Pro His Ser Val Arg Val
   9605 9610 9615
   cgg tgg act tca agg ttt ggt aac atc gga aaa aat ttt ata acc 104884
   Arg Trp Thr Ser Arg Phe Gly Asn Ile Gly Lys Asn Phe Ile Thr
   9620 9625 9630
   gat gca ata caa gaa tat gcc aat gga tta ttt agt tat gtt tcg 104929
   Asp Ala Ile Gln Glu Tyr Ala Asn Gly Leu Phe Ser Tyr Val Ser
   9635 9640 9645
   gcg gta cga att cca cag caa aaa caa atg gat tac cca ccc cca 104974
   Ala Val Arg Ile Pro Gln Gln Lys Gln Met Asp Tyr Pro Pro Pro
   9650 9655 9660
   gcc atc caa tgt aat gtt tta tgg att cgg gat ggc gtc tct aat 105019
   Ala Ile Gln Cys Asn Val Leu Trp Ile Arg Asp Gly Val Ser Asn
   9665 9670 9675
   atg aaa tat tct gct gtc gtt acc cct gac gtc tat cca ttt ccc 105064
   Met Lys Tyr Ser Ala Val Val Thr Pro Asp Val Tyr Pro Phe Pro
   9680 9685 9690
   aac gtg tct ata ggt att att gat gga cac ata gta tgt acg gca 105109
   Asn Val Ser Ile Gly Ile Ile Asp Gly His Ile Val Cys Thr Ala
   9695 9700 9705
   aaa tgt gtg cca cgt ggc gtt gta cat ttc gta tgg tgg gtt aac 105154
   Lys Cys Val Pro Arg Gly Val Val His Phe Val Trp Trp Val Asn
   9710 9715 9720
   gat tct ccc att aac cac gaa aac agt gag att act ggg gtg tgt 105199
   Asp Ser Pro Ile Asn His Glu Asn Ser Glu Ile Thr Gly Val Cys
   9725 9730 9735
   gat caa aac aaa cgg ttt gta aac atg caa agt tct tgt cca aca 105244
   Asp Gln Asn Lys Arg Phe Val Asn Met Gln Ser Ser Cys Pro Thr
   9740 9745 9750
   tcg gaa ctc gac gga cct atc acc tat tcg tgt cat cta gat ggt 105289
   Ser Glu Leu Asp Gly Pro Ile Thr Tyr Ser Cys His Leu Asp Gly
   9755 9760 9765
   tac cct aaa aaa ttc cct ccg ttt tcg gcc gtt tat acc tac gat 105334
   Tyr Pro Lys Lys Phe Pro Pro Phe Ser Ala Val Tyr Thr Tyr Asp
   9770 9775 9780
   gca tct acc tac gcc act aca ttt tcc gtt gta gca gtt ata att 105379
   Ala Ser Thr Tyr Ala Thr Thr Phe Ser Val Val Ala Val Ile Ile
   9785 9790 9795
   ggt gtg ata tct atc ctt ggg aca ttg ggt ctt atc gca gtt atc 105424
   Gly Val Ile Ser Ile Leu Gly Thr Leu Gly Leu Ile Ala Val Ile
   9800 9805 9810
   gca acc cta tgc atc cgt tgc tgt tca taa acagaaacca accaaacgcg 105474
   Ala Thr Leu Cys Ile Arg Cys Cys Ser
   9815
   tctgtgtata tcattttatt acattcgcaa cacatctact gtcttgacaa catttaaaaa 105534
   tccattaaag agccatttcc atttttaggg gggggtgtgg attatatcca tcaagctgaa 105594
   aatcgtccca tttaaagtcg tttatatctg ttacatttcg aataatttta aggcaaggaa 105654
   aaggttttgg ggatcgagct agctgcactt ttaaagcatc tatatgattc aagtaaatat 105714
   gtgcatcccc cattgtatga attaaatctc cggttttaag tcctgtaaca tgcgctacta 105774
   tgtaggtaag aagtgcatat ccagcaatgt tgaacggtac cccaaggccc atatcccccg 105834
   atctctggta tacttggcag gataattcac cgtttgcaac gtaaaactga cataacgtgt 105894
   gacatggagg tagtaccatt aaggggatat cctttggatt ccaagacgat ataatcattc 105954
   gtcggctttc tgggtttgtt ttaattgtat ctataacagt ttgcagctga tcgattcctt 106014
   gctgtaaata gtttgattga cagtctttat attccgctcc aaaatgtctc cactggaagc 106074
   cgtaaatggg gccaaggtcc cccgtgtgtc ttttatggaa gccattccta tttagaaatt 106134
   tgctcgatcc gtatatatcc catatgtgta tatctttagc ggcgagttct ttggaatcgg 106194
   ttgacccgcg gataaaccat aacaactctt ccacgacggc cctccaaaaa acacgctttg 106254
   tagttaaaag aggaaattca tttcgcaaat tgtatcgagc ttgcattcca aataaagata 106314
   acgttccgat tcctgttcga tcgcgtttcc gaactccata ccttaaaata tcatccactt 106374
   gttttaagta ctgaagttcg ccggttaacg taaaacccgg cacctttgtc caacatgaca 106434
   agtctcccat ggtaccggta ataatcgtta aatacaaacg accacttgat attgtgggta 106494
   cattaagtaa aatttaaagg agtaattcct tttataaccc aatcaaccaa tcagaccttt 106554
   aaataacgca gtccaattat tgaacagaaa atacgcaata gactattttc tcccaaatcc 106614
   cccaatttta acctggttgt ggaaatgacc gccaaagtgc ctagtccata actatctatt 106674
   aaccgctgta cttaatgatg actcttaggc gtatttttcc taaacgtaac cgtggtttta 106734
   catctaacgg gaacgtcgtt gggtataaaa tctggtgata agtcatcgtc tgtatgcatg 106794
   tctccggagg tgtacccgtt aaggctatcc acatccaggt ctgaagatga gttaaaaccc 106854
   acttccttgg gtggtatatt ttcatatacg tgatcattta aagatgcttg ggtttctatg 106914
   tgacgtaaat ccgatctttg gtcaaacatt gtgtttgatt catcccgaac cggaccttca 106974
   tattccgcct ttatccgggt gtaatgatca ggatacgttg ggtcaactgg actaccgtcc 107034
   gtatggccta tgtccagaca gtggtttttt cggacagtta catccaaacc cgtgtctgga 107094
   cgaactaaaa cgcgagaagt ccgctgtcta tttgtatcgg gtgctatact gtttaacggt 107154
   gcttctgtat taattacatg cgaacttgca tttacgtcgt cagataaata atttcccgtt 107214
   aaacagacac attccaaatt ccctatacct gtccctattg gagatcccag tcgattaaaa 107274
   cgaatatacg gctgatcaga cacactcgat ggttcggata tacgtcttaa atgtaacgga 107334
   gtataccgtt tactggcggc aagaacctga gcataatact ttgtgggttt acccccatgt 107394
   gctaaaatac tctgcgccac ggcccgggaa aaccacatat gggtgctggt ctctaacaca 107454
   acccaactgt gtgttgacat tgttctgacc aactgacccg caaaatataa aaaccgtccc 107514
   tgggcacgta gcgcctttaa tatataagga tcgtttaatc ccccatcccc ccaacatgca 107574
   tatccaataa gcatcatatt aattaagtat tgaaaatggt ggtgcaaaag cgtaataagt 107634
   tcaaccgccg caacaattgc agctgcggtt ccatttaaac catcccgcca ctcttccttt 107694
   cgccataata cactaatacg caaaagcgca gtaagagcgc tggcagttgt tgctgctaaa 107754
   ataaacgcaa ctcctgtgcc cgctgagacc gcaggacccg tttttaaatg tcgtctaagc 107814
   aaatcatctg gaataacaga cgcgcgcggc cctcgaatcc gtcgaaattg aagtttgaga 107874
   tggcgacata ccctggcgtc cacaacgtcc aagaaccata acatccaccc gtagccagta 107934
   gccgtgtaag ccgcaatttc ttgtacacgt attcttgtat ccgggctggg gtttgtagga 107994
   tcatctggac gcataaaata catatacttt tgcacagctc ctaacgcctc acgtagctcc 108054
   tgacatattc cccggtaatc cgaatttata actttagatt taaaaggatg tctggatatt 108114
   ggtttattgg caactacgga tgaaaacaac aaaaccgtgg atttagactt tttatttacc 108174
   ttctctgaag tcaccggtac ctcggctcct gtggtgtatt tgagatactg ccaatatgta 108234
   ttttcataaa cccaccaaag tccatcccca gaaagtttca ggtcatccaa acatctttga 108294
   attgtttcca tataacgctg cataaacccg tcatgaacat cgttagacat acacgagacc 108354
   accaacgatt caggaacatt attatggtgt aatccaagca gaacactgtg tctttcggta 108414
   ctttgtaaaa ccggcgatgt taggctgtcg gtcttttcag ataaagcctt taccgccgtc 108474
   tccaaagcct cgccgggcgt tggaataaga catccatccc tcacgcgctc gctggttatg 108534
   cgttgggaaa tagaacacca gtcccgggta ggtgtgggca aatcacgttc gccggcatta 108594
   aaactttggt aactaccatc ataagattta cgcgttctat catcgctgga aaaggaacgc 108654
   gcaaaccgag aaaacattgc gatatggaca aattatatct tgtaaaggtc gatcctcacg 108714
   gaccccccac acaacacaca caacaaattt taaggtctaa acagagattt tattttacaa 108774
   actcctttgt gggtgtggct aggaaacgtt cttttcatcc taatgaaaaa aatcacaacc 108834
   cttaatattt tcgtagtaaa tgcatggcta cgcttttcca atccaaaccc agaatttcat 108894
   tcctgtattg cataagattt tcggctaggt ccacggaggg aatgggctgt tctcggggat 108954
   agatggtctc caatcccaca aaacggaagt tcatctctat aggtgatgcc tgaactatcc 109014
   tgtcttgctc tttgggtaaa acgtccgttg tcggtcgaat ccccaaagtt tgcattgcat 109074
   catcgcggtc tttccaaaag gatgttaaag tttggtgagc gtaaagagga cttgctgcca 109134
   acattaaagt attatatgca tctaaaatac ctcgggggat gtaaattgaa catcctccgt 109194
   acaacgcagc accggagaga agcaacaaaa gaagatttgc atgacccaac acccgttgta 109254
   acaataaaac ggcccccagg tacgcgctta ccattaaatc ggtctcactt tccttataac 109314
   aatcggcaaa ctctatcata aaatttgtgg ctgctgtgcg aacagcctca tatccggtta 109374
   attcgtgtgc aatagttgcg taggattcac aaacagttgg gaggtcgacc tcgcctaaca 109434
   gtaatacctc taaatcacat aacaggtcac tattaaccgg gatatagtaa tagggcagag 109494
   attcacagac cgcgatgtaa gccgaacggg aatggtaagg agcagctaaa tacattgcag 109554
   ttgctctaca gatccagttc aacattcctc caccaagagt aacatacaac gtaaaaaatg 109614
   acgctaacat ttcccgttcc gttggggtaa attgtggctt taaactatgt gtacgtgaaa 109674
   ataaaaacca ttcggcaagt tccatgtgac cggtagcata tcgcaccaaa gatgtagatg 109734
   gttcacttat agcaattctt agttcgggcc aatatgccaa cgccccaaaa aaaccacgca 109794
   taatggcaac cgttggaccc cgatttggca aaaccaactg tgtcacttgg agtacgtcgg 109854
   gtacggcttc tcgtggtaat ccggcaagat ggtcttctaa ccaacatgga tcccccacgc 109914
   caaaattatc gttacgtccc aaaaaaatac agtttgcgct aatacgaatt gccgcgtcta 109974
   acaaaaatcc taatccatct ccatgtgaaa tgcgattaga aatagcgcat gctgctgtgg 110034
   ataatatcat gtgatgccaa atggccgttc cttggcccac agcacgtaag gacacgtcat 110094
   aaaacccagg aatgtgttgt acatacattt tacccgcatt atatggaagt gcgtagacgg 110154
   aattccaacg cgggccgtat ttatgtgttt tactggaagc ccgcctatct aaaaatacat 110214
   cttcaactaa aatacgttct atagaaattg gctgggccat aaattcaata ggaaaaatca 110274
   ataaaagttc accaagtgtc atgttaggaa gggctggtat agttctcaaa atctgtgggg 110334
   gtgtcatccg ggaagtatca aactgatacc gtaaatgaat tgggtcgtat aatccttgga 110394
   ccgtaatata ttcgcgacgg gtacattcgc gcatagcaaa ccaggactca tcaaacccat 110454
   tatgcgctaa catagagcct gttagggtaa caggttcaac acaacgtttt gatacattaa 110514
   gttcctttga taccgcaggc tccataacct ttgcaatgcg aagatcggta ctgtaactta 110574
   tttccgggtc taacgtaaaa taaacctcat ccgcgtctcg actgcaaact tgacttacag 110634
   aaaaataatc ctcctccgcc tctccctcgt cgtcgatcgc gtcctcctcc gccgcgtcct 110694
   cctccgcctc tccctcgtcg tcgatcgcgt cctcctccgc ctctccctcc tccgcctctc 110754
   cctcctccgc ctctccctcc tccgcctctc cctcgtcgtc aatcgcgtcc tcctccgcct 110814
   ctccctcctc cgcctctccc tcgtcgtcaa tcgcgtcctc ctccgcctct ccctcctccg 110874
   cctctccctc gtcgtcgatc gcgtcctcct ccgcctctcc ctcctccgcc tctccctcgt 110934
   cgtcgatcgc gtccgtattg atgttgattt cctcattagc ttccctctcc gttaatttaa 110994
   atatgcgatt ttcatgtctg gaagttgatg tattttgttc ggatccatta tatgtagaat 111054
   gatgtaaggc gtatcccgga aaatcatccg atgcgtcaga actgctgtcc tgatccatct 111114
   cggagttcag tagttcttga accgcaacta atgtttctga attggacaat atctggggtg 111174
   ggtgtgtata ccaattggtt gaccgataac gtgttccgtg tgtgtgacgg ggggagtctg 111234
   tggtattaga cgatatccgc tgtcggcggg attgcctccg gttataatga cccgactgca 111294
   tacttataac cgagacaaac aaacgcgcct gtaaaacatc ccagggtcgc ggtacgcaat 111354
   acgcatacac tcgttggggc ttcttttata tatgggactt tagagcacat gacagacata 111414
   ccatatacgg cgcattgtaa aaataaaaaa cgcatgcacg ttttcgtaat ttatttacac 111474
   cctctacccc aatgacgttt aacgcgttaa aaacccacac gtgggtgggc gtggtgggtc 111534
   ccccggcagg atctcggatg gggacggagg tgctaaaatc atagcttcga tgctactacc 111594
   gtagtttcta ttttcaacaa cttttgcgta aggatgatcc gctcgtacat gtctcggttc 111654
   ttgaggatgt ttgaccgcat atgcgtctaa cttacgctta atgtggtggg taagaaaacc 111714
   caccgaccgt ggtaaatgaa ccgaaaatga gggttgttga accaacggag atttgttttc 111774
   ttcaacaaga ccacatctaa ctagaggcag tcccagttct cggcggcggt aatttatttc 111834
   cctcaaggca gacgctgtta gtggtttccc ttctaataac acaatgccgt ggttgcataa 111894
   tacaggatga aacgcacacg tgaactgtcg acgttcggtc caggtgaatt ttaaagccgc 111954
   aaacacgtcc gggtgtgcag attgactggc gtacaaacgc caggaaaatt cacgcgttac 112014
   ggttaaatat aaatgtaggt acagaagacg cgccaaactt gccacctcac gataatatct 112074
   aagtagaata ctttgcctga gttgcgtgta agctttctgg tcagggtttt gtatattaag 112134
   acctattgtc gtccttttcg ccgttccttg gagataacgt ataatcgatt tacaataagt 112194
   aactagtagt tttgtgtatg cttcttcccg ggcgcgtagt tctacggtaa acgaatcctg 112254
   gacctcctga acgtaagctg gaagccccgt cggttgcttt ggtggactag gcaatcgaac 112314
   ggaaccccgg gttgttaaat taaacatttc aacatggtct ttggttgaaa cggttgagat 112374
   aacgtcatct ggatccgggg ataaaaccat catatcggaa tataggtcct cattaatagg 112434
   aaaacatgag aataaatcct cgttccaggt ttcaagacat gatagtaaac gcggcccttc 112494
   tgaaaaatca agatcccgta ttaactgttg atataaaatt ttagggctag ctacccaagg 112554
   cggagacgga gcagttttaa ctgcatgtga atataacgac gtttcaaatc caatatccga 112614
   tgctacatca ccaaacaacg attcatcaaa tgcgtccaca accgcttgtt ccgttttact 112674
   acgattccac gtatctgtac taggatgttc ggttcctaaa ttacactcca tacttataga 112734
   gtaaaatctt tagtttaaat aagcgattcc ctttatcaaa acccgccgtc taatggggtt 112794
   tgtttggtag caactgatta taaactgttc ataataccac gtggtactat ttaaacagtt 112854
   tataatatgt gacatataat acacatttat aataaacaca aaccacgact gtcttttata 112914
   cgtttattta ttatacataa taccgggtaa accgttactg cgtaattata tccctatttt 112974
   cgcgtatcag ttcttgatgc agaacgggcc ctacttcgat tagttatacg cccagtcgtt 113034
   tgtgtatccg ttcgactttt tggcttacga acttgtgcgt acataggttc attacccatg 113094
   cccccctgta aatctccagt ttttcgatta tgtccacgtt tggataccga tgctccttca 113154
   cctaggtctg cttcattagc ggcttgtatt aaatttaaac cctcatgcac cgtaatacga 113214
   ataacggctc cggttaacaa acggtctaat tcggcgttat tccttggggg attactattc 113274
   caagccgctt cggcagcctt ttgtgcctgc atggcggcta cgcgccggac cgcttcacaa 113334
   aagacgcgtt tgttatatga tggcgtagga ccgcaccacg agcttgttgc ggtttttggt 113394
   gcagtgctga aggaaattgg tctcccgctg gcaattgcgc ctgctcccgg gggagctcgc 113454
   ttcggtgaat cctctaattt aggtttaacc gccgcatttt tagtaaaaga tctagatggt 113514
   ttttctacac ttagcgggtt ttcatataca gcatcttcaa ccagttcatg tctcaaacgg 113574
   gcctcccgaa aggcggccac tacttcatcg atgtcttcaa agtcatcttc cgatccgctg 113634
   gagtcgtttg gttgatgtac gcgaggggtc gtatttttat gttcaaaata aagatccgcg 113694
   tacactggag aaggagaatc ggccccaact gtggtaatgt aacccaacga gtcgtctgaa 113754
   tcatcggggg gtcctacgac cacacttcgc cgcgcggttc gatattgtcc ggaataacta 113814
   ggcgttgttt tacgacgcac tgcattagag cgacaaagtc tgtcaccgtc ggaagatgcc 113874
   attacgtaaa taaacgatag ggtcgtgaaa tatccaaaca cggcagaccg cgtattaaac 113934
   aggggccctc ttatacacgc ctgccccttt tataggcaaa cgggtttacc acgtgctgcg 113994
   taatacagaa cgagtaaata accggaaaca cgcatgataa gctaacgaaa taagggctac 114054
   acacaccccc aaaagggatg cgtagaagaa aagggtggtg atcattgatc cgtcgatata 114114
   aactccacga gccgaacagc tggcatgcca aaaattccgt tctgcaaagt ttggctcccc 114174
   actgctgtct tcacaaaaaa ataaaatttg catcgttatt aat atg aac gaa gcg 114229
   Met Asn Glu Ala
   9820
   gta att gat ccc atc ttg gaa acg gca gta aat aca ggt gat atg 114274
   Val Ile Asp Pro Ile Leu Glu Thr Ala Val Asn Thr Gly Asp Met
   9825 9830 9835
   ttt tgt agc caa act att ccg aat cgg tgt tta aaa gat aca att 114319
   Phe Cys Ser Gln Thr Ile Pro Asn Arg Cys Leu Lys Asp Thr Ile
       9840                9845                9850
   tta ata gaa gtt caa cct gaa tgt gca gat acg ctg caa tgc gtg    114364
   Leu Ile Glu Val Gln Pro Glu Cys Ala Asp Thr Leu Gln Cys Val
       9855                9860                9865
   tta gac gat aaa gta agt cga cat caa ccg ttg tta ctc cgg aac    114409
   Leu Asp Asp Lys Val Ser Arg His Gln Pro Leu Leu Leu Arg Asn
       9870                9875                9880
   cac aag aaa ctc gaa ctg cca tct gaa aaa tct gta aca cgg ggc    114454
   His Lys Lys Leu Glu Leu Pro Ser Glu Lys Ser Val Thr Arg Gly
       9885                9890                9895
   ggt ttt tat atg cag cag ttg gag ctg ttg gtt aag tcg gcg cct    114499
   Gly Phe Tyr Met Gln Gln Leu Glu Leu Leu Val Lys Ser Ala Pro
       9900                9905                9910
   ccc aat gaa tac gca ctg ttg tta att caa tgc aaa gat act gcc    114544
   Pro Asn Glu Tyr Ala Leu Leu Leu Ile Gln Cys Lys Asp Thr Ala
       9915                9920                9925
   ctt gct gat gaa gac aat ttt ttt gtc gcc aac gga gtt att gat    114589
   Leu Ala Asp Glu Asp Asn Phe Phe Val Ala Asn Gly Val Ile Asp
       9930                9935                9940
   gcg ggt tac aga gga gta att tca gcc ctt ttg tat tac cgg cca    114634
   Ala Gly Tyr Arg Gly Val Ile Ser Ala Leu Leu Tyr Tyr Arg Pro
       9945                9950                9955
   gga gta acc gtt att tta ccc gga cat tta aca atc tac ttg ttc    114679
   Gly Val Thr Val Ile Leu Pro Gly His Leu Thr Ile Tyr Leu Phe
       9960                9965                9970
   ccg gta aaa tta aga caa agt cgc ctt ctc cca aaa aac gtt ctt    114724
   Pro Val Lys Leu Arg Gln Ser Arg Leu Leu Pro Lys Asn Val Leu
       9975                9980                9985
   aaa cat ctg gat cca att ttt aaa tcg ata caa gtt caa ccc tta    114769
   Lys His Leu Asp Pro Ile Phe Lys Ser Ile Gln Val Gln Pro Leu
       9990                9995                10000
   tca aac tcg ccg tca aat tat gaa aaa ccc gtt ata cct gaa ttt 114814
   Ser Asn Ser Pro Ser Asn Tyr Glu Lys Pro Val Ile Pro Glu Phe
       10005               10010               10015
   gct gat att tcc acg gta cag cag ggg caa cct tta cat agg gat 114859
   Ala Asp Ile Ser Thr Val Gln Gln Gly Gln Pro Leu His Arg Asp
       10020               10025               10030
   tct gca gaa tac cat atc gat gtt ccc tta acc tac aaa cat atc 114904
   Ser Ala Glu Tyr His Ile Asp Val Pro Leu Thr Tyr Lys His Ile
       10035               10040               10045
   atc aat cca aaa cgc caa gaa gac gcg gga tat gat att tgt gta 114949
   Ile Asn Pro Lys Arg Gln Glu Asp Ala Gly Tyr Asp Ile Cys Val
       10050               10055               10060
   cca tat aac cta tat tta aaa agg aat gaa ttt ata aaa att gtc 114994
   Pro Tyr Asn Leu Tyr Leu Lys Arg Asn Glu Phe Ile Lys Ile Val
       10065               10070               10075
   tta ccg att ata aga gac tgg gac tta caa cat ccg agt ata aac 115039
   Leu Pro Ile Ile Arg Asp Trp Asp Leu Gln His Pro Ser Ile Asn
       10080               10085               10090
   gct tat att ttt gga aga tca tcg aaa agc cga tca ggc att atc 115084
   Ala Tyr Ile Phe Gly Arg Ser Ser Lys Ser Arg Ser Gly Ile Ile
   10095 10100 10105
   gtg tgt cca acg gca tgg cct gca gga gaa cac tgt aaa ttc tac 115129
   Val Cys Pro Thr Ala Trp Pro Ala Gly Glu His Cys Lys Phe Tyr
   10110 10115 10120
   gta tat aat ctc acg ggt gat gac ata cgt ata aaa acg gga gat 115174
   Val Tyr Asn Leu Thr Gly Asp Asp Ile Arg Ile Lys Thr Gly Asp
   10125 10130 10135
   cgt ctt gca cag gtc ctg tta ata gat cac aac acc caa ata cac 115219
   Arg Leu Ala Gln Val Leu Leu Ile Asp His Asn Thr Gln Ile His
   10140 10145 10150
   tta aaa cac aac gtt tta agt aat att gca ttt cct tat gct atc 115264
   Leu Lys His Asn Val Leu Ser Asn Ile Ala Phe Pro Tyr Ala Ile
   10155 10160 10165
   cgc ggt aaa tgt ggc ata ccg ggt gta caa tgg tat ttt act aaa 115309
   Arg Gly Lys Cys Gly Ile Pro Gly Val Gln Trp Tyr Phe Thr Lys
   10170 10175 10180
   acg tta gat cta ata gcc aca ccc agc gaa cgg gga acg cgt gga 115354
   Thr Leu Asp Leu Ile Ala Thr Pro Ser Glu Arg Gly Thr Arg Gly
   10185 10190 10195
   ttt ggt tca act gat aaa gaa aca aac gat gtc gat ttt cta cta 115399
   Phe Gly Ser Thr Asp Lys Glu Thr Asn Asp Val Asp Phe Leu Leu
   10200 10205 10210
   aaa cat taa atgtaataac cacgccagcc agcaatgttt taattttata 115448
   Lys His
   10215
   tacaaaataa aaacatacac cagaaacgtt tttagttttt atttcaatat ttatacaagc 115508
   ataacatggg atttcttgat cgcgggggtt gtgcgttgta catcttgcgt ctgttttggg 115568
   gtcaacacgg gctgaagagt ttctgtcgga tacgtttttt ttgttaggtt agatgtgtta 115628
   ttatccgata cttctataag tgggggttta atttcagata attgtgtcgc ctccgattta 115688
   ataggtgatg tttttaaacc cacattttcc cctttagcta tagataattc atggttgtgg 115748
   gaaacatcaa acgatgcctg aggtttagca acgaccccaa gagttttctc caaaagaaca 115808
   acatcagaca tgaccacttc actttcagcg gtcattctca gggtttgatc gacaatatca 115868
   tccgtagtaa catccaccgc gccaaccgat aaatacaaat gggtaattgc agctaaacac 115928
   atatcagcaa gccgttttga gttttccatg tgtgaacgga ccacggcgtt aagaccgggg 115988
   ttatcttcag taaaatggtg ctgttttaaa cattcaatgt tacgagaaca tgcagcgtaa 116048
   gttcgcgcca aagcctgggc gcggaccaaa cgacgggtat tatctgcaga agcgactacg 116108
   tcttctaacg ttagaggtgc aggcaataat ccattcacag cggttaaagc ctcttggagg 116168
   cggagcaggg cggctccttg ggggtgcgtg tttacacgca cctcttcata agatggctct 116228
   tcagttggta ttcgagcata tccacataag ctggcacaca ccgtctgcat gattgactgg 116288
   ctttccaacg tattgaact atg gat aaa tcc tcc aaa ccg acg att cgg 116337
   Met Asp Lys Ser Ser Lys Pro Thr Ile Arg
   10220 10225
   tta tta ttt gcc aca aag gga tgt gca atc tcc cac tcg ctg ttg 116382
   Leu Leu Phe Ala Thr Lys Gly Cys Ala Ile Ser His Ser Leu Leu
   10230 10235 10240
   ttg ctt acc ggg cag ata agc aca gaa cct ctg tat gtg gtg agt 116427
   Leu Leu Thr Gly Gln Ile Ser Thr Glu Pro Leu Tyr Val Val Ser
   10245 10250 10255
   tat act tgg act ccc gac tta gat gac gtc ttt gtc aaa aat ggg 116472
   Tyr Thr Trp Thr Pro Asp Leu Asp Asp Val Phe Val Lys Asn Gly
   10260 10265 10270
   agg gaa gag atc acg caa gta atc cca act aaa cgc cca cgt gaa 116517
   Arg Glu Glu Ile Thr Gln Val Ile Pro Thr Lys Arg Pro Arg Glu
   10275 10280 10285
   gta act gaa aac gat gaa gaa aac caa ata atg cat tta ttt tgt 116562
   Val Thr Glu Asn Asp Glu Glu Asn Gln Ile Met His Leu Phe Cys
   10290 10295 10300
   agt agg gac gtc aac gtt att ttt tat tta att ggt gga ttt tca 116607
   Ser Arg Asp Val Asn Val Ile Phe Tyr Leu Ile Gly Gly Phe Ser
   10305 10310 10315
   act gga gat gta cga tcc cgg gtc tgg cct ata ttt ttt tgt tgt 116652
   Thr Gly Asp Val Arg Ser Arg Val Trp Pro Ile Phe Phe Cys Cys
   10320 10325 10330
   ttt aaa acc caa act gat ttt aaa gct tta tat aag gcg tta tgg 116697
   Phe Lys Thr Gln Thr Asp Phe Lys Ala Leu Tyr Lys Ala Leu Trp
   10335 10340 10345
   tat gga gca ccc cta aat ccg cat ata ata tct gat acc cta tgt 116742
   Tyr Gly Ala Pro Leu Asn Pro His Ile Ile Ser Asp Thr Leu Cys
   10350 10355 10360
   ata tcg gag acg ttt gac att cac tcg gaa gtt ata caa act ctg 116787
   Ile Ser Glu Thr Phe Asp Ile His Ser Glu Val Ile Gln Thr Leu
   10365 10370 10375
   atg gta aca aca cac cat tta aac cga aag gga tta tcg gac aac 116832
   Met Val Thr Thr His His Leu Asn Arg Lys Gly Leu Ser Asp Asn
   10380 10385 10390
   ggc cta tgc atc aca gag gca aca ctc tgc aag tta gtt aaa aaa 116877
   Gly Leu Cys Ile Thr Glu Ala Thr Leu Cys Lys Leu Val Lys Lys
   10395 10400 10405
   tcc gtt ggt cgt cag gag cta aca tca tta tat gcc cat tac gaa 116922
   Ser Val Gly Arg Gln Glu Leu Thr Ser Leu Tyr Ala His Tyr Glu
   10410 10415 10420
   cgt caa gta ttg gct gca tat cga cga ctc tac tgg ggg tat gga 116967
   Arg Gln Val Leu Ala Ala Tyr Arg Arg Leu Tyr Trp Gly Tyr Gly
   10425 10430 10435
   tgc tcg ccg ttt tgg tat att gtt cga ttt gga ccc tct gaa aaa 117012
   Cys Ser Pro Phe Trp Tyr Ile Val Arg Phe Gly Pro Ser Glu Lys
   10440 10445 10450
   acg cta gtg ttg gct aca cgc tat tac ttg tta caa acg gac aca 117057
   Thr Leu Val Leu Ala Thr Arg Tyr Tyr Leu Leu Gln Thr Asp Thr
   10455 10460 10465
   agt tac aat acg ttg gaa acc ccc tta tat gac tta cag gca att 117102
   Ser Tyr Asn Thr Leu Glu Thr Pro Leu Tyr Asp Leu Gln Ala Ile
   10470 10475 10480
   aaa gat ttg ttt tta act tac caa gtc ccg gca tta cct aat tgt 117147
   Lys Asp Leu Phe Leu Thr Tyr Gln Val Pro Ala Leu Pro Asn Cys
   10485 10490 10495
   agt ggg tac aat att tcg gac ttg ttg tct ttt gat aaa ctt tcc 117192
   Ser Gly Tyr Asn Ile Ser Asp Leu Leu Ser Phe Asp Lys Leu Ser
   10500 10505 10510
   atg ttt tgt tgt tcc tca aca tat aca cga ggt ttg aca gcc aaa 117237
   Met Phe Cys Cys Ser Ser Thr Tyr Thr Arg Gly Leu Thr Ala Lys
   10515 10520 10525
   aat gct cta tcg tac att tta cag cga ata cat aca gac aca acg 117282
   Asn Ala Leu Ser Tyr Ile Leu Gln Arg Ile His Thr Asp Thr Thr
   10530 10535 10540
   gaa ata cac gca gta tcg gag tat att acc aac gat aga aaa ggc 117327
   Glu Ile His Ala Val Ser Glu Tyr Ile Thr Asn Asp Arg Lys Gly
   10545 10550 10555
   ctt aaa gtt cca gac cgt gaa ttt gtt gat tat att tat ctg gca 117372
   Leu Lys Val Pro Asp Arg Glu Phe Val Asp Tyr Ile Tyr Leu Ala
   10560 10565 10570
   cat ttt gaa tgt ttc aat cgg aaa cag atc gca gac cac cta caa 117417
   His Phe Glu Cys Phe Asn Arg Lys Gln Ile Ala Asp His Leu Gln
   10575 10580 10585
   gcg gtt aca tac tca gat ttt gtg aat aaa ccg gtc ctc tta aaa 117462
   Ala Val Thr Tyr Ser Asp Phe Val Asn Lys Pro Val Leu Leu Lys
   10590 10595 10600
   tca tcc aac ctg gga aaa aga gct act gct aat ttt ttt aat cat 117507
   Ser Ser Asn Leu Gly Lys Arg Ala Thr Ala Asn Phe Phe Asn His
   10605 10610 10615
   gta cgt tct cgt ctc aac atg cgt gac tat ata aaa aag aac gta 117552
   Val Arg Ser Arg Leu Asn Met Arg Asp Tyr Ile Lys Lys Asn Val
   10620 10625 10630
   att tgt gat gtc act gaa ctt gga cct gag att gga cat aaa tat 117597
   Ile Cys Asp Val Thr Glu Leu Gly Pro Glu Ile Gly His Lys Tyr
   10635 10640 10645
   aca att act aaa aca tat act tta agt ctt acg tat gcc gca aaa 117642
   Thr Ile Thr Lys Thr Tyr Thr Leu Ser Leu Thr Tyr Ala Ala Lys
   10650 10655 10660
   cct agc aag ttt ata ggc gta tgt gac cta gct aca acg cta act 117687
   Pro Ser Lys Phe Ile Gly Val Cys Asp Leu Ala Thr Thr Leu Thr
   10665 10670 10675
   cgt cgt gtg gaa aac att gaa aaa caa ttt agt cca tat gga tgg 117732
   Arg Arg Val Glu Asn Ile Glu Lys Gln Phe Ser Pro Tyr Gly Trp
   10680 10685 10690
   tcc tcc act att ccc tca aat cca ccc ggt ttt gac gaa ttg tct 117777
   Ser Ser Thr Ile Pro Ser Asn Pro Pro Gly Phe Asp Glu Leu Ser
   10695 10700 10705
   aat ttt gag gat tcg ggt gtt tcc gcg gag gcg tta cga gca gcc 117822
   Asn Phe Glu Asp Ser Gly Val Ser Ala Glu Ala Leu Arg Ala Ala
   10710 10715 10720
   aac ttt gca aac gat aca cct aac caa agt ggt cgt act ggt ttt 117867
   Asn Phe Ala Asn Asp Thr Pro Asn Gln Ser Gly Arg Thr Gly Phe
   10725 10730 10735
   gat acg agc ccg ggg att aca aaa cta tta ctg ttt ttc tct gct 117912
   Asp Thr Ser Pro Gly Ile Thr Lys Leu Leu Leu Phe Phe Ser Ala
   10740 10745 10750
   gcc act ggg ata gcc aca cat gat gta tcc atc ctg agt tat aaa 117957
   Ala Thr Gly Ile Ala Thr His Asp Val Ser Ile Leu Ser Tyr Lys
   10755 10760 10765
   act cca tta gaa gcc ctc atc ggc cat tct gag gta act gga cca 118002
   Thr Pro Leu Glu Ala Leu Ile Gly His Ser Glu Val Thr Gly Pro
   10770 10775 10780
   atg cct gta tat cgg gta gcc ttg cct cac ggc gcc caa gca ttt 118047
   Met Pro Val Tyr Arg Val Ala Leu Pro His Gly Ala Gln Ala Phe
   10785 10790 10795
   gct gtt att gct aat gat acg tgg tca tca ata aca aac cgt tac 118092
   Ala Val Ile Ala Asn Asp Thr Trp Ser Ser Ile Thr Asn Arg Tyr
   10800 10805 10810
   act tta ccg cac gag gct cga tta att gcg gag gac ctt aaa caa 118137
   Thr Leu Pro His Glu Ala Arg Leu Ile Ala Glu Asp Leu Lys Gln
   10815 10820 10825
   att aat cca tgt aat ttt gtt gcc gct tca cta cga gat atg cag 118182
   Ile Asn Pro Cys Asn Phe Val Ala Ala Ser Leu Arg Asp Met Gln
   10830 10835 10840
   ttg act tta cta tta tct acg tct gtt aaa aac gtt tct aaa att 118227
   Leu Thr Leu Leu Leu Ser Thr Ser Val Lys Asn Val Ser Lys Ile
   10845 10850 10855
   tca tca aac ata ccc aaa gat cag ctt tat ata aac agg aat gag 118272
   Ser Ser Asn Ile Pro Lys Asp Gln Leu Tyr Ile Asn Arg Asn Glu
   10860 10865 10870
   cta ttt aat aca aat ctt ata atc aca aac ctc ata ctt gat gta 118317
   Leu Phe Asn Thr Asn Leu Ile Ile Thr Asn Leu Ile Leu Asp Val
   10875 10880 10885
   gac ttt cat ata aga aaa ccc atc cca ttg ggt att tta cat gcc 118362
   Asp Phe His Ile Arg Lys Pro Ile Pro Leu Gly Ile Leu His Ala
   10890 10895 10900
   ggc atg cga gca ttt cgt cat ggt att tta acg gcc atg caa tta 118407
   Gly Met Arg Ala Phe Arg His Gly Ile Leu Thr Ala Met Gln Leu
   10905 10910 10915
   ctt ttt cca aag gcc gtg gta aac cct aac aaa gac cca tgt tat 118452
   Leu Phe Pro Lys Ala Val Val Asn Pro Asn Lys Asp Pro Cys Tyr
   10920 10925 10930
   ttt tat aaa act gca tgt cct gaa cct acc gtt gag gtg ttg gat 118497
   Phe Tyr Lys Thr Ala Cys Pro Glu Pro Thr Val Glu Val Leu Asp
   10935 10940 10945
   gat gat aat tta ttg gat ata acc agc cat tct gac atc gat ttt 118542
   Asp Asp Asn Leu Leu Asp Ile Thr Ser His Ser Asp Ile Asp Phe
   10950 10955 10960
   tac ata gaa aat ggc gaa tta tac acg tgt gta gaa gag aat tat 118587
   Tyr Ile Glu Asn Gly Glu Leu Tyr Thr Cys Val Glu Glu Asn Tyr
   10965 10970 10975
   aca gag gat gta tgg ttt ttt gat aca cag aca acg tct gaa gtc 118632
   Thr Glu Asp Val Trp Phe Phe Asp Thr Gln Thr Thr Ser Glu Val
   10980 10985 10990
   cat aca cac gcc gat gta tca aac aat gaa aac ttg cat gaa act 118677
   His Thr His Ala Asp Val Ser Asn Asn Glu Asn Leu His Glu Thr
   10995 11000 11005
   cta ccc tgt aac tgt aaa gag aaa ata ggt ttc agg gta tgc gta 118722
   Leu Pro Cys Asn Cys Lys Glu Lys Ile Gly Phe Arg Val Cys Val
   11010 11015 11020
   cca atc cca aat ccc tat gcg tta gtg ggg tct tcc act tta aag 118767
   Pro Ile Pro Asn Pro Tyr Ala Leu Val Gly Ser Ser Thr Leu Lys
   11025 11030 11035
   ggg ttt gca caa ata tta cag caa gcg gtg ttg ctg gaa cgg gaa 118812
   Gly Phe Ala Gln Ile Leu Gln Gln Ala Val Leu Leu Glu Arg Glu
   11040 11045 11050
   ttt gtt gaa tat att ggt ccg tat tta cgg gac ttt tcg ttt ata 118857
   Phe Val Glu Tyr Ile Gly Pro Tyr Leu Arg Asp Phe Ser Phe Ile
   11055 11060 11065
   gat act ggt gtt tat agc cac gga cat agt tta aga ctg cct ttt 118902
   Asp Thr Gly Val Tyr Ser His Gly His Ser Leu Arg Leu Pro Phe
   11070 11075 11080
   ttc tcc aaa gta aca acc aca ggg acg gcg gtt gga caa cta ctc 118947
   Phe Ser Lys Val Thr Thr Thr Gly Thr Ala Val Gly Gln Leu Leu
   11085 11090 11095
   cca ttt tat gtt gta cct gag cag tgt att gat ata tta gcg ttt 118992
   Pro Phe Tyr Val Val Pro Glu Gln Cys Ile Asp Ile Leu Ala Phe
   11100 11105 11110
   gtg aca tca cat aga aac ccg gca aac ttt cat ttt cat tca aga 119037
   Val Thr Ser His Arg Asn Pro Ala Asn Phe His Phe His Ser Arg
   11115 11120 11125
   ccg cag tcg aat gtt cca gtg caa ttt att tta cat aac ctt ggg 119082
   Pro Gln Ser Asn Val Pro Val Gln Phe Ile Leu His Asn Leu Gly
   11130 11135 11140
   ggg gaa tac gca gag ttt ttt gaa cgt aag gtt gcg cgt aat aaa 119127
   Gly Glu Tyr Ala Glu Phe Phe Glu Arg Lys Val Ala Arg Asn Lys
   11145 11150 11155
   caa ata ttt agc tcc ccg caa ata tct tta aca aag gct cta aaa 119172
   Gln Ile Phe Ser Ser Pro Gln Ile Ser Leu Thr Lys Ala Leu Lys
   11160 11165 11170
   gag cgc ggg gta act tgt ctg gac gca ttt aca ctg gag gcc ttt 119217
   Glu Arg Gly Val Thr Cys Leu Asp Ala Phe Thr Leu Glu Ala Phe
   11175 11180 11185
   gtc gac agc aca ata tta gaa tct att gtg gag cat att gct gtt 119262
   Val Asp Ser Thr Ile Leu Glu Ser Ile Val Glu His Ile Ala Val
   11190 11195 11200
   cat ttc ccc ggg cgt gat cgc gaa tat acc tta aca tca tca aag 119307
   His Phe Pro Gly Arg Asp Arg Glu Tyr Thr Leu Thr Ser Ser Lys
   11205 11210 11215
   tgt atc gcc atc aaa agg gac tgg gtg tta ttt cag ctc ata tgc 119352
   Cys Ile Ala Ile Lys Arg Asp Trp Val Leu Phe Gln Leu Ile Cys
   11220 11225 11230
   gga aca aaa ggg ttc act tgt ctt cga tat ccc cat cgc gga gga 119397
   Gly Thr Lys Gly Phe Thr Cys Leu Arg Tyr Pro His Arg Gly Gly
   11235 11240 11245
   aga acg gct ccc cgg aca ttt gtg tct ctg cga gtg gat cat cac 119442
   Arg Thr Ala Pro Arg   Thr Phe Val Ser Leu   Arg Val Asp His His
                   11250                   11255                   11260
   aac cgt ttg tgt att   tcg ctt gca caa caa   tgt ttt gct aca aag   119487
   Asn Arg Leu Cys Ile   Ser Leu Ala Gln Gln   Cys Phe Ala Thr Lys
                   11265                   11270                   11275
   tgc gat agc aat cgc   atg cat aca atc ttt   act cta gaa gta cct   119532
   Cys Asp Ser Asn Arg   Met His Thr Ile Phe   Thr Leu Glu Val Pro
                   11280                   11285                   11290
   aat tat cca aat tta   act tcg agt taa caccaaccgt gtgatactac       119579
   Asn Tyr Pro Asn Leu   Thr Ser Ser
                   11295
   atcgtgcttg aattgccatc ttccacgggt c atg cag   gct tta gga atc       119628
                                      Met Gln   Ala Leu Gly Ile
                                          11300
   aag   aca gaa cat ttt ata   att atg tgt cta ctt   agc gga cat gct 119673
   Lys   Thr Glu His Phe Ile   Ile Met Cys Leu Leu   Ser Gly His Ala
   11305                 11310                 11315
   gtt   ttt acc cta tgg tat   acc gct cgt gta aag   ttt gaa cat gag 119718
   Val   Phe Thr Leu Trp Tyr   Thr Ala Arg Val Lys   Phe Glu His Glu
   11320                 11325                 11330
   tgt   gtg tat gca acc acg   gtg att aat ggt gga   ccg gtt gta tgg 119763
   Cys   Val Tyr Ala Thr Thr   Val Ile Asn Gly Gly   Pro Val Val Trp
   11335                 11340                 11345
   ggg   tct tat aac aac tct   ctt ata tat gta acg   ttt gta aac cac 119808
   Gly   Ser Tyr Asn Asn Ser   Leu Ile Tyr Val Thr   Phe Val Asn His
   11350                 11355                 11360
   tca   acg ttt ttg gat ggc   cta tct gga tac gat   tac agc tgc cgg 119853
   Ser   Thr Phe Leu Asp Gly   Leu Ser Gly Tyr Asp   Tyr Ser Cys Arg
   11365                 11370                 11375
   gaa   aat cta tta tca gga   gat act atg gta aaa   acc gct att tct 119898
   Glu   Asn Leu Leu Ser Gly   Asp Thr Met Val Lys   Thr Ala Ile Ser
   11380                 11385                 11390
   aca   cct ttg cat gac aaa   att cga att gtt ctg   gga aca cgt aat 119943
   Thr   Pro Leu His Asp Lys   Ile Arg Ile Val Leu   Gly Thr Arg Asn
   11395                 11400                 11405
   tgt   cac gct tat ttt tgg   tgc gtg cag cta aaa   atg att ttt ttt 119988
   Cys   His Ala Tyr Phe Trp   Cys Val Gln Leu Lys   Met Ile Phe Phe
   11410                 11415                 11420
   gca   tgg ttt gta tat ggt   atg tat tta caa ttt   cga cga ata cgt 120033
   Ala   Trp Phe Val Tyr Gly   Met Tyr Leu Gln Phe   Arg Arg Ile Arg
   11425                 11430                 11435
   cgt   atg ttt ggg cca ttc   cga tca tcc tgt gag   tta ata tcc ccc 120078
   Arg   Met Phe Gly Pro Phe   Arg Ser Ser Cys Glu   Leu Ile Ser Pro
   11440                 11445                 11450
   aca   tca tat tca ctg aat   tac gta aca cgg gtt   att tcg aac att 120123
   Thr   Ser Tyr Ser Leu Asn   Tyr Val Thr Arg Val   Ile Ser Asn Ile
   11455                 11460                 11465
   ctt   ctt ggt tac cca tat   aca aag ttg gca agg   ttg tta tgt gat 120168
   Leu   Leu Gly Tyr Pro Tyr   Thr Lys Leu Ala Arg   Leu Leu Cys Asp
   11470                 11475                 11480
   gtt   tcc atg cga cgg gat   ggt atg agt aaa gta   ttt aat gct gac 120213
   Val Ser Met Arg Arg Asp Gly Met Ser Lys Val Phe Asn Ala Asp
   11485 11490 11495
   cct ata agt ttt tta tat atg cat aaa ggt gtt acg tta ttg atg 120258
   Pro Ile Ser Phe Leu Tyr Met His Lys Gly Val Thr Leu Leu Met
   11500 11505 11510
   ctt ttg gag gtt atc gct cat ata tca tct gga tgt att gtg ctt 120303
   Leu Leu Glu Val Ile Ala His Ile Ser Ser Gly Cys Ile Val Leu
   11515 11520 11525
   tta acg ctt ggc gtt gca tat aca cca tgc gcg tta tta tac ccc 120348
   Leu Thr Leu Gly Val Ala Tyr Thr Pro Cys Ala Leu Leu Tyr Pro
   11530 11535 11540
   aca tac att cgg att ctg gcc tgg gtt gtt gta tgc acg ctc gct 120393
   Thr Tyr Ile Arg Ile Leu Ala Trp Val Val Val Cys Thr Leu Ala
   11545 11550 11555
   ata gta gag ctt ata tct tat gtt aga cca aaa cca acc aag gat 120438
   Ile Val Glu Leu Ile Ser Tyr Val Arg Pro Lys Pro Thr Lys Asp
   11560 11565 11570
   aat cat tta aat cat atc aat acg ggg gga ata cgt ggt ata tgc 120483
   Asn His Leu Asn His Ile Asn Thr Gly Gly Ile Arg Gly Ile Cys
   11575 11580 11585
   aca aca tgt tgc gct aca gta atg tcc ggc ctt gct ata aaa tgt 120528
   Thr Thr Cys Cys Ala Thr Val Met Ser Gly Leu Ala Ile Lys Cys
   11590 11595 11600
   ttt tat atc gtc ata ttt gct ata gca gtg gtt att ttt atg cat 120573
   Phe Tyr Ile Val Ile Phe Ala Ile Ala Val Val Ile Phe Met His
   11605 11610 11615
   tac gaa caa agg gtg cag gta agc ttg ttt ggg gaa agt gaa aac 120618
   Tyr Glu Gln Arg Val Gln Val Ser Leu Phe Gly Glu Ser Glu Asn
   11620 11625 11630
   tcc cag aag cat taa tcatgtgact aaacacgccc attgcggggt tgggtgagcc 120673
   Ser Gln Lys His
   11635
   tataaattct acaacattgg cggaagatac aggcaactgc aaacacgcaa ttgtcagata 120733
   ttttgcagcc atg gcc tct gct tca att cca acc gac cca gac gtg 120779
   Met Ala Ser Ala Ser Ile Pro Thr Asp Pro Asp Val
   11640 11645 11650
   tct act att tgt gaa gac ttt atg aat ttg cta cca gac gaa cct 120824
   Ser Thr Ile Cys Glu Asp Phe Met Asn Leu Leu Pro Asp Glu Pro
   11655 11660 11665
   tcg gat gac ttt gca ttg gaa gtc acc gat tgg gca aat gat gaa 120869
   Ser Asp Asp Phe Ala Leu Glu Val Thr Asp Trp Ala Asn Asp Glu
   11670 11675 11680
   gct att ggc tcc act cca ggc gag gac tcc aca acg tct aga act 120914
   Ala Ile Gly Ser Thr Pro Gly Glu Asp Ser Thr Thr Ser Arg Thr
   11685 11690 11695
   gtg tat gtg gag cgt act gca gat aca gca tat aat cca cgg tat 120959
   Val Tyr Val Glu Arg Thr Ala Asp Thr Ala Tyr Asn Pro Arg Tyr
   11700 11705 11710
   tcc aaa cga agg cac gga agg cgt gaa agc tac cac cac aat cgc 121004
   Ser Lys Arg Arg His Gly Arg Arg Glu Ser Tyr His His Asn Arg
   11715 11720 11725
   ccg aaa act ttg gtt gtt gta tta ccc gat tca aac cat cat gga 121049
   Pro Lys Thr Leu Val Val Val Leu Pro Asp Ser Asn His His Gly
   11730 11735 11740
   gga aga gac gtg gag act gga tat gca cgc atc gaa cgg gga cat 121094
   Gly Arg Asp Val Glu Thr Gly Tyr Ala Arg Ile Glu Arg Gly His
   11745 11750 11755
   cga cga tca tcc aga tct tat aac act caa agt tca aga aaa cac 121139
   Arg Arg Ser Ser Arg Ser Tyr Asn Thr Gln Ser Ser Arg Lys His
   11760 11765 11770
   cgt gat cga tcc ctg tca aat cga aga cgg cgt cct aca acg cct 121184
   Arg Asp Arg Ser Leu Ser Asn Arg Arg Arg Arg Pro Thr Thr Pro
   11775 11780 11785
   cct gca atg acc acg gga gaa aga aat gat cag aca cat gac gaa 121229
   Pro Ala Met Thr Thr Gly Glu Arg Asn Asp Gln Thr His Asp Glu
   11790 11795 11800
   tcg tac agg ttg cga ttt tcc aag aga gac gcc cgc cga gag cgt 121274
   Ser Tyr Arg Leu Arg Phe Ser Lys Arg Asp Ala Arg Arg Glu Arg
   11805 11810 11815
   att cga aaa gag tat gat atc ccg gtc gat cga att acg ggc cgt 121319
   Ile Arg Lys Glu Tyr Asp Ile Pro Val Asp Arg Ile Thr Gly Arg
   11820 11825 11830
   gct att gaa gtc gtc tcc acc gcg gga gcc agc gtg acc att gac 121364
   Ala Ile Glu Val Val Ser Thr Ala Gly Ala Ser Val Thr Ile Asp
   11835 11840 11845
   tcg gta cgc cat tta gat gaa aca att gaa aaa ctg gta gtc cga 121409
   Ser Val Arg His Leu Asp Glu Thr Ile Glu Lys Leu Val Val Arg
   11850 11855 11860
   tat gcc aca ata caa gag ggt gat tca tgg gct tcc ggt gga tgt 121454
   Tyr Ala Thr Ile Gln Glu Gly Asp Ser Trp Ala Ser Gly Gly Cys
   11865 11870 11875
   ttt ccg ggg ata aaa caa aac aca tct tgg ccg gag ttg atg ttg 121499
   Phe Pro Gly Ile Lys Gln Asn Thr Ser Trp Pro Glu Leu Met Leu
   11880 11885 11890
   tac gga cat gaa ctt tat cgt acc ttt gag tca tat aaa atg gac 121544
   Tyr Gly His Glu Leu Tyr Arg Thr Phe Glu Ser Tyr Lys Met Asp
   11895 11900 11905
   tca cgt att gcc cgc gcg ttg cgt gag aga gtc ata cgt gga gaa 121589
   Ser Arg Ile Ala Arg Ala Leu Arg Glu Arg Val Ile Arg Gly Glu
   11910 11915 11920
   tct ttg att gaa gcg ttg gag tct gcg gat gaa ctg tta acg tgg 121634
   Ser Leu Ile Glu Ala Leu Glu Ser Ala Asp Glu Leu Leu Thr Trp
   11925 11930 11935
   att aaa atg tta gcg gca aaa aac ttg ccc atc tac aca aat aat 121679
   Ile Lys Met Leu Ala Ala Lys Asn Leu Pro Ile Tyr Thr Asn Asn
   11940 11945 11950
   ccc att gtt gca acc tcg aag tca ctt ttg gag aat tta aag tta 121724
   Pro Ile Val Ala Thr Ser Lys Ser Leu Leu Glu Asn Leu Lys Leu
   11955 11960 11965
   aag ctg ggg cct ttt gta aga tgt ctt ctt cta aac agg gac aac 121769
   Lys Leu Gly Pro Phe Val Arg Cys Leu Leu Leu Asn Arg Asp Asn
   11970 11975 11980
   gat ttg ggg tct cgt act ctc ccc gaa ctg ttg cgc cag caa cgt 121814
   Asp Leu Gly Ser Arg Thr Leu Pro Glu Leu Leu Arg Gln Gln Arg
   11985 11990 11995
   ttt agt gat atc acg tgt att act act tat atg ttt gtt atg att 121859
   Phe Ser Asp Ile Thr Cys Ile Thr Thr Tyr Met Phe Val Met Ile
   12000 12005 12010
   gcc cgc att gct aat ata gtt gtc cgt ggc tct aaa ttt gtg gaa 121904
   Ala Arg Ile Ala Asn Ile Val Val Arg Gly Ser Lys Phe Val Glu
   12015 12020 12025
   tat gat gat atc agt tgt aac gtt caa gtg tta caa gaa tat aca 121949
   Tyr Asp Asp Ile Ser Cys Asn Val Gln Val Leu Gln Glu Tyr Thr
   12030 12035 12040
   ccc ggg tca tgt ctg gcc ggt gtt tta gag gcc cta atc acc cac 121994
   Pro Gly Ser Cys Leu Ala Gly Val Leu Glu Ala Leu Ile Thr His
   12045 12050 12055
   caa cgc gag tgt ggt cgt gtt gaa tgt acc ctc tca act tgg gcc 122039
   Gln Arg Glu Cys Gly Arg Val Glu Cys Thr Leu Ser Thr Trp Ala
   12060 12065 12070
   ggg cat ctt tct gac gcc cgt cca tac ggt aaa tat ttt aag tgt 122084
   Gly His Leu Ser Asp Ala Arg Pro Tyr Gly Lys Tyr Phe Lys Cys
   12075 12080 12085
   agt acc ttt aac tgc taa aataaaaaat acctttttca tgcttgtcaa 122132
   Ser Thr Phe Asn Cys
   12090
   aacatactaa tttgtatttt taatcattaa gcatatttac ctgttagaat agtaaatacc 122192
   tatttcatgc ttgtaaaaca tatcaattta tatttttaat cattatacac caccccttga 122252
   ttttatcaat tgtttatgtg attctatatt aataaaagac taataaaaat gtaatattgt 122312
   gggagttttt aaggcgacgt tggggatata tgggcgggat tacattgctt tcaaaccaat 122372
   atctttgcaa ttccgtctct gattcgggta aaacacacac cagacgtgta ccgaacgttt 122432
   aatta atg gat aca acg gga gct tcc gaa agc agt caa ccc atc cga 122479
   Met Asp Thr Thr Gly Ala Ser Glu Ser Ser Gln Pro Ile Arg
   12095 12100
   gtg aat ctt aaa cct gac ccg ttg gcg tcg ttt aca caa gtt ata 122524
   Val Asn Leu Lys Pro Asp Pro Leu Ala Ser Phe Thr Gln Val Ile
   12105 12110 12115
   ccg cca ctg gcg ttg gaa aca acg tgg aca tgc cct gcc aac tca 122569
   Pro Pro Leu Ala Leu Glu Thr Thr Trp Thr Cys Pro Ala Asn Ser
   12120 12125 12130
   cat gca ccg acg cca tcc cct ctg tac ggt gtt aag agg tta tgt 122614
   His Ala Pro Thr Pro Ser Pro Leu Tyr Gly Val Lys Arg Leu Cys
   12135 12140 12145
   gct ctt cga gca aca tgc ggc cgg gct gat gat tta cac gct ttt 122659
   Ala Leu Arg Ala Thr Cys Gly Arg Ala Asp Asp Leu His Ala Phe
   12150 12155 12160
   ttg att gga ctt gga cgt cga gat aaa cca tct gaa tcc cca atg 122704
   Leu Ile Gly Leu Gly Arg Arg Asp Lys Pro Ser Glu Ser Pro Met
   12165 12170 12175
   tat gtt gac cta cag ccg ttt tgc agc ctc cta aat tcc caa cga 122749
   Tyr Val Asp Leu Gln Pro Phe Cys Ser Leu Leu Asn Ser Gln Arg
   12180 12185 12190
   ctg tta ccg gaa atg gct aat tat aac acc cta tgc gat gca ccc 122794
   Leu Leu Pro Glu Met Ala Asn Tyr Asn Thr Leu Cys Asp Ala Pro
   12195 12200 12205
   ttc agc gcc gca act cag cag atg atg ctg gag tcc gga cag ctg 122839
   Phe Ser Ala Ala Thr Gln Gln Met Met Leu Glu Ser Gly Gln Leu
   12210 12215 12220
   ggt gta cat ctg gcg gct att ggg tat cac tgt cat tgt aaa tcc 122884
   Gly Val His Leu Ala Ala Ile Gly Tyr His Cys His Cys Lys Ser
   12225 12230 12235
   ccc ttc tcg gcg gag tgt tgg acc ggt gca tcc gag gca tac gat 122929
   Pro Phe Ser Ala Glu Cys Trp Thr Gly Ala Ser Glu Ala Tyr Asp
   12240 12245 12250
   cat gtt gta tgt ggg gga aaa gcc cga gcg gct gtc ggc gga cta 122974
   His Val Val Cys Gly Gly Lys Ala Arg Ala Ala Val Gly Gly Leu
   12255 12260 12265
   tga actacacatt taaataaaaa tacgtacaat cgaaaaaagg tgtattttat 123027
   ttagtgatta catcaatacg ccctccgtag gttcggcaaa tctaacccgg tgacagaccg 123087
   gctgttgacg gggggatcct ttcaatattc cacgaatgtt ggaaactgtt ggtcttcctt 123147
   gggctgttag ggttgggaga gtgggtcggt ctgacgtggt aagtgcggct tcaccagagt 123207
   cttctgtcaa gttgcatgtt aacgtcgacg ttatacaagg tgtttcagtg gtattttgcg 123267
   cgatattctc ccaaaacata agacgttgca tatgcatatc ataaatatta ccccgccata 123327
   aacagagatc tctgttactt aaaccgtgtg tgcggagaaa tgtacccata gatggagcca 123387
   gtaaatgcaa gcccgtacca cattcatacc caagcgttgt ggatcgcttt tccttcatcg 123447
   ccaaaataat aaatgtcctt gcacctccag gcgtcattgc acgccctatt agacgggcaa 123507
   ggctaagacg ttcgccgggt ttgctatatt tgccaataat tacataggtt ttggtacagg 123567
   taatgtgtaa cgttcctccg cctggcaggt ctacagttct ccccgcaagc actcgtaata 123627
   aacatcgcgt ttctgacgcg ggattttcct cggcggtggc gtttaaactg ggacgcacca 123687
   gagtggagcc cataataaat gcgggaacat gcccatatgc aagtgtctca gaaattacat 123747
   gcattttatg attttacggg ggtgggttac gataatagct attaaacaaa cacccaataa 123807
   agcatttttt gtagaacctt tattgggtaa cacagtcttt tcacgtgaca ggcaatgtat 123867
   aaataacgta cggatgcact taagatgtat cgcacataaa tttatataag ctgtagcaaa 123927
   gtataagcaa atcctgttaa tattatattt ttgggatccg ca atg tcc agg gta 123981
   Met Ser Arg Val
   12270
   tcg gag tat ggg gta ccg gaa ggt gtt cgg gaa tct gat agc gat 124026
   Ser Glu Tyr Gly Val Pro Glu Gly Val Arg Glu Ser Asp Ser Asp
   12275 12280 12285
   aca gac tct gtg ttt atg tat cag cat aca gag ctt atg cag aac 124071
   Thr Asp Ser Val Phe Met Tyr Gln His Thr Glu Leu Met Gln Asn
   12290 12295 12300
   aac gcg tcg cca ctc gtc gtt caa aca aga cca ccg gcg gtt ctt 124116
   Asn Ala Ser Pro Leu Val Val Gln Thr Arg Pro Pro Ala Val Leu
   12305 12310 12315
   att cca ctg gtt gat gtc cca agg cca cga tcc cgg aga aag gcg 124161
   Ile Pro Leu Val Asp Val Pro Arg Pro Arg Ser Arg Arg Lys Ala
   12320 12325 12330
   tcc gcg caa ctg aaa atg caa atg gac agg tta tgc aac gta ctg 124206
   Ser Ala Gln Leu Lys Met Gln Met Asp Arg Leu Cys Asn Val Leu
   12335 12340 12345
   ggt gta gta ctc cag atg gcg acg ttg gct ttg gtg aca tat ata 124251
   Gly Val Val Leu Gln Met Ala Thr Leu Ala Leu Val Thr Tyr Ile
   12350 12355 12360
   gct ttt gtt gtg cat aca cgc gcg aca agc tgc aag cga gaa taa 124296
   Ala Phe Val Val His Thr Arg Ala Thr Ser Cys Lys Arg Glu
   12365 12370 12375
   ataccttccc cttccggaca gtagtttcat gtagttgagt tgggaggttc ctcgggaaaa 124356
   acggcaacaa tggcgaccac gacagcagta agggtgagaa taccaaaaag aattaaaaaa 124416
   gccggtacac agcatttgcg aattcttaga aaagccagct gaagtctgtg gattccatca 124476
   taagcgcagt ctaggcaatc gtaagactgt tgggtggtgc cattctccac gtgaggaaaa 124536
   agaggcgggg agaacaccag actctcgcgg cttctgtaag gggggggcgc gtctgccacg 124596
   gcctcggcgt atgtgggtag gtaggggatg ggggtcgcaa cgtcatccat gctgggggac 124656
   gacgtgaggg tgaccggcgg ggtcccaggt cggcgggagt agtgcacggt cgccatccga 124716
   gcagtaaacg aggggtggac gcaaaaggcg cgggttttgt taaaggctgg cggggggggg 124776
   tttcccggca aaaaatccca tcccccccga tggtcgcccc gcaaacgcgc ggggaggtgg 124836
   ggtcgctttt ttttttctct ctcgaggggg ccgcgagagg gctggcct 124884

<210> 52
<211> 278
<212> PRT
<213> Varicella zoster

<400> 52

Met Phe Cys Thr Ser Pro Ala Thr Arg Gly Asp Ser Ser Glu Ser Lys
1               5                   10                  15

Pro Gly Ala Ser Val Asp Val Asn Gly Lys Met Glu Tyr Gly Ser Ala
            20                  25                  30

Pro Gly Pro Leu Asn Gly Arg Asp Thr Ser Arg Gly Pro Gly Ala Phe
        35                  40                  45

Cys Thr Pro Gly Trp Glu Ile His Pro Ala Arg Leu Val Glu Asp Ile
    50                  55                  60

Asn Arg Val Phe Leu Cys Ile Ala Gln Ser Ser Gly Arg Val Thr Arg
65                  70                  75                  80

Asp Ser Arg Arg Leu Arg Arg Ile Cys Leu Asp Phe Tyr Leu Met Gly

85                  90                  95

Arg Thr Arg Gln Arg Pro Thr Leu Ala Cys Trp Glu Glu Leu Leu Gln
            100                 105                 110

Leu Gln Pro Thr Gln Thr Gln Cys Leu Arg Ala Thr Leu Met Glu Val
        115                 120                 125

Ser His Arg Pro Pro Arg Gly Glu Asp Gly Phe Ile Glu Ala Pro Asn
    130                 135                 140

Val Pro Leu His Arg Ser Ala Leu Glu Cys Asp Val Ser Asp Asp Gly
145                 150                 155                 160

Gly Glu Asp Asp Ser Asp Asp Asp Gly Ser Thr Pro Ser Asp Val Ile
                165                 170                 175

Glu Phe Arg Asp Ser Asp Ala Glu Ser Ser Asp Gly Glu Asp Phe Ile
            180                 185                 190

Val Glu Glu Glu Ser Glu Glu Ser Thr Asp Ser Cys Glu Pro Asp Gly
        195                 200                 205

Val Pro Gly Asp Cys Tyr Arg Asp Gly Asp Gly Cys Asn Thr Pro Ser
    210                 215                 220

Pro Lys Arg Pro Gln Arg Ala Ile Glu Arg Tyr Ala Gly Ala Glu Thr
225                 230                 235                 240

Ala Glu Tyr Thr Ala Ala Lys Ala Leu Thr Ala Leu Gly Glu Gly Gly
                245                 250                 255

Val Asp Trp Lys Arg Arg Arg His Glu Ala Pro Arg Arg His Asp Ile
            260                 265                 270

Pro Pro Pro His Gly Val
        275

<210>  53
<211>  180
<212>  PRT
<213>  Varicella zoster

<400>  53

Met Asn Leu Cys Gly Ser Arg Gly Glu His Pro Gly Gly Glu Tyr Ala
1               5                   10                  15

Gly Leu Tyr Cys Thr Arg His Asp Thr Pro Ala His Gln Ala Leu Met
            20                  25                  30

Asn Asp Ala Glu Arg Tyr Phe Ala Ala Ala Leu Cys Ala Ile Ser Thr
        35                  40                  45

Glu Ala Tyr Glu Ala Phe Ile His Ser Pro Ser Glu Arg Pro Cys Ala
    50                  55                  60

Ser Leu Trp Gly Arg Ala Lys Asp Ala Phe Gly Arg Met Cys Gly Glu
65                  70                  75                  80

Leu Ala Ala Asp Arg Gln Arg Pro Pro Ser Val Pro Pro Ile Arg Arg
                85                  90                  95

Ala Val Leu Ser Leu Leu Arg Glu Gln Cys Met Pro Asp Pro Gln Ser
            100                 105                 110

His Leu Glu Leu Ser Glu Arg Leu Ile Leu Met Ala Tyr Trp Cys Cys
        115                 120                 125

Leu Gly His Ala Gly Leu Pro Thr Ile Gly Leu Ser Pro Asp Asn Lys
    130                 135                 140

Cys Ile Arg Ala Glu Leu Tyr Asp Arg Pro Gly Gly Ile Cys His Arg
145                 150                 155                 160

Leu Phe Asp Ala Tyr Leu Gly Cys Gly Ser Leu Gly Val Pro Arg Thr
                165                 170                 175

Tyr Glu Arg Ser
            180

<210>  54
<211>  102
<212>  PRT
<213>  Varicella zoster

<400>  54

Met Ala Gly Gln Asn Thr Met Glu Gly Glu Ala Val Ala Leu Leu Met
1               5                   10                  15

Glu Ala Val Val Thr Pro Arg Ala Gln Pro Asn Asn Thr Thr Ile Thr
            20                  25                  30

Ala Ile Gln Pro Ser Arg Ser Ala Glu Lys Cys Tyr Tyr Ser Asp Ser
        35                  40                  45

Glu Asn Glu Thr Ala Asp Glu Phe Leu Arg Arg Ile Gly Lys Tyr Gln
    50                  55                  60

His Lys Ile Tyr His Arg Lys Lys Phe Cys Tyr Ile Thr Leu Ile Ile
65                  70                  75                  80

Val Phe Val Phe Ala Met Thr Gly Ala Ala Phe Ala Leu Gly Tyr Ile
                85                  90                  95

Thr Ser Gln Phe Val Gly
            100

<210>  55
<211>  1310
<212>  PRT
<213>  Varicella zoster

<400>  55

Met Asp Thr Pro Pro Met Gln Arg Ser Thr Pro Gln Arg Ala Gly Ser
1               5                   10                  15

Pro Asp Thr Leu Glu Leu Met Asp Leu Leu Asp Ala Ala Ala Ala Ala
            20                  25                  30

Ala Glu His Arg Ala Arg Val Val Thr Ser Ser Gln Pro Asp Asp Leu
        35                  40                  45

Leu Phe Gly Glu Asn Gly Val Met Val Gly Arg Glu His Glu Ile Val
    50                  55                  60

Ser Ile Pro Ser Val Ser Gly Leu Gln Pro Glu Pro Arg Thr Glu Asp
65                  70                  75                  80

Val Gly Glu Glu Leu Thr Gln Asp Asp Tyr Val Cys Glu Asp Gly Gln
                85                  90                  95

Asp Leu Met Gly Ser Pro Val Ile Pro Leu Ala Glu Val Phe His Thr
            100                 105                 110

Arg Phe Ser Glu Ala Gly Ala Arg Glu Pro Thr Gly Ala Asp Arg Ser
        115                 120                 125

Leu Glu Thr Val Ser Leu Gly Thr Lys Leu Ala Arg Ser Pro Lys Pro
    130                 135                 140

Pro Met Asn Asp Gly Glu Thr Gly Arg Gly Thr Thr Pro Pro Phe Pro
145                 150                 155                 160

Gln Ala Phe Ser Pro Val Ser Pro Ala Ser Pro Val Gly Asp Ala Ala
                165                 170                 175

Gly Asn Asp Gln Arg Glu Asp Gln Arg Ser Ile Pro Arg Gln Thr Thr
            180                 185                 190

Arg Gly Asn Ser Pro Gly Leu Pro Ser Val Val His Arg Asp Arg Gln
        195                 200                 205

Thr Gln Ser Ile Ser Gly Lys Lys Pro Gly Asp Glu Gln Ala Gly His
    210                 215                 220

Ala His Ala Ser Gly Asp Gly Val Val Leu Gln Lys Thr Gln Arg Pro
225                 230                 235                 240

Ala Gln Gly Lys Ser Pro Lys Lys Lys Thr Leu Lys Val Lys Val Pro
                245                 250                 255

Leu Pro Ala Arg Lys Pro Gly Gly Pro Val Pro Gly Pro Val Glu Gln
            260                 265                 270

Leu Tyr His Val Leu Ser Asp Ser Val Pro Ala Lys Gly Ala Lys Ala
        275                 280                 285

Asp Leu Pro Phe Glu Thr Asp Asp Thr Arg Pro Arg Lys His Asp Ala
    290                 295                 300

Arg Gly Ile Thr Pro Arg Val Pro Gly Arg Ser Ser Gly Gly Lys Pro
305                 310                 315                 320

Arg Ala Phe Leu Ala Leu Pro Gly Arg Ser His Ala Pro Asp Pro Ile
                325                 330                 335

Glu Asp Asp Ser Pro Val Glu Lys Lys Pro Lys Ser Arg Glu Phe Val
            340                 345                 350

Ser Ser Ser Ser Ser Ser Ser Ser Trp Gly Ser Ser Ser Glu Asp Glu
        355                 360                 365

Asp Asp Glu Pro Arg Arg Val Ser Val Gly Ser Glu Thr Thr Gly Ser
    370                 375                 380

Arg Ser Gly Arg Glu His Ala Pro Ser Pro Ser Asn Ser Asp Asp Ser
385                 390                 395                 400

Asp Ser Asn Asp Gly Gly Ser Thr Lys Gln Asn Ile Gln Pro Gly Tyr
                405                 410                 415

Arg Ser Ile Ser Gly Pro Asp Pro Arg Ile Arg Lys Thr Lys Arg Leu
            420                 425                 430

Ala Gly Glu Pro Gly Arg Gln Arg Gln Lys Ser Phe Ser Leu Pro Arg
        435                 440                 445

Ser Arg Thr Pro Ile Ile Pro Pro Val Ser Gly Pro Leu Met Met Pro
    450                 455                 460

Asp Gly Ser Pro Trp Pro Gly Ser Ala Pro Leu Pro Ser Asn Arg Val
465                 470                 475                 480

Arg Phe Gly Pro Ser Gly Glu Thr Arg Glu Gly His Trp Glu Asp Glu
                485                 490                 495

Ala Val Arg Ala Ala Arg Ala Arg Tyr Glu Ala Ser Thr Glu Pro Val
            500                 505                 510

Pro Leu Tyr Val Pro Glu Leu Gly Asp Pro Ala Arg Gln Tyr Arg Ala
        515                 520                 525

Leu Ile Asn Leu Ile Tyr Cys Pro Asp Arg Asp Pro Ile Ala Trp Leu
    530                 535                 540

Gln Asn Pro Lys Leu Thr Gly Val Asn Ser Ala Leu Asn Gln Phe Tyr
545                 550                 555                 560

Gln Lys Leu Leu Pro Pro Gly Arg Ala Gly Thr Ala Val Thr Gly Ser
                565                 570                 575

Val Ala Ser Pro Val Pro His Val Gly Glu Ala Met Ala Thr Gly Glu
            580                 585                 590

Ala Leu Trp Ala Leu Pro His Ala Ala Ala Ala Val Ala Met Ser Arg
        595                 600                 605

Arg Tyr Asp Arg Ala Gln Lys His Phe Ile Leu Gln Ser Leu Arg Arg
    610                 615                 620

Ala Phe Ala Ser Met Ala Tyr Pro Glu Ala Thr Gly Ser Ser Pro Ala
625                 630                 635                 640

Ala Arg Ile Ser Arg Gly His Pro Ser Pro Thr Thr Pro Ala Thr Gln
                645                 650                 655

Ala Pro Asp Pro Gln Pro Ser Ala Ala Ala Arg Ser Leu Ser Val Cys
            660                 665                 670

Pro Pro Asp Asp Arg Leu Arg Thr Pro Arg Lys Arg Lys Ser Gln Pro
        675                 680                 685

Val Glu Ser Arg Ser Leu Leu Asp Lys Ile Arg Glu Thr Pro Val Ala
    690                 695                 700

Asp Ala Arg Val Ala Asp Asp His Val Val Ser Lys Ala Lys Arg Arg
705                 710                 715                 720

Val Ser Glu Pro Val Thr Ile Thr Ser Gly Pro Val Val Asp Pro Pro
                725                 730                 735

Ala Val Ile Thr Met Pro Leu Asp Gly Pro Ala Pro Asn Gly Gly Phe
            740                 745                 750

Arg Arg Ile Pro Arg Gly Ala Leu His Thr Pro Val Pro Ser Asp Gln
        755                 760                 765

Ala Arg Lys Ala Tyr Cys Thr Pro Glu Thr Ile Ala Arg Leu Val Asp
    770                 775                 780

Asp Pro Leu Phe Pro Thr Ala Trp Arg Pro Ala Leu Ser Phe Asp Pro
785                 790                 795                 800

Gly Ala Leu Ala Glu Ile Ala Ala Arg Arg Pro Gly Gly Gly Asp Arg
                805                 810                 815

Arg Phe Gly Pro Pro Ser Gly Val Glu Ala Leu Arg Arg Arg Cys Ala
            820                 825                 830

Trp Met Arg Gln Ile Pro Asp Pro Glu Asp Val Arg Leu Leu Ile Ile
        835                 840                 845

Tyr Asp Pro Leu Pro Gly Glu Asp Ile Asn Gly Pro Leu Glu Ser Thr
    850                 855                 860

Leu Ala Thr Asp Pro Gly Pro Ser Trp Ser Pro Ser Arg Gly Gly Leu
865                 870                 875                 880

Ser Val Val Leu Ala Ala Leu Ser Asn Arg Leu Cys Leu Pro Ser Thr
                885                 890                 895

His Ala Trp Ala Gly Asn Trp Thr Gly Pro Pro Asp Val Ser Ala Leu
            900                 905                 910

Asn Ala Arg Gly Val Leu Leu Leu Ser Thr Arg Asp Leu Ala Phe Ala
        915                 920                 925

Gly Ala Val Glu Tyr Leu Gly Ser Arg Leu Ala Ser Ala Arg Arg Arg
    930                 935                 940

Leu Leu Val Leu Asp Ala Val Ala Leu Glu Arg Trp Pro Arg Asp Gly
945                 950                 955                 960

Pro Ala Leu Ser Gln Tyr His Val Tyr Val Arg Ala Pro Ala Arg Pro
                965                 970                 975

Asp Ala Gln Ala Val Val Arg Trp Pro Asp Ser Ala Val Thr Glu Gly
            980                 985                 990

Leu Ala Arg Ala Val Phe Ala Ser  Ser Arg Thr Phe Gly  Pro Ala Ser
        995                 1000                1005

Phe Ala  Arg Ile Glu Thr Ala  Phe Ala Asn Leu Tyr  Pro Gly Glu
    1010                1015                1020

Gln Pro  Leu Cys Leu Cys Arg  Gly Gly Asn Val Ala  Tyr Thr Val
    1025                1030                1035

Cys Thr Arg Ala Gly Pro Lys Thr Arg Val Pro Leu Ser Pro Arg
    1040                1045                1050

Glu Tyr Arg Gln Tyr Val Leu Pro Gly Phe Asp Gly Cys Lys Asp
    1055                1060                1065

Leu Ala Arg Gln Ser Arg Gly Leu Gly Leu Gly Ala Ala Asp Phe
    1070                1075                1080

Val Asp Glu Ala Ala His Ser His Arg Ala Ala Asn Arg Trp Gly
    1085                1090                1095

Leu Gly Ala Ala Leu Arg Pro Val Phe Leu Pro Glu Gly Arg Arg
    1100                1105                1110

Pro Gly Ala Ala Gly Pro Glu Ala Gly Asp Val Pro Thr Trp Ala
    1115                1120                1125

Arg Val Phe Cys Arg His Ala Leu Leu Glu Pro Asp Pro Ala Ala
    1130                1135                1140

Glu Pro Leu Val Leu Pro Pro Val Ala Gly Arg Ser Val Ala Leu
    1145                1150                1155

Tyr Ala Ser Ala Asp Glu Ala Arg Asn Ala Leu Pro Pro Ile Pro
    1160                1165                1170

Arg Val Met Trp Pro Pro Gly Phe Gly Ala Ala Glu Thr Val Leu
    1175                1180                1185

Glu Gly Ser Asp Gly Thr Arg Phe Val Phe Gly His His Gly Gly
    1190                1195                1200

Ser Glu Arg Pro Ser Glu Thr Gln Ala Gly Arg Gln Arg Arg Thr
    1205                1210                1215

Ala Asp Asp Arg Glu His Ala Leu Glu Leu Asp Asp Trp Glu Val
    1220                1225                1230

Gly Cys Glu Asp Ala Trp Asp Ser Glu Glu Gly Gly Gly Asp Asp
    1235                1240                1245

Gly Asp Ala Pro Gly Ser Ser Phe Gly Val Ser Ile Val Ser Val
    1250                1255                1260

Ala Pro Gly Val Leu Arg Asp Arg Arg Val Gly Leu Arg Pro Ala
    1265                1270                1275

Val Lys Val Glu Leu Leu Ser Ser Ser Ser Ser Ser Glu Asp Glu
    1280                1285                1290

Asp Asp Val Trp Gly Gly Arg Gly Gly Arg Ser Pro Pro Gln Ser
    1295                1300                1305

Arg Gly
    1310

<210> 56
<211> 467
<212> PRT
<213> Varicella zoster

<400> 56

Met Asp Thr Ile Leu Ala Gly Gly Ser Gly Thr Ser Asp Ala Ser Asp
1               5                   10                  15

Asn Thr Cys Thr Ile Cys Met Ser Thr Val Ser Asp Leu Gly Lys Thr
            20                  25                  30

Met Pro Cys Leu His Asp Phe Cys Phe Val Cys Ile Arg Ala Trp Thr
        35                  40                  45

Ser Thr Ser Val Gln Cys Pro Leu Cys Arg Cys Pro Val Gln Ser Ile
    50                  55                  60

Leu His Lys Ile Val Ser Asp Thr Ser Tyr Lys Glu Tyr Glu Val His
65                  70                  75                  80

Pro Ser Asp Asp Asp Gly Phe Ser Glu Pro Ser Phe Glu Asp Ser Ile
                85                  90                  95

Asp Ile Leu Pro Gly Asp Val Ile Asp Leu Leu Pro Pro Ser Pro Gly
            100                 105                 110

Pro Ser Arg Glu Ser Ile Gln Gln Pro Thr Ser Arg Ser Ser Arg Glu
        115                 120                 125

Pro Ile Gln Ser Pro Asn Pro Gly Pro Leu Gln Ser Ser Ala Arg Glu
    130                 135                 140

Pro Thr Ala Glu Ser Pro Ser Asp Ser Gln Gln Asp Ser Ile Gln Pro
145                 150                 155                 160

Pro Thr Arg Asp Ser Ser Pro Gly Val Thr Lys Thr Cys Ser Thr Ala
                165                 170                 175

Ser Phe Leu Arg Lys Val Phe Phe Lys Asp Gln Pro Ala Val Arg Ser
            180                 185                 190

Ala Thr Pro Val Val Tyr Gly Ser Ile Glu Ser Ala Gln Gln Pro Arg
        195                 200                 205

Thr Gly Gly Gln Asp Tyr Arg Asp Arg Pro Val Ser Val Gly Ile Asn

210                 215                 220

Gln Asp Pro Arg Thr Met Asp Arg Leu Pro Phe Arg Ala Thr Asp Arg
225                 230                 235                 240

Gly Thr Glu Gly Asn Ala Arg Phe Pro Cys Tyr Met Gln Pro Leu Leu
                245                 250                 255

Gly Trp Leu Asp Asp Gln Leu Ala Glu Leu Tyr Gln Pro Glu Ile Val
            260                 265                 270

Glu Pro Thr Lys Met Leu Ile Leu Asn Tyr Ile Gly Ile Tyr Gly Arg
        275                 280                 285

Asp Glu Ala Gly Leu Lys Thr Ser Leu Arg Cys Leu Leu His Asp Ser
    290                 295                 300

Thr Gly Pro Phe Val Thr Asn Met Leu Phe Leu Leu Asp Arg Cys Thr
305                 310                 315                 320

Asp Pro Thr Arg Leu Thr Met Gln Thr Trp Thr Trp Lys Asp Thr Ala
                325                 330                 335

Ile Gln Leu Ile Thr Gly Pro Ile Val Arg Pro Glu Thr Thr Ser Thr
            340                 345                 350

Gly Glu Thr Ser Arg Gly Asp Glu Arg Asp Thr Arg Leu Val Asn Thr
        355                 360                 365

Pro Gln Lys Val Arg Leu Phe Ser Val Leu Pro Gly Ile Lys Pro Gly
    370                 375                 380

Ser Ala Arg Gly Ala Lys Arg Arg Leu Phe His Thr Gly Arg Asp Val
385                 390                 395                 400

Lys Arg Cys Leu Thr Ile Asp Leu Thr Ser Glu Ser Asp Ser Ala Cys
                405                 410                 415

Lys Gly Ser Lys Thr Arg Lys Val Ala Ser Pro Gln Gly Glu Ser Asn
            420                 425                 430

Thr Pro Ser Thr Ser Gly Ser Thr Ser Gly Ser Leu Lys His Leu Thr
        435                 440                 445

Lys Lys Ser Ser Ala Gly Lys Ala Gly Lys Gly Ile Pro Asn Lys Met
    450                 455                 460

Lys Lys Ser
465

<210>   57

<211>  305
<212>  PRT
<213>  Varicella zoster

<400>  57

Met Asp Val Ser Gly Glu Pro Thr Val Cys Ser Asn Ala Tyr Ala Asn
1               5                   10                  15

Glu Met Lys Leu Ser Asp Ser Lys Asp Ile Tyr Val Leu Ala His Pro
            20                  25                  30

Val Thr Lys Lys Thr Arg Lys Arg Pro Arg Gly Leu Pro Leu Gly Val
        35                  40                  45

Lys Leu Asp Pro Pro Thr Phe Lys Leu Asn Asn Met Ser His His Tyr
    50                  55                  60

Asp Thr Glu Thr Phe Thr Pro Val Ser Ser Gln Leu Asp Ser Val Glu
65                  70                  75                  80

Val Phe Ser Lys Phe Asn Ile Ser Pro Glu Trp Tyr Asp Leu Leu Ser
                85                  90                  95

Asp Glu Leu Lys Glu Pro Tyr Ala Lys Gly Ile Phe Leu Glu Tyr Asn
            100                 105                 110

Arg Leu Leu Asn Ser Gly Glu Glu Ile Leu Pro Ser Thr Gly Asp Ile
        115                 120                 125

Phe Ala Trp Thr Arg Phe Cys Gly Pro Gln Ser Ile Arg Val Val Ile
    130                 135                 140

Ile Gly Gln Asp Pro Tyr Pro Thr Ala Gly His Ala His Gly Leu Ala
145                 150                 155                 160

Phe Ser Val Lys Arg Gly Ile Thr Pro Pro Ser Ser Leu Lys Asn Ile
                165                 170                 175

Phe Ala Ala Leu Met Glu Ser Tyr Pro Asn Met Thr Pro Pro Thr His
            180                 185                 190

Gly Cys Leu Glu Ser Trp Ala Arg Gln Gly Val Leu Leu Leu Asn Thr
        195                 200                 205

Thr Leu Thr Val Arg Arg Gly Thr Pro Gly Ser His Val Tyr Leu Gly
    210                 215                 220

Trp Gly Arg Leu Val Gln Arg Val Leu Gln Arg Leu Cys Glu Asn Arg
225                 230                 235                 240

Thr Gly Leu Val Phe Met Leu Trp Gly Ala His Ala Gln Lys Thr Thr
                245                 250                 255

Gln Pro Asn Ser Arg Cys His Leu Val Leu Thr His Ala His Pro Ser
            260                 265                 270

Pro Leu Ser Arg Val Pro Phe Arg Asn Cys Arg His Phe Val Gln Ala
        275                 280                 285

Asn Glu Tyr Phe Thr Arg Lys Gly Glu Pro Glu Ile Asp Trp Ser Val
    290                 295                 300

Ile
305

<210> 58
<211> 71
<212> PRT
<213> Varicella zoster

<400> 58

Met Asp Val Arg Glu Arg Asn Val Phe Gly Asn Ala Ser Val Ala Thr
1               5                   10                  15

Pro Gly Glu His Gln Lys Phe Val Arg Glu Leu Ile Leu Ser Gly His
            20                  25                  30

Asn Asn Val Val Leu Gln Thr Tyr Thr Gly Lys Trp Ser Asp Cys Arg
        35                  40                  45

Lys His Gly Lys Ser Val Met Tyr Asn Thr Gly Glu Ala Arg His Pro
    50                  55                  60

Thr Cys Lys Ala His Gln Arg
65                  70

<210> 59
<211> 331
<212> PRT
<213> Varicella zoster

<400> 59

Met Gln Arg Ile Arg Pro Tyr Trp Ile Lys Phe Glu Gln Thr Gly Gly
1               5                   10                  15

Ala Gly Met Ala Asp Gly Met Ser Gly Ile Asn Ile Pro Ser Ile Leu
            20                  25                  30

Gly Cys Ser Val Thr Ile Asp Asn Leu Leu Thr Arg Ala Glu Glu Gly
        35                  40                  45

Leu Asp Val Ser Asp Val Ile Glu Asp Leu Arg Ile Gln Ala Ile Pro
    50                  55                  60

Arg Phe Val Cys Glu Ala Arg Glu Val Thr Gly Leu Lys Pro Arg Phe
65                  70                  75                  80

Leu Ala Asn Ser Val Val Ser Leu Arg Val Lys Pro Glu His Gln Glu
                85                  90                  95

Thr Val Leu Val Val Leu Asn Gly Asp Ser Ser Glu Val Ser Cys Asp
            100                 105                 110

Arg Tyr Tyr Met Glu Cys Val Thr Gln Pro Ala Phe Arg Gly Phe Ile
        115                 120                 125

Phe Ser Val Leu Thr Ala Val Glu Asp Arg Val Tyr Thr Val Gly Val
    130                 135                 140

Pro Pro Arg Leu Leu Ile Tyr Arg Met Thr Leu Phe Arg Pro Asp Asn
145                 150                 155                 160

Val Leu Asp Phe Thr Leu Cys Val Ile Leu Met Tyr Leu Glu Gly Ile
                165                 170                 175

Gly Pro Ser Gly Ala Ser Pro Ser Leu Phe Val Gln Leu Ser Val Tyr
            180                 185                 190

Leu Arg Arg Val Glu Cys Gln Ile Gly Pro Leu Glu Lys Met Arg Arg
        195                 200                 205

Phe Leu Tyr Glu Gly Val Leu Trp Leu Leu Asn Thr Leu Met Tyr Val
    210                 215                 220

Val Asp Asn Asn Pro Phe Thr Lys Thr Arg Val Leu Pro His Tyr Met
225                 230                 235                 240

Phe Val Lys Leu Leu Asn Pro Gln Pro Gly Thr Ala Pro Asn Ile Ile
                245                 250                 255

Lys Ala Ile Tyr Ser Cys Gly Val Gly Gln Arg Phe Asp Leu Pro His
            260                 265                 270

Gly Thr Pro Pro Cys Pro Asp Gly Val Val Gln Val Pro Pro Gly Leu
        275                 280                 285

Leu Asn Gly Pro Leu Arg Asp Ser Glu Tyr Gln Lys Ser Val Tyr Phe
    290                 295                 300

Trp Trp Leu Asn Arg Thr Met Val Thr Pro Lys Asn Val Gln Leu Phe
305                 310                 315                 320

Glu Thr Tyr Lys Asn Ser Pro Arg Val Val Lys
                325                 330

<210>  60
<211>  541
<212>  PRT
<213>  Varicella zoster

<400>  60

Met Glu Phe Pro Tyr His Ser Thr Val Ser Tyr Asn Gly Val Thr Phe
1               5                   10                  15

Tyr Phe Asn Glu Arg Ala Thr Arg Ala Tyr Phe Ile Cys Gly Gly Cys
            20                  25                  30

Leu Ile Ser Ile Pro Arg Lys His Gly Gly Glu Ile Ala Lys Phe Gly
        35                  40                  45

His Val Val Arg Gly Val Gly Pro Gly Asp Arg Ser Val Ala Ser Tyr
    50                  55                  60

Val Arg Ser Glu Leu Asn Arg Thr Gly Lys Thr Trp Ala Val Ser Ser
65                  70                  75                  80

Asn Asn Asn Cys Val Phe Leu Asp Arg Val Ala Leu Leu Ala Ala Gly
                85                  90                  95

Ser Gly Ala Val Asp Arg Asp Leu Cys Gly Thr Phe Asp Val Glu Val
            100                 105                 110

Glu Asp Pro Thr Leu Ala Asp Tyr Leu Val Ser Leu Pro Val Thr His
        115                 120                 125

Leu Thr Leu Val Ala Gly Val Asp Val Thr Arg Glu Asn Lys Leu Lys
    130                 135                 140

Leu Phe Pro Thr Pro Thr Ala Ile Asn Thr Thr Asn Gly Phe Met Tyr
145                 150                 155                 160

Val Pro Asn Glu Ala Ser Phe Ser Leu Val Tyr Met Arg Met Leu Glu
                165                 170                 175

Leu Pro Glu Ser Leu Gln Glu Leu Val Ser Gly Leu Phe Asp Gly Thr
            180                 185                 190

Pro Glu Ile Arg Asp Ala Leu Asn Gly Ser Asn Asp Asp Glu Lys Thr
        195                 200                 205

Ser Ile Ile Val Ser Arg Arg Ala Ala Asp Val Val Thr Glu Asp Val
    210                 215                 220

Lys Ala Asp Asp Val Pro Ile Ser Gly Glu Pro Tyr Ser Glu Lys Gln
225                 230                 235                 240

Pro Arg Arg Arg Lys Lys Ser Asp His Ile Thr Leu Ser Asn Phe Val

                245                 250                 255

Gln Ile Arg Thr Ile Pro Arg Val Met Asp Ile Trp Asp Pro Arg His
            260                 265                 270

Lys Ala Thr Thr His Cys Ile Arg Ala Leu Ser Cys Ala Val Phe Phe
        275                 280                 285

Ala Asp Glu Val Ile Phe Lys Ala Arg Lys Trp Pro Gly Leu Glu Asp
    290                 295                 300

Glu Leu Asn Glu Ala Arg Glu Thr Ile Tyr Thr Ala Val Val Ala Val
305                 310                 315                 320

Tyr Gly Glu Arg Gly Glu Leu Pro Phe Phe Gly His Ala Tyr Gly Arg
                325                 330                 335

Asp Leu Thr Ser Cys Gln Arg Phe Val Ile Val Gln Tyr Ile Leu Ser
            340                 345                 350

Arg Trp Glu Ala Phe Asn Cys Tyr Ala Val Ile Glu Asp Leu Thr Arg
        355                 360                 365

Ser Tyr Val Asn Ala Leu Pro Ser Asp Asp Asp Thr Asp Gln Val Ala
    370                 375                 380

Gln Asp Leu Ile Arg Thr Ile Val Asp Thr Ala Asn Ser Leu Leu Arg
385                 390                 395                 400

Glu Val Gly Phe Ile Gly Thr Leu Ala Glu Thr Leu Leu Phe Leu Pro
                405                 410                 415

Leu Pro Gln Leu Pro Cys Tyr Lys Glu Thr Ser His Leu Ala Lys Lys
            420                 425                 430

Glu Gly Val Arg Ile Leu Arg Leu Ala Lys Thr Gly Val Gly Leu Ser
        435                 440                 445

Asp Thr Val Pro Val Asp Val Ser Val Thr Glu Arg His Glu Tyr Glu
    450                 455                 460

Ile Ser Arg Tyr Leu Asp Thr Leu Tyr Ser Gly Asp Pro Cys Tyr Asn
465                 470                 475                 480

Gly Ala Val Arg Leu Cys Arg Leu Leu Gly Ser Ser Ile Pro Ile Ala
                485                 490                 495

Leu Tyr Tyr Asn Thr Ile Ser Gly Asn Ala Phe Glu Pro Tyr Phe Ala
            500                 505                 510

Gly Arg Arg Tyr Ile Ala Tyr Leu Gly Ala Leu Phe Phe Gly Arg Val

515                 520                 525

His Gln Thr Pro Phe Gly Asp Gly Lys Lys Thr Gln Arg
    530                 535                 540

<210>  61
<211>  258
<212>  PRT
<213>  Varicella zoster

<400>  61

Met Ser Ala Ser Arg Ile Arg Ala Lys Cys Phe Arg Leu Gly Gln Arg
1               5                   10                  15

Cys His Thr Arg Phe Tyr Asp Val Leu Lys Lys Asp Ile Asp Asn Val
            20                  25                  30

Arg Arg Gly Phe Ala Asp Ala Phe Asn Pro Arg Leu Ala Lys Leu Leu
        35                  40                  45

Ser Pro Leu Ser His Val Asp Val Gln Arg Ala Val Arg Ile Ser Met
    50                  55                  60

Ser Phe Glu Val Asn Leu Gly Arg Arg Arg Pro Asp Cys Val Cys Ile
65                  70                  75                  80

Ile Gln Thr Glu Ser Ser Gly Ala Gly Lys Thr Val Cys Phe Ile Val
                85                  90                  95

Glu Leu Lys Ser Cys Arg Phe Ser Ala Asn Ile His Thr Pro Thr Lys
            100                 105                 110

Tyr His Gln Phe Cys Glu Gly Met Arg Gln Leu Arg Asp Thr Met Ala
        115                 120                 125

Leu Ile Lys Glu Thr Thr Pro Thr Gly Ser Asp Glu Ile Met Val Thr
    130                 135                 140

Pro Leu Leu Val Phe Val Ser Gln Arg Gly Leu Asn Leu Leu Gln Val
145                 150                 155                 160

Thr Arg Leu Pro Pro Lys Val Ile His Gly Asn Leu Val Met Leu Ala
                165                 170                 175

Ser His Leu Glu Asn Val Ala Glu Tyr Thr Pro Pro Ile Arg Ser Val
            180                 185                 190

Arg Glu Arg Arg Arg Leu Cys Lys Lys Ile His Val Cys Ser Leu
        195                 200                 205

Ala Lys Lys Arg Ala Lys Ser Cys His Arg Ser Ala Leu Thr Lys Phe
    210                 215                 220

Glu Glu Asn Ala Ala Cys Gly Val Asp Leu Pro Leu Arg Arg Pro Ser
225                 230                 235                 240

Leu Gly Ala Cys Gly Gly Ile Leu Gln Ser Ile Thr Gly Met Phe Ser
                245                 250                 255

His Gly

<210>  62
<211>  579
<212>  PRT
<213>  Varicella zoster

<400>  62

Met Thr Ala Arg Tyr Gly Phe Gly Ser Ile Ser Phe Pro Asn Lys Cys
1               5                   10                  15

Gly Ile Phe Leu Ser Thr Thr Lys Asn Phe Ile Ala Pro Asn Phe Pro
            20                  25                  30

Ile His Tyr Trp Thr Ala Pro Ala Phe Glu Leu Arg Gly Arg Met Asn
        35                  40                  45

Pro Asp Leu Glu Lys Asn Thr Leu Thr Leu Lys Asn Ala Ala Ala Val
    50                  55                  60

Ala Ala Leu Asp Asn Leu Arg Gly Glu Thr Ile Thr Leu Pro Thr Glu
65                  70                  75                  80

Ile Asp Arg Arg Leu Lys Pro Leu Glu Glu Gln Leu Thr Arg Met Ala
                85                  90                  95

Lys Val Leu Asp Ser Leu Glu Thr Ala Ala Ala Glu Ala Glu Glu Ala
            100                 105                 110

Asp Ala Gln Ser Glu Glu Cys Thr Arg Thr Glu Ile Ile Arg Asn Glu
        115                 120                 125

Ser Ile His Pro Glu Val Gln Ile Ala Lys Asn Asp Ala Pro Leu Gln
    130                 135                 140

Tyr Asp Thr Asn Phe Gln Val Asp Phe Ile Thr Leu Val Tyr Leu Gly
145                 150                 155                 160

Arg Ala Arg Gly Asn Asn Ser Pro Gly Ile Val Phe Gly Pro Trp Tyr
                165                 170                 175

Arg Thr Leu Gln Glu Arg Leu Val Leu Asp Arg Pro Val Ala Ala Arg
            180                 185                 190

Gly Val Asp Cys Lys Asp Gly Arg Ile Ser Arg Thr Phe Met Asn Thr
        195                 200                 205

Thr Val Thr Cys Leu Gln Ser Ala Gly Arg Met Tyr Val Gly Asp Arg
    210                 215                 220

Ala Tyr Ser Ala Phe Glu Cys Ala Val Leu Cys Leu Tyr Leu Met Tyr
225                 230                 235                 240

Arg Thr Ser Asn Ser Val His Glu Pro Gln Val Ser Ser Phe Gly Asn
                245                 250                 255

Leu Ile Glu His Leu Pro Glu Tyr Thr Glu Thr Phe Val Asn Tyr Met
            260                 265                 270

Thr Thr His Glu Asn Lys Asn Ser Tyr Gln Phe Cys Tyr Asp Arg Leu
        275                 280                 285

Pro Arg Asp Gln Phe His Ala Arg Gly Gly Arg Tyr Asp Gln Gly Ala
    290                 295                 300

Leu Thr Ser His Ser Val Met Asp Ala Leu Ile Arg Leu Gln Val Leu
305                 310                 315                 320

Pro Pro Ala Pro Gly Gln Phe Asn Pro Gly Val Asn Asp Ile Ile Asp
                325                 330                 335

Arg Asn His Thr Ala Tyr Val Asp Lys Ile Gln Gln Ala Ala Ala Ala
            340                 345                 350

Tyr Leu Glu Arg Ala Gln Asn Val Phe Leu Met Glu Asp Gln Thr Leu
        355                 360                 365

Leu Arg Leu Thr Ile Asp Thr Ile Thr Ala Leu Leu Leu Leu Arg Arg
    370                 375                 380

Leu Leu Trp Asn Gly Asn Val Tyr Gly Asp Lys Leu Lys Asn Asn Phe
385                 390                 395                 400

Gln Leu Gly Leu Ile Val Ser Glu Ala Thr Gly Thr Pro Thr Asn Asn
                405                 410                 415

Val Ile Leu Arg Gly Ala Thr Gly Phe Asp Gly Lys Phe Lys Ser Gly
            420                 425                 430

Asn Asn Asn Phe Gln Phe Leu Cys Glu Arg Tyr Ile Ala Pro Leu Tyr
        435                 440                 445

Thr Leu Asn Arg Thr Thr Glu Leu Thr Glu Met Phe Pro Gly Leu Val
    450                 455                 460

Ala Leu Cys Leu Asp Ala His Thr Gln Leu Ser Arg Gly Ser Leu Gly
465                 470                 475                 480

Arg Thr Val Ile Asp Ile Ser Ser Gly Gln Tyr Gln Asp Arg Leu Ile
                485                 490                 495

Ser Leu Ile Ala Leu Glu Leu Glu His Arg Arg Gln Asn Val Thr Ser
            500                 505                 510

Leu Pro Ile Ala Ala Val Val Ser Ile His Asp Ser Val Met Leu Gln
        515                 520                 525

Tyr Glu Arg Gly Leu Gly Met Leu Met His Gln Pro Arg Val Arg Ala
    530                 535                 540

Ala Leu Glu Glu Ser Arg Arg Leu Ala Gln Phe Asn Val Asn Ser Asp
545                 550                 555                 560

Tyr Asp Leu Leu Tyr Phe Val Cys Leu Gly Val Ile Pro Gln Phe Ala
                565                 570                 575

Ser Thr Pro

<210>  63
<211>  605
<212>  PRT
<213>  Varicella zoster

<400>  63

Met Ala Ala Glu Ala Asp Glu Glu Asn Cys Glu Ala Leu Tyr Val Ala
1               5                   10                  15

Gly Tyr Leu Ala Leu Tyr Ser Lys Asp Glu Gly Glu Leu Asn Ile Thr
            20                  25                  30

Pro Glu Ile Val Arg Ser Ala Leu Pro Pro Thr Ser Lys Ile Pro Ile
        35                  40                  45

Asn Ile Asp His Arg Lys Asp Cys Val Val Gly Glu Val Ile Ala Ile
    50                  55                  60

Ile Glu Asp Ile Arg Gly Pro Phe Phe Leu Gly Ile Val Arg Cys Pro
65                  70                  75                  80

Gln Leu His Ala Val Leu Phe Glu Ala Ala His Ser Asn Phe Phe Gly
                85                  90                  95

Asn Arg Asp Ser Val Leu Ser Pro Leu Glu Arg Ala Leu Tyr Leu Val
            100                 105                 110

Thr Asn Tyr Leu Pro Ser Val Ser Leu Ser Ser Lys Arg Leu Ser Pro
        115                 120                 125

Asn Glu Ile Pro Asp Gly Asn Phe Phe Thr His Val Ala Leu Cys Val
    130                 135                 140

Val Gly Arg Arg Val Gly Thr Val Val Asn Tyr Asp Cys Thr Pro Glu
145                 150                 155                 160

Ser Ser Ile Glu Pro Phe Arg Val Leu Ser Met Glu Ser Lys Ala Arg
                165                 170                 175

Leu Leu Ser Leu Val Lys Asp Tyr Ala Gly Leu Asn Lys Val Trp Lys
            180                 185                 190

Val Ser Glu Asp Lys Leu Ala Lys Val Leu Leu Ser Thr Ala Val Asn
        195                 200                 205

Asn Met Leu Leu Arg Asp Arg Trp Asp Val Val Ala Lys Arg Arg Arg
    210                 215                 220

Glu Ala Gly Ile Met Gly His Val Tyr Leu Gln Ala Ser Thr Gly Tyr
225                 230                 235                 240

Gly Leu Ala Arg Ile Thr Asn Val Asn Gly Val Glu Ser Lys Leu Pro
                245                 250                 255

Asn Ala Gly Val Ile Asn Ala Thr Phe His Pro Gly Gly Pro Ile Tyr
            260                 265                 270

Asp Leu Ala Leu Gly Val Gly Glu Ser Asn Glu Asp Cys Glu Lys Thr
        275                 280                 285

Val Pro His Leu Lys Val Thr Gln Leu Cys Arg Asn Asp Ser Asp Met
    290                 295                 300

Ala Ser Val Ala Gly Asn Ala Ser Asn Ile Ser Pro Gln Pro Pro Ser
305                 310                 315                 320

Gly Val Pro Thr Gly Gly Glu Phe Val Leu Ile Pro Thr Ala Tyr Tyr
                325                 330                 335

Ser Gln Leu Leu Thr Gly Gln Thr Lys Asn Pro Gln Val Ser Ile Gly
            340                 345                 350

Ala Pro Asn Asn Gly Gln Tyr Ile Val Gly Pro Tyr Gly Ser Pro His
        355                 360                 365

Pro Pro Ala Phe Pro Pro Asn Thr Gly Gly Tyr Gly Cys Pro Pro Gly
    370                 375                 380

His Phe Gly Gly Pro Tyr Gly Phe Pro Gly Tyr Pro Pro Pro Asn Arg
385                 390                 395                 400

Leu Glu Met Gln Met Ser Ala Phe Met Asn Ala Leu Ala Ala Glu Arg
                405                 410                 415

Gly Ile Asp Leu Gln Thr Pro Cys Val Asn Phe Pro Asp Lys Thr Asp
            420                 425                 430

Val Arg Arg Pro Gly Lys Arg Asp Phe Lys Ser Met Asp Gln Arg Glu
        435                 440                 445

Leu Asp Ser Phe Tyr Ser Gly Glu Ser Gln Met Asp Gly Glu Phe Pro
    450                 455                 460

Ser Asn Ile Tyr Phe Pro Gly Glu Pro Thr Tyr Ile Thr His Arg Arg
465                 470                 475                 480

Arg Arg Val Ser Pro Ser Tyr Trp Gln Arg Arg His Arg Val Ser Asn
                485                 490                 495

Gly Gln His Glu Glu Leu Ala Gly Val Val Ala Lys Leu Gln Gln Glu
            500                 505                 510

Val Thr Glu Leu Lys Ser Gln Asn Gly Thr Gln Met Pro Leu Ser His
        515                 520                 525

His Thr Asn Ile Pro Glu Gly Thr Arg Asp Pro Arg Ile Ser Ile Leu
    530                 535                 540

Leu Lys Gln Leu Gln Ser Val Ser Gly Leu Cys Ser Ser Gln Asn Thr
545                 550                 555                 560

Thr Ser Thr Pro His Thr Asp Thr Val Gly Gln Asp Val Asn Ala Val
                565                 570                 575

Glu Ala Ser Ser Lys Ala Pro Leu Ile Gln Gly Ser Thr Ala Asp Asp
            580                 585                 590

Ala Asp Met Phe Ala Asn Gln Met Met Val Gly Arg Cys
        595                 600                 605

<210>  64
<211>  1194
<212>  PRT
<213>  Varicella zoster

<400>  64

Met Ala Ile Arg Thr Gly Phe Cys Asn Pro Phe Leu Thr Gln Ala Ser
1               5                   10                  15

Gly Ile Lys Tyr Asn Pro Arg Thr Gly Arg Gly Ser Asn Arg Glu Phe

20                  25                  30

Leu His Ser Tyr Lys Thr Thr Met Ser Ser Phe Gln Phe Leu Ala Pro
        35                  40                  45

Lys Cys Leu Asp Glu Asp Val Pro Met Glu Glu Arg Lys Gly Val His
    50                  55                  60

Val Gly Thr Leu Ser Arg Pro Pro Lys Val Tyr Cys Asn Gly Lys Glu
65                  70                  75                  80

Val Pro Ile Leu Asp Phe Arg Cys Ser Ser Pro Trp Pro Arg Arg Val
                85                  90                  95

Asn Ile Trp Gly Glu Ile Asp Phe Arg Gly Asp Lys Phe Asp Pro Arg
            100                 105                 110

Phe Asn Thr Phe His Val Tyr Asp Ile Val Glu Thr Thr Glu Ala Ala
        115                 120                 125

Ser Asn Gly Asp Val Ser Arg Phe Ala Thr Ala Thr Arg Pro Leu Gly
    130                 135                 140

Thr Val Ile Thr Leu Leu Gly Met Ser Arg Cys Gly Lys Arg Val Ala
145                 150                 155                 160

Val His Val Tyr Gly Ile Cys Gln Tyr Phe Tyr Ile Asn Lys Ala Glu
                165                 170                 175

Val Asp Thr Ala Cys Gly Ile Arg Ser Gly Ser Glu Leu Ser Val Leu
            180                 185                 190

Leu Ala Glu Cys Leu Arg Ser Ser Met Ile Thr Gln Asn Asp Ala Thr
        195                 200                 205

Leu Asn Gly Asp Lys Asn Ala Phe His Gly Thr Ser Phe Lys Ser Ala
    210                 215                 220

Ser Pro Glu Ser Phe Arg Val Glu Val Ile Glu Arg Thr Asp Val Tyr
225                 230                 235                 240

Tyr Tyr Asp Thr Gln Pro Cys Ala Phe Tyr Arg Val Tyr Ser Pro Ser
                245                 250                 255

Ser Lys Phe Thr Asn Tyr Leu Cys Asp Asn Phe His Pro Glu Leu Lys
            260                 265                 270

Lys Tyr Glu Gly Arg Val Asp Ala Thr Thr Arg Phe Leu Met Asp Asn
        275                 280                 285

Pro Gly Phe Val Ser Phe Gly Trp Tyr Gln Leu Lys Pro Gly Val Asp

290                 295                 300

Gly Glu Arg Val Arg Val Arg Pro Ala Ser Arg Gln Leu Thr Leu Ser
305                 310                 315                 320

Asp Val Glu Ile Asp Cys Met Ser Asp Asn Leu Gln Ala Ile Pro Asn
                325                 330                 335

Asp Asp Ser Trp Pro Asp Tyr Lys Leu Leu Cys Phe Asp Ile Glu Cys
            340                 345                 350

Lys Ser Gly Gly Ser Asn Glu Leu Ala Phe Pro Asp Ala Thr His Leu
        355                 360                 365

Glu Asp Leu Val Ile Gln Ile Ser Cys Leu Leu Tyr Ser Ile Pro Arg
    370                 375                 380

Gln Ser Leu Glu His Ile Leu Leu Phe Ser Leu Gly Ser Cys Asp Leu
385                 390                 395                 400

Pro Gln Arg Tyr Val Gln Glu Met Lys Asp Ala Gly Leu Pro Glu Pro
                405                 410                 415

Thr Val Leu Glu Phe Asp Ser Glu Phe Glu Leu Leu Ile Ala Phe Met
            420                 425                 430

Thr Leu Val Lys Gln Tyr Ala Pro Glu Phe Ala Thr Gly Tyr Asn Ile
        435                 440                 445

Val Asn Phe Asp Trp Ala Phe Ile Met Glu Lys Leu Asn Ser Ile Tyr
    450                 455                 460

Ser Leu Lys Leu Asp Gly Tyr Gly Ser Ile Asn Arg Gly Gly Leu Phe
465                 470                 475                 480

Lys Ile Trp Asp Val Gly Lys Ser Gly Phe Gln Arg Arg Ser Lys Val
                485                 490                 495

Lys Ile Asn Gly Leu Ile Ser Leu Asp Met Tyr Ala Ile Ala Thr Glu
            500                 505                 510

Lys Leu Lys Leu Ser Ser Tyr Lys Leu Asp Ser Val Ala Arg Glu Ala
        515                 520                 525

Leu Asn Glu Ser Lys Arg Asp Leu Pro Tyr Lys Asp Ile Pro Gly Tyr
    530                 535                 540

Tyr Ala Ser Gly Pro Asn Thr Arg Gly Ile Ile Gly Glu Tyr Cys Ile
545                 550                 555                 560

Gln Asp Ser Ala Leu Val Gly Lys Leu Phe Phe Lys Tyr Leu Pro His

565                 570                 575

Leu Glu Leu Ser Ala Val Ala Arg Leu Ala Arg Ile Thr Leu Thr Lys
            580                 585                 590

Ala Ile Tyr Asp Gly Gln Gln Val Arg Ile Tyr Thr Cys Leu Leu Gly
        595                 600                 605

Leu Ala Ser Ser Arg Gly Phe Ile Leu Pro Asp Gly Gly Tyr Pro Ala
    610                 615                 620

Thr Phe Glu Tyr Lys Asp Val Ile Pro Asp Val Gly Asp Val Glu Glu
625                 630                 635                 640

Glu Met Asp Glu Asp Glu Ser Val Ser Pro Thr Gly Thr Ser Ser Gly
                645                 650                 655

Arg Asn Val Gly Tyr Lys Gly Ala Arg Val Phe Asp Pro Asp Thr Gly
            660                 665                 670

Phe Tyr Ile Asp Pro Val Val Val Leu Asp Phe Ala Ser Leu Tyr Pro
        675                 680                 685

Ser Ile Ile Gln Ala His Asn Leu Cys Phe Thr Thr Leu Thr Leu Asn
    690                 695                 700

Phe Glu Thr Val Lys Arg Leu Asn Pro Ser Asp Tyr Ala Thr Phe Thr
705                 710                 715                 720

Val Gly Gly Lys Arg Leu Phe Phe Val Arg Ser Asn Val Arg Glu Ser
                725                 730                 735

Leu Leu Gly Val Leu Leu Lys Asp Trp Leu Ala Met Arg Lys Ala Ile
            740                 745                 750

Arg Ala Arg Ile Pro Gly Ser Ser Ser Asp Glu Ala Val Leu Leu Asp
        755                 760                 765

Lys Gln Gln Ala Ala Ile Lys Val Val Cys Asn Ser Val Tyr Gly Phe
    770                 775                 780

Thr Gly Val Ala Gln Gly Phe Leu Pro Cys Leu Tyr Val Ala Ala Thr
785                 790                 795                 800

Val Thr Thr Ile Gly Arg Gln Met Leu Leu Ser Thr Arg Asp Tyr Ile
                805                 810                 815

His Asn Asn Trp Ala Ala Phe Glu Arg Phe Ile Thr Ala Phe Pro Asp
            820                 825                 830

Ile Glu Ser Ser Val Leu Ser Gln Lys Ala Tyr Glu Val Lys Val Ile

835                 840                 845

Tyr Gly Asp Thr Asp Ser Val Phe Ile Arg Phe Lys Gly Val Ser Val
    850                 855                 860

Glu Gly Ile Ala Lys Ile Gly Glu Lys Met Ala His Ile Ile Ser Thr
865                 870                 875                 880

Ala Leu Phe Cys Pro Pro Ile Lys Leu Glu Cys Glu Lys Thr Phe Ile
                885                 890                 895

Lys Leu Leu Leu Ile Thr Lys Lys Lys Tyr Ile Gly Val Ile Tyr Gly
            900                 905                 910

Gly Lys Val Leu Met Lys Gly Val Asp Leu Val Arg Lys Asn Asn Cys
        915                 920                 925

Gln Phe Ile Asn Asp Tyr Ala Arg Lys Leu Val Glu Leu Leu Leu Tyr
    930                 935                 940

Asp Asp Thr Val Ser Arg Ala Ala Ala Glu Ala Ser Cys Val Ser Ile
945                 950                 955                 960

Ala Glu Trp Asn Arg Arg Ala Met Pro Ser Gly Met Ala Gly Phe Gly
                965                 970                 975

Arg Ile Ile Ala Asp Ala His Arg Gln Ile Thr Ser Pro Lys Leu Asp
            980                 985                 990

Ile Asn Lys Phe Val Met Thr Ala  Glu Leu Ser Arg Pro  Pro Ser Ala
        995                 1000                 1005

Tyr Ile  Asn Arg Arg Leu Ala  His Leu Thr Val Tyr  Tyr Lys Leu
    1010                 1015                 1020

Val Met  Arg Gln Gly Gln Ile  Pro Asn Val Arg Glu  Arg Ile Pro
    1025                 1030                 1035

Tyr Val  Ile Val Ala Pro Thr  Asp Glu Val Glu Ala  Asp Ala Lys
    1040                 1045                 1050

Ser Val  Ala Leu Leu Arg Gly  Asp Pro Leu Gln Asn  Thr Ala Gly
    1055                 1060                 1065

Lys Arg  Cys Gly Glu Ala Lys  Arg Lys Leu Ile Ile  Ser Asp Leu
    1070                 1075                 1080

Ala Glu  Asp Pro Ile His Val  Thr Ser His Gly Leu  Ser Leu Asn
    1085                 1090                 1095

Ile Asp  Tyr Tyr Phe Ser His  Leu Ile Gly Thr Ala  Ser Val Thr

1100                    1105                    1110

Phe Lys  Ala Leu Phe Gly Asn  Asp Thr Lys Leu Thr  Glu Arg Leu
    1115                    1120                    1125

Leu Lys  Arg Phe Ile Pro Glu  Thr Arg Val Val Asn  Val Lys Met
    1130                    1135                    1140

Leu Asn  Arg Leu Gln Ala Ala  Gly Phe Val Cys Ile  His Ala Pro
    1145                    1150                    1155

Cys Trp  Asp Asn Lys Met Asn  Thr Glu Ala Glu Ile  Thr Glu Glu
    1160                    1165                    1170

Glu Gln  Ser His Gln Ile Met  Arg Arg Val Phe Cys  Ile Pro Lys
    1175                    1180                    1185

Ala Ile  Leu His Gln Ser
    1190

<210>  65
<211>  156
<212>  PRT
<213>  Varicella zoster

<400>  65

Met Tyr Glu Ser Glu Asn Ala Ser Glu His His Pro Glu Leu Glu Asp
1               5                   10                  15

Val Phe Ser Glu Asn Thr Gly Asp Ser Asn Pro Ser Met Gly Ser Ser
            20                  25                  30

Asp Ser Thr Arg Ser Ile Ser Gly Met Arg Ala Arg Asp Leu Ile Thr
        35                  40                  45

Asp Thr Asp Val Asn Leu Leu Asn Ile Asp Ala Leu Glu Ser Lys Tyr
    50                  55                  60

Phe Pro Ala Asp Ser Thr Phe Thr Leu Ser Val Trp Phe Glu Asn Leu
65                  70                  75                  80

Ile Pro Pro Glu Ile Glu Ala Ile Leu Pro Thr Thr Asp Ala Gln Leu
                85                  90                  95

Asn Tyr Ile Ser Phe Thr Ser Arg Leu Ala Ser Val Leu Lys His Lys
            100                 105                 110

Glu Ser Asn Asp Ser Glu Lys Ser Ala Tyr Val Val Pro Cys Glu His
        115                 120                 125

Ser Ala Ser Val Thr Arg Arg Arg Glu Arg Phe Ala Gly Val Met Ala
    130                 135                 140

Lys Phe Leu Asp Leu His Glu Ile Leu Lys Asp Ala
145                 150                 155

<210>  66
<211>  269
<212>  PRT
<213>  Varicella zoster

<400>  66

Met Ser Arg Arg Thr Tyr Val Arg Ser Glu Arg Arg Arg Gly Cys Gly
1               5                   10                  15

Asp Asn Leu Leu Gln Arg Ile Arg Leu Val Val Pro Ser Ala Leu Gln
            20                  25                  30

Cys Cys Asp Gly Asp Leu Pro Ile Phe Asp Pro Gln Arg Pro Pro Ala
        35                  40                  45

Arg Cys Val Phe Gln Phe Asn Gly Glu Asp Asn Val Ser Glu Ala Phe
    50                  55                  60

Pro Val Glu Tyr Ile Met Arg Leu Met Ala Asn Trp Ala Gln Val Asp
65                  70                  75                  80

Cys Asp Pro Tyr Ile Lys Ile Gln Asn Thr Gly Val Ser Val Leu Phe
                85                  90                  95

Gln Gly Phe Phe Phe Arg Pro Thr Asn Ala Pro Val Ala Glu Val Ser
            100                 105                 110

Ile Asp Ser Asn Asn Val Ile Leu Ser Ser Thr Leu Ser Thr Gly Ile
        115                 120                 125

Asn Leu Ser Ala Leu Glu Ser Ile Lys Arg Gly Gly Gly Ile Asp Arg
    130                 135                 140

Arg Pro Leu Gln Ala Leu Met Trp Val Asn Cys Phe Val Arg Met Pro
145                 150                 155                 160

Tyr Val Gln Leu Ser Phe Arg Phe Met Gly Pro Glu Asp Pro Ser Arg
                165                 170                 175

Thr Ile Lys Leu Met Ala Arg Ala Thr Asp Ala Tyr Met Tyr Lys Glu
            180                 185                 190

Thr Gly Asn Asn Leu Asp Glu Tyr Ile Arg Trp Arg Pro Ser Phe Arg
        195                 200                 205

Ser Pro Pro Glu Asn Gly Ser Pro Asn Thr Ser Val Gln Met Gln Ser
    210                 215                 220

Asp Ile Lys Pro Ala Leu Pro Asp Thr Gln Thr Thr Arg Val Trp Lys
225                 230                 235                 240

Leu Ala Leu Pro Val Ala Asn Val Thr Tyr Ala Leu Phe Ile Val Ile
                245                 250                 255

Val Leu Val Val Val Leu Gly Ala Val Leu Phe Trp Lys
                260                 265

<210>  67
<211>  235
<212>  PRT
<213>  Varicella zoster

<400>  67

Met Thr Gln Pro Ala Ser Ser Arg Val Val Phe Asp Pro Ser Asn Pro
1               5                   10                  15

Thr Thr Phe Ser Val Glu Ala Ile Ala Ala Tyr Thr Pro Val Ala Leu
            20                  25                  30

Ile Arg Leu Leu Asn Ala Ser Gly Pro Leu Gln Pro Gly His Arg Val
        35                  40                  45

Asp Ile Ala Asp Ala Arg Ser Ile Tyr Thr Val Gly Ala Ala Ala Ser
    50                  55                  60

Ala Ala Arg Ala Arg Ala Asn His Asn Ala Asn Thr Ile Arg Arg Thr
65                  70                  75                  80

Ala Met Phe Ala Glu Thr Asp Pro Met Thr Trp Leu Arg Pro Thr Val
                85                  90                  95

Gly Leu Lys Arg Thr Phe Asn Pro Arg Ile Ile Arg Pro Gln Pro Pro
            100                 105                 110

Asn Pro Ser Met Ser Leu Gly Ile Ser Gly Pro Thr Ile Leu Pro Gln
        115                 120                 125

Lys Thr Gln Ser Ala Asp Gln Ser Ala Leu Gln Gln Pro Ala Ala Leu
    130                 135                 140

Ala Phe Ser Gly Ser Ser Pro Gln His Pro Pro Pro Gln Thr Thr Ser
145                 150                 155                 160

Ala Ser Val Gly Gln Gln Gln His Val Val Ser Gly Ser Ser Gly Gln
                165                 170                 175

Gln Pro Gln Gln Gly Ala Gln Ser Ser Thr Val Gln Pro Thr Thr Gly
            180                 185                 190

Ser Pro Pro Ala Ala Gln Gly Val Pro Gln Ser Thr Pro Pro Pro Thr
        195                 200                 205

Gln Asn Thr Pro Gln Gly Gly Lys Gly Gln Thr Leu Ser His Thr Gly
    210                 215                 220

Gln Ser Gly Asn Ala Ser Arg Ser Arg Arg Val
225                 230                 235

<210>  68
<211>  483
<212>  PRT
<213>  Varicella zoster

<400>  68

Met Gly Ser Gln Pro Thr Asn Ser His Phe Thr Leu Asn Glu Gln Thr
1               5                   10                  15

Leu Cys Gly Thr Asn Ile Ser Leu Leu Gly Asn Asn Arg Phe Ile Gln
            20                  25                  30

Ile Gly Asn Gly Leu His Met Thr Tyr Ala Pro Gly Phe Phe Gly Asn
        35                  40                  45

Trp Ser Arg Asp Leu Thr Ile Gly Pro Arg Phe Gly Gly Leu Asn Lys
    50                  55                  60

Gln Pro Ile His Val Pro Pro Lys Arg Thr Glu Thr Ala Ser Ile Gln
65                  70                  75                  80

Val Thr Pro Arg Ser Ile Val Ile Asn Arg Met Asn Asn Ile Gln Ile
                85                  90                  95

Asn Pro Thr Ser Ile Gly Asn Pro Gln Val Thr Ile Arg Leu Pro Leu
            100                 105                 110

Asn Asn Phe Lys Ser Thr Thr Gln Leu Ile Gln Gln Val Ser Leu Thr
        115                 120                 125

Asp Phe Phe Arg Pro Asp Ile Glu His Ala Gly Ser Ile Val Leu Ile
    130                 135                 140

Leu Arg His Pro Ser Asp Met Ile Gly Glu Ala Asn Thr Leu Thr Gln
145                 150                 155                 160

Ala Gly Arg Asp Pro Asp Val Leu Leu Glu Gly Leu Arg Asn Leu Phe
                165                 170                 175

Asn Ala Cys Thr Ala Pro Trp Thr Val Gly Glu Gly Gly Gly Leu Arg
            180                 185                 190

Ala Tyr Val Thr Ser Leu Ser Phe Ile Ala Ala Cys Arg Ala Glu Glu

195                 200                 205

Tyr Thr Asp Lys Gln Ala Ala Asp Ala Asn Arg Thr Ala Ile Val Ser
    210                 215                 220

Ala Tyr Gly Cys Ser Arg Met Glu Thr Arg Leu Ile Arg Phe Ser Glu
225                 230                 235                 240

Cys Leu Arg Ala Met Val Gln Cys His Val Phe Pro His Arg Phe Ile
                245                 250                 255

Ser Phe Phe Gly Ser Leu Leu Glu Tyr Thr Ile Gln Asp Asn Leu Cys
            260                 265                 270

Asn Ile Thr Ala Val Ala Lys Gly Pro Gln Glu Ala Ala Arg Thr Asp
        275                 280                 285

Lys Thr Ser Thr Arg Arg Val Thr Ala Asn Ile Pro Ala Cys Val Phe
    290                 295                 300

Trp Asp Val Asp Lys Asp Leu His Leu Ser Ala Asp Gly Leu Lys His
305                 310                 315                 320

Val Phe Leu Val Phe Val Tyr Thr Gln Arg Arg Gln Arg Glu Gly Val
                325                 330                 335

Arg Leu His Leu Ala Leu Ser Gln Leu Asn Glu Gln Cys Phe Gly Arg
            340                 345                 350

Gly Ile Gly Phe Leu Leu Gly Arg Ile Arg Ala Glu Asn Ala Ala Trp
        355                 360                 365

Gly Thr Glu Gly Val Ala Asn Thr His Gln Pro Tyr Asn Thr Arg Ala
    370                 375                 380

Leu Pro Leu Val Gln Leu Ser Asn Asp Pro Thr Ser Pro Arg Cys Ser
385                 390                 395                 400

Ile Gly Glu Ile Thr Gly Val Asn Trp Asn Leu Ala Arg Gln Arg Leu
                405                 410                 415

Tyr Gln Trp Thr Gly Asp Phe Arg Gly Leu Pro Thr Gln Leu Ser Cys
            420                 425                 430

Met Tyr Ala Ala Tyr Thr Leu Ile Gly Thr Ile Pro Ser Glu Ser Val
        435                 440                 445

Arg Tyr Thr Arg Arg Met Glu Arg Phe Gly Gly Tyr Asn Val Pro Thr
    450                 455                 460

Ile Trp Leu Glu Gly Val Val Trp Gly Gly Thr Asn Thr Trp Asn Glu

465                 470                 475                 480

Cys Tyr Tyr

<210>  69
<211>  775
<212>  PRT
<213>  Varicella zoster

<400>  69

Met Glu Phe Lys Arg Ile Phe Asn Thr Val His Asp Ile Ile Asn Arg
1               5                   10                  15

Leu Cys Gln His Gly Tyr Lys Glu Tyr Ile Ile Pro Pro Glu Ser Thr
            20                  25                  30

Thr Pro Val Glu Leu Met Glu Tyr Ile Ser Thr Ile Val Ser Lys Leu
        35                  40                  45

Lys Ala Val Thr Arg Gln Asp Glu Arg Val Tyr Arg Cys Cys Gly Glu
    50                  55                  60

Leu Ile His Cys Arg Ile Asn Leu Arg Ser Val Ser Met Glu Thr Trp
65                  70                  75                  80

Leu Thr Ser Pro Ile Leu Cys Leu Thr Pro Arg Val Arg Gln Ala Ile
                85                  90                  95

Glu Gly Arg Arg Asp Glu Ile Arg Arg Ala Ile Leu Glu Pro Phe Leu
            100                 105                 110

Lys Asp Gln Tyr Pro Ala Leu Ala Thr Leu Gly Leu Gln Ser Ala Leu
        115                 120                 125

Lys Tyr Glu Asp Phe Tyr Leu Thr Lys Leu Glu Glu Gly Lys Leu Glu
    130                 135                 140

Ser Leu Cys Gln Phe Phe Leu Arg Leu Ala Ala Thr Val Thr Thr Glu
145                 150                 155                 160

Ile Val Asn Leu Pro Lys Ile Ala Thr Leu Ile Pro Gly Ile Asn Asp
                165                 170                 175

Gly Tyr Thr Trp Thr Asp Val Cys Arg Val Phe Phe Thr Ala Leu Ala
            180                 185                 190

Cys Gln Lys Ile Val Pro Ala Thr Pro Val Met Met Phe Leu Gly Arg
        195                 200                 205

Glu Thr Gly Ala Thr Ala Ser Cys Tyr Leu Met Asp Pro Glu Ser Ile
    210                 215                 220

Thr Val Gly Arg Ala Val Arg Ala Ile Thr Gly Asp Val Gly Thr Val
225                 230                 235                 240

Leu Gln Ser Arg Gly Gly Val Gly Ile Ser Leu Gln Ser Leu Asn Leu
                245                 250                 255

Ile Pro Thr Glu Asn Gln Thr Lys Gly Leu Leu Ala Val Leu Lys Leu
            260                 265                 270

Leu Asp Cys Met Val Met Ala Ile Asn Ser Asp Cys Glu Arg Pro Thr
        275                 280                 285

Gly Val Cys Val Tyr Ile Glu Pro Trp His Val Asp Leu Gln Thr Val
    290                 295                 300

Leu Ala Thr Arg Gly Met Leu Val Arg Asp Glu Ile Phe Arg Cys Asp
305                 310                 315                 320

Asn Ile Phe Cys Cys Leu Trp Thr Pro Asp Leu Phe Phe Glu Arg Tyr
                325                 330                 335

Leu Ser Tyr Leu Lys Gly Ala Ser Asn Val Gln Trp Thr Leu Phe Asp
            340                 345                 350

Asn Arg Ala Asp Ile Leu Arg Thr Leu His Gly Glu Ala Phe Thr Ser
        355                 360                 365

Thr Tyr Leu Arg Leu Glu Arg Glu Gly Leu Gly Val Ser Ser Val Pro
    370                 375                 380

Ile Gln Asp Ile Ala Phe Thr Ile Ile Arg Ser Ala Ala Val Thr Gly
385                 390                 395                 400

Ser Pro Phe Leu Met Phe Lys Asp Ala Cys Asn Arg Asn Tyr His Met
                405                 410                 415

Asn Thr Gln Gly Asn Ala Ile Thr Gly Ser Asn Leu Cys Thr Glu Ile
            420                 425                 430

Val Gln Lys Ala Asp Ala His Gln His Gly Val Cys Asn Leu Ala Ser
        435                 440                 445

Ile Asn Leu Thr Thr Cys Leu Ser Lys Gly Pro Val Ser Phe Asn Leu
    450                 455                 460

Asn Asp Leu Gln Leu Thr Ala Arg Thr Thr Val Ile Phe Leu Asn Gly
465                 470                 475                 480

Val Leu Ala Ala Gly Asn Phe Pro Cys Lys Lys Ser Cys Lys Gly Val
                485                 490                 495

Lys Asn Asn Arg Ser Leu Gly Ile Gly Ile Gln Gly Leu His Thr Thr
            500                 505                 510

Cys Leu Arg Leu Gly Phe Asp Leu Thr Ser Gln Pro Ala Arg Arg Leu
        515                 520                 525

Asn Val Gln Ile Ala Glu Leu Met Leu Tyr Glu Thr Met Lys Thr Ser
    530                 535                 540

Met Glu Met Cys Lys Ile Gly Gly Leu Ala Pro Phe Lys Gly Phe Thr
545                 550                 555                 560

Glu Ser Lys Tyr Ala Lys Gly Trp Leu His Gln Asp Gly Phe Ser Thr
                565                 570                 575

Ile Ser Tyr Leu Asp Leu Pro Trp Cys Thr Leu Arg Asp Asp Ile Cys
            580                 585                 590

Ala Tyr Gly Leu Tyr Asn Ser Gln Phe Leu Ala Leu Met Pro Thr Val
        595                 600                 605

Ser Ser Ala Gln Val Thr Glu Cys Ser Glu Gly Phe Ser Pro Ile Tyr
    610                 615                 620

Asn Asn Met Phe Ser Lys Val Thr Thr Ser Gly Glu Leu Leu Arg Pro
625                 630                 635                 640

Asn Leu Asp Leu Met Asp Glu Leu Arg Asp Met Tyr Ser Cys Glu Glu
                645                 650                 655

Lys Arg Leu Glu Val Ile Asn Ile Leu Glu Lys Asn Gln Trp Ser Val
            660                 665                 670

Ile Arg Ser Phe Gly Cys Leu Ser Asn Ser His Pro Leu Leu Lys Tyr
        675                 680                 685

Lys Thr Ala Phe Glu Tyr Glu Gln Glu Asp Leu Val Asp Met Cys Ala
    690                 695                 700

Glu Arg Ala Pro Phe Ile Asp Gln Ser Gln Ser Met Thr Leu Phe Ile
705                 710                 715                 720

Glu Glu Arg Pro Asp Gly Thr Ile Pro Ala Ser Lys Ile Met Asn Leu
                725                 730                 735

Leu Ile Arg Ala Tyr Lys Ala Gly Leu Lys Thr Gly Met Tyr Tyr Cys
            740                 745                 750

Lys Ile Arg Lys Ala Thr Asn Ser Gly Leu Phe Ala Gly Gly Glu Leu
        755                 760                 765

Thr Cys Thr Ser Cys Ala Leu
    770                 775


<210>  70
<211>  306
<212>  PRT
<213>  Varicella zoster

<400>  70

Met Asp Gln Lys Asp Cys Ser His Phe Phe Tyr Arg Pro Glu Cys Pro
1               5                   10                  15


Asp Ile Asn Asn Leu Arg Ala Leu Ser Ile Ser Asn Arg Trp Leu Glu
            20                  25                  30


Ser Asp Phe Ile Ile Glu Asp Asp Tyr Gln Tyr Leu Asp Cys Leu Thr
        35                  40                  45


Glu Asp Glu Leu Ile Phe Tyr Arg Phe Ile Phe Thr Phe Leu Ser Ala
    50                  55                  60


Ala Asp Asp Leu Val Asn Val Asn Leu Gly Ser Leu Thr Gln Leu Phe
65                  70                  75                  80


Ser Gln Lys Asp Ile His His Tyr Tyr Ile Glu Gln Glu Cys Ile Glu
                85                  90                  95


Val Val His Ala Arg Val Tyr Ser Gln Ile Gln Leu Met Leu Phe Arg
            100                 105                 110


Gly Asp Glu Ser Leu Arg Val Gln Tyr Val Asn Val Thr Ile Asn Asn
        115                 120                 125


Pro Ser Ile Gln Gln Lys Val Gln Trp Leu Glu Glu Lys Val Arg Asp
    130                 135                 140


Asn Pro Ser Val Ala Glu Lys Tyr Ile Leu Met Ile Leu Ile Glu Gly
145                 150                 155                 160


Ile Phe Phe Val Ser Ser Phe Ala Ala Ile Ala Tyr Leu Arg Asn Asn
                165                 170                 175


Gly Leu Phe Val Val Thr Cys Gln Phe Asn Asp Leu Ile Ser Arg Asp
            180                 185                 190


Glu Ala Ile His Thr Ser Ala Ser Cys Cys Ile Tyr Asn Asn Tyr Val
        195                 200                 205


Pro Glu Lys Pro Ala Ile Thr Arg Ile His Gln Leu Phe Ser Glu Ala
    210                 215                 220

Val Glu Ile Glu Cys Ala Phe Leu Lys Ser His Ala Pro Lys Thr Arg
225                 230                 235                 240

Leu Val Asn Val Asp Ala Ile Thr Gln Tyr Val Lys Phe Ser Ala Asp
                245                 250                 255

Arg Leu Leu Ser Ala Ile Asn Val Pro Lys Leu Phe Asn Thr Pro Pro
            260                 265                 270

Pro Asp Ser Asp Phe Pro Leu Ala Phe Met Ile Ala Asp Lys Asn Thr
        275                 280                 285

Asn Phe Phe Glu Arg His Ser Thr Ser Tyr Ala Gly Thr Val Ile Asn
    290                 295                 300

Asp Leu
305

<210>  71
<211>  408
<212>  PRT
<213>  Varicella zoster

<400>  71

Met Asp Leu Arg Ser Arg Thr Asp Asp Ala Leu Asp Met Glu Leu His
1               5                   10                  15

Ala Gly Phe Asp Ala Pro Glu Ile Ala Arg Ala Val Leu Thr Glu Lys
            20                  25                  30

Thr Leu Thr Gly Leu Ile Ser Ser Ile Ser Pro Leu Val Asn Arg Leu
        35                  40                  45

Arg Asp Ser Ile Leu Ile Phe Ser Asp Glu Gly Leu Ile Ile His Cys
    50                  55                  60

Ser Leu Glu Thr Glu Gln Leu Tyr Ile Pro Ile Pro Ala Asn Met Phe
65                  70                  75                  80

Asp Gln Tyr Asn Trp Thr Gly Pro Arg Met Val Val Leu Ala Ala Thr
                85                  90                  95

Glu Gly Arg Ser Ser Leu Ile Asp Ala Phe Arg His Thr Lys Asp Pro
            100                 105                 110

Ser Thr Pro Thr Arg Leu Tyr Phe Lys Phe Thr Gly Gln Pro Pro Glu
        115                 120                 125

Arg Ser Ile Ile Gln Thr Met Val Trp Gln Arg Pro Gly Asp Cys Gly
    130                 135                 140

Pro Asp Asp Gln Val Gln Cys Tyr Lys Gln Val Val Lys Arg Glu Leu
145                 150                 155                 160

Ala Cys Tyr Thr Met Met Phe Pro Asn Leu Thr Pro Asp Ile Ser Ile
                165                 170                 175

Cys Leu Lys Arg Asp Gln Phe Thr Arg Leu Gln Arg Leu Leu Lys Thr
            180                 185                 190

Phe Gly Phe Thr Thr Cys Phe Ile Leu Thr Ala Thr Asp Met Tyr Ile
        195                 200                 205

Gln Thr Ala Gly Gly Gly Phe Ile Ser Phe Asn Val Ser Leu Asp Ile
    210                 215                 220

Asn Gly Ser Lys Pro Thr Pro Tyr Asn Leu Ile Arg Ser Ile Thr Asn
225                 230                 235                 240

Ser Lys Arg Ile Leu Asn Asn Val Val Tyr Gly Ser Gly Ser Met Arg
                245                 250                 255

Glu Phe Gly Val Leu Leu Glu Thr His Ser Gly Phe Arg Ser Ala Val
            260                 265                 270

Gln Asn Leu Lys Leu Thr Arg Asp Glu Thr Cys Tyr Ile Asn Phe Tyr
        275                 280                 285

Leu Ala Leu Thr Asn Ser Pro Met Val Gly Leu Tyr Ile Gln Arg Ser
    290                 295                 300

Ala Pro Val His Ser Phe Phe Tyr Ala Thr Phe Leu Ser Pro Lys Asp
305                 310                 315                 320

Leu Lys Glu Lys Leu Thr Ser Met Gln Leu Phe Ala Asn Met Glu Ser
                325                 330                 335

Val Lys Asp Glu Pro Pro Leu Lys Lys Arg Arg Asn Leu Leu Thr Lys
            340                 345                 350

Arg Asn Glu Lys Asn Thr Gly Asn Lys Met Gly Gly Lys Leu Pro Glu
        355                 360                 365

Thr Thr Trp Gln Glu Gly Ile Gly Ile Arg Glu Tyr Cys Val Ala Pro
    370                 375                 380

Pro Val Asp Pro Ala Gly Thr Leu Asp Tyr Ser Glu Leu Ser Arg Glu
385                 390                 395                 400

Ser Asp Val Ile Cys Thr Val Lys
                405

<210>  72
<211>  406
<212>  PRT
<213>  Varicella zoster

<400>  72

Met Ala Val Asn Gly Glu Arg Ala Val His Asp Glu Asn Leu Gly Val
1               5                   10                  15

Leu Asp Arg Glu Leu Ile Arg Ala Gln Ser Ile Gln Gly Cys Val Gly
            20                  25                  30

Asn Pro Gln Glu Cys Asn Ser Cys Ala Ile Thr Ser Ala Ser Arg Leu
        35                  40                  45

Phe Leu Val Gly Leu Gln Ala Ser Val Ile Thr Ser Gly Leu Ile Leu
    50                  55                  60

Gln Tyr His Val Cys Glu Ala Ala Val Asn Ala Thr Ile Met Gly Leu
65                  70                  75                  80

Ile Val Val Ser Gly Leu Trp Pro Thr Ser Val Lys Phe Leu Arg Thr
                85                  90                  95

Leu Ala Lys Leu Gly Arg Cys Leu Gln Thr Val Val Val Leu Gly Phe
            100                 105                 110

Ala Val Leu Trp Ala Val Gly Cys Pro Ile Ser Arg Asp Leu Pro Phe
        115                 120                 125

Val Glu Leu Leu Gly Ile Ser Ile Ser Ala Ile Thr Gly Thr Val Ala
    130                 135                 140

Ala Val His Ile His Tyr Tyr Asn Phe Val Thr Thr Phe Asn Gly Pro
145                 150                 155                 160

His Ile Tyr Phe Tyr Val Met Met Leu Gly Thr Gly Leu Gly Gly Leu
                165                 170                 175

Leu Thr Val Ile Leu Tyr Met Tyr Val Ser Lys Tyr Glu Val Leu Ile
            180                 185                 190

Gly Leu Cys Ile Ser Ile Val Thr Leu Val Ser Ile Val Asp Ala Ala
        195                 200                 205

Thr Asp Leu Gln Asp Thr Cys Ile Tyr Arg Lys Asn Arg His Lys Gln
    210                 215                 220

Leu Asn Thr Tyr Thr Asp Leu Gly Phe Ala Val Val Tyr Thr Gln Asn
225                 230                 235                 240

Asp Arg Gly Arg Val Cys Asp His Arg Glu Ser Ser Arg Thr Leu Lys

245                     250                     255

Arg Val Phe Lys Gly Ile Arg Ile Met Ser Val Ile Pro Pro Val Leu
            260                 265                 270

Tyr Ile Val Thr Pro Leu Met Trp Ala Ile Ser His Ile Ile Lys Leu
        275                 280                 285

Asn His Phe Ile Lys Leu Thr Gln Val Thr Leu Ala Val Ser Ile Gly
    290                 295                 300

Gly His Ile Ile Ala Phe Gly Leu Gln Gly Phe Ala Val Leu Tyr Gln
305                 310                 315                 320

Glu Lys Lys Asn Leu Trp Val Ile Val Leu Tyr Thr Thr Thr Ser Val
                325                 330                 335

Thr Gly Ile Ala Val Thr Phe Ala Gly Ile Ser Trp Gly Ala Ile Ile
            340                 345                 350

Ile Leu Thr Ser Thr Val Ala Ala Gly Leu Thr Cys Ile Gln Met Met
        355                 360                 365

Arg Leu Ser Val Lys Pro Ile Asp Cys Phe Met Ala Ser His Ile Thr
    370                 375                 380

Lys Val Tyr His Val Cys Val Tyr Ile Ile Ile Asn Leu Cys Tyr Leu
385                 390                 395                 400

Cys Gly Thr Tyr Val Ser
                405

<210>  73
<211>  560
<212>  PRT
<213>  Varicella zoster

<400>  73

Met Lys Arg Ile Gln Ile Asn Leu Ile Leu Thr Ile Ala Cys Ile Gln
1               5                   10                  15

Leu Ser Thr Glu Ser Gln Pro Thr Pro Val Ser Ile Thr Glu Leu Tyr
            20                  25                  30

Thr Ser Ala Ala Thr Arg Lys Pro Asp Pro Ala Val Ala Pro Thr Ser
        35                  40                  45

Ala Ala Ser Arg Lys Pro Asp Pro Ala Val Ala Pro Thr Ser Ala Ala
    50                  55                  60

Ser Arg Lys Pro Asp Pro Ala Val Ala Pro Thr Ser Ala Ala Ser Arg
65                  70                  75                  80

Lys Pro Asp Pro Ala Val Ala Pro Thr Ser Ala Ala Thr Arg Lys Pro
                85                  90                  95

Asp Pro Ala Val Ala Pro Thr Ser Ala Ala Ser Arg Lys Pro Asp Pro
            100                 105                 110

Ala Val Ala Pro Thr Ser Ala Ala Thr Arg Lys Pro Asp Pro Ala Val
        115                 120                 125

Ala Pro Thr Ser Ala Ala Ser Arg Lys Pro Asp Pro Ala Ala Asn Thr
    130                 135                 140

Gln His Ser Gln Pro Pro Phe Leu Tyr Glu Asn Ile Gln Cys Val His
145                 150                 155                 160

Gly Gly Ile Gln Ser Ile Pro Tyr Phe His Thr Phe Ile Met Pro Cys
                165                 170                 175

Tyr Met Arg Leu Thr Thr Gly Gln Gln Ala Ala Phe Lys Gln Gln Gln
            180                 185                 190

Lys Thr Tyr Glu Gln Tyr Ser Leu Asp Pro Glu Gly Ser Asn Ile Thr
        195                 200                 205

Arg Trp Lys Ser Leu Ile Arg Pro Asp Leu His Ile Glu Val Trp Phe
    210                 215                 220

Thr Arg His Leu Ile Asp Pro His Arg Gln Leu Gly Asn Ala Leu Ile
225                 230                 235                 240

Arg Met Pro Asp Leu Pro Val Met Leu Tyr Ser Asn Ser Ala Asp Leu
                245                 250                 255

Asn Leu Ile Asn Asn Pro Glu Ile Phe Thr His Ala Lys Glu Asn Tyr
            260                 265                 270

Val Ile Pro Asp Val Lys Thr Thr Ser Asp Phe Ser Val Thr Ile Leu
        275                 280                 285

Ser Met Asp Ala Thr Thr Glu Gly Thr Tyr Ile Trp Arg Val Val Asn
    290                 295                 300

Thr Lys Thr Lys Asn Val Ile Ser Glu His Ser Ile Thr Val Thr Thr
305                 310                 315                 320

Tyr Tyr Arg Pro Asn Ile Thr Val Val Gly Asp Pro Val Leu Thr Gly
                325                 330                 335

Gln Thr Tyr Ala Ala Tyr Cys Asn Val Ser Lys Tyr Tyr Pro Pro His
            340                 345                 350

Ser Val Arg Val Arg Trp Thr Ser Arg Phe Gly Asn Ile Gly Lys Asn
        355                 360                 365

Phe Ile Thr Asp Ala Ile Gln Glu Tyr Ala Asn Gly Leu Phe Ser Tyr
    370                 375                 380

Val Ser Ala Val Arg Ile Pro Gln Gln Lys Gln Met Asp Tyr Pro Pro
385                 390                 395                 400

Pro Ala Ile Gln Cys Asn Val Leu Trp Ile Arg Asp Gly Val Ser Asn
                405                 410                 415

Met Lys Tyr Ser Ala Val Val Thr Pro Asp Val Tyr Pro Phe Pro Asn
            420                 425                 430

Val Ser Ile Gly Ile Ile Asp Gly His Ile Val Cys Thr Ala Lys Cys
        435                 440                 445

Val Pro Arg Gly Val Val His Phe Val Trp Trp Val Asn Asp Ser Pro
    450                 455                 460

Ile Asn His Glu Asn Ser Glu Ile Thr Gly Val Cys Asp Gln Asn Lys
465                 470                 475                 480

Arg Phe Val Asn Met Gln Ser Ser Cys Pro Thr Ser Glu Leu Asp Gly
                485                 490                 495

Pro Ile Thr Tyr Ser Cys His Leu Asp Gly Tyr Pro Lys Lys Phe Pro
            500                 505                 510

Pro Phe Ser Ala Val Tyr Thr Tyr Asp Ala Ser Thr Tyr Ala Thr Thr
        515                 520                 525

Phe Ser Val Val Ala Val Ile Ile Gly Val Ile Ser Ile Leu Gly Thr
    530                 535                 540

Leu Gly Leu Ile Ala Val Ile Ala Thr Leu Cys Ile Arg Cys Cys Ser
545                 550                 555                 560

<210>  74
<211>  396
<212>  PRT
<213>  Varicella zoster

<400>  74

Met Asn Glu Ala Val Ile Asp Pro Ile Leu Glu Thr Ala Val Asn Thr
1               5                   10                  15

Gly Asp Met Phe Cys Ser Gln Thr Ile Pro Asn Arg Cys Leu Lys Asp
            20                  25                  30

Thr Ile Leu Ile Glu Val Gln Pro Glu Cys Ala Asp Thr Leu Gln Cys
        35                  40                  45

Val Leu Asp Asp Lys Val Ser Arg His Gln Pro Leu Leu Leu Arg Asn
    50                  55                  60

His Lys Lys Leu Glu Leu Pro Ser Glu Lys Ser Val Thr Arg Gly Gly
65                  70                  75                  80

Phe Tyr Met Gln Gln Leu Glu Leu Leu Val Lys Ser Ala Pro Pro Asn
                85                  90                  95

Glu Tyr Ala Leu Leu Leu Ile Gln Cys Lys Asp Thr Ala Leu Ala Asp
            100                 105                 110

Glu Asp Asn Phe Phe Val Ala Asn Gly Val Ile Asp Ala Gly Tyr Arg
        115                 120                 125

Gly Val Ile Ser Ala Leu Leu Tyr Tyr Arg Pro Gly Val Thr Val Ile
    130                 135                 140

Leu Pro Gly His Leu Thr Ile Tyr Leu Phe Pro Val Lys Leu Arg Gln
145                 150                 155                 160

Ser Arg Leu Leu Pro Lys Asn Val Leu Lys His Leu Asp Pro Ile Phe
                165                 170                 175

Lys Ser Ile Gln Val Gln Pro Leu Ser Asn Ser Pro Ser Asn Tyr Glu
            180                 185                 190

Lys Pro Val Ile Pro Glu Phe Ala Asp Ile Ser Thr Val Gln Gln Gly
        195                 200                 205

Gln Pro Leu His Arg Asp Ser Ala Glu Tyr His Ile Asp Val Pro Leu
    210                 215                 220

Thr Tyr Lys His Ile Ile Asn Pro Lys Arg Gln Glu Asp Ala Gly Tyr
225                 230                 235                 240

Asp Ile Cys Val Pro Tyr Asn Leu Tyr Leu Lys Arg Asn Glu Phe Ile
                245                 250                 255

Lys Ile Val Leu Pro Ile Ile Arg Asp Trp Asp Leu Gln His Pro Ser
            260                 265                 270

Ile Asn Ala Tyr Ile Phe Gly Arg Ser Ser Lys Ser Arg Ser Gly Ile
        275                 280                 285

Ile Val Cys Pro Thr Ala Trp Pro Ala Gly Glu His Cys Lys Phe Tyr
    290                 295                 300

Val Tyr Asn Leu Thr Gly Asp Asp Ile Arg Ile Lys Thr Gly Asp Arg
305                 310                 315                 320

Leu Ala Gln Val Leu Leu Ile Asp His Asn Thr Gln Ile His Leu Lys
                325                 330                 335

His Asn Val Leu Ser Asn Ile Ala Phe Pro Tyr Ala Ile Arg Gly Lys
            340                 345                 350

Cys Gly Ile Pro Gly Val Gln Trp Tyr Phe Thr Lys Thr Leu Asp Leu
        355                 360                 365

Ile Ala Thr Pro Ser Glu Arg Gly Thr Arg Gly Phe Gly Ser Thr Asp
    370                 375                 380

Lys Glu Thr Asn Asp Val Asp Phe Leu Leu Lys His
385                 390                 395

<210>  75
<211>  1083
<212>  PRT
<213>  Varicella zoster

<400>  75

Met Asp Lys Ser Ser Lys Pro Thr Ile Arg Leu Leu Phe Ala Thr Lys
1               5                   10                  15

Gly Cys Ala Ile Ser His Ser Leu Leu Leu Leu Thr Gly Gln Ile Ser
            20                  25                  30

Thr Glu Pro Leu Tyr Val Val Ser Tyr Thr Trp Thr Pro Asp Leu Asp
        35                  40                  45

Asp Val Phe Val Lys Asn Gly Arg Glu Glu Ile Thr Gln Val Ile Pro
    50                  55                  60

Thr Lys Arg Pro Arg Glu Val Thr Glu Asn Asp Glu Glu Asn Gln Ile
65                  70                  75                  80

Met His Leu Phe Cys Ser Arg Asp Val Asn Val Ile Phe Tyr Leu Ile
                85                  90                  95

Gly Gly Phe Ser Thr Gly Asp Val Arg Ser Arg Val Trp Pro Ile Phe
            100                 105                 110

Phe Cys Cys Phe Lys Thr Gln Thr Asp Phe Lys Ala Leu Tyr Lys Ala
        115                 120                 125

Leu Trp Tyr Gly Ala Pro Leu Asn Pro His Ile Ile Ser Asp Thr Leu
    130                 135                 140

Cys Ile Ser Glu Thr Phe Asp Ile His Ser Glu Val Ile Gln Thr Leu
145                 150                 155                 160

Met Val Thr Thr His His Leu Asn Arg Lys Gly Leu Ser Asp Asn Gly
                165                 170                 175

Leu Cys Ile Thr Glu Ala Thr Leu Cys Lys Leu Val Lys Lys Ser Val
            180                 185                 190

Gly Arg Gln Glu Leu Thr Ser Leu Tyr Ala His Tyr Glu Arg Gln Val
        195                 200                 205

Leu Ala Ala Tyr Arg Arg Leu Tyr Trp Gly Tyr Gly Cys Ser Pro Phe
    210                 215                 220

Trp Tyr Ile Val Arg Phe Gly Pro Ser Glu Lys Thr Leu Val Leu Ala
225                 230                 235                 240

Thr Arg Tyr Tyr Leu Leu Gln Thr Asp Thr Ser Tyr Asn Thr Leu Glu
                245                 250                 255

Thr Pro Leu Tyr Asp Leu Gln Ala Ile Lys Asp Leu Phe Leu Thr Tyr
            260                 265                 270

Gln Val Pro Ala Leu Pro Asn Cys Ser Gly Tyr Asn Ile Ser Asp Leu
        275                 280                 285

Leu Ser Phe Asp Lys Leu Ser Met Phe Cys Cys Ser Ser Thr Tyr Thr
    290                 295                 300

Arg Gly Leu Thr Ala Lys Asn Ala Leu Ser Tyr Ile Leu Gln Arg Ile
305                 310                 315                 320

His Thr Asp Thr Thr Glu Ile His Ala Val Ser Glu Tyr Ile Thr Asn
                325                 330                 335

Asp Arg Lys Gly Leu Lys Val Pro Asp Arg Glu Phe Val Asp Tyr Ile
            340                 345                 350

Tyr Leu Ala His Phe Glu Cys Phe Asn Arg Lys Gln Ile Ala Asp His
        355                 360                 365

Leu Gln Ala Val Thr Tyr Ser Asp Phe Val Asn Lys Pro Val Leu Leu
    370                 375                 380

Lys Ser Ser Asn Leu Gly Lys Arg Ala Thr Ala Asn Phe Phe Asn His
385                 390                 395                 400

Val Arg Ser Arg Leu Asn Met Arg Asp Tyr Ile Lys Lys Asn Val Ile
                405                 410                 415

Cys Asp Val Thr Glu Leu Gly Pro Glu Ile Gly His Lys Tyr Thr Ile
            420                 425                 430

Thr Lys Thr Tyr Thr Leu Ser Leu Thr Tyr Ala Ala Lys Pro Ser Lys
        435                 440                 445

Phe Ile Gly Val Cys Asp Leu Ala Thr Thr Leu Thr Arg Arg Val Glu
    450                 455                 460

Asn Ile Glu Lys Gln Phe Ser Pro Tyr Gly Trp Ser Ser Thr Ile Pro
465                 470                 475                 480

Ser Asn Pro Pro Gly Phe Asp Glu Leu Ser Asn Phe Glu Asp Ser Gly
                485                 490                 495

Val Ser Ala Glu Ala Leu Arg Ala Ala Asn Phe Ala Asn Asp Thr Pro
            500                 505                 510

Asn Gln Ser Gly Arg Thr Gly Phe Asp Thr Ser Pro Gly Ile Thr Lys
        515                 520                 525

Leu Leu Leu Phe Phe Ser Ala Ala Thr Gly Ile Ala Thr His Asp Val
    530                 535                 540

Ser Ile Leu Ser Tyr Lys Thr Pro Leu Glu Ala Leu Ile Gly His Ser
545                 550                 555                 560

Glu Val Thr Gly Pro Met Pro Val Tyr Arg Val Ala Leu Pro His Gly
                565                 570                 575

Ala Gln Ala Phe Ala Val Ile Ala Asn Asp Thr Trp Ser Ser Ile Thr
            580                 585                 590

Asn Arg Tyr Thr Leu Pro His Glu Ala Arg Leu Ile Ala Glu Asp Leu
        595                 600                 605

Lys Gln Ile Asn Pro Cys Asn Phe Val Ala Ala Ser Leu Arg Asp Met
    610                 615                 620

Gln Leu Thr Leu Leu Leu Ser Thr Ser Val Lys Asn Val Ser Lys Ile
625                 630                 635                 640

Ser Ser Asn Ile Pro Lys Asp Gln Leu Tyr Ile Asn Arg Asn Glu Leu
                645                 650                 655

Phe Asn Thr Asn Leu Ile Ile Thr Asn Leu Ile Leu Asp Val Asp Phe
            660                 665                 670

His Ile Arg Lys Pro Ile Pro Leu Gly Ile Leu His Ala Gly Met Arg
        675                 680                 685

Ala Phe Arg His Gly Ile Leu Thr Ala Met Gln Leu Leu Phe Pro Lys
    690                 695                 700

Ala Val Val Asn Pro Asn Lys Asp Pro Cys Tyr Phe Tyr Lys Thr Ala
705                 710                 715                 720

Cys Pro Glu Pro Thr Val Glu Val Leu Asp Asp Asp Asn Leu Leu Asp
                725                 730                 735

Ile Thr Ser His Ser Asp Ile Asp Phe Tyr Ile Glu Asn Gly Glu Leu
            740                 745                 750

Tyr Thr Cys Val Glu Glu Asn Tyr Thr Glu Asp Val Trp Phe Phe Asp
        755                 760                 765

Thr Gln Thr Thr Ser Glu Val His Thr His Ala Asp Val Ser Asn Asn
    770                 775                 780

Glu Asn Leu His Glu Thr Leu Pro Cys Asn Cys Lys Glu Lys Ile Gly
785                 790                 795                 800

Phe Arg Val Cys Val Pro Ile Pro Asn Pro Tyr Ala Leu Val Gly Ser
                805                 810                 815

Ser Thr Leu Lys Gly Phe Ala Gln Ile Leu Gln Gln Ala Val Leu Leu
            820                 825                 830

Glu Arg Glu Phe Val Glu Tyr Ile Gly Pro Tyr Leu Arg Asp Phe Ser
        835                 840                 845

Phe Ile Asp Thr Gly Val Tyr Ser His Gly His Ser Leu Arg Leu Pro
    850                 855                 860

Phe Phe Ser Lys Val Thr Thr Thr Gly Thr Ala Val Gly Gln Leu Leu
865                 870                 875                 880

Pro Phe Tyr Val Val Pro Glu Gln Cys Ile Asp Ile Leu Ala Phe Val
                885                 890                 895

Thr Ser His Arg Asn Pro Ala Asn Phe His Phe His Ser Arg Pro Gln
            900                 905                 910

Ser Asn Val Pro Val Gln Phe Ile Leu His Asn Leu Gly Gly Glu Tyr
        915                 920                 925

Ala Glu Phe Phe Glu Arg Lys Val Ala Arg Asn Lys Gln Ile Phe Ser
    930                 935                 940

Ser Pro Gln Ile Ser Leu Thr Lys Ala Leu Lys Glu Arg Gly Val Thr
945                 950                 955                 960

Cys Leu Asp Ala Phe Thr Leu Glu Ala Phe Val Asp Ser Thr Ile Leu
                965                 970                 975

Glu Ser Ile Val Glu His Ile Ala Val His Phe Pro Gly Arg Asp Arg
            980                 985                 990

Glu Tyr Thr Leu Thr Ser Ser Lys  Cys Ile Ala Ile Lys  Arg Asp Trp
        995                 1000                 1005

Val Leu  Phe Gln Leu Ile Cys  Gly Thr Lys Gly Phe  Thr Cys Leu
    1010                 1015                 1020

Arg Tyr  Pro His Arg Gly Gly  Arg Thr Ala Pro Arg  Thr Phe Val
    1025                 1030                 1035

Ser Leu  Arg Val Asp His His  Asn Arg Leu Cys Ile  Ser Leu Ala
    1040                 1045                 1050

Gln Gln  Cys Phe Ala Thr Lys  Cys Asp Ser Asn Arg  Met His Thr
    1055                 1060                 1065

Ile Phe  Thr Leu Glu Val Pro  Asn Tyr Pro Asn Leu  Thr Ser Ser
    1070                 1075                 1080

<210>  76
<211>  340
<212>  PRT
<213>  Varicella zoster

<400>  76

Met Gln Ala Leu Gly Ile Lys Thr Glu His Phe Ile Ile Met Cys Leu
1               5                   10                  15

Leu Ser Gly His Ala Val Phe Thr Leu Trp Tyr Thr Ala Arg Val Lys
            20                  25                  30

Phe Glu His Glu Cys Val Tyr Ala Thr Thr Val Ile Asn Gly Gly Pro
        35                  40                  45

Val Val Trp Gly Ser Tyr Asn Asn Ser Leu Ile Tyr Val Thr Phe Val
    50                  55                  60

Asn His Ser Thr Phe Leu Asp Gly Leu Ser Gly Tyr Asp Tyr Ser Cys
65                  70                  75                  80

Arg Glu Asn Leu Leu Ser Gly Asp Thr Met Val Lys Thr Ala Ile Ser
                85                  90                  95

Thr Pro Leu His Asp Lys Ile Arg Ile Val Leu Gly Thr Arg Asn Cys
            100                 105                 110

His Ala Tyr Phe Trp Cys Val Gln Leu Lys Met Ile Phe Phe Ala Trp

115                 120                 125

Phe Val Tyr Gly Met Tyr Leu Gln Phe Arg Arg Ile Arg Arg Met Phe
    130                 135                 140

Gly Pro Phe Arg Ser Ser Cys Glu Leu Ile Ser Pro Thr Ser Tyr Ser
145                 150                 155                 160

Leu Asn Tyr Val Thr Arg Val Ile Ser Asn Ile Leu Leu Gly Tyr Pro
                165                 170                 175

Tyr Thr Lys Leu Ala Arg Leu Leu Cys Asp Val Ser Met Arg Arg Asp
            180                 185                 190

Gly Met Ser Lys Val Phe Asn Ala Asp Pro Ile Ser Phe Leu Tyr Met
        195                 200                 205

His Lys Gly Val Thr Leu Leu Met Leu Leu Glu Val Ile Ala His Ile
    210                 215                 220

Ser Ser Gly Cys Ile Val Leu Leu Thr Leu Gly Val Ala Tyr Thr Pro
225                 230                 235                 240

Cys Ala Leu Leu Tyr Pro Thr Tyr Ile Arg Ile Leu Ala Trp Val Val
                245                 250                 255

Val Cys Thr Leu Ala Ile Val Glu Leu Ile Ser Tyr Val Arg Pro Lys
            260                 265                 270

Pro Thr Lys Asp Asn His Leu Asn His Ile Asn Thr Gly Gly Ile Arg
        275                 280                 285

Gly Ile Cys Thr Thr Cys Cys Ala Thr Val Met Ser Gly Leu Ala Ile
    290                 295                 300

Lys Cys Phe Tyr Ile Val Ile Phe Ala Ile Ala Val Val Ile Phe Met
305                 310                 315                 320

His Tyr Glu Gln Arg Val Gln Val Ser Leu Phe Gly Glu Ser Glu Asn
                325                 330                 335

Ser Gln Lys His
            340

<210>  77
<211>  452
<212>  PRT
<213>  Varicella zoster

<400>  77
   Met Ala Ser Ala Ser Ile Pro Thr Asp Pro Asp Val Ser Thr Ile Cys
   1               5                   10                  15
   Glu Asp Phe Met Asn Leu Leu Pro Asp Glu Pro Ser Asp Asp Phe Ala
               20                  25                  30
   Leu Glu Val Thr Asp Trp Ala Asn Asp Glu Ala Ile Gly Ser Thr Pro
           35                  40                  45
   Gly Glu Asp Ser Thr Thr Ser Arg Thr Val Tyr Val Glu Arg Thr Ala
       50                  55                  60
   Asp Thr Ala Tyr Asn Pro Arg Tyr Ser Lys Arg Arg His Gly Arg Arg
   65                  70                  75                  80
   Glu Ser Tyr His His Asn Arg Pro Lys Thr Leu Val Val Val Leu Pro
                   85                  90                  95
   Asp Ser Asn His His Gly Gly Arg Asp Val Glu Thr Gly Tyr Ala Arg
               100                 105                 110
   Ile Glu Arg Gly His Arg Arg Ser Ser Arg Ser Tyr Asn Thr Gln Ser
           115                 120                 125
   Ser Arg Lys His Arg Asp Arg Ser Leu Ser Asn Arg Arg Arg Arg Pro
       130                 135                 140
   Thr Thr Pro Pro Ala Met Thr Thr Gly Glu Arg Asn Asp Gln Thr His
   145                 150                 155                 160
   Asp Glu Ser Tyr Arg Leu Arg Phe Ser Lys Arg Asp Ala Arg Arg Glu
                   165                 170                 175
   Arg Ile Arg Lys Glu Tyr Asp Ile Pro Val Asp Arg Ile Thr Gly Arg
               180                 185                 190
   Ala Ile Glu Val Val Ser Thr Ala Gly Ala Ser Val Thr Ile Asp Ser
           195                 200                 205
   Val Arg His Leu Asp Glu Thr Ile Glu Lys Leu Val Val Arg Tyr Ala
       210                 215                 220
   Thr Ile Gln Glu Gly Asp Ser Trp Ala Ser Gly Gly Cys Phe Pro Gly
   225                 230                 235                 240
   Ile Lys Gln Asn Thr Ser Trp Pro Glu Leu Met Leu Tyr Gly His Glu
                   245                 250                 255
   Leu Tyr Arg Thr Phe Glu Ser Tyr Lys Met Asp Ser Arg Ile Ala Arg
               260                 265                 270
   Ala Leu Arg Glu Arg Val Ile Arg Gly Glu Ser Leu Ile Glu Ala Leu
           275                 280                 285
   Glu Ser Ala Asp Glu Leu Leu Thr Trp Ile Lys Met Leu Ala Ala Lys
       290                 295                 300
   Asn Leu Pro Ile Tyr Thr Asn Asn Pro Ile Val Ala Thr Ser Lys Ser
   305                 310                 315                 320
   Leu Leu Glu Asn Leu Lys Leu Lys Leu Gly Pro Phe Val Arg Cys Leu
                   325                 330                 335
   Leu Leu Asn Arg Asp Asn Asp Leu Gly Ser Arg Thr Leu Pro Glu Leu
               340                 345                 350
   Leu Arg Gln Gln Arg Phe Ser Asp Ile Thr Cys Ile Thr Thr Tyr Met
           355                 360                 365
   Phe Val Met Ile Ala Arg Ile Ala Asn Ile Val Val Arg Gly Ser Lys
       370                 375                 380
   Phe Val Glu Tyr Asp Asp Ile Ser Cys Asn Val Gln Val Leu Gln Glu
   385                 390                 395                 400
   Tyr Thr Pro Gly Ser Cys Leu Ala Gly Val Leu Glu Ala Leu Ile Thr
                   405                 410                 415
   His Gln Arg Glu Cys Gly Arg Val Glu Cys Thr Leu Ser Thr Trp Ala
               420                 425                 430
   Gly His Leu Ser Asp Ala Arg Pro Tyr Gly Lys Tyr Phe Lys Cys Ser
           435                 440                 445
   Thr Phe Asn Cys
       450


<210>  78
<211>  179
<212>  PRT
<213>  Varicella zoster

<400>  78

Met Asp Thr Thr Gly Ala Ser Glu Ser Ser Gln Pro Ile Arg Val Asn
1               5                   10                  15

Leu Lys Pro Asp Pro Leu Ala Ser Phe Thr Gln Val Ile Pro Pro Leu
            20                  25                  30

Ala Leu Glu Thr Thr Trp Thr Cys Pro Ala Asn Ser His Ala Pro Thr
        35                  40                  45

Pro Ser Pro Leu Tyr Gly Val Lys Arg Leu Cys Ala Leu Arg Ala Thr
    50                  55                  60

Cys Gly Arg Ala Asp Asp Leu His Ala Phe Leu Ile Gly Leu Gly Arg
65                  70                  75                  80

Arg Asp Lys Pro Ser Glu Ser Pro Met Tyr Val Asp Leu Gln Pro Phe
                85                  90                  95

Cys Ser Leu Leu Asn Ser Gln Arg Leu Leu Pro Glu Met Ala Asn Tyr
            100                 105                 110

Asn Thr Leu Cys Asp Ala Pro Phe Ser Ala Ala Thr Gln Gln Met Met
        115                 120                 125

Leu Glu Ser Gly Gln Leu Gly Val His Leu Ala Ala Ile Gly Tyr His
    130                 135                 140

Cys His Cys Lys Ser Pro Phe Ser Ala Glu Cys Trp Thr Gly Ala Ser
145                 150                 155                 160

Glu Ala Tyr Asp His Val Val Cys Gly Gly Lys Ala Arg Ala Ala Val
                165                 170                 175

Gly Gly Leu

<210>  79
<211>  108
<212>  PRT
<213>  Varicella zoster

<400>  79

Met Ser Arg Val Ser Glu Tyr Gly Val Pro Glu Gly Val Arg Glu Ser
1               5                   10                  15

Asp Ser Asp Thr Asp Ser Val Phe Met Tyr Gln His Thr Glu Leu Met
            20                  25                  30

Gln Asn Asn Ala Ser Pro Leu Val Val Gln Thr Arg Pro Pro Ala Val
        35                  40                  45

Leu Ile Pro Leu Val Asp Val Pro Arg Pro Arg Ser Arg Arg Lys Ala
    50                  55                  60

Ser Ala Gln Leu Lys Met Gln Met Asp Arg Leu Cys Asn Val Leu Gly
65                  70                  75                  80

Val Val Leu Gln Met Ala Thr Leu Ala Leu Val Thr Tyr Ile Ala Phe
                85                  90                  95

Val Val His Thr Arg Ala Thr Ser Cys Lys Arg Glu
            100                 105

<210>  80
<211>  5743
<212>  DNA
<213>  Varicella zoster

<220>
<221>  CDS
<222>  (1)..(1056)

<220>
<221>  CDS
<222>  (4556)..(5740)

<400>  80
atg tca ttg ata atg ttt ggt cgt acg ctt ggt gaa gaa tct gta aga      48
Met Ser Leu Ile Met Phe Gly Arg Thr Leu Gly Glu Glu Ser Val Arg
1               5                   10                  15

tat ttt gaa cgt cta aag cgt cgt agg gat gaa cgc ttt ggg acg ttg      96
Tyr Phe Glu Arg Leu Lys Arg Arg Arg Asp Glu Arg Phe Gly Thr Leu
            20                  25                  30

gag tcc cct acc ccg tgt tcc acg cgg caa ggg tct ctg gga aac gca      144
Glu Ser Pro Thr Pro Cys Ser Thr Arg Gln Gly Ser Leu Gly Asn Ala
        35                  40                  45

acc caa atc ccg ttt ctg aat ttt gct ata gat gta acc cga cgt cat      192
Thr Gln Ile Pro Phe Leu Asn Phe Ala Ile Asp Val Thr Arg Arg His
    50                  55                  60

cag gcc gtt att ccc gga att gga acg ctt cac aac tgt tgt gaa tat      240
Gln Ala Val Ile Pro Gly Ile Gly Thr Leu His Asn Cys Cys Glu Tyr
65                  70                  75                  80

att cca ctg ttc tcg gct act gct cga cgg gca atg ttt ggc gcg ttt      288
Ile Pro Leu Phe Ser Ala Thr Ala Arg Arg Ala Met Phe Gly Ala Phe
                85                  90                  95

cta tcg tca aca ggg tac aac tgt acc ccc aat gta gtt ttg aaa cca      336
Leu Ser Ser Thr Gly Tyr Asn Cys Thr Pro Asn Val Val Leu Lys Pro
            100                 105                 110

tgg cga tat tcg gta aat gca aac gta agc cct gaa tta aaa aag gct      384
Trp Arg Tyr Ser Val Asn Ala Asn Val Ser Pro Glu Leu Lys Lys Ala
        115                 120                 125

gtc agt agt gta cag ttt tat gaa tat tca ccg gaa gaa gca gca cct      432
Val Ser Ser Val Gln Phe Tyr Glu Tyr Ser Pro Glu Glu Ala Ala Pro
    130                 135                 140

cat cga aat gcg tat agc ggt gtt atg aac aca ttt cgc gcg ttt tct      480
His Arg Asn Ala Tyr Ser Gly Val Met Asn Thr Phe Arg Ala Phe Ser
145                 150                 155                 160

ctg tcg gat agt ttc tgt cag ttg tct acc ttt aca caa cgg ttt tcg      528
Leu Ser Asp Ser Phe Cys Gln Leu Ser Thr Phe Thr Gln Arg Phe Ser
                165                 170                 175

tac ctt gtg gaa aca tct ttt gag agt att gaa gag tgt gga agt cat      576
Tyr Leu Val Glu Thr Ser Phe Glu Ser Ile Glu Glu Cys Gly Ser His
            180                 185                 190

ggc aaa cgc gca aag gtt gac gtt cca atc tat ggc aga tat aag ggg      624
Gly Lys Arg Ala Lys Val Asp Val Pro Ile Tyr Gly Arg Tyr Lys Gly
        195                 200                 205

acg ttg gaa ctg ttt caa aaa atg atc ctc atg cac acc acg cat ttt      672

Thr Leu Glu Leu Phe Gln Lys Met Ile Leu Met His Thr Thr His Phe
    210                 215                 220

att tca tcg gtg cta ttg ggc gat cat gcc gac aga gtt gac tgc ttt      720
Ile Ser Ser Val Leu Leu Gly Asp His Ala Asp Arg Val Asp Cys Phe
225                 230                 235                 240

ctg cgt aca gtg ttt aac acg cca agt gtt tct gac agt gtt tta gaa      768
Leu Arg Thr Val Phe Asn Thr Pro Ser Val Ser Asp Ser Val Leu Glu
                245                 250                 255

cac ttc aaa caa aaa tca act gtg ttt ttg gta cca cgt aga cat ggg      816
His Phe Lys Gln Lys Ser Thr Val Phe Leu Val Pro Arg Arg His Gly
            260                 265                 270

aaa aca tgg ttt ctt gta cca tta ata gct tta gta atg gcc acg ttt      864
Lys Thr Trp Phe Leu Val Pro Leu Ile Ala Leu Val Met Ala Thr Phe
        275                 280                 285

aga gga att aaa gtg ggt tat acg gct cat ata cgc aaa gca acg gaa      912
Arg Gly Ile Lys Val Gly Tyr Thr Ala His Ile Arg Lys Ala Thr Glu
    290                 295                 300

ccc gtg ttt gag ggt atc aag tct cgc ctg gaa cag tgg ttt ggg gca      960
Pro Val Phe Glu Gly Ile Lys Ser Arg Leu Glu Gln Trp Phe Gly Ala
305                 310                 315                 320

aat tac gtg gat cat gta aaa ggc gaa tct att acg ttt tca ttt acc     1008
Asn Tyr Val Asp His Val Lys Gly Glu Ser Ile Thr Phe Ser Phe Thr
                325                 330                 335

gac ggg tct tac agc aca gcg gtg ttc gcg tca agt cac aac aca aac     1056
Asp Gly Ser Tyr Ser Thr Ala Val Phe Ala Ser Ser His Asn Thr Asn
            340                 345                 350

gtgagtgttt tataaattta acctttaata tattactgta aatgttgaca tatacctttc     1116

cacaacggcg gttgagttaa ggtatactag gtggttgtag gttccggttc acccgataat     1176

ctttgtgtct cggggaagca aattcgctga agcagaccac agccgttaat aatagcccgg     1236

cttaatgttt ctccaaacat ataaagctgc cacccagatg aatttactgg tacagagaga     1296

ccactggcgt tggttcccgc tataacgtcg ccaagatttg cggtaatgcg aggattttta     1356

gtactcgtaa ttcgaatgca ggtggtgaca tctacaaaaa gaacctgcgt ggcgccaatg     1416

tctacctcca cttttaattc ccgctgaccg gcctttctcc acatacacgg agcccaacac     1476

acacaacctt ccgcatgatt tgtgacatgg ggtaacgcat acagtgcccc cacgtgaact     1536

ctatgattac attcatcaca tccgtccgca tggctgagga gtcgatttaa tacagagcca     1596

agtatccgag catgccatcc ggcgggacat agccctatta aattaggttc catagccagt     1656

acatataaac gccttcgttc gtctgaccac cacactcccg gagaaataac tttacatgcg     1716

tatggatttt tcggaagccg cgggggttgt aagtagttgc ttaagtttgg cgttggtgta     1776

agatctgcgg gggtgggatc tgctcgagga tccggaatag atgttggaag ggggtacgcg     1836

atcgggttct taaacgttgc tccaaaaaca tggtctatgt tttcaaccgg ataaattctt     1896

aaagtcgccg tcattgcgta cgagacctcg taattaaaat ttacaattac atgaaaagtc     1956

ttcggaggta agttcatctg acgtgggcgc gtgatgtaaa ttgtggctac aacaacggca     2016

atattagtag tatccgtttg aagggggata aacggagcga tccttaaagt tataaaagca     2076

gttgatcgca ttattttcac ccggggatcg gtcaggatgg acttccataa tcccatatcc 2136
agcgttaatg catcgcagag tctctgaact gcctcggggg ttaatttgcg cgctgcaccc 2196
gtagcggtgt acagcggaaa tatgcgttgt aattccatga gcagaaaaac agtctagcgg 2256
attgccgccc gggtacttgt gggtttaatg ccacccaccc ggttatttta tattttaaga 2316
gggggtggaa acgggagaaa tgacgtaaaa ttacatatga agagattctg gtgttatgtt 2376
tttatagtga cactaattta tttatggggg ttgggaatag agaagcagaa tctgtctaga 2436
ataggtccga ttaacgatgc aggtagtgct gcctgtaggg tatcggtaat acaaaaacat 2496
gccgcaaatc cccccggtaa aactaaaatg gattgtaatt gctggttaaa tcctagacaa 2556
atgtacgcgt aacattgacc gggtaaatac ttagaacaaa ttccaatatc aacaatatcc 2616
gcgctgcgta taaatttacc cctcagttgt gtggaattac caataccaac cttttctaag 2676
gctacgggaa cggggacctt ggaaagctta agtatttccc ctcctgaatt ataatagtca 2736
aataaatata tagaacgatt acctaaccag catgggaagg aagcgtgaag gtagggtata 2796
taccccccac cttgtggtcg tgtatataca gatgacagat acgccaaaac cgcatacatc 2856
aaggagctgt tataaaacgc atccattgac atttccgtta acaccgaaac tatagtctga 2916
atcaggtctg gtgtgcgggc tacaatttca tcaataaccg tttgggaaga atctgcaata 2976
tcatattcca tgagttgttg tagagtcggg ttcgtttgta actccgttat aagaccttgg 3036
gttagcgatg tcacacacgc ttgtttaaat acctggttta aaaacatttc ggcgcctggt 3096
ttaaaggcgg gtaagggggt ttgattattt aggacgttag ccaaaaacgg taaacgcgcg 3156
actagctctt ggcgagctgt cacatgtagg ctttggggat tgtcaacccg ggcatttata 3216
cacgcagcat caataatagc ctgtgcagag tgatataaaa ttggacttcc ggtaatacgt 3276
cctccccagg cagaggatcc gttgtaagat actacaatca acggactggg ggattctgcg 3336
taatgtcgcg gtacaattga taggggacgc cgtttccaga aatctgctgg agtgtccccg 3396
ctaactaatt gggcataaca gatgtcgaac cattccataa gactttgggg ttctgtcgaa 3456
gctggggtaa acaatagaac gtcttgtaaa ggtgggatgc tggcggacga attgtttttc 3516
tttcccgtaa atcgcccttg tccaggcggc tcaaggacgc catcaaagga accgttattg 3576
atcggatctg tgttggaagt ttgcgctccg tggccctttg cactttgaag caacccagat 3636
gcaacgcggg aactagaagg tcggacgggg tgcctggagt taacaatgtt tacggcccgt 3696
tttattagct caaggacgtc ccgattattt tcctgtatgc gtgtttcagc aggggagtca 3756
tcaatacctc cagaagttaa ctgtcgatca agatcgatta tggatgaaac gggtccaata 3816
ttgtccccat ttgacgtgtg tgattcaccc atggctgcca ccatatgctc tgcgtatatt 3876
tttatagacg atgcaagacg aggggtgcat cggatatacg caatcagctg tttgcataat 3936
aaaagtaccc gttgtccatc agcaaaataa cgcgttccgt ttgggattag ttctgcatac 3996
ataatacaaa tatcacggtg cttgcggttt ccagtattta ttcgtatcgc tacaacgtta 4056
aatgcatcaa agaataaacc ggggctaaga taaacaggca atgataaaat caatccccct 4116

gaattatgcg tggccgaaaa aacgtgtgaa acaaatggtt ccgtttttgg tattaagaga 4176

tttgttaagg cgttatcggg aatgtacgcg gcgaaaactt gacaccacgg ttcgcattga 4236

cctgtagcat gatatcttgt ttgtacttca accttgaagc gttgtccggg tttctttaaa 4296

atcagtaatg cgggatctat tccggccgca ataagccccg cgttaggtat cacaacgtgt 4356

agtaatcctt ttgtgtgatc attatgccaa agtgcatgtt tggtttcatt tgccaaatgg 4416

gcttccatta tacaccggat atggttgtac tggaaaaaaa aaagaaatat gtacgtattc 4476

aaacattttt tacgtacgtg gtatttaagg atacatttaa actttggtgg ggtaactata 4536

tatctttcta tcgttccag ggt atc cga ggt caa gat ttt aat ctt ctg ttt 4588
Gly Ile Arg Gly Gln Asp Phe Asn Leu Leu Phe
        355                 360

gtg gat gaa gct aat ttt att cga cct gat gct gta caa act ata gtc 4636
Val Asp Glu Ala Asn Phe Ile Arg Pro Asp Ala Val Gln Thr Ile Val
    365                 370                 375

gga ttt tta aat caa acc aat tgt aaa att att ttt gtt tca tca aca 4684
Gly Phe Leu Asn Gln Thr Asn Cys Lys Ile Ile Phe Val Ser Ser Thr
380                 385                 390                 395

aat acc gga aaa gca agt aca agt ttt ttg tat aac tta cgt gga tcg 4732
Asn Thr Gly Lys Ala Ser Thr Ser Phe Leu Tyr Asn Leu Arg Gly Ser
                400                 405                 410

tcg gat cag ttg tta aac gtt gtt aca tat gta tgc gac gat cac atg 4780
Ser Asp Gln Leu Leu Asn Val Val Thr Tyr Val Cys Asp Asp His Met
            415                 420                 425

ccg cgt gtt tta gca cat agc gat gtc aca gct tgt tcg tgt tat gta 4828
Pro Arg Val Leu Ala His Ser Asp Val Thr Ala Cys Ser Cys Tyr Val
        430                 435                 440

tta aat aag ccg gtt ttc atc aca atg gat gga gcc atg cgg cgc act 4876
Leu Asn Lys Pro Val Phe Ile Thr Met Asp Gly Ala Met Arg Arg Thr
    445                 450                 455

gca gat tta ttt atg gcc gac tcc ttc gtg cag gaa att gta ggt ggg 4924
Ala Asp Leu Phe Met Ala Asp Ser Phe Val Gln Glu Ile Val Gly Gly
460                 465                 470                 475

cgt aaa cag aat tct ggg ggt gtg ggg ttt gac cgg cca tta ttt aca 4972
Arg Lys Gln Asn Ser Gly Gly Val Gly Phe Asp Arg Pro Leu Phe Thr
                480                 485                 490

aaa act gcc cgt gag agg ttt att tta tat cgg ccg tca acc gtt gcg 5020
Lys Thr Ala Arg Glu Arg Phe Ile Leu Tyr Arg Pro Ser Thr Val Ala
            495                 500                 505

aat tgt gct ata tta tcg tca gtg ttg tac gtt tac gta gac cct gca 5068
Asn Cys Ala Ile Leu Ser Ser Val Leu Tyr Val Tyr Val Asp Pro Ala
        510                 515                 520

ttt acc tca aat aca cga gcg tct ggt act ggt gta gcg att gtt ggt 5116
Phe Thr Ser Asn Thr Arg Ala Ser Gly Thr Gly Val Ala Ile Val Gly
    525                 530                 535

cgt tat aag tcg gat tgg att ata ttt gga ttg gag cac ttt ttt ctt 5164
Arg Tyr Lys Ser Asp Trp Ile Ile Phe Gly Leu Glu His Phe Phe Leu
540                 545                 550                 555

aga gct tta act ggc acg tct tcc agt gag ata ggg cgt tgc gtt act 5212

Arg Ala Leu Thr Gly Thr Ser Ser Ser Glu Ile Gly Arg Cys Val Thr
                560                 565                 570

caa tgc tta ggc cac ata ctc gct tta cac ccc aat aca ttt aca aac      5260
Gln Cys Leu Gly His Ile Leu Ala Leu His Pro Asn Thr Phe Thr Asn
            575                 580                 585

gta cac gtt tct ata gag gga aac agc agc cag gat tct gca gtt gcc      5308
Val His Val Ser Ile Glu Gly Asn Ser Ser Gln Asp Ser Ala Val Ala
        590                 595                 600

ata tcg ttg gct ata gca caa cag ttt gct gtc ctc gaa aag gga aac      5356
Ile Ser Leu Ala Ile Ala Gln Gln Phe Ala Val Leu Glu Lys Gly Asn
    605                 610                 615

gtg cta tct tcc gct cca gtg tta ctg ttt tat cat tcc ata cct ccc      5404
Val Leu Ser Ser Ala Pro Val Leu Leu Phe Tyr His Ser Ile Pro Pro
620                 625                 630                 635

gga tgt agc gtg gcg tac cct ttt ttt tta tta caa aaa caa aaa acg      5452
Gly Cys Ser Val Ala Tyr Pro Phe Phe Leu Leu Gln Lys Gln Lys Thr
                640                 645                 650

ccg gcc gta gac tat ttt gtt aaa cga ttt aac tcc gga aat ata ata      5500
Pro Ala Val Asp Tyr Phe Val Lys Arg Phe Asn Ser Gly Asn Ile Ile
            655                 660                 665

gcc tca cag gag ctt gta tcc cta aca gta aag tta ggt gta gac ccc      5548
Ala Ser Gln Glu Leu Val Ser Leu Thr Val Lys Leu Gly Val Asp Pro
        670                 675                 680

gtg gag tat cta tgt aaa cag ttg gat aac ctg aca gag gta att aaa      5596
Val Glu Tyr Leu Cys Lys Gln Leu Asp Asn Leu Thr Glu Val Ile Lys
    685                 690                 695

ggc ggt atg ggt aat cta gac aca aaa act tac acg ggt aaa ggt acc      5644
Gly Gly Met Gly Asn Leu Asp Thr Lys Thr Tyr Thr Gly Lys Gly Thr
700                 705                 710                 715

acg gga aca atg tca gat gat ctg atg gtt gca tta att atg tcc gtg      5692
Thr Gly Thr Met Ser Asp Asp Leu Met Val Ala Leu Ile Met Ser Val
                720                 725                 730

tat att ggc agt tca tgt ata ccg gat tcc gtg ttt atg cct att aaa      5740
Tyr Ile Gly Ser Ser Cys Ile Pro Asp Ser Val Phe Met Pro Ile Lys
            735                 740                 745

taa                                                                  5743


<210>  81
<211>  747
<212>  PRT
<213>  Varicella zoster

<400>  81

Met Ser Leu Ile Met Phe Gly Arg Thr Leu Gly Glu Glu Ser Val Arg
1               5                   10                  15


Tyr Phe Glu Arg Leu Lys Arg Arg Arg Asp Glu Arg Phe Gly Thr Leu
            20                  25                  30


Glu Ser Pro Thr Pro Cys Ser Thr Arg Gln Gly Ser Leu Gly Asn Ala
        35                  40                  45

Thr Gln Ile Pro Phe Leu Asn Phe Ala Ile Asp Val Thr Arg Arg His
    50                  55                  60

Gln Ala Val Ile Pro Gly Ile Gly Thr Leu His Asn Cys Cys Glu Tyr
65                  70                  75                  80

Ile Pro Leu Phe Ser Ala Thr Ala Arg Arg Ala Met Phe Gly Ala Phe
                85                  90                  95

Leu Ser Ser Thr Gly Tyr Asn Cys Thr Pro Asn Val Val Leu Lys Pro
            100                 105                 110

Trp Arg Tyr Ser Val Asn Ala Asn Val Ser Pro Glu Leu Lys Lys Ala
        115                 120                 125

Val Ser Ser Val Gln Phe Tyr Glu Tyr Ser Pro Glu Glu Ala Ala Pro
    130                 135                 140

His Arg Asn Ala Tyr Ser Gly Val Met Asn Thr Phe Arg Ala Phe Ser
145                 150                 155                 160

Leu Ser Asp Ser Phe Cys Gln Leu Ser Thr Phe Thr Gln Arg Phe Ser
                165                 170                 175

Tyr Leu Val Glu Thr Ser Phe Glu Ser Ile Glu Glu Cys Gly Ser His
            180                 185                 190

Gly Lys Arg Ala Lys Val Asp Val Pro Ile Tyr Gly Arg Tyr Lys Gly
        195                 200                 205

Thr Leu Glu Leu Phe Gln Lys Met Ile Leu Met His Thr Thr His Phe
    210                 215                 220

Ile Ser Ser Val Leu Leu Gly Asp His Ala Asp Arg Val Asp Cys Phe
225                 230                 235                 240

Leu Arg Thr Val Phe Asn Thr Pro Ser Val Ser Asp Ser Val Leu Glu
                245                 250                 255

His Phe Lys Gln Lys Ser Thr Val Phe Leu Val Pro Arg Arg His Gly
            260                 265                 270

Lys Thr Trp Phe Leu Val Pro Leu Ile Ala Leu Val Met Ala Thr Phe
        275                 280                 285

Arg Gly Ile Lys Val Gly Tyr Thr Ala His Ile Arg Lys Ala Thr Glu
    290                 295                 300

Pro Val Phe Glu Gly Ile Lys Ser Arg Leu Glu Gln Trp Phe Gly Ala
305                 310                 315                 320

Asn Tyr Val Asp His Val Lys Gly Glu Ser Ile Thr Phe Ser Phe Thr
                325                 330                 335

Asp Gly Ser Tyr Ser Thr Ala Val Phe Ala Ser Ser His Asn Thr Asn
            340                 345                 350

Gly Ile Arg Gly Gln Asp Phe Asn Leu Leu Phe Val Asp Glu Ala Asn
        355                 360                 365

Phe Ile Arg Pro Asp Ala Val Gln Thr Ile Val Gly Phe Leu Asn Gln
    370                 375                 380

Thr Asn Cys Lys Ile Ile Phe Val Ser Ser Thr Asn Thr Gly Lys Ala
385                 390                 395                 400

Ser Thr Ser Phe Leu Tyr Asn Leu Arg Gly Ser Ser Asp Gln Leu Leu
                405                 410                 415

Asn Val Val Thr Tyr Val Cys Asp Asp His Met Pro Arg Val Leu Ala
            420                 425                 430

His Ser Asp Val Thr Ala Cys Ser Cys Tyr Val Leu Asn Lys Pro Val
        435                 440                 445

Phe Ile Thr Met Asp Gly Ala Met Arg Arg Thr Ala Asp Leu Phe Met
    450                 455                 460

Ala Asp Ser Phe Val Gln Glu Ile Val Gly Gly Arg Lys Gln Asn Ser
465                 470                 475                 480

Gly Gly Val Gly Phe Asp Arg Pro Leu Phe Thr Lys Thr Ala Arg Glu
                485                 490                 495

Arg Phe Ile Leu Tyr Arg Pro Ser Thr Val Ala Asn Cys Ala Ile Leu
            500                 505                 510

Ser Ser Val Leu Tyr Val Tyr Val Asp Pro Ala Phe Thr Ser Asn Thr
        515                 520                 525

Arg Ala Ser Gly Thr Gly Val Ala Ile Val Gly Arg Tyr Lys Ser Asp
    530                 535                 540

Trp Ile Ile Phe Gly Leu Glu His Phe Phe Leu Arg Ala Leu Thr Gly
545                 550                 555                 560

Thr Ser Ser Ser Glu Ile Gly Arg Cys Val Thr Gln Cys Leu Gly His
                565                 570                 575

Ile Leu Ala Leu His Pro Asn Thr Phe Thr Asn Val His Val Ser Ile
            580                 585                 590

Glu Gly Asn Ser Ser Gln Asp Ser Ala Val Ala Ile Ser Leu Ala Ile
        595                 600                 605

Ala Gln Gln Phe Ala Val Leu Glu Lys Gly Asn Val Leu Ser Ser Ala
    610                 615                 620

Pro Val Leu Leu Phe Tyr His Ser Ile Pro Pro Gly Cys Ser Val Ala
625                 630                 635                 640

Tyr Pro Phe Phe Leu Leu Gln Lys Gln Lys Thr Pro Ala Val Asp Tyr
                645                 650                 655

Phe Val Lys Arg Phe Asn Ser Gly Asn Ile Ile Ala Ser Gln Glu Leu
            660                 665                 670

Val Ser Leu Thr Val Lys Leu Gly Val Asp Pro Val Glu Tyr Leu Cys
        675                 680                 685

Lys Gln Leu Asp Asn Leu Thr Glu Val Ile Lys Gly Gly Met Gly Asn
    690                 695                 700

Leu Asp Thr Lys Thr Tyr Thr Gly Lys Gly Thr Thr Gly Thr Met Ser
705                 710                 715                 720

Asp Asp Leu Met Val Ala Leu Ile Met Ser Val Tyr Ile Gly Ser Ser
                725                 730                 735

Cys Ile Pro Asp Ser Val Phe Met Pro Ile Lys
            740                 745

<210>  82
<211>  1308
<212>  DNA
<213>  Varicella zoster

<220>
<221>  CDS
<222>  (1)..(1308)

<400>  82
atg gga act caa aag aag ggg ccg cgt tct gaa aaa gtc tcg ccg tac        48
Met Gly Thr Gln Lys Lys Gly Pro Arg Ser Glu Lys Val Ser Pro Tyr
1               5                   10                  15

gac acc acg aca ccc gag gtg gaa gcg tta gat cat caa atg gat acg        96
Asp Thr Thr Thr Pro Glu Val Glu Ala Leu Asp His Gln Met Asp Thr
            20                  25                  30

ctt aat tgg cga att tgg ata att cag gtg atg atg ttc act ttg ggt       144
Leu Asn Trp Arg Ile Trp Ile Ile Gln Val Met Met Phe Thr Leu Gly
        35                  40                  45

gcg gta atg ctc ctg gct acg tta att gcc gcc tct tct gaa tat acc       192
Ala Val Met Leu Leu Ala Thr Leu Ile Ala Ala Ser Ser Glu Tyr Thr
    50                  55                  60

ggg atc cct tgt ttt tat gct gcc gta gtt gat tat gag tta ttt aac 240
Gly Ile Pro Cys Phe Tyr Ala Ala Val Val Asp Tyr Glu Leu Phe Asn
65                  70                  75                  80

gcc acc cta gat ggg ggg gta tgg tcc gga aat aga ggt gga tac agc 288
Ala Thr Leu Asp Gly Gly Val Trp Ser Gly Asn Arg Gly Gly Tyr Ser
                85                  90                  95

gcc ccg gtt ttg ttt ttg gaa cca cat agc gtt gtg gca ttt act tac 336
Ala Pro Val Leu Phe Leu Glu Pro His Ser Val Val Ala Phe Thr Tyr
            100                 105                 110

tac acg gct tta acg gca atg gcc atg gcg gta tat aca ctg atc acg 384
Tyr Thr Ala Leu Thr Ala Met Ala Met Ala Val Tyr Thr Leu Ile Thr
        115                 120                 125

gcc gcg att ata cac cga gaa acg aaa aat caa cgt gtc cgg caa agc 432
Ala Ala Ile Ile His Arg Glu Thr Lys Asn Gln Arg Val Arg Gln Ser
    130                 135                 140

tcc ggt gtt gca tgg tta gtt gta gat ccc aca aca ctt ttt tgg ggt 480
Ser Gly Val Ala Trp Leu Val Val Asp Pro Thr Thr Leu Phe Trp Gly
145                 150                 155                 160

ctt ttg tca ttg tgg tta tta aac gcc gtt gtg tta tta tta gct tac 528
Leu Leu Ser Leu Trp Leu Leu Asn Ala Val Val Leu Leu Leu Ala Tyr
                165                 170                 175

aag caa atc ggc gtg gct gct aca tta tat ctt gga cat ttt gcg aca 576
Lys Gln Ile Gly Val Ala Ala Thr Leu Tyr Leu Gly His Phe Ala Thr
            180                 185                 190

agt gta ata ttt aca acg tat ttt tgt gga cgc gga aaa ttg gac gaa 624
Ser Val Ile Phe Thr Thr Tyr Phe Cys Gly Arg Gly Lys Leu Asp Glu
        195                 200                 205

acg aac ata aaa gcg gtc gca aat tta cga cag cag agc gtc ttt tta 672
Thr Asn Ile Lys Ala Val Ala Asn Leu Arg Gln Gln Ser Val Phe Leu
    210                 215                 220

tat cgc ctt gcg ggg cct acg cgc gca gtg ttc gtg aat ttg atg gct 720
Tyr Arg Leu Ala Gly Pro Thr Arg Ala Val Phe Val Asn Leu Met Ala
225                 230                 235                 240

gcg ttg atg gcg ata tgt atc cta ttt gta tca tta atg ctg gaa ctt 768
Ala Leu Met Ala Ile Cys Ile Leu Phe Val Ser Leu Met Leu Glu Leu
                245                 250                 255

gtg gtg gcg aat cat cta cat acg gga ctg tgg tca tcg gtg tcc gtg 816
Val Val Ala Asn His Leu His Thr Gly Leu Trp Ser Ser Val Ser Val
            260                 265                 270

gcc atg tct aca ttt agt aca ttg tca gtt gta tat ctt ata gta tca 864
Ala Met Ser Thr Phe Ser Thr Leu Ser Val Val Tyr Leu Ile Val Ser
        275                 280                 285

gaa tta att ttg gcg cat tat ata cac gtg tta ata gga ccg tcc ctg 912
Glu Leu Ile Leu Ala His Tyr Ile His Val Leu Ile Gly Pro Ser Leu
    290                 295                 300

gga acg ctc gtg gcc tgt gct acg ttg gga acc gcg gcg cac tcg tat 960
Gly Thr Leu Val Ala Cys Ala Thr Leu Gly Thr Ala Ala His Ser Tyr
305                 310                 315                 320

atg gac cga tta tat gac cct ata tcg gtt caa tct cca cgg tta att 1008
Met Asp Arg Leu Tyr Asp Pro Ile Ser Val Gln Ser Pro Arg Leu Ile
                325                 330                 335

ccc aca act cgg gga acc ttg gct tgc ctg gcc gtg ttt tcc gtt gtc 1056
Pro Thr Thr Arg Gly Thr Leu Ala Cys Leu Ala Val Phe Ser Val Val
            340                 345                 350

atg ttg ctt ctc aga ttg atg cgt gca tat gtg tat cat cga cag aaa 1104
Met Leu Leu Leu Arg Leu Met Arg Ala Tyr Val Tyr His Arg Gln Lys
        355                 360                 365

cgc agt cgg ttc tac ggt gcc gta aga aga gta ccc gag cgg gta cgg 1152
Arg Ser Arg Phe Tyr Gly Ala Val Arg Arg Val Pro Glu Arg Val Arg
    370                 375                 380

gga tac ata cga aaa gta aaa cct gca cat aga aat tct cgc cgc aca 1200
Gly Tyr Ile Arg Lys Val Lys Pro Ala His Arg Asn Ser Arg Arg Thr
385                 390                 395                 400

aat tac cca tca caa ggc tac ggc tac gtc tat gaa aat gac tca aca 1248
Asn Tyr Pro Ser Gln Gly Tyr Gly Tyr Val Tyr Glu Asn Asp Ser Thr
                405                 410                 415

tat gaa acg gac cgc gag gat gag ctg tta tac gag cga tca aac agt 1296
Tyr Glu Thr Asp Arg Glu Asp Glu Leu Leu Tyr Glu Arg Ser Asn Ser
            420                 425                 430

ggg tgg gag tag 1308
Gly Trp Glu
        435

<210> 83
<211> 435
<212> PRT
<213> Varicella zoster

<400> 83

Met Gly Thr Gln Lys Lys Gly Pro Arg Ser Glu Lys Val Ser Pro Tyr
1               5                   10                  15

Asp Thr Thr Thr Pro Glu Val Glu Ala Leu Asp His Gln Met Asp Thr
            20                  25                  30

Leu Asn Trp Arg Ile Trp Ile Ile Gln Val Met Met Phe Thr Leu Gly
        35                  40                  45

Ala Val Met Leu Leu Ala Thr Leu Ile Ala Ala Ser Ser Glu Tyr Thr
    50                  55                  60

Gly Ile Pro Cys Phe Tyr Ala Ala Val Val Asp Tyr Glu Leu Phe Asn
65                  70                  75                  80

Ala Thr Leu Asp Gly Gly Val Trp Ser Gly Asn Arg Gly Gly Tyr Ser
                85                  90                  95

Ala Pro Val Leu Phe Leu Glu Pro His Ser Val Val Ala Phe Thr Tyr
            100                 105                 110

Tyr Thr Ala Leu Thr Ala Met Ala Met Ala Val Tyr Thr Leu Ile Thr
        115                 120                 125

Ala Ala Ile Ile His Arg Glu Thr Lys Asn Gln Arg Val Arg Gln Ser
    130                 135                 140

Ser Gly Val Ala Trp Leu Val Val Asp Pro Thr Thr Leu Phe Trp Gly
145                 150                 155                 160

Leu Leu Ser Leu Trp Leu Leu Asn Ala Val Val Leu Leu Leu Ala Tyr
                165                 170                 175

Lys Gln Ile Gly Val Ala Ala Thr Leu Tyr Leu Gly His Phe Ala Thr
            180                 185                 190

Ser Val Ile Phe Thr Thr Tyr Phe Cys Gly Arg Gly Lys Leu Asp Glu
        195                 200                 205

Thr Asn Ile Lys Ala Val Ala Asn Leu Arg Gln Gln Ser Val Phe Leu
    210                 215                 220

Tyr Arg Leu Ala Gly Pro Thr Arg Ala Val Phe Val Asn Leu Met Ala
225                 230                 235                 240

Ala Leu Met Ala Ile Cys Ile Leu Phe Val Ser Leu Met Leu Glu Leu
                245                 250                 255

Val Val Ala Asn His Leu His Thr Gly Leu Trp Ser Ser Val Ser Val
            260                 265                 270

Ala Met Ser Thr Phe Ser Thr Leu Ser Val Val Tyr Leu Ile Val Ser
        275                 280                 285

Glu Leu Ile Leu Ala His Tyr Ile His Val Leu Ile Gly Pro Ser Leu
    290                 295                 300

Gly Thr Leu Val Ala Cys Ala Thr Leu Gly Thr Ala Ala His Ser Tyr
305                 310                 315                 320

Met Asp Arg Leu Tyr Asp Pro Ile Ser Val Gln Ser Pro Arg Leu Ile
                325                 330                 335

Pro Thr Thr Arg Gly Thr Leu Ala Cys Leu Ala Val Phe Ser Val Val
            340                 345                 350

Met Leu Leu Leu Arg Leu Met Arg Ala Tyr Val Tyr His Arg Gln Lys
        355                 360                 365

Arg Ser Arg Phe Tyr Gly Ala Val Arg Arg Val Pro Glu Arg Val Arg
    370                 375                 380

Gly Tyr Ile Arg Lys Val Lys Pro Ala His Arg Asn Ser Arg Arg Thr
385                 390                 395                 400

Asn Tyr Pro Ser Gln Gly Tyr Gly Tyr Val Tyr Glu Asn Asp Ser Thr
                405                 410                 415

Tyr Glu Thr Asp Arg Glu Asp Glu Leu Leu Tyr Glu Arg Ser Asn Ser
            420                 425                 430

Gly Trp Glu
        435

<210>  84
<211>  2310
<212>  DNA
<213>  Varicella zoster

<220>
<221>  CDS
<222>  (1)..(2310)

<400>  84
atg gcc gaa ata acg tct ctt ttt aat aac agt tcc ggt agt gaa gaa        48
Met Ala Glu Ile Thr Ser Leu Phe Asn Asn Ser Ser Gly Ser Glu Glu
1               5                   10                  15

aaa agg ata gca agt tct gtt tct att gac cag ggc ttg aat gga agt        96
Lys Arg Ile Ala Ser Ser Val Ser Ile Asp Gln Gly Leu Asn Gly Ser
            20                  25                  30

aac cca aat gac caa tac aag aac atg ttc gat ata tac tgg aat gag       144
Asn Pro Asn Asp Gln Tyr Lys Asn Met Phe Asp Ile Tyr Trp Asn Glu
        35                  40                  45

tac gcc ccg gat ata ggg ttt tgt aca ttt ccg gag gaa gat ggc tgg       192
Tyr Ala Pro Asp Ile Gly Phe Cys Thr Phe Pro Glu Glu Asp Gly Trp
    50                  55                  60

atg tta ata cac cca acc acg caa agt atg ttg ttt cga aaa atc cta       240
Met Leu Ile His Pro Thr Thr Gln Ser Met Leu Phe Arg Lys Ile Leu
65                  70                  75                  80

gcc ggt gac ttt gga tat acc gat gga caa ggc ata tat agc gct gta       288
Ala Gly Asp Phe Gly Tyr Thr Asp Gly Gln Gly Ile Tyr Ser Ala Val
                85                  90                  95

cgg tct acg gaa act gta att cgc caa gtt cag gca acc gtt ttg atg       336
Arg Ser Thr Glu Thr Val Ile Arg Gln Val Gln Ala Thr Val Leu Met
            100                 105                 110

aac gcg ttg gat gca act cgg tat gag gac cta gca gca gat tgg gaa       384
Asn Ala Leu Asp Ala Thr Arg Tyr Glu Asp Leu Ala Ala Asp Trp Glu
        115                 120                 125

cac cac atc caa caa tgt aac ctg cat gcc ggg gct cta gcg gaa cgt       432
His His Ile Gln Gln Cys Asn Leu His Ala Gly Ala Leu Ala Glu Arg
    130                 135                 140

tat ggg cta tgt gga gaa tca gaa gcc gta cgg ctt gca cat cag gtt       480
Tyr Gly Leu Cys Gly Glu Ser Glu Ala Val Arg Leu Ala His Gln Val
145                 150                 155                 160

ttt gaa acc tgg cgt caa aca tta cag tca tcg tta ctt gag ttt ctg       528
Phe Glu Thr Trp Arg Gln Thr Leu Gln Ser Ser Leu Leu Glu Phe Leu
                165                 170                 175

cgt gga ata acc ggt tgt ctc tat acc agt ggt tta aat gga agg gtc       576

Arg Gly Ile Thr Gly Cys Leu Tyr Thr Ser Gly Leu Asn Gly Arg Val
            180                 185                 190

ggt ttt gcc aaa tac gtg gac tgg ata gcc tgt gta ggt att gtg ccc      624
Gly Phe Ala Lys Tyr Val Asp Trp Ile Ala Cys Val Gly Ile Val Pro
        195                 200                 205

gtt gta aga aag gta cga tca gaa cag aat gga acc cct gca cca tta      672
Val Val Arg Lys Val Arg Ser Glu Gln Asn Gly Thr Pro Ala Pro Leu
    210                 215                 220

aat acg tat atg ggt caa gcg gca gaa ctg tcc cag atg tta aaa gtt      720
Asn Thr Tyr Met Gly Gln Ala Ala Glu Leu Ser Gln Met Leu Lys Val
225                 230                 235                 240

gcc gat gca acg ttg gcc aga gga gcg gcg gtt gtc aca agc cta gtt      768
Ala Asp Ala Thr Leu Ala Arg Gly Ala Ala Val Val Thr Ser Leu Val
                245                 250                 255

gag tgt atg caa aat gtt gct att atg gat tat gat agg acg cgt ctt      816
Glu Cys Met Gln Asn Val Ala Ile Met Asp Tyr Asp Arg Thr Arg Leu
            260                 265                 270

tat tat aat tat aac cga aga tta att atg gca aag gat gat gta acg      864
Tyr Tyr Asn Tyr Asn Arg Arg Leu Ile Met Ala Lys Asp Asp Val Thr
        275                 280                 285

ggc atg aag gga gag tgt ttg gtc gtg tgg ccg ccc gtt gta tgt ggg      912
Gly Met Lys Gly Glu Cys Leu Val Val Trp Pro Pro Val Val Cys Gly
    290                 295                 300

gag ggt gta gta ttt gac tca ccc tta cag cgg ctt tct ggg gag gtg      960
Glu Gly Val Val Phe Asp Ser Pro Leu Gln Arg Leu Ser Gly Glu Val
305                 310                 315                 320

ttg gcc tgt tat gca tta cgt gaa cat gct cgc gtc tgc caa gtt tta     1008
Leu Ala Cys Tyr Ala Leu Arg Glu His Ala Arg Val Cys Gln Val Leu
                325                 330                 335

aat aca gcc cct ttg cgc gtg tta ata ggt cgc cgg aat gaa gat gat     1056
Asn Thr Ala Pro Leu Arg Val Leu Ile Gly Arg Arg Asn Glu Asp Asp
            340                 345                 350

aga tct cac agc aca cgt gcg gtt gat cgt ata atg ggc gag aac gat     1104
Arg Ser His Ser Thr Arg Ala Val Asp Arg Ile Met Gly Glu Asn Asp
        355                 360                 365

aca aca cgg gct gga tcg gcc gcg tct aga ctt gta aag cta ata gtt     1152
Thr Thr Arg Ala Gly Ser Ala Ala Ser Arg Leu Val Lys Leu Ile Val
    370                 375                 380

aac tta aaa aac atg aga cat gtt gga gat att acc gaa acc gta cgt     1200
Asn Leu Lys Asn Met Arg His Val Gly Asp Ile Thr Glu Thr Val Arg
385                 390                 395                 400

tcc tat cta gaa gaa acg ggc aat cac att ctg gaa gga agt gga tcg     1248
Ser Tyr Leu Glu Glu Thr Gly Asn His Ile Leu Glu Gly Ser Gly Ser
                405                 410                 415

gtg gac aca tca caa ccg ggg ttt ggc aag gcc aac caa tcc ttt aac     1296
Val Asp Thr Ser Gln Pro Gly Phe Gly Lys Ala Asn Gln Ser Phe Asn
            420                 425                 430

ggg ggg gca atg tcc gga aca aca aac gtt caa agt gcg ttt aaa act     1344
Gly Gly Ala Met Ser Gly Thr Thr Asn Val Gln Ser Ala Phe Lys Thr
        435                 440                 445

tcg gtg gtt aac agt atc aac ggc atg ctc gag ggt tat gtg aat aat     1392

Ser Val Val Asn Ser Ile Asn Gly Met Leu Glu Gly Tyr Val Asn Asn
    450                 455                 460

tta ttc aaa acc att gag ggt ctc aag gat gtg aac agc gat ctg acc      1440
Leu Phe Lys Thr Ile Glu Gly Leu Lys Asp Val Asn Ser Asp Leu Thr
465                 470                 475                 480

gaa agg ctc cag ttc aaa gaa gga gag ctg aaa cgg tta cgg gaa gag      1488
Glu Arg Leu Gln Phe Lys Glu Gly Glu Leu Lys Arg Leu Arg Glu Glu
                485                 490                 495

agg gta aaa ata aag cca tct aaa ggg tca cat att aca atg gca gaa      1536
Arg Val Lys Ile Lys Pro Ser Lys Gly Ser His Ile Thr Met Ala Glu
            500                 505                 510

gaa aca cgt att gcc gat tta aat cac gag gtt ata gat ctt acc ggc      1584
Glu Thr Arg Ile Ala Asp Leu Asn His Glu Val Ile Asp Leu Thr Gly
        515                 520                 525

ata ata ggg gat gat gca tat att gcc aat agt ttt caa tct cgt tat      1632
Ile Ile Gly Asp Asp Ala Tyr Ile Ala Asn Ser Phe Gln Ser Arg Tyr
    530                 535                 540

atc ccc cct tat gga gat gat ata aaa cgt ttg tct gag cta tgg aaa      1680
Ile Pro Pro Tyr Gly Asp Asp Ile Lys Arg Leu Ser Glu Leu Trp Lys
545                 550                 555                 560

cag gaa ctt gtt cgc tgt ttt aag ctt cac cgg gta aac aat aat caa      1728
Gln Glu Leu Val Arg Cys Phe Lys Leu His Arg Val Asn Asn Asn Gln
                565                 570                 575

ggc cag gaa att tct gta tca tat tca aat gcg tca atc tca tta cta      1776
Gly Gln Glu Ile Ser Val Ser Tyr Ser Asn Ala Ser Ile Ser Leu Leu
            580                 585                 590

gtt gcg ccg tat ttt tca ttc ata tta cgg gcc acc cga tta gga ttc      1824
Val Ala Pro Tyr Phe Ser Phe Ile Leu Arg Ala Thr Arg Leu Gly Phe
        595                 600                 605

ttg gta act caa agc gag gta cat agg tca gag gaa gag tta tgc cag      1872
Leu Val Thr Gln Ser Glu Val His Arg Ser Glu Glu Glu Leu Cys Gln
    610                 615                 620

gct att ttt aaa aag gcg aga aca gag tcc tat tta tcc caa atc cga      1920
Ala Ile Phe Lys Lys Ala Arg Thr Glu Ser Tyr Leu Ser Gln Ile Arg
625                 630                 635                 640

ata tta tat gaa atg cag gtt cgc gca gag gta ata aaa cgg ggc cca      1968
Ile Leu Tyr Glu Met Gln Val Arg Ala Glu Val Ile Lys Arg Gly Pro
                645                 650                 655

cgg aga aca cca agt cct tcc tgg ggt ttg cct gac cct aca gaa gat      2016
Arg Arg Thr Pro Ser Pro Ser Trp Gly Leu Pro Asp Pro Thr Glu Asp
            660                 665                 670

gac gaa aga atc ccg gaa ccc aat aaa ata aat aac caa tac atg cat      2064
Asp Glu Arg Ile Pro Glu Pro Asn Lys Ile Asn Asn Gln Tyr Met His
        675                 680                 685

gtt gga tat aaa aac cta tcc cat ttt atg aaa gga cac ccc cct gag      2112
Val Gly Tyr Lys Asn Leu Ser His Phe Met Lys Gly His Pro Pro Glu
    690                 695                 700

agg tta cgg gta cac aag gta aat gca gcg gat tcg acc tta ctg gat      2160
Arg Leu Arg Val His Lys Val Asn Ala Ala Asp Ser Thr Leu Leu Asp
705                 710                 715                 720

aaa att cga gca aac cgg agg cgc ggg gat ggc cga tgg gat gtc cgg      2208

Lys Ile Arg Ala Asn Arg Arg Arg Gly Asp Gly Arg Trp Asp Val Arg
                725                 730                 735

aat aaa tat acc cag cat ttt agg ttg cag cgt aac gat cga caa ctt 2256
Asn Lys Tyr Thr Gln His Phe Arg Leu Gln Arg Asn Asp Arg Gln Leu
            740                 745                 750

act aac acg agc cga aga ggg gtt gga tgt gag cga cgt gat cga aga 2304
Thr Asn Thr Ser Arg Arg Gly Val Gly Cys Glu Arg Arg Asp Arg Arg
        755                 760                 765

tct tag 2310
Ser

<210> 85
<211> 769
<212> PRT
<213> Varicella zoster

<400> 85

Met Ala Glu Ile Thr Ser Leu Phe Asn Asn Ser Ser Gly Ser Glu Glu
1               5                   10                  15

Lys Arg Ile Ala Ser Ser Val Ser Ile Asp Gln Gly Leu Asn Gly Ser
            20                  25                  30

Asn Pro Asn Asp Gln Tyr Lys Asn Met Phe Asp Ile Tyr Trp Asn Glu
        35                  40                  45

Tyr Ala Pro Asp Ile Gly Phe Cys Thr Phe Pro Glu Glu Asp Gly Trp
    50                  55                  60

Met Leu Ile His Pro Thr Thr Gln Ser Met Leu Phe Arg Lys Ile Leu
65                  70                  75                  80

Ala Gly Asp Phe Gly Tyr Thr Asp Gly Gln Gly Ile Tyr Ser Ala Val
                85                  90                  95

Arg Ser Thr Glu Thr Val Ile Arg Gln Val Gln Ala Thr Val Leu Met
            100                 105                 110

Asn Ala Leu Asp Ala Thr Arg Tyr Glu Asp Leu Ala Ala Asp Trp Glu
        115                 120                 125

His His Ile Gln Gln Cys Asn Leu His Ala Gly Ala Leu Ala Glu Arg
    130                 135                 140

Tyr Gly Leu Cys Gly Glu Ser Glu Ala Val Arg Leu Ala His Gln Val
145                 150                 155                 160

Phe Glu Thr Trp Arg Gln Thr Leu Gln Ser Ser Leu Leu Glu Phe Leu
                165                 170                 175

Arg Gly Ile Thr Gly Cys Leu Tyr Thr Ser Gly Leu Asn Gly Arg Val

180                    185                    190

Gly Phe Ala Lys Tyr Val Asp Trp Ile Ala Cys Val Gly Ile Val Pro
        195                 200                 205

Val Val Arg Lys Val Arg Ser Glu Gln Asn Gly Thr Pro Ala Pro Leu
    210                 215                 220

Asn Thr Tyr Met Gly Gln Ala Ala Glu Leu Ser Gln Met Leu Lys Val
225                 230                 235                 240

Ala Asp Ala Thr Leu Ala Arg Gly Ala Ala Val Val Thr Ser Leu Val
                245                 250                 255

Glu Cys Met Gln Asn Val Ala Ile Met Asp Tyr Asp Arg Thr Arg Leu
            260                 265                 270

Tyr Tyr Asn Tyr Asn Arg Arg Leu Ile Met Ala Lys Asp Asp Val Thr
        275                 280                 285

Gly Met Lys Gly Glu Cys Leu Val Val Trp Pro Pro Val Val Cys Gly
    290                 295                 300

Glu Gly Val Val Phe Asp Ser Pro Leu Gln Arg Leu Ser Gly Glu Val
305                 310                 315                 320

Leu Ala Cys Tyr Ala Leu Arg Glu His Ala Arg Val Cys Gln Val Leu
                325                 330                 335

Asn Thr Ala Pro Leu Arg Val Leu Ile Gly Arg Arg Asn Glu Asp Asp
            340                 345                 350

Arg Ser His Ser Thr Arg Ala Val Asp Arg Ile Met Gly Glu Asn Asp
        355                 360                 365

Thr Thr Arg Ala Gly Ser Ala Ala Ser Arg Leu Val Lys Leu Ile Val
    370                 375                 380

Asn Leu Lys Asn Met Arg His Val Gly Asp Ile Thr Glu Thr Val Arg
385                 390                 395                 400

Ser Tyr Leu Glu Glu Thr Gly Asn His Ile Leu Glu Gly Ser Gly Ser
                405                 410                 415

Val Asp Thr Ser Gln Pro Gly Phe Gly Lys Ala Asn Gln Ser Phe Asn
            420                 425                 430

Gly Gly Ala Met Ser Gly Thr Thr Asn Val Gln Ser Ala Phe Lys Thr
        435                 440                 445

Ser Val Val Asn Ser Ile Asn Gly Met Leu Glu Gly Tyr Val Asn Asn

450                 455                 460

Leu Phe Lys Thr Ile Glu Gly Leu Lys Asp Val Asn Ser Asp Leu Thr
465                 470                 475                 480

Glu Arg Leu Gln Phe Lys Glu Gly Glu Leu Lys Arg Leu Arg Glu Glu
                485                 490                 495

Arg Val Lys Ile Lys Pro Ser Lys Gly Ser His Ile Thr Met Ala Glu
            500                 505                 510

Glu Thr Arg Ile Ala Asp Leu Asn His Glu Val Ile Asp Leu Thr Gly
        515                 520                 525

Ile Ile Gly Asp Asp Ala Tyr Ile Ala Asn Ser Phe Gln Ser Arg Tyr
    530                 535                 540

Ile Pro Pro Tyr Gly Asp Asp Ile Lys Arg Leu Ser Glu Leu Trp Lys
545                 550                 555                 560

Gln Glu Leu Val Arg Cys Phe Lys Leu His Arg Val Asn Asn Asn Gln
                565                 570                 575

Gly Gln Glu Ile Ser Val Ser Tyr Ser Asn Ala Ser Ile Ser Leu Leu
            580                 585                 590

Val Ala Pro Tyr Phe Ser Phe Ile Leu Arg Ala Thr Arg Leu Gly Phe
        595                 600                 605

Leu Val Thr Gln Ser Glu Val His Arg Ser Glu Glu Glu Leu Cys Gln
    610                 615                 620

Ala Ile Phe Lys Lys Ala Arg Thr Glu Ser Tyr Leu Ser Gln Ile Arg
625                 630                 635                 640

Ile Leu Tyr Glu Met Gln Val Arg Ala Glu Val Ile Lys Arg Gly Pro
                645                 650                 655

Arg Arg Thr Pro Ser Pro Ser Trp Gly Leu Pro Asp Pro Thr Glu Asp
            660                 665                 670

Asp Glu Arg Ile Pro Glu Pro Asn Lys Ile Asn Asn Gln Tyr Met His
        675                 680                 685

Val Gly Tyr Lys Asn Leu Ser His Phe Met Lys Gly His Pro Pro Glu
    690                 695                 700

Arg Leu Arg Val His Lys Val Asn Ala Ala Asp Ser Thr Leu Leu Asp
705                 710                 715                 720

Lys Ile Arg Ala Asn Arg Arg Arg Gly Asp Gly Arg Trp Asp Val Arg

725                    730                    735

Asn Lys Tyr Thr Gln His Phe Arg Leu Gln Arg Asn Asp Arg Gln Leu
            740                 745                 750

Thr Asn Thr Ser Arg Arg Gly Val Gly Cys Glu Arg Arg Asp Arg Arg
            755                 760                 765

Ser

<210>  86
<211>  666
<212>  DNA
<213>  Varicella zoster

<220>
<221>  CDS
<222>  (1)..(666)

<400>  86
atg ttt tcg gag ttg cct cct tcc gta ccg acg gca ttg ctt caa tgg      48
Met Phe Ser Glu Leu Pro Pro Ser Val Pro Thr Ala Leu Leu Gln Trp
1               5                   10                  15

ggt tgg gga ttg cat cgt gga ccg tgt tcg atc cca aat ttt aaa cag      96
Gly Trp Gly Leu His Arg Gly Pro Cys Ser Ile Pro Asn Phe Lys Gln
            20                  25                  30

gta gcc agc caa cac agt gtt cag aac gat ttt aca gaa aat agc gtt      144
Val Ala Ser Gln His Ser Val Gln Asn Asp Phe Thr Glu Asn Ser Val
        35                  40                  45

gat gca aat gaa aaa ttt ccg att ggg cac gcg ggc tgt att gag aaa      192
Asp Ala Asn Glu Lys Phe Pro Ile Gly His Ala Gly Cys Ile Glu Lys
    50                  55                  60

acc aaa gac gac tat gta cca ttt gat acg ttg ttc atg gta tca tct      240
Thr Lys Asp Asp Tyr Val Pro Phe Asp Thr Leu Phe Met Val Ser Ser
65                  70                  75                  80

att gac gaa ctt ggg cgg aga caa tta acc gac acc atc cgc cgc agc      288
Ile Asp Glu Leu Gly Arg Arg Gln Leu Thr Asp Thr Ile Arg Arg Ser
                85                  90                  95

ttg gtt atg aac gcc tgt gaa ata acg gtc gcg tgt acg aaa acc gca      336
Leu Val Met Asn Ala Cys Glu Ile Thr Val Ala Cys Thr Lys Thr Ala
            100                 105                 110

gcc ttt tct ggt cga ggc gtg tca cga caa aaa cac gtg acc cta tct      384
Ala Phe Ser Gly Arg Gly Val Ser Arg Gln Lys His Val Thr Leu Ser
        115                 120                 125

aaa aat aaa ttc aat cca tcc agt cat aag agc ctg caa atg ttt gtg      432
Lys Asn Lys Phe Asn Pro Ser Ser His Lys Ser Leu Gln Met Phe Val
    130                 135                 140

ttg tgt caa aaa acc cat gca ccc cgt gtc aga aac cta ctg tac gag      480
Leu Cys Gln Lys Thr His Ala Pro Arg Val Arg Asn Leu Leu Tyr Glu
145                 150                 155                 160

agt att cgt gca aga aga cct cgc cga tat tac acc cgc tca acg gac      528
Ser Ile Arg Ala Arg Arg Pro Arg Arg Tyr Tyr Thr Arg Ser Thr Asp

                    165                     170                     175

gga aaa tcg cgt ccg ttg gta cca gtg ttt gtg tat gag ttt acg gct        576
Gly Lys Ser Arg Pro Leu Val Pro Val Phe Val Tyr Glu Phe Thr Ala
                180                 185                 190

tta gat cgt gtc ctt tta cat aag gaa aat act ttg acc gac caa cca        624
Leu Asp Arg Val Leu Leu His Lys Glu Asn Thr Leu Thr Asp Gln Pro
            195                 200                 205

att aat act gaa aat agc ggt cat gga cgt acg aga acg taa                666
Ile Asn Thr Glu Asn Ser Gly His Gly Arg Thr Arg Thr
    210                 215                 220

<210>  87
<211>  221
<212>  PRT
<213>  Varicella zoster

<400>  87

Met Phe Ser Glu Leu Pro Pro Ser Val Pro Thr Ala Leu Leu Gln Trp
1               5                   10                  15

Gly Trp Gly Leu His Arg Gly Pro Cys Ser Ile Pro Asn Phe Lys Gln
            20                  25                  30

Val Ala Ser Gln His Ser Val Gln Asn Asp Phe Thr Glu Asn Ser Val
        35                  40                  45

Asp Ala Asn Glu Lys Phe Pro Ile Gly His Ala Gly Cys Ile Glu Lys
    50                  55                  60

Thr Lys Asp Asp Tyr Val Pro Phe Asp Thr Leu Phe Met Val Ser Ser
65                  70                  75                  80

Ile Asp Glu Leu Gly Arg Arg Gln Leu Thr Asp Thr Ile Arg Arg Ser
                85                  90                  95

Leu Val Met Asn Ala Cys Glu Ile Thr Val Ala Cys Thr Lys Thr Ala
            100                 105                 110

Ala Phe Ser Gly Arg Gly Val Ser Arg Gln Lys His Val Thr Leu Ser
        115                 120                 125

Lys Asn Lys Phe Asn Pro Ser Ser His Lys Ser Leu Gln Met Phe Val
    130                 135                 140

Leu Cys Gln Lys Thr His Ala Pro Arg Val Arg Asn Leu Leu Tyr Glu
145                 150                 155                 160

Ser Ile Arg Ala Arg Arg Pro Arg Arg Tyr Tyr Thr Arg Ser Thr Asp
                165                 170                 175

Gly Lys Ser Arg Pro Leu Val Pro Val Phe Val Tyr Glu Phe Thr Ala
            180                 185                 190

Leu Asp Arg Val Leu Leu His Lys Glu Asn Thr Leu Thr Asp Gln Pro
        195                 200                 205

Ile Asn Thr Glu Asn Ser Gly His Gly Arg Thr Arg Thr
    210                 215                 220

<210>  88
<211>  480
<212>  DNA
<213>  Varicella zoster

<220>
<221>  CDS
<222>  (1)..(480)

<400>  88
atg gca tca cat aaa tgg tta ctg cag ata gtt ttt tta aaa act atc        48
Met Ala Ser His Lys Trp Leu Leu Gln Ile Val Phe Leu Lys Thr Ile
1               5                   10                  15

aca atc gcg tat tgt ctt cat ctc caa gac gac act ccg ttg ttt ttt        96
Thr Ile Ala Tyr Cys Leu His Leu Gln Asp Asp Thr Pro Leu Phe Phe
            20                  25                  30

gga gcc aaa ccg cta tcg gat gtg agt ttg att ata acg gaa ccg tgc       144
Gly Ala Lys Pro Leu Ser Asp Val Ser Leu Ile Ile Thr Glu Pro Cys
        35                  40                  45

gtg tca tcg gta tat gag gcg tgg gac tat gcg gca ccc ccg gta tca       192
Val Ser Ser Val Tyr Glu Ala Trp Asp Tyr Ala Ala Pro Pro Val Ser
    50                  55                  60

aac ctc agc gag gcg cta tcg gga atc gtg gtt aag aca aaa tgt cca       240
Asn Leu Ser Glu Ala Leu Ser Gly Ile Val Val Lys Thr Lys Cys Pro
65                  70                  75                  80

gta ccg gaa gtt ata ctt tgg ttt aaa gac aaa caa atg gcg tac tgg       288
Val Pro Glu Val Ile Leu Trp Phe Lys Asp Lys Gln Met Ala Tyr Trp
                85                  90                  95

aca aat cca tac gtc acc tta aag ggg ctg gca caa tct gtt ggt gaa       336
Thr Asn Pro Tyr Val Thr Leu Lys Gly Leu Ala Gln Ser Val Gly Glu
            100                 105                 110

gaa cat aaa agc ggg gac ata cgc gat gct ttg ttg gat gcc ctt tcc       384
Glu His Lys Ser Gly Asp Ile Arg Asp Ala Leu Leu Asp Ala Leu Ser
        115                 120                 125

ggt gta tgg gta gac tct act cca tct tcc aca aat atc ccg gaa aat       432
Gly Val Trp Val Asp Ser Thr Pro Ser Ser Thr Asn Ile Pro Glu Asn
    130                 135                 140

gga tgt gtc tgg gga gcc gac cgt ttg ttc caa cgc gta tgc caa tga       480
Gly Cys Val Trp Gly Ala Asp Arg Leu Phe Gln Arg Val Cys Gln
145                 150                 155

<210>  89
<211>  159
<212>  PRT
<213>  Varicella zoster

<400>  89

Met Ala Ser His Lys Trp Leu Leu Gln Ile Val Phe Leu Lys Thr Ile
1               5                   10                  15

Thr Ile Ala Tyr Cys Leu His Leu Gln Asp Asp Thr Pro Leu Phe Phe
            20                  25                  30

Gly Ala Lys Pro Leu Ser Asp Val Ser Leu Ile Ile Thr Glu Pro Cys
        35                  40                  45

Val Ser Ser Val Tyr Glu Ala Trp Asp Tyr Ala Ala Pro Pro Val Ser
    50                  55                  60

Asn Leu Ser Glu Ala Leu Ser Gly Ile Val Val Lys Thr Lys Cys Pro
65                  70                  75                  80

Val Pro Glu Val Ile Leu Trp Phe Lys Asp Lys Gln Met Ala Tyr Trp
                85                  90                  95

Thr Asn Pro Tyr Val Thr Leu Lys Gly Leu Ala Gln Ser Val Gly Glu
            100                 105                 110

Glu His Lys Ser Gly Asp Ile Arg Asp Ala Leu Leu Asp Ala Leu Ser
        115                 120                 125

Gly Val Trp Val Asp Ser Thr Pro Ser Ser Thr Asn Ile Pro Glu Asn
    130                 135                 140

Gly Cys Val Trp Gly Ala Asp Arg Leu Phe Gln Arg Val Cys Gln
145                 150                 155

<210>  90
<211>  909
<212>  DNA
<213>  Varicella zoster

<220>
<221>  CDS
<222>  (1)..(909)

<400>  90
atg gct tct gta gca ggt aac gct agt aat atc tca cca cag ccc ccg       48
Met Ala Ser Val Ala Gly Asn Ala Ser Asn Ile Ser Pro Gln Pro Pro
1               5                   10                  15

tcg ggc gtt cca acc gga ggg gaa ttt gta ctg ata cct acc gcg tat       96
Ser Gly Val Pro Thr Gly Gly Glu Phe Val Leu Ile Pro Thr Ala Tyr
            20                  25                  30

tat tca cag ctg tta acc ggg cag act aaa aat ccg cag gta tca att      144
Tyr Ser Gln Leu Leu Thr Gly Gln Thr Lys Asn Pro Gln Val Ser Ile
        35                  40                  45

gga gct cca aat aac gga cag tat atc gtc ggg cca tat gga tct cca      192
Gly Ala Pro Asn Asn Gly Gln Tyr Ile Val Gly Pro Tyr Gly Ser Pro
    50                  55                  60

cac ccg cct gcc ttc cca cct aat aca ggg ggt tat ggt tgc cct ccg 240
His Pro Pro Ala Phe Pro Pro Asn Thr Gly Gly Tyr Gly Cys Pro Pro
65                  70                  75                  80

gga cac ttc ggg gga ccg tac ggg ttt ccg gga tat cca cca ccc aat 288
Gly His Phe Gly Gly Pro Tyr Gly Phe Pro Gly Tyr Pro Pro Pro Asn
                85                  90                  95

cgt ttg gaa atg caa atg tcc gca ttt atg aac gca ttg gcc gcc gaa 336
Arg Leu Glu Met Gln Met Ser Ala Phe Met Asn Ala Leu Ala Ala Glu
            100                 105                 110

cgg ggt att gac ttg cag acc ccg tgt gta aat ttt cca gac aaa acc 384
Arg Gly Ile Asp Leu Gln Thr Pro Cys Val Asn Phe Pro Asp Lys Thr
        115                 120                 125

gat gtc cgt cgt cca gga aaa cgg gat ttc aag agc atg gat caa agg 432
Asp Val Arg Arg Pro Gly Lys Arg Asp Phe Lys Ser Met Asp Gln Arg
    130                 135                 140

gaa ttg gat tct ttt tat agt ggg gag tct caa atg gac gga gag ttt 480
Glu Leu Asp Ser Phe Tyr Ser Gly Glu Ser Gln Met Asp Gly Glu Phe
145                 150                 155                 160

ccc tca aat ata tat ttt ccc ggt gaa cca acg tat ata acg cat cgg 528
Pro Ser Asn Ile Tyr Phe Pro Gly Glu Pro Thr Tyr Ile Thr His Arg
                165                 170                 175

aga cgt cga gtt tct cca tca tat tgg cag agg aga cac aga gtt tct 576
Arg Arg Arg Val Ser Pro Ser Tyr Trp Gln Arg Arg His Arg Val Ser
            180                 185                 190

aat ggt cag cac gaa gag ctt gct ggg gtt gtg gca aaa ctg caa cag 624
Asn Gly Gln His Glu Glu Leu Ala Gly Val Val Ala Lys Leu Gln Gln
        195                 200                 205

gag gtt aca gag cta aaa tca caa aat ggg aca caa atg cct ttg tcg 672
Glu Val Thr Glu Leu Lys Ser Gln Asn Gly Thr Gln Met Pro Leu Ser
    210                 215                 220

cac cat aca aat ata cca gag ggg aca cgg gat cct cga ata tcg att 720
His His Thr Asn Ile Pro Glu Gly Thr Arg Asp Pro Arg Ile Ser Ile
225                 230                 235                 240

tta tta aaa cag ctt caa agc gtt tcg ggt cta tgc tca tcc caa aat 768
Leu Leu Lys Gln Leu Gln Ser Val Ser Gly Leu Cys Ser Ser Gln Asn
                245                 250                 255

aca aca agc acc cca cat aca gat aca gtt gga caa gat gta aat gca 816
Thr Thr Ser Thr Pro His Thr Asp Thr Val Gly Gln Asp Val Asn Ala
            260                 265                 270

gtg gag gcg agt tcc aag gcc cct tta ata cag ggg tcc acg gca gac 864
Val Glu Ala Ser Ser Lys Ala Pro Leu Ile Gln Gly Ser Thr Ala Asp
        275                 280                 285

gac gcc gat atg ttt gca aat cag atg atg gtg ggg cgg tgt taa 909
Asp Ala Asp Met Phe Ala Asn Gln Met Met Val Gly Arg Cys
    290                 295                 300

<210> 91
<211> 302
<212> PRT
<213> Varicella zoster

<400> 91

Met Ala Ser Val Ala Gly Asn Ala Ser Asn Ile Ser Pro Gln Pro Pro
1               5                   10                  15

Ser Gly Val Pro Thr Gly Gly Glu Phe Val Leu Ile Pro Thr Ala Tyr
            20                  25                  30

Tyr Ser Gln Leu Leu Thr Gly Gln Thr Lys Asn Pro Gln Val Ser Ile
        35                  40                  45

Gly Ala Pro Asn Asn Gly Gln Tyr Ile Val Gly Pro Tyr Gly Ser Pro
    50                  55                  60

His Pro Pro Ala Phe Pro Pro Asn Thr Gly Gly Tyr Gly Cys Pro Pro
65                  70                  75                  80

Gly His Phe Gly Gly Pro Tyr Gly Phe Pro Gly Tyr Pro Pro Pro Asn
                85                  90                  95

Arg Leu Glu Met Gln Met Ser Ala Phe Met Asn Ala Leu Ala Ala Glu
            100                 105                 110

Arg Gly Ile Asp Leu Gln Thr Pro Cys Val Asn Phe Pro Asp Lys Thr
        115                 120                 125

Asp Val Arg Arg Pro Gly Lys Arg Asp Phe Lys Ser Met Asp Gln Arg
    130                 135                 140

Glu Leu Asp Ser Phe Tyr Ser Gly Glu Ser Gln Met Asp Gly Glu Phe
145                 150                 155                 160

Pro Ser Asn Ile Tyr Phe Pro Gly Glu Pro Thr Tyr Ile Thr His Arg
                165                 170                 175

Arg Arg Arg Val Ser Pro Ser Tyr Trp Gln Arg Arg His Arg Val Ser
            180                 185                 190

Asn Gly Gln His Glu Glu Leu Ala Gly Val Val Ala Lys Leu Gln Gln
        195                 200                 205

Glu Val Thr Glu Leu Lys Ser Gln Asn Gly Thr Gln Met Pro Leu Ser
    210                 215                 220

His His Thr Asn Ile Pro Glu Gly Thr Arg Asp Pro Arg Ile Ser Ile
225                 230                 235                 240

Leu Leu Lys Gln Leu Gln Ser Val Ser Gly Leu Cys Ser Ser Gln Asn
                245                 250                 255

Thr Thr Ser Thr Pro His Thr Asp Thr Val Gly Gln Asp Val Asn Ala
            260                 265                 270

Val Glu Ala Ser Ser Lys Ala Pro Leu Ile Gln Gly Ser Thr Ala Asp
        275                 280                 285

Asp Ala Asp Met Phe Ala Asn Gln Met Met Val Gly Arg Cys
    290                 295                 300